(19) Europäisches Patentamt
European Patent Office
Office européen des brevets

(11) EP 4 524 139 A1

(12) **EUROPEAN PATENT APPLICATION**
published in accordance with Art. 153(4) EPC

(43) Date of publication:
**19.03.2025 Bulletin 2025/12**

(21) Application number: **23802529.0**

(22) Date of filing: **29.03.2023**

(51) International Patent Classification (IPC):
***C07D 487/04*** (2006.01) ***C07D 487/00*** (2006.01)
***C07D 417/14*** (2006.01) ***A61K 31/5025*** (2006.01)
***A61P 25/00*** (2006.01) ***A61P 29/00*** (2006.01)

(52) Cooperative Patent Classification (CPC):
**A61K 31/5025; A61K 31/538; A61K 31/5386; A61K 31/553; A61P 25/00; A61P 25/04; A61P 25/28; A61P 29/00; C07D 417/14; C07D 487/00; C07D 487/04; C07D 519/00**

(86) International application number:
**PCT/CN2023/084772**

(87) International publication number:
**WO 2023/216753 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.05.2022 CN 202210542611**

(71) Applicant: **Shanghai Simr Biotechnology Co., Ltd.**
**Shanghai 201318 (CN)**

(72) Inventors:
- **WANG, Fei**
  **Shanghai 201318 (CN)**
- **CHEN, Nanyang**
  **Shanghai 201318 (CN)**
- **SUN, Yong**
  **Shanghai 201318 (CN)**

(74) Representative: **Dai, Simin**
**Reyda IP**
**A073**
**157, Quai du Président Roosevelt**
**92130 Issy-les-Moulineaux (FR)**

(54) **IMIDAZOPYRIDAZINE SUBSTITUTED BENZENE RING DERIVATIVE, PREPARATION METHOD, PHARMACEUTICAL COMPOSITION AND USE**

(57) The present invention relates to an imidazopyridazine derivative, and a preparation method therefor, a pharmaceutical composition thereof and the use thereof. Provided in the present invention are a compound as represented by formula (1), and a stereoisomer, a tautomer, a prodrug, a pharmaceutically acceptable salt, an amorphous substance, an isotope, a polymorph or a solvate thereof. Further provided are a pharmaceutical composition comprising the compound, and the use of the compound as a $GABA_A$ receptor modulator.

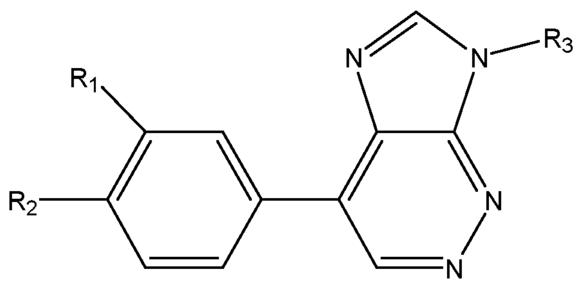

(1)

EP 4 524 139 A1

## Description

CROSS REFERENCE TO RELATED APPLICATIONS

The present application claims the right of the priority of Chinese patent application No. 202210542611.3 filed with the State Intellectual Property Office of the People's Republic of China on May 10, 2022 and entitled "IMIDAZOPYRIDAZINE SUBSTITUTED BENZENE RING DERIVATIVE, PREPARATION METHOD, PHARMACEUTICAL COMPOSITION AND USE". The contents of the above Chinese patent application are incorporated herein by reference in its entirety.

TECHNICAL FIELD

The present disclosure relates to an imidazopyridazine-substituted benzene ring derivative with a modulatory function on a $GABA_A$ receptor, a preparation method therefor, a pharmaceutical composition thereof, and a use thereof as a medicament.

BACKGROUND

Gamma-aminobutyric acid (GABA) is an important inhibitory neurotransmitter in the mammalian central nervous system. Substances that modulate GABAergic neurotransmission are widely used in the treatment of various conditions such as epilepsy, anxiety, and depression. There are two types of GABA receptors in nature. One is $GABA_A$ receptor ($GABA_AR$), which is a member of ligand-gated ion channel superfamily, and the other is $GABA_B$ receptor ($GABA_BR$), which is a member of G protein-coupled receptor superfamily. $GABA_A$ receptor subunits found in mammals include α1-6, β1-4, γ1-3, δ, ε, θ, ρ1-2, *etc.*, among which α subunit, β subunit, and γ subunit are essential for the formation of a complete and functional $GABA_A$ receptor, and α subunit is crucial for the binding of benzodiazepines to the $GABA_A$ receptor.

Drugs bound at an allosteric binding site can be positive allosteric modulators (or forward allosteric modulators) that increase receptor activity, negative allosteric modulators (or reverse allosteric modulators) that decrease receptor activity, or neutral allosteric modulators that do not alter receptor activity (which refer to compounds that bind to the allosteric binding site but do not modulate receptor activity). Recent evidence suggests that $GABA_A$ receptors containing α2 or α3 subunits (referred to herein as α2/3-$GABA_A$ receptors) may be involved in certain pain states, and that positive allosteric modulators of these receptors may be potent analgesics (Mirza, N. R. and Munro, G., Drug News and Perspectives, 2010, 23(6), 351-360).

International patent applications PCT/GB01/04948 (published as WO2002/038568) and PCT/GB02/03114 (published as WO2003/008418) describe 7-phenylimidazo[1,2-b][1,2,4]triazine derivatives, which have an affinity for α2, α3, and/or α5 subunits. International patent application PCT/US99/14935 (published as WO2000/001697) discloses, *inter alia,* 4-phenyl-7*H*-imidazo[4,5-*c*]pyridazine derivatives, which are corticotropin releasing factor antagonists. International patent applications PCT/IB2013/060631 (published as WO2014/091368) and PCT/IB2015/054200 (published as WO2015/189744) and the article by Robert M. Owen (Robert M. Owen, J. Med. Chem. 2019, 62, 5773-5796) describe 4-(biphenyl-3-yl)-7*H*-imidazo[4,5-c]pyridazine derivatives, which interact with the α2/3-$GABA_A$ receptor and are used in the treatment of a variety of diseases including pain. This class of compounds are also used in the treatment of pruritus (International patent application PCT/US2019/033598, published as WO2019/26820A1) and epilepsy (Duveau, V., CNS Neurosci. Ther. 2019. 25(2): p. 255-260).

The prevailing view is that the modulatory activity of $GABA_A$ receptors containing α1 subunits is the main source of side effects (*e.g.*, sedation, addiction, lethargy, and amnesia) of current $GABA_A$ modulators (*e.g.,* benzodiazepines) (Uwe Rudolph and Frederic Knoflach, Nature Reviews: Drug Discovery, 2011, 10(9), 685-697). The search for new compounds that interact with the $GABA_A$ receptor and have fewer α1-$GABA_A$ receptor-associated side effects will have great therapeutic potential.

CONTENT OF THE PRESENT INVENTION

To this end, the technical problem to be solved by the present disclosure is to provide an imidazopyridazine-substituted benzene ring compound of formula (1), or a derivative, a stereoisomer, a tautomer, a prodrug, a pharmaceutically acceptable salt, an amorphous substance, a polymorph, or a solvate thereof:

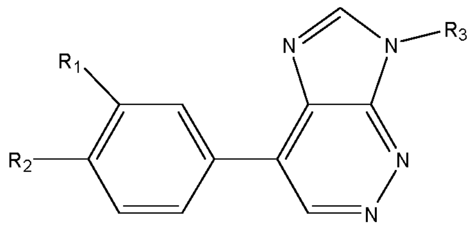

formula (1)

wherein

$R_1$ is a fused group formed by a substituted or unsubstituted benzene ring and a heterocyclic ring;
$R_2$ is selected from an H group, a halogen group, an OH group, a $C_1$-$C_6$ alkoxy group, or a CN group;
$R_3$ is selected from an H group, a substituted or unsubstituted linear or branched $C_1$-$C_6$ alkyl group, or a substituted or unsubstituted $C_3$-$C_6$ cycloalkyl group.

Unless otherwise indicated, the following definitions are provided to illustrate and define the meaning and scope of the various terms used herein in describing the present disclosure.

Whether used alone or in combination, the following definitions of general terms apply.

The nomenclature used in the present disclosure is based on AutoNom™ 2000, a Beilstein Institute computerized system for the generation of IUPAC systematic nomenclature. The presence of any open valence bond on a carbon, oxygen, sulfur, or nitrogen atom in the structures provided herein indicates the presence of a hydrogen atom.

Unless otherwise specified, the term "substituted" means that a specified group or moiety may have 1, 2, 3, 4, 5, or 6 substituents thereon. When a group may have a plurality of substituents thereon and a plurality of possible substituents are given, the substituents are independently selected and need not be identical.

The term "unsubstituted" means that a specified group has no substituents thereon.

The term "optionally substituted" means that a specified group is unsubstituted or substituted by one or more substituents independently selected from the possible substituents.

When specifying the number of substituents, the term "one or more" refers to one substitution to the maximum possible number of substitutions, *i.e.,* substitution of one hydrogen to substitution of all hydrogens by substituents. Unless otherwise indicated, 1, 2, 3, 4, or 5 substituents are preferred.

Specifically, in the definitions of $R_1$ and $R_3$ groups, "substituted" means that one or more hydrogens on the group are substituted by a group selected from $C_1$-$C_4$ alkyl, $C_1$-$C_4$ alkoxy, halo-$C_1$-$C_4$ alkyl, or halogen.

The term "halogen" refers to fluorine, chlorine, bromine, and iodine, preferably fluorine.

The compound of the present disclosure may contain an asymmetric or chiral center, and is thus present in different stereoisomeric forms. All stereoisomeric forms of the compound of the present disclosure, including, but not limited to, diastereomers, enantiomers, atropisomers, and mixtures thereof such as racemic mixtures, will form part of the present disclosure. In the present disclosure, all stereoisomers are contemplated when the stereochemistry of any particular chiral atom is not determined. Furthermore, the present disclosure relates to all geometric and positional isomers. The compound of the present disclosure may be present in different tautomeric forms, and all those forms fall within the scope of the present disclosure. All stereoisomers of the compound of the present disclosure are expected to include a mixture form or a pure or substantially pure form. The resolution may be achieved by physical methods such as fractional crystallization, separation or crystallization of diastereomeric derivatives, or separation by chiral column chromatography.

The term "prodrug" is a functional derivative of the compound of formula (1) that is readily converted *in vivo* to the compound of formula (1). Suitable derivatives may be selected and prepared by conventional techniques well known to those skilled in the art, see, for example, Design of Prodrugs, ed. H. Bundgaard, Elsevier, 1985.

The term "pharmaceutically acceptable salt" as used herein refers to a pharmaceutically acceptable organic or inorganic salt of the compound of the present disclosure. Exemplary salts include, but are not limited to, sulfate, citrate, acetate, oxalate, chloride, bromide, iodide, nitrate, bisulfate, isonicotinate, lactate, salicylate, acid citrate, succinate, maleate, fumarate, gluconate, formate, methanesulfonate, and pamoate. The "pharmaceutically acceptable salt" may relate to the inclusion of another molecule such as a maleate or other counterions. The counterion stabilizes the charge in the parent compound. The "pharmaceutically acceptable salt" may have more than one charged atom, and the more than one charged atom may have multiple counterions.

If the compound of the present disclosure is a base, the desired "pharmaceutically acceptable salt" may be prepared by a suitable method, for example, treating the free base with an inorganic acid, including hydrochloric acid, hydrobromic acid, sulfuric acid, nitric acid, or phosphoric acid; or with an organic acid, including acetic acid, maleic acid,

succinic acid, mandelic acid, fumaric acid, malonic acid, pyruvic acid, salicylic acid, pyranoside acid such as glucuronic acid or galacturonic acid, α-hydroxy acid such as citric acid or tartaric acid, amino acid such as glutamic acid, aromatic acid such as benzoic acid or cinnamic acid, or sulfonic acid such as methanesulfonic acid or *p*-toluenesulfonic acid.

If the compound of the present disclosure is an acid, the desired "pharmaceutically acceptable salt" may be prepared by a suitable method, for example, treating the free acid with an inorganic or organic base, including an amine, an alkali metal hydroxide, or an alkaline earth metal hydroxide, *etc.* Illustrative examples of suitable salts include, but are not limited to, organic salts derived from amino acids, salts of primary, secondary, and tertiary amines, salts of cyclic amines such as piperidine, morpholine, and piperazine, and inorganic salts derived from sodium, calcium, potassium, magnesium, manganese, iron, copper, zinc, aluminum, and lithium.

The compound of the present disclosure may be present in a continuous solid state ranging from completely amorphous to completely crystalline. The term "amorphous" refers to a state in which a material lacks long-range order at the molecular level and, depending on the temperature, can exhibit the physical properties of a solid or a liquid. Typically, without giving a distinct X-ray diffraction pattern while showing the properties of a solid, the material is more formally described as a liquid. On heating, a change occurs from the properties of a solid to the properties of a liquid, characterized by a change of state, usually second order ("glass transition"). The term "crystal" refers to a solid phase in which a material has a regularly ordered internal structure at the molecular level and gives a unique X-ray diffraction pattern with defined peaks. Such a material will also exhibit the properties of a liquid when sufficiently heated, but the change from solid to liquid is characterized by a phase change, usually first order ("melting point").

The term "polymorph" as used herein refers to different solid crystalline phases of certain compounds of the present disclosure in the solid state due to the presence of two or more different molecular arrangements. Certain compounds of the present disclosure may be present in more than one crystal form, and the present disclosure is intended to include various crystal forms and mixtures thereof.

The term "solvate" as used herein refers to a conjugate or complex of one or more solvent molecules with the compound of the present disclosure. Examples of solvents that form solvates include, but are not limited to, water, isopropanol, ethanol, methanol, dimethyl sulfoxide, ethyl acetate, acetic acid, and ethanolamine. The compound of the present disclosure may be present in an unsolvated form or in a solvated form with a pharmaceutically acceptable solvent such as water and ethanol, and thus the present disclosure is intended to include both solvated and unsolvated forms.

The compound of the present disclosure may contain an unnatural proportion of atomic isotopes on one or more of the atoms constituting the compound. The term "isotopic variant" refers to having the same atomic number, but having an atomic mass or mass number different from the atomic mass or mass number found predominantly in nature. For example, the compound can be radiolabeled with a radioactive isotope, such as deuterium ($^2H$), tritium ($^3H$), iodine-125 ($^{125}I$), or C-14 ($^{14}C$). All isotopic variations of the compound of the present disclosure, whether radioactive or not, are encompassed within the scope of the present disclosure. Isotopic variants may enhance certain therapeutic benefits, for example, deuterium enrichment can increase *in vivo* half-life or reduce dosage requirements, or can provide standard compounds that can be used as characterization of biological samples. Isotope-enriched compounds of formula (1) can be prepared by conventional techniques well known to those skilled in the art, or by methods similar to those described in the routes and examples herein, using appropriate isotope-enriched reagents and/or intermediates without undue experimentation.

**[$R_2$]**

In the compound of the present disclosure, the $R_2$ is selected from an H group, a halogen group, an OH group, a $C_1$-$C_6$ alkoxy group, or a CN group; preferably, the $R_2$ is preferably an H group or a halogen group, more preferably an H group or an F group.

**[$R_3$]**

In the compound of the present disclosure, the $R_3$ is selected from an H group, a substituted or unsubstituted linear or branched $C_1$-$C_6$ alkyl group, or a substituted or unsubstituted $C_3$-$C_6$ cycloalkyl group. In $R_3$, the $C_1$-$C_6$ alkyl group may be linear or branched, and exemplary $C_1$-$C_6$ alkyl groups include a methyl group, an ethyl group, an *n*-propyl (1-propyl) group, an isopropyl (2-propyl, 1-methylethyl) group, a *n*-butyl (1-butyl) group, a *sec*-butyl (2-butyl, 1-methylpropyl) group, an isobutyl (2-methylpropyl) group, or a Fe/F-butyl (1,1-dimethylethyl) group. One to more hydrogen atoms on the above alkyl group may be substituted by a substituent such as a methyl group, an ethyl group, an *n*-propyl group, a methoxy group, an ethoxy group, a propoxy group, a fluoromethyl group, a fluoroethyl group, a fluorine group, a chlorine group, or a bromine group.

The term "cycloalkyl group" refers to a monovalent saturated cyclic hydrocarbon group. In $R_3$, exemplary $C_3$-$C_5$ cycloalkyl groups include a cyclopropyl group, a cyclobutyl group, or a cyclopentyl group.

In $R_3$, exemplary $C_3$-$C_6$ cycloalkyl groups include a 1-methylcyclopropyl group, a 2-methylcyclopropyl group, a 1-methylcyclobutyl group, a 2-methylcyclobutyl group, a 3-methylcyclobutyl group, a 1-methylcyclopentyl group, a 2-methylcyclopentyl group, or a 3-methylcyclopentyl group. One to more hydrogen atoms on the above cycloalkyl group may be substituted by a substituent such as a methyl group, an ethyl group, an *n*-propyl group, a methoxy group, an ethoxy group, a propoxy group, a fluoromethyl group, a fluoroethyl group, a fluorine group, a chlorine group, or a bromine group.

The term "alkoxy group" refers to an alkyloxy group. In $R_3$, exemplary $C_1$-$C_6$ alkoxy groups include a methoxy group, an ethoxy group, an *n*-propoxy group, an isopropoxy group, an *n*-butoxy group, a *sec*-butoxy group, *etc.* One to more hydrogen atoms on the above alkoxy group may be substituted by a substituent such as a methyl group, an ethyl group, an *n*-propyl group, a methoxy group, an ethoxy group, a propoxy group, a fluoromethyl group, a fluoroethyl group, a fluorine group, a chlorine group, or a bromine group.

In a preferred embodiment of the present disclosure, $R_3$ is selected from a linear or branched $C_1$-$C_6$ alkyl group, a $C_1$-$C_6$ alkoxy group, and is more preferably a $C_2$-$C_4$ alkyl group, more preferably an ethyl group or an isopropyl group.

**[$R_1$]**

In the schemes of the present disclosure, the $R_1$ is a fused group of a benzene ring and a heterocyclic ring; the heterocyclic ring refers to a saturated or partially unsaturated monocyclic or polycyclic group having a heteroatom of N, O, or S. Preferably, the two heterocyclic rings are independently a 3- to 7-membered saturated or partially unsaturated monocyclic or polycyclic group containing 1 to 3 ring heteroatoms selected from N, O, or S. S and O may be present as -SO or $SO_2$.

As a preferred structure, in the compound of the present disclosure, the heterocyclic ring forming the fused group in $R_1$ is a 5- to 7-membered saturated or unsaturated monocyclic or bicyclic group containing 1 to 3 ring heteroatoms selected from N, O, or S. More preferably, the heterocyclic ring is a 5- to 6-membered saturated or unsaturated monocyclic group containing 1, 2, or 3 ring heteroatoms selected from N and/or O, more preferably a heterocyclic ring containing only N or both N and O as heteroatoms.

Preferably, the $R_1$ has a structure of formula (M) as follows:

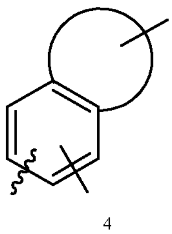

(M);

wherein m is 1, 2, or 3, n is 1 or 2, and 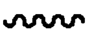 represents a connection site;
ring A refers to a 5- to 7-membered saturated or partially unsaturated monocyclic or bicyclic ring containing at least one heteroatom selected from N, O, or S.

The ring A refers to a 5- to 7-membered saturated or partially unsaturated monocyclic ring containing at least one heteroatom selected from N, O, or S; m is 1, 2, or 3, n is 1 or 2, and 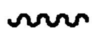 represents a connection site.

$R_1$ is attached to the phenyl group of the compound of formula (1) via 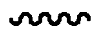.

$R_4$ or $R_5$ is a substituent on the heterocyclic ring, and may be attached to a heteroatom or to a carbon atom.

As a more preferred structure, the ring A is a 5- to 6-membered saturated or unsaturated monocyclic group containing 1 to 3 heteroatoms selected from at least one of N, O, or S; specifically, when the $R_1$ has the structure of formula (M), the $R_1$ has a structure of (a) to (e) as follows:

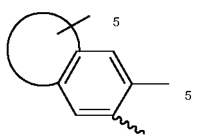, 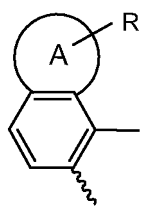, 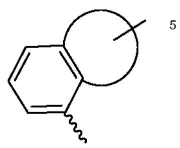, 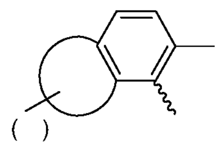, or 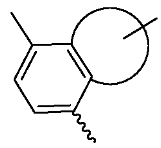.

(a) (b) (c) (d) (e)

More preferably, for the above structure of ring A, when the $R_1$ has the structure of formula (M), the ring A may be selected from the following structures:

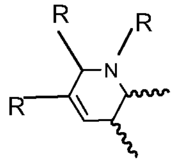 , 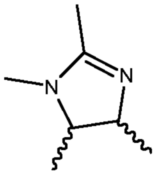 , 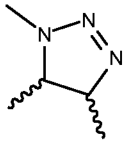 , 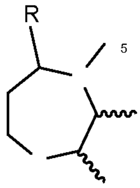 , 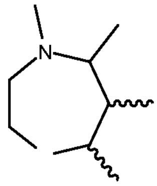 , 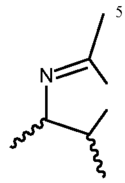 ,

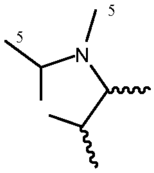 , 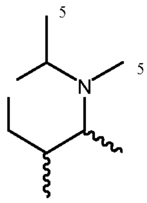 , 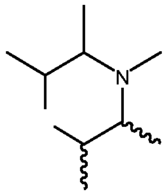 , 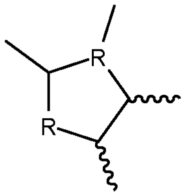 , 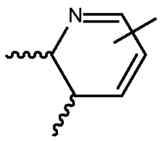 , 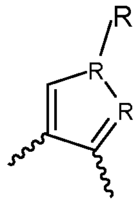 ,

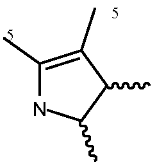 , 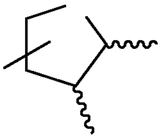 , 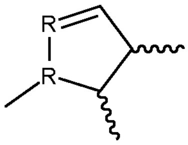 , 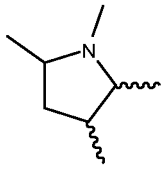 , 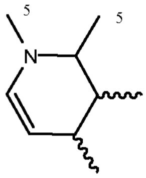 , 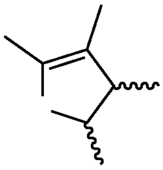 ,

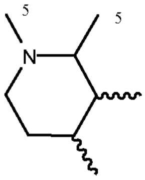 , 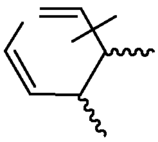 , 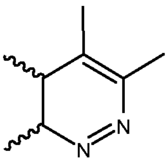 , 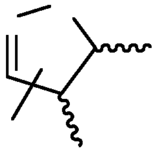 , 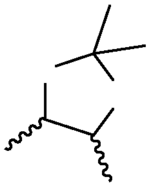 , 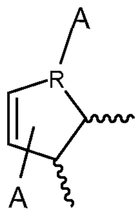 ,

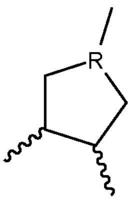 , 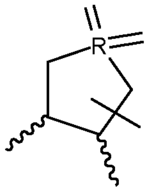 , 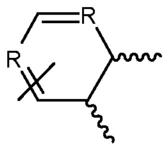 , 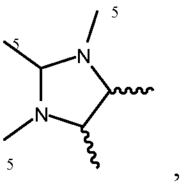 , 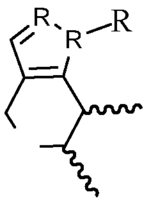 , 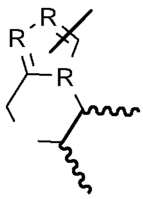 ,

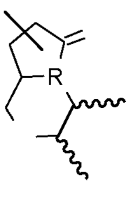 , or 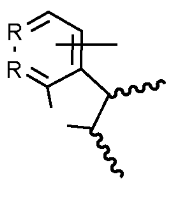 .

As an optional structure, the $R_1$ has a structure of formula (N) as follows:

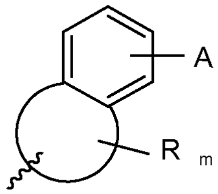

(N);

as an optional structure, when the $R_1$ has the structure of formula (N), the ring A may be selected from the following structure:

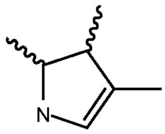

.

For the optional structure of $R_1$, the $R_4$ is selected from a hydrogen group, a $C_1$-$C_6$ alkyl group, a $C_3$-$C_6$ cycloalkyl group, or a $C_1$-$C_6$ alkoxy group; the above groups are optionally unsubstituted or each independently substituted by 1 to 4 groups each selected from at least one of halogen, hydroxyl, $C_1$-$C_3$ alkyl, halo-$C_1$-$C_3$ alkyl, $C_1$-$C_3$ alkoxy, or halo-$C_1$-$C_3$ alkoxy.

Preferably, the $R_4$ is selected from a hydrogen group, a $C_1$-$C_3$ alkyl group, or a $C_1$-$C_3$ alkoxy group; the above groups are optionally unsubstituted or each independently substituted by a $C_1$-$C_3$ alkoxy group or a halogen group.

More preferably, the $R_4$ is selected from a methoxy-$C_1$-$C_3$ alkyl group or a $C_1$-$C_3$ alkoxy group; more preferably a methoxymethyl group or a methoxy group.

For the optional structure of $R_1$, in $R_5$, the heteroatom is selected from N, O, or S.

The $R_5$ is selected from a hydrogen group, a halogen group, a hydroxyl group, an oxo group, a $C_1$-$C_6$ alkylacyl group, a $C_1$-$C_6$ alkylamido group, a $C_1$-$C_6$ alkoxyimino group, a $C_1$-$C_6$ alkyl group, a $C_3$-$C_6$ cycloalkyl group, a $C_1$-$C_6$ alkoxy group, a $C_6$-$C_{10}$ aryl group, a 5- to 7-membered heteroaryl group containing 1 to 3 heteroatoms, or a $C_3$-$C_7$ non-aromatic heterocyclyl group containing 1 to 3 heteroatoms; the above groups are optionally unsubstituted or each independently substituted by 1 to 4 groups each selected from at least one of halogen, hydroxyl, oxo, $C_1$-$C_4$ acyl, $C_1$-$C_3$ alkyl, halo-$C_1$-$C_3$ alkyl, $C_1$-$C_3$ alkoxy, halo-$C_1$-$C_3$ alkoxy, or sulfonyl.

As a preferred structure, the $R_5$ is selected from a hydrogen group, a halogen group, an oxo group, a $C_1$-$C_3$ alkylacyl group, a $C_1$-$C_3$ alkylamido group, a $C_1$-$C_3$ alkoxyimino group, a $C_1$-$C_3$ alkyl group, a $C_3$-$C_4$ cycloalkyl group, a $C_1$-$C_3$ alkoxy group, a $C_6$-$C_8$ aryl group, a 5-to 6-membered heteroaryl group containing 1 to 2 heteroatoms, or a $C_4$-$C_6$ non-aromatic heterocyclyl group containing 1 to 2 heteroatoms; the above groups are optionally unsubstituted or each independently substituted by 1 to 2 groups each selected from at least one of halogen, hydroxyl, oxo, $C_1$-$C_3$ acyl, $C_1$-$C_3$ alkyl, halo-$C_1$-$C_3$ alkyl, $C_1$-$C_3$ alkoxy, halo-$C_1$-$C_3$ alkoxy, or sulfonyl.

As a more preferred structure, the $R_5$ is selected from an H group, an oxo group, a methyl group, an ethyl group, an isopropyl group, an acetyl group, a methylmethoxy group, an ethylmethoxy group, a cyclopropyl group, a methylcyclopropyl group, an ethylcyclopropyl group, a methylcyclobutyl group, a methylamino group, a phenyl group,

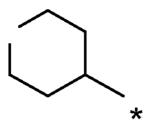

,

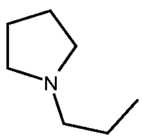 , 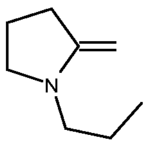 , 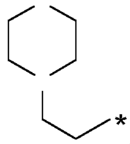 , 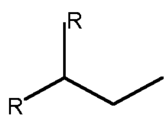 , 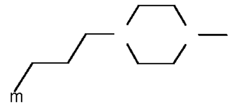 , 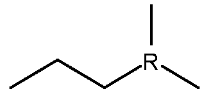 ,

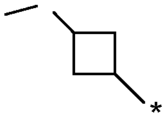, 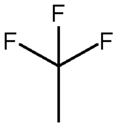, 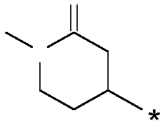, 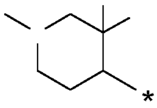, 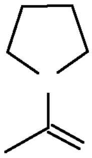, 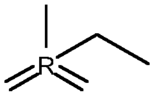, 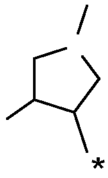,

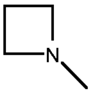, 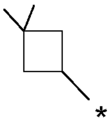, 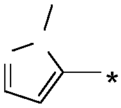, 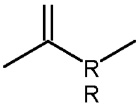, 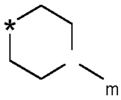, 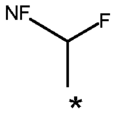, 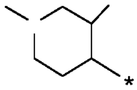, 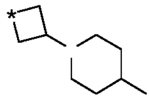,

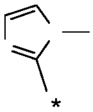, 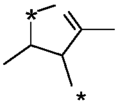, 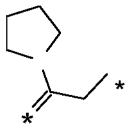, 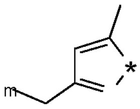, 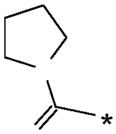, 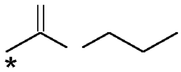, 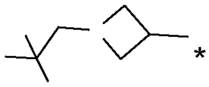,

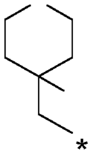, 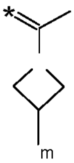, 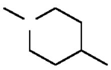, 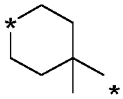, 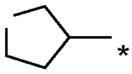, 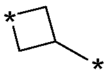, 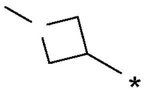, 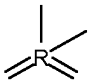,

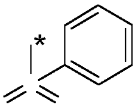, 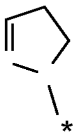, or 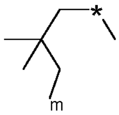.

In the compound of the present disclosure, the $R_1$ is selected from any one of the following structures:

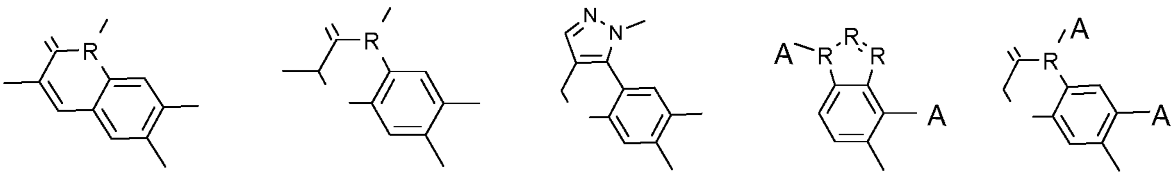, , , , ,

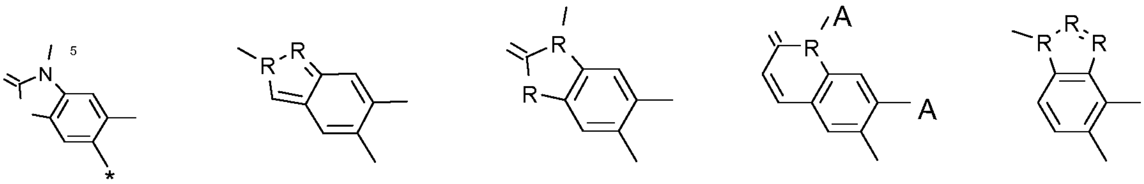, , , , ,

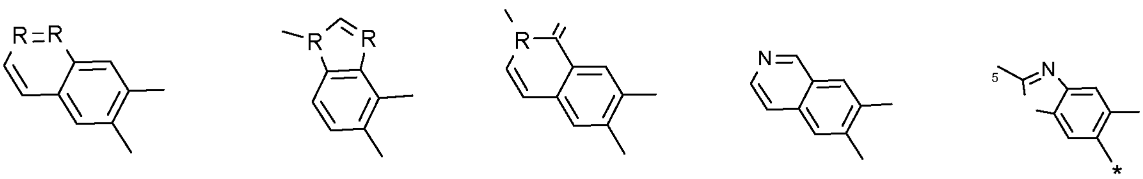, , , , ,

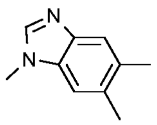, 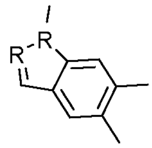, 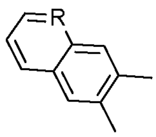, 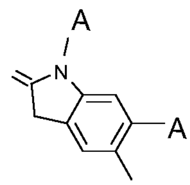, or 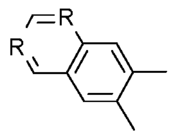;

and,

the $R_4$ is selected from an H group, a $C_1$-$C_3$ alkoxy group, or a substituted or unsubstituted methoxy-$C_1$-$C_3$ alkyl group; preferably an H group, a methylmethoxy group, an ethylmethoxy group, a methoxy group, or a difluoromethoxy group; the $R_5$ is selected from an H group, a methyl group, an ethyl group, an acetyl group, an ethylmethoxy group, a methylmethoxy group, a methylcyclopropyl group, a phenyl group,

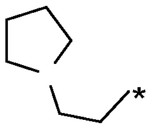, 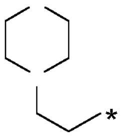, 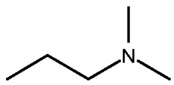, 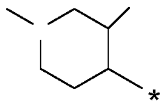, 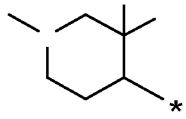, 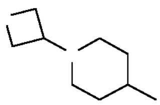,

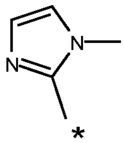, 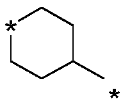, 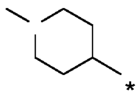, 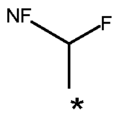, 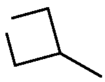, 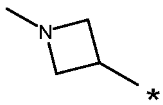, or 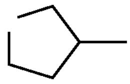.

In the most preferred structure, the compound is selected from any one of the compounds in Table 1 below.

Table 1: Preferred compounds

| No. | Structural formula | No. | Structural formula | No. | Structural formula |
|---|---|---|---|---|---|
| 1 | 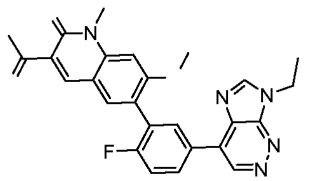 | 57 | 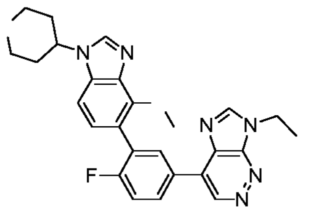 | 113 | 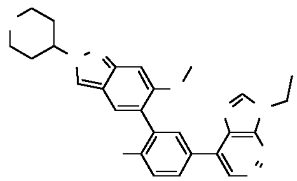 |
| 2 | 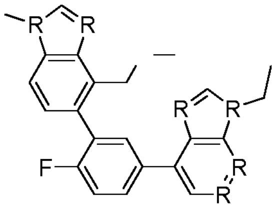 | 58 | 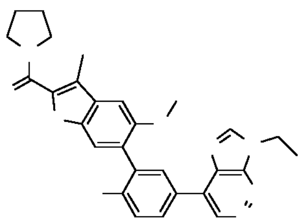 | 114 | 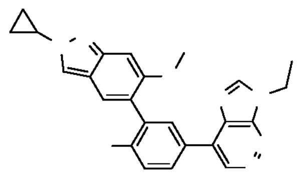 |
| 3 | 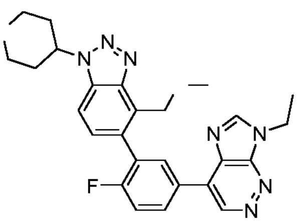 | 59 | 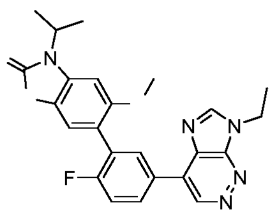 | 115 | 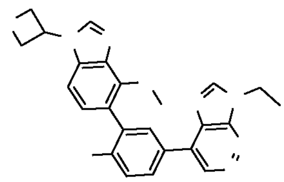 |

(continued)

| No. | Structural formula | No. | Structural formula | No. | Structural formula |
|---|---|---|---|---|---|
| 4 | 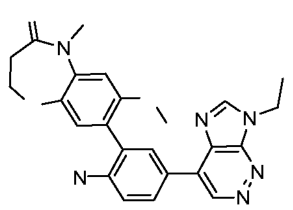 | 60 | 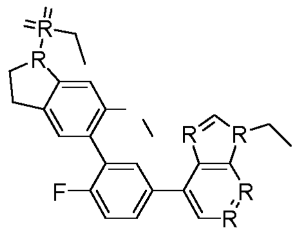 | 116 | 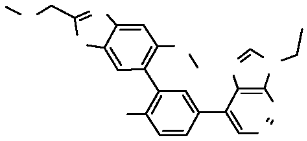 |
| 5 | 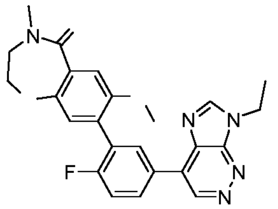 | 61 | 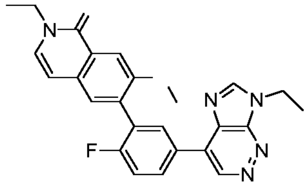 | 117 | 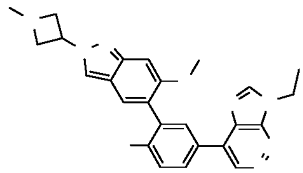 |
| 6 | 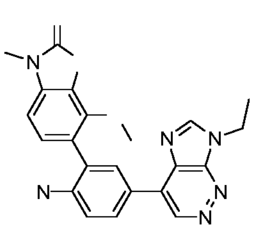 | 62 | 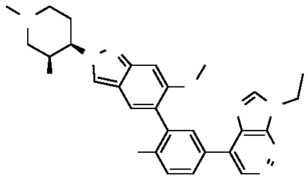 | 118 | 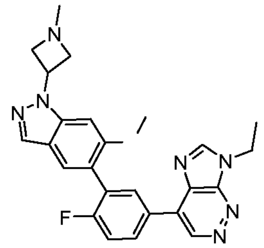 |
| 7 | 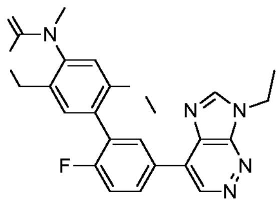 | 63 | 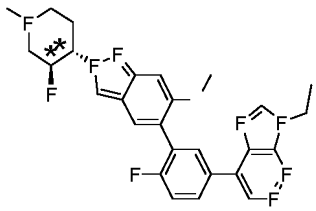 | 119 | 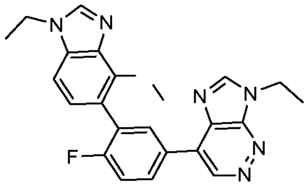 |
| 8 | 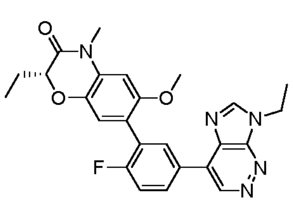 | 64 | 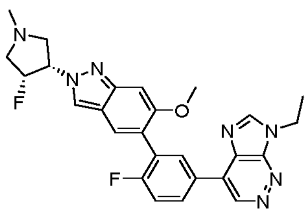 | 120 | 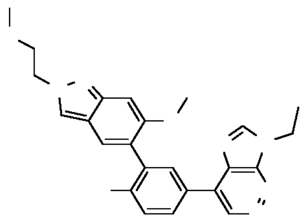 |
| 9 | 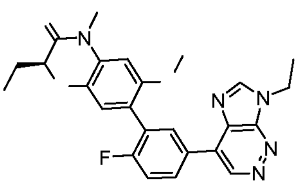 | 65 | 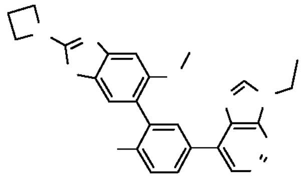 | 121 | 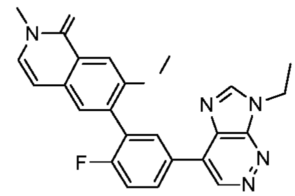 |
| 10 | 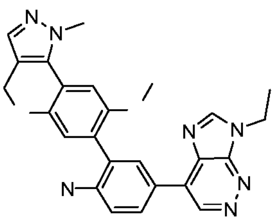 | 66 | 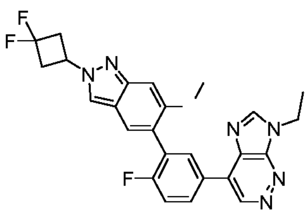 | 122 | 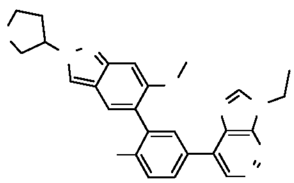 |

(continued)

| No. | Structural formula | No. | Structural formula | No. | Structural formula |
|---|---|---|---|---|---|
| 11 | 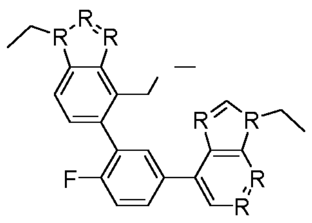 | 67 | 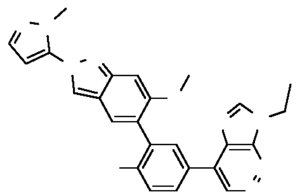 | 123 | 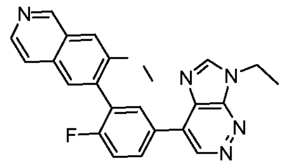 |
| 12 | 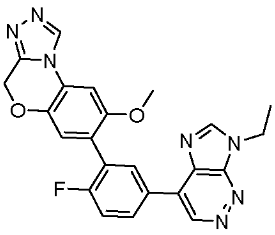 | 68 | 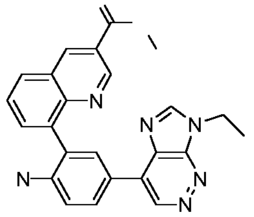 | 124 | 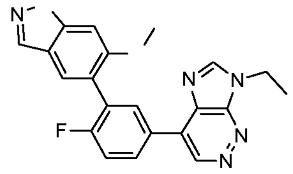 |
| 13 | 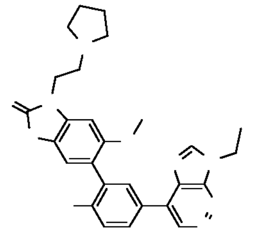 | 69 | 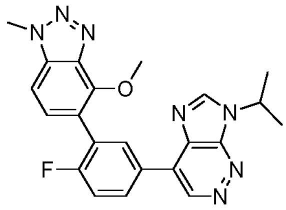 | 125 | 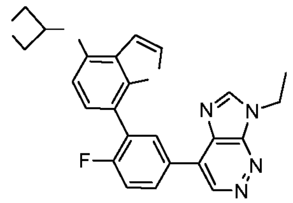 |
| 14 | 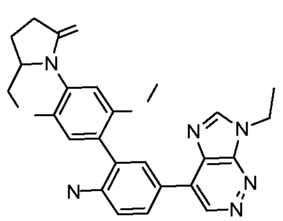 | 70 | 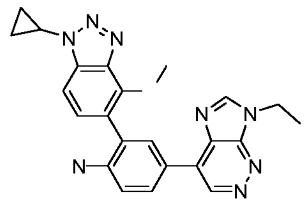 | 126 | 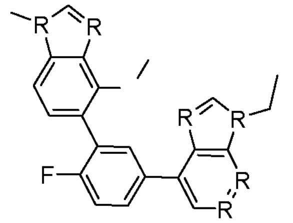 |
| 15 | 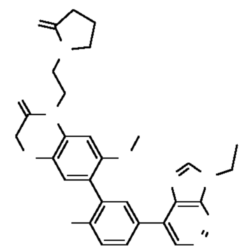 | 71 | 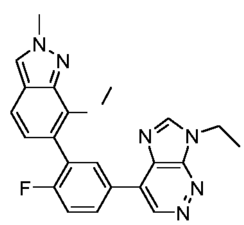 | 127 | 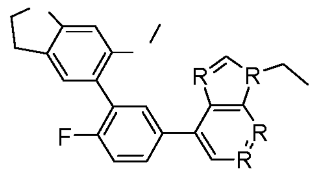 |
| 16 | 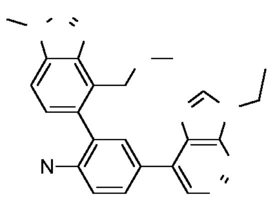 | 72 | 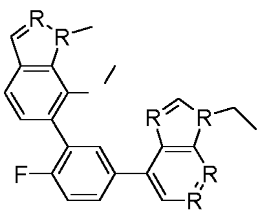 | 128 | 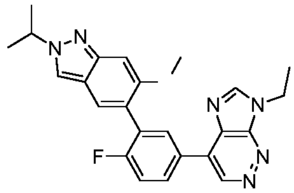 |
| 17 | 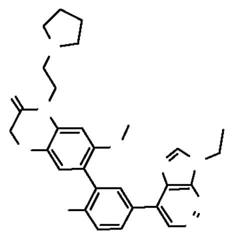 | 73 | 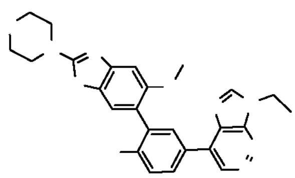 | 129 | 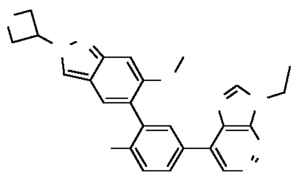 |

(continued)

| No. | Structural formula | No. | Structural formula | No. | Structural formula |
|---|---|---|---|---|---|
| 18 | 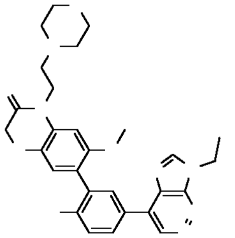 | 74 | 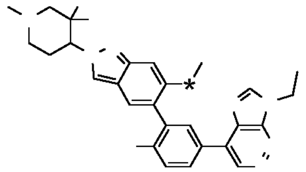 | 130 | 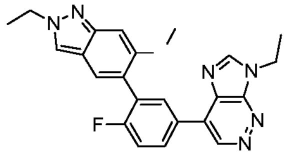 |
| 19 | 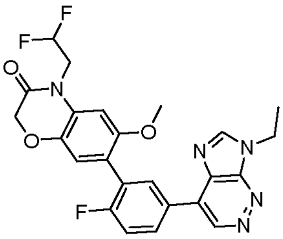 | 75 | 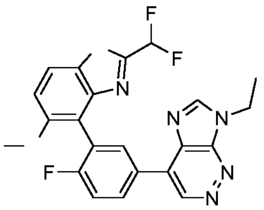 | 131 | 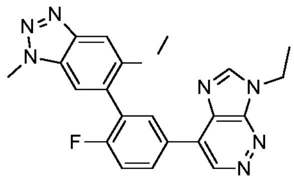 |
| 20 | 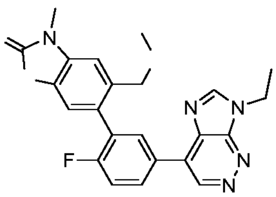 | 76 | 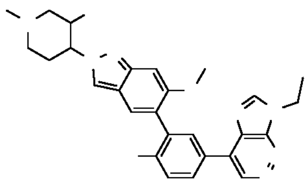 | 132 | 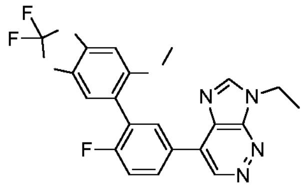 |
| 21 | 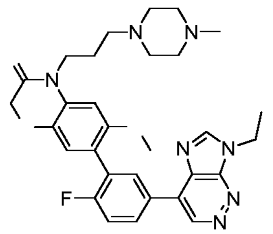 | 77 | 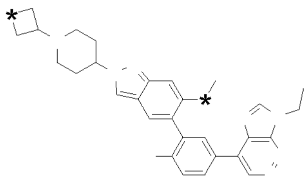 | 133 | 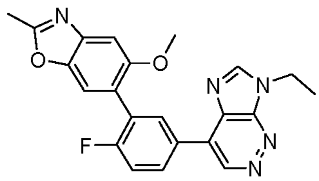 |
| 22 | 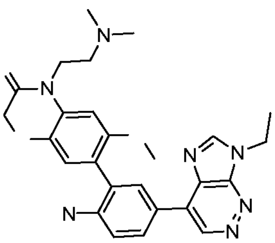 | 78 | 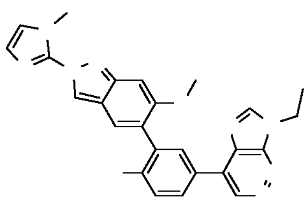 | 134 | 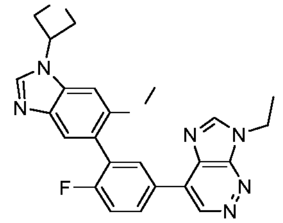 |
| 23 | 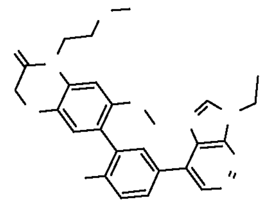 | 79 | 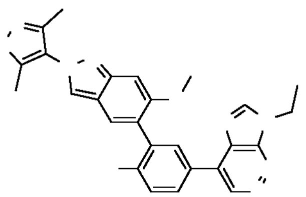 | 135 | 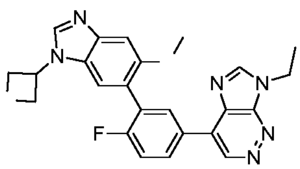 |
| 24 | 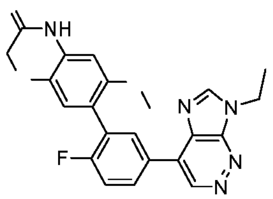 | 80 | 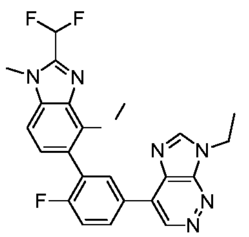 | 136 | 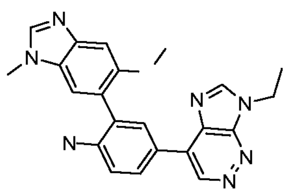 |

(continued)

| No. | Structural formula | No. | Structural formula | No. | Structural formula |
|---|---|---|---|---|---|
| 25 | 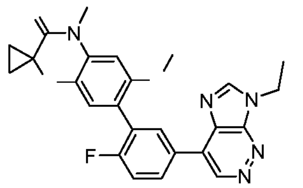 | 81 | 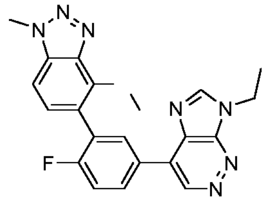 | 137 | 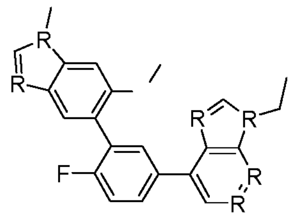 |
| 26 | 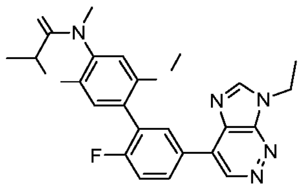 | 82 | 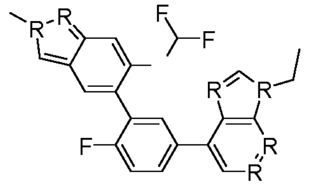 | 138 | 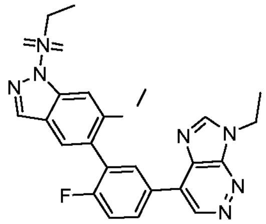 |
| 27 | 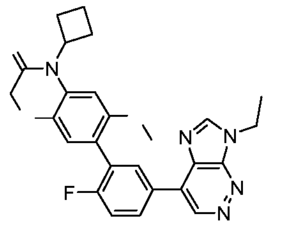 | 83 | 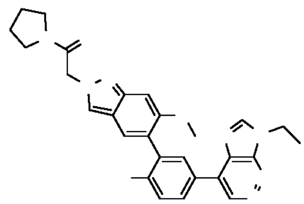 | 139 | 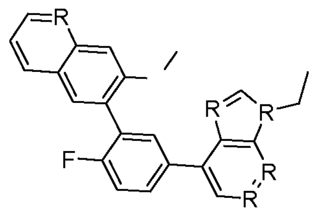 |
| 28 | 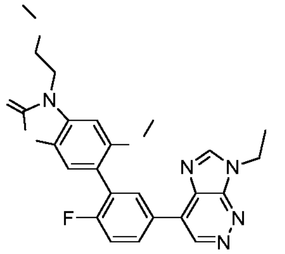 | 84 | 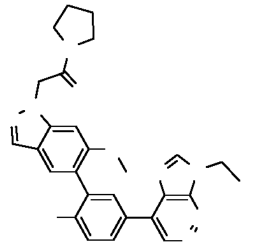 | 140 | 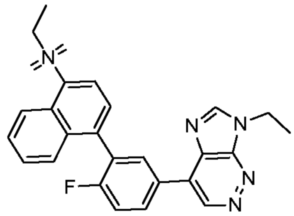 |
| 29 | 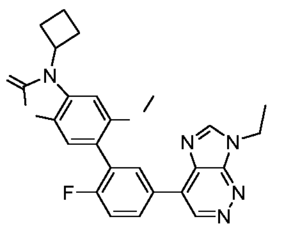 | 85 | 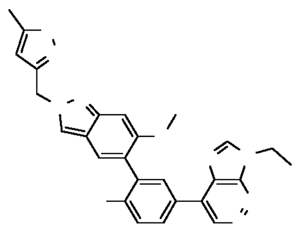 | 141 | 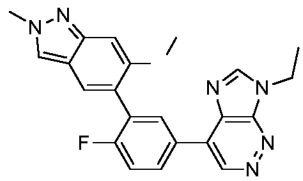 |
| 30 | 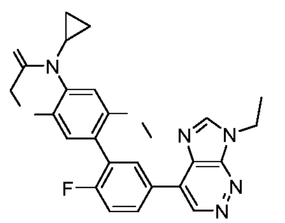 | 86 | 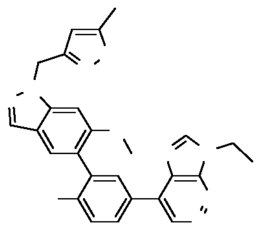 | 142 | 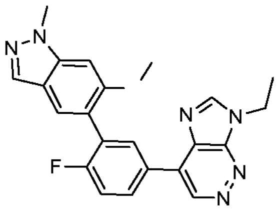 |
| 31 | 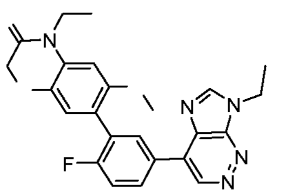 | 87 | 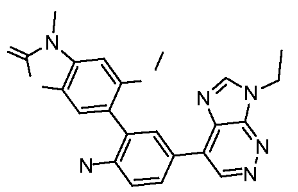 | 143 | 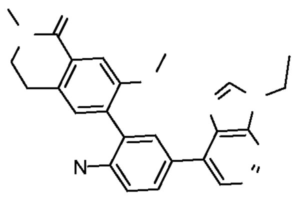 |

(continued)

| No. | Structural formula | No. | Structural formula | No. | Structural formula |
|---|---|---|---|---|---|
| 32 | 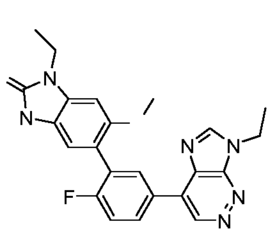 | 88 | 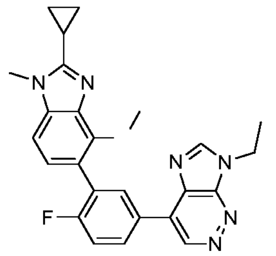 | 144 | 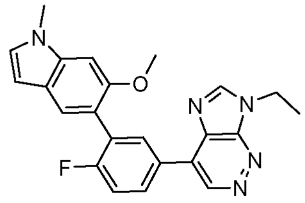 |
| 33 | 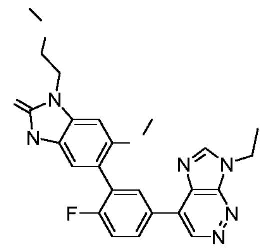 | 89 | 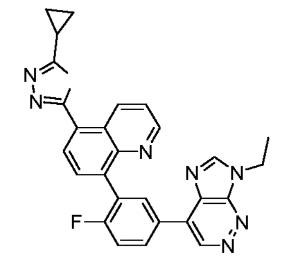 | 145 | 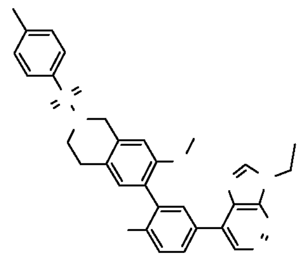 |
| 34 | 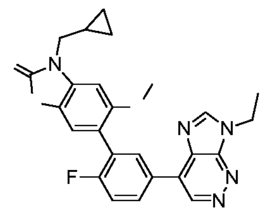 | 90 | 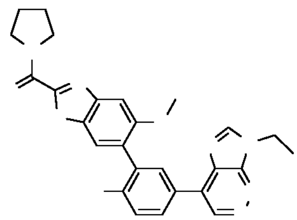 | 146 | 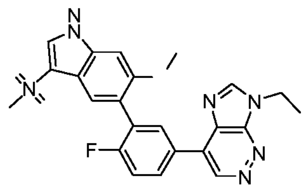 |
| 35 | 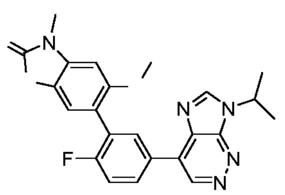 | 91 | 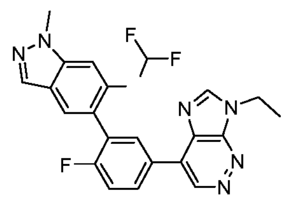 | 147 | 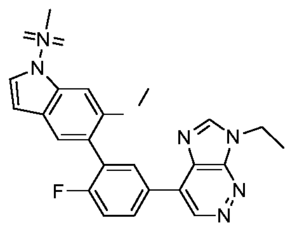 |
| 36 | 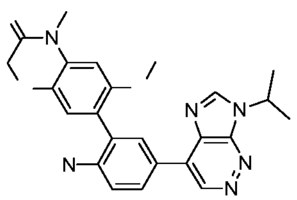 | 92 | 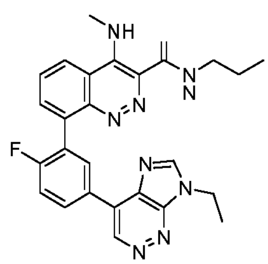 | 148 | 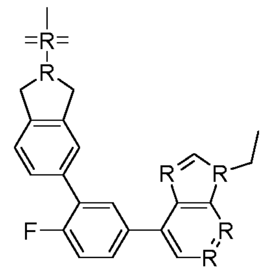 |
| 37 | 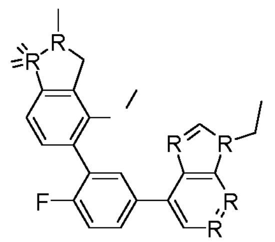 | 93 | 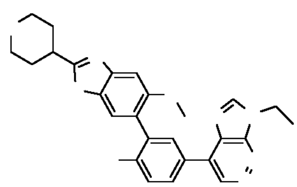 | 149 | 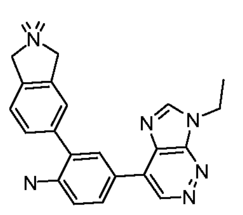 |

(continued)

| No. | Structural formula | No. | Structural formula | No. | Structural formula |
|---|---|---|---|---|---|
| 38 | 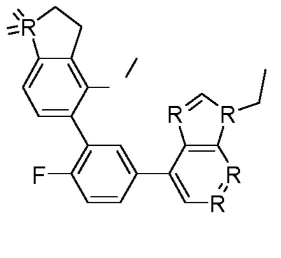 | 94 | 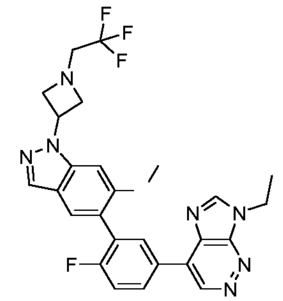 | 150 | 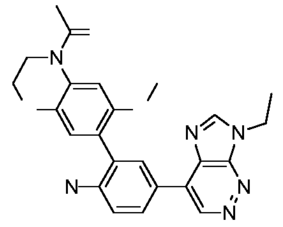 |
| 39 | 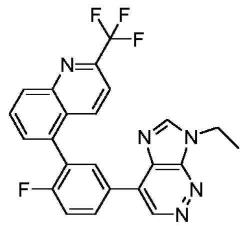 | 95 | 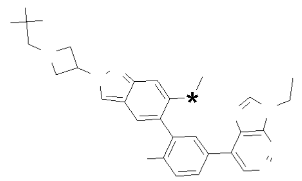 | 151 | 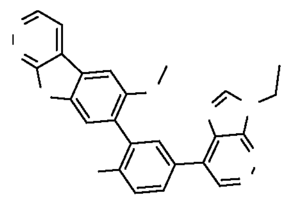 |
| 40 | 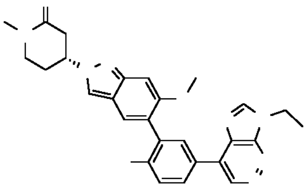 | 96 | 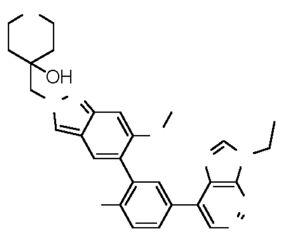 | 152 | 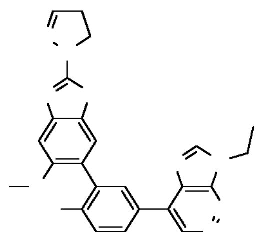 |
| 41 | 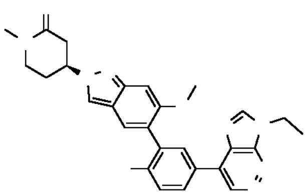 | 97 | 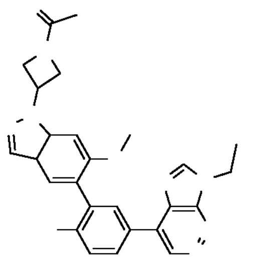 | 153 | 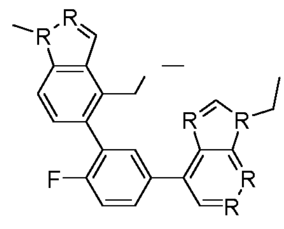 |
| 42 | 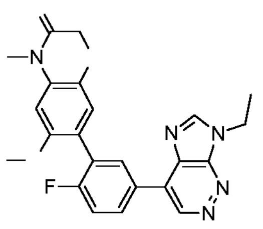 | 98 | 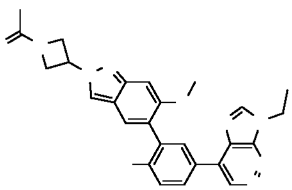 | 154 | 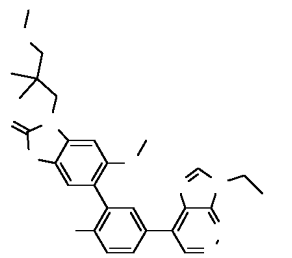 |
| 43 | 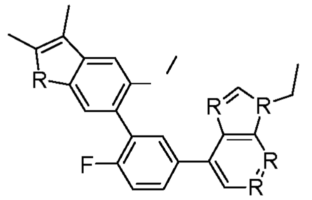 | 99 | 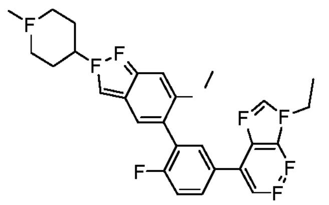 | 155 | 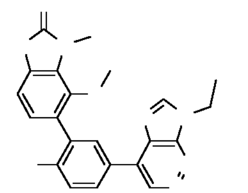 |

(continued)

| No. | Structural formula | No. | Structural formula | No. | Structural formula |
|---|---|---|---|---|---|
| 44 | 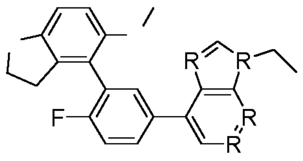 | 100 | 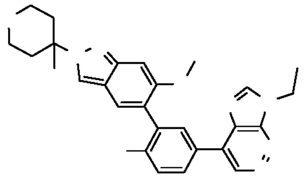 | 156 | 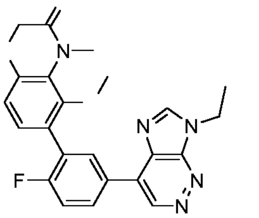 |
| 45 | 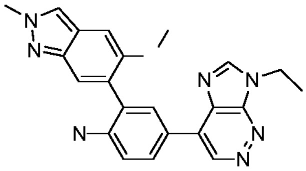 | 101 | 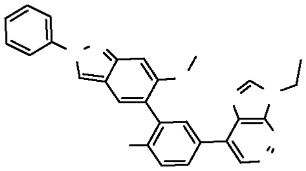 | 157 | 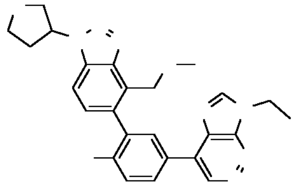 |
| 46 | 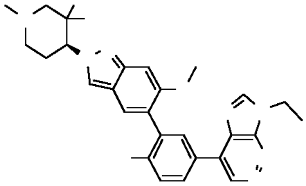 | 102 | 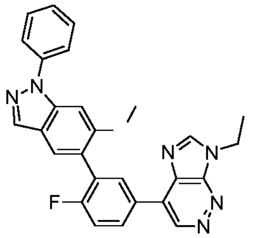 | 158 | 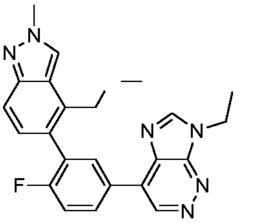 |
| 47 | 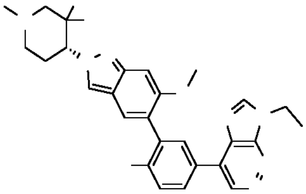 | 103 | 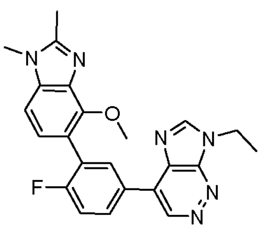 | 159 | 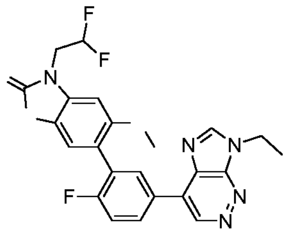 |
| 48 | 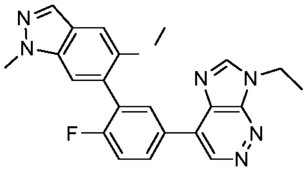 | 104 | 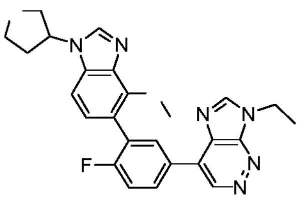 | 160 | 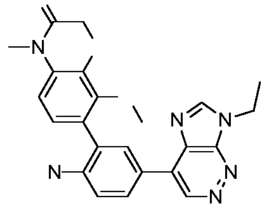 |
| 49 | 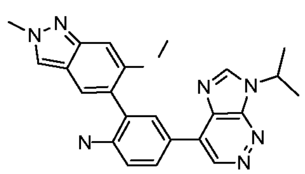 | 105 | 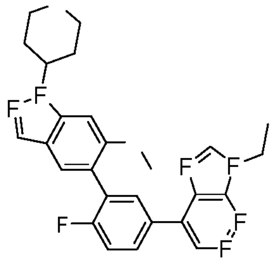 | 161 | 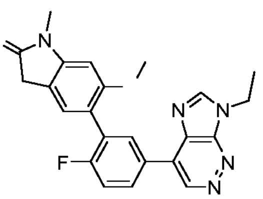 |
| 50 | 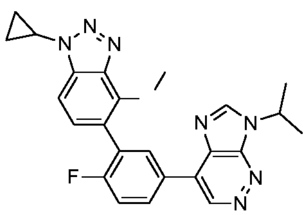 | 106 | 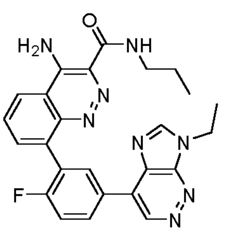 | 162 | 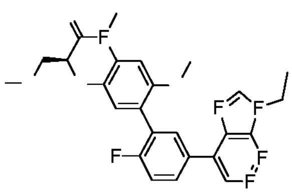 |

(continued)

| No. | Structural formula | No. | Structural formula | No. | Structural formula |
|---|---|---|---|---|---|
| 51 | 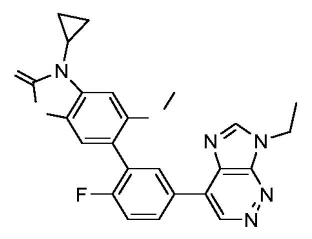 | 107 | 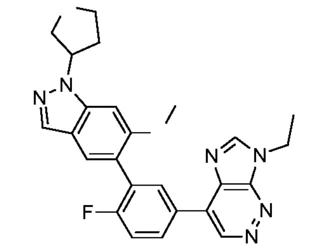 | 163 | 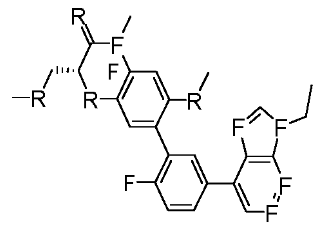 |
| 52 | 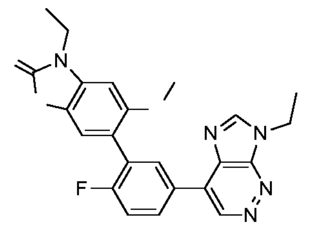 | 108 | 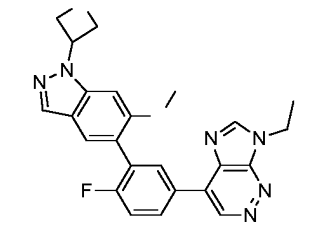 | 164 | 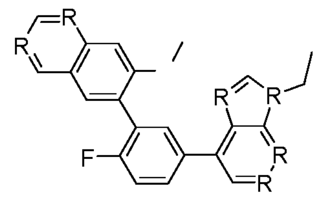 |
| 53 | 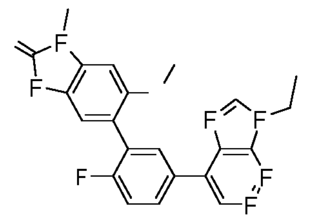 | 109 | 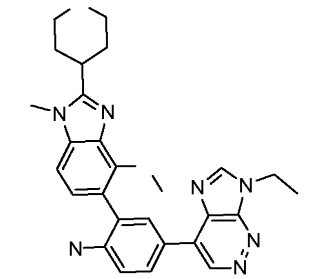 | 165 | 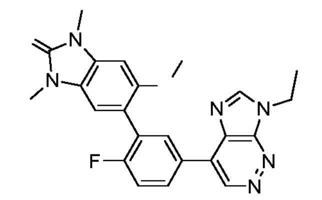 |
| 54 | 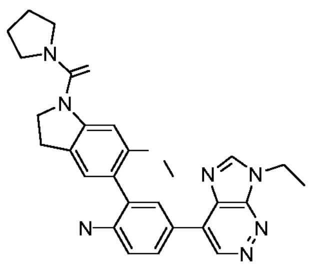 | 110 | 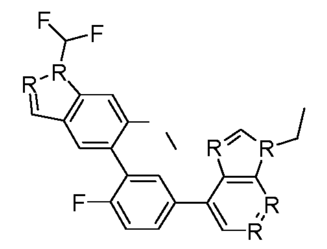 | 166 | 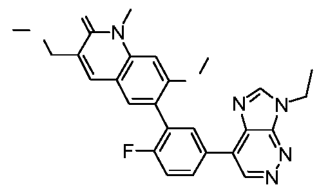 |
| 55 | 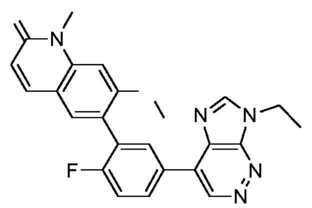 | 111 | 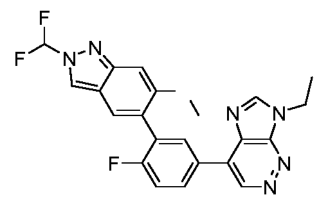 | 167 | 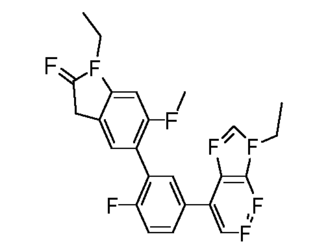 |
| 56 | 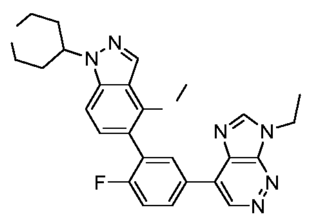 | 112 | 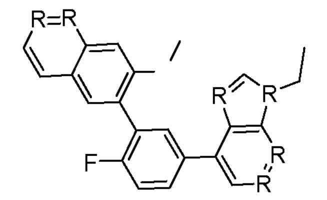 | 168 | 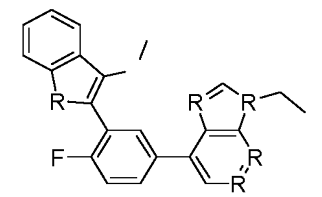 |

The present disclosure further provides a pharmaceutical mixture comprising two or more compounds selected from the group consisting of: the compound or the stereoisomer, the tautomer, the prodrug, the pharmaceutically acceptable salt, the amorphous substance, the isotopic variant, the polymorph, or the solvate thereof according to

any one aspect of the present disclosure.

The present disclosure further provides a pharmaceutical composition comprising at least one of the compounds or the stereoisomers, the tautomers, the prodrugs, the pharmaceutically acceptable salts, the amorphous substances, the isotopic variants, the polymorphs, or the solvates thereof according to any one aspect of the present disclosure, and optionally with the addition of a pharmaceutically acceptable carrier and/or adjuvant.

Provided is a use of the compound or the stereoisomer, the tautomer, the prodrug, the pharmaceutically acceptable salt, the amorphous substance, the polymorph, or the solvate thereof, the pharmaceutical mixture, or the pharmaceutical composition according to any one aspect of the present disclosure in the manufacture of a medicament for treating or preventing a $GABA_A$ receptor-associated disease.

Further, the $GABA_A$ receptor-associated disease is selected from at least one of pain, Alzheimer's disease, multi-infarct dementia, or stroke.

Further, the pain is neuropathic pain, inflammatory pain, or cancer pain.

Further, the pain is selected from: headache, facial pain, neck pain, shoulder pain, back pain, chest pain, abdominal pain, dorsalgia, lower back pain, lower limb pain, musculoskeletal pain, vascular pain, gout, arthritic pain, visceral pain, pain due to infectious disease, polyostotic pain, pain associated with sickle cell anemia, autoimmune disease, multiple sclerosis, or inflammation, chronic pain due to injury or surgery, nociceptive pain, diabetic pain, trigeminal neuralgia, pain of lumbar or cervical radiculopathy, glossopharyngeal neuralgia, autonomic nerve reflex pain, or pain associated with reflex sympathetic dystrophy, nerve root avulsion, cancer, chemical injury, toxin, nutritional deficiency, viral or bacterial infection, or degenerative osteoarthropathy.

The present disclosure further provides a method for treating or preventing a $GABA_A$ receptor-associated disease, comprising administering to a patient an effective dose of the compound or the stereoisomer, the tautomer, the prodrug, the pharmaceutically acceptable salt, the amorphous substance, the polymorph, or the solvate thereof, the pharmaceutical mixture, or the pharmaceutical composition according to any one aspect of the present disclosure.

The present disclosure further provides a method for treating or preventing pain, Alzheimer's disease, multi-infarct dementia, or stroke, comprising administering to a patient an effective dose of the compound or the stereoisomer, the tautomer, the prodrug, the pharmaceutically acceptable salt, the amorphous substance, the polymorph, or the solvate thereof, the pharmaceutical mixture, or the pharmaceutical composition according to any one aspect of the present disclosure.

If the preparation method is not described in the examples, the compound of formula (1) and the intermediate product thereof can be prepared according to a similar method or the method as described above. The raw materials known in the art can be commercially available, or can be prepared according to methods known in the art or a similar method based on the known methods.

It is understandable that the compound of formula (1) of the present disclosure can be derivatized on the functional group to obtain a derivative capable of being reconverted *in vivo* to the parent compound.

Accordingly, the present disclosure further relates to a pharmaceutically acceptable composition comprising the compound or the pharmaceutically acceptable salt thereof or the prodrug thereof and the pharmaceutically acceptable carrier and/or adjuvant as defined above.

Similarly, the present disclosure further comprises a use of the compound or the composition as described above in the manufacture of a medicament for treating or preventing an $\alpha$2/3-$GABA_A$ receptor-associated disease, especially for treating or preventing the following diseases: pain, epilepsy, anxiety, pruritus, and depression.

Preferably, pain is treated or prevented.

Particularly preferably, neuropathic pain, inflammatory pain, and cancer pain are treated or prevented.

As used herein, "cancer pain" refers to the pain that occurs during the development of a malignant tumor. Currently, it is thought that there are three mechanisms of cancer pain, namely, pain caused directly by the development of cancer, pain following cancer treatment, and painful diseases associated with cancer patients.

As used herein, "neuropathic pain" refers to the pain triggered or caused by the primary damage and dysfunction of the nervous system.

As used herein, "inflammatory pain" refers to the pain caused by local acute inflammation or chronic inflammation that stimulates nerves.

As used herein, "treatment" also includes prophylactic administration to alleviate or eliminate a condition once the condition is established.

As used herein, a "patient" is defined as any warm-blooded animal, for example, including, but not limited to, a mouse, guinea pig, dog, horse, or human; preferably, the patient is a human.

As used herein, "acute pain" is defined as the pain caused by the injury of skin, body structure or internal organs and/or noxious stimulation of the disease, or the pain caused by the abnormal function of muscle or internal organs without actual tissue damage.

As used herein, "chronic pain" is defined as the pain that persists beyond the usual course of an acute disease or a reasonable period of time for injury healing, or that is associated with a chronic pathological process that causes persistent

pain, or that recurs at regular intervals of months or years. The pain that persists after the disease should have been cured or beyond the usual course of treatment can be regarded as chronic pain. The length of time that the pain lasts depends on the nature of pain and the course of treatment associated with pain. The pain that lasts longer than the usual course of treatment is chronic pain. The chronic pain includes, but is not limited to, headache, facial pain, neck pain, shoulder pain, chest pain, abdominal pain, dorsalgia, lower back pain, lower limb pain, musculoskeletal pain, pain associated with somatoform disorders, visceral pain, painful diabetic neuropathy, vascular pain, gout, arthritic pain, cancer pain, autonomic nerve reflex pain, pain due to infectious diseases (e.g., AIDS and shingles), pain due to autoimmune diseases (rheumatism), pain due to acute and chronic inflammation, postoperative pain, and post-burn pain.

The medicaments disclosed in the present disclosure can efficiently treat the chronic pain as defined above, and the medicaments disclosed in the present disclosure can be used to treat hyperalgia accompanied with other diseases, including hyperalgesia, allodynia, enhanced algesia, and enhanced pain memory, for which the present disclosure will improve the treatment of pain.

As used herein, "headache" can be divided into primary headache, including tension headache, migraine headache, and cluster headache, and secondary headache, which is caused by other diseases. Headache can be caused when pain-sensitive tissues of the head and face are diseased or stimulated. These pain-sensitive tissues are distributed in the scalp, face, mouth, and throat. Since they are mainly muscles or blood vessels in head with abundant nerve fibers and sensitive to pain, headache can be caused when these tissues are injured.

As used herein, "facial pain" includes, but is not limited to, trigeminal neuralgia, atypical facial pain, facial palsy, and facial spasm.

As used herein, "trigeminal neuralgia" is a unique chronic painful disease, also known as tic douloureux, which refers to transient, paroxysmal, and recurring electric shock-like severe pain in the distribution area of the trigeminal nerve, or accompanied with ipsilateral facial spasm. Trigeminal neuralgia is divided into primary trigeminal neuralgia, which means that no neurological sign is found clinically and no organic disease is detected; and secondary trigeminal neuralgia, which means that neurological signs are found clinically and organic diseases such as tumor and inflammation are detected.

As used herein, "atypical facial pain" refers to the pain caused by various etiologies, appearing as persistent burning pain that is non-intermittent and independent of particular action or stimulation. The pain is mostly bilateral and often extends beyond the distribution range of the trigeminal nerve to even cervical skin. The etiology can be the stimulation of nasosinusitis, malignant tumor, jaw and skull base infection, or pain caused by injured trigeminal nerve.

As used herein, "neck pain, back pain, and shoulder pain" refers to the pain caused by acute or chronic muscle strain and bone joint degeneration and injury. The common diseases that cause neck, shoulder, and upper limb pain include cervicoshoulder myofascitis, nuchal ligamentitis, cervical spondylosis, scapulohumeral periarthritis, thoracic outlet syndrome, lateral epicondylitis, *etc.* Alternatively, the pain caused by autoimmune diseases is common in rheumatoid arthritis, ankylosing spondylitis, rheumatic arthritis, *etc.* Other diseases that may cause neck pain, back pain, and shoulder pain include neck and shoulder tumors, neuritis, arteriovenous disease, and various infections as well as referred pain caused by chest and abdominal organ lesions.

As used herein, "chest pain, abdominal pain, and dorsalgia" refers to the pain due to diseases of the thoracic and abdominal viscera and the thoracic and abdominal wall tissues, including, but not limited to, intercostal neuralgia, intercostal chondritis, angina pectoris, abdominal pain (acute abdominal visceral pain), and myofascial pain syndrome of the lower back.

As used herein, "lower back and lower limb pain" refers to lower back, lumbosacral, sacroiliac, hip, buttock, and lower limb pain. The lower back and lower limb pain is not an independent disease but a common feature of multiple diseases, with various clinical manifestations and very complex etiologies, of which degeneration and injury are the most common, including, but not limited to, pain related to lumbar disc herniation, acute lumbar sprain, sciatica, osteoporosis, third lumbar transverse process syndrome, piriformis syndrome, knee osteoarthritis, coccygodynia, and heel pain.

As used herein, "musculoskeletal pain" includes, but is not limited to, myofascial pain, trauma-induced pain, and chronic regional pain syndrome.

As used herein, "painful diabetes" refers to the pain caused by nerve injury complicated by diabetes, and the nerve injury in diabetes is at least partially caused by reduced blood flow and hyperglycemia. Some diabetics do not develop neuropathy, while other patients develop the disease early. Diabetic neuropathy can be divided into mononeuropathy and generalized polyneuropathy involving one or more focal sites, of which the polyneuropathy may be diffuse and symmetrical, usually involving mainly sensory modalities (Merrit's Textbook of Neurology, 9th edition, edited by LP Rowland LP). Manifestations of diabetic neuropathy may include autonomic dysfunction, leading to dysregulation of the heart, smooth muscle, and glands, resulting in hypotension, diarrhea, constipation, and impotence. Diabetic neuropathy tends to develop in stages. In the early stage, it occurs in the nerve ending region, specifically, in the feet in the case of autonomic neuropathy or sensory neuropathy and in the face and around the eyes in the case of cerebral neuropathy, with intermittent pain and tingling sensations. In the subsequent stages, the pain becomes stronger and more frequent. Finally, when the pain is lost in a certain region, painless neuropathy occurs, which greatly increases the risk of severe tissue injury

in the absence of pain as an indication of injury.

As used herein, "visceral pain" includes, but is not limited to, pain due to irritable bowel syndrome (IBS), with or without chronic fatigue syndrome (CFS), inflammatory bowel disease (IBD), and interstitial cystitis.

As used herein, "vascular pain" refers to the pain resulting from one or more of the following factors. Firstly, improper perfusion of tissue, resulting in temporary or continuous ischemia, such as the ischemia in limb muscles during exercise; secondly, delayed change, such as ulcer or gangrene in skin or abdominal viscera; thirdly, sudden or accelerated change in macrovascular caliber, such as the change in aneurysm, fourthly, aortic rupture, resulting in overflow of blood and stimulation of nociceptive fibers in peritoneal or pleura parietal layers; fifthly, strong spasm caused by severe stimulation of artery endothelium by intra-arterial injection; sixthly, impairment of venous return, leading to massive edema of rapidly expanded fascial compartment (Bonica et al., The Management of Pain, Volume 1 (2nd edition), Philadelphia; Leas & Feboger, 1990). Examples include, but are not limited to, arteriosclerosis obliterans, thromboangiitis obliterans, acute arterial occlusion, embolism, congenital arteriovenous aneurysm, vasospastic disease, Raynaud's disease, acrocyanosis, acute venous occlusion, thrombophlebitis, varicose veins, and lymphedema.

As used herein, "autonomic nerve reflex pain" refers to the pain caused by "reflex sympathetic dystrophy syndrome". The reflex sympathetic dystrophy syndrome refers to a condition in which the body suffers an acute or chronic injury with severe spontaneous pain and hypersensitivity to touch and pain, which may be accompanied by edema and blood circulation disorders, and which may be followed by symptoms such as dystrophy and atrophy of the skin and musculoskeletal system.

As used herein, "postoperative pain" refers to a complex physiological response of the body to the disease itself and the tissue injury caused by surgery, which is manifested as an unpleasant psychological and behavioral experience.

As used herein, "arthritic pain" includes, but is not limited to, pain resulting from osteoarthritis, rheumatoid arthritis, ankylosing spondylitis, psoriatic arthritis, gout, pseudogout, infectious arthritis, tendinitis, bursitis, bone damage, joint soft tissue inflammation, *etc.*

As used herein, "postherpetic neuralgia" refers to severe pain that persists in the subcutaneous area of the original rash area after the shingles rash has healed.

As used herein, "nociceptive pain" refers to the pain caused by stimulation of afferent tissue damaging process by nociceptors, or the pain caused by prolonged excitation of nociceptors. The pain caused by prolonged excitation of nociceptors may be due to persistent noxious stimulation of nociceptors or sensitization of nociceptors or both, or the pain may be caused by these factors and prolonged by the persistence, various reflex mechanisms, and other factors.

The present disclosure provides a use of a pharmaceutical compound comprising a therapeutically effective amount of an $\alpha$2/3-$GABA_A$ positive allosteric modulator. Although the $\alpha$2/3-$GABA_A$ positive allosteric modulator for use in the treatment of the present disclosure may be administered in the form of a starting compound, it is preferred to mix the active ingredient, optionally in the form of a physiologically acceptable salt, together with one or more additives, excipients, carriers, buffers, diluents, and/or other conventional pharmaceutical auxiliary materials into a pharmaceutical composition.

In a preferred embodiment, the present disclosure provides a pharmaceutical composition comprising an $\alpha$2/3-$GABA_A$ positive allosteric modulator, wherein the $\alpha$2/3-$GABA_A$ positive allosteric modulator is mixed with one or more pharmaceutically acceptable carriers, and optionally with other therapeutic and/or prophylactic components known or used in the art. The carrier must be "acceptable" in the sense of being compatible with the other ingredients of the preparation and not harmful to the recipient thereof.

The compound for use in the present disclosure, together with conventional additives or diluents, may thus be formulated into pharmaceutical compositions and unit dose forms thereof. Such forms include solids (especially in the form of tablets, filled capsules, powders, and pills), liquids (especially aqueous or non-aqueous solutions, suspensions, emulsions, and elixirs), capsules filled with the above forms, all forms for oral administration, suppositories for rectal administration, and sterile injectable solutions for parenteral administration. Such pharmaceutical compositions and unit dose forms thereof may comprise conventional ingredients in conventional proportions, with or without additional active compounds or ingredients, and such unit dose forms may contain any suitable effective amount of the active ingredient commensurate with the desired daily dose range to be employed.

The compound for use in the present disclosure can be administered in a variety of oral and parenteral dosage forms. It will be apparent to those skilled in the art that the following dosage forms may comprise the compound or the pharmaceutically acceptable salt thereof of the present disclosure as the active ingredient.

For preparing a pharmaceutical composition from the compound for use in the present disclosure, the pharmaceutically acceptable carrier may be a solid or liquid. Solid preparations include powders, tablets, pills, capsules, cachets, suppositories, and dispersible granules. A solid carrier may be one or more substances which also function as a diluent, flavoring agent, solubilizer, lubricant, suspending agent, binder, preservative, tablet disintegrating agent, or encapsulating material.

In powders, the carrier is a finely divided solid, which is mixed with the finely divided active ingredient.

In tablets, the active ingredient is mixed with the carrier having the necessary binding capacity in appropriate

proportions and compressed into the desired shape and size.

The powders and tablets preferably contain 5% or 10% to about 70% of the active compound. Suitable carriers are magnesium carbonate, magnesium stearate, talc, sugar, lactose, pectin, dextrin, starch, gelatin, tragacanth, methylcellulose, sodium carboxymethylcellulose, low melting waxes, cocoa butter, *etc.* The term "preparation" includes an active compound formulated with an encapsulating material as the carrier providing a capsule in which the active component, with or without carriers, is surrounded by the carrier so as to be associated therewith. Similarly, the preparations include cachets and lozenges. Tablets, powders, capsules, pills, cachets, and lozenges can be used as solid forms suitable for oral administration.

For preparing suppositories, a low melting wax, such as a mixture of fatty acid glyceride or cocoa butter, is first melted and then the active ingredient is homogeneously dispersed therein by stirring. The molten homogeneous mixture is then poured into appropriately sized molds, allowed to cool, and thereby to solidify.

Compositions suitable for vaginal administration may be in the form of pessaries, tampons, creams, gels, pastes, foams, or sprays containing, in addition to the active ingredient, suitable carriers known in the art.

Liquid preparations include solutions, suspensions, and emulsions, for example, aqueous solutions or water-propylene glycol solutions. For example, liquid preparations for parenteral injection can be formulated as water-polyethylene glycol solutions.

The compound for use in the present disclosure may thus be formulated for parenteral administration (e.g., injection, such as bolus injection or continuous infusion) and may be present in unit dose form in ampoules, pre-filled syringes, small volume infusion bags, or multi-dose containers with an added preservative. The compositions may take the form of suspensions, solutions, or emulsions in oily or aqueous carriers, and may contain formulation ingredients such as suspending agents, stabilizers, and/or dispersants. Alternatively, the active ingredient may be in powder form, which may be obtained by aseptic isolation from a sterile solid or by lyophilization from a solution for constitution with a suitable carrier, such as sterile, pyrogen-free water, before use.

Aqueous solutions suitable for oral administration can be prepared by dissolving the active ingredient in water and adding desired colorants, flavoring agents, stabilizers, and thickeners.

Aqueous suspensions suitable for oral administration can be prepared by dispersing the finely divided active ingredient in water containing a viscous substance, such as natural or synthetic gums, resins, methylcellulose, sodium carboxymethylcellulose, or other well-known suspending agents.

Also included are solid preparations designed for conversion shortly before use to liquid preparations for oral administration. Such liquid preparations include solutions, suspensions, and emulsions. In addition to the active ingredient, such preparations may include colorants, flavoring agents, stabilizers, buffers, artificial and natural sweeteners, dispersants, thickeners, solubilizers, *etc.*

For topical application to the epidermis, the compound of the present disclosure may be formulated as an ointment, cream, or lotion, or transdermal patch. For example, ointments and creams may be formulated with an aqueous or oily matrix plus suitable thickeners and/or gelling agents. Lotions may be formulated with an aqueous or oily matrix and in general also contain one or more emulsifiers, stabilizers, dispersants, suspending agents, thickeners, or colorants.

Compositions suitable for oral or topical administration include lozenges containing the active ingredient in a flavored matrix, typically sucrose and arabic gum or tragacanth; pastilles containing the active ingredient in an inert matrix such as gelatin and glycerol or sucrose and arabic gum; and mouthwashes containing the active ingredient in a suitable liquid carrier.

Solutions or suspensions may be applied directly to the nasal cavity by conventional methods, for example with a dropper, pipette, or nebulizer. The composition may be provided in single-dose or multi-dose form.

Respiratory administration can also be achieved by means of an aerosol in which the active ingredient is provided in a pressurized pack with a suitable propellant including chlorofluorocarbon (CFC) such as dichlorodifluoromethane, trichlorofluoromethane, or dichlorotetrafluoroethane, carbon dioxide, or other suitable gas. The aerosol may also contain a surfactant such as lecithin, as appropriate. The dose of drug can be controlled by a metered valve.

Alternatively, the active ingredient may be in the form of a dry powder, for example, a powder mixture of the compound with a suitable powder matrix such as lactose, starch, or starch derivatives such as hydroxypropyl methylcellulose and polyvinylpyrrolidone (PVP). The powder carrier allows for easy gel formation in the nasal cavity. The powder composition may be present in unit dose form, for example, in capsules or cartridges (e.g., gelatin capsules or cartridges), or in blister packs in which the powder can be administered by means of an inhaler.

In compositions for respiratory administration (including intranasal compositions), the compound generally has a small particle size, for example, a particle size of 5 microns or less. Such a particle size can be obtained by methods known in the art, for example by micronization.

If desired, compositions suitable for sustained release of the active ingredient can be applied.

The pharmaceutical preparation is preferably in unit dose form. In such form, the preparation is subdivided into unit doses containing the appropriate amount of the active ingredient. The unit dose form can be an encapsulated preparation where the sealed package contains a large number of separated preparations, such as encapsulated tablets,

capsules, and powders in vials or ampoules. In addition, the unit dose form can be a capsule, tablet, cachet, or lozenge itself, or it can be the appropriate amount of any of the above capsules and tablets in encapsulated form.

Tablets or capsules for oral administration and liquids for intravenous administration and continuous infusion are preferred compositions.

More detailed information on preparation and administration techniques can be found in the latest edition of Remington's Pharmaceutical Sciences (Maack Publishing Co., Easton, PA).

The amount of active ingredient in a unit dose preparation can vary depending on the specific application and the efficacy of the active ingredient, and can be adjusted from 0.01 mg to about 0.1 g. For example, in pharmaceutical use, the drug can be administrated in capsules of 0.01 to about 100 mg three times daily, and the composition may also contain other compatible therapeutic agents if necessary.

In therapeutic use, the compound for use in the present disclosure is administered at a starting dose of 0.001 mg/kg to 10 mg/kg body weight per day. However, the doses may vary depending on the needs of the patient, the severity of the condition being treated, and the compound being used. Generally, treatment is initiated at a smaller dose than the optimal dose of the compound, after which the dose is increased in small increments to achieve the optimal effect. For convenience, the total daily dose can be further subdivided for administration at several times over the course of the day if desired.

The pharmaceutical composition of the present disclosure can also be used simultaneously in combination with other drugs for the treatment of pain, epilepsy, anxiety, and depression, including, but not limited to, morphine, gabapentin, *etc.* Accordingly, the present disclosure provides a drug for the treatment of pain, epilepsy, anxiety, and depression, which is not only effective but also has no significant side effects. Another purpose of the present disclosure is to provide a drug having high safety for a specific patient group, such as the elderly, patients with liver or kidney failure or cardiovascular diseases.

The present disclosure further provides a method for treating or preventing a disease, comprising administering to a patient an effective dose of the compound or the composition as described above.

The present disclosure further provides a method for treating or preventing a $GABA_A$ receptor-associated disease, comprising administering to a patient an effective dose of the compound or the composition as described above.

The present disclosure further provides a use of the compound or the composition as described above in the manufacture of a medicament for treating or preventing the following diseases: pain, Alzheimer's disease, multi-infarct dementia, and stroke.

The pain is neuropathic pain, inflammatory pain, or cancer pain. Preferably, the pain is selected from: headache, facial pain, neck pain, shoulder pain, back pain, chest pain, abdominal pain, dorsalgia, lower back pain, lower limb pain, musculoskeletal pain, vascular pain, gout, arthritic pain, visceral pain, pain due to infectious disease, polyostotic pain, pain associated with sickle cell anemia, autoimmune disease, multiple sclerosis, or inflammation, chronic pain due to injury or surgery, nociceptive pain, painful diabetes, trigeminal neuralgia, pain of lumbar or cervical radiculopathy, glossopharyngeal neuralgia, autonomic nerve reflex pain, or pain associated with reflex sympathetic dystrophy, nerve root avulsion, cancer, chemical injury, toxin, nutritional deficiency, viral or bacterial infection, or degenerative osteoarthropathy.

The present disclosure further provides a method for treating or preventing pain, Alzheimer's disease, multi-infarct dementia, or stroke, comprising administering to a patient an effective dose of the compound or the composition as described above.

The present disclosure further relates to a preparation method for the imidazopyridazine-substituted benzene ring compound of formula (1) as described above, comprising:

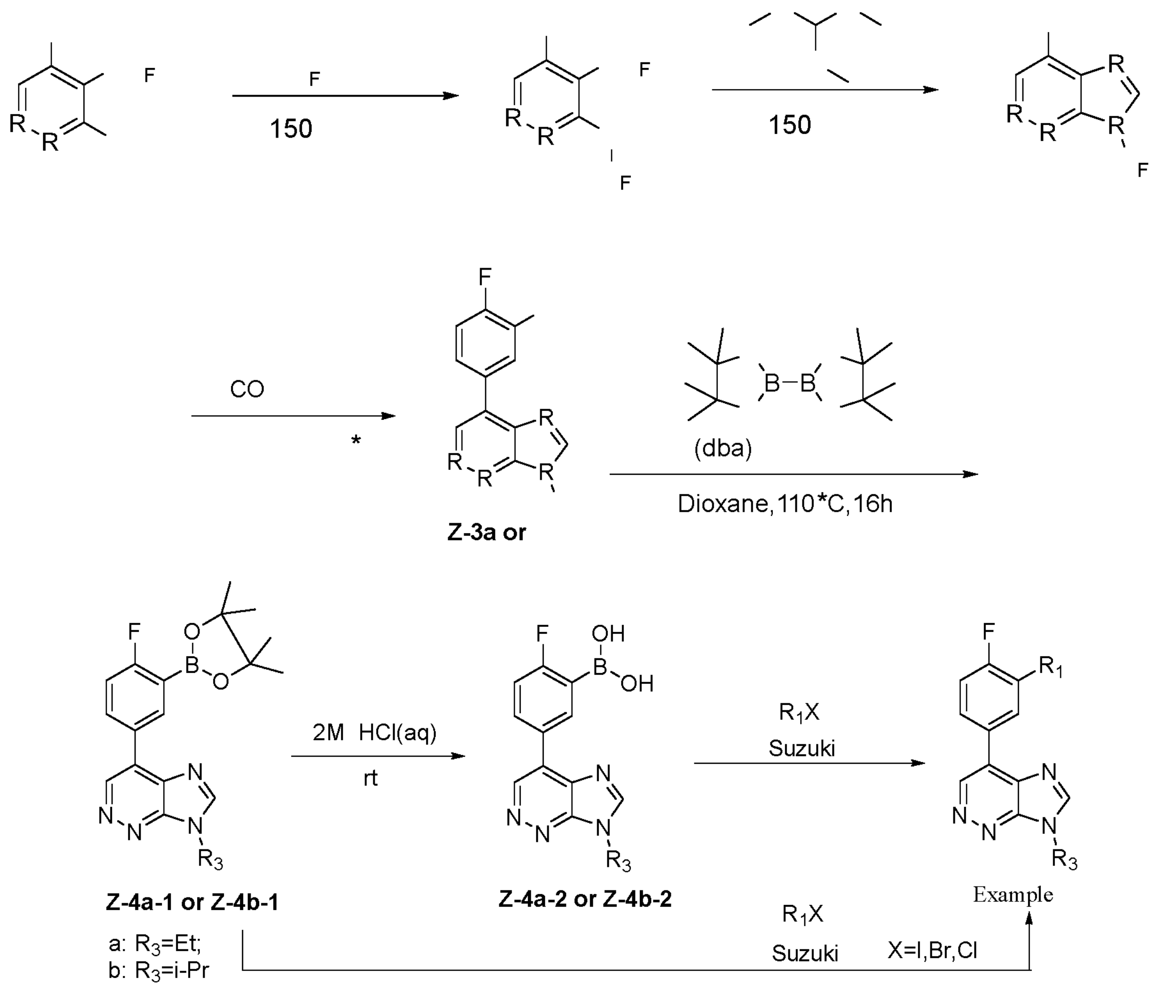


Z-3a or

Z-4a-1 or Z-4b-1

a: $R_3$=Et;
b: $R_3$=i-Pr

Z-4a-2 or Z-4b-2

Note: $R_3$ is an ethyl group or an isopropyl group for which the experimental procedure is the same.

**Z-1a: 5-Chloro-*N*3-ethylpyridazine-3,4-diamine**

3,5-Dichloropyridazin-4-amine (80.5 g, 0.5 mol) and a solution of ethylamine in ethanol (30%, 600 mL) are placed in an autoclave, and the mixture is reacted at 150°C for 16 hours. The reaction mixture is cooled to room temperature, and a solid is precipitated. The mixture is filtered, and the filter cake is washed with dichloromethane (300 mL). The mother liquor is concentrated (without evaporation to dryness), and a solid is precipitated. The mixture is filtered again, and the procedure is repeated three times. All the filter cakes are collected to obtain a crude product (containing ethylamine hydrochloride). The crude product is stirred and slurried with water, filtered, and the filter cake is rotary evaporated to dryness to obtain 65 g of the target product (yield: 76.5%) as a light yellow solid. LC-MS: m/z $[M+H]^+$ = 173.

**Z-2a: 4-Chloro-7-ethyl-7*H*-imidazo[4,5-c]pyridazine**

5-Chloro-*N*3-ethylpyridazine-3,4-diamine (30 g, 0.17 mol) is added to trimethyl orthoformate (600 mL). The mixture is reacted at 120°C for 4 hours. The reaction mixture is directly rotary evaporated to dryness to obtain a crude product, which is dissolved in dichloromethane and methanol and mixed with silica gel, then subjected to column chromatography (dichloromethane, dichloromethane/methanol = 50/1, V/V) to obtain 20 g of the target product (yield: 63%) as a yellow solid. LC-MS: m/z $[M+H]^+$ = 183.

**Z-3a: 4-(3-Chloro-4-fluorophenyl)-7-ethyl-7*H*-imidazo[4,5-c]pyridazine**

4-Chloro-7-ethyl-7*H*-imidazo[4,5-c]pyridazine (20 g,108 mmol), (3-chloro-4-fluorophenyl)boronic acid (19.08 g, 108 mmol), sodium carbonate (24 g, 216 mmol), and [1,1'-bis(diphenylphosphino)ferrocene]dichloropalladium(II) (9.2 g, 10.8 mmol) are added to 1,4-dioxane/water (160 mL/40 mL). The mixture is reacted at 90°C for 2 hours. The reaction mixture is directly purified by column chromatography (petroleum ether/ethyl acetate = 10/1, 5/1, 1/1, ethyl acetate, V/V) to

obtain the title compound (26 g, yield: 85.7%) as a yellow solid. LC-MS: m/z $[M+H]^+$ = 277.

**Z-4a-1: 7-Ethyl-4-(4-fluoro-3-(4,4,5,5-tetramethyl-1,3,2-dioxaborolan-2-yl)phenyl)-7*H*-imidazo[4,5-c]pyridazine**

4-(3-Chloro-4-fluorophenyl)-7-ethyl-7*H*-imidazo[4,5-c]pyridazine (15 g, 54 mmol), bis(pinacolato)diboron (27.6 g, 108 mmol), sodium acetate (13 g, 163 mmol), 2-dicyclohexylphosphino-2,4,6-triisopropylbiphenyl (2.7 g, 5.4 mmol), and tris(dibenzylideneacetone)dipalladium (5 g, 5.4 mmol) are added to anhydrous 1,4-dioxane (150 mL). The mixture is stirred at 110°C overnight. The reaction mixture is directly purified by column chromatography (dichloromethane/anhydrous methanol = 50/1) to obtain the title compound (12 g, yield: 60%) as a light yellow solid. $^1$H NMR (400 MHz, CHLOROFORM-d) ppm 1.40 (s, 12 H) 1.69 (t, *J* = 7.09 Hz, 3 H) 4.58 (q, *J* = 7.34 Hz, 2 H) 7.26 (d, *J* = 6.36 Hz, 1 H) 8.29 (s, 1 H) 8.45 (d, *J* = 1.96 Hz, 2 H) 9.39 (s, 1 H). LC-MS: m/z $[M+H]^+$ = 287, 369.

**Z-4a-2: (5-(7-Ethyl-7*H*-imidazo[4,5-c]pyridazin-4-yl)-2-fluorophenyl)boronic acid**

7-Ethyl-4-(4-fluoro-3-(4,4,5,5-tetramethyl-1,3,2-dioxaborolan-2-yl)phenyl)-7*H*-imidazo[4,5-*c*]pyridazine (12 g, 32.5 mmol) is dissolved in 200 mL of hydrochloric acid aqueous solution (2 M). The resulting mixture is stirred at room temperature for 2 hours, and lyophilized to obtain the title compound (9.3 g, crude product) as a white solid.

Beneficial effects:

The compound and the pharmaceutically acceptable salt thereof or the prodrug thereof of the present disclosure have important pharmacological properties, and are positive allosteric modulators of $\alpha$2/3-$GABA_A$ receptors. The compound of the present disclosure has excellent affinity activity and positive regulatory activity on $\alpha$2/3-$GABA_A$ receptors, and has good genotoxic safety and druggability. Therefore, the compound and the pharmaceutically acceptable salt thereof or the prodrug thereof of the present disclosure can be used alone or in combination with other drugs for treating or preventing an $\alpha$2/3-$GABA_A$-associated disease.

DETAILED DESCRIPTION OF THE PREFERRED EMBODIMENT

The technical solutions of the present disclosure are clearly and completely described below, and it is obvious that the described examples are some, but not all, examples of the present disclosure. Based on the examples of the present disclosure, all other examples obtained by those of ordinary skill in the art without creative efforts shall fall within the scope of protection of the present disclosure. Among them, the ratio of solvents used in the purification step (*e.g.*, preparative thin-layer chromatography and column chromatography) in the following examples is a volume ratio.

Example 1

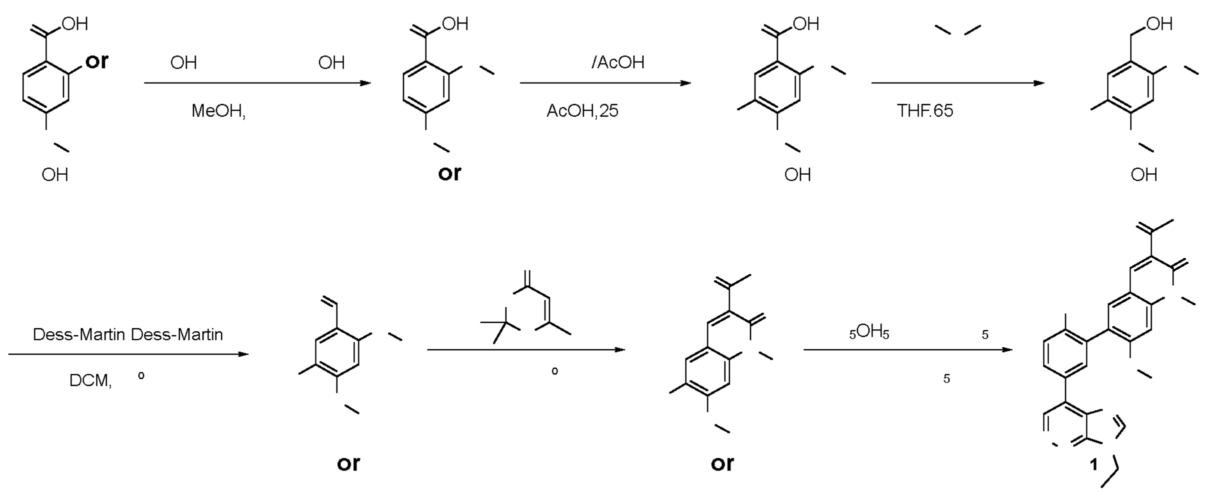


or or or or or 1

**1-1: 4-Methoxy-2-(methylamino)benzoic acid**

2-Amino-4-methoxybenzoic acid (2 g, 12 mmol, 1.0 eq) was dissolved in methanol (20 mL) at 25°C, then 37% formaldehyde solution (1 mL, 13.2 mmol, 1.1 eq) was added thereto, and sodium cyanoborohydride (791 mg, 12.6 mmol, 1.05 eq) was added thereto in batches while stirring. The mixture was stirred for 2 hours. The completion of the reaction was detected by LCMS. The reaction mixture was poured into 100 mL of water and extracted with ethyl acetate (100 mL × 2). The organic phase was washed with water and brine, then dried, and rotary evaporated to dryness to obtain crude 4-methoxy-2-(methylamino)benzoic acid (2.2 g, 100%). LC-MS: m/z $[M+H]^+$ = 182.

**1-2: 5-Bromo-4-methoxy-2-(methylamino)benzoic acid**

4-Methoxy-2-(methylamino)benzoic acid (1.5 g, 8.3 mmol, 1.0 eq) was dissolved in acetic acid (20 mL), then a homemade 1.4 M bromoacetic acid solution (6.2 mL, 8.7 mmol, 1.05 eq) was added dropwise thereto, and the mixture was reacted at 25°C for 3 hours. The reaction mixture was poured into 100 mL of water, extracted with ethyl acetate, dried, and rotary evaporated to dryness to obtain crude 5-bromo-4-methoxy-2-(methylamino)benzoic acid (2.05 g, 100%). LC-MS: m/z $[M+H]^+$ = 261.

**1-3: (5-Bromo-4-methoxy-2-(methylamino)phenyl)methanol**

5-Bromo-4-methoxy-2-(methylamino)benzoic acid (2.05 g, 7.7 mmol, 1.0 eq) was dissolved in tetrahydrofuran (40 mL) at 25°C, and a 2 M solution of borane-dimethyl sulfide in tetrahydrofuran (20 mL) was added thereto. The mixture was stirred at 65°C for 16 hours. The reaction mixture was poured into 150 mL of water, extracted with ethyl acetate (100 mL × 2), and subjected to rotary evaporation to remove about half of the solvent. The remaining organic phase was dried over anhydrous sodium sulfate to yield the organic phase containing the product (200 mL), which turned blue and was very unstable upon being rotary evaporated to dryness. LC-MS: m/z $[M+H]^+$ = 248.

**1-4: 5-Bromo-4-methoxy-2-(methylamino)benzaldehyde**

Crude (5-bromo-4-methoxy-2-(methylamino)phenyl)methanol (50 mL) was added to dichloromethane (50 mL) at 25°C, followed by addition of Dess-Martin periodinane (CAS: 87413-09-0, 3 g). The mixture was stirred at 25°C for 4 hours. The reaction mixture was filtered. The filtrate was added with xylene (50 mL), then subjected to rotary evaporation to remove low boiling solvents such as ethyl acetate, and dried over anhydrous sodium sulfate to obtain a dry xylene solution containing the product (50 mL, 500 mg of crude product, 100%). LC-MS: m/z $[M+H]^+$ = 244.

**1-5: 3-Acetyl-6-bromo-7-methoxy-1-methylquinolin-2(1*H*)-one**

Crude 5-bromo-4-methoxy-2-(methylamino)benzaldehyde (20 mL, content: 200 mg) was added to 2,2,6-trimethyl-4*H*-1,3-dioxin-4-one (1.13 g, 8 mmol, 10.0 eq) at 25°C. The mixture was stirred at 120°C for 2 hours. The reaction mixture was subjected to rotary evaporation to remove a large amount of xylene, and then subjected to column chromatography (dichloromethane: methanol = 10:1) to obtain the crude title product (150 mg, 60%). LC-MS: m/z $[M+H]^+$ = 311.

1: **3-Acetyl-6-(5-(7-ethyl-7*H*-imidazo[4,5-c]pyridazin-4-yl)-2-fluorophenyl)-7-methoxy-1-methylquinolin-2(1*H*)-one**

3-Acetyl-6-bromo-7-methoxy-1-methylquinolin-2(1*H*)-one (150 mg, 0.48 mmol), (5-(7-ethyl-7*H*-imidazo[4,5-*c*]pyridazin-4-yl)-2-fluorophenyl)boronic acid (138 mg, 0.48 mmol), [1,1'-bis(di-*tert*-butylphosphino)ferrocene]dichloropalladium(II) (CAS: 95408-45-0, 32 mg, 0.05 mmol), and cesium carbonate (325 mg, 1.0 mmol) were added to 1,4-dioxane/water (3 mL/0.3 mL). The mixture was reacted at 100°C for 2 hours under an argon atmosphere. The reaction mixture was subjected to preparative HPLC and lyophilized to obtain the title compound (100 mg, 21%).

$^1$H NMR (400 MHz, DMSO-$d_6$) 9.59 (s, 1 H), 8.89 (s, 1 H), 8.52 (dd, *J* = 9.2, 5.9 Hz, 2 H), 8.47 (s, 1 H), 8.00 (s, 1 H), 7.54 (t, *J* = 9.0 Hz, 1 H), 7.12 (s, 1 H), 4.51 (q, *J* = 7.2 Hz, 2 H), 4.00 (s, 3 H), 3.77 (s, 3 H), 2.63 (s, 3 H), 1.56 (t, *J* = 7.3 Hz, 3 H). LC-MS: m/z $[M+H]^+$ = 474.

Example 2

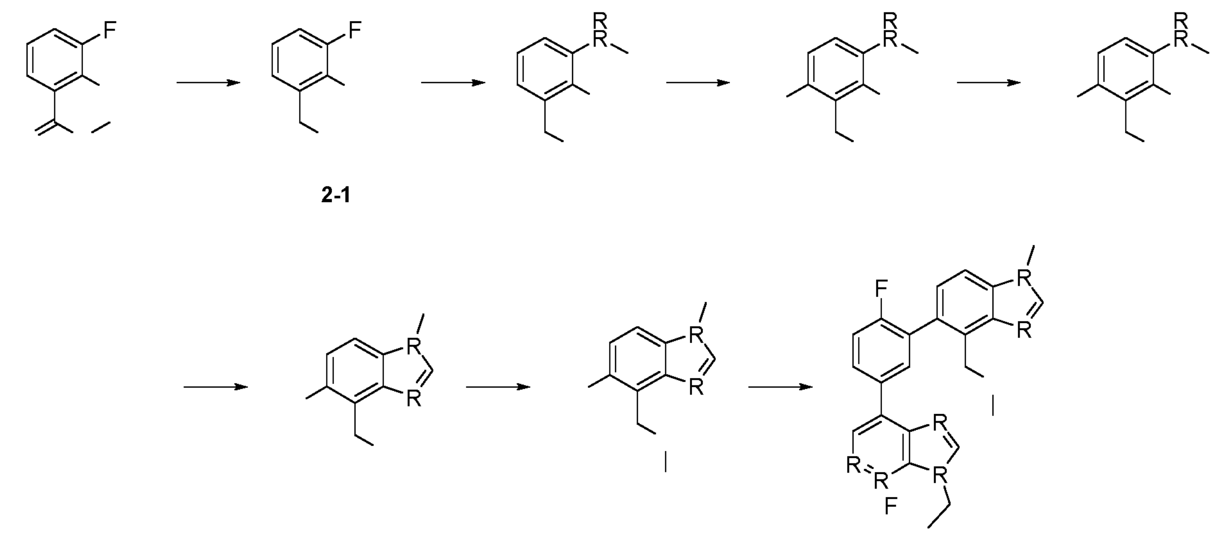

2-1

**2-1: (3-Fluoro-2-nitrophenyl)methanol**

Methyl 3-fluoro-2-nitrobenzoate (1 g, 5.02 mmol) and sodium borohydride (570 mg, 15.06 mmol) were added to a mixed solvent of tetrahydrofuran/methanol (10/10 mL), and the mixture was stirred at room temperature for 1 hour. The reaction mixture was subjected to column chromatography (dichloromethane/methanol = 20/1) to obtain the title compound (800 mg, 93%).

**2-2: (3-(Methylamino)-2-nitrophenyl)methanol**

(3-Fluoro-2-nitrophenyl)methanol (600 mg, 3.51 mmol) and a methylamine solution in alcohol (5 mL) were stirred in a sealed container at 100°C overnight. The reaction mixture was subjected to column chromatography (dichloromethane/methanol = 20/1) to obtain the title compound (500 mg, 78%).

**2-3: (6-Bromo-3-(methylamino)-2-nitrophenyl)methanol**

(3-(Methylamino)-2-nitrophenyl)methanol (500 mg, 2.74 mmol) and *N*-bromosuccinimide (439 mg, 2.47 mmol) were added to acetonitrile (20 mL), and the mixture was stirred at room temperature for 2 hours. The reaction mixture was added with water (150 mL) and extracted with ethyl acetate (200 mL × 2). The ethyl acetate was washed once with water and once with saturated brine, and then concentrated to obtain the title compound (600 mg, 84%) as a red solid. LC-MS: m/z $[M+H]^+$ = 243.

**2-4: (2-Amino-6-bromo-3-(methylamino)phenyl)methanol**

(6-Bromo-3-(methylamino)-2-nitrophenyl)methanol (600 mg, 2.3 mmol) and Raney nickel (68 mg) were added to methanol (20 mL), and the reaction system was stirred at room temperature for 2 hours under a hydrogen atmosphere. The reaction mixture was filtered, then the filter cake was washed twice with methanol, and the filtrate was concentrated to obtain the title compound (500 mg, 94%) as a yellow solid. LC-MS: m/z $[M+H]^+$ = 231.

**2-5: (5-Bromo-1-methyl-1*H*-benzo[d]imidazol-4-yl)methanol**

(2-Amino-6-bromo-3-(methylamino)phenyl)methanol (500 mg, 2.16 mmol) was added to trimethyl orthoformate (10 mL), and the mixture was stirred at 90°C overnight. The reaction mixture was directly subjected to thin-layer chromatography (petroleum ether/ethyl acetate = 1/1) to obtain the title compound (400 mg, 76%) as a yellow oil. LC-MS: m/z $[M+H]^+$ = 241.

**2-6: 5-Bromo-4-(methoxymethyl)-1-methyl-1*H*-benzo[d]imidazole**

(5-Bromo-1-methyl-1*H*-benzo[*d*]imidazol-4-yl)methanol (400 mg, 1.65 mmol) was added to tetrahydrofuran (10 mL), then sodium hydride (119 mg, 4.95 mmol) was added to the reaction system, and the mixture was stirred at room temperature for 10 minutes. Iodomethane (1171 mg, 8.25 mmol) was then added thereto, and the reaction mixture was

stirred at room temperature for 1 hour. The reaction mixture was added with ethyl acetate (200 mL), then washed with water (100 mL × 2), washed once with saturated brine, dried over anhydrous sodium sulfate, and concentrated to obtain the title compound (240 mg, 57%) as a yellow oil. LC-MS: m/z $[M+H]^+$ = 255.

**2: 7-Ethyl-4-(4-fluoro-3-(4-(methoxymethyl)-1-methyl-1*H*-benzo[*d*]imidazol-5-yl)phenyl)-7*H*-imidazo[4,5-c]pyridazine**

Following the same experimental procedure as in Example 1, 5-bromo-4-(methoxymethyl)-1-methyl-1*H*-benzo[*d*]imidazole (100 mg, 0.39 mmol) and (5-(7-ethyl-7*H*-imidazo[4,5-*c*]pyridazin-4-yl)-2-fluorophenyl)boronic acid (112 mg, 0.39 mmol) were used as starting materials to obtain the title compound (14 mg, 9%) as a yellow solid.

$^1$H NMR (400 MHz, CHLOROFORM-d) ppm 1.68 (t, *J* = 7.34 Hz, 3 H) 3.33 (s, 3 H) 3.89 (s, 3 H) 4.57 (q, *J* = 7.34 Hz, 2 H) 4.83 (br. s., 2 H) 7.33 - 7.41 (m, 2 H) 7.43 - 7.49 (m, 1 H) 7.97 (s, 1 H) 8.27 (s, 1 H) 8.31 (d, *J* = 7.34 Hz, 1 H) 8.37 - 8.44 (m, 1 H) 9.40 (s, 1 H). LC-MS: m/z $[M+H]^+$ = 417.

Example 3

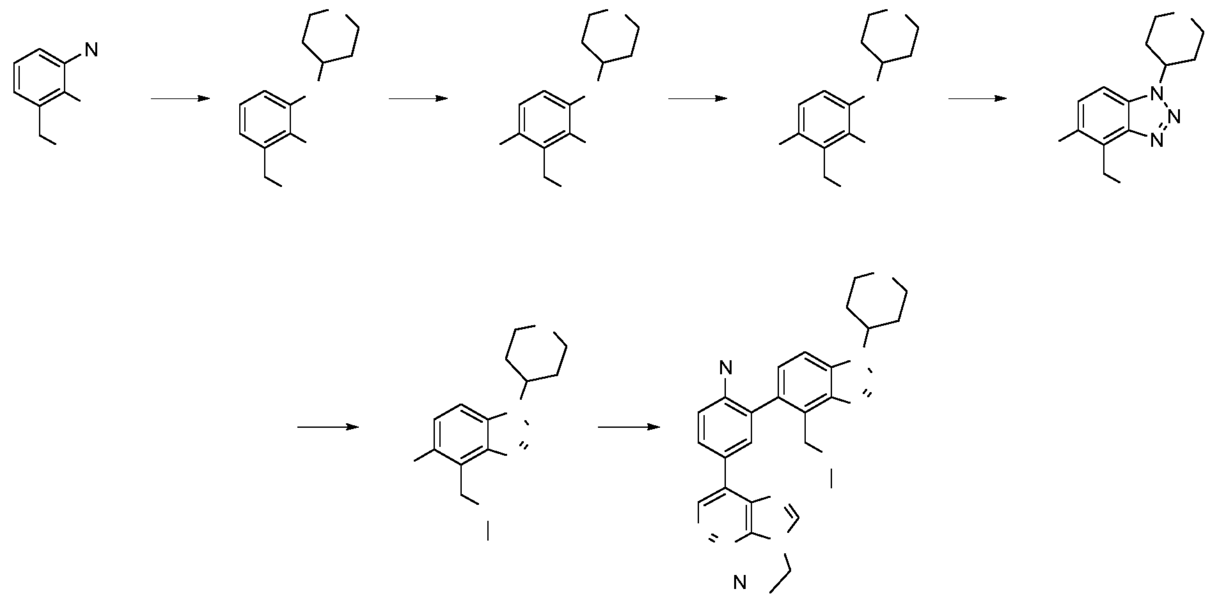

**3-1: (2-Nitro-3-((tetrahydro-2*H*-pyran-4-yl)amino)phenyl)methanol**

(3-Fluoro-2-nitrophenyl)methanol (500 mg, 2.92 mmol) and tetrahydro-2H-pyran-4-amine (570 mg, 5.84 mmol) were added to anhydrous ethanol (10 mL), and the mixture was stirred in a sealed container at 100°C overnight. The reaction mixture was directly purified by preparative thin-layer chromatography (petroleum ether/ethyl acetate = 3/1) to obtain the title compound (680 mg, 92%). LC-MS: m/z $[M+H]^+$ = 253.

**3-2: (6-Bromo-2-nitro-3-((tetrahydro-2*H*-pyran-4-yl)amino)phenyl)methanol**

Following the same experimental procedure as in the synthesis method of 2-3 in Example 2, (2-nitro-3-((tetrahydro-2H-pyran-4-yl)amino)phenyl)methanol (600 mg, 2.4 mmol) was used as the starting material to obtain the title compound (300 mg, 38%). LC-MS: m/z $[M+H]^+$ = 331, 333.

**3-3: (2-Amino-6-bromo-3-((tetrahydro-2*H*-pyran-4-yl)amino)phenyl)methanol**

Following the same experimental procedure as in the synthesis method of 2-4 in Example 2, (6-bromo-2-nitro-3-((tetrahydro-2*H*-pyran-4-yl)amino)phenyl)methanol (300 mg, 0.9 mmol) was used as the starting material to obtain the title compound (220 mg, 82%). LC-MS: m/z $[M+H]^+$ = 301, 303.

**3-4: (5-Bromo-1-(tetrahydro-2*H*-pyran-4-yl)-1*H*-benzo[*d*][1,2,3]triazol-4-yl)methanol**

(2-Amino-6-bromo-3-((tetrahydro-2*H*-pyran-4-yl)amino)phenyl)methanol (200 mg, 0.67 mol) and sodium nitrite (231 mg, 3.35 mmol) were added to hydrochloric acid solution (1 M, 8 mL), and the mixture was stirred at room temperature for 2 hours. The reaction mixture was extracted with ethyl acetate, and the organic phase was purified by preparative thin-layer chromatography (petroleum ether/ethyl acetate = 1/1) to obtain the title compound (150 mg, 72%). LC-MS: m/z $[M+H]^+$ = 312, 314.

**3-5: 5-Bromo-4-(methoxymethyl)-1-(tetrahydro-2*H*-pyran-4-yl)-1*H*-benzo[*d*][1,2,3]triazole**

Following the same experimental procedure as in the synthesis method of 2-6 in Example 2, (5-bromo-1-(tetrahydro-2*H*-pyran-4-yl)-1*H*-benzo[*d*][1,2,3]triazol-4-yl)methanol (150 mg, 0.48 mmol) was used as the starting material to obtain the title compound (140 mg, 90%). LC-MS: m/z $[M+H]^+$ = 326, 328.

**3: 7-Ethyl-4-(4-fluoro-3-(4-(methoxymethyl)-1-(tetrahydro-2H-pyran-4-yl)-1H-benzo[*d*][1,2,3]triazol-5-yl)phenyl)-7*H*-imidazo[4,5-*c*]pyridazine**

Following the same experimental procedure as in Example 1, 5-bromo-4-(methoxymethyl)-1-(tetrahydro-2*H*-pyran-4-yl)-1*H*-benzo[d][1,2,3]triazole (70 mg, 0.22 mmol) and (5-(7-ethyl-7*H*-imidazo[4,5-*c*]pyridazin-4-yl)-2-fluorophenyl)boronic acid (75 mg, 0.26 mmol) were used as starting materials to obtain the title compound (65 mg, 61%).

$^1$H NMR (400 MHz, CHLOROFORM-d) 9.40 (s, 1 H), 8.42 (br. s., 1 H), 8.35 (d, *J* = 6.8 Hz, 1 H), 8.27 (s, 1 H), 7.65 (d, *J* = 8.3 Hz, 1 H), 7.55 (d, *J* = 8.3 Hz, 1 H), 7.40 (t, *J* = 8.8 Hz, 1 H), 5.06 - 4.89 (m, 3 H), 4.58 (q, *J* = 7.0 Hz, 2 H), 4.24 (d, *J* = 9.8 Hz, 2 H), 3.69 (t, *J* = 11.5 Hz, 2 H), 3.40 (s, 3 H), 2.63 - 2.50 (m, 2 H), 2.17 (d, *J* = 13.2 Hz, 2 H), 1.72 - 1.67 (m, 3 H). LC-MS: m/z $[M+H]^+$ = 488.

Example 4

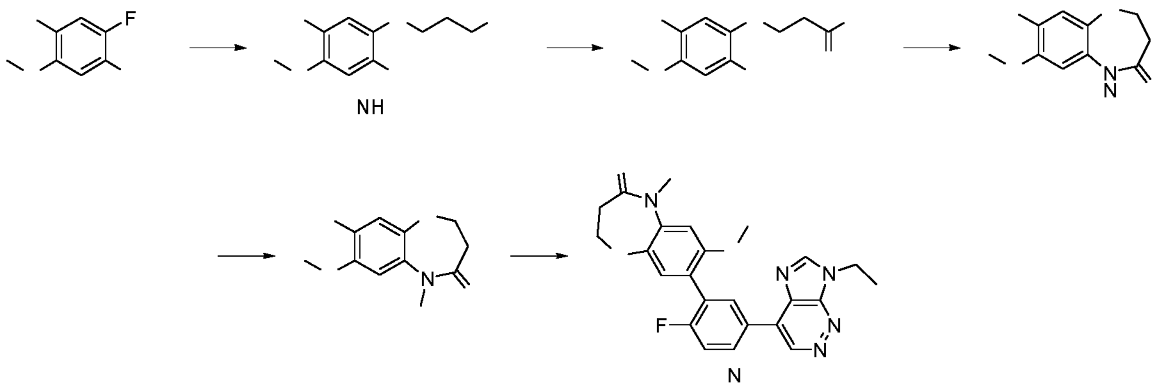

**4-1: 3-(5-Chloro-4-methoxy-2-nitrophenoxy)propan-1-ol**

1-Chloro-5-fluoro-2-methoxy-4-nitrobenzene (1 g, 4.86 mmol), 1,3-propanediol (740 mg, 9.72 mmol), and cesium carbonate (3.17 g, 9.72 mmol) were added to *N,N*-dimethylformamide (5 mL), and the mixture was reacted at 120°C for 3 hours. The reaction mixture was poured into water (30 mL) and extracted with dichloromethane (30 mL × 3). The organic phases were combined, washed with saturated sodium chloride aqueous solution (30 mL), concentrated, and mixed with silica gel, and subjected to column chromatography (petroleum ether/ethyl acetate = 4/1) to obtain the title compound (648 mg, 51%) as a white solid.

$^1$H NMR (400 MHz, CHLOROFORM-d) d = 7.54 (s, 1 H), 7.20 (s, 1 H), 4.24 (t, *J* = 5.9 Hz, 2 H), 3.94 - 3.89 (m, 5 H), 2.11 (quin, *J* = 5.5 Hz, 2 H).

**4-2: 3-(5-Chloro-4-methoxy-2-nitrophenoxy)propanoic acid**

Jones reagent (2.5 M, 2 mL) was added to acetone (4 mL). 3-(5-Chloro-4-methoxy-2-nitrophenoxy)propan-1-ol (200 mg, 0.76 mmol) was dissolved in acetone (4 mL) and slowly added dropwise to the reaction mixture, which was stirred

at room temperature for 30 minutes. The reaction mixture was poured into water (30 mL) and extracted with dichloromethane (30 mL × 3). The organic phases were combined, dried over anhydrous sodium sulfate, and concentrated to obtain the title compound (178 mg, 84.97%) as a light yellow solid.

$^1$H NMR (400 MHz, CHLOROFORM-d) d = 7.49 (s, 1 H), 7.22 (s, 1 H), 4.36 (t, *J* = 6.1 Hz, 2 H), 3.93 (s, 3 H), 2.93 (t, *J* = 6.1 Hz, 2 H).

**4-3: 8-Chloro-7-methoxy-2,3-dihydrobenzo[*b*][1,4]oxazepin-4(5*H*)-one**

3-(5-Chloro-4-methoxy-2-nitrophenoxy)propanoic acid (170 mg, 0.62 mmol) and iron powder (69 mg, 1.24 mmol) were added to acetic acid (3 mL), and the mixture was reacted at 110°C overnight. The reaction mixture was poured into saturated sodium bicarbonate aqueous solution (30 mL) and subjected to suction filtration to obtain the title compound (120 mg, 85%) as a white solid. LC-MS: m/z $[M+H]^+$ = 228, 230.

**4-4: 8-Chloro-7-methoxy-5-methyl-2,3-dihydrobenzo[*b*][1,4]oxazepin-4(5*H*)-one**

8-Chloro-7-methoxy-2,3-dihydrobenzo[*b*][1,4]oxazepin-4(5*H*)-one (60 mg, 0.26 mmol) was added to tetrahydrofuran (2 mL), then sodium hydride (12 mg, 0.40 mmol) was added thereto, and the reaction mixture was stirred at room temperature for 5 minutes. Iodomethane (55 mg, 0.39 mmol) was then slowly added thereto, and the reaction mixture was stirred at room temperature for 30 minutes. The reaction mixture was slowly poured into water (30 mL) and extracted with dichloromethane (30 mL × 3). The organic phases were combined, dried over anhydrous sodium sulfate, concentrated, and subjected to preparative thin-layer chromatography (dichloromethane/methanol = 30/1) to obtain the title compound (40 mg, 64%) as a gray solid. LC-MS: m/z $[M+H]^+$ = 242, 244.

**4: 8-(5-(7-Ethyl-7*H*-imidazo[4,5-*c*]pyridazin-4-yl)-2-fluorophenyl)-7-methoxy-5-methyl-2,3-dihydrobenzo[*b*][1,4]oxazepin-4(5*H*)-one**

8-Chloro-7-methoxy-5-methyl-2,3-dihydrobenzo[*b*][1,4]oxazepin-4(5*H*)-one (30 mg, 0.12 mmol), (5-(7-ethyl-7*H*-imidazo[4,5-*c*]pyridazin-4-yl)-2-fluorophenyl)boronic acid (34 mg, 0.12 mmol), cesium carbonate (78 mg, 0.24 mmol), and methanesulfonato(2-dicyclohexylphosphino-2',4',6'-tri-i-propyl-1,1'-biphenyl)(2'-amino-1,1'-biphenyl-2-yl)palladium(II) (Xphos-Pd-G3, CAS: 1445085-55-1, 10 mg, 0.01 mmol) were added to 1,4-dioxane (2 mL). The mixture was reacted at 100°C for 2 hours under an argon atmosphere. The reaction mixture was subjected to preparative thin-layer chromatography (dichloromethane/methanol = 15/1) to obtain the title compound (11 mg, 20%) as a brown solid.

$^1$H NMR (400 MHz, CHLOROFORM-*d*) 9.37 (s, 1 H), 8.28 (s, 3 H), 7.37 - 7.35 (m, 1 H), 7.16 (s, 1 H), 6.80 (s, 1 H), 4.58 (br. s., 2 H), 4.45 (br. s., 2 H), 3.83 (s, 3 H), 3.42 (s, 3 H), 2.73 (s, 2 H), 1.71 - 1.70 (m, 3 H). LC-MS: m/z $[M+H]^+$ = 448.

Example 5

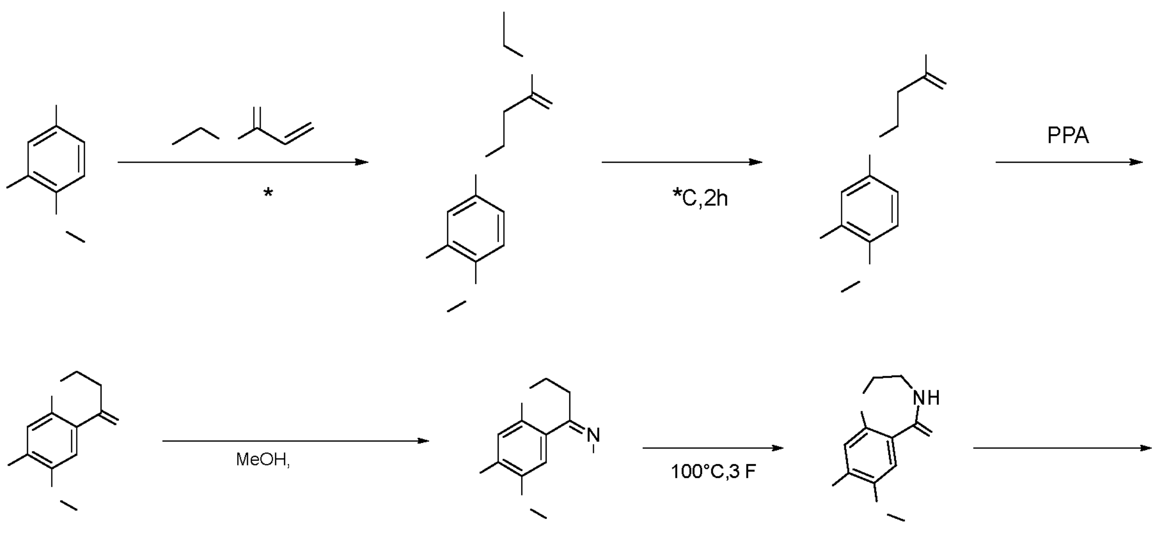

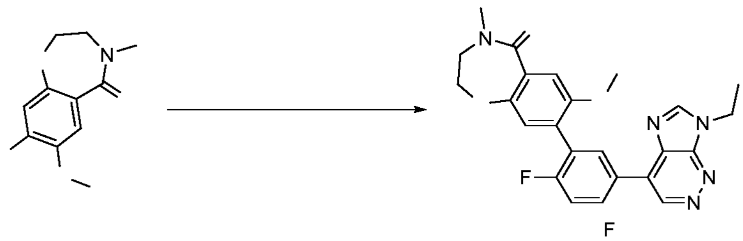

### 5-1: Ethyl 3-(3-bromo-4-methoxyphenoxy)propanoate

3-Bromo-4-methoxyphenol (10 g, 49 mmol) was added to ethyl acrylate (70 mL) at 25°C, then a catalytic amount of 4-dimethylaminopyridine (0.5 g) was added thereto, and the mixture was stirred at 100°C for 24 hours. After LCMS detection, the reaction mixture was subjected to rotary evaporation to remove ethyl acrylate, dissolved in ethyl acetate, then mixed with silica gel, and purified by column chromatography (petroleum ether: ethyl acetate = 3:1) to obtain the product (7 g, 47%) as an off-white solid. LC-MS: m/z $[M+H]^+$ = 303.

### 5-2: 3-(3-Bromo-4-methoxyphenoxy)propanoic acid

Ethyl 3-(3-bromo-4-methoxyphenoxy)propanoate (600 mg, 2 mmol) was added to concentrated hydrochloric acid (5 mL), and the mixture was heated to reflux at 100°C for 2 hours. The reaction mixture was diluted with water and extracted with ethyl acetate (100 mL × 2). The organic phases were combined, washed with water and brine, then dried over anhydrous sodium sulfate, and concentrated under vacuum. The residue was then purified by column chromatography (petroleum ether: ethyl acetate = 1:2) to obtain the product (400 mg, 72%) as an off-white solid. LC-MS: m/z $[M+H]^+$ = 276.

### 5-3: 7-Bromo-6-methoxychroman-4-one

3-(3-Bromo-4-methoxyphenoxy)propanoic acid (400 mg, 1.45 mmol) was added to polyphosphoric acid (5 mL) at 25°C, and the mixture was stirred at 80°C for 2 hours. The reaction mixture was poured into 20 mL of water, stirred for 30 minutes, and extracted with ethyl acetate (50 mL × 2). The organic phases were combined, dried over anhydrous sodium sulfate, and concentrated under vacuum to obtain the crude product (500 mg, 100%) as a yellow solid. LC-MS: m/z $[M+H]^+$ = 257.

### 5-4: (Z)-7-Bromo-6-methoxychroman-4-one oxime

7-Bromo-6-methoxychroman-4-one (500 mg, 1.94 mmol) was dissolved in ethanol (7 mL) at 25°C, then hydroxylamine hydrochloride (404 mg, 5.82 mmol) was added thereto, and 2 M sodium hydroxide solution (5 mL) was added dropwise thereto. The mixture was stirred at 25°C for 16 hours. The reaction mixture was poured into water, extracted with ethyl acetate, dried, and rotary evaporated to dryness to obtain the crude product (240 mg, 45%) as a yellow solid. LC-MS: m/z $[M+H]^+$ = 273.

### 5-5: 8-Bromo-7-methoxy-3,4-dihydrobenzo[*f*][1,4]oxazepin-5(2H)-one

(*Z*)-7-Bromo-6-methoxychroman-4-one oxime (240 mg, 0.22 mmol) was dissolved in polyphosphoric acid (5 mL) at 25°C, and the mixture was stirred at 100°C for 3 hours. The reaction mixture was poured into water, extracted with ethyl acetate, dried, and rotary evaporated to dryness to obtain the crude product (180 mg, 75%). LC-MS: m/z $[M+H]^+$ = 273.

### 5-6: 8-Bromo-7-methoxy-4-methyl-3,4-dihydrobenzo[*f*][1,4]oxazepin-5(2H)-one

8-Bromo-7-methoxy-3,4-dihydrobenzo[*f*][1,4]oxazepin-5(2*H*)-one (180 mg, 0.36 mmol) was dissolved in *N,N*-dimethylformamide (5 mL) at 25°C, then NaH (23 mg, 0.55 mmol) was added thereto in batches, and the mixture was stirred for 30 minutes. Iodomethane (142 mg, 1 mmol) was then added thereto, and the mixture was stirred at 25°C for 2 hours. The reaction mixture was poured into water, extracted with ethyl acetate, dried, and rotary evaporated to dryness to obtain the crude product (100 mg, 96%) as a yellow solid. LC-MS: m/z $[M+H]^+$ = 287.

**5: 8-(5-(7-Ethyl-7*H*-imidazo[4,5-c]pyridazin-4-yl)-2-fluorophenyl)-7-methoxy-4-methyl-3,4-dihydrobenzo[*f*][1,4]oxazepin-5(2*H*)-one**

Following the same experimental procedure as in Example 1, 8-bromo-7-methoxy-4-methyl-3,4-dihydrobenzo[*f*][1,4]oxazepin-5(2*H*)-one (100 mg, 0.35 mmol) and (5-(7-ethyl-7*H*-imidazo[4,5-*c*]pyridazin-4-yl)-2-fluorophenyl)boronic acid (100 mg, 0.35 mmol) were used as starting materials to obtain the title compound (25 mg, 16%) as an off-white solid.
$^1$H NMR (400 MHz, DMSO-$d_6$) 9.56 (s, 1 H), 8.86 (s, 1 H), 8.47 (tt, *J* = 7.3, 2.3 Hz, 2 H), 7.57 - 7.45 (m, 1 H), 7.31 (s, 1 H), 7.07 (s, 1 H), 4.51 (q, *J* = 7.3 Hz, 2 H), 4.35 (t, *J* = 5.1 Hz, 2 H), 3.78 (s, 3 H), 3.59 (t, *J* = 5.2 Hz, 2 H), 3.13 (s, 3 H), 1.56 (t, *J* = 7.3 Hz, 3 H). LC-MS: m/z [M+H]$^+$ = 448.

Example 6

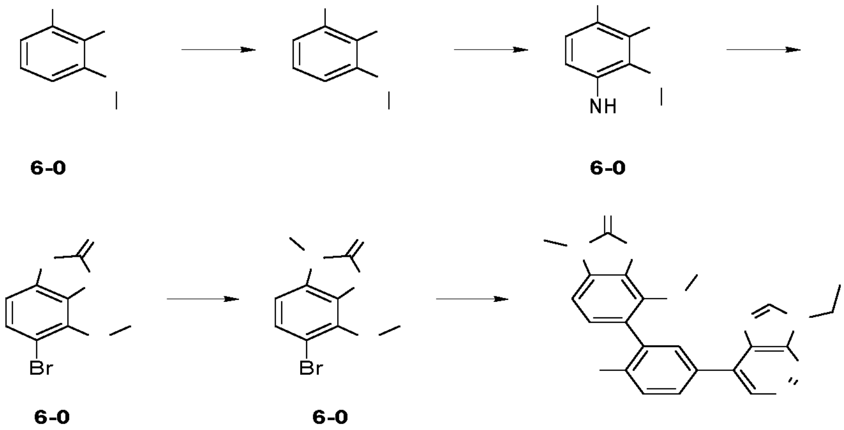
6-0
6-0
6-0
6-0

**6-1: 2-Amino-6-methoxyphenol**

2-Nitro-6-methoxyphenol (CAS: 15969-08-1, 900 mg, 5.3 mmol) was dissolved in 10 mL of methanol, then Raney nickel (1 g) was added thereto, and the mixture was stirred at room temperature for 30 minutes under a hydrogen atmosphere. The reaction mixture was filtered and concentrated to obtain the title compound (600 mg, 80%) as a yellow solid. LC-MS: m/z [M+H]$^+$ = 140.

**6-2: 6-Amino-3-bromo-2-methoxyphenol**

2-Amino-6-methoxyphenol (600 mg, 4.3 mmol) was dissolved in 10 mL of dichloromethane, and the mixture was cooled to 0°C. N-Bromosuccinimide (613 mg, 3.45 mmol) was added thereto, and the mixture was stirred at 0°C for 30 minutes. The reaction mixture was concentrated and purified by column chromatography (petroleum ether: ethyl acetate = 5:1) to obtain the title compound (110 mg, 12%) as a yellow solid. LC-MS: m/z [M+H]$^+$ = 218.

**6-3: 6-Bromo-7-methoxybenzo[d]oxazol-2(3H)-one**

6-Amino-3-bromo-2-methoxyphenol (100 mg, 0.46 mmol) was dissolved in 10 mL of tetrahydrofuran, then triphosgene (273 mg, 0.92 mmol) was added thereto, and the mixture was stirred at room temperature for 2 hours. The reaction mixture was concentrated and purified by preparative thin-layer chromatography (dichloromethane: methanol = 20:1) to obtain the title compound (100 mg, 89%) as a white solid. LC-MS: m/z [M+H]$^+$ = 244.

**6-4: 6-Bromo-7-methoxy-3-methylbenzo[*d*]oxazol-2(3*H*)-one**

6-Bromo-7-methoxybenzo[*d*]oxazol-2(3*H*)-one (100 mg, 0.41 mmol) was dissolved in 10 mL of acetonitrile, then iodomethane (116 mg, 0.82 mmol) and cesium carbonate (270 mg, 0.82 mmol) were added thereto, and the mixture was stirred at room temperature for 2 hours. The reaction mixture was filtered to remove the solid, and concentrated to obtain the title compound (100 mg, 94%) as a yellow solid. LC-MS: m/z [M+H]$^+$ = 258.

**6: 6-(5-(7-Ethyl-7*H*-imidazo[4,5-c]pyridazin-4-yl)-2-fluorophenyl)-7-methoxy-3-methylbenzo[*d*]oxazol-2(3*H*)-one**

Following the same experimental procedure as in Example 4, 7-ethyl-4-(4-fluoro-3-(4,4,5,5-tetramethyl-1,3,2-dioxaborolan-2-yl)phenyl)-7*H*-imidazo[4,5-c]pyridazine (100 mg, 0.35 mmol) and 6-bromo-7-methoxy-3-methylbenzo[*d*]oxazol-2(3*H*)-one (90 mg, 0.35 mmol) were used as starting materials to obtain the title compound (10 mg, 7%) as a white solid.

$^1$H NMR (400 MHz, CHLOROFORM-*d*) δ 9.39 (s, 1 H) 8.26 - 8.37 (m, 3 H) 7.40 (s, 1 H) 7.08 (d, *J* = 8.31 Hz, 1 H) 6.82 (d, *J* = 8.80 Hz, 1 H) 4.60 (d, *J* = 7.34 Hz, 2 H) 4.04 (s, 3 H) 3.16 (s, 3 H) 1.70 (t, *J* = 7.58 Hz, 3 H). LC-MS: m/z [M+H]$^+$ = 420.

Example 7

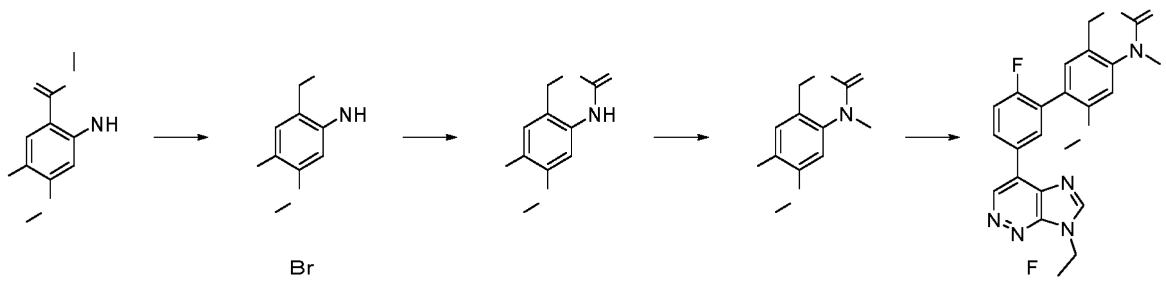

**7-1: (2-Amino-5-bromo-4-methoxyphenyl)methanol**

Methyl 2-amino-5-bromo-4-methoxybenzoate (500 mg, 1.92 mmol) was added to tetrahydrofuran (10 mL), then lithium aluminum hydride (291 mg, 7.68 mmol) was added to the reaction system, and the reaction mixture was stirred at room temperature for 2 hours. The reaction mixture was directly subjected to thin-layer chromatography (dichloromethane/methanol = 20/1) to obtain the title compound (380 mg, 85%) as a yellow oil. LC-MS: m/z [M+H]$^+$ = 213.9.

**7-2: 6-Bromo-7-methoxy-1,4-dihydro-2*H*-benzo[*d*][1,3]oxazin-2-one**

(2-Amino-5-bromo-4-methoxyphenyl)methanol (380 mg, 1.64 mmol) and bis(trichloromethyl)carbonate (1947 mg, 6.56 mmol) were added to tetrahydrofuran (10 mL), and the mixture was stirred at room temperature for 2 hours. The reaction mixture was directly concentrated and then purified by thin-layer chromatography (dichloromethane/methanol = 20/1) to obtain the title compound (180 mg, 42%) as a yellow solid. LC-MS: m/z [M+H]$^+$ = 258.

**7-3: 6-Bromo-7-methoxy-1-methyl-1,4-dihydro-2*H*-benzo[*d*][1,3]oxazin-2-one**

6-Bromo-7-methoxy-1,4-dihydro-2*H*-benzo[*d*][1,3]oxazin-2-one (150 mg, 0.58 mmol), potassium carbonate (80 mg, 0.58 mol), and iodomethane (247 mg, 1.74 mmol) were added to methanol (5 mL), and the mixture was stirred at 40°C overnight. The reaction mixture was directly subjected to thin-layer chromatography (petroleum ether/ethyl acetate = 5/1) to obtain the title compound (150 mg, 95%) as a yellow solid. LC-MS: m/z [M+H]$^+$ = 272.

**7: 6-(5-(7-Ethyl-7*H*-imidazo[4,5-*c*]pyridazin-4-yl)-2-fluorophenyl)-7-methoxy-1-methyl-1,4-dihydro-2*H*-benzo[*d*][1,3]oxazin-2-one**

6-Bromo-7-methoxy-1-methyl-1,4-dihydro-2*H*-benzo[*d*][1,3]oxazin-2-one (90 mg, 0.33 mmol), (5-(7-ethyl-7*H*-imidazo[4,5-*c*]pyridazin-4-yl)-2-fluorophenyl)boronic acid (122 mg, 0.33 mmol), bis(triphenylphosphine)palladium(II) chloride (23 mg, 0.033 mmol), and sodium carbonate (70 mg, 0.66 mmol) were added to tetrahydrofuran/water (5 mL/1 mL). The mixture was reacted at 80°C overnight under an argon atmosphere. The reaction mixture was directly purified by thin-layer chromatography (dichloromethane/methanol = 20/1) to obtain the title compound (28 mg, 19%) as a yellow solid.

$^1$H NMR (400 MHz, CHLOROFORM-*d*) ppm 1.68 - 1.73 (m, 3 H) 3.47 (s, 3 H) 3.89 (s, 3 H) 4.54 - 4.63 (m, 2 H) 5.22 (s, 2 H) 7.15 (s, 1 H) 7.34 (t, *J* = 9.29 Hz, 1 H) 7.64 - 7.72 (m, 1 H) 8.19 (d, *J* = 6.85 Hz, 1 H) 8.28 (s, 2 H) 9.39 (br. s., 1 H). LC-MS: m/z [M+H]$^+$ = 434.

Example 8 and Example 9

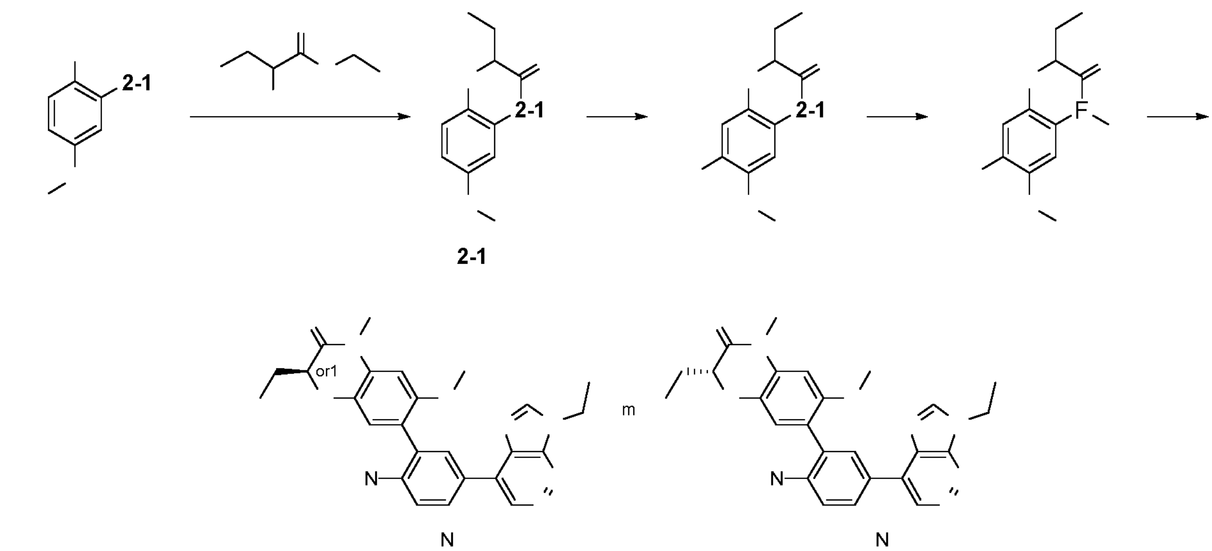

**8-1: 2-Ethyl-6-methoxy-2*H*-benzo[*b*][1,4]oxazin-3(4*H*)-one**

2-Amino-4-methoxyphenol (1.9 g, 13.8 mmol) and ethyl 2-bromo-butyrate (3 g, 15.2 mmol) were dissolved in 20 mL of *N,N*-dimethylformamide under a nitrogen atmosphere, then cesium carbonate (9 g, 27.6 mmol) was added thereto, and the mixture was reacted at 95°C for 16 hours. After the reaction was completed, the reaction mixture was added to 50 mL of water, extracted with ethyl acetate (50 mL × 3), and back-washed with water (30 mL × 3). The organic phase was rotary evaporated to dryness and subjected to column chromatography (petroleum ether/ethyl acetate = 5/1) to obtain the title compound (1 g, 35%) as a white solid. LC-MS: m/z $[M+H]^+$ = 208.

**8-2: 7-Bromo-2-ethyl-6-methoxy-2*H*-benzo[*b*][1,4]oxazin-3(4*H*)-one**

2-Ethyl-6-methoxy-2*H*-benzo[*b*][1,4]oxazin-3(4*H*)-one (3.4 g, 16.4 mmol) was dissolved in dimethylformamide (40 mL), then N-bromosuccinimide (5.8 g, 32.8 mmol) was added thereto in batches, and the mixture was reacted at room temperature for 2 hours. After the reaction was completed, the system was poured into ice water, extracted with ethyl acetate (50 mL × 3), dried over anhydrous sodium sulfate, and purified by prep-HPLC to obtain the title product, which was rotary evaporated to dryness to obtain 7-bromo-2-ethyl-6-methoxy-2H-benzo[b][1,4]oxazin-3(4H)-one (2.1 g, 45%) as a yellow solid. LC-MS: m/z $[M+1]^+$ = 208.

**8-3: 7-Bromo-2-ethyl-6-methoxy-4-methyl-2*H*-benzo[*b*][1,4]oxazin-3(4*H*)-one**

7-Bromo-2-ethyl-6-methoxy-2*H*-benzo[*b*][1,4]oxazin-3(4*H*)-one 1c (2.1 g, 7.4 mmol) was dissolved in tetrahydrofuran (30 mL), and the mixture was stirred for 10 minutes. Sodium hydride (596 mg, 14.8 mmol) was added thereto at 0°C, and the mixture was stirred for 20 minutes. Iodomethane (2.1 g, 14.8 mmol) was then added thereto, the ice-water bath was removed, and the mixture was reacted at room temperature for 2 hours. The reaction mixture was poured into ice water (20 mL), extracted with ethyl acetate (50 mL × 3), dried over anhydrous sodium sulfate, purified by prep-HPLC, and rotary evaporated to dryness to obtain the title compound (2.0 g, oily liquid, yield: 91%). LCMS: m/z $[M+1]^+$ = 301.

**8: *rel*-(*S*)-2-Ethyl-7-(5-(7-ethyl-7*H*-imidazo[4,5-*c*]pyridazin-4-yl)-2-fluorophenyl)-6-methoxy-4-methyl-2*H*-benzo [*b*][1,4]oxazin-3(4*H*)-one**

**9: *rel*-(*R*)-2-Ethyl-7-(5-(7-ethyl-7*H*-imidazo[4,5-*c*]pyridazin-4-yl)-2-fluorophenyl)-6-methoxy-4-methyl-2*H*-benzo [*b*][1,4]oxazin-3(4*H*)-one**

Following the same experimental procedure as in Example 1, 7-bromo-2-ethyl-6-methoxy-4-methyl-2*H*-benzo [*b*][1,4]oxazin-3(4H*)*-one (2.0 g, 6.7 mmol) and (5-(7-ethyl-7*H*-imidazo[4,5-*c*]pyridazin-4-yl)-2-fluorophenyl)boronic acid

(1.9 g, 6.7 mmol) were used as starting materials to obtain the title compound (1.0 g, 33%) as an off-white solid. The solid was further subjected to chiral resolution to obtain compounds of Example 8 (first peak, 10 mg, white solid) and Example 9 (second peak, 10 mg, white solid).

Resolution method: instrument: MG II preparative SFC (SFC-1); column: ChiralPak AD, 250 × 30 mm I.D., 10 μm; mobile phase: A for $CO_2$ and B for isopropanol; gradient: B 55%; flow rate: 80 mL/min; back pressure: 100 bar; column temperature: 38°C; wavelength: 220 nm.

Example 8: $^1$H NMR (400 MHz, DMSO-$d_6$) 9.55 (s, 1 H), 8.86 (s, 1 H), 8.44 (d, *J* = 6.0 Hz, 2 H), 7.48 (t, *J* = 9.4 Hz, 1 H), 7.05 (s, 1 H), 6.92 (s, 1 H), 4.51 (d, *J* = 7.2 Hz, 3 H), 3.81 (s, 3 H), 3.39 (s, 3 H), 1.83 (ddd, *J* = 21.7, 14.2, 6.8 Hz, 2 H), 1.56 (t, *J* = 7.3 Hz, 3 H), 1.01 (s, 3 H). LC-MS: m/z [M+1]$^+$ = 462.

Example 9: $^1$H NMR (400 MHz, DMSO-$d_6$) 9.37 (s, 1 H), 8.34 - 8.24 (m, 2 H), 8.19 (dd, *J* = 6.9, 2.1 Hz, 1 H), 7.33 (t, *J* = 9.0 Hz, 1 H), 7.05 (s, 1 H), 6.62 (s, 1 H), 4.58 (q, *J* = 7.3 Hz, 2 H), 4.48 (dd, *J* = 8.5, 4.4 Hz, 1 H), 3.83 (s, 3 H), 3.43 (s, 3 H), 2.11 - 1.83 (m, 3 H), 1.69 (t, *J* = 7.3 Hz, 4 H), 1.09 (t, *J* = 7.4 Hz, 3 H). LC-MS: m/z [M+1]$^+$ = 462.

Example 10

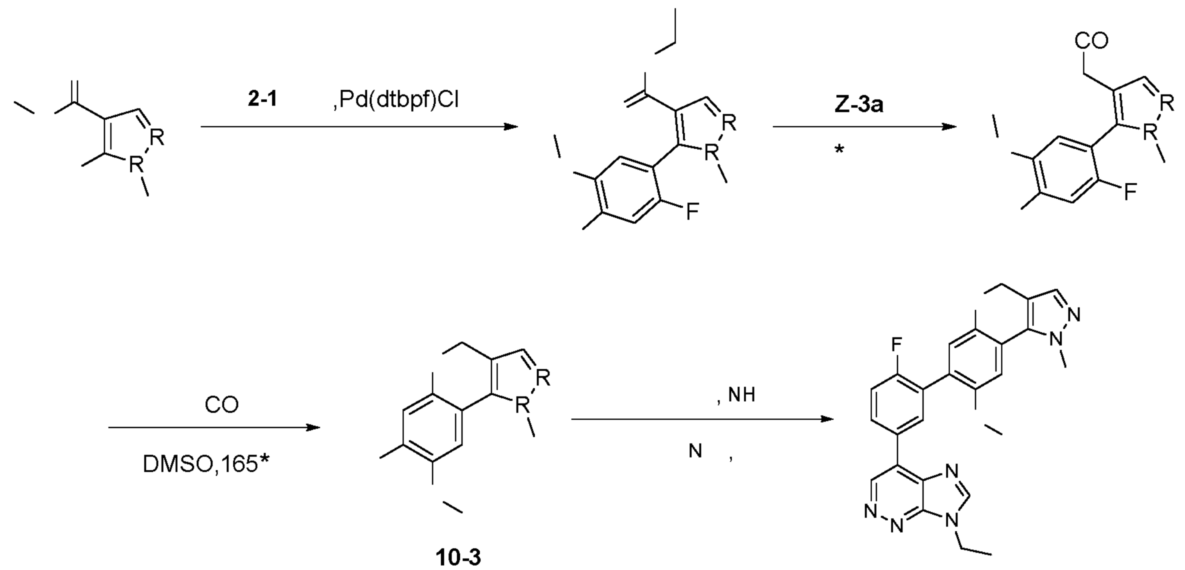


10-3

**10-1: Ethyl 5-(4-chloro-2-fluoro-5-methoxyphenyl)-1-methyl-1*H*-pyrazole-4-carboxylate**

Following the same experimental procedure as in Example 1, methyl 5-bromo-1-methyl-1*H*-pyrazole-4-carboxylate (300 mg, 1.37 mmol) and (4-chloro-2-fluoro-5-methoxyphenyl)boronic acid (279 mg, 1.37 mmol) were used as starting materials to obtain the title compound (200 mg, yield: 47%) as a yellow solid. LC-MS: m/z [M+1]$^+$ = 313.

**10-2: (5-(4-Chloro-2-fluoro-5-methoxyphenyl)-1-methyl-1*H*-pyrazol-4-yl)methanol**

Ethyl 5-(4-chloro-2-fluoro-5-methoxyphenyl)-1-methyl-1*H*-pyrazole-4-carboxylate (400 mg, 1.28 mmol) was dissolved in tetrahydrofuran (40 mL), and the mixture was cooled to 0°C. Lithium borohydride (84 mg, 3.84 mmol) was then added thereto. The mixture was reacted at room temperature for 3 hours. After the reaction was completed, the reaction mixture was poured into ice water (20 mL), extracted with ethyl acetate (50 mL × 3), dried over anhydrous sodium sulfate, purified by prep-HPLC, and rotary evaporated to dryness to obtain the title compound (150 mg, 43%) as an oily liquid. LC-MS: m/z [M+1]$^+$ = 272.

**10-3: 7-Chloro-8-methoxy-1-methyl-1,4-dihydrochromeno[4,3-*c*]pyrazole**

(5-(4-Chloro-2-fluoro-5-methoxyphenyl)-1-methyl-1*H*-pyrazol-4-yl)methanol (200 mg, 0.74 mmol) was dissolved in dimethyl sulfoxide (10 mL), then potassium carbonate (200 mg) was added thereto, and the mixture was heated to 165°C and reacted for 3 hours. After the reaction was completed, the reaction mixture was poured into ice water (20 mL), extracted with ethyl acetate (50 mL × 3), dried over anhydrous sodium sulfate, purified by prep-HPLC, and rotary evaporated to dryness to obtain the title compound (75 mg, oily liquid, yield: 40%). LC-MS: m/z [M+1]$^+$ = 251.

**10: 7-(5-(7-Ethyl-7*H*-imidazo[4,5-c]pyridazin-4-yl)-2-fluorophenyl)-8-methoxy-1-methyl-1,4-dihydrochromeno [4,3-*c*]pyrazole**

Following the same experimental procedure as in Example 1, 7-chloro-8-methoxy-1-methyl-1,4-dihydrochromeno[4,3-c]pyrazole (20.0 mg, 0.08 mmol) and (5-(7-isopropyl-7*H*-imidazo[4,5-*c*]pyridazin-4-yl)-2-fluorophenyl)boronic acid (23 mg, 0.08 mmol) were used as starting materials to obtain the title compound (8 mg, 22%) an off-white solid.

$^1$H NMR (400 MHz, $CDCl_3$) 9.39 (s, 1 H), 8.29 (s, 2 H), 8.24 - 8.17 (m, 1 H), 7.35 (t, *J* = 9.0 Hz, 2 H), 7.17 (s, 1 H), 7.10 (s, 1 H), 5.22 (s, 2 H), 4.58 (q, *J* = 7.2 Hz, 2 H), 4.22 (s, 3 H), 3.86 (s, 3 H), 1.69 (t, *J* = *7.3* Hz, 3 H). LC-MS: m/z $[M+1]^+$ = 457.

Example 11

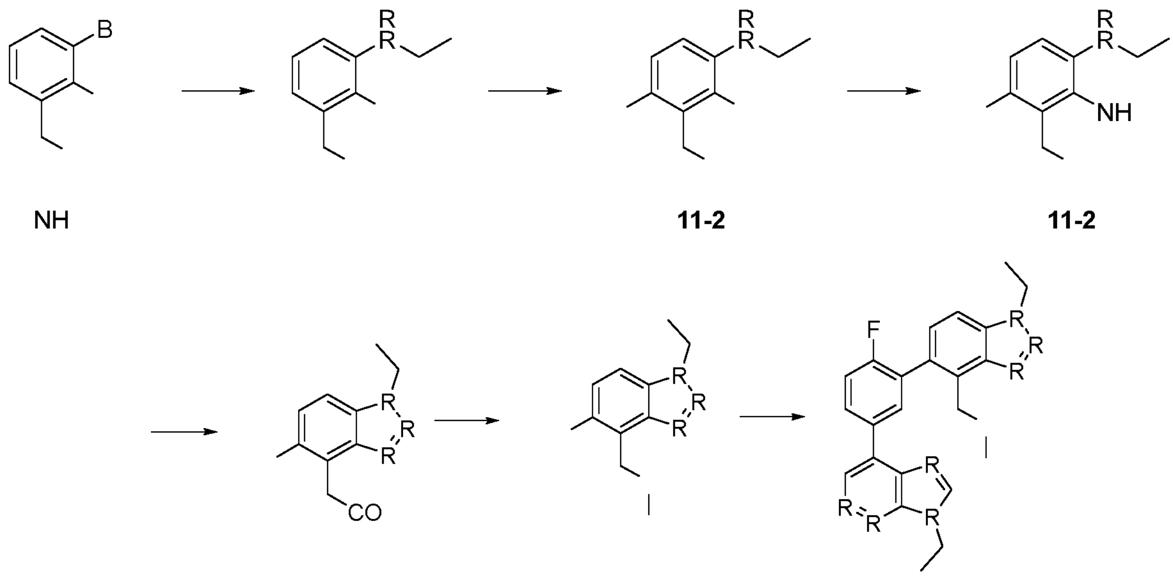

NH 11-2 11-2

**11-1: (3-(Ethylamino)-2-nitrophenyl)methanol**

(3-Fluoro-2-nitrophenyl)methanol (1000 mg, 5.84 mmol) and ethylamine aqueous solution (50%, 4 mL) were added to ethanol (10 mL), and the mixture was stirred in a sealed tube at 100°C overnight. The reaction mixture was directly subjected to column chromatography (dichloromethane/methanol = 30/1) to obtain the title compound (1100 mg, 96%) as a red solid. LC-MS: m/z $[M+H]^+$ = 197.

**11-2: (6-Bromo-3-(ethylamino)-2-nitrophenyl)methanol**

Following the same experimental procedure as in the synthesis method of 2-3 in Example 2, (3-(ethylamino)-2-nitrophenyl)methanol (1100 mg, 5.61 mmol) was used as the starting material to obtain the title compound (1500 mg, 97%) as a red solid. LC-MS: m/z $[M+H]^+$ = 275.

**11-3: (2-Amino-6-bromo-3-(ethylamino)phenyl)methanol**

Following the same experimental procedure as in the synthesis method of 2-4 in Example 2, (6-bromo-3-(ethylamino)-2-nitrophenyl)methanol (1500 mg, 5.47 mmol) was used to obtain the title compound (1300 mg, 97%) as a yellow solid. LC-MS: m/z $[M+H]^+$ = 245.

**11-4: (5-Bromo-1-ethyl-1*H*-benzo[*d*][1,2,3]triazol-4-yl)methanol**

Following the same experimental procedure as in the synthesis method of 3-4 in Example 3, (2-amino-6-bromo-3-(ethylamino)phenyl)methanol (1300 mg, 5.3 mmol) was used as the starting material to obtain the title compound (1100 mg, 81%) as a brown solid. LC-MS: m/z $[M+H]^+$ = 256.

**11-5: 5-Bromo-1-ethyl-4-(methoxymethyl)-1*H*-benzo[*d*][1,2,3]triazole**

Following the same experimental procedure as in the synthesis method of 2-6 in Example 2, (5-bromo-1-ethyl-1*H*-benzo[*d*][1,2,3]triazol-4-yl)methanol (1100 mg, 4.3 mmol) was used as the starting material to obtain the title compound (1100 mg, 95%) as a yellow oil. LC-MS: m/z $[M+H]^+$ = 270.

**11: 7-Ethyl-4-(3-(1-ethyl-4-(methoxymethyl)-1*H*-benzo[*d*][1,2,3]triazol-5-yl)-4-fluorophenyl)-7*H*-imidazo[4,5-*c*]pyridazine**

Following the same experimental procedure as in Example 1, 5-bromo-1-ethyl-4-(methoxymethyl)-1*H*-benzo[*d*][1,2,3]triazole (1100 mg, 4.07 mmol) and (5-(7-ethyl-7*H*-imidazo[4,5-*c*]pyridazin-4-yl)-2-fluorophenyl)boronic acid (932 mg, 3.26 mmol) were used as starting materials to obtain the title compound (545 mg, 31%) as a white solid.

$^1$H NMR (400 MHz, DMSO-$d_6$) 1.54 (s, 6 H) 3.12 (s, 3 H) 4.46 - 4.54 (m, 2 H) 4.76 - 4.85 (m, 4 H) 7.55 - 7.62 (m, 2 H) 7.96 - 8.00 (m, 1 H) 8.52 - 8.59 (m, 2 H) 8.83 - 8.86 (m, 1 H) 9.53 - 9.56 (m, 1 H). LC-MS: m/z $[M+H]^+$ = 432.2.

Example 12

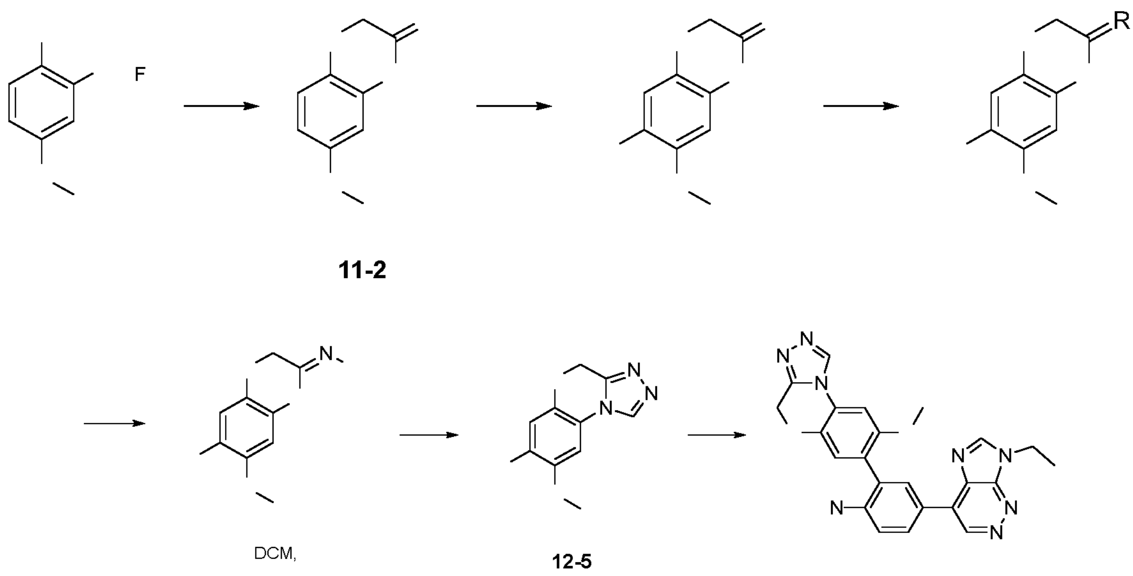


11-2

12-5

**12-1: 6-Methoxy-2*H*-benzo[*b*][1,4]oxazin-3(4*H*)-one**

2-Amino-4-methoxyphenol (10000 mg, 71.68 mmol), potassium carbonate (19860 mg, 147.72 mmol), and chloroacetyl chloride (8120 mg, 71.68 mmol) were sequentially added to acetonitrile (80 mL), and the mixture was stirred at 90°C for 16 hours. The reaction mixture was added with water (200 mL), filtered to remove the precipitated solid, and concentrated to obtain the title compound (12000 mg, 93%) as a purple solid. LC-MS: m/z $[M+H]^+$ = 259, 261.

**12-2: 7-Bromo-6-methoxy-2*H*-benzo[*b*][1,4]oxazin-3(4*H*)-one**

6-Methoxy-2*H*-benzo[*b*][1,4]oxazin-3(4*H*)-one (12000 mg, 66.98 mmol) and a solution of hydrobromic acid in acetic acid (33 wt%) (24633 mg, 100.47 mmol) were sequentially added to acetic acid (80 mL), and the mixture was stirred at room temperature for 0.5 hours. Hydrogen peroxide (2278 mg, 66.98 mmol) was then added thereto, and the mixture was stirred at room temperature for 2 hours. The reaction mixture was added with water (200 mL), filtered to remove the precipitated solid, and concentrated to obtain the title compound (10000 mg, 46%) as a purple solid. LC-MS: m/z $[M+H]^+$ = 259, 261.

**12-3: 7-Bromo-6-methoxy-2*H*-benzo[*b*][1,4]oxazine-3(4*H*)-thione**

Phosphorus pentasulfide (947.3 mg, 4.26 mmol), potassium carbonate (802.9 mg, 5.81 mmol), and TEBA (44.1

mg, 0.19 mmol) were suspended in 1,2-dichloroethane (40 mL) under a nitrogen atmosphere, and the mixture was stirred at 100°C for 2 hours. After cooling to approximately 40°C, 7-bromo-6-methoxy-2*H*-benzo[*b*][1,4]oxazin-3(4*H*)-one (1.0 g, 3.87 mmol) was added to the reaction mixture and reacted at 100°C for 15 minutes. The reaction mixture was subjected to hot filtration, and the filter cake was washed three times with dichloromethane. The filtrate was concentrated and purified by column chromatography (dichloromethane/ethyl acetate = 3/1) to obtain the title product (0.51 g, 48%) as a yellow solid. LC-MS: m/z $[M+H]^+$ = 275.

**12-4: (*Z*)-7-Bromo-3-hydrazono-6-methoxy-3,4-dihydro-2*H*-benzo[*b*][1,4]oxazine**

7-Bromo-6-methoxy-2*H*-benzo[*b*][1,4]oxazine-3(4*H*)-thione (470 mg, 1.7 mmol) was dissolved in 20 mL of ethanol under a nitrogen atmosphere, then hydrazine hydrate (103 mg, 2.1 mmol) was added thereto, and the mixture was reacted at room temperature for 2 hours. The reaction mixture was added with water (100 mL), stirred for 30 minutes, and filtered. The filter cake was washed three times with water and dried under vacuum to obtain the title product (290 mg, brown solid) with a yield of 62%. LC-MS: m/z $[M+H]^+$ = 273.

**12-5: 7-Bromo-8-methoxy-4*H*-benzo[*b*][1,2,4]triazolo[4,3-*d*][1,4]oxazine**

(*Z*)-7-Bromo-3-hydrazono-6-methoxy-3,4-dihydro-2*H*-benzo[*b*][1,4]oxazine (290 mg, 1.07 mmol) was suspended in 12 mL of trimethyl orthoformate under a nitrogen atmosphere, and the mixture was stirred at 125°C for 2 hours. The reaction mixture was then purified by column chromatography (dichloromethane/ethyl acetate = 3/1) to obtain the title product (202 mg, 67%) as a white solid. LC-MS: m/z $[M+H]^+$ = 283.

**12: 7-(5-(7-Ethyl-7*H*-imidazo[4,5-*c*]pyridazin-4-yl)-2-fluorophenyl)-8-methoxy-4*H*-benzo[*b*][1,2,4]triazolo[4,3-*d*][1,4]oxazine**

Following the same experimental procedure as in Example 1, 7-bromo-8-methoxy-4H-benzo[*b*][1,2,4]triazolo[4,3-*d*][1,4]oxazine (100.0 mg, 0.35 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (100.1 mg, 0.35 mmol) were used as starting materials to obtain the title compound (23 mg, 15%) as an off-white solid.

$^1$H NMR (400 MHz, DMSO-$d_6$) 9.57 (s, 1 H), 9.47 (s, 1 H), 8.87 (s, 1 H), 8.47 (d, *J* = 6.7 Hz, 2 H), 7.72 (s, 1 H), 7.51 (t, *J* = 9.5 Hz, 1 H), 7.27 (s, 1 H), 5.50 (s, 2 H), 4.51 (dd, *J* = 14.5, 7.3 Hz, 2H), 3.85 (s, 3 H), 1.56 (t, *J* = 7.2 Hz, 3 H). LC-MS: m/z $[M+H]^+$ = 444.

Example 13

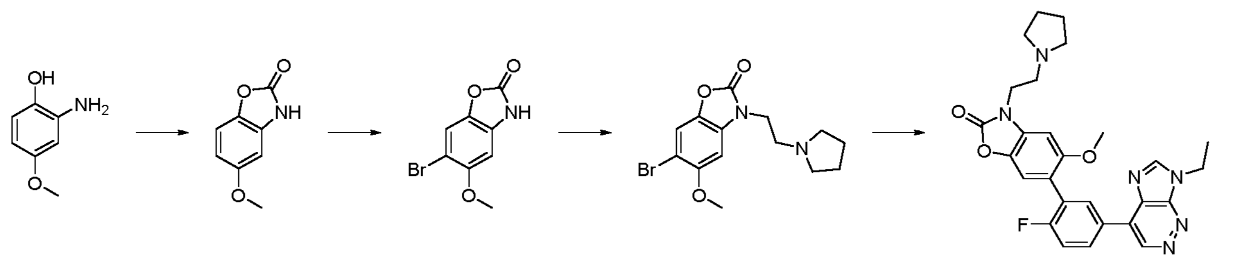

12-0 13-1 13-2 13-3 13

**13-1: 5-Methoxybenzo[*d*]oxazol-2(3*H*)-one**

2-Amino-4-methoxyphenol (5.01 g, 35.93 mmol) was dissolved in anhydrous dichloromethane (100.0 mL), then *N,N*-carbonyldiimidazole (6.41 g, 39.53 mmol) was added thereto in batches, and the mixture was stirred at room temperature for 16 hours. The reaction mixture was quenched with water (100 mL), then the phases were separated, and the organic phase was collected. The aqueous phase was then extracted with dichloromethane (100 mL × 3), and rotary evaporated to dryness to obtain a crude product, which was purified by column chromatography (dichloromethane: ethyl acetate) to obtain the title product (4.51 g, brown solid) with a yield of 76%. LC-MS: m/z $[M+H]^+$ = 166.

**13-2: 6-Bromo-5-methoxybenzo[*d*]oxazol-2(3*H*)-one**

5-Methoxybenzo[d]oxazol-2(3H)-one (1 g, 6.06 mmol) and a solution of hydrobromic acid in acetic acid (33%, 3.71 g, 15.1 mmol) were dissolved in acetic acid (8 mL), then hydrogen peroxide (30%, 0.895 g, 7.88 mmol) was added dropwise thereto at 0°C, and the mixture was stirred at room temperature for 2 hours. Water was added to the reaction system, and a pink solid was precipitated. The mixture was filtered to remove the solid, and concentrated to obtain the title compound (500 mg, 34%). $^1$H NMR (400 MHz, DMSO-$d_6$) 11.78 (s, 1 H), 7.59 (s, 1 H), 6.86 (s, 1 H), 3.84 (s, 3 H). LC-MS: m/z [M+H]$^+$ = 244, 244.

**13-3: 6-Bromo-5-methoxy-3-(2-(pyrrolidin-1-yl)ethyl)benzo[*d*]oxazol-2(3*H*)-one**

6-Bromo-5-methoxybenzo[*d*]oxazol-2(3*H*)-one (100 mg, 0.41 mmol), potassium carbonate (170 mg, 1.23 mmol), and 1-(2-chloroethyl)pyrrolidine hydrochloride (104.6 mmol, 0.61 mmol) were dissolved in dimethyl sulfoxide (4 mL), and the mixture was stirred at 80°C overnight. The reaction mixture was extracted three times with ethyl acetate and water. The organic phase was concentrated and purified by thin-layer chromatography (dichloromethane/methanol = 20/1) to obtain the title compound (70 mg, 50%). LC-MS: m/z [M+H]$^+$ = 341, 343.

**13: 6-(5-(7-Ethyl-7*H*-imidazo[4,5-*c*]pyridazin-4-yl)-2-fluorophenyl)-5-methoxy-3-(2-(pyrrolidin-1-yl)ethyl)benzo[*d*]oxazol-2(3*H*)-one**

Following the same experimental procedure as in Example 1, 6-bromo-5-methoxy-3-(2-(pyrrolidin-1-yl)ethyl) benzo[*d*]oxazol-2(3*H*)-one (68 mg, 0.2 mmol) and 7-ethyl-4-(4-fluoro-3-(4,4,5,5-tetramethyl-1,3,2-dioxaborolan-2-yl) phenyl)-7*H*-imidazo[4,5-*c*]pyridazine (75 mg, 0.2 mmol) were used as starting materials to obtain the title compound (30 mg, 33%).

$^1$H NMR (400 MHz, CHLOROFORM-*d*) 9.37 (s, 1 H), 8.30 - 8.17 (m, 3 H), 7.34 (t, *J* = 9.0 Hz, 1 H), 7.23 (s, 1 H), 7.00 (br. s., 1 H), 4.58 (q, *J* = 7.2 Hz, 2 H), 4.17 (br. s., 2 H), 3.87 (s, 3 H), 3.08 (br. s., 2 H), 2.85 (br. s., 4 H), 1.92 (br. s., 4 H), 1.72 - 1.68 (m, 3 H). LC-MS: m/z [M+H]$^+$ = 448.

Example 14

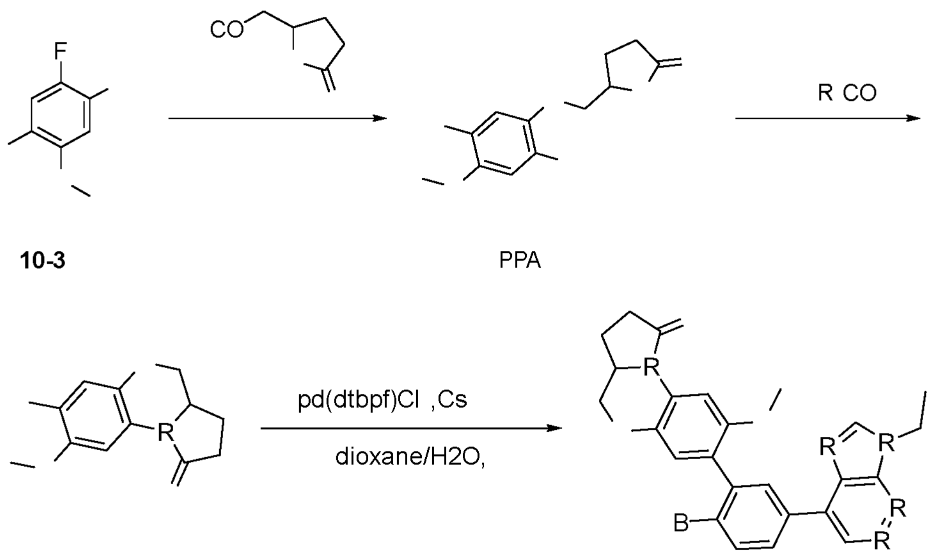

10-3

PPA

**14-1: 5-((2-Bromo-5-chloro-4-methoxyphenoxy)methyl)pyrrolidin-2-one**

1-Bromo-4-chloro-2-fluoro-5-methoxybenzene (500 mg, 2.0 mmol, 1.0 eq) was dissolved in dimethyl sulfoxide (4 mL) at 25°C, then 5-(hydroxymethyl)pyrrolidin-2-one (350 mg, 3.0 mmol, 1.5 eq) was added thereto, and cesium carbonate (500 mg, 1.5 mmol, 0.77 eq) was added thereto. After gas exchange, the mixture was reacted under microwave irradiation at 100°C for 2 hours. After LCMS detection, the reaction mixture was filtered, then purified by reverse HPLC, and lyophilized to obtain 5-((2-bromo-5-chloro-4-methoxyphenoxy)methyl)pyrrolidin-2-one (200 mg, 30%). LC-MS: m/z

$[M+H]^+$ = 334.

**14-2: 7-Chloro-8-methoxy-2,3,3a,4-tetrahydro-1*H*-benzo[*b*]pyrrolo[1,2-*d*][1,4]oxazin-1-one**

5-((2-Bromo-5-chloro-4-methoxyphenoxy)methyl)pyrrolidin-2-one (150 mg, 0.45 mmol, 1.0 eq) was dissolved in toluene (3 mL), then 2,2'-bis(diphenylphosphino)-1,1'-binaphthyl (150 mg, 0.24 mmol, 0.5 eq) was added thereto, and cesium carbonate (300 mg, 0.92 mmol, 2.0 eq) and palladium acetate (60 mg, 0.2 mmol, 0.45 eq) were added thereto. After the gas in the system was replaced with nitrogen, the mixture was reacted under microwave irradiation at 100°C for 1.5 hours. After LCMS detection, the reaction mixture was filtered, then purified by reverse HPLC, and lyophilized to obtain 7-chloro-8-methoxy-2,3,3a,4-tetrahydro-1*H*-benzo[b]pyrrolo[1,2-*d*][1,4]oxazin-1-one (70 mg, 61%). LC-MS: m/z $[M+H]^+$ = 254.

**14: 7-(5-(7-Ethyl-7*H*-imidazo[4,5-*c*]pyridazin-4-yl)-2-fluorophenyl)-8-methoxy-2,3,3a,4-tetrahydro-1*H*-benzo[b]pyrrolo[1,2-*d*][1,4]oxazin-1-one**

Following the same experimental procedure as in Example 1, 7-chloro-8-methoxy-2,3,3a,4-tetrahydro-1*H*-benzo[*b*]pyrrolo[1,2-*d*][1,4]oxazin-1-one (50 mg, 0.2 mmol) and (5-(7-ethyl-7*H*-imidazo[4,5-c]pyridazin-4-yl)-2-fluorophenyl)boronic acid (112 mg, 0.4 mmol) were used as starting materials to obtain the title compound (10 mg, 11%).

$^1$H NMR (400 MHz, DMSO-$d_6$) 9.54 (s, 1 H), 8.86 (s, 1 H), 8.43 (t, *J* = 6.4 Hz, 2 H), 8.29 (s, 1 H), 7.47 (t, *J* = 9.3 Hz, 1 H), 6.98 (s, 1 H), 4.52 (dt, *J* = 14.7, 5.2 Hz, 3 H), 4.06 (d, *J* = 9.5 Hz, 1 H), 3.77 (t, *J* = *10.3* Hz, 1 H), 3.70 (s, 3 H), 2.77 - 2.57 (m, 1 H), 2.44 - 2.31 (m, 1 H), 2.24 (t, *J* = 13.7 Hz, 1 H), 1.79 - 1.62 (m, 1 H), 1.55 (t, *J* = 7.2 Hz, 3 H). LC-MS: m/z $[M+H]^+$ = 460.

Example 15

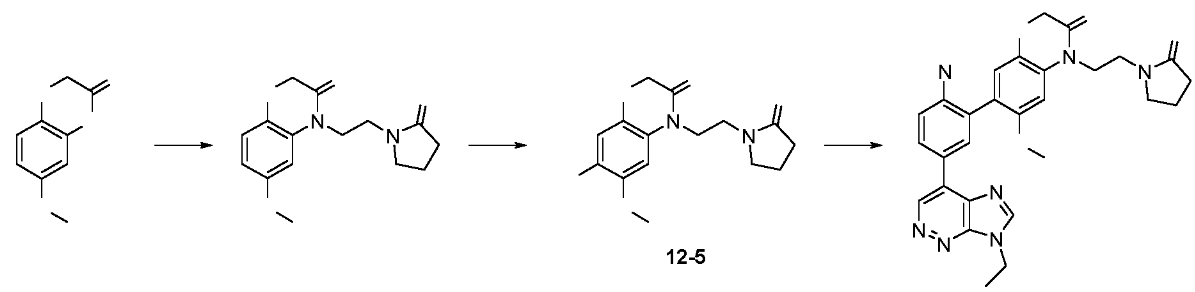

12-5

**15-1: 6-Methoxy-4-(2-(2-oxopyrrolidin-1-yl)ethyl)-2*H*-benzo[*b*][1,4]oxazin-3(4*H*)-one**

6-Methoxy-2*H*-benzo[*b*][1,4]oxazin-3(4*H*)-one (200 mg, 1.12 mmol), cesium carbonate (730 mg, 2.24 mmol), and 1-(2-chloroethyl)pyrrolidin-2-one (248 mg, 1.68 mmol) were added to acetonitrile (8 mL), and the mixture was stirred at 60°C overnight. The reaction mixture was concentrated and purified by preparative thin-layer chromatography (petroleum ether/ethyl acetate = 1/1) to obtain the title compound (220 mg, 38%).

**15-2: 7-Bromo-6-methoxy-4-(2-(2-oxopyrrolidin-1-yl)ethyl)-2*H*-benzo[*b*][1,4]oxazin-3(4*H*)-one**

6-Methoxy-4-(2-(2-oxopyrrolidin-1-yl)ethyl)-2*H*-benzo[*b*][1,4]oxazin-3(4*H*)-one (200 mg, 0.69 mmol), HBr (0.3 mL), and hydrogen peroxide (0.1 mL) were added to acetic acid (10 mL), and the mixture was stirred at room temperature for 1 hour. A large amount of water was added to the reaction mixture, and the product was precipitated from the reaction system. The reaction mixture was filtered and concentrated to obtain the title compound (140 mg, 55%).

**15: 7-(5-(7-Ethyl-7*H*-imidazo[4,5-*c*]pyridazin-4-yl)-2-fluorophenyl)-6-methoxy-4-(2-(2-oxopyrrolidin-1-yl)ethyl)-2*H*-benzo[*b*][1,4]oxazin-3(4*H*)-one**

Following the same experimental procedure as in Example 1, 7-bromo-6-methoxy-4-(2-(2-oxopyrrolidin-1-yl)ethyl)-2*H*-benzo[*b*][1,4]oxazin-3(4*H*)-one (130 mg, 0.17 mmol) and 7-ethyl-4-(4-fluoro-3-(4,4,5,5-tetramethyl-1,3,2-dioxaborolan-2-yl)phenyl)-7*H*-imidazo[4,5-c]pyridazine (130 mg, 0.35 mmol) were used as starting materials to obtain the title compound (100 mg, 54%).

$^1$H NMR (CHLOROFORM-*d*, 400 MHz) 9.36 (s, 1 H), 8.23 - 8.29 (m, 2 H), 8.19 (d, *J* = 5.9 Hz, 1 H), 7.33 (t, *J* = 8.8 Hz, 1 H), 7.14 (s, 1 H), 7.04 (s, 1 H), 4.61 (s, 2 H), 4.55 - 4.60 (m, 2 H), 4.16 (t, *J* = 7.1 Hz, 2 H), 3.92 (s, 3 H), 3.59 (t, *J* = 7.1 Hz, 2 H), 3.50 (t, *J* = 7.1 Hz, 2 H), 2.36 (t, *J* = 8.1 Hz, 2H), 1.99 - 2.06 (m, 2 H), 1.68 - 1.72 ppm (m, 3 H). LC-MS: m/z [M+H]$^+$ = 531.

Example 16

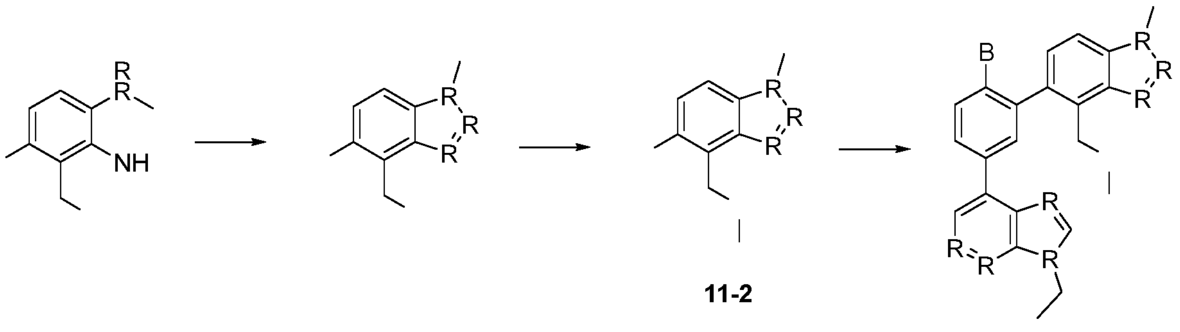

11-2

**16-1: (5-Bromo-1-methyl-1*H*-benzo[*d*][1,2,3]triazol-4-yl)methanol**

Following the same experimental procedure as in the synthesis method of 3-4 in Example 3, (2-amino-6-bromo-3-(methylamino)phenyl)methanol (200 mg, 0.87 mol) was used as the starting material to obtain the title compound (170 mg, 81%).

**16-2: 5-Bromo-4-(methoxymethyl)-1-methyl-1*H*-benzo[*d*][1,2,3]triazole**

Following the same experimental procedure as in the synthesis method of 2-6 in Example 2, (5-bromo-1-methyl-1*H*-benzo[*d*][1,2,3]triazol-4-yl)methanol (150 mg, 0.69 mmol) was used as the starting material to obtain the title compound (140 mg, 88%).

**16: 7-Ethyl-4-(4-fluoro-3-(4-(methoxymethyl)-1-methyl-1H-benzo[*d*][1,2,3]triazol-5-yl)phenyl)-7*H*-imidazo[4,5-*c*] pyridazine**

Following the same experimental procedure as in Example 1, 5-bromo-4-(methoxymethyl)-1-methyl-1*H*-benzo [d][1,2,3]triazole (101 mg, 0.4 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (120 mg, 0.33 mmol) were used as starting materials to obtain the title compound (80 mg, 58%).

$^1$H NMR (400 MHz, CHLOROFORM-d) 9.41 (s, 1 H), 8.45 - 8.39 (m, 1 H), 8.35 (d, *J* = 5.9 Hz, 1 H), 8.28 (s, 1 H), 7.57 (s, 2 H), 7.40 (t, *J* = *9.0* Hz, 1 H), 4.95 (br. s., 2 H), 4.58 (q, *J* = 7.0 Hz, 2 H), 4.36 (s, 3 H), 3.38 (s, 3 H), 1.72 - 1.67 (m, 3 H). LC-MS: m/z [M+H]$^+$ = 418.

Example 17

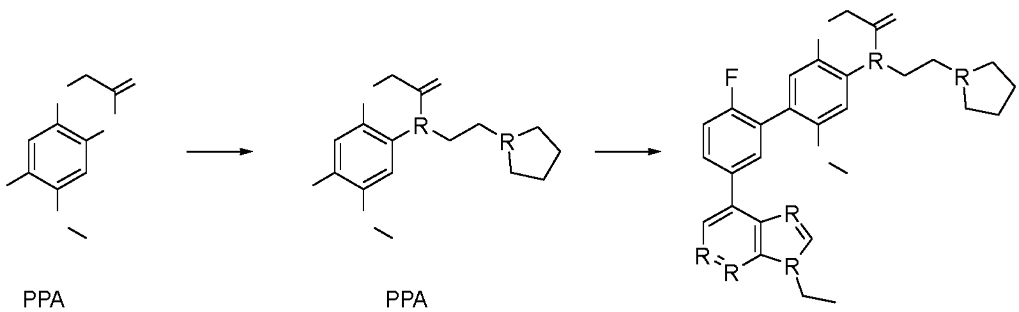

PPA PPA

**17-1: 7-Bromo-6-methoxy-4-(2-(pyrrolidin-1-yl)ethyl)-2H-benzo[b][1,4]oxazin-3(4H)-one**

7-Bromo-6-methoxy-2*H*-benzo[*b*][1,4]oxazin-3(4*H*)-one (200 mg, 0.77 mmol), 1-(2-chloroethyl)pyrrolidine hydrochloride (262 mg, 1.54 mmol), and cesium carbonate (753 mg, 2.31 mmol) were added to acetonitrile (10 mL), and the mixture was stirred at 80°C for 0.5 hours. 2-Bromo-*N,N*-dimethylethylamine hydrochloride (360 mg, 1.54 mmol) was then added thereto, and the mixture was stirred at room temperature for 2 hours. The reaction mixture was filtered, concentrated, and separated by preparative thin-layer chromatography (dichloromethane/methanol = 10/1) to obtain the title compound (110 mg, 40%). LC-MS: m/z $[M+H]^+$ = 356, 358.

**17: 7-(5-(7-Ethyl-7*H*-imidazo[4,5-*c*]pyridazin-4-yl)-2-fluorophenyl)-6-methoxy-4-(2-(pyrrolidin-1-yl)ethyl)-2*H*-benzo[*b*][1,4]oxazin-3(4*H*)-one**

Following the same experimental procedure as in Example 1, 7-bromo-6-methoxy-4-(2-(pyrrolidin-1-yl) ethyl)-2*H*-benzo[*b*][1,4]oxazin-3(4*H*)-one (100 mg, 0.28 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (100 mg, 0.28 mmol) were used as starting materials to obtain the title compound (46 mg, 32%).

$^1$H NMR (400 MHz, CHLOROFORM-d) 9.35 (s, 1 H), 8.31 - 8.21 (m, 2 H), 8.19 (d, *J* = 6.4 Hz, 1 H), 7.32 (t, *J* = 8.8 Hz, 1 H), 7.05 (d, *J* = 6.4 Hz, 2 H), 4.62 - 4.53 (m, 4 H), 4.43 (br. s., 2 H), 3.93 (s, 3 H), 3.10 (br. s., 6 H), 2.03 (br. s., 4 H), 1.68 (t, *J* = 7.1 Hz, 3 H). LC-MS: m/z $[M+H]^+$ = 517.

Example 18

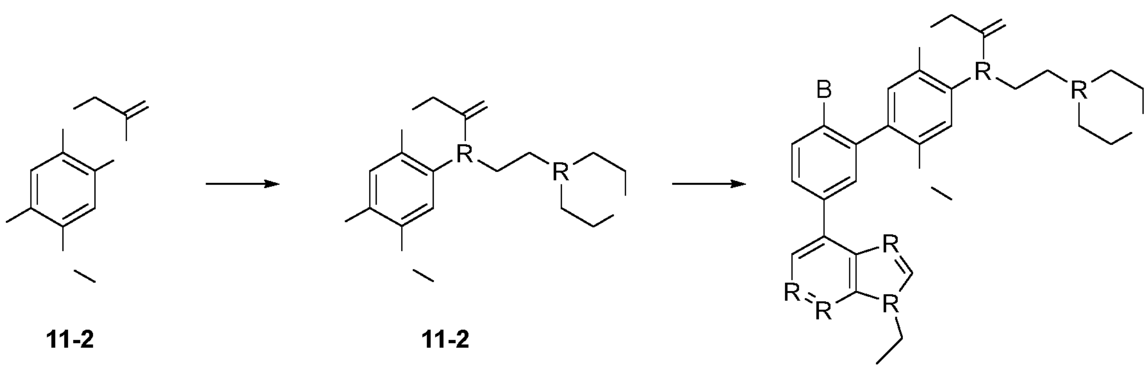

**11-2** **11-2**

**18-1: 7-Bromo-6-methoxy-4-(2-morpholinoethyl)-2*H*-benzo[*b*][1,4]oxazin-3(4H)-one**

7-Bromo-6-methoxy-2*H*-benzo[b][1,4]oxazin-3(4H)-one (200 mg, 0.77 mmol), cesium carbonate (752.6 mg, 2.31 mmol), and 4-(2-chloroethyl)morpholine (172.8 mg, 1.16 mmol) were added to acetonitrile (6 mL), and the mixture was stirred at 60°C overnight. The reaction mixture was directly purified by preparative thin-layer chromatography (dichloromethane/methanol = 20/1) to obtain the title compound (190 mg, 66%).

**18: 7-(5-(7-Ethyl-7*H*-imidazo[4,5-*c*]pyridazin-4-yl)-2-fluorophenyl)-6-methoxy-4-(2-morpholinoethyl)-2*H*-benzo [*b*][1,4]oxazin-3(4*H*)-one**

Following the same experimental procedure as in Example 7, 7-bromo-6-methoxy-4-(2-morpholinoethyl)-2*H*-benzo[*b*][1,4]oxazin-3(4*H*)-one (80 mg, 0.22 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (100 mg, 0.27 mmol) were used as starting materials to obtain the title compound (35 mg, 24%).

$^1$H NMR (400 MHz, CHLOROFORM-d) 9.36 (s, 1 H), 8.28 (s, 2 H), 8.19 (d, *J*=6.8 Hz, 1 H), 7.34 (t, *J* = *8.8* Hz, 1 H), 7.05 (s, 1 H), 6.78 (s, 1 H), 4.61 (s, 2 H), 4.60 - 4.55 (m, 2 H), 4.13 (t, *J* = *6.8* Hz, 2 H), 3.82 (s, 3 H), 3.73 (br. s., 4 H), 2.68 (t, *J* = 6.8 Hz, 2 H), 2.59 (br. s., 4 H), 1.69 (s, 3 H). LC-MS: m/z $[M+H]^+$ = 533.

Example 19

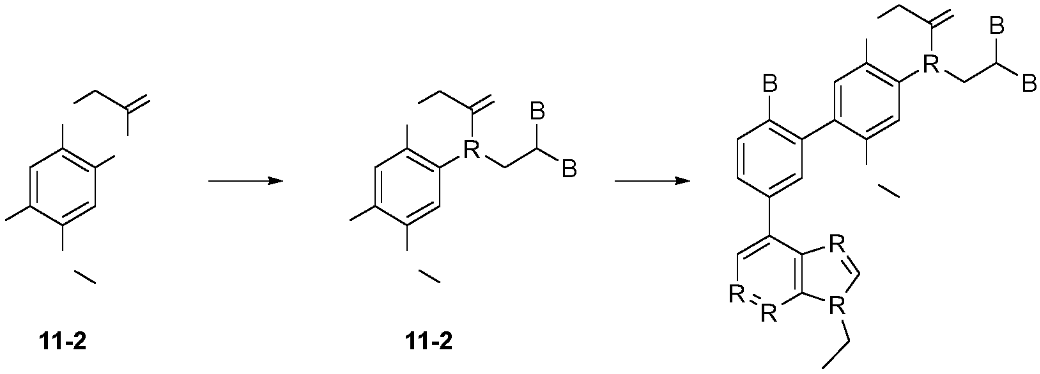

11-2 11-2

### 19-1: 7-Bromo-4-(2,2-difluoroethyl)-6-methoxy-2*H*-benzo[*b*][1,4]oxazin-3(4*H*)-one

7-Bromo-6-methoxy-2*H*-benzo[*b*][1,4]oxazin-3(4*H*)-one (100 mg, 0.39 mmol), cesium carbonate (381 mg, 1.17 mmol), and 2,2-difluoroethyl trifluoromethanesulfonate (36 mg, 0.09 mmol) were added to acetonitrile (6 mL), and the mixture was stirred at 60°C overnight. The reaction mixture was directly purified by preparative thin-layer chromatography (dichloromethane/methanol = 20/1) to obtain the title compound (90 mg, 72%).

### 19: 4-(2,2-Difluoroethyl)-7-(5-(7-ethyl-7*H*-imidazo[4,5-*c*]pyridazin-4-yl)-2-fluorophenyl)-6-methoxy-2*H*-benzo[*b*][1,4]oxazin-3(4*H*)-one

Following the same experimental procedure as in Example 1, 7-bromo-4-(2,2-difluoroethyl)-6-methoxy-2*H*-benzo[*b*][1,4]oxazin-3(4*H*)-one (92 mg, 0.29 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (90 mg, 0.24 mmol) were used as starting materials to obtain the title compound (70 mg, 63%).

$^1$H NMR (400 MHz, CHLOROFORM-d) 9.37 (s, 1 H), 8.28 (s, 2 H), 8.19 (d, *J*= 6.8 Hz, 1 H), 7.34 (t, *J* = 9.0 Hz, 1 H), 7.08 (s, 1 H), 6.79 (s, 1 H), 6.31 - 5.98 (m, 1 H), 4.66 (s, 2 H), 4.58 (q, *J* = 7.3 Hz, 2 H), 4.37 - 4.27 (m, 2 H), 3.83 (s, 3 H), 1.69 (t, *J* = 7.3 Hz, 3 H). LC-MS: m/z [M+H]$^+$ = 484.

Example 20

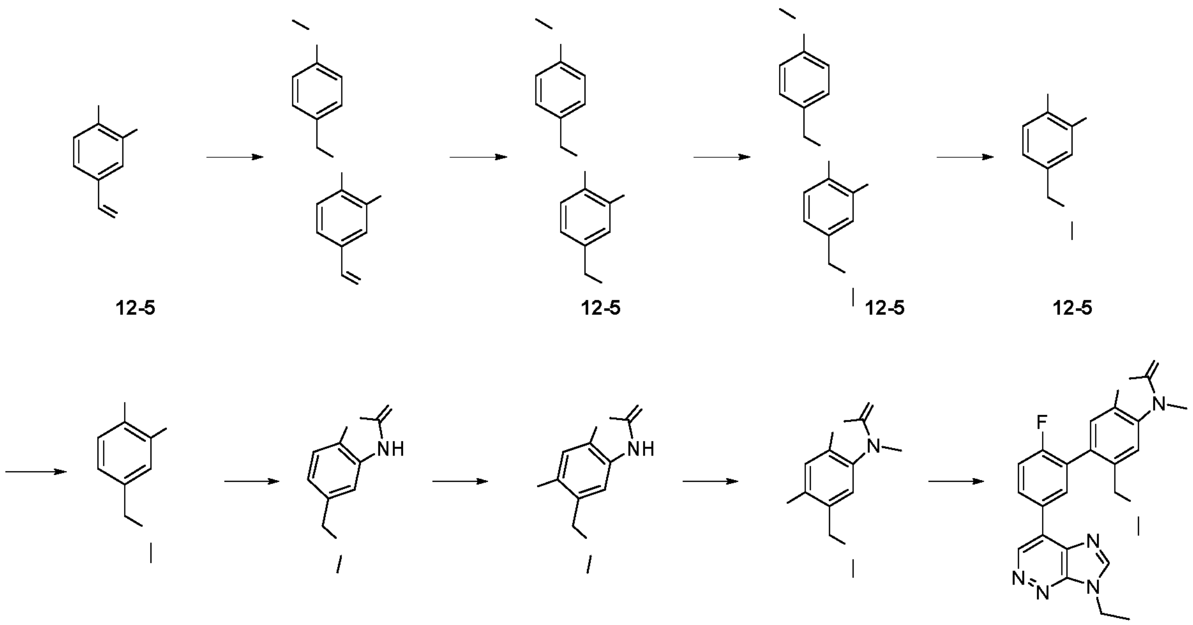

12-5 12-5 12-5 12-5

PPA

**20-1: 4-((4-Methoxybenzyl)oxy)-3-nitrobenzaldehyde**

4-Hydroxy-3-nitrobenzaldehyde (5.0 g, 29.92 mmol), 1-(chloromethyl)-4-methoxybenzene (7.03 g, 44.88 mmol), and cesium carbonate (19.50 g, 59.84 mmol) were added to *N,N*-dimethylformamide (100 mL), and the mixture was stirred at 65°C for 16 hours. The reaction mixture was poured into water (200 mL), extracted with ethyl acetate (100 mL × 3), respectively washed with 5% lithium chloride and saturated brine, dried over anhydrous sodium sulfate, and concentrated to obtain the title compound (7.0 g, yellow oil, yield: 81%).

$^1$H NMR (400 MHz, DMSO-$d_6$) 9.94 (s, 1 H), 8.42 (d, *J* = 2.0 Hz, 1 H), 8.18 (dd, *J* = 8.7, 2.1 Hz, 1 H), 7.67 (d, *J* = 8.8 Hz, 1 H), 7.45 - 7.37 (m, 2 H), 7.01 - 6.95 (m, 2 H), 5.35 (s, 2 H), 3.76 (s, 3 H).

**20-2: (4-((4-Methoxybenzyl)oxy)-3-nitrophenyl)methanol**

4-((4-Methoxybenzyl)oxy)-3-nitrobenzaldehyde (7.0 g, 24.37 mmol) was dissolved in methanol (150 mL), then sodium borohydride (4.63 g, 121.84 mmol) was slowly added thereto at room temperature, and the mixture was stirred at room temperature for 16 hours. The reaction mixture was poured into water (200 mL), extracted with ethyl acetate (100 mL × 3), washed with saturated brine, dried over anhydrous sodium sulfate, and concentrated to obtain the title compound (7.0 g, yellow oil, yield: 99%).

$^1$H NMR (400 MHz, $CDCl_3$) 7.84 (d, *J* = 2.1 Hz, 1 H), 7.49 (dd, *J* = 8.6, 2.1 Hz, 1 H), 7.37 (d, *J* = 8.7 Hz, 2H), 7.11 (d, *J* = 8.6 Hz, 1 H), 6.93 - 6.89 (m, 2 H), 5.17 (s, 2 H), 4.67 (s, 2 H), 3.81 (s, 3 H).

**20-3: 1-((4-Methoxybenzyl)oxy)-4-(methoxymethyl)-2-nitrobenzene**

(4-((4-Methoxybenzyl)oxy)-3-nitrophenyl)methanol (6.7 g, 23.16 mmol) and iodomethane (2.2 mL, 34.74 mmol) were dissolved in anhydrous N,N-dimethylformamide (134 mL), then 60% sodium hydride (1.39 g, 34.74 mmol) was slowly added thereto under a nitrogen atmosphere, and the mixture was stirred at room temperature for 16 hours. The reaction mixture was poured into saturated ammonium chloride (200 mL), extracted with ethyl acetate (100 mL × 3), respectively washed with 5% lithium chloride and saturated brine, dried over anhydrous sodium sulfate, and concentrated to obtain the title compound (6.8 g, yellow oil, yield: 97.1%).

$^1$H NMR (400 MHz, $CDCl_3$) 7.82 (d, *J* = 2.1 Hz, 1 H), 7.46 (dd, *J* = 8.6, 2.2 Hz, 1 H), 7.37 (d, *J* = 8.7 Hz, 2 H), 7.10 (d, *J* = 8.6 Hz, 1 H), 6.93 - 6.90 (m, 2 H), 5.17 (s, 2 H), 4.41 (s, 2 H), 3.81 (s, 3 H), 3.39 (s, 3 H).

**20-4: 4-(Methoxymethyl)-2-nitrophenol**

1-((4-Methoxybenzyl)oxy)-4-(methoxymethyl)-2-nitrobenzene (7.0 g, 23.08 mmol) was dissolved in dichloromethane (100 mL), then trifluoroacetic acid (10.0 mL) was added thereto, and the mixture was stirred at room temperature for 16 hours. The reaction mixture was poured into water (100 mL), extracted with dichloromethane (100 mL × 3), and washed with 10% potassium carbonate (50 mL × 5). The aqueous phase was collected, washed with ethyl acetate (50 mL × 3), added with concentrated hydrochloric acid to adjust the pH to 1, extracted with ethyl acetate (100 mL × 3), washed with saturated brine, dried over anhydrous sodium sulfate, and concentrated to obtain the title compound (3.4 g, yellow oil, yield: 81%).

$^1$H NMR (400 MHz, $CDCl_3$) 10.50 (s, 1 H), 8.01 (d, *J* = 2.0 Hz, 1 H), 7.50 (dd, *J* = 8.6, 2.1 Hz, 1 H), 7.09 (d, *J* = 8.6 Hz, 1 H), 4.36 (s, 2 H), 3.34 (s, 3 H).

**20-5: 2-Amino-4-(methoxymethyl)phenol**

4-(Methoxymethyl)-2-nitrophenol (3.5 g, 19.10 mmol) was dissolved in ethanol (70 mL), then platinum dioxide (350 mg) was added thereto, and the reaction mixture was stirred at room temperature for 16 hours under a hydrogen atmosphere (1 atm). After filtration, the filtrate was concentrated to obtain the title compound (2.1 g, brown solid, yield: 72%).

**20-6: 5-(Methoxymethyl)benzo[*d*]oxazol-2(3*H*)-one**

2-Amino-4-(methoxymethyl)phenol (2.1 g, 13.71 mmol) was dissolved in anhydrous dichloromethane (100.0 mL), then N,N-carbonyldiimidazole (2.45 g, 15.08 mmol) was added thereto in batches, and the mixture was stirred at room temperature for 16 hours. The reaction mixture was quenched with water (100 mL), then the phases were separated, and the organic phase was collected. The aqueous phase was then extracted with dichloromethane (100 mL × 3), and rotary evaporated to dryness to obtain a crude product, which was purified by normal-phase chromatography (dichloromethane: ethyl acetate) to obtain the title product (1.7 g, brown solid) with a yield of 69%. $^1$H NMR (400 MHz, DMSO-$d_6$) 7.23 (d, 1 H),

7.01 (m, 2 H), 4.40 (s, 2 H), 3.27 (s, 3 H). LCMS: m/z $[M+1]^+$ = 180.0.

**20-7: 6-Bromo-5-(methoxymethyl)benzo[*d*]oxazol-2(3*H*)-one**

5-(Methoxymethyl)benzo[d]oxazol-2(3H)-one (500 mg, 2.79 mmol) was dissolved in a solution of glacial acetic acid (12.5 mL), and (33%) hydrobromic acid/acetic acid (1.72 g, 6.98 mmol) was added thereto. The mixture was stirred at room temperature for 30 minutes, cooled to 10°C, and (30%) hydrogen peroxide (284.6 mg, 2.51 mmol) was slowly added dropwise thereto. The temperature was controlled at 10°C or less, and the mixture was reacted at 10°C to room temperature for 2 hours. The reaction mixture was poured into 100 mL of water, extracted with ethyl acetate (50 mL × 3), concentrated, and purified by prep-HPLC (0.5% HCOOH to acetonitrile) to obtain the title compound (415.0 mg, white solid, yield: 57.6%).

$^1H$ NMR (400 MHz, DMSO-$d_6$) 11.82 (s, 1 H), 7.63 (s, 1 H), 7.15 (s, 1 H), 4.43 (s, 2 H), 3.36 (s, 3 H).

**20-8: 6-Bromo-5-(methoxymethyl)-3-methylbenzo[*d*]oxazol-2(3*H*)-one**

6-Bromo-5-(methoxymethyl)benzo[*d*]oxazol-2(3*H*)-one (431 mg, 1.67 mmol) was suspended in a solution of dimethyl sulfoxide (10.0 mL), then iodomethane (3.1 mL, 50.1 mmol) and potassium carbonate (461.1 mg, 3.34 mmol) were added thereto at room temperature, and the mixture was reacted at room temperature for 16 hours. The reaction mixture was quenched with water (100 mL), extracted with ethyl acetate (50 mL × 3), washed once with saturated brine (50 mL), and rotary evaporated to dryness to obtain a crude product, which was purified by prep-HPLC to obtain the title product (267.7 mg, white solid) with a yield of 59%.

$^1H$ NMR (400 MHz, DMSO-$d_6$) 7.68 (s, 1 H), 7.34 (s, 1 H), 4.47 (s, 2 H), 3.39 (s, 3 H), 3.34 (s, 3 H).

**20: 6-(5-(7-Ethyl-7*H*-imidazo[4,5-*c*]pyridazin-4-yl)-2-fluorophenyl)-5-(methoxymethyl)-3-methylbenzo[*d*]oxazol-2(3*H*)-one**

Following the same experimental procedure as in Example 1, 6-bromo-5-(methoxymethyl)-3-methylbenzo[*d*]oxazol-2(3*H*)-one (100.0 mg, 0.37 mmol) and (2-fluoro-5-(7-ethyl-7*H*-imidazo[4,5-*c*]pyridazin-4-yl)phenyl)boronic acid (105.1 mg, 0.37 mmol) were used as starting materials to obtain the title compound (82 mg, white solid) with a yield of 52%.

$^1H$ NMR (400 MHz, DMSO-$d_6$) 9.57 (s, 1 H), 8.87 (s, 1 H), 8.53 (m, 1 H), 8.46 (m, 1 H), 7.56 (t, 1 H), 7.40 (m, 2 H), 4.51 (q, *J* = 7.3 Hz, 2 H), 4.33 (s, 2 H), 3.41 (s, 3 H), 3.19 (s, 3 H), 1.56 (t, *J* = 7.3 Hz, 3 H). LCMS: m/z $[M+1]^+$ = 434.

Example 21

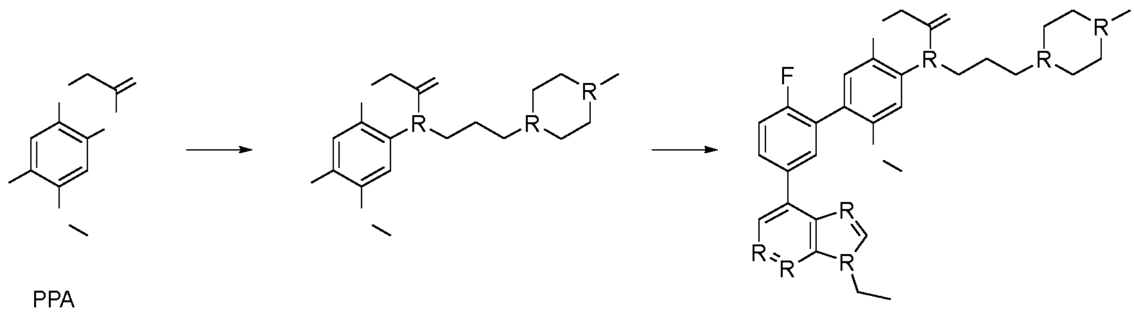

PPA

**21-1: 7-Bromo-6-methoxy-4-(3-(4-methylpiperazin-1-yl)propyl)-3,4-dihydro-2*H*-1,4-benzoxazin-3-one**

7-Bromo-6-methoxy-2*H*-benzo[*b*][1,4]oxazin-3(4*H*)-one (200 mg, 0.77 mmol), 1-(3-chloropropyl)-4-methylpiperazine hydrochloride (380 mg, 1.54 mmol), and cesium carbonate (750 mg, 2.31 mmol) were sequentially added to acetonitrile (5 mL), and the mixture was stirred at 80°C for 2 hours. The reaction mixture was filtered, concentrated, and separated by preparative thin-layer chromatography (dichloromethane/methanol = 10/1) to obtain the title compound (200 mg, 65%). LC-MS: m/z $[M+H]^+$ = 399, 401.

**21: 7-(5-(7-Ethyl-7*H*-imidazo[4,5-c]pyridazin-4-yl)-2-fluorophenyl)-6-methoxy-4-(3-(4-methylpiperazin-1-yl)propyl)-3,4-dihydro-2*H*-1,4-benzoxazin-3-one**

Following the same experimental procedure as in Example 7, 7-bromo-4-(2-(dimethylamino)ethyl)-6-methoxy-2*H*-benzo[*b*][1,4]oxazin-3(4*H*)-one (150 mg, 0.38 mmol) and 7-ethyl-4-(4-fluoro-3-(4,4,5,5-tetramethyl-1,3,2-dioxaborolan-2-yl)phenyl)-7H-imidazo[4,5-*c*]pyridazine (140 mg, 0.38 mmol) were used as starting materials to obtain the title compound (120 mg, 56%).

$^1$H NMR (400 MHz, CHLOROFORM-d) 9.36 (s, 1 H), 8.29 (s, 1 H), 8.28 - 8.22 (m, 1 H), 8.19 (d, *J* = 5.4 Hz, 1 H), 7.33 (t, *J* = 9.0 Hz, 1 H), 7.04 (s, 1 H), 6.70 (s, 1 H), 4.62 - 4.54 (m, 4 H), 4.06 (t, *J* = 7.1 Hz, 2 H), 3.81 (s, 3 H), 2.60 (br. s., 8 H), 2.51 (t, *J* = 6.8 Hz, 2 H), 2.38 (s, 3 H), 1.98 - 1.89 (m, 2 H), 1.69 (t, *J* = 7.3 Hz, 3 H). LC-MS: m/z [M+H]$^+$ = 560.

Example 22

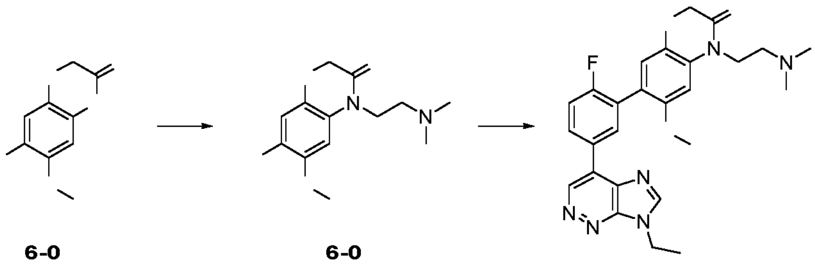

6-0 6-0

**22-1: 7-Bromo-4-(2-(dimethylamino)ethyl)-6-methoxy-2*H*-benzo[*b*][1,4]oxazin-3(4*H*)-one**

7-Bromo-6-methoxy-2*H*-benzo[b][1,4]oxazin-3(4*H*)-one (200 mg, 0.77 mmol) was added to tetrahydrofuran (10 mL), then sodium hydride (92 mg, 3.85 mmol) was added thereto in an ice bath, and the mixture was stirred at room temperature for 0.5 hours. 2-Bromo-*N,N*-dimethylethylamine hydrochloride (360 mg, 1.54 mmol) was then added thereto, and the mixture was stirred at room temperature for 2 hours. The reaction mixture was filtered, concentrated, and separated by preparative thin-layer chromatography (ethyl acetate) to obtain the title compound (120 mg, 47%). LC-MS: m/z [M+H]$^+$ = 330, 332.

**22: 4-(2-(Dimethylamino)ethyl)-7-(5-(7-ethyl-7*H*-imidazo[4,5-*c*]pyridazin-4-yl)-2-fluorophenyl)-6-methoxy-2*H*-benzo[*b*][1,4]oxazin-3(4*H*)-one**

Following the same experimental procedure as in Example 7, 7-bromo-4-(2-(dimethylamino)ethyl)-6-methoxy-2*H*-benzo[*b*][1,4]oxazin-3(4*H*)-one (120 mg, 0.36 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (133 mg, 0.36 mmol) were used as starting materials to obtain the title compound (24 mg, 14%) as a white solid.

$^1$H NMR (400 MHz, CHLOROFORM-d) 9.37 (s, 1 H), 8.28 (s, 2 H), 8.20 (d, *J* = 6.4 Hz, 1 H), 7.34 (t, *J* = 8.8 Hz, 1 H), 7.05 (s, 1 H), 6.93 (br. s., 1 H), 4.62 - 4.56 (m, 4 H), 4.23 (br. s., 2 H), 3.87 (s, 3 H), 2.77 (br. s., 2 H), 2.50 (br. s., 6 H), 1.70 (t, *J* = 7.1 Hz, 3 H). LC-MS: m/z [M+H]$^+$ = 491.

Example 23

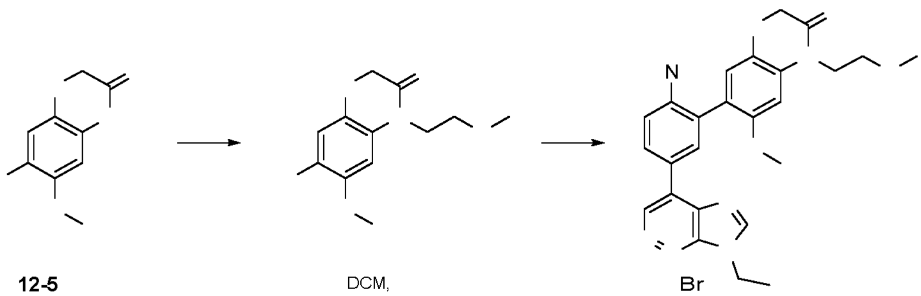

12-5

DCM,

Br

**23-1: 7-Bromo-6-methoxy-4-(2-methoxyethyl)-2*H*-benzo[*b*][1,4]oxazin-3(4*H*)-one**

7-Bromo-6-methoxy-2*H*-benzo[*b*][1,4]oxazin-3(4*H*)-one (300 mg, 1.16 mmol), 1-bromo-2-methoxyethane (322 mg, 2.32 mmol), and potassium carbonate (481 mg, 3.48 mmol) were sequentially added to methanol (5 mL), and the mixture was stirred at 45°C for 16 hours. The reaction mixture was filtered, concentrated, and separated by preparative thin-layer chromatography (petroleum ether/ethyl acetate = 3/1) to obtain the title compound (40 mg, 11%) as a yellow solid. LC-MS: m/z $[M+H]^+$ = 317, 319.

**23: 7-(5-(7-Ethyl-7*H*-imidazo[4,5-*c*]pyridazin-4-yl)-2-fluorophenyl)-6-methoxy-4-(2-methoxyethyl)-2*H*-benzo[*b*][1,4]oxazin-3(4*H*)-one**

Following the same experimental procedure as in Example 7, 7-bromo-6-methoxy-4-(2-methoxyethyl)-2*H*-benzo[*b*][1,4]oxazin-3(4H)-one (60 mg, 0.22 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (81 mg, 0.22 mmol) were used as starting materials to obtain the title compound (10 mg, 10%) as a yellow solid.

$^1H$ NMR (400 MHz, CHLOROFORM-*d*) 9.40 (br. s., 1 H), 8.34 - 8.22 (m, 2 H), 8.18 (d, *J* = 6.4 Hz, 1 H), 7.33 (t, *J* = 8.8 Hz, 1 H), 7.05 (s, 1 H), 6.54 (s, 1 H), 4.62 - 4.52 (m, 3 H), 4.50 (s, 2 H), 3.80 (s, 3 H), 2.62 (br. s., 2 H), 2.55 - 2.42 (m, 2 H), 1.86 (d, *J* = 8.3 Hz, 2 H), 1.69 (t, *J* = 7.3 Hz, 3 H). LC-MS: m/z $[M+H]^+$ = 474.

Example 24

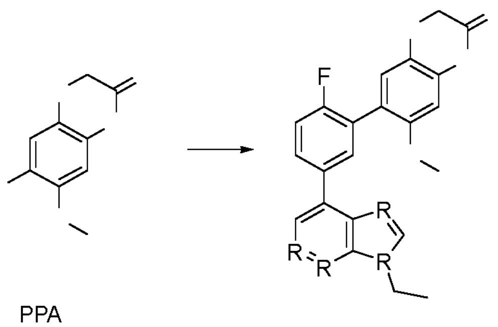


**24: 7-(5-(7-Ethyl-7*H*-imidazo[4,5-*c*]pyridazin-4-yl)-2-fluorophenyl)-6-methoxy-2*H*-benzo[*b*][1,4]oxazin-3(4*H*)-one**

Following the same experimental procedure as in Example 7, 7-bromo-6-methoxy-2H-benzo[*b*][1,4]oxazin-3(4*H*)-one (100 mg, 0.39 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (144 mg, 0.39 mmol) were used as starting materials to obtain the title compound (68 mg, 42%).

$^1H$ NMR (400 MHz, CHLOROFORM-*d*) 10.76 (s, 1 H), 9.50 (s, 1 H), 8.83 (s, 1 H), 8.41 - 8.35 (m, 2 H), 7.43 (s, 1 H), 6.95 (s, 1 H), 6.66 (s, 1 H), 4.54 (s, 2 H), 4.51 - 4.43 (m, 2 H), 3.67 (s, 3 H), 1.51 (t, *J* = 7.3 Hz, 3 H). LC-MS: m/z $[M+H]^+$ = 420.

Example 25

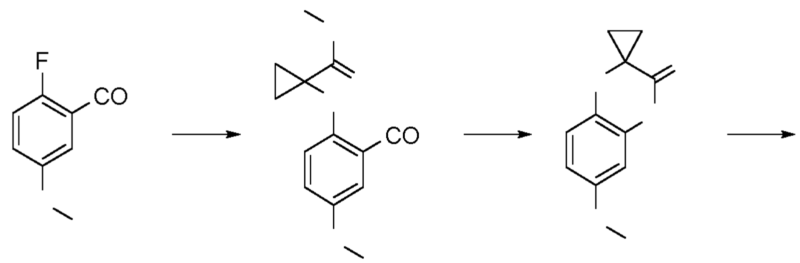

**10-3**

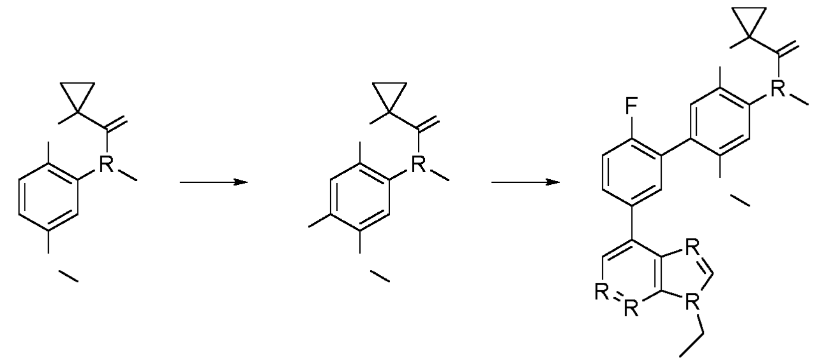

**25-1: Methyl 1-(4-methoxy-2-nitrophenoxy)cyclopropane-1-carboxylate**

1-Fluoro-4-methoxy-2-nitrobenzene (1 g, 5.85 mmol), sodium hydride (420 mg, 17.52 mmol), and methyl 1-hydroxycyclopropane-1-carboxylate (1.06 g, 8.76 mmol) were added to tetrahydrofuran (10 mL), and the mixture was stirred at 0°C for 2 hours. The reaction mixture was concentrated and purified by preparative thin-layer chromatography (petroleum ether/ethyl acetate = 3/1) to obtain the title compound (1.1 g, 71%).

**25-2: 6-Methoxyspiro[benzo[*b*][1,4]oxazine-2,1'-cyclopropan]-3(4*H*)-one**

Methyl 1-(4-methoxy-2-nitrophenoxy)cyclopropane-1-carboxylate (1 g, 3.74 mmol) and Fe (1.04 g, 18.7 mmol) were added to acetic acid (6 mL), and the mixture was stirred at 80°C for 1 hour. The reaction mixture was concentrated, added with an appropriate amount of methanol, and purified by preparative thin-layer chromatography (petroleum ether/ethyl acetate = 1/1) to obtain the title compound (550 mg, 71%).

**25-3: 6-Methoxy-4-methylspiro[benzo[*b*][1,4]oxazine-2,1'-cyclopropan]-3(4*H*)-one**

6-Methoxyspiro[benzo[*b*][1,4]oxazine-2,1'-cyclopropan]-3(4*H*)-one (550 mg, 2.65 mmol), cesium carbonate (2.59 g, 7.95 mmol), and iodomethane (2.59 mg, 7.95 mmol) were added to methanol (8 mL), and the mixture was stirred at room temperature overnight. The reaction mixture was filtered and concentrated, and the mother liquor was purified by preparative thin-layer chromatography (petroleum ether/ethyl acetate = 1/1) to obtain the title compound (400 mg, 68.22%).

**25-4: 7-Bromo-6-methoxy-4-methylspiro[benzo[*b*][1,4]oxazine-2,1'-cyclopropan]-3(4*H*)-one**

6-Methoxy-4-methylspiro[benzo[*b*][1,4]oxazine-2,1'-cyclopropan]-3(4*H*)-one (200 mg, 0.9 mmol), HBr (0.3 mL), and hydrogen peroxide (0.1 mL) were added to acetic acid (10 mL), and the mixture was stirred at room temperature for 1 hour. A large amount of water was added to the reaction mixture, and the product was precipitated from the reaction system. The reaction mixture was filtered and concentrated to obtain the title compound (110 mg, 41%).

**25: 7-(5-(7-Ethyl-7*H*-imidazo[4,5-*c*]pyridazin-4-yl)-2-fluorophenyl)-6-methoxy-4-methylspiro[benzo[*b*][1,4]oxazine-2,1'-cyclopropan]-3(4*H*)-one**

Following the same experimental procedure as in Example 1, 7-bromo-6-methoxy-4-methylspiro[benzo[*b*][1,4]oxazine-2,1'-cyclopropan]-3(4*H*)-one (97 mg, 0.32 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (100 mg, 0.27 mmol) were used as starting materials to obtain the title compound (57 mg, 46%).

$^1$H NMR (400 MHz, CHLOROFORM-d) 9.36 (br. s., 1 H), 8.31 - 8.23 (m, 2 H), 8.18 (d, *J* = 6.8 Hz, 1 H), 7.33 (t, *J* = 9.0 Hz, 1 H), 6.94 (s, 1 H), 6.64 (s, 1 H), 4.58 (q, *J* = 7.3 Hz, 2 H), 3.85 (s, 3 H), 3.49 - 3.41 (m, 3 H), 1.73 - 1.68 (m, 3 H), 1.45 - 1.38 (m, 2 H), 1.25 - 1.21 (m, 2 H). LCMS: m/z [M+H]$^+$ = 460.

Example 26

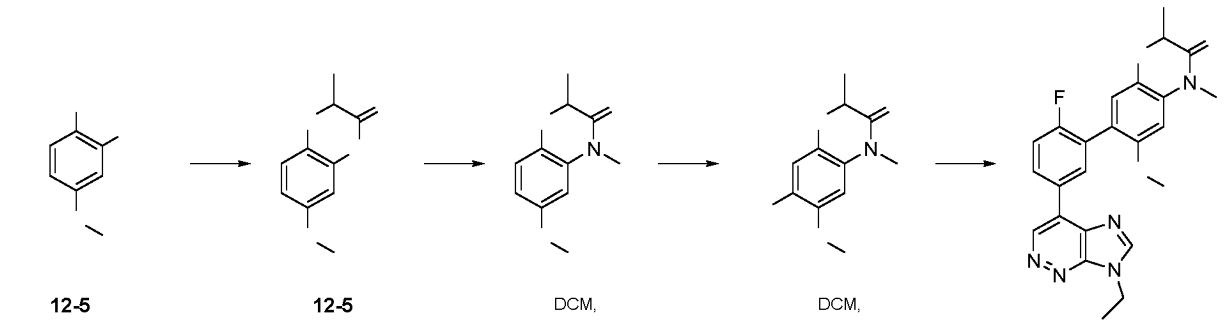


**26-1: 6-Methoxy-2-methyl-2*H*-benzo[*b*][1,4]oxazin-3(4*H*)-one**

2-Amino-4-methoxyphenol (500 mg, 3.59 mmol), cesium carbonate (2.34 g, 7.18 mmol), and ethyl 2-bromopropionate (650 mg, 3.59 mmol) were added to *N,N*-dimethylformamide (8 mL), and the mixture was stirred at 95°C overnight. The reaction mixture was extracted three times with ethyl acetate and water. The organic phase was concentrated and directly purified by preparative thin-layer chromatography (petroleum ether/ethyl acetate = 1/1) to obtain the title compound (500 mg, 72.09%).

**26-2: 6-Methoxy-2,4-dimethyl-2H-benzo[b][1,4]oxazin-3(4H)-one**

6-Methoxy-2-methyl-2*H*-benzo[*b*][1,4]oxazin-3(4*H*)-one (500 mg, 2.59 mmol), cesium carbonate (2.53 g, 7.77 mmol), and iodomethane (1.85 mg, 12.95 mmol) were added to methanol (20 mL), and the mixture was stirred at room temperature overnight. The reaction mixture was directly purified by preparative thin-layer chromatography (dichloromethane/methanol = 30/1) to obtain the title compound (400 mg, 74.6%).

**26-3: 7-Bromo-6-methoxy-2,4-dimethyl-2*H*-benzo[*b*][1,4]oxazin-3(4*H*)-one**

6-Methoxy-2,4-dimethyl-2*H*-benzo[*b*][1,4]oxazin-3(4*H*)-one (400 mg, 1.93 mmol), HBr (0.5 mL), and hydrogen peroxide (0.2 mL) were added to acetic acid (20 mL), and the mixture was stirred at room temperature for 1 hour. A large amount of water was added to the reaction mixture, and the product was precipitated from the reaction system. The reaction mixture was filtered and concentrated to obtain the title compound (190 mg, 34%).

**26: 7-(5-(7-Ethyl-7*H*-imidazo[4,5-*c*]pyridazin-4-yl)-2-fluorophenyl)-6-methoxy-2,4-dimethyl-2H-benzo[*b*][1,4]oxazin-3(4*H*)-one**

Following the same experimental procedure as in Example 1, 2-(5-bromo-6-ethoxypyridin-2-yl)-5-methyl-1,3,4-oxadiazole (92 mg, 0.32 mmol) and 7-ethyl-4-(4-fluoro-3-(4,4,5,5-tetramethyl-1,3,2-dioxaborolan-2-yl)phenyl)-7*H*-imidazo[4,5-c]pyridazine (100 mg, 0.27 mmol) were used as starting materials to obtain the title compound (60 mg, 50%).

$^1$H NMR (400 MHz, CHLOROFORM-d) 9.37 (s, 1 H), 8.30 - 8.25 (m, 2 H), 8.19 (d, *J* = 6.8 Hz, 1 H), 7.34 (t, *J* = 9.0 Hz, 1 H), 7.05 (s, 1 H), 6.63 (s, 1 H), 4.67 - 4.62 (m, 1 H), 4.59 (d, *J* = 7.3 Hz, 2 H), 3.84 (s, 3 H), 3.44 (s, 3 H), 1.72 - 1.69 (m, 3 H), 1.60 (d, *J* = 6.8 Hz, 3 H). LC-MS: m/z $[M+H]^+$ = 448.

Example 27

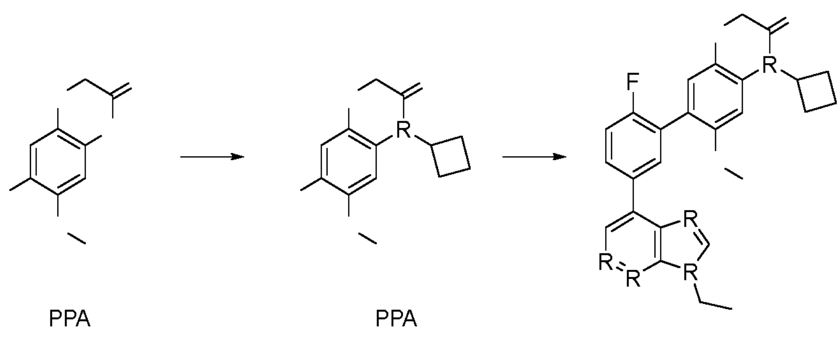

**27-1: 7-Bromo-4-cyclobutyl-6-methoxy-2*H*-benzo[*b*][1,4]oxazin-3(4*H*)-one**

7-Bromo-6-methoxy-2H-benzo[*b*][1,4]oxazin-3(4*H*)-one (300 mg, 1.16 mmol), cyclobutyl bromide (235 mg, 1.74 mmol), and potassium carbonate (481 mg, 3.48 mmol) were sequentially added to *N,N*-dimethylformamide (5 mL), and the mixture was stirred at 80°C for 2 hours. The reaction mixture was filtered, concentrated, and separated by preparative thin-layer chromatography (petroleum ether/ethyl acetate = 3/1) to obtain the title compound (110 mg, 30%) as a white solid. LCMS: m/z $[M+H]^+$ = 313, 315.

**27: 4-Cyclobutyl-7-(5-(7-ethyl-7*H*-imidazo[4,5-*c*]pyridazin-4-yl)-2-fluorophenyl)-6-methoxy-2*H*-benzo[*b*][1,4]oxazin-3(4*H*)-one**

Following the same experimental procedure as in Example 1, 7-bromo-4-cyclobutyl-6-methoxy-2*H*-benzo[*b*][1,4]oxazin-3(4*H*)-one (100 mg, 0.32 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (117 mg, 0.32 mmol) were used as starting materials to obtain the title compound (17 mg, 11%) as a yellow solid.

$^1H$ NMR (400 MHz, CHLOROFORM-d) δ = 9.40 (br. s., 1 H), 8.34 - 8.22 (m, 2 H), 8.18 (d, *J* = 6.4 Hz, 1 H), 7.33 (t, *J* = 8.8 Hz, 1 H), 7.05 (s, 1 H), 6.54 (s, 1 H), 4.62 - 4.52 (m, 3 H), 4.50 (s, 2 H), 3.80 (s, 3 H), 2.62 (br. s., 2 H), 2.55 - 2.42 (m, 2 H), 1.86 (d, *J* = 8.3 Hz, 2 H), 1.69 (t, *J* = 7.3 Hz, 3 H). LCMS: m/z $[M+H]^+$ = 474.

Example 28

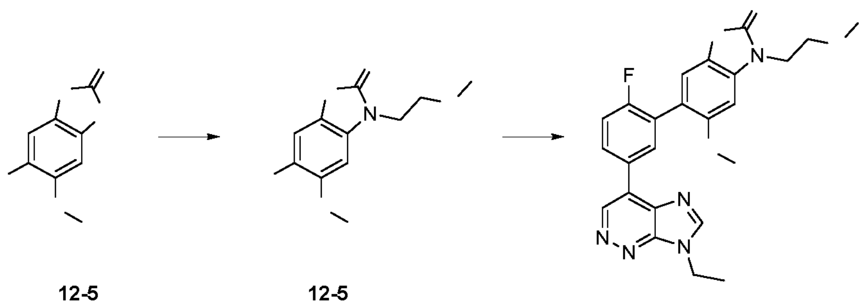

**12-5** **12-5**

**28-1: 6-Bromo-5-methoxy-3-(2-methoxyethyl)benzo[d]oxazol-2(3H)-one**

6-Bromo-5-methoxybenzo[*d*]oxazol-2(3*H*)-one (200 mg, 0.82 mmol) was added to *N,N*-dimethylformamide (10 mL), then potassium carbonate (113 mg, 0.82 mmol) and 1-bromo-2-methoxyethane (0.77 mL, 0.82 mmol) were added thereto, and the mixture was stirred at 80°C for 5 hours. The reaction mixture was poured into water, extracted with ethyl acetate (100 mL × 3), concentrated, and purified by column chromatography (petroleum ether: ethyl acetate = 2:1) to obtain the title compound (70 mg, yield: 57%) as a white solid. LCMS: m/z $[M+H]^+$ = 302.

**28: 6-(5-(7-Ethyl-7*H*-imidazo[4,5-*c*]pyridazin-4-yl)-2-fluorophenyl)-5-methoxy-3-(2-methoxyethyl)benzo[*d*]oxazol-2(3*H*)-one**

Following the same experimental procedure as in Example 1, 6-bromo-5-methoxy-3-(2-methoxyethyl)benzo[*d*]oxazol-2(3H)-one (70 mg, 0.23 mmol) and 7-ethyl-4-(4-fluoro-3-(4,4,5,5-tetramethyl-1,3,2-dioxaborolan-2-yl)phenyl)-7*H*-imidazo[4,5-c]pyridazine (66.28 mg, 0.23 mmol) were used as starting materials to obtain the title compound (43 mg, yield: 40%).

$^1H$ NMR (400 MHz, DMSO-$d_6$) 9.56 (s, 1 H), 8.86 (s, 1 H), 8.45 (m, 2 H), 7.47 (m, 1 H), 7.37 - 7.32 (m, 1 H), 7.24 (s, 1 H), 4.52 (m, 2 H), 4.08 (m, 2 H), 3.81 (s, 3 H), 3.71 (m, 2 H), 1.56 (t, *J* = 7.1 Hz, 3 H). LC-MS: m/z $[M+H]^+$ = 464.2.

Example 29

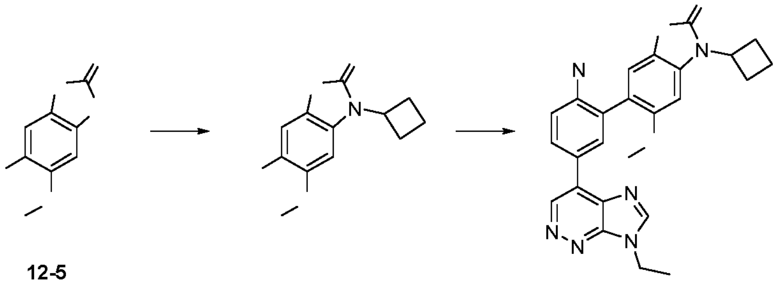

12-5

29-1: 6-Bromo-3-cyclobutyl-5-methoxybenzo[*d*]oxazol-2(3*H*)-one

6-Bromo-5-methoxybenzo[*d*]oxazol-2(3*H*)-one (100 mg, 0.41 mmol) was added to *N,N*-dimethylformamide (10 mL), then bromocyclobutane (1.11 g, 8.19 mmol) and potassium carbonate (113.27 mg, 0.82 mmol) were added thereto, and the mixture was heated to 80°C and stirred for 5 hours. The reaction mixture was poured into water, extracted with ethyl acetate (50 mL × 3), concentrated, and purified by column chromatography (petroleum ether: ethyl acetate = 2:1) to obtain the title compound (130 mg) as a pink solid. LC-MS: m/z $[M+H]^+$ = 298.

**29: 3-Cyclobutyl-6-(5-(7-ethyl-7*H*-imidazo[4,5-*c*]pyridazin-4-yl)-2-fluorophenyl)-5-methoxybenzo[*d*]oxazol-2(3*H*)-one**

Following the same experimental procedure as in Example 1, 6-bromo-3-cyclobutyl-5-methoxybenzo[*d*]oxazol-2(3*H*)-one (130 mg, 0.44 mmol) and 5-(7-ethyl-7*H*-imidazo[4,5-*c*]pyridazin-4-yl)-2-fluorophenyl)boronic acid (124.74 mg, 0.44 mmol) were used as starting materials to obtain the title compound (45 mg, yield: 22%).

$^1$H NMR (400 MHz, DMSO-$d_6$) 9.59 (s, 1 H), 8.91 (s, 1 H), 8.56 - 8.35 (m, 2 H), 7.50 (t, 1 H), 7.39 (s, 1 H), 7.22 (s, 1 H), 4.95 - 4.74 (m, 1 H), 4.52 (q, *J* = 7.3 Hz, 2 H), 3.84 (s, 3 H), 2.84 (m, 2 H), 2.42 - 2.30 (m, 2 H), 1.99 - 1.75 (m, 2 H), 1.56 (t, *J* = 7.2 Hz, 3 H). LC-MS: m/z $[M+H]^+$ = 460.

Example 30

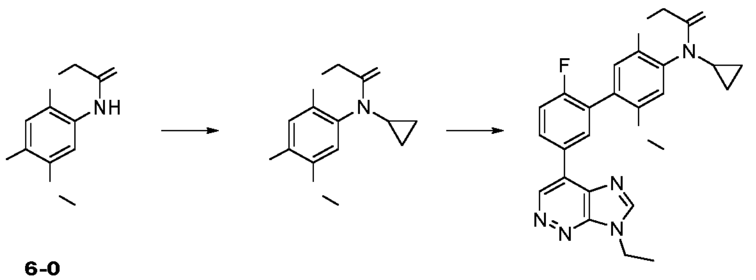

6-0

**30-1: 7-Bromo-4-cyclopropyl-6-methoxy-2*H*-benzo[*b*][1,4]oxazin-3(4*H*)-one**

7-Bromo-6-methoxy-2*H*-benzo[*b*][1,4]oxazin-3(4*H*)-one (500 mg, 1.94 mmol), copper acetate (352 mg, 1.94 mmol), cesium carbonate (316 mg, 0.97 mmol), cyclopropylboronic acid (330 mg, 3.88 mmol), and bipyridine (460 mg, 5.82 mmol) were sequentially added to toluene (10 mL), and the mixture was stirred at 110°C for 2 hours. The reaction mixture was filtered, concentrated, and separated by preparative thin-layer chromatography (petroleum ether/ethyl acetate = 3/1) to obtain the title compound (190 mg, 33%). LC-MS: m/z $[M+H]^+$ = 299, 231.

**30: 4-Cyclopropyl-7-(5-(7-ethyl-7*H*-imidazo[4,5-*c*]pyridazin-4-yl)-2-fluorophenyl)-6-methoxy-2*H*-benzo[*b*][1,4]oxazin-3(4*H*)-one**

Following the same experimental procedure as in Example 7, 7-bromo-4-cyclopropyl-6-methoxy-2*H*-benzo[*b*][1,4]oxazin-3(4*H*)-one (190 mg, 0.70 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (258 mg, 0.70 mmol) were used as starting materials to obtain the title compound (99 mg, 33%) as a white solid.

$^1$H NMR (400 MHz, CHLOROFORM-d) 9.38 (s, 1 H), 8.31 - 8.22 (m, 2 H), 8.18 (d, *J* = 6.4 Hz, 1 H), 7.33 (t, *J* = 9.0 Hz, 1 H), 7.00 (br. s., 2 H), 4.63 - 4.51 (m, 4 H), 3.83 (s, 3 H), 2.80 (br. s., 1 H), 1.69 (t, *J* = 7.1 Hz, 3 H), 1.26 - 1.19 (m, 4 H). LC-MS: m/z [M+H]$^+$ = 434.

Example 31

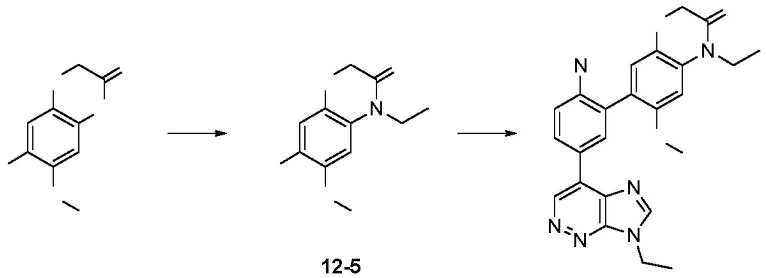

12-5

**31-1: 7-Bromo-4-ethyl-6-methoxy-2*H*-benzo[*b*][1,4]oxazin-3(4*H*)-one**

7-Bromo-6-methoxy-2*H*-benzo[*b*][1,4]oxazin-3(4*H*)-one (500 mg, 1.94 mmol), iodoethane (454 mg, 2.91 mmol), and potassium carbonate (804 mg, 5.82 mmol) were sequentially added to methanol (5 mL), and the mixture was stirred at 45°C for 16 hours. The reaction mixture was filtered, concentrated, and separated by preparative thin-layer chromatography (petroleum ether/ethyl acetate = 3/1) to obtain the title compound (200 mg, 36%). LC-MS: m/z [M+H]$^+$ = 287, 289.

**31: 4-Ethyl-7-(5-(7-ethyl-7*H*-imidazo[4,5-*c*]pyridazin-4-yl)-2-fluorophenyl)-6-methoxy-2*H*-benzo[*b*][1,4]oxazin-3(4*H*)-one**

Following the same experimental procedure as in Example 7, 7-bromo-6-methoxy-4-(2-methoxyethyl)-2H-benzo[*b*][1,4]oxazin-3(4*H*)-one (200 mg, 0.70 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (258 mg, 0.70 mmol) were used as starting materials to obtain the title compound (72 mg, 23%) as a yellow solid.

$^1$H NMR (400 MHz, CHLOROFORM-*d*) 9.34 (s, 1 H), 8.31 - 8.20 (m, 2 H), 8.17 (d, *J* = 6.8 Hz, 1 H), 7.31 (t, *J* = 8.8 Hz, 1 H), 7.02 (s, 1 H), 6.64 (s, 1 H), 4.61 - 4.50 (m, 4 H), 4.02 (q, *J* = 6.8 Hz, 2 H), 3.81 (s, 3 H), 1.66 (t, *J* = 7.3 Hz, 3 H), 1.33 (t, *J* = 6.8 Hz, 3 H). LC-MS: m/z [M+H]$^+$ = 448.

Example 32

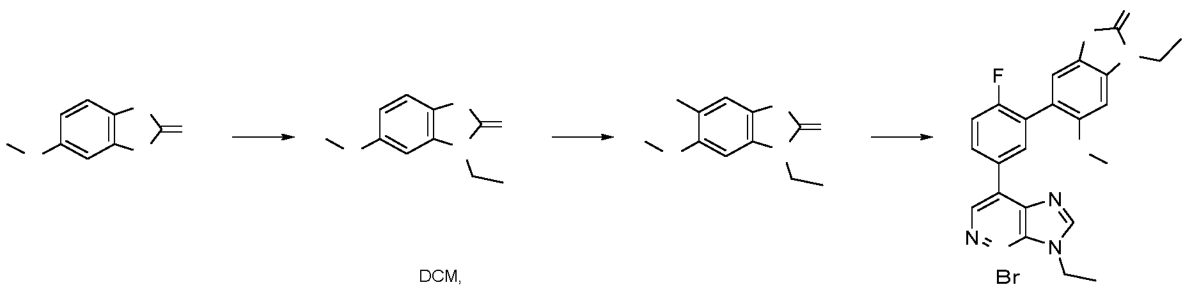


**32-1: 3-Ethyl-5-methoxybenzo[*d*]thiazol-2(3*H*)-one**

5-Methoxy-2,3-dihydro-1,3-benzothiazol-2-one (CAS: 15193-51-8, 300 mg, 1.7 mmol) was dissolved in 10 mL of methanol, then iodoethane (1.3 g, 8.3 mmol) and potassium carbonate (0.5 g, 3.3 mmol) were added thereto, and the mixture was heated to 40°C and stirred for 2 hours. The reaction mixture was filtered to remove potassium carbonate, concentrated, and purified by preparative thin-layer chromatography (petroleum ether: ethyl acetate = 3:1) to obtain the title compound (200 mg, 57%) as a yellow solid. LC-MS: m/z [M+H]$^+$ = 210.

**32-2: 6-Bromo-3-ethyl-5-methoxybenzo[*d*]thiazol-2(3*H*)-one**

3-Ethyl-5-methoxybenzo[*d*]thiazol-2(3*H*)-one (200 mg, 0.96 mmol) was dissolved in 2 mL of acetic acid, then a solution of 33% hydrobromic acid in acetic acid (588 mg, 2.4 mmol) was added thereto, and the mixture was stirred at room temperature for 30 minutes. The reaction mixture was then cooled to 0°C, added with hydrogen peroxide (163 mg, 1.44 mmol), warmed to room temperature, and stirred for 2 hours. Water was added thereto, and a large amount of solid was precipitated. The reaction mixture was filtered, dried, concentrated, and purified by preparative thin-layer chromatography (petroleum ether: ethyl acetate = 3:1) to obtain the title compound (100 mg, 36%) as a white solid. LC-MS: m/z $[M+H]^+$ = 288.

**32: 3-Ethyl-6-(5-(7-ethyl-7*H*-imidazo[4,5-*c*]pyridazin-4-yl)-2-fluorophenyl)-5-methoxybenzo[*d*]thiazol-2(3*H*)-one**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (120 mg, 0.33 mmol) and 6-bromo-3-ethyl-5-methoxybenzo[*d*]thiazol-2(3H)-one (100 mg, 0.33 mmol) were used as starting materials to obtain the title compound (16 mg, 11%) as a white solid.

$^1$H NMR (400 MHz, CHLOROFORM-d) 9.37 (br. s., 1 H) 8.18 - 8.34 (m, 3 H) 7.41 (s, 1 H) 7.34 (t, *J* = 8.80 Hz, 1 H) 6.72 (s, 1 H) 4.59 (q, *J* = 6.85 Hz, 2 H) 4.03 - 4.12 (m, 2 H) 3.89 (s, 3 H) 1.67 - 1.70 (m, 3 H) 1.41 (t, *J* = 6.85 Hz, 3 H). LC-MS: m/z $[M+H]^+$ = 450.

Example 33

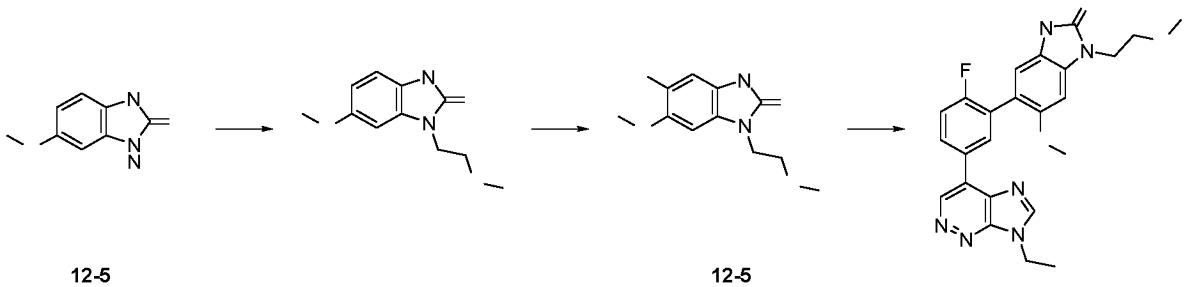

12-5 12-5

**33-1: 5-Methoxy-3-(2-methoxyethyl)benzo[d]thiazol-2(3H)-one**

5-Methoxy-2,3-dihydro-1,3-benzothiazol-2-one (CAS: 15193-51-8, 300 mg, 1.7 mmol) was dissolved in 10 mL of acetonitrile, then 1-bromo-2-methoxyethane (346 mg, 2.5 mmol) and cesium carbonate (1.1 g, 03.3 mmol) were added thereto, and the mixture was heated to 60°C and stirred overnight. The reaction mixture was filtered to remove cesium carbonate, concentrated, and purified by preparative thin-layer chromatography (petroleum ether: ethyl acetate = 3:1) to obtain the title compound (200 mg, 50%) as a yellow solid. LC-MS: m/z $[M+H]^+$ = 241.

**33-2: 6-Bromo-5-methoxy-3-(2-methoxyethyl)benzo[d]thiazol-2(3H)-one**

5-Methoxy-3-(2-methoxyethyl)benzo[*d*]thiazol-2(3*H*)-one (200 mg, 0.96 mmol) was dissolved in 2 mL of acetic acid, then a solution of 33% hydrobromic acid in acetic acid (588 mg, 2.4 mmol) was added thereto, and the mixture was stirred at room temperature for 30 minutes. The reaction mixture was then cooled to 0°C, added with hydrogen peroxide (163 mg, 1.44 mmol), warmed to room temperature, and stirred for 2 hours. Water was added thereto, and a large amount of solid was precipitated. The reaction mixture was filtered, dried, concentrated, and purified by preparative thin-layer chromatography (petroleum ether: ethyl acetate = 2:1) to obtain the title compound (120 mg, 39%) as a white solid. LC-MS: m/z $[M+H]^+$ = 318.

**33: 6-(5-(7-Ethyl-7*H*-imidazo[4,5-*c*]pyridazin-4-yl)-2-fluorophenyl)-5-methoxy-3-(2-methoxyethyl)benzo[*d*]thiazol-2(3*H*)-one**

Following the same experimental procedure as in Example 1, 7-ethyl-4-(4-fluoro-3-(4,4,5,5-tetramethyl-1,3,2-dioxaborolan-2-yl)phenyl)-7H-imidazo[4,5-*c*]pyridazine (129 mg, 0.35 mmol) and 6-bromo-5-methoxy-3-(2-methoxyethyl)benzo[*d*]thiazol-2(3*H*)-one (110 mg, 0.35 mmol) were used as starting materials to obtain the title compound (125 mg, 74%) as a white solid.

$^1$H NMR (400 MHz, CHLOROFORM-*d*) 9.37 (s, 1 H) 8.20 - 8.32 (m, 3 H) 7.31 - 7.40 (m, 2 H) 6.95 (s, 1 H) 4.59 (q, *J* = 7.34 Hz, 2 H) 4.19 (t, *J* = 5.14 Hz, 2 H) 3.87 (s, 3 H) 3.74 (t, *J* = 5.14 Hz, 2 H) 3.38 (s, 3 H) 1.69 (t, *J* = 7.34 Hz, 3 H). LC-MS: m/z [M+H]$^+$ = 480.

Example 34

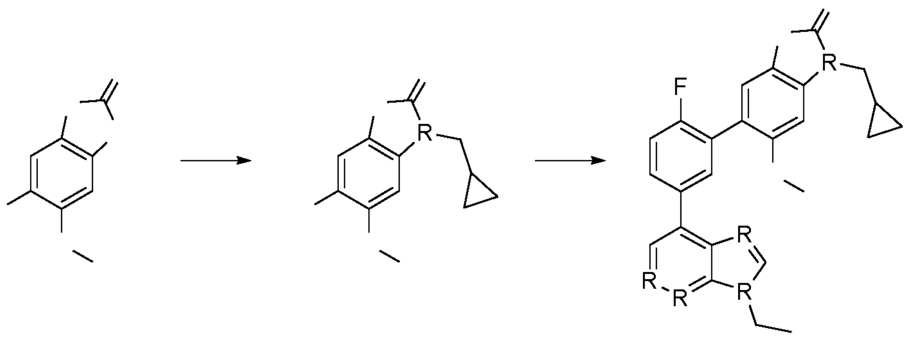

10-3 2-1

**34-1: 6-Bromo-3-(cyclopropylmethyl)-5-methoxybenzo[*d*]oxazol-2(3*H*)-one**

6-Bromo-5-methoxybenzo[*d*]oxazol-2(3*H*)-one (100 mg, 0.4 mmol) was dissolved in N-methylpyrrolidone (10 mL) under an inert atmosphere of nitrogen, then sodium cyanide (40 mg, 1.0 mmol) was added thereto at 0°C, and the mixture was stirred at room temperature for 15 minutes after the addition was completed. (Bromomethyl)cyclopropane (108 mg, 0.8 mmol) was then added thereto, and the mixture was stirred for 2 hours. The reaction mixture was poured into ice water (50 mL), extracted with ethyl acetate (100 mL × 3), rotary evaporated to dryness, and purified by column chromatography (petroleum ether: ethyl acetate = 4:1) to obtain the title product (110 g, yield: 90%) as a yellow solid. LC-MS: m/z [M+H]$^+$ = 298.0.

**34: 3-(Cyclopropylmethyl)-6-(5-(7-ethyl-7*H*-imidazo[4,5-*c*]pyridazin-4-yl)-2-fluorophenyl)-5-methoxybenzo[*d*]oxazol-2(3*H*)-one**

Following the same experimental procedure as in Example 1, 6-bromo-3-(cyclopropylmethyl)-5-methoxybenzo[*d*]oxazol-2(3*H*)-one (75 mg, 0.25 mmol) and 5-(7-ethyl-7*H*-imidazo[4,5-*c*]pyridazin-4-yl)-2-fluorophenyl)boronic acid (86 mg, 0.3 mmol) were used as starting materials to obtain the title compound (30 mg, yield: 26%) as a white solid.

$^1$H NMR (400 MHz, $CDCl_3$) 9.40 (s, 1 H), 8.35 (s, 1 H), 8.31 - 8.22 (m, 2 H), 7.35 (t, 1 H), 7.24 (s, 1 H), 6.73 (s, 1 H), 4.59 (q, *J* = 7.3 Hz, 2 H), 3.86 (s, 3 H), 3.77 (d, *J* = 7.0 Hz, 2 H), 1.70 (t, *J* = 7.3 Hz, 3 H), 1.28 (m, 1 H), 0.70 - 0.60 (m, 2 H), 0.49 (m, 2 H). LC-MS: m/z [M+H]$^+$ = 460.

Example 35

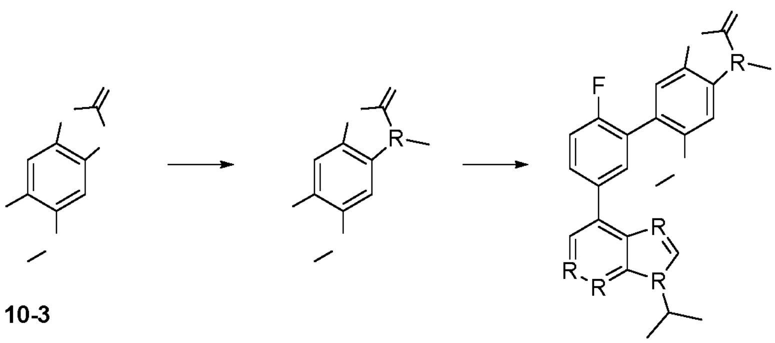

10-3

**35-1: 6-Bromo-5-methoxy-3-methylbenzo[*d*]oxazol-2(3*H*)-one**

6-Bromo-5-methoxybenzo[*d*]oxazol-2(3*H*)-one (200 mg, 0.82 mmol) was added to dimethyl sulfoxide (20 mL), then iodomethane (1.53 mL, 24.59 mmol) and cesium carbonate (534 mg, 1.64 mmol) were added thereto, and the mixture was stirred at 80°C for 3 hours. The reaction mixture was poured into water, extracted with ethyl acetate (40 mL × 3), concentrated, and purified by column chromatography (petroleum ether: ethyl acetate = 2:1) to obtain the title compound (130 mg, yield: 49%) as a purple solid.

$^1$H NMR (400 MHz, DMSO-$d_6$) 7.64 (s, 1 H), 7.19 (s, 1 H), 3.88 (s, 3 H), 3.35 (s, 3 H). LC-MS: m/z [M+H]$^+$ = 258.

**35: 6-(2-Fluoro-5-(7-isopropyl-7*H*-imidazo[4,5-*c*]pyridazin-4-yl)phenyl)-5-methoxy-3-methylbenzo[*d*]oxazol-2(3*H*)-one**

Following the same experimental procedure as in Example 1, 6-bromo-5-methoxy-3-methylbenzo[*d*]oxazol-2(3*H*)-one (30 mg, 0.12 mmol) and (2-fluoro-5-(7-isopropyl-7*H*-imidazo[4,5-*c*]pyridazin-4-yl)phenyl)boronic acid (44 mg, 0.12 mmol) were used as starting materials to obtain the title compound (40 mg, yield: 77%). LC-MS: m/z [M+H]$^+$ = 434.2.

$^1$H NMR (400 MHz, DMSO-$d_6$) 9.60 (s, 1 H), 8.99 (s, 1 H), 8.70 - 8.40 (m, 2 H), 7.61 - 7.45 (m, 1 H), 7.38 (s, 1 H), 7.21 (s, 1 H), 5.11 (m, 1 H), 3.82 (s, 3 H), 3.42 (s, 3 H), 1.68 (m, 6 H).

Example 36

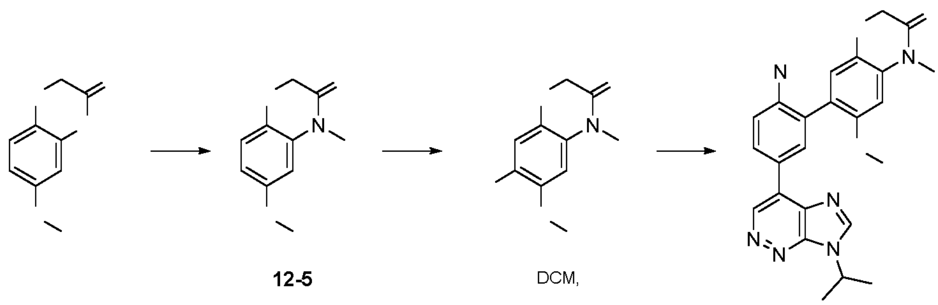


**36-1: 6-Methoxy-4-methyl-2*H*-benzo[*b*][1,4]oxazin-3(4*H*)-one**

6-Methoxy-2*H*-benzo[*b*][1,4]oxazin-3(4*H*)-one (500 mg, 2.8 mmol), cesium carbonate (2.8 g, 8.4 mmol), and iodomethane (800 mg, 5.6 mmol) were sequentially added to 20 mL of methanol, and the mixture was heated to 35°C and stirred for 2 hours. The reaction mixture was filtered to remove cesium carbonate, concentrated, and purified by preparative thin-layer chromatography (dichloromethane: methanol = 30:1) to obtain the title compound (400 mg, 74%) as a brown solid. LC-MS: m/z [M+H]$^+$ = 194.

**36-2: 7-Bromo-6-methoxy-4-methyl-2H-benzo[b][1,4]oxazin-3(4H)-one**

6-Methoxy-4-methyl-2*H*-benzo[*b*][1,4]oxazin-3(4*H*)-one (400 mg, 2 mmol) was dissolved in 5 mL of acetic acid, then 2 mL of a 3 M solution of hydrogen bromide in acetic acid was added thereto, and the mixture was stirred at room temperature for 30 minutes. The reaction mixture was then cooled to 0°C, added with 1 mL of hydrogen peroxide, warmed to room temperature, and stirred for 2 hours. Water was added thereto, and a large amount of solid was precipitated. The reaction mixture was extracted with dichloromethane, dried, concentrated, and purified by preparative thin-layer chromatography (dichloromethane: methanol = 30:1) to obtain the title compound (180 mg, 32%) as a white solid. LC-MS: m/z [M+H]$^+$ = 272.

**36: 7-(2-Fluoro-5-(7-isopropyl-7*H*-imidazo[4,5-*c*]pyridazin-4-yl)phenyl)-6-methoxy-4-methyl-2*H*-benzo[*b*][1,4]oxazin-3(4*H*)-one**

Following the same experimental procedure as in Example 1, 7-bromo-6-methoxy-4-methyl-2*H*-benzo[*b*][1,4]oxazin-3(4*H*)-one (151 mg, 0.55 mmol) and 4-(4-fluoro-3-(4,4,5,5-tetramethyl-1,3,2-dioxaborolan-2-yl)phenyl)-7-isopropyl-7*H*-imidazo[4,5-*c*]pyridazine (141 mg, 0.37 mmol) were used as starting materials to obtain the title compound (140

mg, 85%).

$^{1}$H NMR (400 MHz, DMSO-$d_6$) 9.53 (s, 1 H), 8.92 (s, 1 H), 8.42 (d, $J$ = 7.3 Hz, 2 H), 7.47 (t, $J$ = 9.0 Hz, 1 H), 7.03 (s, 1 H), 6.92 (s, 1 H), 5.13 - 5.08 (m, 1 H), 4.67 - 4.62 (m, 2 H), 3.80 (s, 3 H), 3.37 (s, 3 H), 1.66 (d, $J$ = 6.8 Hz, 6 H). LC-MS: m/z [M+H]$^+$ = 448.

Example 37

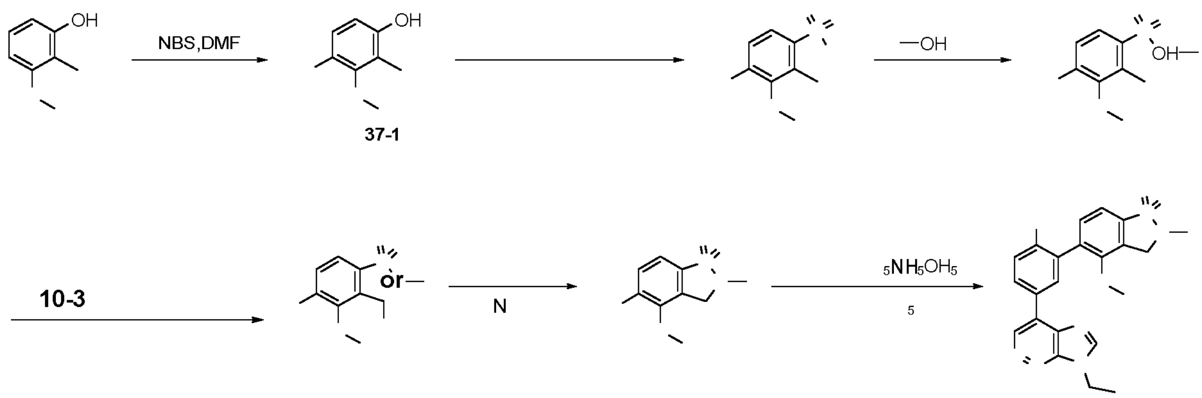


**37-1: 4-Bromo-3-methoxy-2-methylaniline**

3-Methoxy-2-methylaniline (5.48 g, 40.0 mmol) was added to *N,N*-dimethylformamide (50 mL). *N*-Bromosuccinimide (7.2 g, 40.0 mmol) was then added thereto in batches, and the mixture was reacted at room temperature for 2 hours. The reaction mixture was poured into water (200 mL) and extracted with ethyl acetate (100 mL × 3). The organic phase was rotary evaporated to dryness and separated by column chromatography (petroleum ether/ethyl acetate = 4:1) to obtain the title compound (3.5 g, yield: 41%) as a gray solid. LCMS: m/z [M+H]$^+$ = 216.1.

**37-2: 4-Bromo-3-methoxy-2-methylbenzenesulfonyl chloride**

4-Bromo-3-methoxy-2-methylaniline (2.0 g, 9.3 mmol) was dissolved in a mixture of hydrochloric acid (8 mL), acetic acid (4 mL), and acetonitrile (30 mL). The mixture was stirred for 10 minutes and cooled to 0°C. Sodium nitrite (5 N, 2 mL) was added dropwise thereto, and the mixture was reacted for 30 minutes. A saturated solution of sulfur dioxide in acetic acid (5 mL) was added dropwise thereto, and the temperature was controlled at 5°C or less for 10 minutes. Copper chloride aqueous solution (2.4 g, 2 mL/g) was then added dropwise thereto, and the mixture was returned to room temperature and reacted for 16 hours. The reaction mixture was poured into water (100 mL) and extracted with ethyl acetate (100 mL × 3). The organic phase was washed with brine, then washed with saturated sodium bicarbonate until weakly alkaline, dried, and concentrated to obtain 1.5 g of the crude title compound as a gray solid. LCMS: m/z [M+H]$^+$ = 298.9.

**37-3: 4-Bromo-3-methoxy-*N*,2-dimethylbenzenesulfonamide**

4-Bromo-3-methoxy-2-methylbenzenesulfonyl chloride (200 mg, 0.67 mmol) and methylamine hydrochloride (90 mg, 1.33 mmol) were sequentially added to dichloromethane (10 mL). Triethylamine (203 mg, 2.01 mmol) was then added dropwise thereto, and the mixture was reacted at room temperature for 2 hours. The reaction mixture was poured into water (50 mL), extracted with ethyl acetate (50 mL × 3), and rotary evaporated to dryness to obtain the title compound (200 mg) as a gray solid. $^{1}$H NMR (400 MHz, $CDCl_3$) 7.64 (d, $J$ = 8.5 Hz, 1 H), 7.58 -7.50 (m, 1 H), 3.83 (s, 3 H), 2.66 (d, $J$ = 4.5 Hz, 3H), 2.61 (s, 3 H). LCMS: m/z [M+H]$^+$ = 294.

**37-4: 4-Bromo-2-(bromomethyl)-3-methoxy-*N*-methylbenzenesulfonamide**

The starting materials 4-bromo-3-methoxy-N,2-dimethylbenzenesulfonamide (200 mg, 0.67 mmol) and dibenzoyl peroxide (100 mg, 0.41 mmol) were added to a mixture of carbon tetrachloride (10 mL) and dichloromethane (4 mL). The starting material N-bromosuccinimide (145 mg, 0.81 mmol) was added to the reaction mixture in batches. The mixture was reacted at 90°C overnight. The reaction mixture was added with water (100 mL), extracted with ethyl acetate (50 mL ×

3), concentrated, and purified by column chromatography (petroleum ether/ethyl acetate = 20% to 30%) to obtain the title compound (110 mg, yield: 44%) as a white solid. $^1$H NMR (400 MHz, DMSO-$d_6$) 7.94 - 7.84 (m, 2 H), 7.55 (t, *J* = 12.4 Hz, 1 H), 4.96 (s, 2 H), 4.04 - 3.97 (m, 3 H), 2.49 (s, 3 H). LCMS: m/z $[M+H]^+$ = 373.

**37-5: 5-Bromo-4-methoxy-2-methyl-2,3-dihydrobenzo[*d*]isothiazole 1,1-dioxide**

4-Bromo-2-(bromomethyl)-3-methoxy-*N*-methylbenzenesulfonamide (110 mg, 0.29 mmol) was added to *N,N*-dimethylformamide (10 mL). Sodium hydride (14 mg, 0.35 mmol) was added thereto at room temperature, and the mixture was reacted for 1 hour. The reaction mixture was poured into water (50 mL), extracted with ethyl acetate (50 mL × 3), rotary evaporated to dryness, and purified by TLC plate (petroleum ether/ethyl acetate = 4:1) to obtain the title compound (10 mg, yield: 11.8%) as a white solid. $^1$H NMR (400 MHz, $CDCl_3$) 7.65 (m, 1 H), 7.49 - 7.30 (m, 1 H), 4.29 (s, 2 H), 3.86 (s, 3 H), 2.89 (s, 3 H). LCMS: m/z $[M+H]^+$ = 292.

**37: 5-(5-(7-Ethyl-7*H*-imidazo[4,5-*c*]pyridazin-4-yl)-2-fluorophenyl)-4-methoxy-2-methyl-2,3-dihydrobenzo[*d*]isothiazole 1,1-dioxide**

Following the same experimental procedure as in Example 1, 5-bromo-4-methoxy-2-methyl-2,3-dihydrobenzo [*d*]isothiazole 1,1-dioxide (10 mg, 0.034 mmol) and (5-(7-ethyl-7*H*-imidazo[4,5-*c*]pyridazin-4-yl)-2-fluorophenyl)boronic acid (10 mg, 0.035 mmol) were used as starting materials to obtain the title compound (2.7 mg, yield: 18%) as a white solid.

$^1$H NMR (400 MHz, DMSO-$d_6$) 9.59 (s, 1 H), 8.88 (s, 1 H), 8.57 (t, 2 H), 7.74 (d, 1 H), 7.68 (d, 1 H), 7.60 (t, 1 H), 4.63 (s, 2 H), 4.52 (q, *J* = 7.2 Hz, 2 H), 3.72 (s, 3 H), 2.89 (s, 3 H), 1.56 (t, *J* = 7.3 Hz, 3 H). LCMS: m/z $[M+1]^+$ = 454.

Example 38

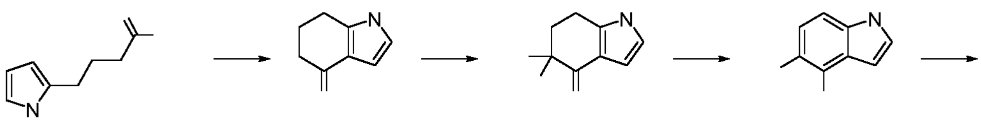

12-5 12-5 12-5

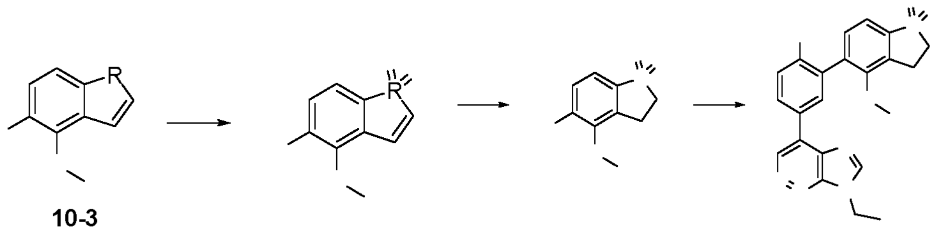

10-3

**38-1: 6,7-Dihydrobenzo[*b*]thiophen-4(5*H*)-one**

4-(Thiophen-2-yl)butanoic acid (5000 mg, 29.37 mmol) and trifluoroacetic anhydride (18510 mg, 88.11 mmol) were sequentially added to trifluoroacetic acid (40 mL), and the mixture was stirred at room temperature for 2 hours. The reaction mixture was concentrated, added with water (40 mL), and the aqueous phase was extracted with ethyl acetate (30 mL × 3). The organic phases were combined, washed with saturated brine (30 mL), dried over anhydrous sodium sulfate, filtered, and the filtrate was concentrated under reduced pressure to obtain the title compound (5400 mg, crude product) as a brown liquid. $^1$H NMR (400 MHz, CHLOROFORM-*d*) δ = 7.39 (d, *J* = 5.4 Hz, 1 H), 7.07 (d, *J* = 5.4 Hz, 1 H), 3.04 (t, *J* = 5.9 Hz, 2 H), 2.59 (t, *J* = 6.4 Hz, 2 H), 2.22 (q, *J* = 6.2 Hz, 2 H).

**38-2: 5,5-Dibromo-6,7-dihydrobenzo[*b*]thiophen-4(5*H*)-one**

Ethyl acetate (20 mL), 6,7-dihydrobenzo[*b*]thiophen-4(5H)-one (5000 mg, 32.85 mmol), and copper bromide (14670 mg, 65.7 mmol) were sequentially added to chloroform (20 mL), and the mixture was stirred at 80°C in an open system for 16 hours. The reaction mixture was filtered, and the filtered solid was purified by silica gel column chromatography (petroleum ether: ethyl acetate = 100:1) to obtain the product, which was concentrated to obtain the title compound

(1500 mg, 15%) as a brown solid. $^1$H NMR (400 MHz, CHLOROFORM-*d*) δ = 7.47 (d, *J* = 4.9 Hz, 1 H), 7.16 (d, *J* = 4.9 Hz, 1 H), 3.15 (s, 4 H).

**38-3: 5-Bromobenzo[*b*]thiophen-4-ol**

5,5-Dibromo-6,7-dihydrobenzo[*b*]thiophen-4(5*H*)-one (1400 mg, 4.52 mmol) and lithium carbonate (1000 mg, 13.56 mmol) were sequentially added to*N,N*-dimethylformamide (20 mL), and the mixture was stirred at 100°C for 3 hours. The reaction mixture was added with water (50 mL), and the aqueous phase was extracted with ethyl acetate (30 mL × 3). The organic phases were combined, washed with saturated brine (30 mL), dried over anhydrous sodium sulfate, filtered, and the filtrate was concentrated under reduced pressure to obtain the title compound (1000 mg, 94%) as a white solid. $^1$H NMR (400 MHz, CHLOROFORM-*d*) δ = 7.52 (d, *J* = 5.4 Hz, 1 H), 7.40 - 7.35 (m, 2 H), 7.34 - 7.30 (m, 1 H), 6.32 (br. s., 1 H).

**38-4: 5-Bromo-4-methoxybenzo[b]thiophene**

5-Bromobenzo[*b*]thiophen-4-ol (1000 mg, 4.37 mmol), dimethyl sulfate (1650 mg, 13.11 mmol), and potassium carbonate (1810 mg, 13.11 mmol) were sequentially added to acetone (40 mL), and the mixture was stirred at 50°C for 16 hours. The reaction mixture was filtered, and the filter residue was washed with acetone (5 mL × 3). The filtrate was concentrated under reduced pressure and dissolved in methanol (20 mL). Sodium hydroxide (600 mg) was added thereto, and the mixture was stirred at room temperature for 0.5 hours to quench the excess dimethyl sulfate. After filtration, the filtrate was concentrated under reduced pressure to obtain the title compound (1000 mg, 97%) as a brown solid. $^1$H NMR (400 MHz, CHLOROFORM-*d*) δ = 7.52 - 7.42 (m, 4 H), 4.00 (s, 3 H).

**38-5: 5-Bromo-4-methoxybenzo[*b*]thiophene 1,1-dioxide**

5-Bromo-4-methoxybenzo[*b*]thiophene (600 mg, 2.47 mmol) and potassium peroxymonosulfate (4555 mg, 7.41 mmol) were sequentially added to a mixed solvent of methanol (5 mL), tetrahydrofuran (5 mL), and water (5 mL), and the mixture was stirred at 40°C for 16 hours. The reaction mixture was diluted with water (100 mL), filtered, and dried to obtain the title compound (500 mg, 74%) as a white solid. LC-MS: m/z $[M+H]^+$ = 276, 278.

**38-6: 5-Bromo-4-methoxy-2,3-dihydrobenzo[*b*]thiophene 1,1-dioxide**

5-Bromo-4-methoxybenzo[*b*]thiophene 1,1-dioxide (200 mg, 0.73 mmol) and sodium cyanoborohydride (55 mg, 1.46 mmol) were sequentially added to ethanol (10 mL), and the mixture was stirred at room temperature for 2 hours. The reaction mixture was added with hydrochloric acid aqueous solution (1 M) to adjust the pH to weakly acidic, and diluted with water (20 mL). The phases were separated, and the aqueous phase was extracted with dichloromethane (20 mL × 3). The organic phases were combined, washed with saturated brine (20 mL), dried over anhydrous sodium sulfate, filtered, and the filtrate was concentrated under reduced pressure to remove the solvent to obtain the title compound (150 mg, 74%) as a white solid. LC-MS: m/z $[M+H]^+$ = 278, 280.

**38: 5-(5-(7-Ethyl-7*H*-imidazo[4,5-c]pyridazin-4-yl)-2-fluorophenyl)-4-methoxy-2,3-dihydrobenzo[*b*]thiophene 1,1-dioxide**

Following the same experimental procedure as in Example 1, 5-bromo-4-methoxy-2,3-dihydrobenzo[*b*]thiophene 1,1-dioxide (120 mg, 0.43 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (158 mg, 0.43 mmol) were used as starting materials to obtain the title compound (40 mg, 21%) as a white solid.

$^1$H NMR (400 MHz, CHLOROFORM-d) δ = 9.40 (br. s., 1 H), 8.38 - 8.28 (m, 3 H), 7.64 - 7.52 (m, 2 H), 7.41 (t, *J* = 8.8 Hz, 1 H), 4.59 (q, *J* = 7.3 Hz, 2 H), 3.63 (s, 3 H), 3.60 - 3.52 (m, 2 H), 3.51 - 3.42 (m, 2 H), 1.70 (t, *J* = 7.1 Hz, 3 H). LC-MS: m/z $[M+H]^+$ = 439.

Example 39

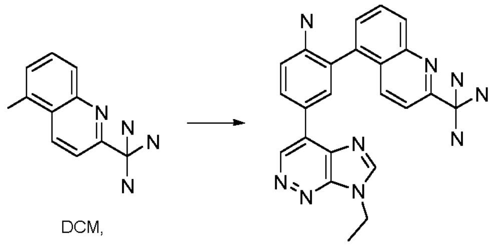


**39: 5-(5-(7-Ethyl-7*H*-imidazo[4,5-*c*]pyridazin-4-yl)-2-fluorophenyl)-2-(trifluoromethyl)quinoline**

Following the same experimental procedure as in Example 1, ethyl-4-(4-fluoro-3-(4,4,5,5-tetramethyl-1,3,2-dioxaborolan-2-yl)phenyl)-7*H*-imidazo[4,5-*c*]pyridazine (100 mg, 0.27 mmol) and 5-bromo-2-(trifluoromethyl)quinoline (74 mg, 0.27 mmol) were used as starting materials to obtain the title compound (68 mg, 55.31%) as a white solid.

$^1$H NMR (400 MHz, CHLOROFORM-d) d = 9.41 (s, 1 H), 8.42 - 8.22 (m, 5 H), 7.94 (t, *J* = 7.8 Hz, 1 H), 7.81 - 7.70 (m, 2 H), 7.49 (t, *J* = 8.8 Hz, 1 H), 4.60 (q, *J* = 7.0 Hz, 2 H), 1.70 (t, *J* = 7.3 Hz, 3 H). LC-MS: m/z $[M+H]^+$ = 438.

Example 40 and Example 41

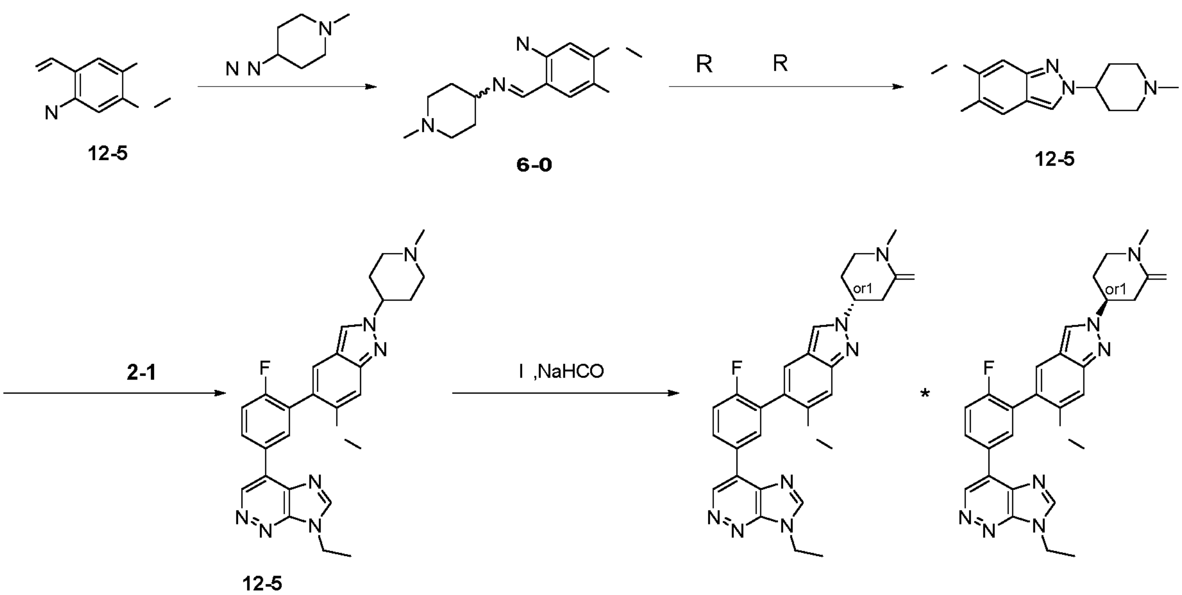


12-5

6-0

12-5

12-5

**40-1: 1-(5-Bromo-4-methoxy-2-nitrophenyl)-*N*-(1-methylpiperidin-4-yl)methanimine**

5-Bromo-4-methoxy-2-nitrobenzaldehyde (780 mg, 3.0 mmol) and 1-methylpiperidin-4-amine (342 mg, 3.0 mmol) were added to toluene (50 mL), and the mixture was stirred at 110°C for 16 hours. The reaction mixture was concentrated to obtain the title compound (1.0 g, yield: 94%) as a yellow-brown oil. LC-MS: m/z $[M+H]^+$ = 356.

**40-2: 5-Bromo-6-methoxy-2-(1-methylpiperidin-4-yl)-2*H*-indazole**

1-(5-Bromo-4-methoxy-2-nitrophenyl)-*N*-(1-methylpiperidin-4-yl)methanimine (1.0 g, 2.8 mmol) was dissolved in triethyl phosphate (20 mL), and the mixture was reacted at 150°C for 2 hours. The reaction mixture was directly purified by column chromatography (dichloromethane: methanol = 12:1) to obtain the title compound (650 mg, yield: 71%) as a yellow solid. LC-MS: m/z $[M+H]^+$ = 324.

**40-3: 7-Ethyl-4-(4-fluoro-3-(6-methoxy-2-(1-methylpiperidin-4-yl)-2*H*-indazol-5-yl)phenyl)-7*H*-imidazo[4,5-c]pyridazine**

Following the same experimental procedure as in Example 1, 5-bromo-6-methoxy-2-(1-methylpiperidin-4-yl)-2*H*-indazole (324 mg, 1.0 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (286 g, 1.0 mmol) were used as starting materials to obtain a yellow-brown solid (200 mg, yield: 37%). LC-MS: m/z $[M+H]^+$ = 486.

**Example 40: *rel*-(R)-4-(5-(5-(7-Ethyl-7*H*-imidazo[4,5-*c*]pyridazin-4-yl)-2-fluorophenyl)-6-methoxy-2*H*-indazol-2-yl)-1-methylpiperidin-2-one**

**Example 41: *rel*-(*S*)-4-(5-(5-(7-Ethyl-7*H*-imidazo[4,5-*c*]pyridazin-4-yl)-2-fluorophenyl)-6-methoxy-2*H*-indazol-2-yl)-1-methylpiperidin-2-one**

7-Ethyl-4-(4-fluoro-3-(6-methoxy-2-(1-methylpiperidin-4-yl)-2*H*-indazol-5-yl)phenyl)-7*H*-imidazo[4,5-*c*]pyridazine (175 mg, 0.36 mmol) was dissolved in tetrahydrofuran (20 mL), then sodium bicarbonate (303 mg, 3.6 mmol), iodine (916 mg, 3.6 mmol), and water (8 mL) were added thereto, and the mixture was stirred at 30°C for 24 hours. The reaction mixture was then poured into water (50 mL), extracted with ethyl acetate (50 mL × 3), dried, concentrated, and separated by column chromatography (dichloromethane: methanol = 10:1) to obtain a yellow solid (120 mg), which was purified by prep-HPLC to obtain a white solid (60 mg). The solid was then subjected to SFC chiral resolution to obtain compounds of Example 40 and Example 41. Resolution method: instrument: MG II preparative SFC (SFC-1); column: ChiralPak AD, 250 × 30 mm I.D., 10 μm; mobile phase: A for $CO_2$ and B for isopropanol; gradient: B 55%; flow rate: 80 mL/min; back pressure: 100 bar; column temperature: 38°C; wavelength: 220 nm.

Example 40: The title product (7.2 mg, white solid, second peak) was obtained. $^1H$ NMR (400 MHz, $CDCl_3$) 9.43 (brs, 1 H), 8.35 (m, 2 H), 8.23 (d, 1 H), 7.92 (s, 1 H), 7.62 (s, 1 H), 7.35 (t, 1 H), 7.11 (s, 1 H), 4.91 (m, 1 H), 4.59 (d, *J = 7.1* Hz, 2 H), 3.87 (s, 3 H), 3.43 (m, 1 H), 3.35 (m, 1 H), 3.09 (m, 2 H), 3.02 (s, 3 H), 2.56 (m, 1 H), 2.49 (m, 1 H), 1.70 (t, *J* = 7.0 Hz, 3 H). LC-MS: m/z $[M+H]^+$ = 500.

Example 41: The title product (6.2 mg, white solid, first peak) was obtained. $^1H$ NMR (400 MHz, $CDCl_3$) 9.41 (brs, 1 H), 8.31 (m, 2 H), 8.21 (d, 1 H), 7.91 (s, 1 H), 7.62 (s, 1 H), 7.34 (t, 1 H), 7.11 (s, 1 H), 4.89 (m, 1 H), 4.59 (q, *J* = 7.1 Hz, 2 H), 3.87 (s, 3 H), 3.53 - 3.39 (m, 1 H), 3.39 - 3.28 (m, 1 H), 3.19 - 3.05 (m, 2 H), 3.02 (s, 3 H), 2.67 - 2.52 (m, 1 H), 2.48 (s, 1 H), 1.69 (t, *J* = 7.1 Hz, 3 H). LC-MS: m/z $[M+H]^+$ = 500.

Example 42

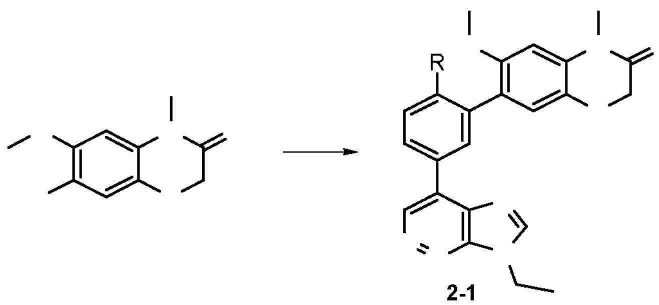

2-1

**42: 7-(5-(7-Ethyl-7*H*-imidazo[4,5-*c*]pyridazin-4-yl)-2-fluorophenyl)-6-methoxy-4-methyl-2*H*-benzo[*b*][1,4]oxazin-3(4*H*)-one**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (220 mg, 0.6 mmol) and 7-bromo-6-methoxy-4-methyl-2*H*-benzo[*b*][1,4]oxazin-3(4H)-one (160 mg, 0.6 mmol) were used as starting materials to obtain the title compound (97 mg, 37%) as a brown solid.

$^1H$ NMR (400 MHz, CHLOROFORM-d) 9.54 (s, 1 H) 8.86 (s, 1 H) 8.37 - 8.46 (m, 2 H) 7.43 - 7.51 (m, 1 H) 7.04 (s, 1 H) 6.93 (s, 1 H) 4.65 (s, 2 H) 4.50 (d, *J = 7.34* Hz, 2 H) 3.81 (s, 3 H) 3.38 (s, 3 H) 1.55 (t, *J* = 7.09 Hz, 3 H). LC-MS: m/z $[M+H]^+$ = 434.

Example 43

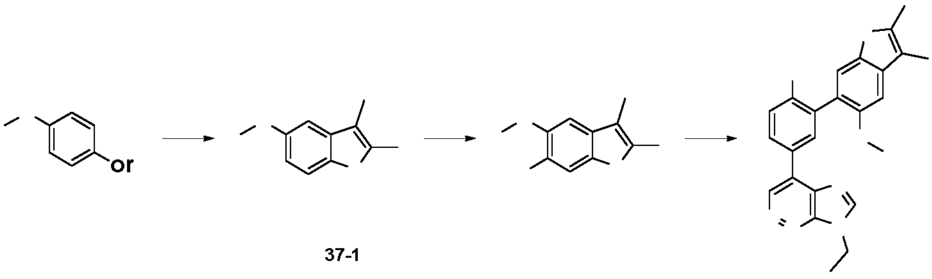

37-1

### 43-1: 5-Methoxy-2,3-dimethylbenzo[*b*]thiophene

4-Methoxybenzenethiol (501 mg, 3.57 mmol) was added to a reaction flask, and the mixture was stirred at 0°C for 15 minutes. Sodium hydroxide (285 mg, 7.14 mmol) was dissolved in water (3 mL) and slowly added to the reaction mixture. After the addition was completed, the reaction mixture was stirred at 0°C for 30 minutes. 3-Bromo-2-butanone (1 g, 6.62 mmol) was slowly added to the reaction mixture. After the addition was completed, the reaction mixture was stirred at room temperature for 1 hour. The reaction mixture was poured into water (30 mL) and extracted with dichloromethane. The organic phase was dried over anhydrous sodium sulfate and concentrated to obtain a crude intermediate. Polyphosphoric acid (3.24 g, 39.5 mmol) and the crude intermediate from the previous step were slowly added to a reaction flask, and the mixture was stirred at 150°C for 1 hour. The reaction mixture was cooled to room temperature, poured into water (30 mL), extracted with dichloromethane, and the organic phase was concentrated. The residue was subjected to column chromatography (petroleum ether/ethyl acetate = 50/1) to obtain the title compound (480 mg, 57%) as a yellow oily liquid. $^1$H NMR (400 MHz, CHLOROFORM-d) 7.61 (d, *J* = 8.8 Hz, 1 H), 7.05 (d, *J* = 2.0 Hz, 1 H), 6.93 (dd, *J* = 2.0, 8.8 Hz, 1 H), 3.89 (s, 3 H), 2.48 (s, 3 H), 2.27 (s, 3 H).

### 43-2: 6-Bromo-5-methoxy-2,3-dimethylbenzo[b]thiophene

5-Methoxy-2,3-dimethylbenzo[*b*]thiophene (480 mg, 2.5 mmol) was added to diethyl ether (3 mL), and the mixture was stirred at 0°C for 10 minutes. Liquid bromine (400 mg, 2.5 mmol) was dissolved in diethyl ether (2 mL) and slowly added dropwise to the reaction mixture at 0°C. After the addition was completed, the reaction mixture was slowly warmed to room temperature and stirred for another 1 hour. The reaction mixture was poured into saturated sodium bicarbonate aqueous solution (30 mL), stirred at room temperature for 10 minutes, and extracted with ethyl acetate. The organic phase was dried, concentrated, and subjected to prep-HPLC to obtain the title compound and 4-bromo-5-methoxy-2,3-dimethylbenzo[*b*]thiophene (240 mg) as a brown solid. $^1$H NMR (400 MHz, CHLOROFORM-*d*) 7.87 (s, 1 H), 7.00 (s, 1 H), 3.97 (s, 3 H), 2.46 (s, 3 H), 2.26 (s, 3 H).

### 43: 7-Ethyl-4-(4-fluoro-3-(5-methoxy-2,3-dimethylbenzo[*b*]thiophen-6-yl)phenyl)-7*H*-imidazo[4,5-*c*]pyridazine

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-*c*]pyridazine (70 mg, 0.19 mmol) and 6-bromo-5-methoxy-2,3-dimethylbenzo [b]thiophene (103 mg, 0.19 mmol) were used as starting materials to obtain the title compound (11 mg, 13%) as a white solid.

$^1$H NMR (400 MHz, CHLOROFORM-*d*) d = 9.37 (s, 1 H), 8.32 - 8.19 (m, 3 H), 7.69 (s, 1 H), 7.33 (t, *J* = 8.8 Hz, 1 H), 7.13 (s, 1 H), 4.57 (q, *J* = 7.3 Hz, 2 H), 3.90 (s, 3 H), 2.50 (s, 3 H), 2.32 (s, 3 H), 1.68 (t, *J* = 7.3 Hz, 3 H). LC-MS: m/z $[M+H]^+$ = 433.

Example 44

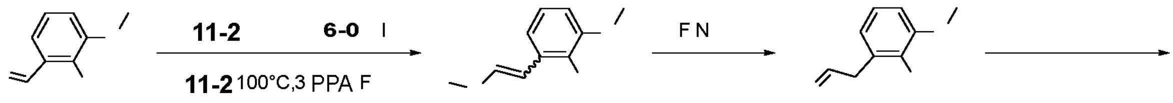


12-5 6-0 12-5

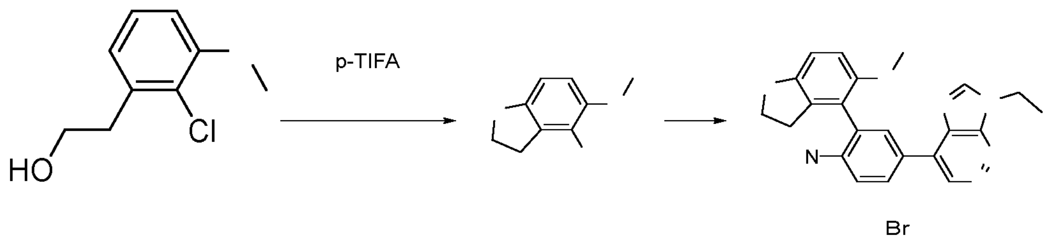


**44-1: 2-Chloro-1-methoxy-3-(2-methoxyvinyl)benzene**

Potassium *tert*-butoxide (855 mg, 7.62 mmol) was added to a solution of (methoxymethyl)triphenyl chloride (2.61 g, 7.62 mmol) in tetrahydrofuran (20 mL) at 0°C, and the mixture was stirred for 1 hour. 2-Chloro-3-methoxybenzaldehyde (1 g, 5.86 mmol) was then added dropwise thereto, and the mixture was stirred at 0°C for 1 hour. Saturated ammonium chloride aqueous solution was added thereto, and the mixture was extracted with diethyl ether (40 mL × 3). The organic phases were combined, washed with water and brine, then dried over anhydrous sodium sulfate, and concentrated under vacuum to obtain 2-chloro-1-methoxy-3-(2-methoxyvinyl)benzene (1 g, yield: 85%), which was used without further purification.

**44-2: 2-(2-Chloro-3-methoxyphenyl)acetaldehyde**

6 N HCl (20 mL) was added to a solution of 2-chloro-1-methoxy-3-(2-methoxyvinyl)benzene (1 g) in tetrahydrofuran (20 mL), and the mixture was heated to reflux for 4 hours. Water was added thereto, and the mixture was extracted with diethyl ether (40 mL × 3). The organic phases were combined, washed with water and brine, then dried over anhydrous sodium sulfate, and concentrated under vacuum. The residue was then purified by column chromatography (petroleum ether: ethyl acetate = 100:1 to 10:1) to obtain 2-(2-chloro-3-methoxyphenyl)acetaldehyde (500 mg, yield: 45%) as a colorless oil.

**44-3: 2-(2-Chloro-3-methoxyphenyl)ethan-1-ol**

Sodium borohydride (0.61 g, 15 mmol) was added to a solution of 2-(2-chloro-3-methoxyphenyl)acetaldehyde (1 g, 15 mmol) in methanol at 0°C. The mixture was stirred at 0°C for 2 hours. Saturated sodium bicarbonate aqueous solution was added thereto, and the mixture was extracted with diethyl ether (10 mL × 3). The organic phases were combined, dried over anhydrous sodium sulfate, and concentrated under vacuum. The residue was then purified by column chromatography (petroleum ether: ethyl acetate = 100:1 to 10:1) to obtain 2-(2-chloro-3-methoxyphenyl)ethan-1-ol (0.3 g, yield: 29%) as a colorless oil. $^1$H NMR (400 MHz, $CDCl_3$) 7.15 (dd, 1 H), 6.88 (dd, 1 H), 6.81(dd, 1 H), 3.86 (m, 5 H), 3.01 (t, 2 H), 1.85 (s, 1 H).

**44-4: 4-Chloro-5-methoxy-2,3-dihydrobenzofuran**

[Bis(trifluoroacetoxy)iodo]*p*-toluene (720 mg, 1.76 mmol) and triethylamine (40 mg, 0.4 mmol) were added to a solution of 2-(2-chloro-3-methoxyphenyl)ethan-1-ol (300 mg, 1.6 mmol) in trifluoroethanol (30 mL) at 0°C under an argon atmosphere. The mixture was stirred at 50°C for 36 hours. Triethylamine (640 mg, 6.4 mmol) was added thereto at 50°C, immediately followed by addition of copper(II) hexafluoroacetylacetonate (760 mg, 1.6 mmol). The mixture was heated to 50°C and stirred until the iodonium salt intermediate was consumed. Saturated sodium bicarbonate aqueous solution was added thereto, and the mixture was extracted with diethyl ether (10 mL × 3). The organic phases were combined, dried over anhydrous sodium sulfate, and concentrated under vacuum. The residue was then purified by column chromatography (petroleum ether: ethyl acetate = 100:1 to 10:1) to obtain 4-chloro-5-methoxy-2,3-dihydrobenzofuran (40 mg, yield: 14%) as a white solid. $^1$H NMR (400 MHz, $CDCl_3$) 6.69 (d, 1 H), 6.61 (d, H), 4.59 (t, 2 H), 3.84 (s, 3 H), 3.24 (t, 2 H).

**44: 7-Ethyl-4-(4-fluoro-3-(5-methoxy-2,3-dihydrobenzofuran-4-yl)phenyl)-7*H*-imidazo[4,5-*c*]pyridazine**

Following the same experimental procedure as in Example 1, 4-chloro-5-methoxy-2,3-dihydrobenzofuran (30 mg, 0.16 mmol) and 5-(7-ethyl-7*H*-imidazo[4,5-*c*]pyridazin-4-yl)-2-fluorophenyl)boronic acid (45 mg, 0.16 mmol) were used as starting materials to obtain the title compound (14 mg, yield: 16%).

$^1$H NMR (400 MHz, DMSO-$d_6$) 9.39 (s, 1 H), 8.43 - 8.10 (m, 3 H), 7.36 (t, 1 H), 6.85 - 6.73 (m, 2 H), 4.57 (t, 4H), 3.74 (s, 3 H), 3.10 (m, 2 H), 1.70 (m, 3 H). LC-MS: m/z $[M+H]^+$ = 391.

Example 45

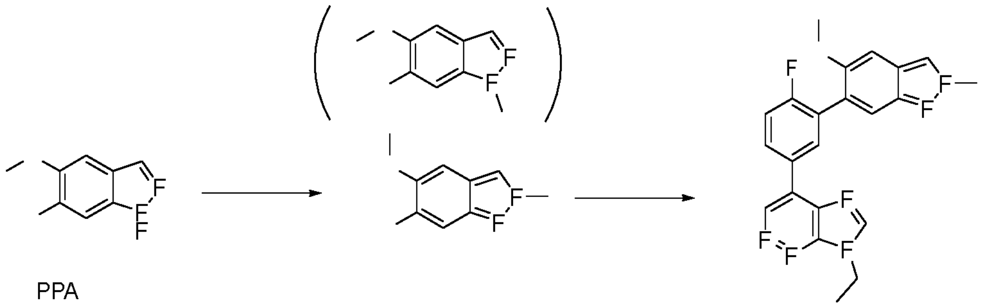


**45-A1: 6-Bromo-5-methoxy-1-methyl-1*H*-indazole**

**45-A2: 6-Bromo-5-methoxy-2-methyl-2*H*-indazole**

6-Bromo-5-methoxy-1*H*-indazole (200 mg, 0.88 mmol), cesium carbonate (287 mg, 0.88 mmol), and iodomethane (125 mg, 0.88 mmol) were sequentially added to tetrahydrofuran (5 mL). The mixture was stirred at 35°C overnight. The reaction mixture was directly subjected to thin-layer chromatography (petroleum ether/ethyl acetate = 5/1) to obtain compound A1 (76 mg, 36%, Rf = 0.4) and compound A2 (70 mg, 33%, Rf = 0.3). LC-MS: m/z $[M+H]^+$ = 241.

**45: 7-Ethyl-4-(4-fluoro-3-(5-methoxy-2-methyl-2H-indazol-6-yl)phenyl)-7*H*-imidazo[4,5-*c*]pyridazine**

Following the same experimental procedure as in Example 1, 6-bromo-5-methoxy-2-methyl-2*H*-indazole (70 mg, 0.29 mmol) and 7-ethyl-4-(4-fluoro-3-(4,4,5,5-tetramethyl-1,3,2-dioxaborolan-2-yl)phenyl)-7*H*-imidazo[4,5-c]pyridazine (107 mg, 0.29 mmol) were used as starting materials to obtain the title compound (20 mg, 16%). LC-MS: m/z $[M+H]^+$ = 403.

$^1$H NMR (400 MHz, DMSO-$d_6$) d ppm 1.51 (br. s., 3 H) 3.72 (br. s., 3 H) 4.12 (br. s., 3 H) 4.47 (br. s., 2 H) 7.14 (br. s., 1 H) 7.35 - 7.63 (m, 2 H) 8.21 (br. s., 1 H) 8.45 (br. s., 2 H) 8.81 (br. s., 1 H) 9.53 (br. s., 1 H).

Example 46 and Example 47

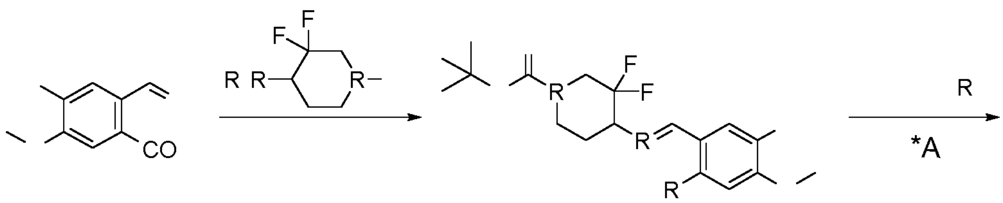

**10-3**

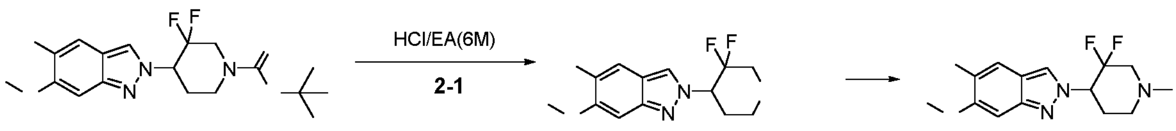


12-5 12-5

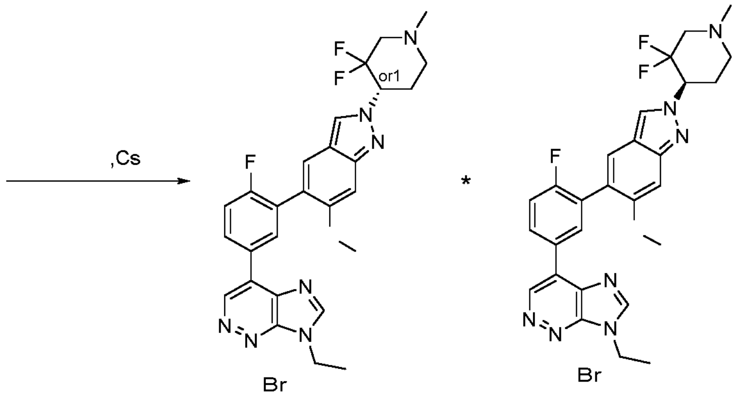


**46-1: *tert-Butyl* 4-((5-bromo-4-methoxy-2-nitrobenzylidene)amino)-3,3-difluoropiperidine-1-carboxylate**

Following the same experimental procedure as in the synthesis method of 40-1 in Example 40, 5-bromo-4-methoxy-2-nitrobenzaldehyde (780 mg, 3.0 mmol) and *tert*-butyl 4-amino-3,3-difluoropiperidine-1-carboxylate (1.06 g, 4.5 mmol) were used as starting materials to obtain the title compound (950 mg, yield: 66%) as a yellow oil. LC-MS: m/z $[M+H-56]^+$ = 422.

**46-2: *tert*-Butyl 4-(5-bromo-6-methoxy-2*H*-indazol-2-yl)-3,3-difluoropiperidine-1-carboxylate**

Following the same experimental procedure as in the synthesis method of 40-1 in Example 40, *tert-butyl* 4-((5-bromo-4-methoxy-2-nitrobenzylidene)amino)-3,3-difluoropiperidine-1-carboxylate (950 mg, 2.0 mmol) was used as the starting material to obtain the title compound (500 mg, yield: 56%) as a blue-brown solid. LC-MS: m/z $[M+H]^+$ = 446.

**46-3: 5-Bromo-2-(3,3-difluoropiperidin-4-yl)-6-methoxy-2*H*-indazole hydrochloride**

*tert*-Butyl 4-(5-bromo-6-methoxy-2*H*-indazol-2-yl)-3,3-difluoropiperidine-1-carboxylate (500 mg, 1.1 mmol) was dissolved in methanol (25 mL), then a solution of hydrochloric acid in 1,4-dioxane (4 mol/L, 25 mL) was added thereto, and the mixture was reacted at room temperature for 2 hours. The reaction mixture was then directly concentrated to obtain the title compound (420 mg, yield: 98%) as a white solid. LC-MS: m/z $[M+H]^+$ = 346.

**46-4: 5-Bromo-2-(3,3-difluoro-1-methylpiperidin-4-yl)-6-methoxy-2*H*-indazole**

5-Bromo-2-(3,3-difluoropiperidin-4-yl)-6-methoxy-2*H*-indazole hydrochloride (420 mg, 1.1 mmol) was dissolved in dichloromethane (20 mL), then formaldehyde (37% aqueous solution, 1 mL) and acetic acid (0.5 mL) were sequentially added thereto, and the mixture was stirred for 30 minutes. Sodium triacetoxyborohydride (467 mg, 2.2 mmol) was then added thereto, and the mixture was stirred at room temperature for 16 hours. The reaction mixture was directly concentrated and purified by column chromatography (dichloromethane: methanol = 10:1) to obtain the title compound (396 mg, yield: 99.5%) as an off-white solid. LC-MS: m/z $[M+H]^+$ = 360.

**Example 46: *rel*-(*S*)-4-(3-(2-(3,3-difluoro-1-methylpiperidin-4-yl)-6-methoxy-2*H*-indazol-5-yl)-4-fluorophenyl)-7-ethyl-7H-imidazo[4,5-*c*]pyridazine**

**Example 47: *rel*-(*R*)-4-(3-(2-(3,3-difluoro-1-methylpiperidin-4-yl)-6-methoxy-2*H*-indazol-5-yl)-4-fluorophenyl)-7-ethyl-7H-imidazo[4,5-*c*]pyridazine**

Following the same experimental procedure as in Example 1, 5-bromo-2-(3,3-difluoro-1-methylpiperidin-4-yl)-6-methoxy-2*H*-indazole (396 mg, 1.1 mmol) and 7-ethyl-4-(4-fluoro-3-(4,4,5,5-tetramethyl-1,3,2-dioxaborolan-2-yl)phenyl)-7*H*-imidazo[4,5-c]pyridazine (315 g, 1.1 mmol) were used as starting materials to obtain a yellow solid (570 mg). The

solid was further subjected to chiral resolution. Resolution method: instrument: MG II preparative SFC (SFC-1); column: ChiralPak AD, 250 × 30 mm I.D., 10 μm; mobile phase: A for $CO_2$ and B for isopropanol; gradient: B 55%; flow rate: 80 mL/min; back pressure: 100 bar; column temperature: 38°C; wavelength: 220 nm.

Example 46: The title compound (275 mg, first peak) was obtained. $^1$H NMR (400 MHz, $CDCl_3$) 9.38 (s, 1 H), 8.35 - 8.25 (m, 2 H), 8.22 (m, 1 H), 8.07 (s, 1 H), 7.65 (s, 1 H), 7.33 (t, 1 H), 7.09 (s, 1 H), 4.81 (s, 1 H), 4.58 (q, *J* = 7.3 Hz, 2 H), 3.92 - 3.80 (m, 3 H), 3.40 (m, 2 H), 2.56 (m, 6 H), 1.69 (t, *J* = 7.3 Hz, 3 H), 1.32 (m, 1 H). LC-MS: m/z $[M+H]^+$ = 522.

Example 47: The title compound (273 mg, second peak) was obtained. $^1$H NMR (400 MHz, DMSO-$d_6$) 9.56 (s, 1 H), 8.85 (s, 1 H), 8.55 - 8.36 (m, 3 H), 7.71 (s, 1 H), 7.48 (t, *J* = 9.1 Hz, 1 H), 7.14 (s, 1 H), 5.06 (m, 1 H), 4.51 (q, *J = 7.2* Hz, 2 H), 3.80 (s, 3 H), 3.22 (m, 1 H), 2.99 (m, 1 H), 2.64 - 2.53 (m, 1 H), 2.35 (m, 4 H), 2.16 (m, 1 H), 1.55 (t, *J = 7.3* Hz, 3 H), 1.26 (m, 1 H). LC-MS: m/z $[M+H]^+$ = 522.

Example 48

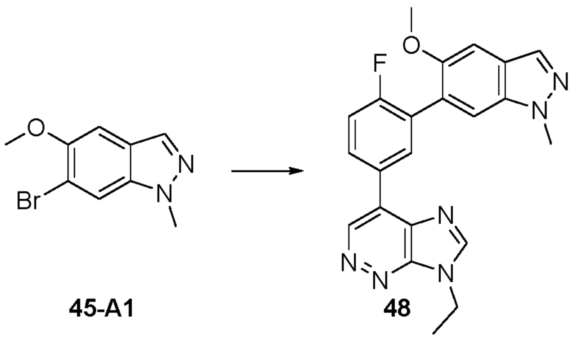

**45-A1** **48**

**48: 7-Ethyl-4-(4-fluoro-3-(5-methoxy-1-methyl-1*H*-indazol-6-yl)phenyl)-7*H*-imidazo[4,5-*c*]pyridazine**

Following the same experimental procedure as in Example 1, 6-bromo-5-methoxy-1-methyl-1*H*-indazole (70 mg, 0.29 mmol) and 7-ethyl-4-(4-fluoro-3-(4,4,5,5-tetramethyl-1,3,2-dioxaborolan-2-yl)phenyl)-7*H*-imidazo[4,5-c]pyridazine (107 mg, 0.29 mmol) were used as starting materials to obtain the title compound (33 mg, 28%).

$^1$H NMR (400 MHz, CHLOROFORM-*d*) 9.38 (br. s., 1 H), 8.41 - 8.17 (m, 3 H), 7.93 (br. s., 1 H), 7.45 - 7.32 (m, 2 H), 7.19 (br. s., 1 H), 4.66 - 4.46 (m, 2 H), 4.07 (s, 3 H), 3.85 (s, 3 H), 1.68 (t, *J*= 6.8 Hz, 3 H). LC-MS: m/z $[M+H]^+$ = 403.

Example 49

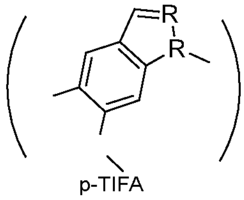

p-TIFA

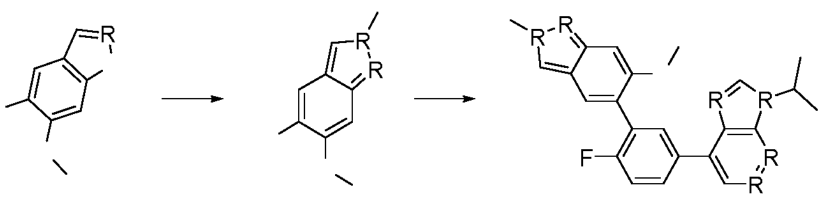

**49-A1: 5-Bromo-6-methoxy-1-methyl-1*H*-indazole**

**49-A2: 5-Bromo-6-methoxy-2-methyl-2*H*-indazole**

5-Bromo-6-methoxy-1H-indazole (200 mg, 0.88 mmol) was added to anhydrous N,N-dimethylformamide (5 mL), then sodium hydride (43 mg, 1.06 mmol) was added thereto at room temperature, and the mixture was stirred at room temperature for 0.5 hours. Iodomethane (151 mg, 1.06 mmol) was then added thereto, and the mixture was stirred at room temperature for another 0.5 hours. The reaction mixture was added dropwise to saturated ammonium chloride aqueous solution (20 mL) and extracted with ethyl acetate (20 mL × 3). The organic phases were combined, washed with saturated brine (30 mL), concentrated, and separated by preparative thin-layer chromatography (petroleum ether/ethyl acetate = 1/1) to obtain 5-bromo-6-methoxy-1-methyl-1*H*-indazole (150 mg, 70%) as a yellow solid and 5-bromo-6-methoxy-2-methyl-2*H*-indazole (60 mg, 28%) as a white solid.

A1: 5-bromo-6-methoxy-1-methyl-1*H*-indazole (developing solvent: petroleum ether: ethyl acetate = 3:1, Rf = 0.5), $^1$H NMR (400 MHz, CHLOROFORM-d) 7.89 (s, 1 H), 7.83 (s, 1 H), 6.74 (s, 1 H), 4.03 (s, 3 H), 3.98 (s, 3 H). LC-MS: m/z $[M+H]^+$ = 241, 243.

A2: 5-bromo-6-methoxy-2-methyl-2H-indazole (developing solvent: petroleum ether: ethyl acetate = 3:1, Rf = 0.3), $^1$H NMR (400 MHz, CHLOROFORM-*d*) 7.85 (s, 1 H), 7.76 (s, 1 H), 7.02 (s, 1 H), 4.16 (s, 26 H), 3.94 (s, 3 H). LC-MS: m/z $[M+H]^+$ = 241, 243.

**49: 4-(4-Fluoro-3-(6-methoxy-2-methyl-2*H*-indazol-5-yl)phenyl)-7-isopropyl-7*H*-imidazo[4,5-*c*]pyridazine**

Following the same experimental procedure as in Example 1, 5-bromo-6-methoxy-2-methyl-2*H*-indazole (52 mg, 0.22 mmol) and 4-(4-fluoro-3-(4,4,5,5-tetramethyl-1,3,2-dioxaborolan-2-yl)phenyl)-7-isopropyl-7*H*-imidazo[4,5-c] pyridazine (68.8 mg, 0.18 mmol) were used as starting materials to obtain the title compound (45 mg, 60%).

$^1$H NMR (400 MHz, CHLOROFORM-d) 9.37 (s, 1 H), 8.34 - 8.24 (m, 2 H), 8.19 (d, *J* = 4.9 Hz, 1 H), 7.85 (s, 1 H), 7.60 (s, 1 H), 7.32 (t, *J* = 9.0 Hz, 1 H), 7.08 (s, 1 H), 5.18 (br. s., 1 H), 4.19 (s, 3 H), 3.86 (s, 3 H), 1.75 (d, *J* = 6.8 Hz, 6 H). LC-MS: m/z $[M+H]^+$ = 417.

Example 50

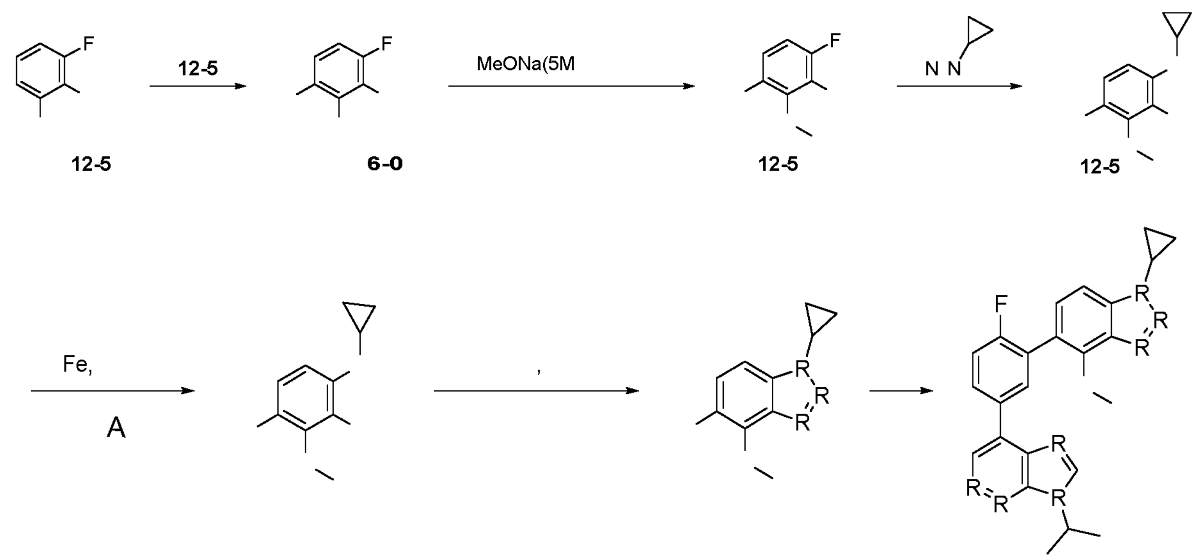


**50-1: 1-Bromo-2,4-difluoro-3-nitrobenzene**

1,3-Difluoro-2-nitrobenzene (5.0 g, 31.4 mmol) was dissolved in a mixture of concentrated sulfuric acid (10 mL) and trifluoroacetic acid (50 mL), then N-bromosuccinimide (6.7 g, 37.7 mmol) was added thereto, and the mixture was stirred at room temperature for 16 hours. The reaction mixture was poured into ice water (300 mL) and extracted with ethyl acetate (150 mL × 3). The organic phases were combined, then respectively washed with 2 mol/L sodium hydroxide solution (200 mL × 2), saturated sodium bicarbonate solution (200 mL), and saturated brine (200 mL), dried over

anhydrous sodium sulfate, and concentrated to obtain a red oily liquid (5.0 g, yield: 65%).

**50-2: 1-Bromo-4-fluoro-2-methoxy-3-nitrobenzene**

1-Bromo-2,4-difluoro-3-nitrobenzene (4.5 g, 18.9 mmol) was dissolved in methanol (50 mL), then a solution of sodium methoxide in methanol (4.0 mL, 5 mol/L) was added dropwise thereto, and the mixture was stirred at room temperature for 2 hours. The reaction mixture was then poured into ice water (200 mL) and extracted with ethyl acetate (150 mL × 3). The organic phases were combined, dried over anhydrous sodium sulfate, concentrated, and purified by column chromatography (petroleum ether: ethyl acetate = 10:1) to obtain a white solid (1.0 g, yield: 21%). $^1$H NMR (400 MHz, DMSO-$d_6$) 8.04 (dd, *J* = 9.2 Hz, 5.9 Hz, 1 H), 7.44 (t, *J* = 9.3 Hz, 1 H), 3.95 (s, 3 H).

**50-3: 4-Bromo-*N*-cyclopropyl-3-methoxy-2-nitroaniline**

1-Bromo-4-fluoro-2-methoxy-3-nitrobenzene (500 mg, 2.0 mmol), cyclopropylamine (1.14 g, 20 mmol), and dimethyl sulfoxide (5 mL) were added to a microwave tube, and the mixture was reacted under microwave irradiation at 80°C for 2 hours. The reaction mixture was poured into ice water (50 mL) and extracted with ethyl acetate (50 mL × 3). The organic phases were combined, dried over anhydrous sodium sulfate, concentrated, and purified by column chromatography (petroleum ether: ethyl acetate = 15:1) to obtain a yellow solid (300 mg, yield: 85%). LC-MS: m/z $[M+H]^+$ = 287.

**50-4: 4-Bromo-*N*$^1$-cyclopropyl-3-methoxybenzene-1,2-diamine**

4-Bromo-*N*-cyclopropyl-3-methoxy-2-nitroaniline (300.0 mg, 1.04 mmol) was dissolved in methanol (20 mL), then Raney nickel (100 mg) was added thereto, and the mixture was stirred at room temperature for 2 hours under a hydrogen balloon to complete the reaction. After filtration, the filtrate was concentrated to obtain a brown solid (256 mg, yield: 94%). LC-MS: m/z $[M+H]^+$ = 257.

**50-5: 5-Bromo-1-cyclopropyl-4-methoxy-1*H*-benzo[*d*][1,2,3]triazole**

4-Bromo-*N*$^1$-cyclopropyl-3-methoxybenzene-1,2-diamine (256 mg, 1.0 mmol) was dissolved in acetic acid (5 mL), and the mixture was added to a reaction flask. An aqueous solution (1 mL) of sodium nitrite (173 mg, 2.5 mmol) was then added dropwise to the reaction flask at 0°C, and the mixture was stirred at room temperature for 30 minutes. The reaction mixture was poured into water (30 mL), extracted with ethyl acetate (50 mL × 5), concentrated, and purified by column chromatography (petroleum ether: ethyl acetate = 2:1) to obtain a yellow solid (150 mg, yield: 56%). LC-MS: m/z $[M+H]^+$ = 268.

**50: 4-(3-(1-Cyclopropyl-4-methoxy-1*H*-benzo[*d*][1,2,3]triazol-5-yl)-4-fluorophenyl)-7-isopropyl-7*H*-imidazo[4,5-*c*]pyridazine**

Following the same experimental procedure as in Example 1, 5-bromo-1-cyclopropyl-4-methoxy-1*H*-benzo[*d*][1,2,3]triazole (58 mg, 0.22 mmol) and 4-(4-fluoro-3-(4,4,5,5-tetramethyl-1,3,2-dioxaborolan-2-yl)phenyl)-7-isopropyl-7H-imidazo[4,5-c]pyridazine (68.8 mg, 0.18 mmol) were used as starting materials to obtain the title compound (45 mg, 60%).

$^1$H NMR (400 MHz, CHLOROFORM-d) 9.38 (s, 1 H), 8.32 (s, 1 H), 8.29 - 8.20 (m, 2 H), 7.49 (d, *J = 8.3* Hz, 1 H), 7.41 - 7.31 (m, 2 H), 5.32 - 5.15 (m, 1 H), 4.60 (s, 3 H), 3.78 (d, *J* = 3.9 Hz, 1 H), 1.77 (d, *J* = 6.4 Hz, 6 H), 1.41 (br. s., 2 H), 1.33 (d, *J* = 5.9 Hz, 2 H). LC-MS: m/z $[M+H]^+$ = 444.

Example 51

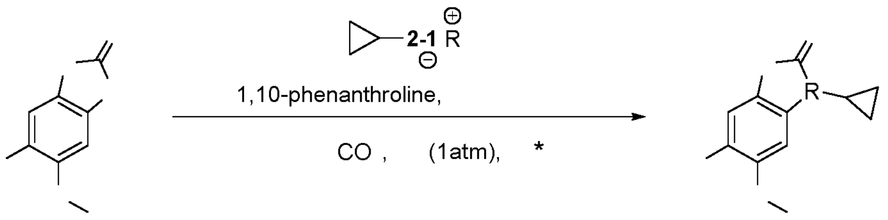


10-3

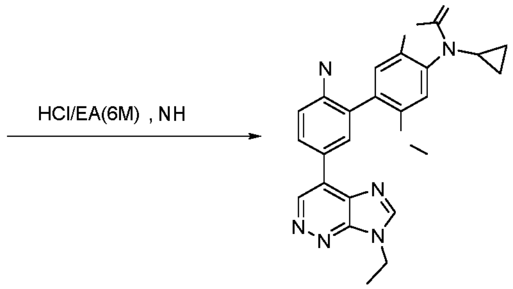


**51-1: 6-Bromo-3-cyclopropyl-5-methoxybenzo[*d*]oxazol-2(3*H*)-one**

6-Bromo-5-methoxybenzo[*d*]oxazol-2(3*H*)-one (500 mg, 2.05 mmol), copper acetate (102 mg, 0.51 mmol), 1,10-phenanthroline (56 mg, 0.15 mmol), and potassium carbonate (566 mg, 4.10 mmol) were suspended in a solution of toluene (10 mL) and water (2.5 mL). The mixture was heated to 70°C under an oxygen atmosphere (1 atm) and reacted for 16 hours. The reaction mixture was quenched by pouring into saturated ammonium chloride (100 mL), then extracted with ethyl acetate (50 mL × 3), and rotary evaporated to dryness to obtain a crude product, which was purified by prep-HPLC to obtain the title product (144 mg, white solid) with a yield of 21%. LCMS: m/z $[M+H]^+$ = 284.1.

**51: 3-Cyclopropyl-6-(5-(7-ethyl-7*H*-imidazo[4,5-*c*]pyridazin-4-yl)-2-fluorophenyl)-5-methoxybenzo[*d*]oxazol-2(3*H*)-one**

Following the same experimental procedure as in Example 1, 6-bromo-3-cyclopropyl-5-methoxybenzo[*d*]oxazol-2(3H)-one (144.0 mg, 0.51 mmol) and (5-(7-ethyl-7*H*-imidazo[4,5-c]pyridazin-4-yl)-2-fluorophenyl)boronic acid (144.9 mg, 0.51 mmol) were used as starting materials to obtain the title product (25.1 mg, white solid) with a yield of 11%.

$^1$H NMR (400 MHz, DMSO-$d_6$) δ 9.55 (s, 1 H), 8.85 (s, 1 H), 8.44 (m, 2 H), 7.48 (t, 1 H), 7.36 (s, 1 H), 7.04 (s, 1 H), 4.51 (q, *J* = 7.2 Hz, 2 H), 3.84 (s, 3 H), 3.05 (m, 1 H), 1.56 (t, *J* = 7.2 Hz, 3 H), 1.21 - 0.96 (m, 4 H). LCMS: m/z $[M+H]^+$ = 446.1.

Example 52

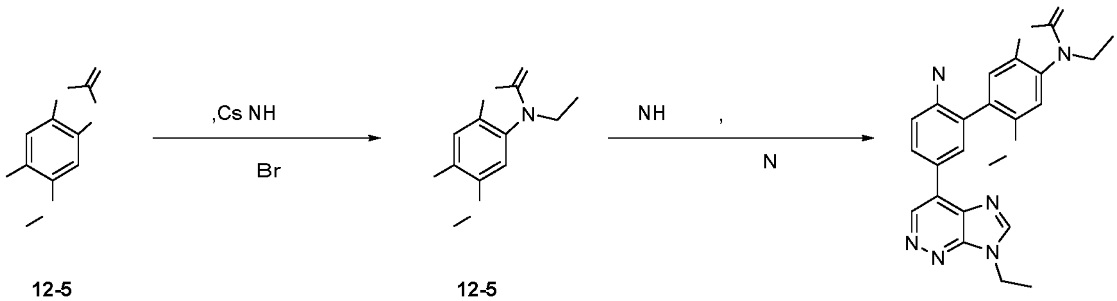


12-5 12-5

**52-1: 6-Bromo-3-ethyl-5-methoxybenzo[*d*]oxazol-2(3*H*)-one**

6-Bromo-5-methoxybenzo[d]oxazol-2(3*H*)-one (200 mg, 0.82 mmol) was added to dimethyl sulfoxide (20 mL), then iodoethane (1.97 mL, 0.82 mmol) and cesium carbonate (534.43 mg, 1.64 mmol) were added thereto, and the mixture

was stirred at 80°C for 3 hours. The reaction mixture was poured into water, extracted with ethyl acetate (40 mL × 3), concentrated, and purified by column chromatography (petroleum ether: ethyl acetate = 2:1) to obtain the title compound (175 mg, yield: 78%) as a pink solid. LC-MS: m/z $[M+H]^+$ = 272.

**52: 3-Ethyl-6-(5-(7-ethyl-7*H*-imidazo[4,5-c]pyridazin-4-yl)-2-fluorophenyl)-5-methoxybenzo[*d*]oxazol-2(3*H*)-one**

Following the same experimental procedure as in Example 1, 6-bromo-3-ethyl-5-methoxybenzo[d]oxazol-2(3H)-one (175 mg, 0.64 mmol) and 5-(7-ethyl-7*H*-imidazo[4,5-c]pyridazin-4-yl)-2-fluorophenyl)boronic acid (184.01 mg, 0.64 mmol) were used as starting materials to obtain the title compound (100 mg, yield: 36%).

$^1$H NMR (400 MHz, DMSO-$d_6$) 9.55 (s, 1 H), 8.85 (s, 1 H), 8.50 - 8.38 (m, 2 H), 7.48 (t, *J* = 9.3 Hz, 1 H), 7.39 (s, 1 H), 7.25 (s, 1 H), 4.51 (q, *J* = 7.3 Hz, 2 H), 3.94 (q, *J* = 7.2 Hz, 2H), 3.83 (s, 3 H), 1.56 (t, *J* = 7.3 Hz, 3 H), 1.32 (t, *J* = 7.2 Hz, 3 H). LC-MS: m/z $[M+H]^+$ = 434.2.

Example 53

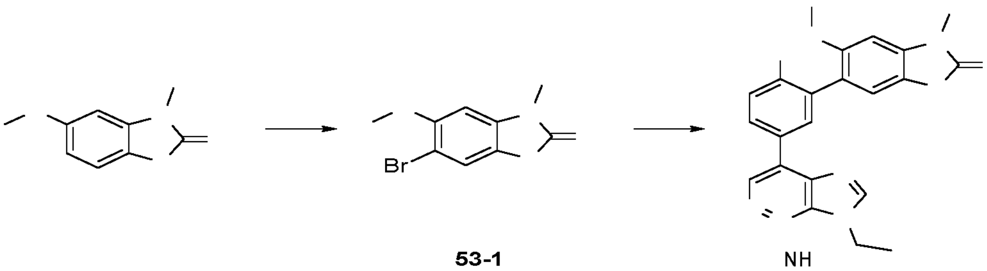

53-1

**53-1: 6-Bromo-5-methoxy-3-methylbenzo[d]thiazol-2(3H)-one**

5-Methoxy-3-methylbenzo[d]thiazol-2(3H)-one (230 mg, 1.2 mmol) was dissolved in 5 mL of acetic acid, then 2 mL of a 3 M solution of hydrogen bromide in acetic acid was added thereto, and the mixture was stirred at room temperature for 30 minutes. The reaction mixture was then cooled to 0°C, added with 1 mL of hydrogen peroxide, warmed to room temperature, and stirred for 2 hours. Water was added thereto, and a large amount of solid was precipitated. The reaction mixture was extracted with dichloromethane, dried, and concentrated to obtain the title compound (170 mg, 52%) as a white solid. LC-MS: m/z $[M+H]^+$ = 272.

**53: 6-(5-(7-Ethyl-7*H*-imidazo[4,5-c]pyridazin-4-yl)-2-fluorophenyl)-5-methoxy-3-methylbenzo[*d*]thiazol-2(3*H*)-one**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (220 mg, 0.6 mmol) and 6-bromo-5-methoxy-3-methylbenzo[d]thiazol-2(3*H*)-one (160 mg, 0.6 mmol) were used as starting materials to obtain the title compound (118 mg, 45%) as a white solid.

$^1$H NMR (400 MHz, CHLOROFORM-d) 9.28 - 9.41 (m, 1 H) 8.23 - 8.31 (m, 2 H) 8.21 (d, *J*= 6.85 Hz, 1 H) 7.41 (s, 1 H) 7.35 (t, *J* = 8.80 Hz, 1 H) 6.71 (s, 1 H) 4.59 (q, *J* = 7.17 Hz, 2 H) 3.90 (s, 3 H) 3.52 (s, 3 H) 1.68 - 1.73 (m, 3 H). LC-MS: m/z $[M+H]^+$ = 436.

Example 54

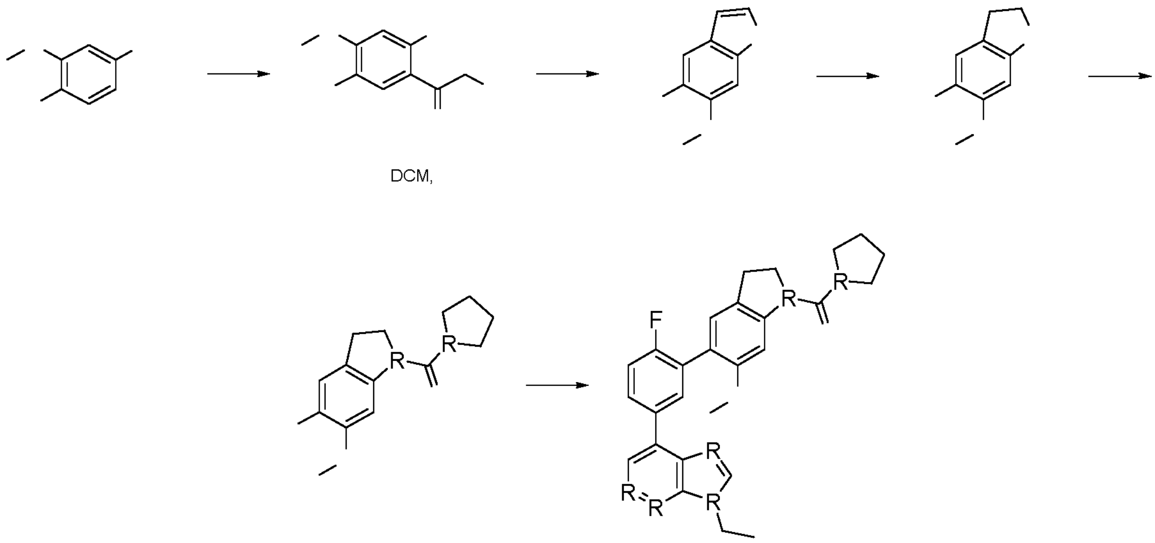


**54-1: 1-(2-Amino-5-bromo-4-methoxyphenyl)-2-chloroethan-1-one**

4-Bromo-3-methoxyaniline (CAS: 19056-40-7, 100 mg, 0.5 mmol) was added to 5 mL of 1,2-dichloroethane, then 1 M boron chloride (1.5 mL, 1.5 mmol) was added thereto, and the mixture was stirred for 10 minutes. Chloroacetonitrile (112 mg, 1.5 mmol) and zinc chloride (204 mg, 1.5 mmol) were then added thereto, and the mixture was reacted at 40°C overnight. The reaction mixture was cooled to room temperature, then neutralized with 1 M hydrochloric acid, heated to reflux for 1 hour, extracted with dichloromethane, dried, and concentrated to obtain the title compound (100 mg, 90%) as a yellow solid. LC-MS: m/z $[M+H]^+$ = 278.

**54-2: 5-Bromo-6-methoxy-1*H*-indole**

1-(2-Amino-5-bromo-4-methoxyphenyl)-2-chloroethan-1-one (500 mg, 1.8 mmol) and sodium borohydride (62 mg, 1.6 mmol) were sequentially added to 20 mL of dioxane and 2 mL of water, and the mixture was reacted at 100°C overnight. The reaction mixture was added with water, extracted with ethyl acetate, concentrated, and purified by preparative thin-layer chromatography (petroleum ether: ethyl acetate = 5:1) to obtain the title compound (170 mg, 42%) as a yellow solid. LC-MS: m/z $[M+H]^+$ = 226.

**54-3: 5-Bromo-6-methoxyindoline**

5-Bromo-6-methoxy-1*H*-indole (100 mg, 0.45 mmol) was added to dichloromethane (1 mL) and methanol (0.5 mL), and sodium cyanoborohydride (56 mg, 0.89 mmol) was added to the reaction mixture. Acetic acid (1 mL) was then added dropwise to the reaction system, and the mixture was stirred at room temperature for 2 hours. The reaction mixture was added with 10 mL of saturated sodium hydroxide aqueous solution to adjust the pH to alkaline. The reaction mixture was then extracted with ethyl acetate (50 mL × 3). The ethyl acetate phase was washed three times with water and once with saturated brine, then dried over anhydrous sodium sulfate, and concentrated to obtain the title compound (80 mg, 79%). LC-MS: m/z $[M+H]^+$ = 228.

**54-4: (5-Bromo-6-methoxyindol-1-yl)(pyrrolidin-1-yl)methanone**

5-Bromo-6-methoxyindoline (80 mg, 0.35 mmol), pyrrolidine-1-carbonyl chloride (93.5 mg, 0.7 mmol), and triethylamine (106 mg, 1.05 mmol) were added to dichloromethane (4 mL), and the mixture was stirred at 30°C overnight. The reaction mixture was directly purified by thin-layer chromatography (dichloromethane/methanol = 20/1) to obtain the title compound (20 mg, 18%). LC-MS: m/z $[M+H]^+$ = 325.

**54: *(5-(5-(7-Ethyl-7H-imidazo[4,5-c]pyridazin-4-yl)-2-fluorophenyl)-6*-methoxyindol-1-yl)(pyrrolidin-1-yl)methanone**

Following the same experimental procedure as in Example 1, (5-bromo-6-methoxyindol-1-yl)(pyrrolidin-1-yl) methanone (20 mg, 0.062 mmol) and 7-ethyl-4-(4-fluoro-3-(4,4,5,5-tetramethyl-1,3,2-dioxaborolan-2-yl)phenyl)-7*H*-imidazo[4,5-c]pyridazine (23 mg, 0.06 mmol) were used as starting materials to obtain the title compound (9 mg, 27%). LC-MS: m/z $[M+H]^+$ = 487.

$^1$H NMR (400 MHz, CHLOROFORM-d) 1.69 (br. s., 3 H) 1.94 (br. s., 4 H) 3.11 (t, *J* = 8.07 Hz, 2 H) 3.52 (br. s., 4 H) 3.82 (s, 3 H) 4.04 (t, *J*= 8.31 Hz, 2 H) 4.54 - 4.62 (m, 2 H) 7.13 (s, 2 H) 7.30 - 7.36 (m, 1 H) 8.16 (d, *J* = 6.85 Hz, 1 H) 8.21 - 8.30 (m, 2 H) 9.37 (s, 1 H).

Example 55

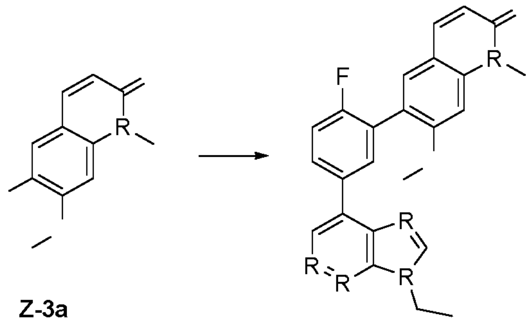

Z-3a

**55: 6-(5-(7-Ethyl-7*H*-imidazo[4,5-*c*]pyridazin-4-yl)-2-fluorophenyl)-7-methoxy-1-methylquinolin-2(1*H*)-one**

Following the same experimental procedure as in Example 1, 7-ethyl-4-(4-fluoro-3-(4,4,5,5-tetramethyl-1,3,2-dioxaborolan-2-yl)phenyl)-7H-imidazo[4,5-c]pyridazine (136 mg, 0.37 mmol) and 6-bromo-7-methoxy-1-methylquinolin-2(1*H*)-one (99 mg, 0.37 mmol) were used as starting materials to obtain the title compound (90 mg, 57%) as a white solid.

$^1$H NMR (400 MHz, CHLOROFORM-d) 9.38 (s, 1 H) 8.26 - 8.34 (m, 2 H) 8.24 (dd, *J* = 6.85, 1.96 Hz, 1 H) 7.67 (d, *J* = 9.29 Hz, 1H) 7.57 (s, 1H) 7.36 (t, *J* = 9.05 Hz, 1 H) 6.87 (s, 1 H) 6.63 (d, *J*= 9.29 Hz, 1 H) 4.59 (q, *J*= 7.34 Hz, 2 H) 3.97 (s, 3 H) 3.79 (s, 3 H) 1.68 - 1.72 (m, 3 H). LC-MS: m/z $[M+H]^+$ = 430.

Example 56

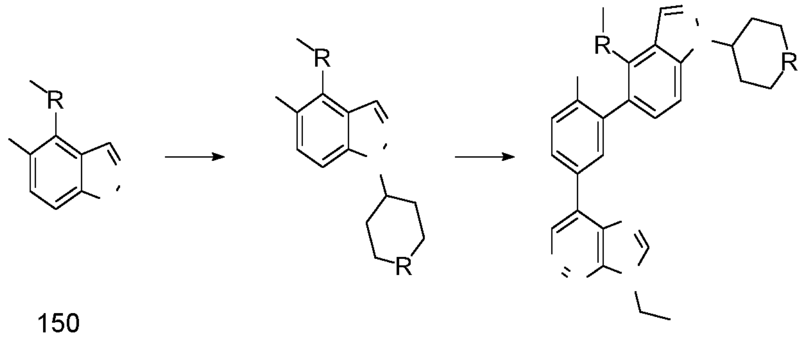

150

**56-1: 5-Bromo-4-methoxy-1-(tetrahydro-2H-pyran-4-yl)-1H-indazole**

5-Bromo-4-methoxy-1*H*-indazole (CAS: 850363-67-6, 250 mg, 1.1 mmol) was dissolved in 10 mL of *N,N*-dimethylformamide, then tetrahydro-2*H*-pyran-4-yl methanesulfonate (400 mg, 2.2 mmol) and cesium carbonate (1 g, 3.3 mmol) were added thereto, and the mixture was heated to 70°C and stirred for 3 hours. The reaction mixture was diluted with water, extracted with ethyl acetate, concentrated, and purified by preparative thin-layer chromatography (petroleum ether: ethyl acetate = 3:1) to obtain the title compound (240 mg, 70%) as a white solid. LC-MS: m/z $[M+H]^+$ = 311.

**56: 7-Ethyl-4-(4-fluoro-3-(4-methoxy-1-(tetrahydro-2H-pyran-4-yl)-1H-indazol-5-yl)phenyl)-7H-imidazo[4,5-c] pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (129 mg, 0.35 mmol) and 5-bromo-4-methoxy-1-(tetrahydro-2*H*-pyran-4-yl)-1*H*-indazole (110 mg, 0.35 mmol) were used as starting materials to obtain the title compound (50 mg, 30%) as a white solid.

$^1$H NMR (400 MHz, CHLOROFORM-d) δ ppm 9.39 (s, 1 H) 8.19 - 8.32 (m, 4 H) 7.33 - 7.45 (m, 2 H) 7.22 (d, *J*= 8.31 Hz, 1 H) 4.63 - 4.73 (m, 1 H) 4.59 (q, *J = 7.34* Hz, 2 H) 4.13 - 4.27 (m, 5 H) 3.66 (t, *J*= 11.49 Hz, 2 H) 2.45 (qd, *J* = 12.23, 4.40 Hz, 2 H) 2.04 (d, *J*= 12.72 Hz, 2 H) 1.70 (t, *J* = 7.34 Hz, 3 H). LC-MS: m/z [M+H]$^+$ = 473.

Example 57

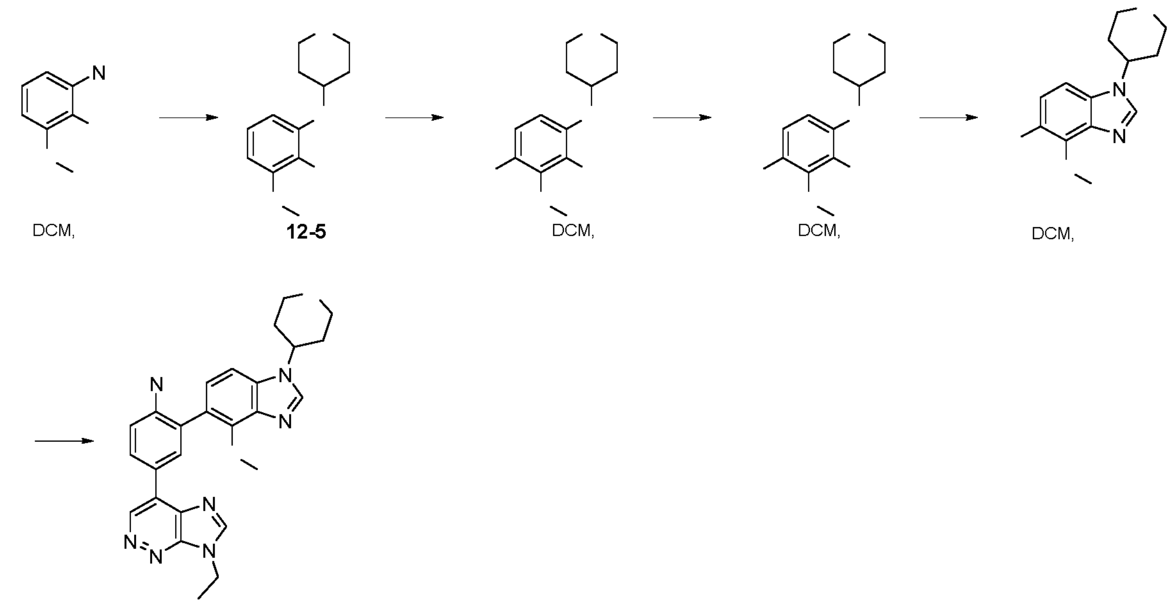


**57-1: *N-(3-Methoxy-2-nitrophenyl)tetrahydro-2H-pyran-4-amine***

1-Fluoro-3-methoxy-2-nitrobenzene (500 mg, 2.9 mmol), tetrahydro-2*H*-pyran-4-amine (590 mg, 5.8 mmol), and potassium carbonate (1200 mg, 8.8 mmol) were added to dimethyl sulfoxide (20 mL). The mixture was stirred at 90°C overnight. The reaction mixture was added with ethyl acetate (300 mL) and washed with water (200 mL × 3). The ethyl acetate phase was dried and concentrated to obtain the title compound (730 mg, 99%). LC-MS: m/z [M+H]$^+$ = 253.

**57-2: *N-(4-Bromo-3-methoxy-2-nitrophenyl)tetrahydro-2H-pyran-4-amine***

N-(3-Methoxy-2-nitrophenyl)tetrahydro-2*H*-pyran-4-amine (730 mg, 2.9 mmol) and N-bromosuccinimide (620 mg, 3.48 mmol) were added to acetonitrile (20 mL). The mixture was stirred at room temperature overnight. The reaction mixture was added with ethyl acetate (300 mL) and washed with water (200 mL × 3). The ethyl acetate phase was dried and concentrated to obtain the title compound (750 mg, 75%). LC-MS: m/z [M+H]$^+$ = 331.

**57-3: 4-Bromo-3-methoxy-*N*$^1$-(tetrahydro-2*H*-pyran-4-yl)benzene-1,2-diamine**

*N*-(4-Bromo-3-methoxy-2-nitrophenyl)tetrahydro-2H-pyran-4-amine (750 mg, 2.3 mmol) and Raney Nickel (100 mg, 1.7 mmol) were added to methanol (20 mL). The mixture was stirred at room temperature overnight. The reaction mixture was filtered and concentrated to obtain the title compound (600 mg, 88%). LC-MS: m/z [M+H]$^+$ = 301.

**57-4: 5-Bromo-4-methoxy-1-(tetrahydro-2*H*-pyran-4-yl)-1*H*-benzo[*d*]imidazole**

4-Bromo-3-methoxy-*N*[1]-(tetrahydro-2*H*-pyran-4-yl)benzene-1,2-diamine (500 mg, 1.66 mmol) was added to triethyl orthoformate (20 mL). The mixture was stirred at 90°C overnight. The reaction mixture was directly subjected to thin-layer chromatography (dichloromethane/methanol = 30/1) to obtain the title compound (400 mg, 70%). LC-MS: m/z $[M+H]^+$ = 311.

**57: 7-Ethyl-4-(4-fluoro-3-(4-methoxy-1-(tetrahydro-2*H*-pyran-4-yl)-1*H*-benzo[*d*]imidazol-5-yl)phenyl)-7*H*-imidazo[4,5-*c*]pyridazine**

Following the same experimental procedure as in Example 1, 5-bromo-4-methoxy-1-(tetrahydro-2*H*-pyran-4-yl)-1H-benzo[d]imidazole (100 mg, 0.32 mmol) and 7-ethyl-4-(4-fluoro-3-(4,4,5,5-tetramethyl-1,3,2-dioxaborolan-2-yl) phenyl)-7*H*-imidazo[4,5-c]pyridazine (118 mg, 0.32 mmol) were used as starting materials to obtain the title compound (35 mg, 22%).

$^1$H NMR (400 MHz, CHLOROFORM-d) d ppm 1.68 (t, *J* = 7.09 Hz, 3 H) 2.13 - 2.28 (m, 4 H) 3.64 (t, *J*= 11.00 Hz, 2 H) 4.20 (d, *J*= 10.27 Hz, 2 H) 4.38 (s, 3 H) 4.44 (d, *J*= 11.25 Hz, 1 H) 4.57 (d, *J*= 7.34 Hz, 2 H) 7.19 (d, *J*= 7.83 Hz, 1 H) 7.25 - 7.40 (m, 2 H) 7.98 (br. s., 1 H) 8.14 - 8.34 (m, 3 H) 9.37 (s, 1 H). LC-MS: m/z $[M+H]^+$ = 473.

Example 58

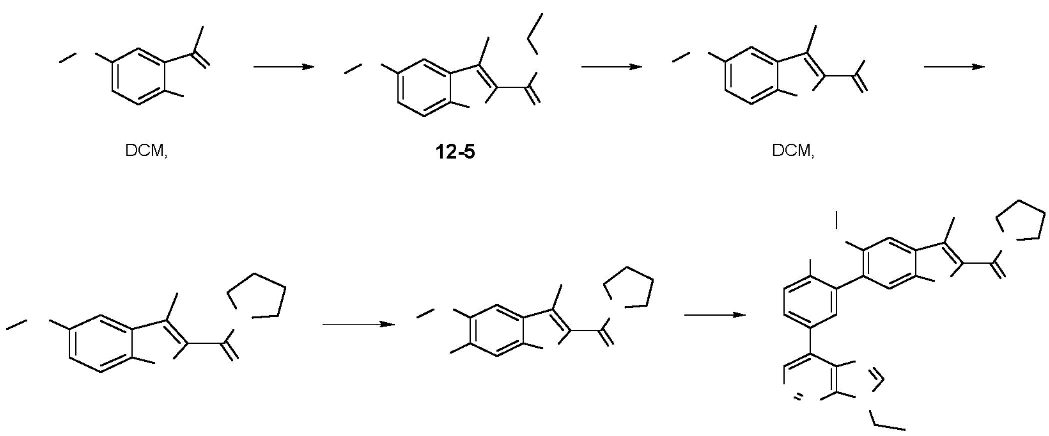


**58-1: Ethyl 5-methoxy-3-methylbenzofuran-2-carboxylate**

1-(2-Hydroxy-5-methoxyphenyl)ethan-1-one (CAS: 705-15-7, 1 g, 6 mmol) was dissolved in 10 mL of N,N-dimethylformamide under an argon atmosphere, then ethyl bromoacetate (1.3 g, 7.8 mmol) and potassium carbonate (1.7 g, 4.5 mmol) were added thereto, and the mixture was heated to 100°C and stirred overnight. The reaction mixture was diluted with water, extracted with ethyl acetate, and concentrated to obtain the title compound (1 g, 71%) as a red solid. LC-MS: m/z $[M+H]^+$ = 235.

**58-2: 5-Methoxy-3-methylbenzofuran-2-carboxylic acid**

Ethyl 5-methoxy-3-methylbenzofuran-2-carboxylate (1 g, 4.2 mmol) was dissolved in 10 mL of ethanol, then 4 M sodium hydroxide aqueous solution (10 mL) was added thereto, and the mixture was stirred at room temperature overnight. The reaction mixture was diluted with water, neutralized with 1 M hydrochloric acid, filtered to obtain the solid, and dried to obtain the title compound (600 mg, 70%) as a red solid. LC-MS: m/z $[M+H]^+$ = 207.

**58-3: (5-Methoxy-3-methylbenzofuran-2-yl)(pyrrolidin-1-yl)methanone**

5-Methoxy-3-methylbenzofuran-2-carboxylic acid (600 mg, 2.9 mmol), 2-(7-azabenzotriazol-1-yl)-*N*,*N*,*N*',*N*'-tetramethyluronium hexafluorophosphate (2.2 g, 5.8 mmol), pyrrolidine (310 mg, 4.3 mmol), and *N,N*-diisopropylethylamine (2 mL) were sequentially added to 10 mL of dichloromethane, and the mixture was stirred at room temperature for 3 hours. The reaction mixture was washed with saturated ammonium chloride aqueous solution, dried, and concentrated to

obtain the crude title compound (1 g, 100%) as a red solid. LC-MS: m/z $[M+H]^+$ = 260.

**58-4: (6-Bromo-5-methoxy-3-methylbenzofuran-2-yl)(pyrrolidin-1-yl)methanone**

(5-Methoxy-3-methylbenzofuran-2-yl)(pyrrolidin-1-yl)methanone (300 mg, 1 mmol) was dissolved in 10 mL of acetonitrile, then N-bromosuccinimide (150 mg, 1 mmol) was added thereto, and the mixture was heated to 40°C and stirred for 2 hours. The reaction mixture was purified by preparative thin-layer chromatography (petroleum ether: ethyl acetate = 3:1) to obtain the title compound (60 mg, 18%) as a white solid. LC-MS: m/z $[M+H]^+$ = 338.

**58: (6-(5-(7-Ethyl-7H-imidazo[4,5-c]pyridazin-4-yl)-2-fluorophenyl)-5-methoxy-3-methylbenzofuran-2-yl)(pyrrolidin-1-yl)methanone**

Following the same experimental procedure as in Example 1, 7-ethyl-4-(4-fluoro-3-(4,4,5,5-tetramethyl-1,3,2-dioxaborolan-2-yl)phenyl)-7H-imidazo[4,5-c]pyridazine (77 mg, 0.21 mmol) and (6-bromo-5-methoxy-3-methylbenzofuran-2-yl)(pyrrolidin-1-yl)methanone (70 mg, 0.21 mmol) were used as starting materials to obtain the title compound (15 mg, 14%) as a white solid.

$^1H$ NMR (400 MHz, CHLOROFORM-d) 9.38 (s, 1 H) 8.28 (s, 2 H) 8.19 - 8.26 (m, 1 H) 7.47 (s, 1 H) 7.32 - 7.40 (m, 1 H) 7.11 (s, 1 H) 4.54 - 4.62 (m, 2 H) 3.87 - 3.95 (m, 5 H) 3.65 - 3.72 (m, 2 H) 2.60 (s, 3 H) 1.92 - 2.02 (m, 4 H) 1.66 - 1.73 (m, 3 H). LC-MS: m/z $[M+H]^+$ = 500.

Example 59

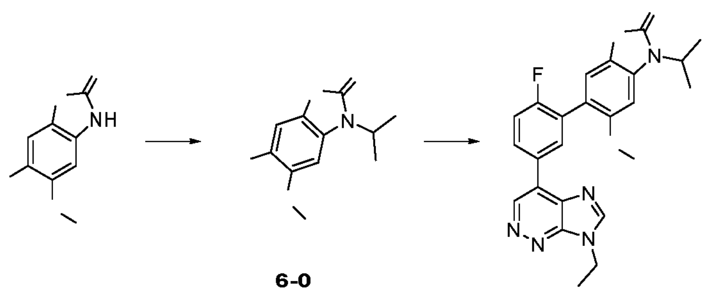

6-0

**59-1: 6- Bromo-3-isopropyl-5-methoxybenzo[*d*]oxazol-2(3*H*)-one**

6-Bromo-5-methoxybenzo[d]oxazol-2(3*H*)-one (1.0 g, 4.10 mmol), 2-iodopropane (17.4 mL, 122.95 mmol), and cesium carbonate (2.67 g, 8.20 mmol) were dissolved in dimethyl sulfoxide (20.0 mL), and the mixture was heated to 80°C in a sealed container and reacted for 16 hours. The reaction mixture was poured into water (100 mL), extracted with ethyl acetate (50 mL × 3), washed with saturated brine, dried over anhydrous sodium sulfate, and rotary evaporated to dryness to obtain a crude product, which was purified by HPLC to obtain the title product (344 mg, white solid) with a yield of 29%. $^1H$ NMR (400 MHz, DMSO-$d_6$) 7.64 (s, 1 H), 7.19 (s, 1 H), 4.52 (m, 1 H), 3.89 (s, 3 H), 1.47 (d, *J*= 6.9 Hz, 6 H). LCMS: m/z $[M+H]^+$ = 286.1.

**59: 6-(5-(7-Ethyl-7*H*-imidazo[4,5-*c*]pyridazin-4-yl)-2-fluorophenyl)-3-isopropyl-5-methoxybenzo[*d*]oxazol-2(3*H*)-one**

Following the same experimental procedure as in Example 1, 6-bromo-3-isopropyl-5-methoxybenzo[d]oxazol-2(3*H*)-one (244 mg, 0.85 mmol), 5-(7-ethyl-7*H*-imidazo[4,5-c]pyridazin-4-yl)-2-fluorophenyl)boronic acid (244 mg, 0.85 mmol), and cesium carbonate (277.9 mg, 0.85 mmol) were used as starting materials to obtain the title product (144.1 mg, white solid) with a yield of 38%.

$^1H$ NMR (400 MHz, DMSO-$d_6$) 9.55 (s, 1 H), 8.86 (s, 1 H), 8.45 (m, 2 H), 7.50 (m, 1 H), 7.39 (s, 1 H), 7.22 (s, 1 H), 4.53 (m, 3 H), 3.84 (s, 3 H), 1.55 (m, 9 H). LCMS: m/z $[M+H]^+$ = 448.

Example 60

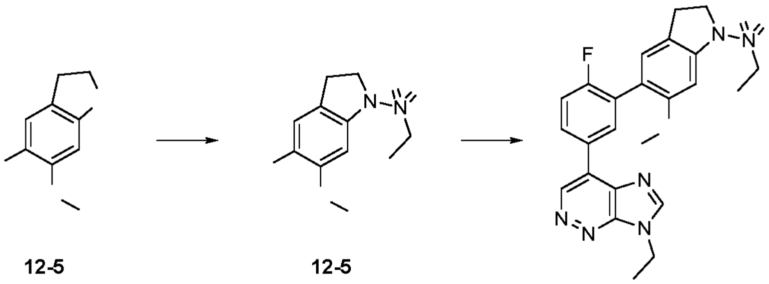

12-5 12-5

**60-1: 5-Bromo-1-(ethylsulfonyl)-6-methoxyindoline**

5-Bromo-6-methoxyindoline (70 mg, 0.31 mmol), ((chloromethyl)sulfonyl)ethane (79 mg, 0.62 mmol), and triethylamine (0.5 mL) were added to dichloromethane (3 mL). The mixture was stirred at room temperature for 3 hours. The reaction mixture was directly subjected to thin-layer chromatography (petroleum ether/ethyl acetate = 3/1) to obtain the title compound (40 mg, 40.1%). LC-MS: m/z $[M+H]^+$ = 320.

**60: 7-Ethyl-4-(3-(1-(ethylsulfonyl)-6-methoxyindol-5-yl)-4-fluorophenyl)-7*H*-imidazo[4,5-*c*]pyridazine**

Following the same experimental procedure as in Example 1, 5-bromo-1-(ethylsulfonyl)-6-methoxyindoline (40 mg, 0.125 mmol) and 7-ethyl-4-(4-fluoro-3-(4,4,5,5-tetramethyl-1,3,2-dioxaborolan-2-yl)phenyl)-7*H*-imidazo[4,5-c]pyridazine (46 mg, 0.125 mmol) were used as starting materials to obtain the title compound (20 mg, 33%).

$^1$H NMR (400 MHz, CHLOROFORM-*d*) 1.45 (t, *J* = 7.58 Hz, 3 H) 1.69 (t, *J* = 7.34 Hz, 3 H) 3.10 - 3.24 (m, 4 H) 3.83 (s, 3 H) 4.12 (t, *J*= 8.56 Hz, 2 H) 4.58 (q, *J*= 7.17 Hz, 2 H) 7.16 (d, *J*= 15.16 Hz, 2 H) 7.33 (t, *J*= 9.05 Hz, 1 H) 8.15 (d, *J = 6.36* Hz, 1 H) 8.22 - 8.33 (m, 2 H) 9.36 (s, 1 H). LC-MS: m/z $[M+H]^+$ = 482.

Example 61

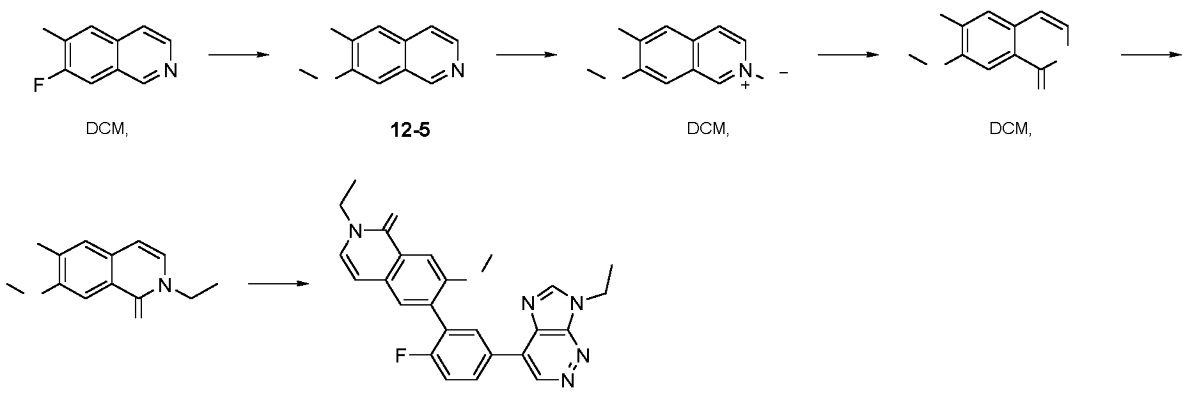


**61-1: 6-Bromo-7-methoxyisoquinoline**

Sodium methoxide (1 g, 18 mmol) was dissolved in 10 mL of methanol, then 6-bromo-7-fluoroisoquinoline (CAS: 1258833-80-5, 1 g, 4.2 mmol) was added thereto, and the mixture was heated to reflux for 2 hours. The reaction mixture was diluted with water, extracted with ethyl acetate, dried, and concentrated to obtain the title compound (800 mg, 80%) as a white solid. LC-MS: m/z $[M+H]^+$ = 238.

**61-2: 6-Bromo-7-methoxyisoquinoline 2-oxide**

6-Bromo-7-methoxyisoquinoline (800 mg, 3.9 mmol) was dissolved in 10 mL of dichloromethane, then *m*-chloroperoxybenzoic acid (1.3 g, 8 mmol) was added thereto, and the mixture was stirred at room temperature overnight. The reaction mixture was concentrated and purified by preparative thin-layer chromatography (dichloromethane: methanol = 40:1) to obtain the title compound (700 mg, 80%) as a white solid. LC-MS: m/z $[M+H]^+$ = 254.

**61-3: 6-Bromo-7-methoxyisoquinolin-1(2H)-one**

6-Bromo-7-methoxyisoquinoline 2-oxide (700 mg, 2.7 mmol) was dissolved in 10 mL of acetic anhydride, and the mixture was heated to reflux for 2 hours. After cooling, 20 mL of water was added thereto, and the mixture was heated to reflux for another 2 hours. The reaction mixture was extracted with ethyl acetate, dried over anhydrous sodium sulfate, concentrated, and purified by preparative thin-layer chromatography (dichloromethane: methanol = 20:1) to obtain the title compound (400 mg, 55%) as a reddish brown solid. LC-MS: m/z $[M+H]^+$ = 254.

**61-4: 6-Bromo-2-ethyl-7-methoxyisoquinolin-1(2H)-one**

6-Bromo-7-methoxyisoquinolin-1(2H)-one (100 mg, 0.39 mmol) was dissolved in 5 mL of methanol, then potassium carbonate (170 mg, 1.2 mmol) and 0.2 mL of iodoethane were added thereto, and the mixture was stirred at room temperature for 3 hours. The reaction mixture was added with 20 mL of water, extracted with ethyl acetate, dried over anhydrous sodium sulfate, concentrated, and purified by preparative thin-layer chromatography (dichloromethane: methanol = 20:1) to obtain the title compound (60 mg, 55%) as a yellow solid. LC-MS: m/z $[M+H]^+$ = 282.

**61: 2-Ethyl-6-(5-(7-ethyl-7*H*-imidazo[4,5-*c*]pyridazin-4-yl)-2-fluorophenyl)-7-methoxyisoquinolin-1(2*H*)-one**

Following the same experimental procedure as in Example 1, 7-ethyl-4-(4-fluoro-3-*(4,4,5,5-tetramethyl-1,3,2-dioxaborolan-2-yl)phenyl)-7H-imidazo[4,5-c]pyridazine* (78 mg, 0.21 mmol) and 6-bromo-2-ethyl-7-methoxyisoquinolin-1(2*H*)-one (60 mg, 0.21 mmol) were used as starting materials to obtain the title compound (50 mg, 54%) as a white solid.

$^1$H NMR (400 MHz, CHLOROFORM-d) 9.38 (s, 1 H) 8.20 - 8.38 (m, 3 H) 7.98 (s, 1 H) 7.55 (s, 1 H) 7.36 (t, *J*= 9.05 Hz, 1 H) 7.03 (d, *J*= 7.34 Hz, 1 H) 6.53 *(d, J= 7.34* Hz, 1 H) 4.58 (q, *J*= 7.17 Hz, 2 H) 4.11 (q, *J* = 7.34 Hz, 2 H) 3.95 (s, 3 H) 1.69 (t, *J* = 7.34 Hz, 3 H) 1.41 (t, J= 7.09 Hz, 3 H). LC-MS: m/z $[M+H]^+$ = 444.

Example 62

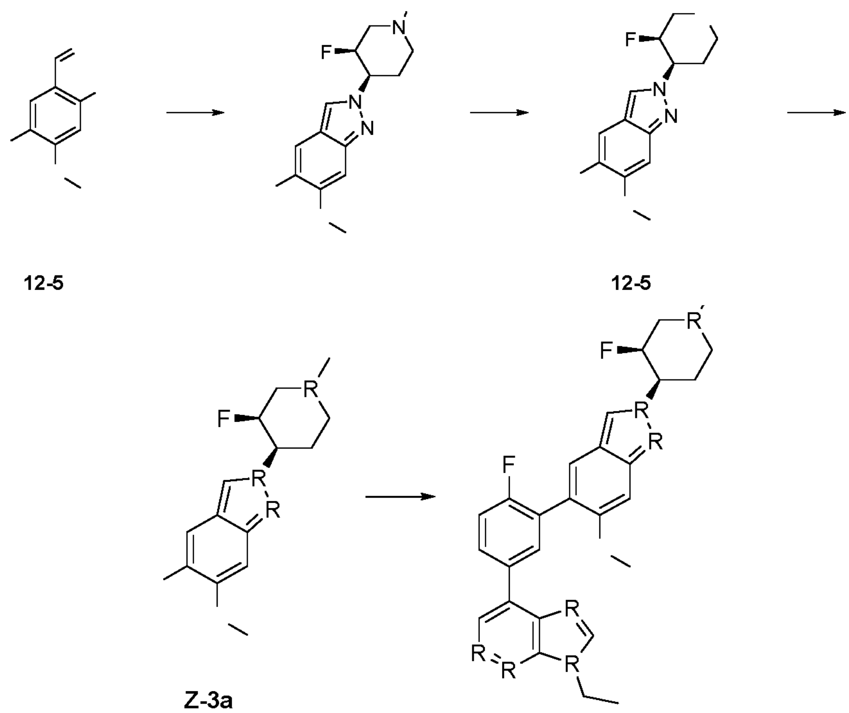

12-5

12-5

Z-3a

**62-1: *tert-Butyl tert-butyl* (3S,4R)-4-(5-bromo-6-methoxy-2H-indazol-2-yl)-3-fluoropiperidine-1-carboxylate**

Following the same experimental procedure as in the synthesis method of 40-1 and 40-2 in Example 40, 5-bromo-4-methoxy-2-nitrobenzaldehyde (300 mg, 1.15 mmol) and *tert*-butyl (3*S*,4*R*)-4-amino-3-fluoropiperidine-1-carboxylate (500 mg, 2.29 mmol) were used as starting materials to obtain the title compound (250 mg, 51%) as a light yellow oily liquid. LC-MS: m/z $[M+H]^+$ = 428, 430.

**62-2: 5-Bromo-2-((3S,4R)-3-fluoropiperidin-4-yl)-6-methoxy-2H-indazole hydrochloride**

Following the same experimental procedure as in the synthesis method of 46-3 in Example 46, *tert*-butyl (3*S*,4*R*)-4-(5-bromo-6-methoxy-2*H*-indazol-2-yl)-3-fluoropiperidine-1-carboxylate (250 mg, 0.58 mmol) was used as the starting material to obtain the title compound (203 mg, crude product) as a yellow solid. LC-MS: m/z $[M+H]^+$ = 328, 330.

**62-3: 5-Bromo-2-((3S,4R)-3-fluoro-1-methylpiperidin-4-yl)-6-methoxy-2H-indazole**

Following the same experimental procedure as in the synthesis method of 46-4 in Example 46, 5-bromo-2-((3S,4R)-3-fluoropiperidin-4-yl)-6-methoxy-2*H*-indazole hydrochloride (203 mg, 0.58 mmol) was used as the starting material to obtain the title compound (150 mg, 76%) as a yellow oily liquid. LC-MS: m/z $[M+H]^+$ = 342, 344.

**62: 7-Ethyl-4-(4-fluoro-3-(2-((3S,4R)-3-fluoro-1-methylpiperidin-4-yl)-6-methoxy-2H-indazol-5-yl)phenyl)-7H-imidazo[4,5-c]pyridazine**

Following the same experimental procedure as in Example 1, 5-bromo-2-((3*S*,4*R*)-3-fluoro-1-methylpiperidin-4-yl)-6-methoxy-2*H*-indazole (150 mg, 0.439 mmol) and 7-ethyl-4-(4-fluoro-3-(4,4,5,5-tetramethyl-1,3,2-dioxaborolan-2-yl)phenyl)-7*H*-imidazo[4,5-c]pyridazine (162 mg, 0.439 mmol) were used as starting materials to obtain the title compound (18 mg, 8%) as a brown solid.

$^1$H NMR (400 MHz, CHLOROFORM-*d*) δ 9.38 (s, 1 H), 8.27 (s, 2 H), 8.22 (d, *J* = 5.4 Hz, 1 H), 8.07 (s, 1 H), 7.65 (s, 1 H), 7.33 (t, *J*= *8.8* Hz, 1 H), 7.07 (s, 1 H), 5.37 - 5.02 (m, 1 H), 4.69 - 4.52 (m, 3 H), 3.87 (s, 3 H), 3.35 (br. s., 1 H), 3.16 (br. s., 1 H), 2.71 - 2.57 (m, 1 H), 2.49 - 2.27 (m, 5 H), 2.22 (br. s., 1 H), 1.69 (s, 3 H). LC-MS: m/z $[M+H]^+$ = 504.

Example 63

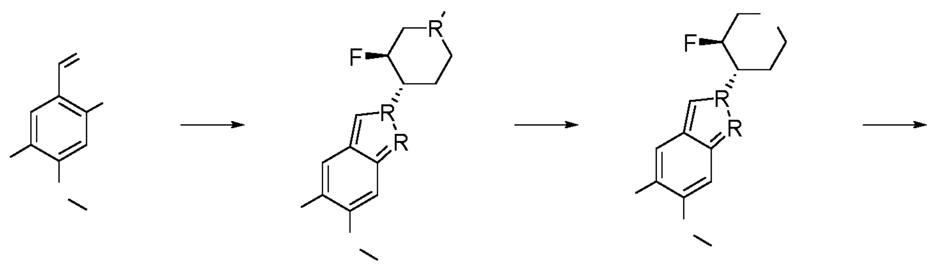

**10-3** PPA

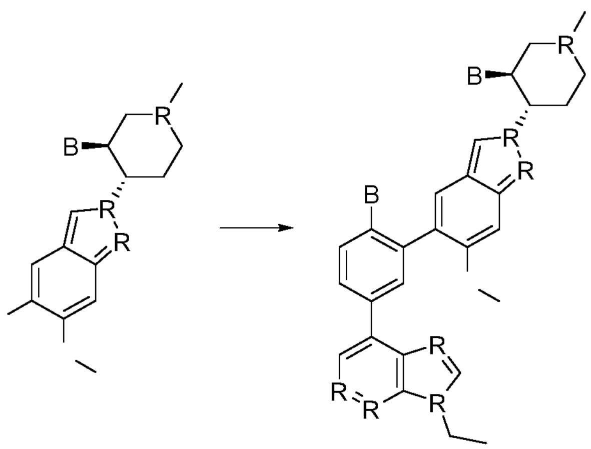

**63-1: *tert-Butyl tert-butyl* (3S,4S)-4-(5-bromo-6-methoxy-2H-indazol-2-yl)-3-fluoropiperidine-1-carboxylate**

Following the same experimental procedure as in the synthesis method of 40-1 and 40-2 in Example 40, 5-bromo-4-methoxy-2-nitrobenzaldehyde (300 mg, 1.15 mmol) and *tert*-butyl (3*S*,4*S*)-4-amino-3-fluoropiperidine-1-carboxylate (500 mg, 2.29 mmol) were used as starting materials to obtain the title compound (280 mg, 57%) as a light yellow oily liquid. LC-MS: m/z $[M+H]^+$ = 428, 430.

**63-2: 5-Bromo-2-((3S,4S)-3-fluoropiperidin-4-yl)-6-methoxy-2H-indazole hydrochloride**

Following the same experimental procedure as in the synthesis method of 46-3 in Example 46, *tert-butyl* (3*S*,4*S*)-4-(5-bromo-6-methoxy-2*H*-indazol-2-yl)-3-fluoropiperidine-1-carboxylate (280 mg, 0.65 mmol) was used as the starting material to obtain the title compound (227 mg, crude product) as a yellow solid. LC-MS: m/z $[M+H]^+$ = 328, 330.

**63-3: 5-Bromo-2-((3S,4S)-3-fluoro-1-methylpiperidin-4-yl)-6-methoxy-2H-indazole**

Following the same experimental procedure as in the synthesis method of 46-4 in Example 46, 5-bromo-2-((3S,4S)-3-fluoropiperidin-4-yl)-6-methoxy-2*H*-indazole hydrochloride (227 mg, 0.65 mmol) was used as the starting material to obtain the title compound (180 mg, 81%) as a white solid. LC-MS: m/z $[M+H]^+$ = 342, 344.

**63: 7-Ethyl-4-(4-fluoro-3-(2-((3S,4S)-3-fluoro-1-methylpiperidin-4-yl)-6-methoxy-2H-indazol-5-yl)phenyl)-7H-imidazo[4,5-c]pyridazine**

Following the same experimental procedure as in Example 1, 5-bromo-2-((3S,4S)-3-fluoro-1-methylpiperidin-4-yl)-6-methoxy-2*H*-indazole (170 mg, 0.497 mmol) and 7-ethyl-4-(4-fluoro-3-(4,4,5,5-tetramethyl-1,3,2-dioxaborolan-2-yl) phenyl)-7*H*-imidazo[4,5-*c*]pyridazine (183 mg, 0.497 mmol) were used as starting materials to obtain the title compound (89 mg, 36%) as an off-white solid.

$^1$H NMR (400 MHz, CHLOROFORM-d) δ 9.38 (s, 1 H), 8.27 (s, 2 H), 8.21 (d, *J*= 6.8 Hz, 1 H), 7.99 (s, 1 H), 7.63 (s, 1 H), 7.33 (t, *J = 9.0* Hz, 1 H), 7.13 (s, 1 H), 5.32 - 4.97 (m, 1 H), 4.57 (q, *J*= 7.0 Hz, 2 H), 4.35 (d, *J*= 11.7 Hz, 1 H), 3.87 (s, 3 H), 3.40 - 3.29 (m, 1 H), 2.99 (d, *J*= 10.8 Hz, 1 H), 2.57 - 2.35 (m, 3 H), 2.30 - 2.16 (m, 3 H), 1.68 (t, *J*= 7.3 Hz, 3 H). LC-MS: m/z $[M+H]^+$ = 504.

Example 64

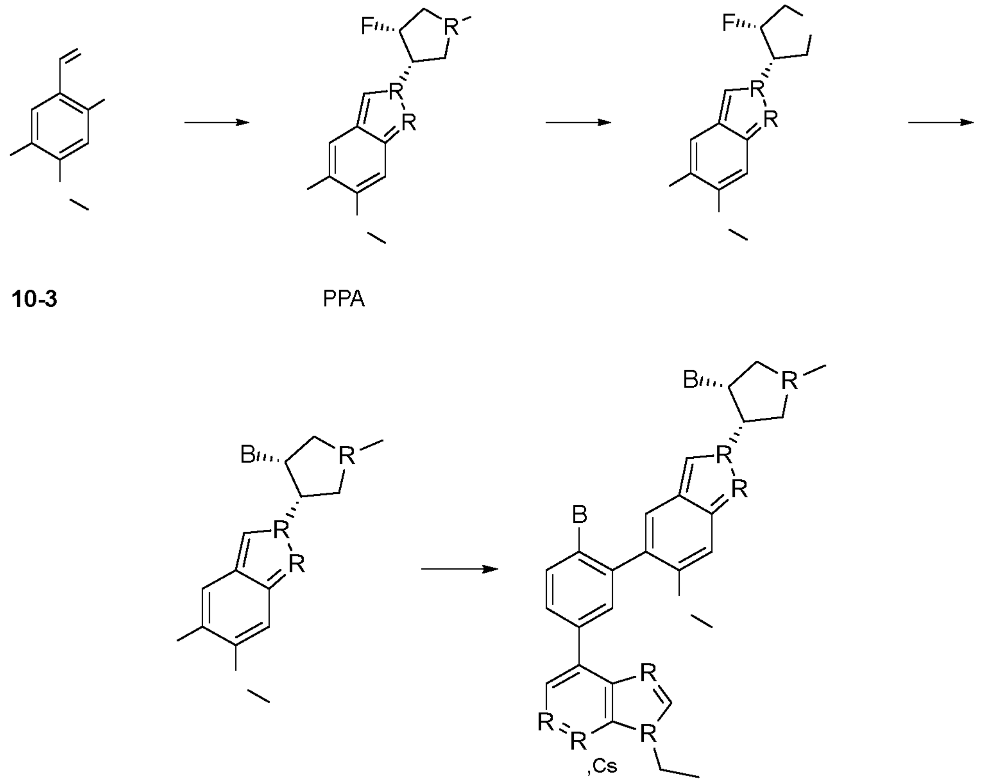


**64-1: *tert-Butyl tert-butyl* (3S,4R)-3-(5-bromo-6-methoxy-2H-indazol-2-yl)-4-fluoropyrrolidine-1-carboxylate**

Following the same experimental procedure as in the synthesis method of 40-1 and 40-2 in Example 40, 5-bromo-4-methoxy-2-nitrobenzaldehyde (500 mg, 1.92 mmol) and *tert*-butyl (3*S*,4*R*)-3-amino-4-fluoropyrrolidine-1-carboxylate (783 mg, 3.84 mmol) were used as starting materials to obtain the title compound (760 mg, 95%) as a light yellow oily liquid. LC-MS: m/z $[M+H]^+$ = 414, 416.

**64-2: 5-Bromo-2-((3S,4R)-4-fluoropyrrolidin-3-yl)-6-methoxy-2H-indazole hydrochloride**

Following the same experimental procedure as in the synthesis method of 46-3 in Example 46, *tert-butyl* (3*S*,4*R*)-3-(5-bromo-6-methoxy-2*H*-indazol-2-yl)-4-fluoropyrrolidine-1-carboxylate (760 mg, 1.84 mmol) was used as the starting material to obtain the title compound (720 mg, crude product) as a yellow solid. LC-MS: m/z $[M+H]^+$ = 314, 316.

**64-3: 5-Bromo-2-((3S,4R)-4-fluoro-1-methylpyrrolidin-3-yl)-6-methoxy-2H-indazole**

Following the same experimental procedure as in the synthesis method of 46-4 in Example 46, 5-bromo-2-((3S,4R)-4-fluoropyrrolidin-3-yl)-6-methoxy-2H-indazole hydrochloride (720 mg, 2.06 mmol) was used as the starting material to obtain the title compound (150 mg, 22%) as a yellow oily liquid. LC-MS: m/z $[M+H]^+$ = 414, 416.

64: **7-Ethyl-4-(4-fluoro-3-(2-((3S,4R)-4-fluoro-1-methylpyrrolidin-3-yl)-6-methoxy-2H-indazol-5-yl)phenyl)-7H-imidazo[4,5-c]pyridazine**

Following the same experimental procedure as in Example 1, 5-bromo-2-((3S,4R)-4-fluoro-1-methylpyrrolidin-3-yl)-6-methoxy-2*H*-indazole (150 mg, 0.46 mmol) and 7-ethyl-4-(4-fluoro-3-(4,4,5,5-tetramethyl-1,3,2-dioxaborolan-2-yl)phenyl)-7*H*-imidazo[4,5-c]pyridazine (169 mg, 0.46 mmol) were used as starting materials to obtain the title compound (60 mg, 27%) as a brown solid.

$^1$H NMR (400 MHz, CHLOROFORM-d) 9.37 (s, 1 H), 8.30 - 8.24 (m, 2 H), 8.23 - 8.18 (m, 1 H), 8.12 (s, 1 H), 7.62 (s, 1 H), 7.32 (t, *J* = 8.8 Hz, 1 H), 7.08 (s, 1 H), 5.42 - 5.19 (m, 2 H), 4.56 (q, *J* = 7.3 Hz, 2 H), 3.86 (s, 3 H), 3.37 - 3.30 (m, 1 H), 3.27 - 3.12 (m, 2 H), 3.11 - 2.96 (m, 1 H), 2.52 (s, 3 H), 1.67 (t, *J* = 7.3 Hz, 3 H). LC-MS: m/z $[M+H]^+$ = 490.

Example 65

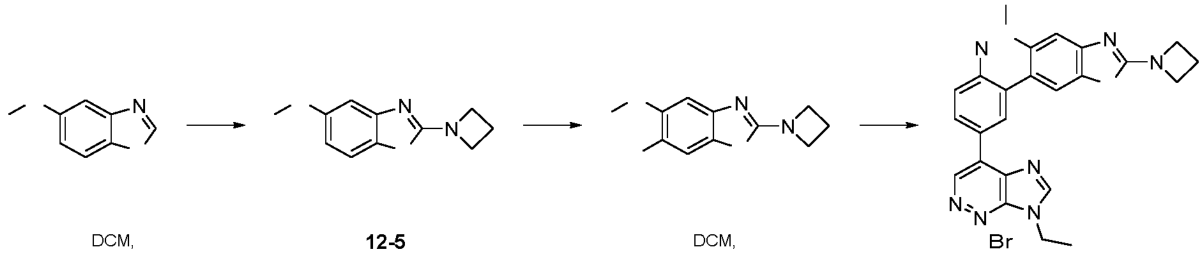


12-5

**65-1: 2-(Azetidin-1-yl)-5-methoxybenzo[*d*]oxazole**

5-Methoxybenzo[d]oxazole (400 mg, 2.7 mmol) was dissolved in 10 mL of acetonitrile, then azetidine (300 mg, 5.3 mmol), NIS (30 mg, 0.13 mmol), acetic acid (0.5 mL), and 30% hydrogen peroxide (0.6 mL) were added thereto, and the mixture was stirred at room temperature overnight. The reaction mixture was diluted with water, extracted with dichloromethane, concentrated, and purified by preparative thin-layer chromatography (petroleum ether: ethyl acetate = 2:1) to obtain the title compound (70 mg, 4%) as a brown solid. LC-MS: m/z $[M+H]^+$ = 205.

**65-2: 2-(Azetidin-1-yl)-6-bromo-5-methoxybenzo[*d*]oxazole**

2-(Azetidin-1-yl)-5-methoxybenzo[*d*]oxazole (70 mg, 0.34 mmol) was dissolved in 10 mL of acetonitrile, then *N*-bromosuccinimide (30 mg, 0.17 mmol) was added thereto, and the mixture was heated to 40°C and stirred overnight. The reaction mixture was purified by preparative thin-layer chromatography (dichloromethane: methanol = 20:1) to obtain the title compound (55 mg, 57%) as a brown solid. LC-MS: m/z $[M+H]^+$ = 283.

**65: 2-(Azetidin-1-yl)-6-(5-(7-ethyl-7*H*-imidazo[4,5-*c*]pyridazin-4-yl)-2-fluorophenyl)-5-methoxybenzo[*d*]oxazole**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-ic]pyridazine (72 mg, 0.19 mmol) and 2-(azetidin-1-yl)-6-bromo-5-methoxybenzo[d]oxazole (55 mg, 0.19 mmol) were used as starting materials to obtain the title compound (40 mg, 48%) as a white solid.

$^1$H NMR (400 MHz, CHLOROFORM-d) 9.38 (s, 1 H) 8.23 - 8.32 (m, 2 H) 8.21 (dd, *J*= 7.09, 2.20 Hz, 1 H) 7.33 (t, *J* = 9.05 Hz, 1 H) 7.26 (s, 1 H) 7.06 (s, 1 H) 4.58 (q, *J* = 7.34 Hz, 2 H) 4.32 (t, *J* = 7.58 Hz, 4 H) 3.83 (s, 3 H) 2.53 (quin, *J* = 7.58 Hz, 2 H) 1.69 (t, *J* = 7.34 Hz, 3 H). LC-MS: m/z $[M+H]^+$ = 445.

Example 66

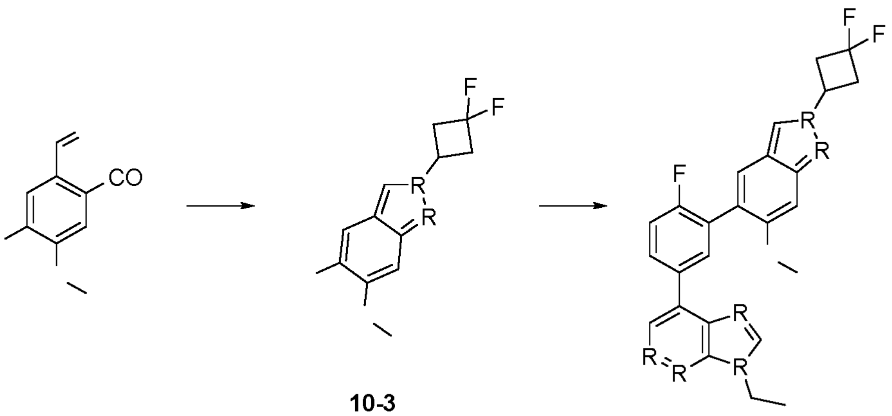

10-3

**66-1: 5-Bromo-2-(3,3-difluorocyclobutyl)-6-methoxy-2H-indazole**

Following the same experimental procedure as in the synthesis method of 40-1 and 40-2 in Example 40, 5-bromo-4-methoxy-2-nitrobenzaldehyde (200 mg, 0.77 mmol) and 3,3-difluorocyclobutan-1-amine (165 mg, 1.54 mmol) were used as starting materials to obtain the title compound (150 mg, 62%) as a light yellow oily liquid. LC-MS: m/z $[M+H]^+$ = 317, 319.

**66: 4-(3-(2-(3,3-Difluorocyclobutyl)-6-methoxy-2*H*-indazol-5-yl)-4-fluorophenyl)-7-ethyl-7*H*-imidazo[4,5-*c*]pyridazine**

Following the same experimental procedure as in Example 1, 5-bromo-2-(3,3-difluorocyclobutyl)-6-methoxy-2H-indazole (150 mg, 0.475 mmol) and 7-ethyl-4-(4-fluoro-3-(4,4,5,5-tetramethyl-1,3,2-dioxaborolan-2-yl)phenyl)-7H-imidazo[4,5-c]pyridazine (175 mg, 0.475 mmol) were used as starting materials to obtain the title compound (120 mg, 53%) as a white solid.

$^1H$ NMR (400 MHz, CHLOROFORM-*d*) $\delta$ = 9.38 (s, 1 H), 8.27 (br. s., 2 H), 8.20 (d, *J*= 6.8 Hz, 1 H), 7.95 (s, 1 H), 7.60 (s, 1 H), 7.33 (t, *J*= 8.8 Hz, 1 H), 7.13 (s, 1 H), 4.95 (d, *J* = 5.9 Hz, 1 H), 4.58 (q, *J*= 7.3 Hz, 2 H), 3.87 (s, 3 H), 3.52 - 3.37 (m, 2 H), 3.23 (d, *J*= 8.3 Hz, 2H), 1.69 (t, *J* = 7.3 Hz, 3 H). LC-MS: m/z $[M+H]^+$ = 479.

Example 67

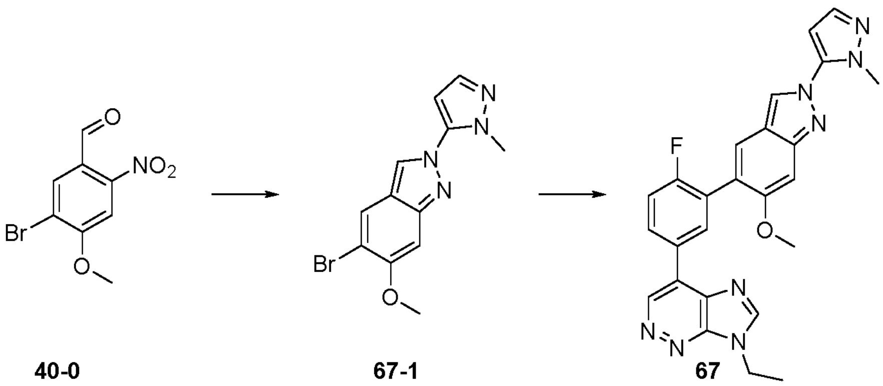

**40-0** **67-1** **67**

**67-1: 5-Bromo-6-methoxy-2-(1-methyl-1H-pyrazol-5-yl)-2H-indazole**

Following the same experimental procedure as in the synthesis method of 40-1 and 40-2 in Example 40, 5-bromo-4-methoxy-2-nitrobenzaldehyde (200 mg, 0.77 mmol) and 1-methyl-1H-pyrazol-5-amine (224 mg, 2.31 mmol) were used as starting materials to obtain the title compound (50 mg, 21%) as a white solid. LC-MS: m/z $[M+H]^+$ = 307, 309.

**67: 7-Ethyl-4-(4-fluoro-3-(6-methoxy-2-(1-methyl-1H-pyrazol-5-yl)-2H-indazol-5-yl)phenyl)-7H-imidazo[4,5-c] pyridazine**

Following the same experimental procedure as in Example 1, 5-bromo-6-methoxy-2-(1-methyl-1*H*-pyrazol-5-yl)-2*H*-indazole (50 mg, 0.16 mmol) and 7-ethyl-4-(4-fluoro-3-(4,4,5,5-tetramethyl-1,3,2-dioxaborolan-2-yl)phenyl)-7*H*-imidazo[4,5-c]pyridazine (59 mg, 0.16 mmol) were used as starting materials to obtain the title compound (12 mg, 16%) as a yellow solid.

$^1H$ NMR (400 MHz, CHLOROFORM-*d*) $\delta$ = 9.39 (s, 1 H), 8.33 - 8.26 (m, 2 H), 8.25 - 8.21 (m, 1 H), 8.14 (s, 1 H), 7.69 (s, 1 H), 7.57 (d, *J*= 2.0 Hz, 1 H), 7.35 (t, *J*= 8.8 Hz, 1 H), 7.12 (s, 1 H), 6.43 (d, *J*= 2.0 Hz, 1 H), 4.58 (q, *J* = 7.3 Hz, 2 H), 3.99 (s, 3 H), 3.90 (s, 3 H), 1.69 (t, *J*= 7.3 Hz, 3 H). LC-MS: m/z $[M+H]^+$ = 469.

Example 68

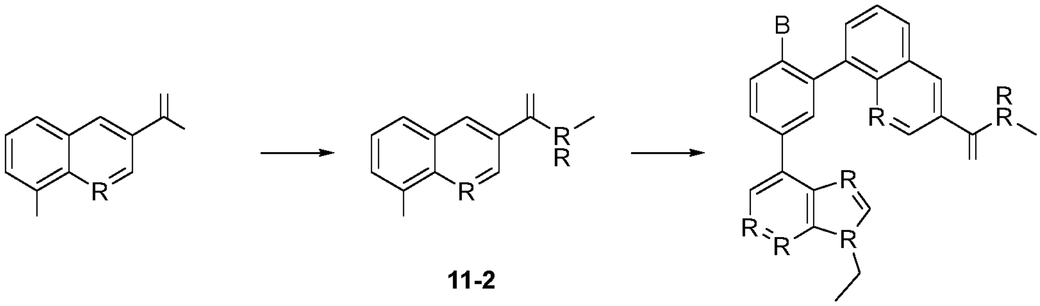

11-2

### 68-1: 8-Bromo-N-methylquinoline-3-carboxamide

8-Bromoquinoline-3-carboxylic acid (80 mg, 0.32 mmol), methylamine hydrochloride (65 mg, 0.96 mmol), 2-(7-azabenzotriazol-1-yl)-*N*,*N*,*N'*,*N'*-tetramethyluronium hexafluorophosphate (360 mg, 0.96 mmol), and triethylamine (97 mg, 0.96 mmol) were added to dichloromethane (5 mL). The mixture was stirred at 40°C overnight. The reaction mixture was directly subjected to thin-layer chromatography (dichloromethane/methanol = 20/1) to obtain the title compound (20 mg, 24%). LC-MS: m/z $[M+H]^+$ = 265.

### 68: 8-(5-(7-Ethyl-7H-imidazo[4,5-c]pyridazin-4-yl)-2-fluorophenyl)-N-methylquinoline-3-carboxamide

Following the same experimental procedure as in Example 1, 8-bromo-N-methylquinoline-3-carboxamide (20 mg, 0.076 mmol) and 7-ethyl-4-(4-fluoro-3-(4,4,5,5-tetramethyl-1,3,2-dioxaborolan-2-yl)phenyl)-7H-imidazo[4,5-c]pyridazine (28 mg, 0.076 mmol) were used as starting materials to obtain the title compound (10 mg, 31%).

$^1$H NMR (400 MHz, CHLOROFORM-d) d ppm 1.69 - 1.78 (m, 3 H) 3.10 (br. s., 3 H) 4.57 (d, *J*= 6.85 Hz, 2 H) 6.52 (br. s., 1 H) 7.43 (br. s., 1 H) 7.71 (br. s., 1 H) 7.85 - 8.06 (m, 2 H) 8.20 - 8.45 (m, 3 H) 8.70 (br. s., 1 H) 9.23 (br. s., 1 H) 9.40 (br. s., 1 H). LC-MS: m/z $[M+H]^+$ = 427.

Example 69

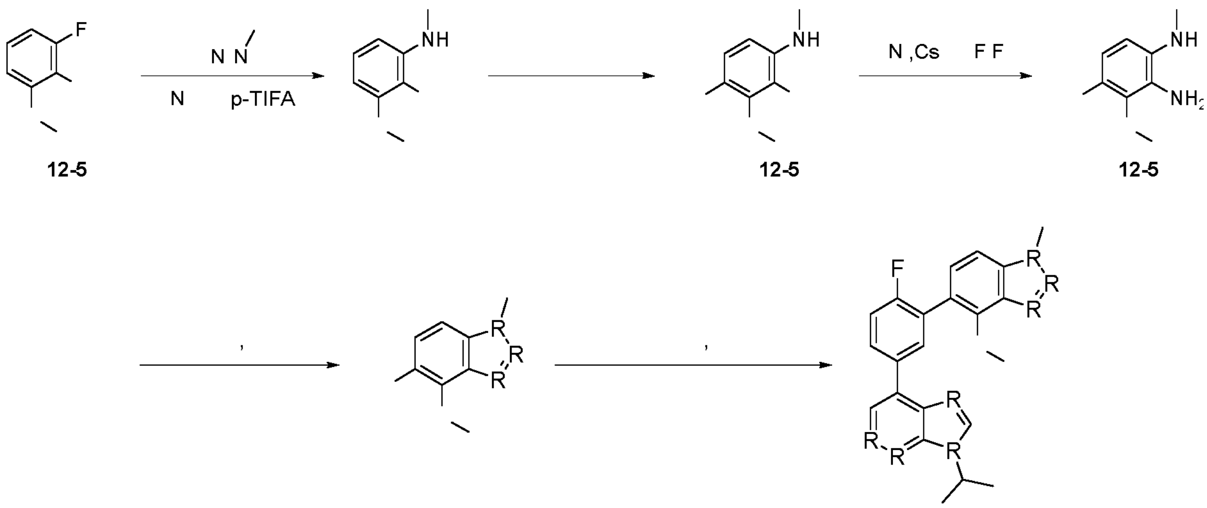


12-5 12-5 12-5

### 69-1: 3-Methoxy-N-methyl-2-nitroaniline

Following the same experimental procedure as in the synthesis method of 57-1 in Example 57, 1-fluoro-3-methoxy-2-nitrobenzene (800 mg, 4.7 mmol) and methylamine hydrochloride (635 mg, 9.4 mmol) were used as starting materials to obtain the title compound (700 mg, yield: 53%) as a red oily liquid. LC-MS: m/z $[M+H]^+$ = 183.

**69-2: 4-Bromo-3-methoxy-N-methyl-2-nitroaniline**

Following the same experimental procedure as in the synthesis method of 57-2 in Example 57, 3-methoxy-N-methyl-2-nitroaniline (700 mg, 3.8 mmol) was used as the starting material to obtain the title compound (914 mg, yield: 91%) as a yellow oily liquid. LC-MS: m/z $[M+H]^+$ = 261.

**69-3: 4-Bromo-3-methoxy-*N*$^1$-methylbenzene-1,2-diamine**

Following the same experimental procedure as in the synthesis method of 57-3 in Example 57, 4-bromo-3-methoxy-N-methyl-2-nitroaniline (914 mg, 3.5 mmol) was used as the starting material to obtain the title compound (0.116 g, yield: 55%) as a yellow solid. LC-MS: m/z $[M+H]^+$ = 231.

**69-4: 5-Bromo-4-methoxy-1-methyl-1H-benzo[d][1,2,3]triazole**

4-Bromo-3-methoxy-*N*$^1$-methylbenzene-1,2-diamine (696 mg, 3.0 mmol) was dissolved in acetic acid (7 mL), then an aqueous solution (5 mL) of sodium nitrite (414 mg, 6.0 mmol) was added dropwise to the system at 0°C, and the mixture was stirred at 0°C for 30 minutes to complete the reaction. The reaction mixture was concentrated and purified by column chromatography (petroleum ether: ethyl acetate = 1:1) to obtain the title compound (365 mg, yield: 50%) as a yellow solid. LC-MS: m/z $[M+H]^+$ = 242.

**69: 4-(4-Fluoro-3-(4-methoxy-1-methyl-1*H*-benzo[*d*][1,2,3]triazol-5-yl)phenyl)-7-isopropyl-7*H*-imidazo[4,5-*c*] pyridazine**

Following the same experimental procedure as in Example 1, 5-bromo-4-methoxy-1-methyl-1*H*-benzo[d][1,2,3] triazole (242 mg, 1.0 mmol) and (2-fluoro-5-(7-isopropyl-7*H*-imidazo[4,5-c]pyridazin-4-yl)phenyl)boronic acid (314 mg, 1.1 mmol) were used as starting materials to obtain the title compound (157 mg, yield: 38%).

$^1$H NMR (400 MHz, $CDCl_3$) 9.41 (s, 1 H), 8.38 (s, 1 H), 8.34 - 8.22 (m, 2 H), 7.48 (d, 1 H), 7.38 (t, 1 H), 7.18 (d, 1 H), 5.32 - 5.07 (m, 1 H), 4.61 (s, 3 H), 4.31 (s, 3 H), 1.77 (d, *J* = 6.7 Hz, 6 H). LC-MS: m/z $[M+H]^+$ = 418.

Example 70

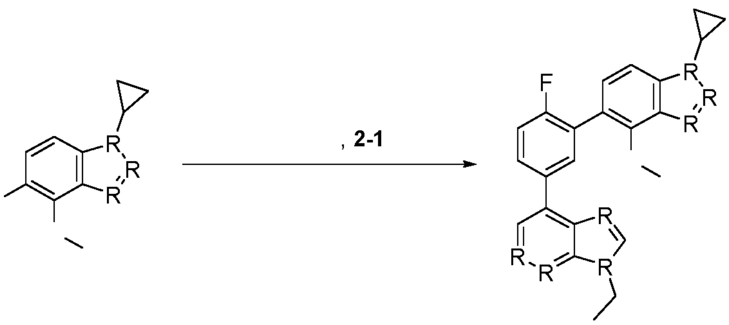


**70: 4-(3-(1-Cyclopropyl-4-methoxy-1*H*-benzo[*d*][1,2,3]triazol-5-yl)-4-fluorophenyl)-7-ethyl-7*H*-imidazo[4,5-*c*] pyridazine**

Following the same experimental procedure as in Example 1, 5-bromo-1-cyclopropyl-4-methoxy-1*H*-benzo[*d*] [1,2,3]triazole (100 mg, 0.37 mmol) and (5-(7-ethyl-7*H*-imidazo[4,5-c]pyridazin-4-yl)-2-fluorophenyl)boronic acid (126 mg, 0.45 mmol) were used as starting materials to obtain the title compound (83 mg, white solid) with a yield of 41%.

$^1$H NMR (400 MHz, $CDCl_3$) 9.41 (s, 1 H), 8.38 - 8.21 (m, 3 H), 7.48 (d, 1 H), 7.38 (t, 1 H), 7.33 (d, 1 H), 4.65 - 4.49 (m, 5 H), 3.84 - 3.72 (m, 1 H), 1.70 (t, *J*= 7.3 Hz, 3 H), 1.46 - 1.38 (m, 2 H), 1.37 - 1.30 (m, 2 H). LC-MS: m/z $[M+H]^+$ = 430.

Example 71

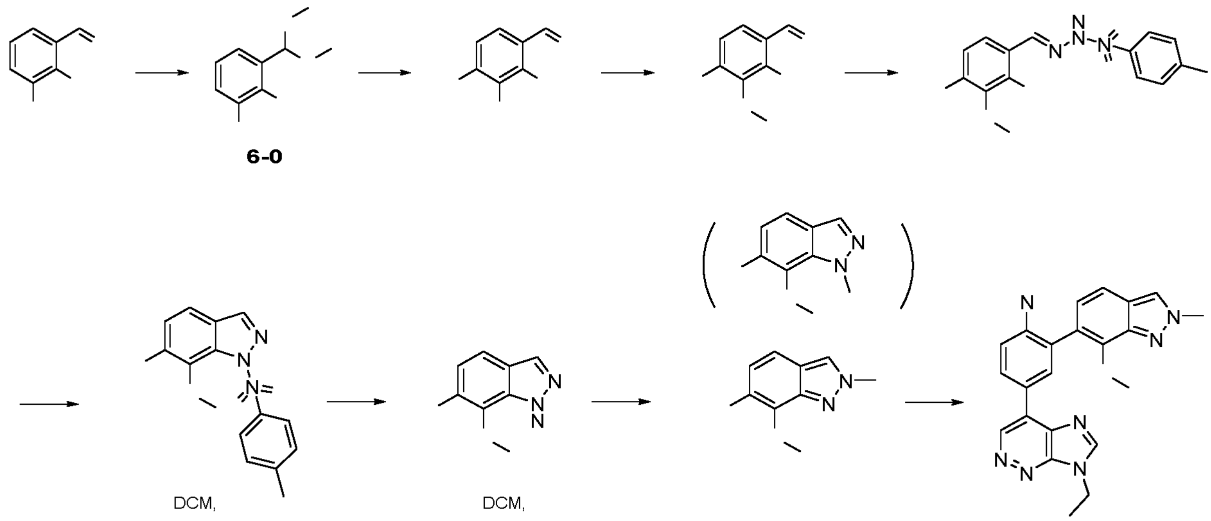


**71-1: 2-Bromo-3-(dimethoxymethyl)phenol**

2-Bromo-3-hydroxybenzaldehyde (4 g, 20 mmol) and trimethyl orthoformate (4.1 g, 40 mmol) were added to methanol (10 mL), then concentrated hydrochloric acid (0.02 mL) was added dropwise thereto, and the mixture was reacted at 60°C overnight. The reaction mixture was evaporated to dryness to obtain the crude title compound (5338 mg).

**71-2: 2,4-Dibromo-3-hydroxybenzaldehyde**

2-Bromo-3-(dimethoxymethyl)phenol (5338 mg, 20 mmol) was added to chloroform (10 mL), and the mixture was stirred at 0°C for 10 minutes. Liquid bromine (1.8 g, 12 mmol) was dissolved in chloroform (10 mL) and slowly added dropwise to the reaction mixture at 0°C. After the dropwise addition was completed, the mixture was reacted at 0°C for 1 hour. The reaction mixture was quenched with saturated sodium bicarbonate aqueous solution (50 mL), extracted with dichloromethane, and concentrated. The resulting intermediate was dissolved in dichloromethane (20 mL), then trifluoroacetic acid (2 mL) was added thereto, and the mixture was stirred at 25°C for 30 minutes. The reaction mixture was concentrated and subjected to column chromatography (petroleum ether/ethyl acetate = 6/1) to obtain the title compound (570 mg, 10%) as a yellow solid. LC-MS: m/z $[M+H]^+$ = 279, 281, 283.

**71-3: 2,4-Dibromo-3-methoxybenzaldehyde**

2,4-Dibromo-3-hydroxybenzaldehyde (570 mg, 2.036 mmol), potassium carbonate (2484 mg, 18 mmol), and iodomethane (1406 mg, 9.9 mmol) were added to acetonitrile (5 mL), and the mixture was reacted at 50°C overnight. The reaction mixture was poured into water (50 mL), extracted with ethyl acetate, and concentrated to obtain the crude title compound (688 mg) as a yellow solid. LC-MS: m/z $[M+H]^+$ = 293, 295, 297.

**71-4: *N'*-(2,4-Dibromo-3-methoxybenzylidene)-4-methylbenzenesulfonohydrazide**

2,4-Dibromo-3-methoxybenzaldehyde (688 mg, 2.340 mmol) and p-toluenesulfonyl hydrazide (1637 mg, 8.8 mmol) were added to ethanol (5 mL), and the mixture was reacted at 80°C overnight. The reaction mixture was concentrated and subjected to column chromatography (dichloromethane/methanol = 30/1) to obtain the title compound (717 mg, 66%) as a yellow solid. LC-MS: m/z $[M+H]^+$ = 461, 463, 465.

**71-5: 6-Bromo-7-methoxy-1-tosyl-1*H*-indazole**

*N'*-(2,4-Dibromo-3-methoxybenzylidene)-4-methylbenzenesulfonohydrazide (717 mg, 1.552 mmol) and cuprous oxide (343 mg, 2.4 mmol) were added to isopropanol (10 mL), and the mixture was reacted at 100°C overnight. The reaction mixture was subjected to suction filtration, concentrated, and subjected to column chromatography (dichloromethane/methanol = 30/1) to obtain the title compound (327 mg, 55%) as a brown oily liquid. LC-MS: m/z $[M+H]^+$ = 381, 383.

**71-6: 6-Bromo-7-methoxy-1H-indazole**

6-Bromo-7-methoxy-1-tosyl-1H-indazole (327 mg, 0.858 mmol) was added to methanol (6 mL), then magnesium chips (42 mg, 1.716 mmol) were added thereto, and the mixture was reacted at 0°C for 3 hours under an argon atmosphere. The reaction mixture was subjected to suction filtration, concentrated, and subjected to preparative thin-layer chromatography (petroleum ether/dichloromethane/methanol = 10/30/1) to obtain the title compound (141 mg, 72%) as a yellow solid. LC-MS: m/z $[M+H]^+$ = 227, 229.

**71-A1: 6-Bromo-7-methoxy-1-methyl-1H-indazole**

**71-A2: 6-Bromo-7-methoxy-2-methyl-2H-indazole**

6-Bromo-7-methoxy-1*H*-indazole (135 mg, 0.595 mmol), iodomethane (85 mg, 0.595 mmol), and cesium carbonate (395 mg, 1.190 mmol) were added to N,N-dimethylformamide (2 mL), and the mixture was reacted at 25°C for 15 minutes. The reaction mixture was subjected to suction filtration and subjected to preparative thin-layer chromatography (petroleum ether/ethyl acetate = 3/1) to obtain compounds (A1, 49 mg, 34.17%, Rf = 0.6) and (A2, 51 mg, 34.23%, Rf = 0.4) as white solids. LC-MS: m/z $[M+H]^+$ = 241, 243.

**71: 7-Ethyl-4-(4-fluoro-3-(7-methoxy-2-methyl-2*H*-indazol-6-yl)phenyl)-7*H*-imidazo[4,5-*c*]pyridazine**

Following the same experimental procedure as in Example 1, 7-ethyl-4-(4-fluoro-3-(4,4,5,5-tetramethyl-1,3,2-dioxaborolan-2-yl)phenyl)-7H-imidazo[4,5-c]pyridazine (61 mg, 0.166 mmol) and 6-bromo-7-methoxy-2-methyl-indazole (40 mg, 0.166 mmol) were used as starting materials to obtain the title compound (25 mg, 37%) as a light yellow solid.

$^1$H NMR (400 MHz, CHLOROFORM-d) 9.39 (s, 1 H), 8.32 - 8.25 (m, 3 H), 7.95 (s, 1 H), 7.42 - 7.34 (m, 2 H), 7.07 (d, *J* = 8.8 Hz, 1 H), 4.58 (q, *J* = 7.3 Hz, 2 H), 4.27 (s, 3 H), 4.24 (s, 3 H), 1.69 (t, *J*= 7.3 Hz, 3 H). LC-MS: m/z $[M+H]^+$ = 403.

Example 72

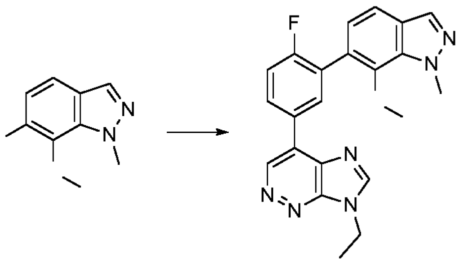

72: **7-Ethyl-4-(4-fluoro-3-(7-methoxy-1-methyl-1H-indazol-6-yl)phenyl)-7H-imidazo[4,5-*c*]pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (61 mg, 0.166 mmol) and 6-bromo-7-methoxy-1-methyl-indazole (40 mg, 0.166 mmol) were used as starting materials to obtain the title compound (14 mg, 21%) as a light yellow solid.

$^1$H NMR (400 MHz, CHLOROFORM-*d*) 9.40 (br. s., 1 H), 8.39 - 8.32 (m, 2 H), 8.29 (s, 1 H), 8.00 (s, 1 H), 7.53 (d, *J*= 8.3 Hz, 1 H), 7.41 (t, *J*= 8.8 Hz, 1 H), 7.14 (d, *J* = 7.8 Hz, 1 H), 4.58 (q, *J* = 7.3 Hz, 2H), 4.33 (s, 3 H), 3.60 (s, 3 H), 1.69 (t, *J* = 7.3 Hz, 3 H). LC-MS: m/z $[M+H]^+$ = 403.

Example 73

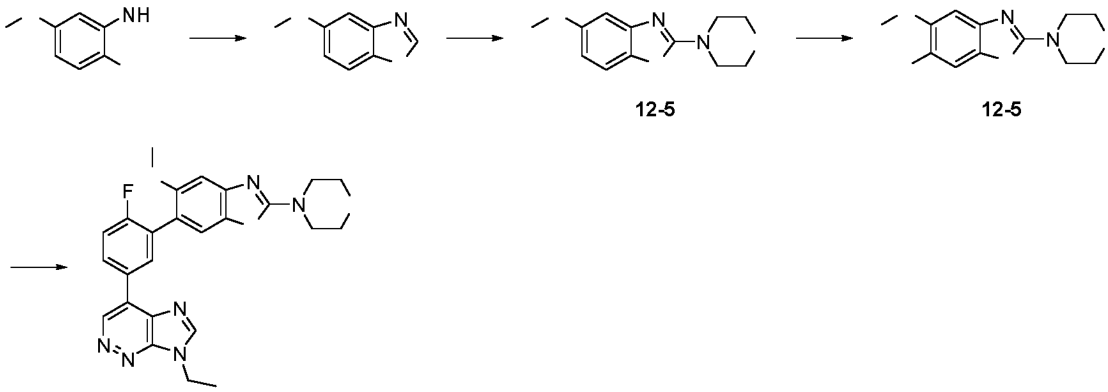

12-5 12-5

**73-1: 5-Methoxybenzo[d]oxazole**

2-Amino-4-methoxyphenol (CAS: 20734-76-3, 1 g, 7.2 mmol) was dissolved in 10 mL of toluene, then 3A molecular sieve (1 g) and triethyl orthoformate (1.5 g, 10 mmol) were added thereto, and the mixture was heated to 100°C and stirred overnight. The reaction mixture was diluted with saturated ammonium chloride aqueous solution, extracted with ethyl acetate, concentrated, and purified by column chromatography (petroleum ether: ethyl acetate = 30:1) to obtain the title compound (900 mg, 83%) as a yellow solid. LC-MS: m/z $[M+H]^+$ = 150.

**73-2: 5-Methoxy-2-morpholinobenzo[*d*]oxazole**

5-Methoxybenzo[*d*]oxazole (40 mg, 2.7 mmol) was dissolved in 10 mL of acetonitrile, then morpholine (464 mg, 5.3 mmol), *N*-iodosuccinimide (30 mg, 0.13 mmol), acetic acid (0.5 mL), and 30% hydrogen peroxide (0.6 mL) were added thereto, and the mixture was stirred at room temperature overnight. The reaction mixture was diluted with water, extracted with dichloromethane, concentrated, and purified by preparative thin-layer chromatography (petroleum ether: ethyl acetate = 5:1) to obtain the title compound (120 mg, 20%) as a white solid. LC-MS: m/z $[M+H]^+$ = 235.

**73-3: 6-Bromo-5-methoxy-2-morpholinobenzo[*d*]oxazole**

5-Methoxy-2-morpholinobenzo[*d*]oxazole (120 mg, 0.5 mmol) was dissolved in 10 mL of acetonitrile, then *N*-bromosuccinimide (108 mg, 0.6 mmol) was added thereto, and the mixture was stirred at room temperature for 30 minutes. The reaction mixture was purified by preparative thin-layer chromatography (dichloromethane) to obtain the title compound (200 mg, 100%) as a white solid. LC-MS: m/z $[M+H]^+$ = 313.

**73: 6-(5-(7-Ethyl-7*H*-imidazo[4,5-*c*]pyridazin-4-yl)-2-fluorophenyl)-5-methoxy-2-morpholinobenzo[*d*]oxazole**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-*c*]pyridazine (100 mg, 0.27 mmol) and 6-bromo-5-methoxy-2-morpholinobenzo[*d*]oxazole (100 mg, 0.27 mmol) were used as starting materials to obtain the title compound (42 mg, 33%) as a brown solid.

$^1$H NMR (400 MHz, CHLOROFORM-*d*) 9.38 (br. s., 1 H) 8.24 - 8.34 (m, 2 H) 8.11 - 8.22 (m, 1 H) 7.34 (t, *J* = 9.05 Hz, 1 H) 7.25 (s, 1 H) 7.06 (s, 1 H) 4.58 (q, *J* = 7.34 Hz, 2 H) 3.78 - 3.89 (m, 7 H) 3.67 - 3.75 (m, 4 H) 1.69 (t, *J* = 7.34 Hz, 3 H). LC-MS: m/z $[M+H]^+$ = 475.

Example 74

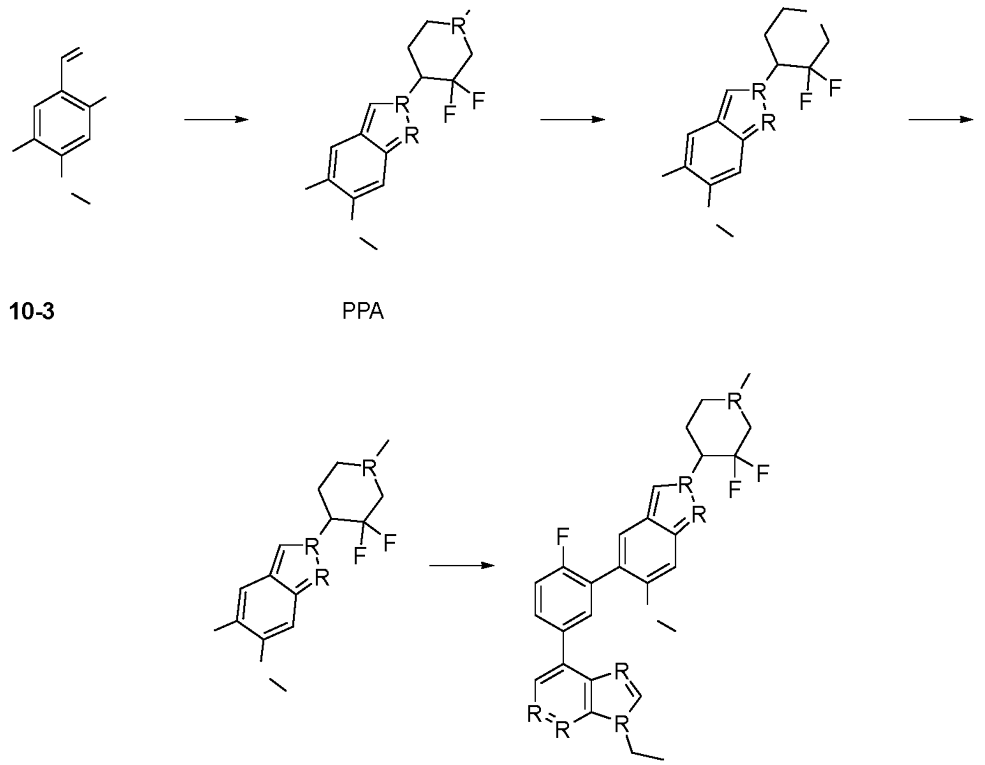

**10-3** PPA

**74-1: *tert*-Butyl 4-(5-bromo-6-methoxy-2*H*-indazol-2-yl)-3,3-difluoropiperidine-1-carboxylate**

Following the same experimental procedure as in the synthesis method of 40-1 and 40-2 in Example 40, 5-bromo-4-methoxy-2-nitrobenzaldehyde (275 mg, 1.06 mmol) and *tert*-butyl 4-amino-3,3-difluoropiperidine-1-carboxylate (250 mg, 1.06 mmol) were used as starting materials to obtain the title compound (330 mg, 70%) as a light yellow oily liquid. LC-MS: m/z $[M+H]^+$ = 446, 448.

**74-2: 5-Bromo-2-(3,3-difluoropiperidin-4-yl)-6-methoxy-2*H*-indazole hydrochloride**

Following the same experimental procedure as in the synthesis method of 46-3 in Example 46, *tert*-butyl 4-(5-bromo-6-methoxy-2*H*-indazol-2-yl)-3,3-difluoropiperidine-1-carboxylate (330 mg, 0.74 mmol) was used as the starting material to obtain the title compound (190 mg, 67%) as a yellow solid. LC-MS: m/z $[M+H]^+$ = 346, 348.

**74-3: 5-Bromo-2-(3,3-difluoro-1-methylpiperidin-4-yl)-6-methoxy-2H-indazole**

Following the same experimental procedure as in the synthesis method of 46-4 in Example 46, 5-bromo-2-(3,3-difluoropiperidin-4-yl)-6-methoxy-2H-indazole hydrochloride (190 mg, 0.50 mmol) was used as the starting material to obtain the title compound (160 mg, 90%) as a colorless oily liquid. LC-MS: m/z $[M+H]^+$ = 360, 362.

**74: 4-(3-(2-(3,3-Difluoro-1-methylpiperidin-4-yl)-6-methoxy-2*H*-indazol-5-yl)-4-fluorophenyl)-7-ethyl-7*H*-imidazo [4,5-*c*]pyridazine**

Following the same experimental procedure as in Example 1, 5-bromo-2-(3,3-difluoro-1-methylpiperidin-4-yl)-6-methoxy-2*H*-indazole (150 mg, 0.42 mmol) and 7-ethyl-4-(4-fluoro-3-(4,4,5,5-tetramethyl-1,3,2-dioxaborolan-2-yl)phenyl)-7*H*-imidazo[4,5-c]pyridazine (155 mg, 0.42 mmol) were used as starting materials to obtain the title compound (37 mg, 17%) as an off-white solid.

$^1H$ NMR (400 MHz, CHLOROFORM-*d*) 9.38 (s, 1 H), 8.28 (s, 2 H), 8.22 (d, *J*=6.4 Hz, 1 H), 8.08 (s, 1 H), 7.65 (s, 1 H), 7.33 (t, *J* = 8.8 Hz, 1 H), 7.10 (s, 1 H), 4.84 - 4.71 (m, 1 H), 4.58 (q, *J*= 7.3 Hz, 2 H), 3.87 (s, 3 H), 3.29 (br. s., 1 H), 3.13 (d, *J*= 11.7 Hz, 1 H), 2.60 - 2.52 (m, 1 H), 2.46 (s, 3 H), 2.40 - 2.30 (m, 3 H), 1.69 (t, *J* = 7.3 Hz, 3 H). LC-MS: m/z $[M+H]^+$ = 522.

Example 75

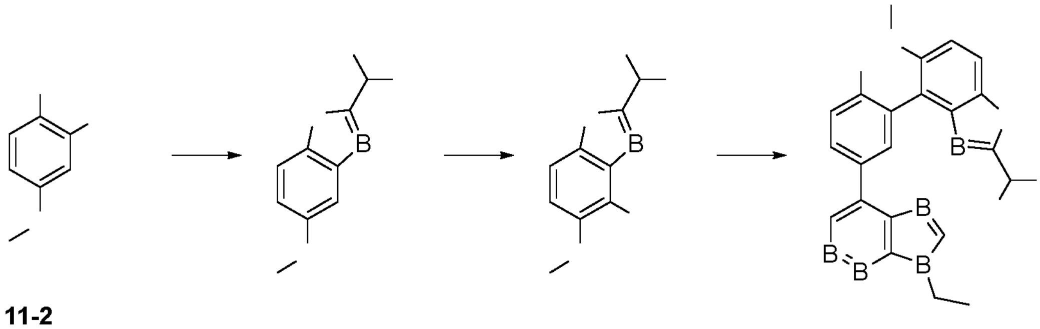

**11-2**

**75-1: 2-(Difluoromethyl)-5-methoxybenzo[dJoxazole**

2-Amino-4-methoxyphenol (500 mg, 3.59 mmol), triethylamine (725 mg, 7.18 mmol), triphenylphosphine (941 mg, 3.59 mmol), and difluoroacetic acid (344.7 mg, 3.59 mmol) were added to carbon tetrachloride (8 mL), and the mixture was stirred in a sealed container at 100°C overnight. The reaction mixture was filtered, and the mother liquor was purified by preparative thin-layer chromatography (petroleum ether/ethyl acetate = 3/1) to obtain the title compound (110 mg, 15%).

**75-2: 4-Bromo-2-(difluoromethyl)-5-methoxybenzo[d]oxazole**

2-(Difluoromethyl)-5-methoxybenzo[*d*]oxazole (100 mg, 0.5 mmol) and *N*-bromosuccinimide (90 mg, 0.5 mmol) were added to acetic acid (4 mL), and the mixture was stirred at 90°C for 2 hours. The reaction mixture was concentrated, added with an appropriate amount of methanol, and purified by preparative thin-layer chromatography (petroleum ether/ethyl acetate = 6/1) to obtain the title compound (68 mg, 49%). $^1$H NMR (400 MHz, $CDCl_3$) 7.55 (d, *J*= *8.8* Hz, 1 H), 7.13 (d, *J*= 9.3 Hz, 1 H), 6.91 - 6.69 (m, 1 H), 3.99 (s, 3 H).

**75: 2-(Difluoromethyl)-4-(5-(7-ethyl-7*H*-imidazo[4,5-*c*]pyridazin-4-yl)-2-fluorophenyl)-5-methoxybenzo[*d*]oxazole**

Following the same experimental procedure as in Example 1, 4-bromo-2-(difluoromethyl)-5-methoxybenzo[d]oxazole (60 mg, 0.215 mmol) and 7-ethyl-4-(4-fluoro-3-(4,4,5,5-tetramethyl-1,3,2-dioxaborolan-2-yl)phenyl)-7*H*-imidazo[4,5-c]pyridazine (67 mg, 0.17 mmol) were used as starting materials to obtain the title compound (40 mg, 54%).

$^1$H NMR (400 MHz, CHLOROFORM-d) 9.39 (s, 1 H), 8.39 (br. s., 1 H), 8.32 - 8.20 (m, 3 H), 7.72 - 7.62 (m, 1 H), 7.45 - 7.37 (m, 1 H), 7.24 (d, *J*= *9.8* Hz, 1 H), 6.90 - 6.62 (m, 1 H), 4.63 - 4.56 (m, 1 H), 3.90 (s, 3 H), 1.71 - 1.68 (m, 3 H). LC-MS: m/z $[M+H]^+$ = 440.

Example 76

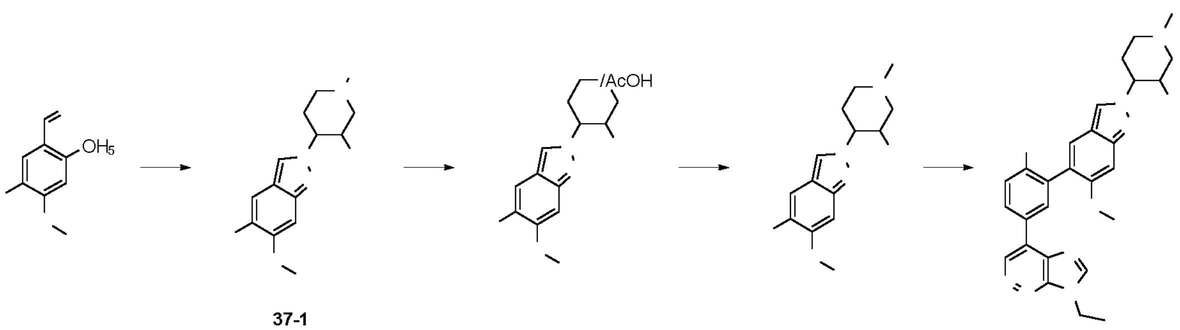


**37-1**

**76-1: *tert-Butyl* 4-(5-bromo-6-methoxy-2*H*-indazol-2-yl)-3-fluoropiperidine-1-carboxylate**

Following the same experimental procedure as in the synthesis method of 40-1 and 40-2 in Example 40, 5-bromo-4-methoxy-2-nitrobenzaldehyde (300 mg, 1.15 mmol) and *tert*-butyl 4-amino-3-fluoropiperidine-1-carboxylate (377 mg, 1.73 mmol) were used as starting materials to obtain the title compound (400 mg, 81%) as a light yellow oily liquid. LC-MS: m/z $[M+H]^+$ = 428, 430.

**76-2: 5-Bromo-2-(3-fluoropiperidin-4-yl)-6-methoxy-2H-indazole hydrochloride**

Following the same experimental procedure as in the synthesis method of 46-3 in Example 46, *tert-butyl* 4-(5-bromo-6-methoxy-2*H*-indazol-2-yl)-3-fluoropiperidine-1-carboxylate (400 mg, 0.93 mmol) was used as the starting material to obtain the title compound (290 mg, 86%) as a yellow solid. LC-MS: m/z $[M+H]^+$ = 328, 330.

**76-3: 5-Bromo-2-(3-fluoro-1-methylpiperidin-4-yl)-6-methoxy-2H-indazole**

Following the same experimental procedure as in the synthesis method of 46-4 in Example 46, 5-bromo-2-(3-fluoropiperidin-4-yl)-6-methoxy-2*H*-indazole hydrochloride (290 mg, 0.80 mmol) was used as the starting material to obtain the title compound (100 mg, 37%) as a colorless oily liquid. LC-MS: m/z $[M+H]^+$ = 342, 346.

**76: 7-Ethyl-4-(4-fluoro-3-(2-(3-fluoro-1-methylpiperidin-4-yl)-6-methoxy-2H-indazol-5-yl)phenyl)-7H-[4,5-c]pyridazine**

Following the same experimental procedure as in Example 1, 5-bromo-2-(3-fluoro-1-methylpiperidin-4-yl)-6-methoxy-2*H*-indazole (100 mg, 0.29 mmol) and 7-ethyl-4-(4-fluoro-3-(4,4,5,5-tetramethyl-1,3,2-dioxaborolan-2-yl)phenyl)-7*H*-imidazo[4,5-c]pyridazine (95 mg, 0.29 mmol) were used as starting materials to obtain the title compound (68 mg, 46%) as a yellow solid.

$^1$H NMR (400 MHz, CHLOROFORM-d) 9.38 (s, 1 H), 8.26 (br. s., 2 H), 8.22 (d, *J* = 6.4 Hz, 1 H), 7.99 (s, 1 H), 7.63 (s, 1 H), 7.32 (t, *J= 9.0* Hz, 1 H), 7.13 (s, 1 H), 5.18 - 4.98 (m, 1 H), 4.57 (q, *J* = 6.8 Hz, 2 H), 4.35 (d, *J* = 9.3 Hz, 1 H), 3.87 (s, 3 H), 3.40 - 3.30 (m, 1 H), 2.98 (d, *J*= 10.3 Hz, 1 H), 2.56 - 2.38 (m, 4 H), 2.30 - 2.16 (m, 3 H), 1.68 (t, *J*= 7.1 Hz, 3 H). LC-MS: m/z $[M+H]^+$ = 504.

Example 77

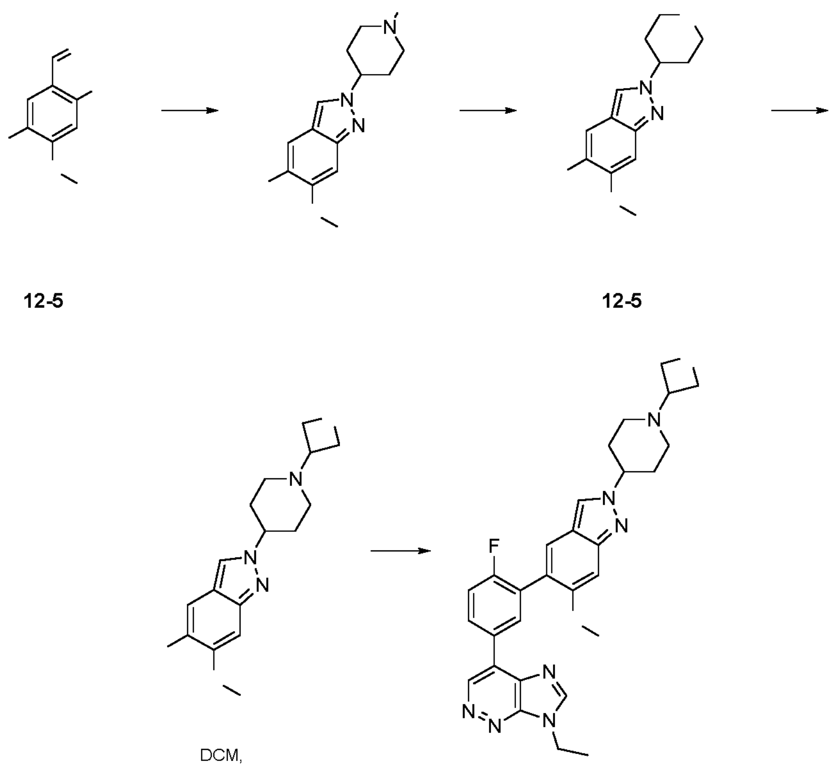


12-5 12-5

77-1: ***tert-Butyl* 4-(5-bromo-6-methoxy-2H-indazol-2-yl)piperidine-1-carboxylate**

Following the same experimental procedure as in the synthesis method of 40-1 and 40-2 in Example 40, 5-bromo-4-methoxy-2-nitrobenzaldehyde (300 mg, 1.15 mmol) and *tert*-butyl 4-aminopiperidine-1-carboxylate (345 mg, 1.73 mmol) were used as starting materials to obtain the title compound (300 mg, 64%) as a light yellow oily liquid. LC-MS: m/z $[M+H]^+$ = 410, 412.

**77-2: 5-Bromo-6-methoxy-2-(piperidin-4-yl)-2H-indazole hydrochloride**

Following the same experimental procedure as in the synthesis method of 46-3 in Example 46, *tert-butyl* 4-(5-bromo-6-methoxy-2H-indazol-2-yl)piperidine-1-carboxylate (300 mg, 0.73 mmol) was used as the starting material to obtain the title compound (251 mg, 100%) as a white solid. LC-MS: m/z $[M+H]^+$ = 310, 312.

**77-3: 5-Bromo-6-methoxy-2-(1-(oxetan-3-yl)piperidin-4-yl)-2*H*-indazole**

Following the same experimental procedure as in the synthesis method of 46-4 in Example 46, 5-bromo-6-methoxy-2-(piperidin-4-yl)-2*H*-indazole hydrochloride (280 mg, 0.81 mmol) and oxetan-3-one (62 mg, 1.62 mmol) were used as starting materials to obtain the title compound (170 mg, 58%) as a yellow solid. LC-MS: m/z $[M+H]^+$ = 366, 368.

**77: 7-Ethyl-4-(4-fluoro-3-(6-methoxy-2-(1-(oxetan-3-yl)piperidin-4-yl)-2H-indazol-5-yl)phenyl)-7H-imidazo[4,5-c] pyridazine**

Following the same experimental procedure as in Example 1, 5-bromo-6-methoxy-2-(1-(oxetan-3-yl)piperidin-4-yl)-2*H*-indazole (70 mg, 0.19 mmol) and 7-ethyl-4-(4-fluoro-3-(4,4,5,5-tetramethyl-1,3,2-dioxaborolan-2-yl)phenyl)-7*H*-imidazo[4,5-c]pyridazine (70 mg, 0.19 mmol) were used as starting materials to obtain the title compound (28 mg, 28%) as a white solid.

$^1$H NMR (400 MHz, CHLOROFORM-*d*) 9.37 (s, 1 H), 8.31 - 8.24 (m, 2 H), 8.20 (d, *J*= 5.4 Hz, 1 H), 7.93 (s, 1 H), 7.62 (s, 1 H), 7.32 (t, *J*= 8.8 Hz, 1 H), 7.10 (s, 1 H), 4.67 (td, *J* = 6.4, 18.1 Hz, 4 H), 4.56 (q, *J*= 7.3 Hz, 2 H), 4.46 - 4.37 (m, 1 H), 3.86 (s, 3 H), 3.56 (quin, *J* = 6.4 Hz, 1 H), 2.93 (d, *J* = 11.2 Hz, 2 H), 2.36 - 2.28 (m, 2 H), 2.28 - 2.16 (m, 2 H), 2.13 - 2.04 (m, 2 H), 1.68 (t, *J*= *7.3* Hz, 3 H). LC-MS: m/z $[M+H]^+$ = 528.

Example 78

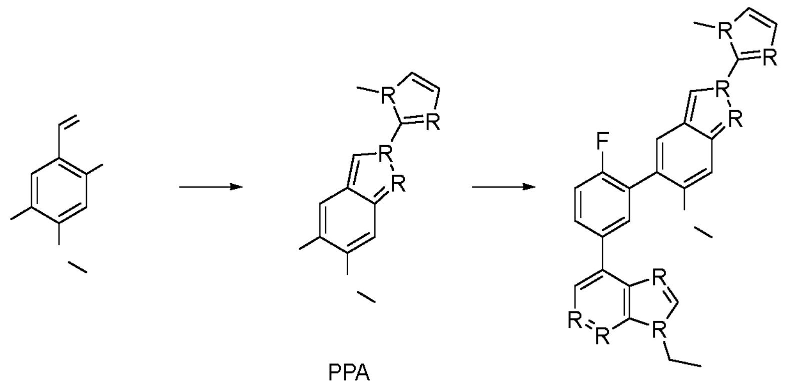

PPA

**78-1: 5-Bromo-6-methoxy-2-(1-methyl-1*H*-imidazol-2-yl)-2*H*-indazole**

Following the same experimental procedure as in the synthesis method of 40-1 and 40-2 in Example 40, 5-bromo-4-methoxy-2-nitrobenzaldehyde (200 mg, 0.77 mmol) and 1-methyl-1H-imidazol-2-amine (112 mg, 3.45 mmol) were used as starting materials to obtain the title compound (50 mg, 54%) as a light yellow oily liquid. LC-MS: m/z $[M+H]^+$ = 307, 309.

78: **7-Ethyl-4-(4-fluoro-3-(6-methoxy-2-(1-methyl-1*H*-imidazol-2-yl)-2*H*-indazol-5-yl)phenyl)-7*H*-imidazo[4,5-*c*] pyridazine**

Following the same experimental procedure as in Example 1, 5-bromo-6-methoxy-2-(1-methyl-1*H*-imidazol-2-yl)-2*H*-indazole (50 mg, 0.16 mmol) and 7-ethyl-4-(4-fluoro-3-(4,4,5,5-tetramethyl-1,3,2-dioxaborolan-2-yl)phenyl)-7H-imidazo[4,5-c]pyridazine (59 mg, 0.16 mmol) were used as starting materials to obtain the title compound (6 mg, 8%) as a yellow solid.

$^1$H NMR (400 MHz, CHLOROFORM-d) 9.39 (s, 1 H), 8.58 (s, 1 H), 8.30 - 8.24 (m, 3 H), 7.70 (s, 1 H), 7.34 (t, *J= 8.8* Hz, 1 H), 7.09 (s, 1 H), 7.03 (s, 1 H), 6.94 (s, 1 H), 4.58 (q, *J* = 7.3 Hz, 2 H), 4.00 (s, 3 H), 3.90 (s, 3 H), 1.69 (t, *J* = 7.3 Hz, 3 H). LC-MS: m/z $[M+H]^+$ = 469.

Example 79

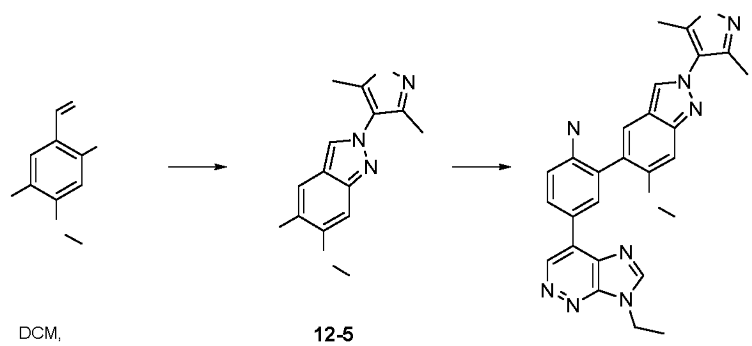

DCM,

**12-5**

**79-1: 4-(5-Bromo-6-methoxy-2H-indazol-2-yl)-3,5-dimethylisoxazole**

Following the same experimental procedure as in the synthesis method of 40-1 and 40-2 in Example 40, 5-bromo-4-methoxy-2-nitrobenzaldehyde (300 mg, 1.15 mmol) and 3,5-dimethylisoxazol-4-amine (386 mg, 3.45 mmol) were used as starting materials to obtain the title compound (200 mg, 54%) as a light yellow oily liquid. LC-MS: m/z $[M+H]^+$ = 322, 324.

**79: 4-(5-(5-(7-Ethyl-7H-imidazo[4,5-c]pyridazin-4-yl)-2-fluorophenyl)-6-methoxy-2H-indazol-2-yl)-3,5-dimethylisoxazole**

Following the same experimental procedure as in Example 1, 4-(5-bromo-6-methoxy-2H-indazol-2-yl)-3,5-dimethylisoxazole (200 mg, 0.62 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (228 mg, 0.62 mmol) were used as starting materials to obtain the title compound (28 mg, 9%) as a white solid.

$^1$H NMR (400 MHz, CHLOROFORM-d) 9.38 (s, 1 H), 8.33 - 8.26 (m, 2 H), 8.22 (d, *J*= 6.4 Hz, 1 H), 8.00 (s, 1 H), 7.68 (s, 1 H), 7.34 (t, *J= 8.8* Hz, 1 H), 7.12 (s, 1 H), 4.57 (q, *J* = 7.3 Hz, 2 H), 3.89 (s, 3 H), 2.51 (s, 3 H), 2.34 (s, 3 H), 1.68 (t, *J* = 7.1 Hz, 3 H). LC-MS: m/z $[M+H]^+$ = 484.

Example 80

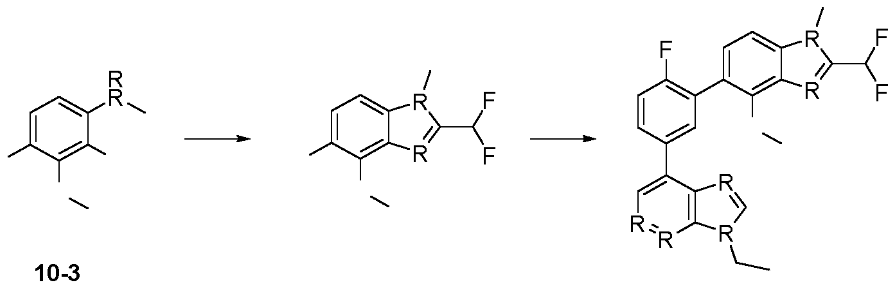

**10-3**

**80-1: 5-Bromo-2-(difluoromethyl)-4-methoxy-1-methyl-1*H*-benzo[*d*]imidazole**

4-Bromo-3-methoxy-*N*$^1$-methylbenzene-1,2-diamine (200 mg, 0.87 mmol), pyrogallol (20 mg, 0.159 mmol), and 2,2-difluoroethan-1-amine (211 mg, 2.61 mmol) were sequentially added to methanol (5 mL), and the mixture was stirred at 80°C for 16 hours. The reaction mixture was concentrated and separated by preparative thin-layer chromatography (petroleum ether/ethyl acetate = 3/1) to obtain the title compound (30 mg, 49%) as a brown solid. LC-MS: m/z $[M+H]^+$ = 291, 293.

**80: 4-(3-(2-(Difluoromethyl)-4-methoxy-1-methyl-1*H*-benzo[*d*]imidazol-5-yl)-4-fluorophenyl)-7-ethyl-7*H*-imidazo [4,5-*c*]pyridazine**

Following the same experimental procedure as in Example 1, 5-bromo-2-(difluoromethyl)-4-methoxy-1-methyl-1H-benzo[d]imidazole (30 mg, 0.1 mmol) and 7-ethyl-4-(4-fluoro-3-(4,4,5,5-tetramethyl-1,3,2-dioxaborolan-2-yl)phenyl)-7H-imidazo[4,5-c]pyridazine (37 mg, 0.1 mmol) were used as starting materials to obtain the title compound (2 mg, 4%) as a yellow solid.

$^1$H NMR (400 MHz, CHLOROFORM-d) 9.40 (br. s., 1 H), 8.28 (s, 2 H), 8.23 (d, *J* = 6.8 Hz, 1 H), 7.42 - 7.35 (m, 2 H), 7.18 (d, *J*= 8.3 Hz, 1 H), 7.08 - 6.80 (m, 1 H), 4.58 (q, *J* = 7.3 Hz, 2 H), 4.37 (s, 3 H), 3.99 (s, 3 H), 1.71 - 1.67 (m, 3 H). LC-MS: m/z $[M+H]^+$ = 453.

Example 81

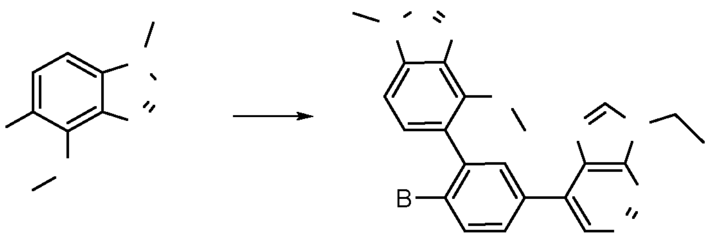

**81: 7-Ethyl-4-(4-fluoro-3-(4-methoxy-1-methyl-1*H*-benzo[*d*][1,2,3]triazol-5-yl)phenyl)-7*H*-imidazo[4,5-*c*]pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (90 mg, 0.24 mmol) and 5-bromo-4-methoxy-1-methyl-1H-benzo [d][1,2,3]triazole (60 mg, 0.24 mmol) were used as starting materials to obtain the title compound (48 mg, 48%) as a brown solid.

$^1$H NMR (400 MHz, CHLOROFORM-d) 9.39 (s, 1 H), 8.34 - 8.19 (m, 3 H), 7.49 (d, *J*= 8.3 Hz, 1 H), 7.37 (t, *J*= 9.0 Hz, 1 H), 7.17 (d, *J*= 8.8 Hz, 1 H), 4.64 - 4.52 (m, 5 H), 4.34 - 4.28 (m, 3 H), 1.70 - 1.66 (m, 3 H). LC-MS: m/z [M+H]$^+$ = 404.

Example 82

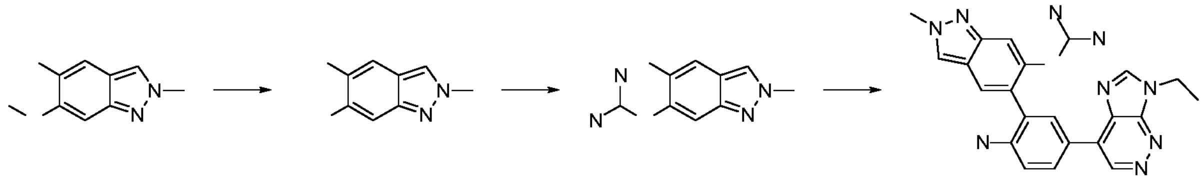

12-5

**82-1: 5-Bromo-2-methyl-2H-indazol-6-ol**

5-Bromo-6-methoxy-2-methyl-2*H*-indazole (500 mg, 2 mmol) was dissolved in 10 mL of dichloromethane, then a 1 M solution of boron tribromide in dichloromethane (5 mL) was added thereto, and the mixture was stirred at room temperature overnight. The reaction mixture was concentrated and purified by preparative thin-layer chromatography (dichloromethane: methanol = 20:1) to obtain the title compound (800 mg, 100%) as a yellow solid. LC-MS: m/z [M+H]$^+$ = 227.

**82-2: 5-Bromo-6-(difluoromethoxy)-2-methyl-2*H*-indazole**

5-Bromo-2-methyl-2*H*-indazol-6-ol (500 mg, 2 mmol) was dissolved in 10 mL of acetonitrile, then potassium hydroxide aqueous solution (200 mg dissolved in 5 mL) was added thereto, and the mixture was cooled to 0°C. Sodium difluorochloroacetate (600 mg, 4 mmol) was added thereto, and the mixture was stirred for 1 hour. The phases were separated, concentrated, and purified by preparative thin-layer chromatography (petroleum ether: ethyl acetate = 1:1) to obtain the title compound (160 mg, 29%) as a yellow solid. LC-MS: m/z [M+H]$^+$ = 277.

**82: 4-(3-(6-(Difluoromethoxy)-2-methyl-2*H*-indazol-5-yl)-4-fluorophenyl)-7-ethyl-7*H*-imidazo[4,5-*c*]pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (90 mg, 0.24 mmol) and 5-bromo-6-(difluoromethoxy)-2-methyl-2H-indazole (66 mg, 0.24 mmol) were used as starting materials to obtain the title compound (47 mg, 49%) as a brown solid.

$^1$H NMR (400 MHz, CHLOROFORM-d) 9.39 (s, 1 H), 8.36 - 8.20 (m, 3 H), 7.96 (s, 1 H), 7.74 (s, 1 H), 7.50 (s, 1 H), 7.36 (t, *J* = *9.0* Hz, 1 H), 6.75 - 6.31 (m, 1 H), 4.58 (q, *J* = 7.3 Hz, 2 H), 4.25 (s, 3 H), 1.69 (s, 3 H). LC-MS: m/z [M+H]$^+$ = 439.

Example 83 and Example 84

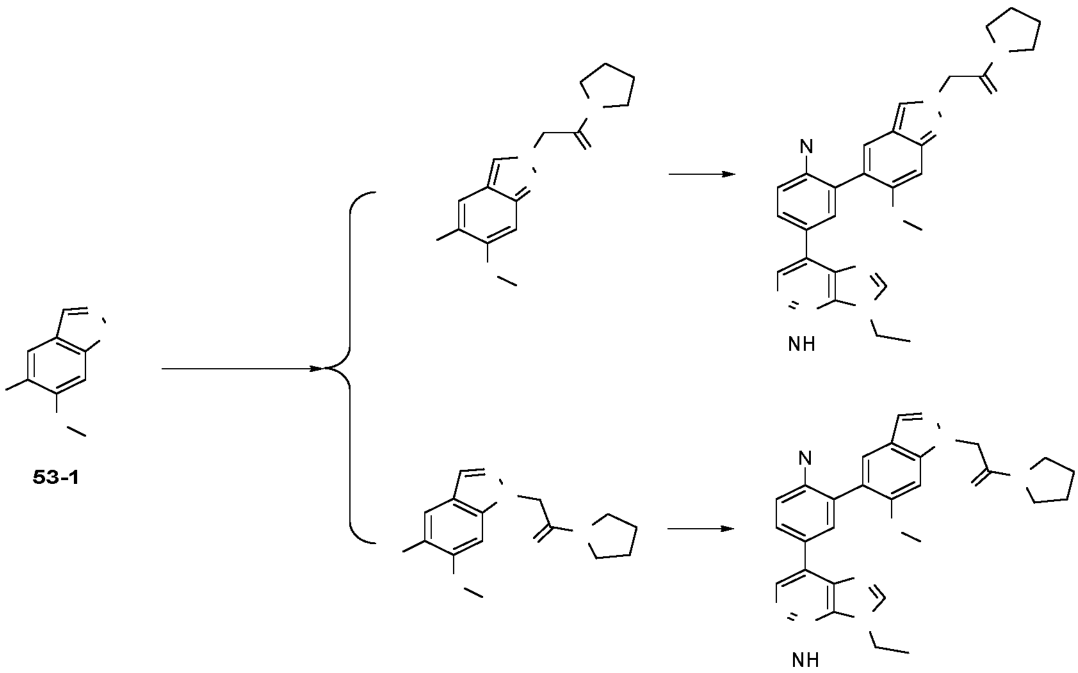

53-1

**83-A1: 2-(5-Bromo-6-methoxy-2*H*-indazol-2-yl)-1-(pyrrolidin-1-yl)ethan-1-one**

**83-A2: 2-(5-Bromo-6-methoxy-1*H*-indazol-1-yl)-1-(pyrrolidin-1-yl)ethan-1-one**

5-Bromo-6-(methoxy)-1*H*-benzo[d]imidazole (CAS: 1008361-65-6, 300 mg, 1.3 mmol) was dissolved in 10 mL of acetonitrile, then cesium carbonate (1 g, 2.6 mmol) and 2-chloro-1-(pyrrolidin-1-yl)ethan-1-one (200 mg, 1.3 mmol) were added thereto, and the mixture was stirred at 80°C for 1 hour. The reaction mixture was filtered to remove the solid, diluted with water, and a solid was precipitated. The residue was purified by preparative thin-layer chromatography (dichloromethane: methanol = 10:1) to obtain the title compound A1 (70 mg, 16%, Rf = 0.3) as a white solid and the title compound A2 (270 mg, 60%, Rf = 0.4) as a white solid. LC-MS: m/z $[M+H]^+$ = 338.

**83: 2-(5-(5-(7-Ethyl-7*H*-imidazo[4,5-*c*]pyridazin-4-yl)-2-fluorophenyl)-6-methoxy-2*H*-indazol-2-yl)-1-(pyrrolidin-1-yl)ethan-1-one**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (80 mg, 0.22 mmol) and 2-(5-bromo-6-methoxy-2*H*-indazol-2-yl)-1-(pyrrolidin-1-yl)ethan-1-one (70 mg, 0.22 mmol) were used as starting materials to obtain the title compound (70 mg, 64%) as a brown solid.

$^1H$ NMR (400 MHz, CHLOROFORM-d) 9.38 (s, 1 H) 8.25 - 8.30 (m, 2 H) 8.23 (dd, *J*= 6.85, 2.45 Hz, 1 H) 8.07 (s, 1 H) 7.63 (s, 1 H) 7.32 (t, *J= 9.05 Hz,* 1 H) 7.07 (s, 1 H) 5.16 (s, 2 H) 4.51 - 4.61 (m, 2 H) 3.85 (s, 3 H) 3.54 (dt, *J*= 14.06, 6.91 Hz, 4 H) 1.97 - 2.05 (m, 2 H) 1.86 - 1.93 (m, 2 H) 1.68 (t, *J* = 7.09 Hz, 3 H). LC-MS: m/z $[M+H]^+$ = 500.

**84: 2-(5-(5-(7-Ethyl-7H-imidazo[4,5-c]pyridazin-4-yl)-2-fluorophenyl)-6-methoxy-1H-indazol-1-yl)-1-(pyrrolidin-1-yl)ethan-1-one**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (80 mg, 0.22 mmol) and 2-(5-bromo-6-methoxy-1*H*-indazol-1-yl)-1-(pyrrolidin-1-yl)ethan-1-one (70 mg, 0.22 mmol) were used as starting materials to obtain the title compound (46 mg, 42%) as a brown solid.

$^1H$ NMR (400 MHz, CHLOROFORM-d) 9.38 (s, 1 H) 8.19 - 8.29 (m, 3 H) 7.98 (s, 1 H) 7.68 (s, 1 H) 7.34 (t*, J = 9.05 Hz,* 1 H) 6.99 (s, 1 H) 5.17 (s, 2 H) 4.54 - 4.63 (m, 2 H) 3.90 (s, 3 H) 3.53 (q, *J*= 6.85 Hz, 4 H) 1.99 (quin, *J* = 6.73 Hz, 2 H) 1.84 - 1.92 (m, 2 H) 1.69 (t, *J* = 7.09 Hz, 3 H). LC-MS: m/z $[M+H]^+$ = 500.

Example 85 and Example 86

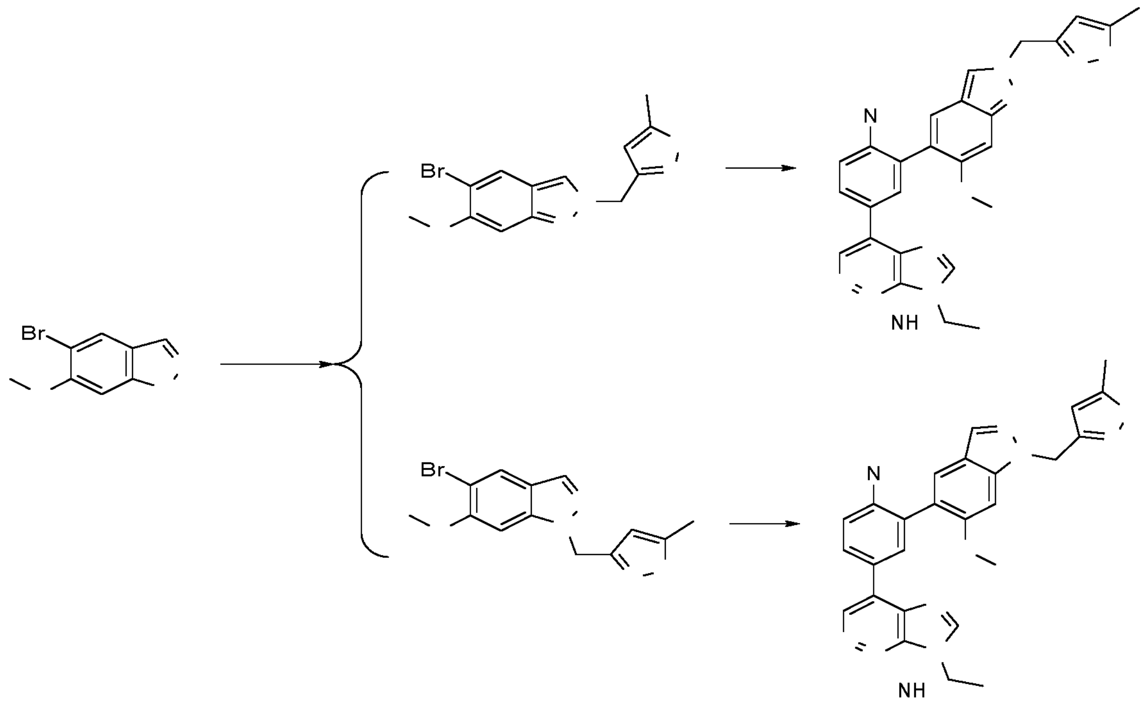

**85-A1: 3-((5-Bromo-6-methoxy-2*H*-indazol-2-yl)methyl)-5-methylisoxazole**

**85-A2: 3-((5-Bromo-6-methoxy-1*H*-indazol-1-yl)methyl)-5-methylisoxazole**

5-Bromo-6-(methoxy)-1*H*-benzo[d]imidazole (CAS: 1008361-65-6, 300 mg, 1.3 mmol) was dissolved in 10 mL of acetonitrile, then cesium carbonate (845 mg, 2.6 mmol) and 3-(chloromethyl)-5-methylisoxazole (180 mg, 1.3 mmol) were added thereto, and the mixture was heated to 80°C and stirred for 2 hours. The reaction mixture was filtered to remove the solid, and purified by preparative thin-layer chromatography (petroleum ether: ethyl acetate = 10:1) to obtain the title compound A1 (110 mg, 26%, Rf = 0.3) as a yellow solid and the title compound A2 (260 mg, 61%, Rf = 0.5) as a yellow solid. LC-MS: m/z $[M+H]^+$ = 322.

**85: 3-((5-(5-(7-Ethyl-7H-imidazo[4,5-c]pyridazin-4-yl)-2-fluorophenyl)-6-methoxy-2H-indazol-2-yl)methyl)-5-methylisoxazole**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (80 mg, 0.22 mmol) and 3-((5-bromo-6-methoxy-2*H*-indazol-2-yl)methyl)-5-methylisoxazole (70 mg, 0.22 mmol) were used as starting materials to obtain the title compound (29 mg, 27%) as a white solid.

$^1H$ NMR (400 MHz, CHLOROFORM-d) 9.37 (s, 1 H) 8.27 (br. s., 2 H) 8.20 (d, *J* = 5.38 Hz, 1 H) 7.96 (s, 1 H) 7.61 (s, 1 H) 7.33 (t, *J*= 8.80 Hz, 1 H) 7.10 (s, 1 H) 5.98 (s, 1 H) 5.60 (s, 2 H) 4.58 (q, *J*= 6.85 Hz, 2 H) 3.87 (s, 3 H) 2.39 (s, 3 H) 1.69 (t, *J*= 7.09 Hz, 3 H). LC-MS: m/z $[M+H]^+$ = 484.

**86: 3-((5-(5-(7-Ethyl-7H-imidazo[4,5-c]pyridazin-4-yl)-2-fluorophenyl)-6-methoxy-1H-indazol-1-yl)methyl)-5-methylisoxazole**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (80 mg, 0.22 mmol) and 3-((5-bromo-6-methoxy-1*H*-indazol-1-yl)methyl)-5-methylisoxazole (70 mg, 0.22 mmol) were used as starting materials to obtain the title compound (38 mg, 35%) as a brown solid.

$^1H$ NMR (400 MHz, CHLOROFORM-d) 9.28 - 9.43 (m, 1 H) 8.24 - 8.31 (m, 2 H) 8.21 (d, *J*= 6.85 Hz, 1 H) 7.98 (s, 1

H) 7.68 (s, 1 H) 7.33 (t, *J*= 9.05 Hz, 1 H) 6.92 (s, 1 H) 5.87 (s, 1 H) 5.61 (s, 2 H) 4.58 (q, *J*= 7.34 Hz, 2 H) 3.87 (s, 3 H) 2.37 (s, 3 H) 1.69 (t, *J*= 7.34 Hz, 3 H). LC-MS: m/z $[M+H]^+$ = 484.

Example 87

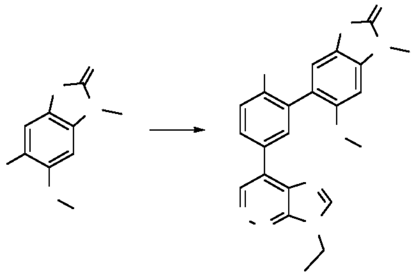

**87: 6-(5-(7-Ethyl-7*H*-imidazo[4,5-*c*]pyridazin-4-yl)-2-fluorophenyl)-5-methoxy-3-methylbenzo[*d*]oxazol-2(3*H*)-one**

Following the same experimental procedure as in Example 1, 6-bromo-5-methoxy-3-methylbenzo[d]oxazol-2(3*H*)-one (500.0 mg, 1.94 mmol) and 5-(7-ethyl-7*H*-imidazo[4,5-c]pyridazin-4-yl)-2-fluorophenyl)boronic acid (554 mg, 1.94 mmol) were used as starting materials to obtain the title product (310 mg, white solid) with a yield of 38%.
$^1$H NMR (400 MHz, DMSO-$d_6$) 9.55 (s, 1 H), 8.86 (s, 1 H), 8.45 (dd, 2 H), 7.48 (t, 1 H), 7.38 (s, 1 H), 7.21 (s, 1 H), 4.51 (q, 2 H), 3.82 (s, 3 H), 3.42 (s, 3 H), 1.56 (t, 3 H). LC-MS: m/z $[M+H]^+$ = 420.

Example 88

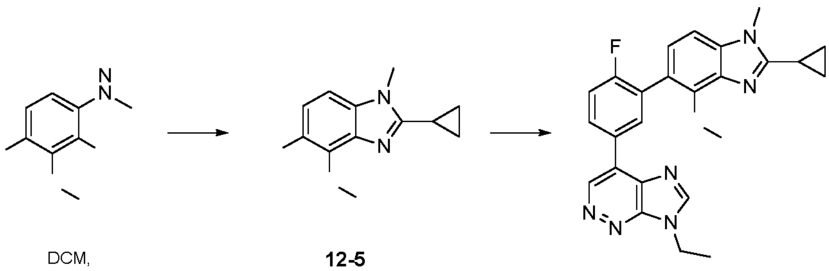

DCM,

**12-5**

**88-1: 5-Bromo-2-cyclopropyl-4-methoxy-1-methyl-1H-benzo[d]imidazole**

4-Bromo-3-methoxy-$N^1$-methylbenzene-1,2-diamine (200 mg, 0.87 mmol), pyrogallol (20 mg, 0.159 mmol), and cyclopropylmethylamine (93 mg, 1.31 mmol) were sequentially added to methanol (5 mL), and the mixture was stirred at 60°C for 16 hours. The reaction mixture was concentrated and separated by preparative thin-layer chromatography (petroleum ether/ethyl acetate = 3/1) to obtain the title compound (120 mg, 49%) as a brown oily liquid. LC-MS: m/z $[M+H]^+$ = 281, 283.

**88: 4-(3-(2-Cyclopropyl-4-methoxy-1-methyl-1*H*-benzo[*d*]imidazol-5-yl)-4-fluorophenyl)-7-ethyl-7*H*-imidazo [4,5-*c*]pyridazine**

Following the same experimental procedure as in Example 1, 5-bromo-2-cyclopropyl-4-methoxy-1-methyl-1H-benzo[d]imidazole (120 mg, 0.43 mmol) and 7-ethyl-4-(4-fluoro-3-(4,4,5,5-tetramethyl-1,3,2-dioxaborolan-2-yl)phenyl)-7*H*-imidazo[4,5-c]pyridazine (158 mg, 0.43 mmol) were used as starting materials to obtain the title compound (17 mg, 9%) as a white solid.
$^1$H NMR (400 MHz, CHLOROFORM-d) 9.37 (s, 1 H), 8.30 - 8.24 (m, 2 H), 8.21 (dd, *J* = 2.0, 6.8 Hz, 1 H), 7.34 (t, *J* = 8.8 Hz, 1 H), 7.21 (d, *J* = 8.3 Hz, 1 H), 7.04 (d, *J* = 8.3 Hz, 1 H), 4.57 (q, *J*= 7.0 Hz, 2 H), 4.29 (s, 3 H), 3.85 (s, 3 H), 2.05 - 1.97 (m, 1 H), 1.68 (t, *J*= *7.3* Hz, 3 H), 1.25 (br. s., 2H), 1.14 - 1.07 (m, 2 H). LC-MS: m/z $[M+H]^+$ = 443.

Example 89

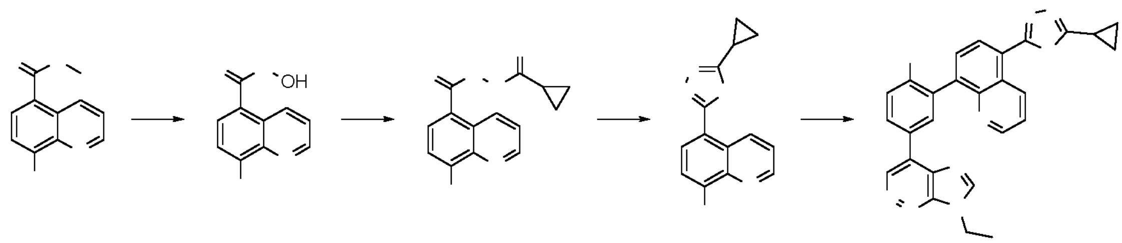

**89-1: 8-Bromoquinoline-5-carbohydrazide**

Methyl 8-bromoquinoline-5-carboxylate (500 mg, 1.88) and hydrazine hydrate (280 mg, 5.6 mmol) were added to anhydrous ethanol (10 mL), and the mixture was stirred at 50°C overnight. The reaction mixture was directly purified by preparative thin-layer chromatography (dichloromethane/methanol = 15/1) to obtain the title compound (260 mg, 52%).

**89-2: 8-Bromo-*N'*-(cyclopropanecarbonyl)quinoline-5-carbohydrazide**

8-Bromoquinoline-5-carbohydrazide (230 mg, 0.86 mmol), *N,N*-diisopropylethylamine (223 mg, 1.73 mmol), 2-(7-azabenzotriazol-1-yl)-*N*,*N*,*N'*,*N'*-tetramethyluronium hexafluorophosphate (162 mg, 1.73 mmol), and cyclopropanecarboxylic acid (150 mg, 1.73 nnol) were added to dichloromethane (6 mL), and the mixture was stirred at room temperature overnight. The reaction mixture was directly purified by preparative thin-layer chromatography (dichloromethane/methanol = 20/) to obtain the title compound (150 mg, 52%).

**89-3: 2-(8-Bromoquinolin-5-yl)-5-cyclopropyl-1,3,4-oxadiazole**

8-Bromo-*N'*-(cyclopropanecarbonyl)quinoline-5-carbohydrazide (150 mg, 0.45 mmol), *N*,*N*-diisopropylethylamine (174 mg, 1.35 mmol), and *p*-toluenesulfonyl chloride (256 mg, 1.35 mmol) were added to dichloromethane (6 mL), and the mixture was stirred at room temperature overnight. The reaction mixture was directly purified by preparative thin-layer chromatography (petroleum ether ethyl acetate = 11) to obtain the title compound (20 mg, 14.1%).

**89: 2-Cyclopropyl-5-(8-(5-(7-ethyl-7*H*-imidazo[4,5-*c*]pyridazin-4-yl)-2-fluorophenyl)quinolin-5-yl)-1,3,4-oxadiazole**

Following the same experimental procedure as in Example 1, 2-(8-bromoquinolin-5-yl)-5-cyclopropyl-1,3,4-oxadiazole (23 mg, 0.06 mmol) and 7-ethyl-4-(4-fluoro-3-(4,4,5,5-tetramethyl-1,3,2-dioxaborolan-2-yl)phenyl)-7*H*-imidazo[4,5-c]pyridazine (23 mg, 0.06 mmol) were used as starting materials to obtain the title compound (13 mg, 45%).

$^1$H NMR (400 MHz, CHLOROFORM-d) 9.77 (d, *J* = 8.8 Hz, 1 H), 9.42 (s, 1 H), 9.00 (br. s., 1 H), 8.40 (d, *J*=6.4 Hz, 2 H), 8.30 - 8.22 (m, 2 H), 7.92 (d, *J*=7.8 Hz, 1 H), 7.60 (br. s., 1 H), 7.45 (s, 1 H), 4.58 (d, *J* = *6.8* Hz, 2 H), 2.33 (br. s., 1 H), 1.71 - 1.67 (m, 3 H), 1.32 - 1.30 (m, 4 H).

Example 90

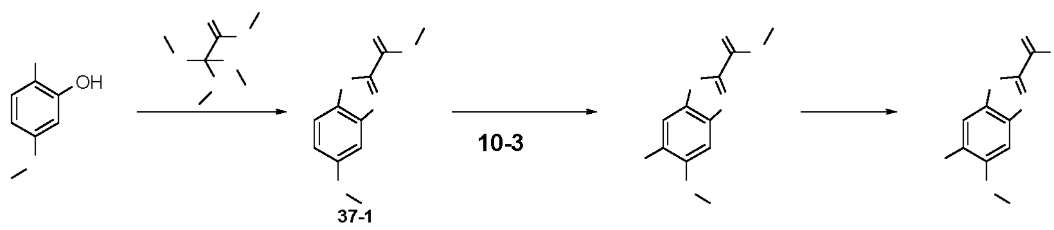

10-3

37-1

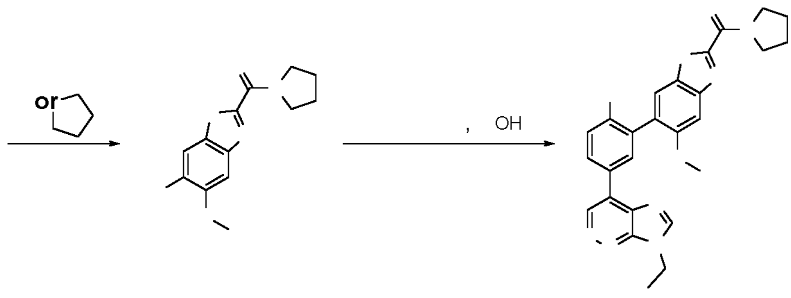

**90-1: Methyl 5-methoxybenzo[d]oxazole-2-carboxylate**

2-Amino-4-methoxyphenol (1.0 g, 7.19 mmol) and methyl 2,2,2-trimethoxyacetate (11.8 g, 72 mmol) were heated to 110°C and reacted for 3 hours in a microwave reactor. The reaction mixture was concentrated and separated by column chromatography (petroleum ether/ethyl acetate = 10:1 to 5:1) to obtain the title compound (0.46 g, yield: 31%) as a reddish brown solid. LC-MS: m/z $[M+H]^+$ = 208.

**90-2: Methyl 6-bromo-5-methoxybenzo[d]oxazole-2-carboxylate**

Methyl 5-methoxybenzo[*d*]oxazole-2-carboxylate (0.4 g, 1.93 mmol) and *N*-bromosuccinimide (0.376 g, 2.1 mmol) were sequentially added to acetonitrile (20 mL). The mixture was then reacted at 50°C for 4 hours. The reaction mixture was concentrated and purified to obtain the crude title compound (0.4 g, yield: 72%). LC-MS: m/z $[M+H]^+$ = 286.

**90-3: 6-Bromo-5-methoxybenzo[d]oxazole-2-carboxylic acid**

Methyl 6-bromo-5-methoxybenzo[d]oxazole-2-carboxylate (0.4 g, 1.4 mmol) and lithium hydroxide (0.117 g, 2.8 mmol) were sequentially added to a solution (5 mL) of tetrahydrofuran and water with a ratio of 10:1. The mixture was then reacted at room temperature for 4 hours. The reaction mixture was added with 1 N hydrochloric acid to adjust the pH to 3 to 4, and extracted three times with ethyl acetate. The organic phase was concentrated and purified to obtain the title compound (300 mg, yield: 79%). LC-MS: m/z $[M+H]^+$ = 272.

**90-4: (6-Bromo-5-methoxybenzo[d]oxazol-2-yl)(pyrrolidin-1-yl)methanone**

The starting materials 6-bromo-5-methoxybenzo[*d*]oxazole-2-carboxylic acid (300 mg, 1.1 mmol), tetrahydropyrrole (90 mg, 1.2 mmol), 2-(7-azabenzotriazol-1-yl)-*N*,*N*,*N'*,*N'*-tetramethyluronium hexafluorophosphate (630 mg, 1.6 mmol), and*N*,*N*-diisopropylethylamine (280 mg, 2.2 mmol) were added to N,N-dimethylformamide (10 mL). The mixture was reacted at room temperature overnight under a nitrogen atmosphere. The reaction mixture was quenched with water, concentrated, and separated by column chromatography (dichloromethane/methanol = 30:1 to 10:1) to obtain the title compound (200 mg, yield: 56%) as a brown solid. LC-MS: m/z $[M+H]^+$ = 325.

**90: (6-(5-(7-Ethyl-7*H*-imidazo[4,5-*c*]pyridazin-4-yl)-2-fluorophenyl)-5-methoxybenzo[*d*]oxazol-2-yl)(pyrrolidin-1-yl)methanone**

Following the same experimental procedure as in Example 1, (6-bromo-5-methoxybenzo[*d*]oxazol-2-yl)(pyrrolidin-1-yl)methanone (100 mg, 0.3 mmol) and 5-(7-ethyl-7*H*-imidazo[4,5-*c*]pyridazin-4-yl)-2-fluorophenyl)boronic acid (124 mg, 0.33 mmol) were used as starting materials to obtain the title compound (80 mg, yield: 54%) as an off-white solid.

$^1H$ NMR (400 MHz, $CDCl_3$): δ 9.38 (s, 1 H), 8.28 (t, 3 H), 7.65 (s, 1 H), 7.42 - 7.32 (m, 2 H), 4.58 (q, 2 H), 4.15 (t, 2 H), 3.89 (s, 3 H), 3.77 (t, 2 H), 2.03 (m, 4 H), 1.69 (t, 3 H).

Example 91

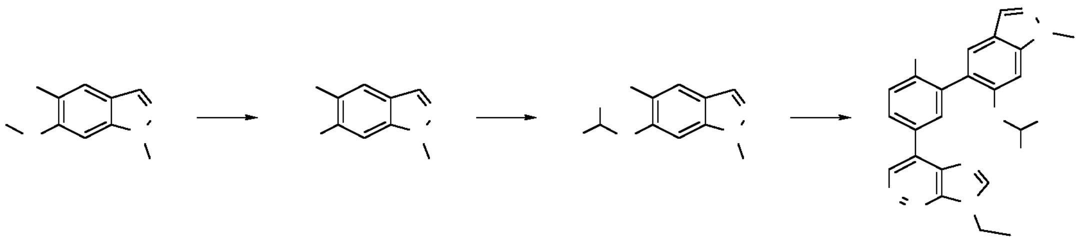

**91-1: 5-Bromo-1-methyl-1*H*-indazol-6-ol**

5-Bromo-6-methoxy-1-methyl-1*H*-indazole (500 mg, 2 mmol) was dissolved in 10 mL of dichloromethane, then a 1 M solution of boron tribromide in dichloromethane (5 mL) was added thereto, and the mixture was stirred at room temperature for 2 hours. The reaction mixture was concentrated and purified by preparative thin-layer chromatography (petroleum ether: ethyl acetate = 1:1) to obtain the title compound (300 mg, 65%) as a yellow liquid. LC-MS: m/z $[M+H]^+$ = 227.

**91-2: 5-Bromo-6-(difluoromethoxy)-1-methyl-1*H*-indazole**

5-Bromo-1-methyl-1*H*-indazol-6-ol (300 mg, 1 mmol) was dissolved in 10 mL of *N*,*N*-dimethylformamide, then cesium carbonate (700 mg, 1 mmol) and sodium difluorochloroacetate (230 mg, 1.5 mmol) were added thereto, and the mixture was heated to 100°C and stirred for 2 hours. The reaction mixture was diluted with water, extracted with ethyl acetate, concentrated, and purified by preparative thin-layer chromatography (petroleum ether: ethyl acetate = 3:1) to obtain the title compound (150 mg, 50%) as a white solid. LC-MS: m/z $[M+H]^+$ = 277.

**91: 4-(3-(6-(Difluoromethoxy)-1-methyl-1*H*-indazol-5-yl)-4-fluorophenyl)-7-ethyl-7*H*-imidazo[4,5-*c*]pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (80 mg, 0.22 mmol) and 5-bromo-6-(difluoromethoxy)-1-methyl-1*H*-indazole (75 mg, 0.22 mmol) were used as starting materials to obtain the title compound (25 mg, 27%) as a brown solid.

$^1$H NMR (400 MHz, CHLOROFORM-*d*) 9.41 (s, 1 H) 8.30 (s, 3 H) 8.04 (s, 1 H) 7.82 (s, 1 H) 7.34 - 7.42 (m, 1 H) 7.27 (s, 1 H) 6.32 - 6.78 (m, 1 H) 4.59 (d, *J*= 7.34 Hz, 2 H) 4.12 (s, 3 H) 1.70 (t, *J* = 7.34 Hz, 3 H). LC-MS: m/z $[M+H]^+$ = 439.

Example 92

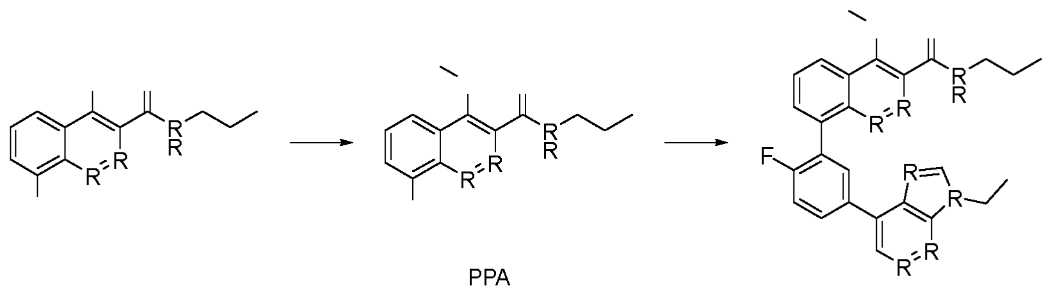


**92-1: 8-Bromo-4-(methylamino)-*N*-propylcinnoline-3-carboxamide**

4-Amino-8-bromo-N-propylcinnoline-3-carboxamide (400 mg, 1.3 mmol), cesium carbonate (1300 mg, 3.9 mmol), and iodomethane (553 mg, 3.9 mmol) were added to tetrahydrofuran (10 mL). The mixture was stirred at 40°C for 72 hours. The reaction mixture was directly purified by thin-layer chromatography (dichloromethane/methanol = 30/1) to obtain the title compound (200 mg, 48%). LC-MS: m/z $[M+H]^+$ = 323.

**92: 8-(5-(7-Ethyl-7H-imidazo[4,5-c]pyridazin-4-yl)-2-fluorophenyl)-4-(methylamino)-N-propylcinnoline-3-carboxamide**

Following the same experimental procedure as in Example 1, 8-bromo-4-(methylamino)-*N*-propylcinnoline-3-carboxamide (53 mg, 0.16 mmol) and 7-ethyl-4-(4-fluoro-3-(4,4,5,5-tetramethyl-1,3,2-dioxaborolan-2-yl)phenyl)-7*H*-imidazo[4,5-c]pyridazine (50 mg, 0.13 mmol) were used as starting materials to obtain the title compound (26 mg, 40%).
$^1$H NMR (400 MHz, CHLOROFORM-d) 0.97 (t, *J* = 6.36 Hz, 3 H) 1.63 (br. s., 5 H) 3.40 (d, *J*= 5.38 Hz, 2 H) 3.55 (d, *J*= 4.40 Hz, 3 H) 4.57 (d, *J* = 6.85Hz, 2 H) 7.41 (t, *J*= 8.56 Hz, 1 H) 7.64 (br. s., 1 H) 7.84 (d, *J*= 5.87 Hz, 1 H) 8.26 (br. s., 1 H) 8.34 (br. s., 2 H) 8.48 (d, *J* = 7.83 Hz, 1 H) 8.60 (br. s., 1 H) 9.39 (br. s., 1 H) 10.54 (br. s., 1 H). LC-MS: m/z $[M+H]^+$ = 485.

Example 93

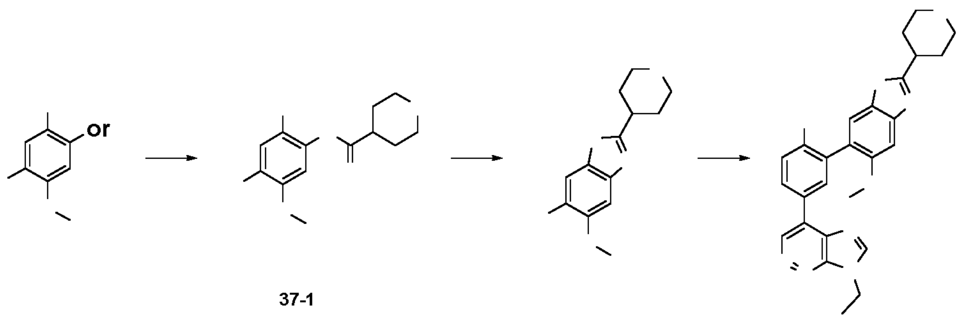


37-1

**93-1: *N*-(2,4-Dibromo-5-methoxyphenyl)tetrahydro-2*H*-pyran-4-carboxamide**

2,4-Dibromo-5-methoxyaniline (500 mg, 1.8 mmol), methyl tetrahydro-2*H*-pyran-4-carboxylate (308 mg, 2.1 mmol), and potassium *tert*-butoxide (400 mg, 3.6 mmol) were added to tetrahydrofuran (20 mL). The mixture was stirred in air at room temperature for 5 hours. The reaction mixture was directly added with ethyl acetate (200 mL) and washed three times with water (100 mL). The ethyl acetate was then dried over anhydrous sodium sulfate and concentrated to obtain the title compound (600 mg, 85.8%). LC-MS: m/z $[M+H]^+$ = 394.

**93-2: 6-Bromo-5-methoxy-2-(tetrahydro-2*H*-pyran-4-yl)benzo[*d*]oxazole**

*N*-(2,4-Dibromo-5-methoxyphenyl)tetrahydro-2*H*-pyran-4-carboxamide (300 mg, 0.76 mmol), potassium carbonate (316 mg, 2.3 mmol), copper powder (147 mg, 2.3 mmol), and aluminum oxide (233 mg, 2.3 mmol) were added to *N,N*-dimethylformamide (10 mL). The mixture was stirred at 110°C for 4 hours. The reaction mixture was directly subjected to column chromatography to obtain the title compound (170 mg, 71.7%). LC-MS: m/z $[M+H]^+$ = 312.

**93: 6-(5-(7-Ethyl-7*H*-imidazo[4,5-*c*]pyridazin-4-yl)-2-fluorophenyl)-5-methoxy-2-(tetrahydro-2*H*-pyran-4-yl)benzo[*d*]oxazole**

Following the same experimental procedure as in Example 1, 6-bromo-5-methoxy-2-(tetrahydro-2*H*-pyran-4-yl) benzo[d]oxazole (50 mg, 0.16 mmol) and 7-ethyl-4-(4-fluoro-3-(4,4,5,5-tetramethyl-1,3,2-dioxaborolan-2-yl)phenyl)-7*H*-imidazo[4,5-c]pyridazine (50 mg, 0.13 mmol) were used as starting materials to obtain the title compound (12 mg, 19%).
$^1$H NMR (400 MHz, CHLOROFORM-d) 1.66 - 1.70 (m, 3 H) 2.03 - 2.16 (m, 4 H) 3.23 (br. s., 1 H) 3.59 (t, *J*= 10.76 Hz, 2 H) 3.85 (s, 3 H) 4.08 (d, *J*= 11.25 Hz, 2 H) 4.57 (q, *J* = 7.34 Hz, 2 H) 7.28 - 7.40 (m, 2 H) 7.49 (s, 1 *H) 8.22 (d, J = 6.85 Hz,* 1 H) 8.27 (br. s., 2 H) 9.38 (br. s., 1 H). LC-MS: m/z $[M+H]^+$ = 474.

Example 94 and Example 95

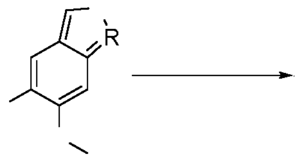

10-3

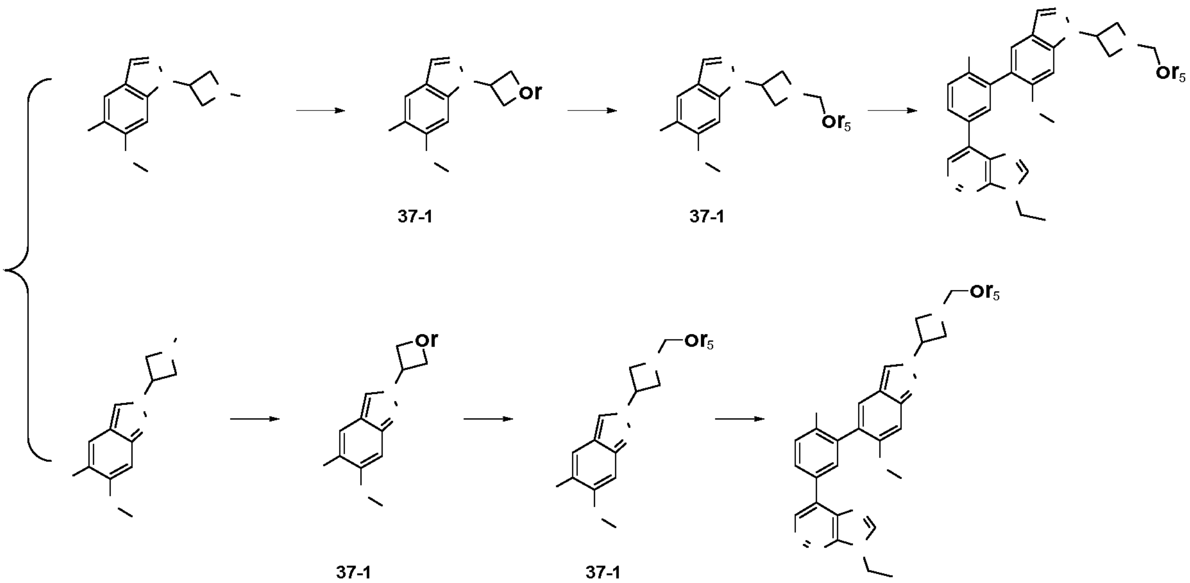

37-1 37-1

37-1 37-1

**94-A1: *tert*-Butyl 3-(5-bromo-6-methoxy-1*H*-indazol-1-yl)azetidine-1-carboxylate**

**94-A2: *tert*-Butyl 3-(5-bromo-6-methoxy-2*H*-indazol-2-yl)azetidine-1-carboxylate**

5-Bromo-6-methoxy-1H-indazole (2 g, 8.81 mmol) was added to anhydrous *N,N*-dimethylformamide (20 mL), then sodium hydride (705 mg, 17.62 mmol) was added thereto at room temperature, and the mixture was stirred at room temperature for 0.5 hours. *tert*-Butyl 3-iodoazetidine-1-carboxylate (4.19 g, 17.62 mmol) was then added thereto, and the mixture was stirred at room temperature for another 16 hours. The reaction mixture was added with sodium sulfate decahydrate (500 mg), filtered, concentrated, and separated by preparative thin-layer chromatography (petroleum ether/ethyl acetate = 3/1) to obtain *tert*-butyl 3-(5-bromo-6-methoxy-2*H*-indazol-2-yl)azetidine-1-carboxylate (340 mg, 10%, Rf = 0.3) and *tert*-butyl 3-(5-bromo-6-methoxy-1*H*-indazol-1-yl)azetidine-1-carboxylate (900 mg, 27%, Rf = 0.5). LC-MS: m/z $[M+H]^+$ = 382, 384.

**94-2: 1-(Azetidin-3-yl)-5-bromo-6-methoxy-1*H*-indazole hydrochloride**

*tert-Butyl* 3-(5-bromo-6-methoxy-1H-indazol-1-yl)azetidine-1-carboxylate (900 mg, 2.36 mmol) was added to dichloromethane (5 mL), then a solution of hydrochloric acid in ethyl acetate (2 mL, 6 M) was added thereto, and the mixture was stirred at room temperature for 10 minutes. The precipitated solid was filtered out to obtain the title compound (663 mg, 100%) as a white solid. LC-MS: m/z $[M+H]^+$ = 282, 284.

**94-3: 5-Bromo-6-methoxy-1-(1-(2,2,2-trifluoroethyl)azetidin-3-yl)-1*H*-indazole**

1-(Azetidin-3-yl)-5-bromo-6-methoxy-1*H*-indazole hydrochloride (80 mg, 0.25 mmol), 2,2,2-trifluoroethyl trifluoromethanesulfonate (116 mg, 0.50 mmol), and triethylamine (126 mg, 1.25 mmol) were sequentially added to dichloromethane (2 mL), and the mixture was stirred at room temperature for 2 hours. The reaction mixture was concentrated and separated by preparative thin-layer chromatography (petroleum ether/ethyl acetate = 1/1) to obtain the title compound (60 mg, 66%) as a white solid. LC-MS: m/z $[M+H]^+$ = 364, 366.

**94: 7-Ethyl-4-(4-fluoro-3-(6-methoxy-1-(1-(2,2,2-trifluoroethyl)azetidin-3-yl)-3a,7a-dihydro-1*H*-indazol-5-yl)phenyl)-7*H*-imidazo[4,5-*c*]pyridazine**

Following the same experimental procedure as in Example 1, 5-bromo-6-methoxy-1-(1-(2,2,2-trifluoroethyl) azetidin-3-yl)-1*H*-indazole (60 mg, 0.16 mmol) and 7-ethyl-4-(4-fluoro-3-(4,4,5,5-tetramethyl-1,3,2-dioxaborolan-2-yl) phenyl)-7*H*-imidazo[4,5-*c*]pyridazine (59 mg, 0.16 mmol) were used as starting materials to obtain the title compound (35 mg, 42%) as a yellow solid.

$^1$H NMR (400 MHz, CHLOROFORM-d) 9.37 (s, 1 H), 8.26 (s, 2 H), 8.20 (d, *J*= 6.8 Hz, 1 H), 7.99 (s, 1 H), 7.67 (s, 1 H), 7.33 (t, *J* = *9.0* Hz, 1 H), 7.08 (s, 1 H), 5.39 - 5.30 (m, 1 H), 4.57 (q, *J*= 7.2 Hz, 2 H), 4.14 - 4.06 (m, 2 H), 3.99 (t, *J*= 7.1 Hz, 2 H), 3.90 (s, 3 H), 3.24 (q, *J*= 9.1 Hz, 2 H), 1.68 (t, *J*= 7.1 Hz, 3 H). LC-MS: m/z [M+H]$^+$ = 526.

**95-2: 2-(Azetidin-3-yl)-5-bromo-6-methoxy-2*H*-indazole hydrochloride**

Following the same operation method as 94-2, *tert*-butyl 3-(5-bromo-6-methoxy-2*H*-indazol-2-yl)azetidine-1-carboxylate (900 mg, 2.36 mmol) was used as the starting material to obtain the title compound (660 mg, 100%) as a white solid. LC-MS: m/z [M+H]$^+$ = 282, 284.

**95-3: 5-Bromo-6-methoxy-2-(1-(2,2,2-trifluoroethyl)azetidin-3-yl)-2*H*-indazole**

Following the same operation method as 94-2, 2-(azetidin-3-yl)-5-bromo-6-methoxy-2*H*-indazole hydrochloride (80 mg, 0.25 mmol) was used as the starting material to obtain the title compound (50 mg, 55%) as a light yellow oily liquid. LC-MS: m/z [M+H]$^+$ = 364, 366.

**95: 7-Ethyl-4-(4-fluoro-3-(6-methoxy-2-(1-(2,2,2-trifluoroethyl)azetidin-3-yl)-2H-indazol-5-yl)phenyl)-7H-imidazo [4,5-c]pyridazine**

Following the same experimental procedure as in Example 1, 5-bromo-6-methoxy-2-(1-(2,2,2-trifluoroethyl) azetidin-3-yl)-2*H*-indazole (50 mg, 0.14 mmol) and 7-ethyl-4-(4-fluoro-3-(4,4,5,5-tetramethyl-1,3,2-dioxaborolan-2-yl) phenyl)-7*H*-imidazo[4,5-*c*]pyridazine (52 mg, 0.14 mmol) were used as starting materials to obtain the title compound (35 mg, 48%) as a yellow solid.

$^1$H NMR (400 MHz, CHLOROFORM-d) 9.37 (s, 1 H), 8.26 (s, 2 H), 8.20 (d, *J*= *6.4* Hz, 1 H), 8.01 (s, 1 H), 7.60 (s, 1 H), 7.32 (t, *J* = *8.6* Hz, 1 H), 7.11 (s, 1 H), 5.24 (t, *J* = 6.4 Hz, 1 H), 4.61 - 4.53 (m, 2 H), 4.13 - 4.06 (m, 2 H), 3.96 (t, *J*= 6.6 Hz, 2 H), 3.87 (s, 3 H), 3.23 (q, *J* = 9.1 Hz, 2 H), 1.68 (t, *J* = 7.1 Hz, 3 H). LC-MS: m/z [M+H]$^+$ = 526.

Example 96

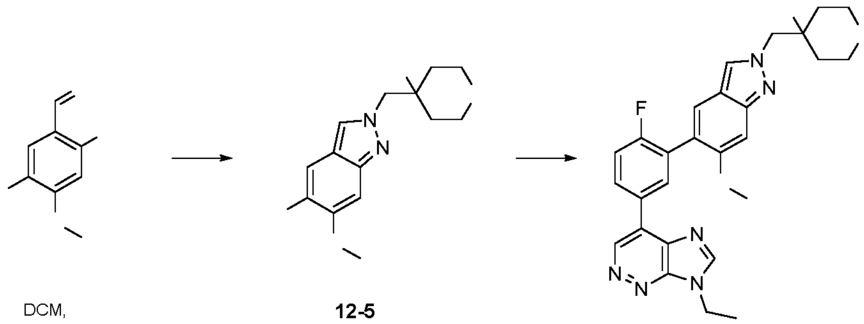


**96-1: 4-((5-Bromo-6-methoxy-2H-indazol-2-yl)methyl)tetrahydro-2H-pyran-4-ol**

Following the same experimental procedure as in the synthesis method of 40-1 and 40-2 in Example 40, 5-bromo-4-methoxy-2-nitrobenzaldehyde (150 mg, 0.58 mmol) and 4-(aminomethyl)tetrahydro-2*H*-pyran-4-ol (152 mg, 1.16 mmol) were used as starting materials to obtain the title compound (50 mg, 25%) as a light yellow oily liquid. LC-MS: m/z [M+H]$^+$ = 341, 343.

**96: 4-((5-(5-(7-Ethyl-7H-imidazo[4,5-c]pyridazin-4-yl)-2-fluorophenyl)-6-methoxy-2H-indazol-2-yl)methyl)tetrahydro-2H-pyran-4-ol**

Following the same experimental procedure as in Example 1, 4-((5-bromo-6-methoxy-2*H*-indazol-2-yl)methyl) tetrahydro-2H-pyran-4-ol (50 mg, 0.15 mmol) and 7-ethyl-4-(4-fluoro-3-(4,4,5,5-tetramethyl-1,3,2-dioxaborolan-2-yl) phenyl)-7*H*-imidazo[4,5-c]pyridazine (55 mg, 0.15 mmol) were used as starting materials to obtain the title compound (8 mg, 11%) as a yellow solid.

$^1$H NMR (400 MHz, CHLOROFORM-*d*) 9.38 (s, 1 H), 8.28 (s, 2 H), 8.20 (d, *J*= 6.8 Hz, 1 H), 7.90 (s, 1 H), 7.63 (s, 1 H), 7.36 - 7.30 (m, 1 H), 7.08 (s, 1 H), 4.57 (q, *J*= *7.3* Hz, 2 H), 4.34 (s, 2 H), 3.87 (s, 3 H), 3.83 - 3.73 (m, 4 H), 2.23 - 2.11 (m, 4 H), 1.70 - 1.67 (m, 3 H). LC-MS: m/z [M+H]$^+$ = 503.

Example 97

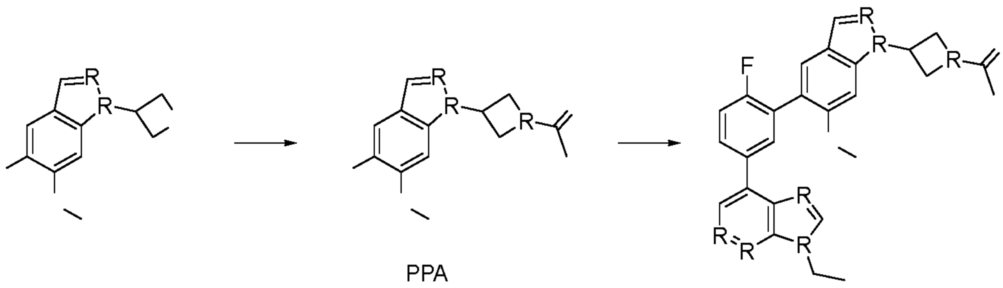


**97-1: 1-(3-(5-Bromo-6-methoxy-1*H*-indazol-1-yl)azetidin-1-yl)ethan-1-one**

1-(Azetidin-3-yl)-5-bromo-6-methoxy-1*H*-indazole hydrochloride (100 mg, 0.31 mmol), acetic anhydride (63 mg, 0.62 mmol), and triethylamine (116 mg, 1.15 mmol) were sequentially added to dichloromethane (5 mL), and the mixture was stirred at room temperature for 2 hours. The reaction mixture was added dropwise to saturated ammonium chloride aqueous solution (10 mL), and after phase separation, the organic phase was separated by preparative thin-layer chromatography (ethyl acetate) to obtain the title compound (100 mg, 100%) as a light yellow oily liquid. LC-MS: m/z [M+H]$^+$ = 324, 326.

**97: 1-(3-(5-(5-(7-Ethyl-7*H*-imidazo[4,5-*c*]pyridazin-4-yl)-2-fluorophenyl)-6-methoxy-3a,7a-dihydro-1*H*-indazol-1-yl)azetidin-1-yl)ethan-1-one**

Following the same experimental procedure as in Example 1, 1-(3-(5-bromo-6-methoxy-1*H*-indazol-1-yl)azetidin-1-yl)ethan-1-one (60 mg, 0.19 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (70 mg, 0.19 mmol) were used as starting materials to obtain the title compound (30 mg, 33%) as a yellow solid.

$^1$H NMR (400 MHz, CHLOROFORM-d) 9.37 (s, 1 H), 8.28 (s, 2 H), 8.20 (d, *J*= 6.8 Hz, 1 H), 8.04 (s, 1 H), 7.70 (s, 1 H), 7.33 (t, *J* = 8.8 Hz, 1 H), 6.84 (s, 1 H), 5.42 (d, *J = 6.4* Hz, 1 H), 4.86 - 4.79 (m, 1 H), 4.66 (t, *J* = 8.3 Hz, 1 H), 4.60 - 4.54 (m, 4 H), 3.89 (s, 3 H), 1.99 (s, 3 H), 1.68 (t, *J* = 7.1 Hz, 3 H). LC-MS: m/z [M+H]$^+$ = 486.

Example 98

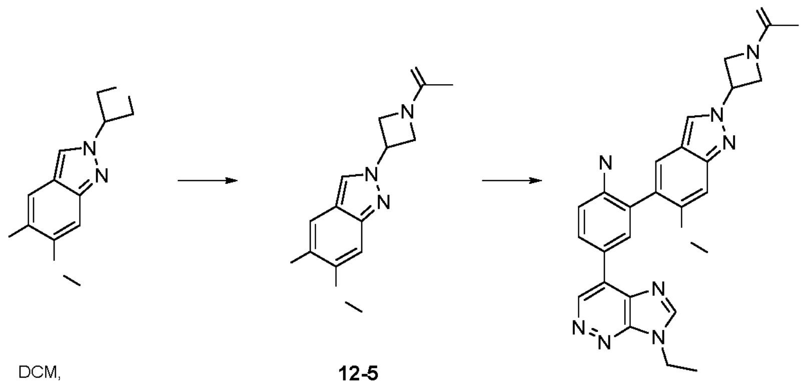

DCM,

**12-5**

**98-1: 1-(3-(5-Bromo-6-methoxy-2*H*-indazol-2-yl)azetidin-1-yl)ethan-1-one**

2-(Azetidin-3-yl)-5-bromo-6-methoxy-2*H*-indazole hydrochloride (100 mg, 0.31 mmol), acetic anhydride (63 mg, 0.62 mmol), and triethylamine (116 mg, 1.15 mmol) were sequentially added to dichloromethane (5 mL), and the mixture was stirred at room temperature for 2 hours. The reaction mixture was added dropwise to saturated ammonium chloride aqueous solution (10 mL), and after phase separation, the organic phase was separated by preparative thin-layer chromatography (ethyl acetate) to obtain the title compound (50 mg, 50%) as a light yellow oily liquid. LC-MS: m/z $[M+H]^+$ = 324, 326.

**98: 1-(3-(5-(5-(7-Ethyl-7*H*-imidazo[4,5-*c*]pyridazin-4-yl)-2-fluorophenyl)-6-methoxy-2*H*-indazol-2-yl)azetidin-1-yl)ethan-1-one**

Following the same experimental procedure as in Example 1, 1-(3-(5-bromo-6-methoxy-2*H*-indazol-2-yl)azetidin-1-yl)ethan-1-one (50 mg, 0.15 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (55 mg, 0.15 mmol) were used as starting materials to obtain the title compound (20 mg, 27%) as a yellow solid.

$^1$H NMR (400 MHz, CHLOROFORM-*d*) 9.36 (s, 1 H), 8.26 (s, 2 H), 8.19 (d, *J* = *6.4* Hz, 1 H), 7.98 (s, 1 H), 7.59 (s, 1 H), 7.31 (t, *J*= 9.0 Hz, 1 H), 7.11 (s, 1 H), 5.36 - 5.27 (m, 1 H), 4.80 - 4.73 (m, 1 H), 4.65 (t, *J*= 8.3 Hz, 1 H), 4.59 - 4.52 (m, 4 H), 3.86 (s, 3 H), 1.96 (s, 3 H), 1.66 (t, *J*= 7.3 Hz, 3 H). LC-MS: m/z $[M+H]^+$ = 486.

Example 99

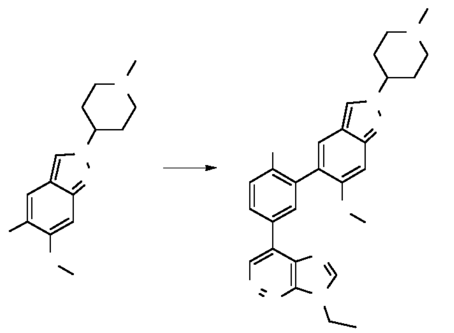

**99: 7-Ethyl-4-(4-fluoro-3-(6-methoxy-2-(1-methylpiperidin-4-yl)-2*H*-indazol-5-yl)phenyl)-7H-imidazo[4,5-c]pyridazine**

Following the same experimental procedure as in Example 1, 5-bromo-6-methoxy-2-(1-methylpiperidin-4-yl)-2*H*-indazole (200 mg, 0.62 mmol) and 7-ethyl-4-(4-fluoro-3-(4,4,5,5-tetramethyl-1,3,2-dioxaborolan-2-yl)phenyl)-7*H*-imidazo[4,5-c]pyridazine (228 mg, 0.62 mmol) were used as starting materials to obtain the title compound (160 mg, 53%) as a white solid.

$^1$H NMR (400 MHz, CHLOROFORM-*d*) 9.38 (s, 1 H), 8.27 (br. s., 2 H), 8.20 (d, *J* = 5.9 Hz, 1 H), 7.95 (s, 1 H), 7.62 (s, 1 H), 7.33 (t, *J* = 8.8 Hz, 1 H), 7.10 (s, 1 H), 4.63 - 4.53 (m, 2 H), 4.47 (br. s., 1 H), 3.87 (s, 3 H), 3.17 (br. s., 2 H), 2.52 - 2.26

(m, 9 H), 1.69 (t, *J* = 7.1 Hz, 3 H). LC-MS: m/z $[M+H]^+$ = 486.

Example 100

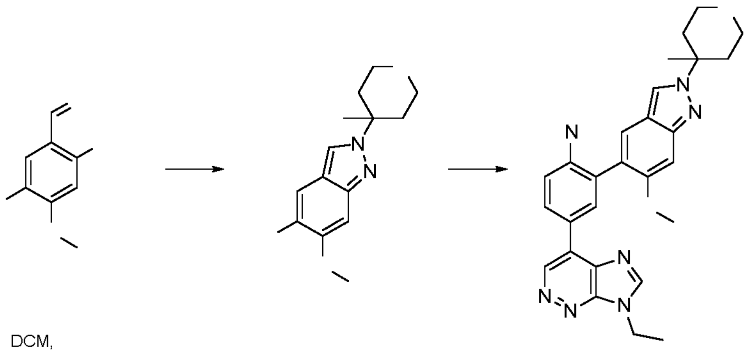


**100-1: 5-Bromo-6-methoxy-2-(4-methyltetrahydro-2*H*-pyran-4-yl)-2*H*-indazole**

Following the same experimental procedure as in the synthesis method of 40-1 and 40-2 in Example 40, 5-bromo-4-methoxy-2-nitrobenzaldehyde (150 mg, 0.58 mmol) and tetrahydro-4-methyl-2*H*-pyran-4-amine (132 mg, 1.16 mmol) were used as starting materials to obtain the title compound (80 mg, 43%) as a light yellow oily liquid. LC-MS: m/z $[M+H]^+$ = 325, 327.

**100: 7-Ethyl-4-(4-fluoro-3-(6-methoxy-2-(4-methyltetrahydro-2*H*-pyran-4-yl)-2*H*-indazol-5-yl)phenyl)-7*H*-imidazo[4,5-*c*]pyridazine**

Following the same experimental procedure as in Example 1, 5-bromo-6-methoxy-2-(4-methyltetrahydro-2*H*-pyran-4-yl)-2*H*-indazole (80 mg, 0.25 mmol) and 7-ethyl-4-(4-fluoro-3-(4,4,5,5-tetramethyl-1,3,2-dioxaborolan-2-yl)phenyl)-7*H*-imidazo[4,5-c]pyridazine (92 mg, 0.25 mmol) were used as starting materials to obtain the title compound (25 mg, 21%) as a yellow solid.

$^1$H NMR (400 MHz, CHLOROFORM-d) 9.37 (s, 1 H), 8.31 - 8.24 (m, 2 H), 8.20 (dd, *J* = 2.0, 6.8 Hz, 1 H), 8.01 (s, 1 H), 7.62 (s, 1 H), 7.32 (t, *J* = 9.0 Hz, 1 H), 7.14 (s, 1 H), 4.56 (q, *J* = 7.3 Hz, 2 H), 3.90 - 3.79 (m, 5 H), 3.74 - 3.66 (m, 2 H), 2.65 - 2.56 (m, 2 H), 2.17 - 2.08 (m, 2 H), 1.71 - 1.61 (m, 6 H). LC-MS: m/z $[M+H]^+$ = 487.

Example 101 and Example 102

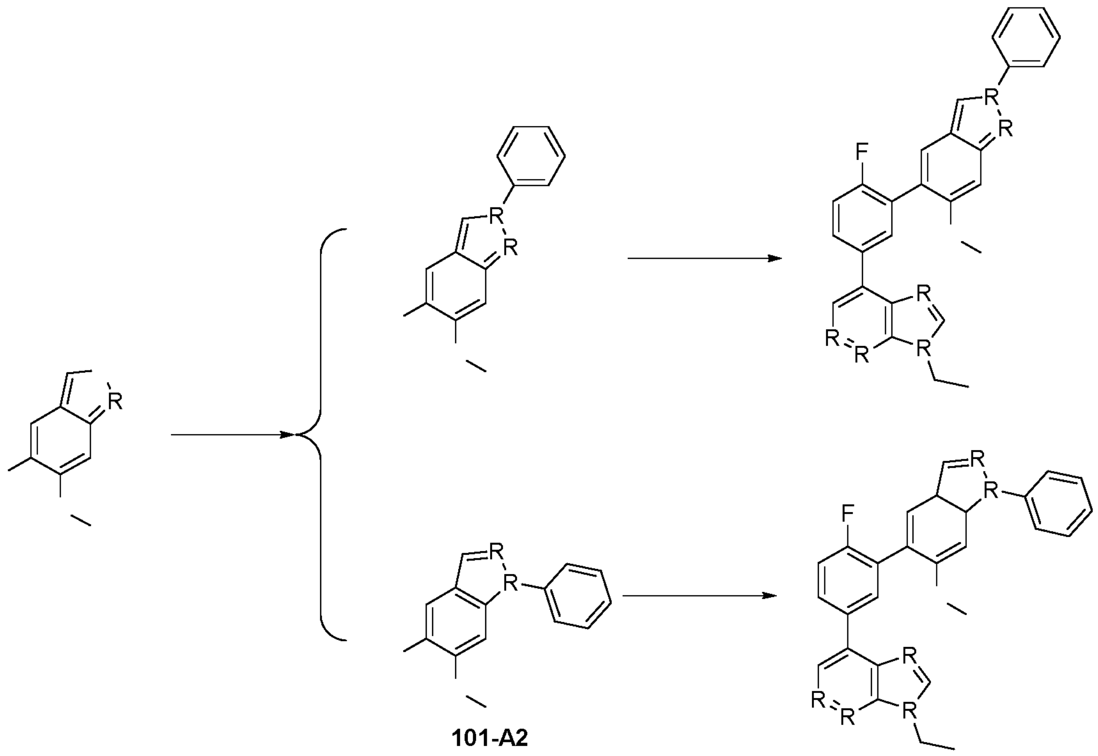

101-A2

**101-A1: 5-Bromo-6-methoxy-2-phenyl-2*H*-indazole**

**101-A2: 5-Bromo-6-methoxy-1-phenyl-1*H*-indazole**

5-Bromo-6-methoxy-1*H*-indazole (200 mg, 0.88 mmol) was dissolved in *N,N*-dimethylformamide (4 mL), then iodobenzene (359 mg, 1.76 mmol), cuprous iodide (17 mg, 0.088 mmol), *N,N*-dimethylethylenediamine (8 mg, 0.088 mmol), and potassium phosphate (560 mg, 2.64 mmol) were sequentially added thereto, and the mixture was stirred at room temperature for 16 hours. The reaction mixture was separated by preparative thin-layer chromatography (petroleum ether/ethyl acetate = 5/1) to obtain 5-bromo-6-methoxy-2-phenyl-2*H*-indazole (20 mg, 8%, Rf = 0.3) and 5-bromo-6-methoxy-1-phenyl-1*H*-indazole (120 mg, 45%, Rf = 0.5). LC-MS: m/z $[M+H]^+$ = 303, 305.

**Example 101: 7-Ethyl-4-(4-fluoro-3-(6-methoxy-2-phenyl-2*H*-indazol-5-yl)phenyl)-7*H*-imidazo[4,5-*c*]pyridazine**

Following the same experimental procedure as in Example 1, 5-bromo-6-methoxy-2-phenyl-2*H*-indazole (20 mg, 0.07 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (37 mg, 0.07 mmol) were used as starting materials to obtain the title compound (8 mg, 25%) as a yellow solid.

$^1$H NMR (400 MHz, CHLOROFORM-d) 9.43 (s, 1 H), 8.39 (s, 1 H), 8.34 - 8.23 (m, 3 H), 7.91 (s, 1 H), 7.89 (s, 1 H), 7.69 (s, 1 H), 7.56 - 7.51 (m, 2 H), 7.42 - 7.34 (m, 2 H), 7.17 (s, 1 H), 4.62 - 4.55 (m, 2 H), 3.90 (s, 3 H), 1.70 (d, *J* = 6.4 Hz, 3 H). LC-MS: m/z $[M+H]^+$ = 465.

**Example 102: 7-Ethyl-4-(4-fluoro-3-(6-methoxy-1-phenyl-3a,7a-dihydro-1*H*-indazol-5-yl)phenyl)-7*H*-imidazo [4,5-c]pyridazine**

Following the same experimental procedure as in Example 1, 5-bromo-6-methoxy-1-phenyl-1*H*-indazole (60 mg, 0.20 mmol) and 7-ethyl-4-(4-fluoro-3-(4,4,5,5-tetramethyl-1,3,2-dioxaborolan-2-yl)phenyl)-7*H*-imidazo[4,5-c]pyridazine (74 mg, 0.20 mmol) were used as starting materials to obtain the title compound (35 mg, 38%) as a yellow solid.

$^1$H NMR (400 MHz, CHLOROFORM-d) 9.40 (s, 1 H), 8.32 - 8.23 (m, 3 H), 8.16 (s, 1 H), 7.79 - 7.74 (m, 3 H), 7.59 (t, *J* = 7.8 Hz, 2 H), 7.44 - 7.32 (m, 2 H), 7.20 (s, 1 H), 4.62 - 4.55 (m, 2 H), 3.88 (s, 3 H), 1.72 - 1.66 (m, 3 H). LC-MS: m/z $[M+H]^+$ = 465.

Example 103

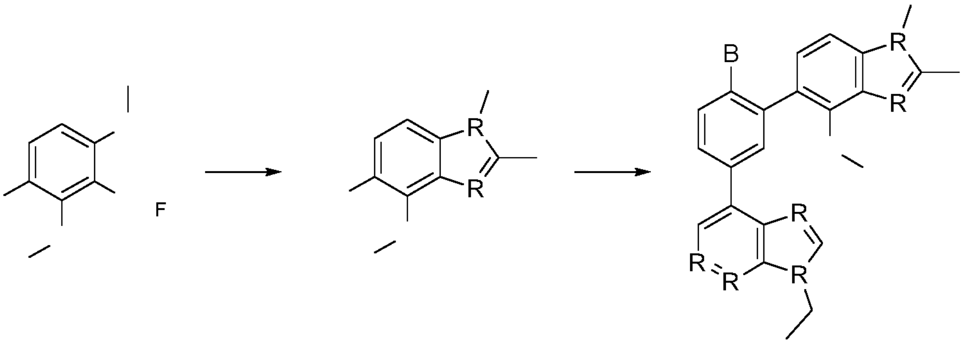

**103-1: 5-Bromo-4-methoxy-1,2-dimethyl-1*H*-benzo[*d*]imidazole**

4-Bromo-3-methoxy-*N*[1]-methylbenzene-1,2-diamine (200 mg, 0.87 mmol) was added to triethyl orthoacetate (5 mL). The mixture was stirred at 90°C overnight. The reaction mixture was directly subjected to thin-layer chromatography (dichloromethane/methanol = 40/1) to obtain the title compound (200 mg, 90%). LC-MS: m/z $[M+H]^+$ = 255.

**103: 7-Ethyl-4-(4-fluoro-3-(4-methoxy-1,2-dimethyl-1*H*-benzo[d]imidazol-5-yl)phenyl)-7*H*-imidazo[4,5-c]pyridazine**

Following the same experimental procedure as in Example 1, 5-bromo-4-methoxy-1,2-dimethyl-1*H*-benzo[*d*]imidazole (42 mg, 0.16 mmol) and 7-ethyl-4-(4-fluoro-3-(4,4,5,5-tetramethyl-1,3,2-dioxaborolan-2-yl)phenyl)-7*H*-imidazo[4,5-c]pyridazine (50 mg, 0.13 mmol) were used as starting materials to obtain the title compound (25 mg, 44%).

$^1$H NMR (400 MHz, CHLOROFORM-d) 1.63 - 1.72 (m, 3 H) 2.63 (br. s., 3 H) 3.73 (br. s., 3 H) 4.29 (br. s., 3 H) 4.56 (d, *J* = 7.34 Hz, 2 H) 7.05 (d, *J* = 7.83 Hz, 1 H) 7.22 (br. s., 1 H) 7.34 (t, *J* = 8.07 Hz, 1 H) 8.16 - 8.32 (m, 3 H) 9.37 (br. s., 1 H). LC-MS: m/z $[M+H]^+$ = 417.

Example 104

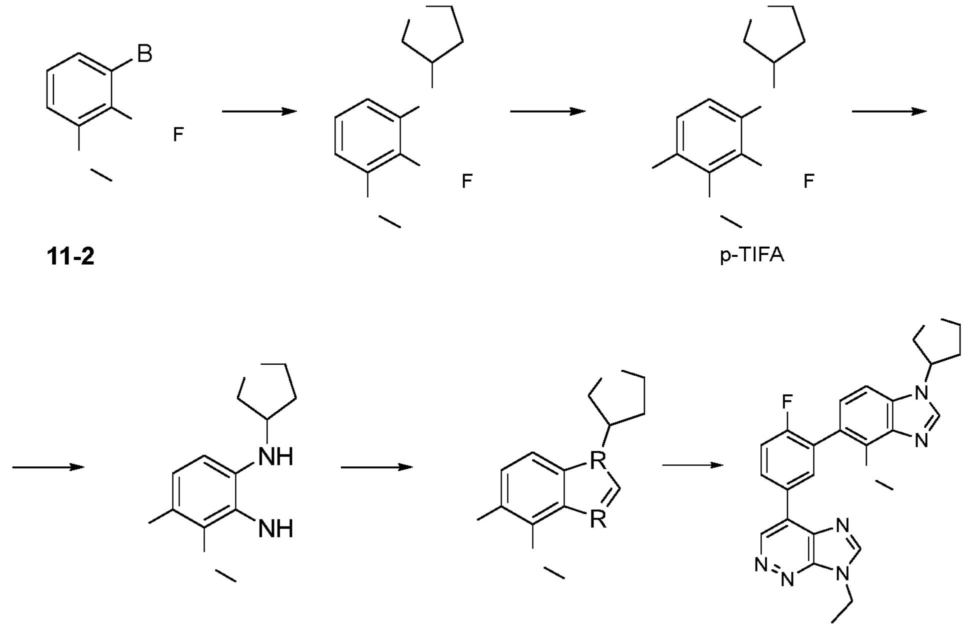

**11-2**

p-TIFA

**104-1: *N*-(3-Methoxy-2-nitrophenyl)tetrahydrofuran-3-amine**

Following the same experimental procedure as in the synthesis method of 57-1 in Example 57, 1-fluoro-3-methoxy-2-nitrobenzene (500 mg, 2.9 mmol) and tetrahydrofuran-3-amine (508 mg, 5.8 mmol) were used as starting

materials to obtain the title compound (700 mg, 100%). LC-MS: m/z $[M+H]^+$ = 239.

**104-2: *N*-(4-Bromo-3-methoxy-2-nitrophenyl)tetrahydrofuran-3-amine**

Following the same experimental procedure as in the synthesis method of 57-2 in Example 57, *N*-(3-methoxy-2-nitrophenyl)tetrahydrofuran-3-amine (700 mg, 2.94 mmol) was used as the starting material to obtain the title compound (1000 mg, 100%). LC-MS: m/z $[M+H]^+$ = 317.

**104-3: 4-Bromo-3-methoxy-*N*$^1$-(tetrahydrofuran-3-yl)benzene-1,2-diamine**

Following the same experimental procedure as in the synthesis method of 57-3 in Example 57, *N*-(4-bromo-3-methoxy-2-nitrophenyl)tetrahydrofuran-3-amine (1000 mg, 3.2 mmol) was used as the starting material to obtain the title compound (700 mg, 77%). LC-MS: m/z $[M+H]^+$ = 287.

**104-4: 5-Bromo-4-methoxy-1-(tetrahydrofuran-3-yl)-1*H*-benzo[d]imidazole**

4-Bromo-3-methoxy-*N*$^1$-(tetrahydrofuran-3-yl)benzene-1,2-diamine (200 mg, 1.75 mmol) was added to triethyl orthoformate (10 mL). The mixture was stirred at 90°C overnight. The reaction mixture was directly subjected to thin-layer chromatography (dichloromethane/methanol = 30/1) to obtain the title compound (140 mg, 68%). LC-MS: m/z $[M+H]^+$ = 297.

**104: 7-Ethyl-4-(4-fluoro-3-(4-methoxy-1-(tetrahydrofuran-3-yl)-1*H*-benzo[*d*]imidazol-5-yl)phenyl)-7*H*-imidazo [4,5-*c*]pyridazine**

Following the same experimental procedure as in Example 1, 5-bromo-4-methoxy-1-(tetrahydrofuran-3-yl)-1*H*-benzo[*d*]imidazole (85 mg, 0.16 mmol) and 7-ethyl-4-(4-fluoro-3-(4,4,5,5-tetramethyl-1,3,2-dioxaborolan-2-yl)phenyl)-7*H*-imidazo[4,5-c]pyridazine (50 mg, 0.13 mmol) were used as starting materials to obtain the title compound (14 mg, 23%).

$^1$H NMR (400 MHz, CHLOROFORM-*d*) 1.68 (br. s., 3 H) 2.32 (br. s., 1 H) 2.60 (dd, *J* = 13.69, 6.36 Hz, 1 H) 3.95 - 4.04 (m, 1 H) 4.11 (dd, *J* = 10.03, 6.11 Hz, 1 H) 4.21 - 4.28 (m, 2 H) 4.37 (br. s., 3 H) 4.55 - 4.60 (m, 2 H) 5.07 (br. s., 1 H) 7.22 (br. s., 1 H) 7.29 - 7.39 (m, 2 H) 8.01 (br. s., 1 H) 8.22 (d, *J* = 5.87 Hz, 1 H) 8.26 (s, 2 H) 9.37 (s, 1 H). LC-MS: m/z $[M+H]^+$ = 459.

Example 105

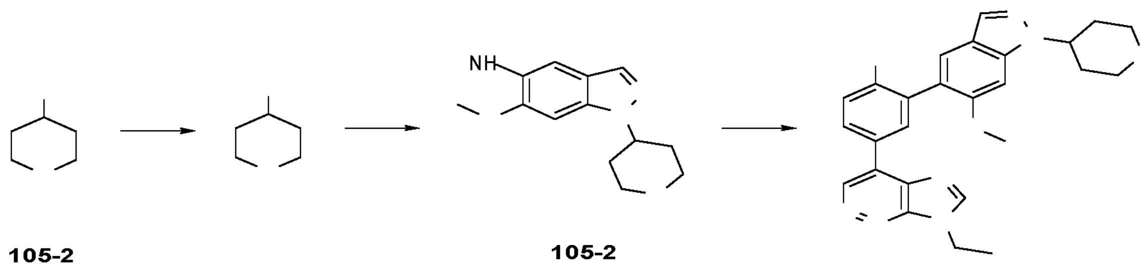

105-2 105-2

**105-1: Tetrahydro-2*H*-pyran-4-yl 4-methylbenzenesulfonate**

Tetrahydropyran-4-ol (CAS: 2081-44-9, 500 mg, 5 mmol) was dissolved in 10 mL of dichloromethane, then *p*-toluenesulfonyl chloride (1 g, 5.3 mmol) and *N,N*-diisopropylethylamine (2 mL) were added thereto, and the mixture was stirred at room temperature for 2 hours. The reaction mixture was washed with saturated ammonium chloride aqueous solution, concentrated, and purified by preparative thin-layer chromatography (petroleum ether: ethyl acetate = 20:1) to obtain the title compound (400 mg, 30%) as a white solid. LC-MS: m/z $[M+H]^+$ = 257.

**105-2: 5-Bromo-6-methoxy-1-(tetrahydro-2H-pyran-4-yl)-1H-indazole**

5-Bromo-6-(methoxy)-1*H*-benzo[d]imidazole (CAS: 1008361-65-6, 354 mg, 1.5 mmol) was dissolved in 10 mL of acetonitrile, then cesium carbonate (1 g, 3 mmol) and tetrahydro-2*H*-pyran-4-yl 4-methylbenzenesulfonate (400 mg, 1.5

mmol) were added thereto, and the mixture was heated to 60°C and stirred for 3 hours. The reaction mixture was filtered to remove the solid, concentrated, and purified by preparative thin-layer chromatography (petroleum ether: ethyl acetate = 5:1) to obtain the title compound (300 mg, 62%) as a white solid. LC-MS: m/z $[M+H]^+$ = 311.

**105: 7-Ethyl-4-(4-fluoro-3-(6-methoxy-1-(tetrahydro-2H-pyran-4-yl)-1H-indazol-5-yl)phenyl)-7H-imidazo[4,5-c] pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-*c*]pyridazine (60 mg, 0.16 mmol) and 5-bromo-6-methoxy-1-(tetrahydro-2*H*-pyran-4-yl)-1*H*-indazole (50 mg, 0.16 mmol) were used as starting materials to obtain the title compound (36 mg, 48%) as a white solid.

$^1$H NMR (400 MHz, CHLOROFORM-d) 9.40 (s, 1 H) 8.28 (s, 2 H) 8.23 (d, *J* = 6.36 Hz, 1 H) 7.98 (s, 1 H) 7.70 (s, 1 H) 7.35 (t, *J* = 9.05 Hz, 1 H) 6.89 (s, 1 H) 4.54 - 4.68 (m, 3 H) 4.22 (d, *J* = 11.74 Hz, 2 H) 3.93 (s, 3 H) 3.68 (t, *J* = 11.74 Hz, 2 H) 2.40 - 2.54 (m, 2 H) 2.04 (d, *J* = 11.74 Hz, 2 H) 1.68 - 1.71 (m, 3 H). LC-MS: m/z $[M+H]^+$ = 473.

Example 106

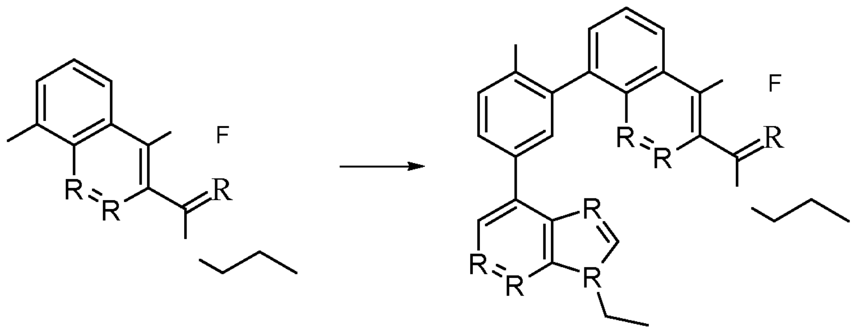

106: **4-Amino-8-(5-(7-ethyl-7*H*-imidazo[4,5-*c*]pyridazin-4-yl)-2-fluorophenyl)-*N*-propylcinnoline-3-carboxamide**

4-Amino-8-iodo-*N*-propylcinnoline-3-carboxamide (40 mg, 0.11 mmol), 7-ethyl-4-(4-fluoro-3-(4,4,5,5-tetramethyl-1,3,2-dioxaborolan-2-yl)phenyl)-7*H*-imidazo[4,5-c]pyridazine (40 mg, 0.11 mmol), [1,1'-bis(diphenylphosphino) ferrocene]dichloropalladium(II) (9 mg, 0.011 mmol), cesium carbonate (108 mg, 0.33 mmol), and water (0.4 mL) were added to 1,4-dioxane (4 mL). After the gas in the system was replaced with nitrogen, the mixture was stirred at 100°C for 2 hours. The reaction mixture was filtered, concentrated, and separated by preparative thin-layer chromatography (dichloromethane/methanol = 20/1) to obtain the title compound (30 mg, 58%) as a brown solid.

$^1$H NMR (400 MHz, CHLOROFORM-*d*) 9.39 (s, 1 H), 8.52 (br. s., 1 H), 8.42 - 8.30 (m, 2 H), 8.26 (s, 1 H), 7.99 (d, *J* = 7.8 Hz, 1 H), 7.86 (d, *J* = 6.8 Hz, 1 H), 7.72 (t, *J* = 7.6 Hz, 1 H), 7.62 - 7.53 (m, 1 H), 7.48 - 7.36 (m, 2 H), 4.56 (q, *J* = 7.3 Hz, 2 H), 3.43 (q, *J* = 6.4 Hz, 2 H), 1.69 - 1.59 (m, 5 H), 0.97 (t, *J* = 7.3 Hz, 3 H). LC-MS: m/z $[M+H]^+$ = 471.

Example 107

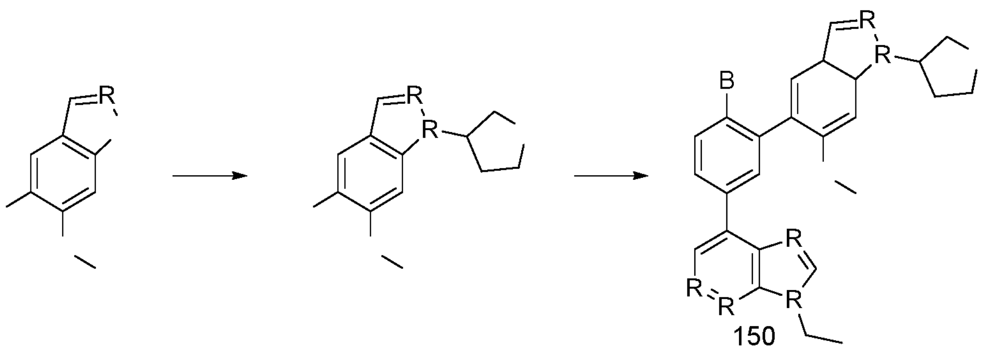

150

**107-1: 5-Bromo-6-methoxy-1-(tetrahydrofuran-3-yl)-1*H*-indazole**

5-Bromo-6-methoxy-1*H*-indazole (200 mg, 0.88 mmol) was dissolved in *N,N*-dimethylformamide (4 mL), then sodium hydride (70 mg, 1.76 mmol) was added thereto at room temperature, and the mixture was stirred at room temperature for 0.5 hours. 3-Iodo-tetrahydrofuran (349 mg, 1.76 mmol) was then added thereto, and the mixture was stirred at room temperature for another 16 hours. The reaction mixture was added with sodium sulfate decahydrate (100 mg), filtered, concentrated, and separated by preparative thin-layer chromatography (petroleum ether/ethyl acetate = 3/1) to obtain the title compound (90 mg, 35%) as a light yellow solid. LC-MS: m/z $[M+H]^+$ = 297, 299.

**107: 7-Ethyl-4-(4-fluoro-3-(6-methoxy-1-(tetrahydrofuran-3-yl)-3a,7a-dihydro-1H-indazol-5-yl)phenyl)-7*H*-imidazo[4,5-*c*]pyridazine**

Following the same experimental procedure as in Example 1, 5-bromo-6-methoxy-1-(tetrahydrofuran-3-yl)-1*H*-indazole (55 mg, 0.19 mmol) and 7-ethyl-4-(4-fluoro-3-(4,4,5,5-tetramethyl-1,3,2-dioxaborolan-2-yl)phenyl)-7*H*-imidazo [4,5-c]pyridazine (70 mg, 0.19 mmol) were used as starting materials to obtain the title compound (40 mg, 46%) as a yellow solid.

$^1$H NMR (400 MHz, CHLOROFORM-d) δ = 9.38 (br. s., 1 H), 8.35 - 8.14 (m, 3 H), 7.96 (s, 1 H), 7.68 (s, 1 H), 7.33 (t, *J* = 8.8 Hz, 1 H), 6.97 (s, 1 H), 5.29 (br. s., 1 H), 4.57 (q, *J* = 7.0 Hz, 2 H), 4.34 - 4.17 (m, 3 H), 4.06 - 3.99 (m, 1 H), 3.90 (s, 3 H), 2.53 (d, *J* = 6.8 Hz, 2 H), 1.68 (t, *J* = 7.1 Hz, 3 H). LC-MS: m/z $[M+H]^+$ = 461.

Example 108

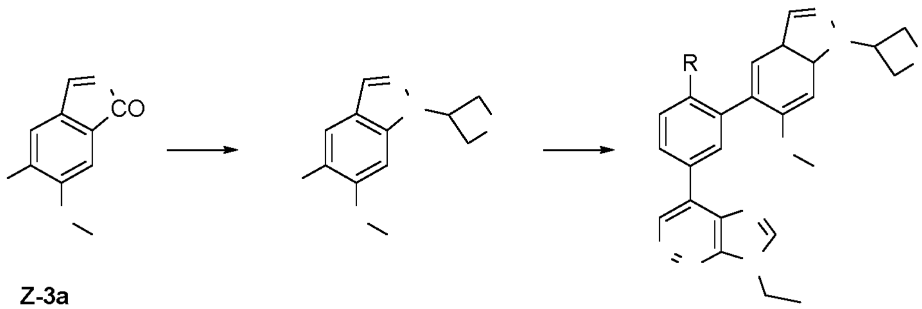

**Z-3a**

**108-1: 5-Bromo-6-methoxy-1-(oxetan-3-yl)-1*H*-indazole**

5-Bromo-6-methoxy-1*H*-indazole (1000 mg, 4.40 mmol) was dissolved in *N,N*-dimethylformamide (8 mL), then sodium hydride (176 mg, 4.40 mmol) was added thereto at room temperature, and the mixture was stirred at room temperature for 0.5 hours. 3-Iodooxetane (1619 mg, 8.80 mmol) was then added thereto, and the mixture was stirred at room temperature for another 16 hours. The reaction mixture was added with sodium sulfate decahydrate (500 mg), filtered, concentrated, and separated by preparative thin-layer chromatography (petroleum ether/ethyl acetate = 3/1) to obtain the title compound (310 mg, 25%) as a light yellow solid. LC-MS: m/z $[M+H]^+$ = 283, 285.

**108: 7-Ethyl-4-(4-fluoro-3-(6-methoxy-1-(oxetan-3-yl)-3a,7a-dihydro-1*H*-indazol-5-yl)phenyl)-7*H*-imidazo[4,5-*c*] pyridazine**

Following the same experimental procedure as in Example 1, 5-bromo-6-methoxy-1-(oxetan-3-yl)-1*H*-indazole (50 mg, 0.18 mmol) and 7-ethyl-4-(4-fluoro-3-(4,4,5,5-tetramethyl-1,3,2-dioxaborolan-2-yl)phenyl)-7*H*-imidazo[4,5-*c*] pyridazine (66 mg, 0.18 mmol) were used as starting materials to obtain the title compound (40 mg, 50%) as a yellow solid.

$^1$H NMR (400 MHz, CHLOROFORM-d) 9.38 (br. s., 1 H), 8.27 (s, 2 H), 8.21 (d, *J* = 6.8 Hz, 1 H), 8.04 (s, 1 H), 7.70 (s, 1 H), 7.34 (t, *J*= *9.0* Hz, 1 H), 7.00 (s, 1 H), 5.79 (quin, *J* = 6.8 Hz, 1 H), 5.34 (t, *J* = 6.4 Hz, 2 H), 5.18 (t, *J* = 7.1 Hz, 2 H), 4.57 (q, *J* = 7.3 Hz, 2 H), 3.91 (s, 3 H), 1.68 (t, *J* = 7.1 Hz, 3 H). LC-MS: m/z $[M+H]^+$ = 447.

Example 109

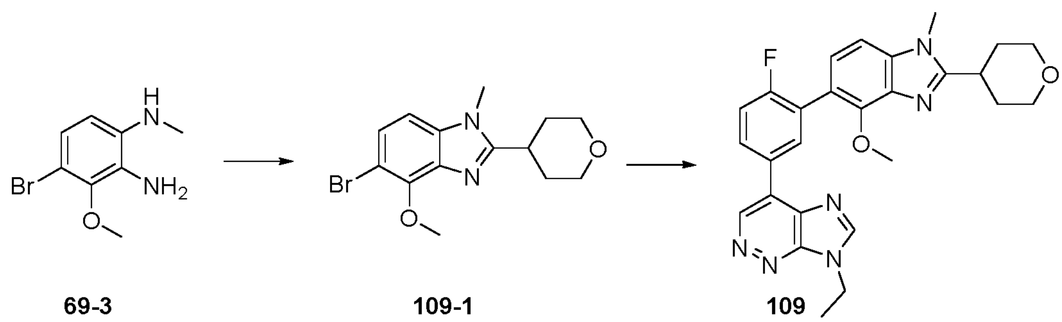

69-3 109-1 109

**109-1: 5-Bromo-4-methoxy-1-methyl-2-(tetrahydro-2*H*-pyran-4-yl)-1*H*-benzo[*d*]imidazole**

4-Bromo-3-methoxy-$N^1$-methylbenzene-1,2-diamine (200 mg, 0.87 mmol) and tetrahydropyran-4-carbaldehyde (100 mg, 0.87 mmol) were added to tetrahydrofuran (10 mL) and acetic acid (0.5 mL). The mixture was stirred at 65°C overnight under an argon atmosphere. The reaction mixture was directly subjected to thin-layer chromatography to obtain the title compound (100 mg, 36%). LC-MS: m/z $[M+H]^+$ = 325.

**109: 7-Ethyl-4-(4-fluoro-3-(4-methoxy-1-methyl-2-(tetrahydro-2*H*-pyran-4-yl)-1*H*-benzo[d]imidazol-5-yl)phenyl)-7*H*-imidazo[4,5-c]pyridazine**

Following the same experimental procedure as in Example 1, 5-bromo-4-methoxy-1-methyl-2-(tetrahydro-2*H*-pyran-4-yl)-1*H*-benzo[d]imidazole (85 mg, 0.26 mmol) and 7-ethyl-4-(4-fluoro-3-(4,4,5,5-tetramethyl-1,3,2-dioxaborolan-2-yl)phenyl)-7*H*-imidazo[4,5-c]pyridazine (50 mg, 0.13 mmol) were used as starting materials to obtain the title compound (24 mg, 36.3%).
$^1$H NMR (400 MHz, CHLOROFORM-d) 1.70 (br. s., 3 H) 1.92 (d, *J* = 11.74 Hz, 2 H) 2.21 (br. s., 2 H) 3.15 (br. s., 1 H) 3.62 (t, *J* = 10.03 Hz, 2 H) 3.78 (br. s., 3 H) 4.15 (d, *J* = 10.76 Hz, 2 H) 4.37 (s, 3 H) 4.57 (d, *J* = 6.85 Hz, 2 H) 7.05 (d, *J* = 6.85 Hz, 1 H) 7.20 - 7.23 (m, 1 H) 7.31 - 7.38 (m, 1 H) 8.19 - 8.29 (m, 3 H) 9.37 (br. s., 1 H). LC-MS: m/z $[M+H]^+$ = 487.

Example 110 and Example 111

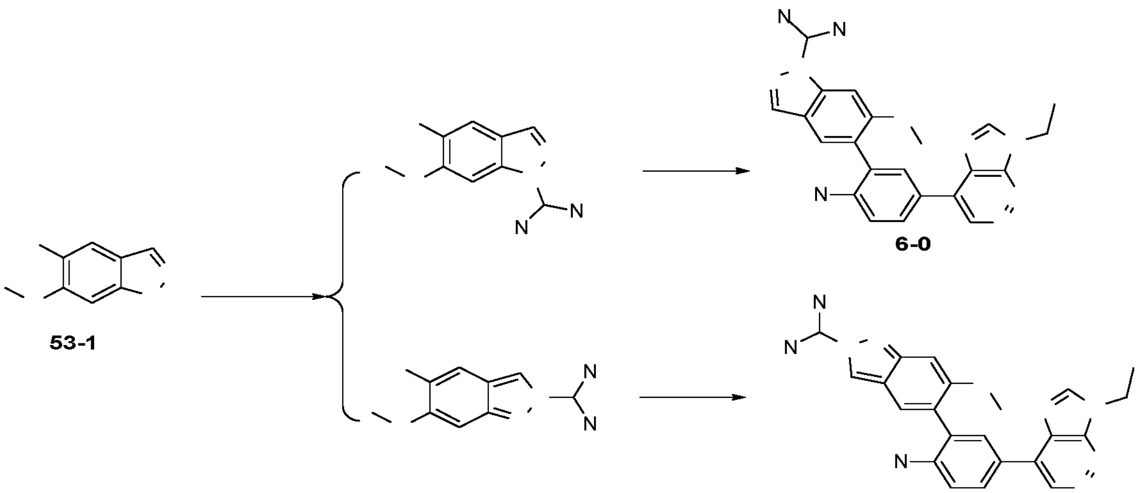

53-1 6-0

**110-A1: 5-Bromo-1-(difluoromethyl)-6-methoxy-1H-indazole**

**110-A2: 5-Bromo-2-(difluoromethyl)-6-methoxy-2H-indazole**

5-Bromo-6-(methoxy)-1*H*-benzo[*d*]imidazole (CAS: 1008361-65-6, 440 mg, 1.9 mmol) was dissolved in 10 mL of *N,N*-dimethylformamide, then cesium carbonate (2 g, 5.8 mmol), potassium fluoride (1 g, 5.8 mmol), and sodium difluorochloroacetate (589 mg, 3.8 mmol) were added thereto, and the mixture was heated to 100°C and stirred overnight. The reaction mixture was filtered to remove the solid, and purified by preparative thin-layer chromatography (petroleum

ether: ethyl acetate = 3:1) to obtain the title compound A1 (80 mg, 15%, developing solvent: petroleum ether: ethyl acetate = 3:1, Rf = 0.7) as a white solid relatively small polarity and the title compound A2 (200 mg, 38%, developing solvent: petroleum ether: ethyl acetate = 3:1, Rf = 0.5) as a yellow solid relatively large polarity. LC-MS: m/z $[M+H]^+$ = 277.

**110: 4-(3-(1-(Difluoromethyl)-6-methoxy-1H-indazol-5-yl)-4-fluorophenyl)-7-ethyl-7H-imidazo[4,5-c]pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (60 mg, 0.16 mmol) and 5-bromo-1-(difluoromethyl)-6-methoxy-1*H*-indazole (50 mg, 0.16 mmol) were used as starting materials to obtain the title compound (36 mg, 51%) as a white solid.

$^1$H NMR (400 MHz, CHLOROFORM-*d*) δ 9.38 (s, 1 H) 8.28 (s, 2 H) 8.23 (d, *J* = 6.85 Hz, 1 H) 8.04 (s, 1 H) 7.72 (s, 1 H) 7.33 - 7.65 (m, 2 H) 7.27 (s, 1 H) 4.58 (q, *J* = 7.34 Hz, 2 H) 3.93 (s, 3 H) 1.69 (t, *J* = 7.34 Hz, 3 H). LC-MS: m/z $[M+H]^+$ = 439.

**111: 4-(3-(2-(Difluoromethyl)-6-methoxy-2*H*-indazol-5-yl)-4-fluorophenyl)-7-ethyl-7*H*-imidazo[4,5-*c*]pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (60 mg, 0.16 mmol) and 5-bromo-2-(difluoromethyl)-6-methoxy-2*H*-indazole (50 mg, 0.16 mmol) were used as starting materials to obtain the title compound (32 mg, 46%) as a yellow solid.

$^1$H NMR (400 MHz, CHLOROFORM-d) 9.40 (s, 1 H) 8.30 (d, *J* = 5.87 Hz, 3 H) 8.23 (d, *J* = 6.85 Hz, 1 H) 7.67 (s, 1 H) 7.28 - 7.61 (m, 2 H) 7.06 (s, 1 H) 4.59 (q, *J* = 7.17 Hz, 2 H) 3.89 (s, 3 H) 1.70 (s, 3 H). LC-MS: m/z $[M+H]^+$ = 439.

Example 112

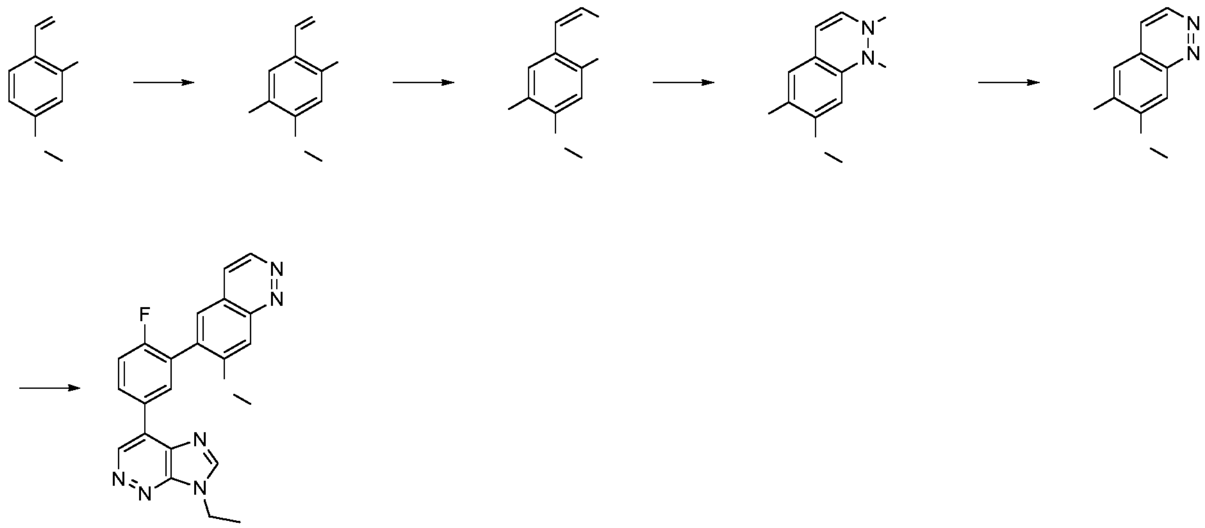

**112-1: 2,5-Dibromo-4-methoxybenzaldehyde**

2-Bromo-4-methoxybenzaldehyde (5000 mg, 23.26 mmol) and pyridinium tribromide (14886 mg, 46.52 mmol) were added to methanol (50 mL), and the mixture was stirred at 35°C for 16 hours. The reaction mixture was added dropwise to saturated ammonium chloride aqueous solution (100 mL) and filtered to obtain the title compound (6000 mg, 88%) as a white solid. LC-MS: m/z $[M+H]^+$ = 293, 295.

**112-2: (Z)-1,4-Dibromo-2-(2-bromovinyl)-5-methoxybenzene**

(Bromomethyl)triphenylphosphonium bromide (8000 mg, 18.35 mmol) was dissolved in tetrahydrofuran (50 mL), then a solution of potassium *tert*-butoxide (3394 mg, 30.30 mmol) in tetrahydrofuran (20 mL) was added dropwise to the reaction mixture at -78°C, and the mixture was stirred at -78°C for 2 hours. 2,5-Dibromo-4-methoxybenzaldehyde (4000 mg, 15.15 mmol) was then added thereto, and the mixture was stirred at room temperature for another 16 hours. The reaction mixture was concentrated and separated by column chromatography (petroleum ether) to obtain the title

compound (1500 mg, 27%) as a white solid. LC-MS: m/z $[M+H]^+$ = 369, 371.

**112-3: Diethyl 6-bromo-7-methoxycinnoline-1,2-dicarboxylate**

(*Z*)-1,4-Dibromo-2-(2-bromovinyl)-5-methoxybenzene (1500 mg, 4.05 mmol), diethyl hydrazine-1,2-dicarboxylate (1426 mg, 8.10 mmol), potassium carbonate (1397 mg, 10.13 mmol), cuprous iodide (77 mg, 0.405 mmol), and *N,N*-dimethylethylenediamine (179 mg, 2.03 mmol) were added to 1,4-dioxane (10 mL). After the gas in the system was replaced with nitrogen, the mixture was stirred at 90°C for 16 hours. The reaction mixture was added with water (40 mL), extracted with ethyl acetate (30 mL × 3), and the organic phase was separated by column chromatography (petroleum ether/ethyl acetate = 10/1) to obtain the title compound (950 mg, 61%) as a yellow oily liquid. LC-MS: m/z $[M+H]^+$ = 385, 387.

**112-4: 6-Bromo-7-methoxycinnoline**

Diethyl 6-bromo-7-methoxycinnoline-1,2-dicarboxylate (950 mg, 2.47 mmol) and sodium hydroxide (99 mg, 2.47 mmol) were added to ethanol (20 mL), and the mixture was stirred at room temperature for 16 hours. The ethanol was concentrated to dryness, and the residue was separated by preparative thin-layer chromatography (petroleum ether/ethyl acetate = 1/1) to obtain the title compound (300 mg, 51%) as a yellow solid. LC-MS: m/z $[M+H]^+$ = 239, 241.

**112: 6-(5-(7-Ethyl-7H-imidazo[4,5-c]pyridazin-4-yl)-2-fluorophenyl)-7-methoxycinnoline**

Following the same experimental procedure as in Example 1, 6-bromo-7-methoxycinnoline (200 mg, 0.84 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (309 mg, 0.84 mmol) were used as starting materials to obtain the title compound (135 mg, 27%) as a white solid.

$^1$H NMR (400 MHz, CHLOROFORM-*d*) 9.39 (s, 1 H), 9.27 (d, *J* = 5.4 Hz, 1 H), 8.39 - 8.26 (m, 3 H), 7.92 (s, 1 H), 7.88 - 7.80 (m, 2 H), 7.45 - 7.36 (m, 1 H), 4.59 (q, *J* = 7.0 Hz, 2 H), 4.05 (s, 3 H), 1.69 (t, *J* = 7.1 Hz, 3 H). LC-MS: m/z $[M+H]^+$ = 401.

Example 113

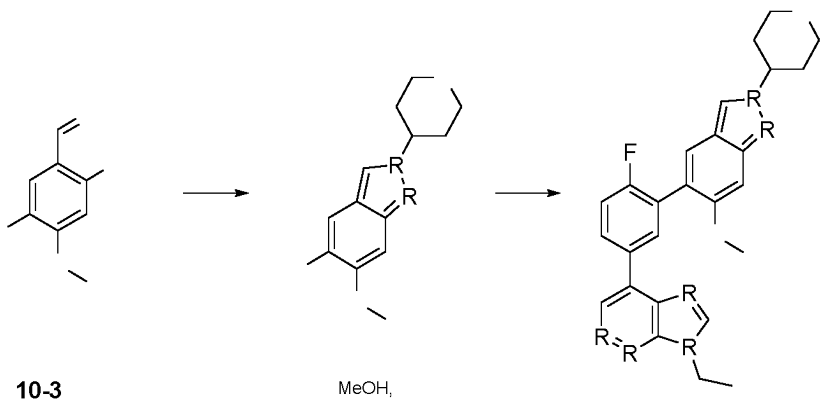

**10-3**

MeOH,

**113-1: 5-Bromo-6-methoxy-2-(tetrahydro-2H-pyran-4-yl)-2H-indazole**

Following the same experimental procedure as in the synthesis method of 40-1 and 40-2 in Example 40, 5-bromo-4-methoxy-2-nitrobenzaldehyde (500 mg, 1.92 mmol) and tetrahydro-2*H*-pyran-4-amine (388 mg, 3.84 mmol) were used as starting materials to obtain the title compound (500 mg, 84%) as a light yellow oily liquid. LC-MS: m/z $[M+H]^+$ = 311, 313.

**113: 7-Ethyl-4-(4-fluoro-3-(6-methoxy-2-(tetrahydro-2H-pyran-4-yl)-2H-indazol-5-yl)phenyl)-7H-imidazo[4,5-c] pyridazine**

Following the same experimental procedure as in Example 1, 5-bromo-6-methoxy-2-(tetrahydro-2*H*-pyran-4-yl)-2*H*-indazole (200 mg, 0.64 mmol) and 7-ethyl-4-(4-fluoro-3-(4,4,5,5-tetramethyl-1,3,2-dioxaborolan-2-yl)phenyl)-7*H*-

imidazo[4,5-c]pyridazine (236 mg, 0.64 mmol) were used as starting materials to obtain the title compound (140 mg, 46%) as a yellow solid.

$^1$H NMR (400 MHz, CHLOROFORM-d) 9.36 (s, 1 H), 8.25 (s, 2 H), 8.19 (d, *J* = 6.8 Hz, 1 H), 7.92 (s, 1 H), 7.61 (s, 1 H), 7.31 (t, *J* = 9.0 Hz, 1 H), 7.10 (s, 1 H), 4.56 (td, *J* = 7.5, 14.8 Hz, 3 H), 4.15 (d, *J* = 11.2 Hz, 2 H), 3.85 (s, 3 H), 3.64 - 3.54 (m, 2 H), 2.23 (br. s., 4 H), 1.66 (t, *J* = 7.1 Hz, 3 H). LC-MS: m/z $[M+H]^+$ = 473.

Example 114

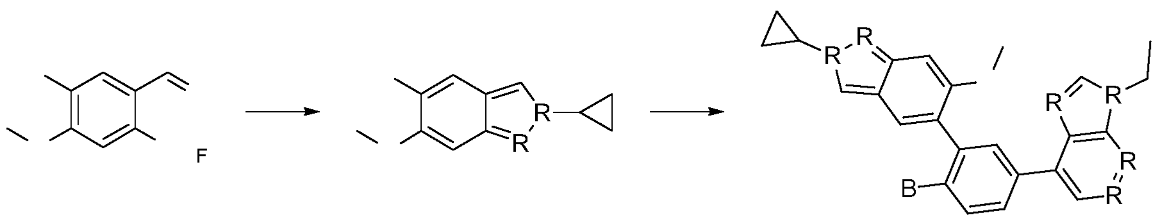

**114-1**

**114-1: 5-Bromo-2-cyclopropyl-6-methoxy-2H-indazole**

Following the same experimental procedure as in the synthesis method of 40-1 and 40-2 in Example 40, 5-bromo-4-methoxy-2-nitrobenzaldehyde (100 mg, 0.38 mmol) and cyclopropylamine (43 mg, 0.76 mmol) were used as starting materials to obtain the title compound (70 mg, 69%) as a light yellow oily liquid. LC-MS: m/z $[M+H]^+$ = 267, 269.

**114: 4-(3-(2-Cyclopropyl-6-methoxy-2H-indazol-5-yl)-4-fluorophenyl)-7-ethyl-7H-imidazo[4,5-c]pyridazine**

Following the same experimental procedure as in Example 1, 5-bromo-2-cyclopropyl-6-methoxy-2*H*-indazole (70 mg, 0.26 mmol) and 7-ethyl-4-(4-fluoro-3-(4,4,5,5-tetramethyl-1,3,2-dioxaborolan-2-yl)phenyl)-7*H*-imidazo[4,5-c]pyridazine (96 mg, 0.26 mmol) were used as starting materials to obtain the title compound (15 mg, 13%) as a yellow solid.

$^1$H NMR (400 MHz, CHLOROFORM-d) $\delta$ = 9.35 (br. s., 1 H), 8.24 (br. s., 2 H), 8.17 (d, *J* = 5.9 Hz, 1 H), 7.93 (s, 1 H), 7.56 (s, 1 H), 7.30 (t, *J* = 8.8 Hz, 1 H), 7.05 (br. s., 1 H), 4.55 (d, *J* = 7.3 Hz, 2 H), 3.89 (br. s., 1 H), 3.83 (s, 3 H), 1.66 (t, *J* = 7.1 Hz, 3 H), 1.31 (br. s., 2 H), 1.14 (d, *J* = 6.4 Hz, 2 H). LC-MS: m/z $[M+H]^+$ = 429.

Example 115

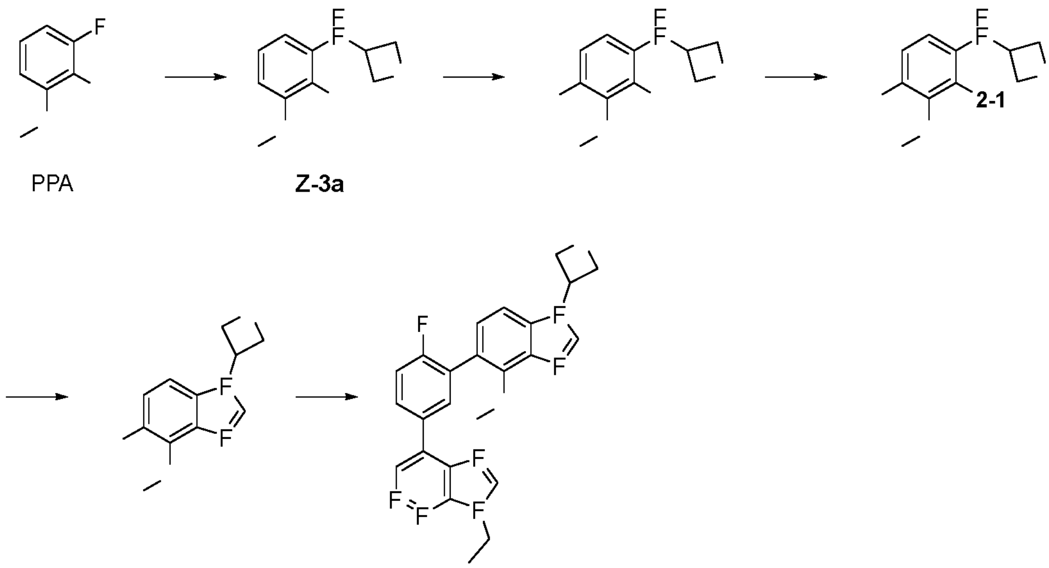

PPA Z-3a 2-1

**115-1: *N*-(3-Methoxy-2-nitrophenyl)oxetan-3-amine**

Following the same experimental procedure as in the synthesis method of 57-1 in Example 57, 1-fluoro-3-methoxy-2-nitrobenzene (500 mg, 2.9 mmol) was used as the starting material to obtain the title compound (400 mg, 62%) as a yellow oil. LC-MS: m/z $[M+H]^+$ = 225.

**115-2: *N*-(4-Bromo-3-methoxy-2-nitrophenyl)oxetan-3-amine**

Following the same experimental procedure as in the synthesis method of 57-2 in Example 57, *N*-(3-methoxy-2-nitrophenyl)oxetan-3-amine (400 mg, 1.79 mmol) was used as the starting material to obtain the title compound (400 mg, 74%) as a yellow oil. LC-MS: m/z $[M+H]^+$ = 303.

**115-3: 4-Bromo-3-methoxy-*N*1-(oxetan-3-yl)benzene-1,2-diamine**

Following the same experimental procedure as in the synthesis method of 57-3 in Example 57, *N*-(4-bromo-3-methoxy-2-nitrophenyl)oxetan-3-amine (400 mg, 1.32 mmol) was used as the starting material to obtain the title compound (300 mg, 84%) as a red oil. LC-MS: m/z $[M+H]^+$ = 273.

**115-4: 5-Bromo-4-methoxy-1-(oxetan-3-yl)-1*H*-benzo[*d*]imidazole**

Following the same experimental procedure as in the synthesis method of 57-4 in Example 57, 4-bromo-3-methoxy-*N*1-(oxetan-3-yl)benzene-1,2-diamine (300 mg, 1.1 mmol) was used as the starting material to obtain the title compound (140 mg, 45%) as a red oil. LC-MS: m/z $[M+H]^+$ = 283.

**115: 7-Ethyl-4-(4-fluoro-3-(4-methoxy-1-(oxetan-3-yl)-1*H*-benzo[d]imidazol-5-yl)phenyl)-7*H*-imidazo[4,5-*c*]pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (50 mg, 0.136 mmol) and 5-bromo-4-methoxy-1-(oxetan-3-yl)-1*H*-benzo[d]imidazole (60 mg, 0.21 mmol) were used as starting materials to obtain the title compound (13 mg, 22%).

$^1H$ NMR (400 MHz, CHLOROFORM-*d*) 9.37 (br. s., 1 H) 8.19 - 8.32 (m, 3 H) 8.03 (br. s., 1 H) 7.45 (d, *J* = 7.83 Hz, 1 H) 7.31 - 7.39 (m, 2 H) 5.56 (br. s., 1 H) 5.14 - 5.27 (m, 4 H) 4.56 (d, *J* = 7.34 Hz, 2 H) 4.36 (br. s., 3 H) 1.66 - 1.69 (m, 3 H). LC-MS: m/z $[M+H]^+$ = 445.

Example 116

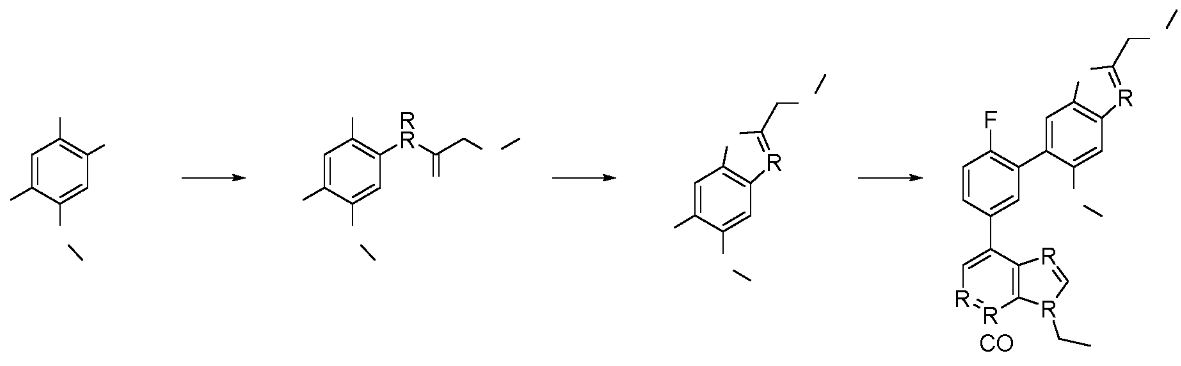

**116-1: *N*-(2,4-Dibromo-5-methoxyphenyl)-2-methoxyacetamide**

2,4-Dibromo-5-methoxyaniline (200 mg, 0.71 mmol), triethylamine (36 mg, 0.35 mmol), and 2-methoxyacetyl chloride (154 mg, 1.42 mmol) were added to dichloromethane (4 mL), and the mixture was stirred at room temperature for 1 hour. The reaction mixture was concentrated and directly purified by preparative thin-layer chromatography (petroleum ether/ethyl acetate = 3/1) to obtain the title compound (300 mg, 80%). LC-MS: m/z $[M+H]^+$ = 354.

**116-2: 6-Bromo-5-methoxy-2-(methoxymethyl)benzo[d]oxazole**

*N*-(2,4-Dibromo-5-methoxyphenyl)-2-methoxyacetamide (300 mg, 0.85 mmol), potassium carbonate (293 mg, 2.12 mmol), cuprous iodide (16 mg, 0.09 mmol), and *N,N*-dimethylethylenediamine (37.4 mg, 0.43 mmol) were added to toluene (6 mL), and the mixture was stirred at 110°C for 16 hours. The reaction mixture was concentrated and directly purified by preparative thin-layer chromatography (petroleum ether/ethyl acetate = 3/1) to obtain the title compound (60 mg, 26%). LC-MS: m/z $[M+H]^+$ = 272, 274.

**116: 6-(5-(7-Ethyl-7H-imidazo[4,5-c]pyridazin-4-yl)-2-fluorophenyl)-5-methoxy-2-(methoxymethyl)benzo[d]oxazole**

Following the same experimental procedure as in Example 1, 6-bromo-5-methoxy-2-(methoxymethyl)benzoxazole (60 mg, 0.22 mmol) and 7-ethyl-4-(4-fluoro-3-(4,4,5,5-tetramethyl-1,3,2-dioxaborolan-2-yl)phenyl)-7*H*-imidazo [4,5-c]pyridazine (68 mg, 0.17 mmol) were used as starting materials to obtain the title compound (10 mg, 11%).

$^1$H NMR (400 MHz, $CDCl_3$) δ 9.38 (br. s., 1 H), 8.32 - 8.20 (m, 3 H), 7.54 (s, 1 H), 7.38 - 7.34 (m, 1 H), 7.32 (s, 1 H), 4.72 (s, 2 H), 4.57 (q, *J* = 7.0 Hz, 2 H), 3.92 - 3.83 (m, 3 H), 3.54 (s, 3 H), 1.72 - 1.67 (m, 3 H). LC-MS: m/z $[M+H]^+$ = 434.

Example 117

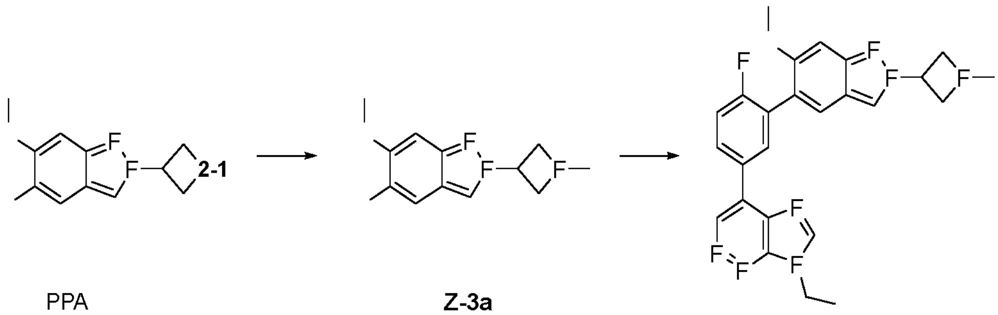

PPA

**Z-3a**

**117-1: 5-Bromo-6-methoxy-2-(1-methylazetidin-3-yl)-2*H*-indazole**

2-(Azetidin-3-yl)-5-bromo-6-methoxy-2*H*-indazole (150 mg, 0.5 mmol) was dissolved in 20 mL of methanol, then 37% formaldehyde aqueous solution (1 mL) and sodium cyanoborohydride (620 mg, 10 mmol) were added thereto, and the mixture was stirred at room temperature overnight. The reaction mixture was concentrated and dissolved in dichloromethane. The resulting mixture was filtered to remove the insoluble matter, concentrated, and purified by preparative thin-layer chromatography (dichloromethane: methanol = 10:1) to obtain the title compound (100 mg, 64%) as a white solid. LC-MS: m/z $[M+H]^+$ = 296.

**117: 7-Ethyl-4-(4-fluoro-3-(6-methoxy-2-(1-methylazetidin-3-yl)-2*H*-indol-5-yl)phenyl)-7*H*-imidazo[4,5-*c*]pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-*c*]pyridazine (60 mg, 0.16 mmol) and 5-bromo-6-methoxy-2-(1-methylazetidin-3-yl)-2*H*-indazole (50 mg, 0.16 mmol) were used as starting materials to obtain the title compound (20 mg, 27%) as a yellow solid.

$^1$H NMR (400 MHz, CHLOROFORM-*d*) δ 9.38 (s, 1 H), 8.27 (s, 2 H), 8.21 (d, *J* = 6.4 Hz, 1 H), 8.03 (s, 1 H), 7.62 (s, 1 H), 7.34 (t, *J* = 9.0 Hz, 1 H), 7.12 (s, 1 H), 5.28 (d, *J* = 17.1 Hz, 1 H), 4.58 (q, *J* = 7.2 Hz, 2 H), 4.17 (br. s., 2 H), 3.88 (br. s., 5 H), 2.67 (br. s., 3 H), 1.71 - 1.68 (m, 3 H). LC-MS: m/z $[M+H]^+$ = 458.

Example 118

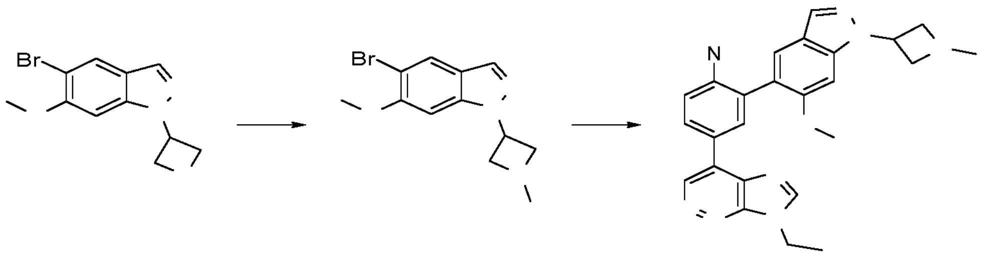

### 118-1: 5-Bromo-6-methoxy-1-(1-methylazetidin-3-yl)-1*H*-indazole

1-(Azetidin-3-yl)-5-bromo-6-methoxy-1*H*-indazole (150 mg, 0.5 mmol) was dissolved in 20 mL of methanol, then 37% formaldehyde aqueous solution (1 mL) and sodium cyanoborohydride (620 mg, 10 mmol) were added thereto, and the mixture was stirred at room temperature overnight. The reaction mixture was concentrated and dissolved in dichloromethane. The resulting mixture was filtered to remove the insoluble matter, concentrated, and purified by preparative thin-layer chromatography (dichloromethane: methanol = 10:1) to obtain the title compound (100 mg, 64%) as a white solid. LC-MS: m/z $[M+H]^+$ = 296.

### 118: 7-Ethyl-4-(4-fluoro-3-(6-methoxy-1-(1-methylazetidin-3-yl)-1*H*-indazol-5-yl)phenyl)-7*H*-imidazo[4,5-*c*]pyridazine

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (60 mg, 0.16 mmol) and 5-bromo-6-methoxy-1-(1-methylazetidin-3-yl)-1*H*-indazole (50 mg, 0.16 mmol) were used as starting materials to obtain the title compound (20 mg, 27%) as a yellow solid.

$^1$H NMR (400 MHz, CHLOROFORM-d) δ 9.38 (s, 1 H) 8.28 (s, 2 H) 8.20 (d, *J* = 5.87 Hz, 1 H) 8.05 (s, 1 H) 7.68 (s, 1 H) 7.34 (t, *J* = 8.80 Hz, 1 H) 6.93 (s, 1 H) 5.50 (br. s., 1 H) 4.58 (q, *J* = 7.34 Hz, 2 H) 4.51 (t, *J* = 7.58 Hz, 2 H) 4.13 (t, *J* = 7.83 Hz, 2 H) 3.92 (s, 3 H) 2.85 (s, 3 H) 1.69 (t, *J* = 7.34 Hz, 3 H). LC-MS: m/z $[M+H]^+$ = 458.

Example 119

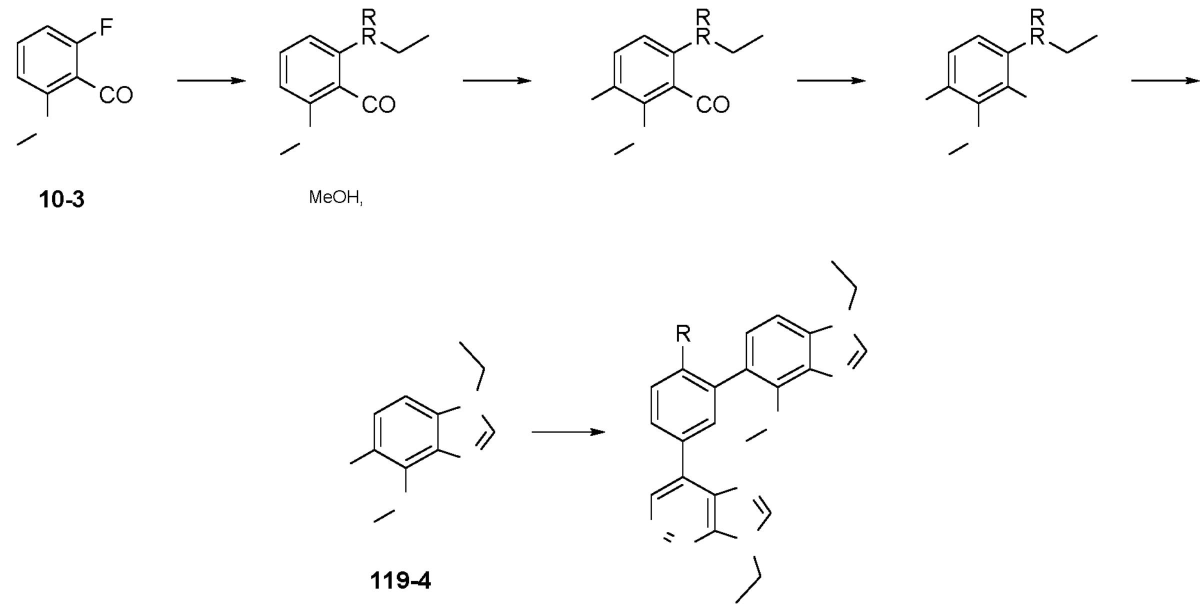


**10-3**

**119-4**

### 119-1: *N*-Ethyl-3-methoxy-2-nitroaniline

Following the same experimental procedure as in the synthesis method of 57-1 in Example 57, 1-fluoro-3-methoxy-2-nitrobenzene (500 mg, 2.9 mmol) was used as the starting material to obtain the title compound (450 mg, 79%)

as a yellow oil. LC-MS: m/z $[M+H]^+$ = 197.

**119-2: 4-Bromo-*N*-ethyl-3-methoxy-2-nitroaniline**

Following the same experimental procedure as in the synthesis method of 57-2 in Example 57, *N*-ethyl-3-methoxy-2-nitroaniline (400 mg, 2.04 mmol) was used as the starting material to obtain the title compound (350 mg, 62.6%) as a yellow oil. LC-MS: m/z $[M+H]^+$ = 275.

**119-3: 4-Bromo-*N*$^1$-ethyl-3-methoxybenzene-1,2-diamine**

Following the same experimental procedure as in the synthesis method of 57-3 in Example 57, 4-bromo-*N*-ethyl-3-methoxy-2-nitroaniline (350 mg, 1.28 mmol) was used as the starting material to obtain the yellow title compound (300 mg, 96%). LC-MS: m/z $[M+H]^+$ = 245.

**119-4: 5-Bromo-1-ethyl-4-methoxy-1*H*-benzo[*d*]imidazole**

Following the same experimental procedure as in the synthesis method of 57-4 in Example 57, 4-bromo-*N*$^1$-ethyl-3-methoxybenzene-1,2-diamine (300 mg, 1.2 mmol) was used as the starting material to obtain the title compound (200 mg, 66%) as a yellow solid. LC-MS: m/z $[M+H]^+$ = 255.

**119: 7-Ethyl-4-(3-(1-ethyl-4-methoxy-1*H*-benzo[*d*]imidazol-5-yl)-4-fluorophenyl)-7*H*-imidazo[4,5-c]pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-*c*]pyridazine (50 mg, 0.136 mmol) and 5-bromo-1-ethyl-4-methoxy-1*H*-benzo [d]imidazole (69 mg, 0.27 mmol) were used as starting materials to obtain the title compound (48 mg, 85%).

$^1$H NMR (400 MHz, CHLOROFORM-*d*) 9.37 (s, 1 H) 8.26 (s, 2 H) 8.22 (d, *J* = 5.87 Hz, 1 H), 7.93 (br. s., 1 H) 7.28 - 7.39 (m, 2 H) 7.16 (d, *J* = 8.31 Hz, 1 H) 4.57 (q, *J* = 7.01 Hz, 2 H) 4.37 (br. s., 3 H) 4.26 (q, *J* = 7.01 Hz, 2 H) 1.68 (t, *J* = 7.34 Hz, 3 H) 1.58 (t, *J* = 7.09 Hz, 3 H). LC-MS: m/z $[M+H]^+$ = 417.

Example 120

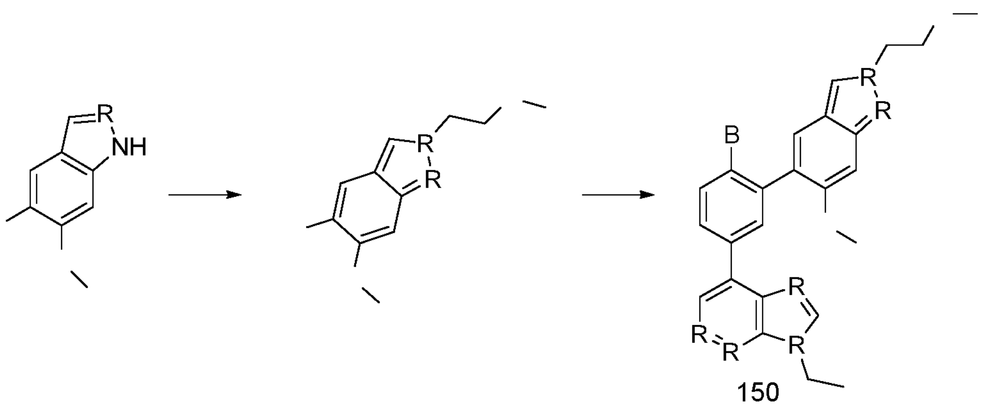

150

**120-1: 5-Bromo-6-methoxy-2-(2-methoxyethyl)-2*H*-indazole**

5-Bromo-6-methoxy-1*H*-indazole (200 mg, 0.9 mmol), sodium hydride (108 mg, 4.5 mmol), and 1-bromo-2-methoxyethane (187 mg, 1.4 mmol) were added to anhydrous *N,N*-dimethylformamide (8 mL), and the mixture was stirred at room temperature for 16 hours. The reaction mixture was concentrated, diluted with water, and extracted with ethyl acetate. The organic phase was concentrated and purified by preparative thin-layer chromatography (ethyl acetate) to obtain the title compound (80 mg, 31.3%). LC-MS: m/z $[M+H]^+$ = 285, 287.

**120: 7-Ethyl-4-(4-fluoro-3-(6-methoxy-2-(2-methoxyethyl)-2*H*-indazol-5-yl)phenyl)-7*H*-imidazo[4,5-*c*]pyridazine**

Following the same experimental procedure as in Example 1, 5-bromo-6-methoxy-2-(2-methoxyethyl)-2*H*-indazole (80 mg, 0.28 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (87 mg, 0.28 mmol) were used as starting materials to obtain the title compound (60 mg, 61%).

$^{1}H$ NMR (400 MHz, $CDCl_3$) δ 9.37 (s, 1 H), 8.30 - 8.24 (m, 2 H), 8.23 - 8.18 (m, 1 H), 7.97 (s, 1 H), 7.61 (s, 1 H), 7.32 (t, *J* = 9.0 Hz, 1 H), 7.09 (s, 1 H), 4.59 - 4.52 (m, 4 H), 3.90 - 3.86 (m, 2 H), 3.85 (s, 3 H), 3.34 (s, 3 H), 1.71 - 1.66 (m, 3 H). LC-MS: m/z $[M+H]^+$ = 447.

Example 121

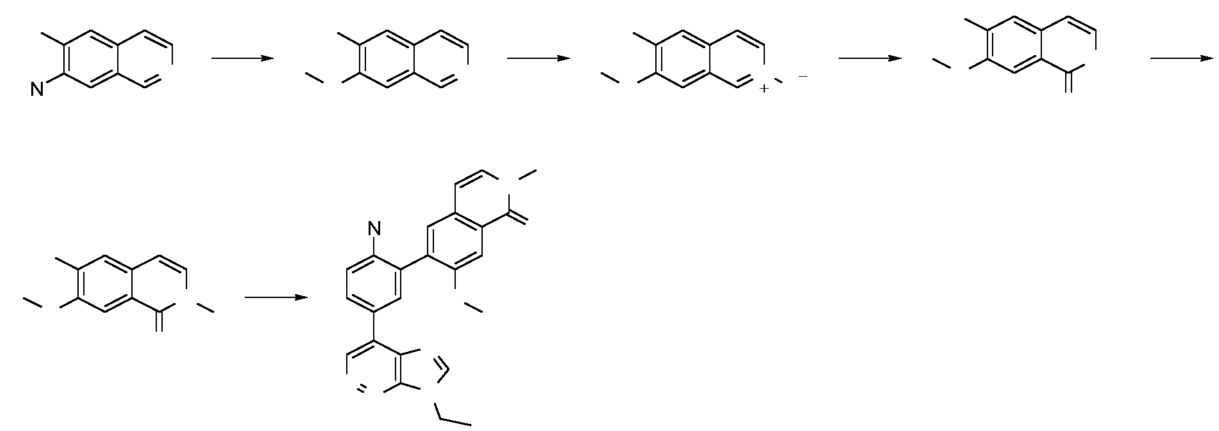

**121-1: 6-Bromo-7-methoxyisoquinoline**

6-Bromo-7-fluoroisoquinoline (CAS: 1258833-80-5, 130 mg, 0.5 mmol) and a 4 M solution of sodium methoxide in methanol (2 mL) were sequentially dissolved in 10 mL of methanol, and the mixture was heated to reflux for 2 hours. The reaction mixture was added with water, neutralized with 1 M hydrochloric acid, extracted with dichloromethane, dried, and concentrated to obtain the title compound (800 mg, 76%) as a white solid. LC-MS: m/z $[M+H]^+$ = 238.

**121-2: 6-Bromo-7-methoxyisoquinoline 2-oxide**

6-Bromo-7-methoxyisoquinoline (700 mg, 3.4 mmol) was dissolved in 10 mL of dichloromethane, then *m*-chloroperoxybenzoic acid (1.1 g, 6.8 mmol) was added thereto, and the mixture was stirred at room temperature overnight. The reaction mixture was concentrated and purified by preparative thin-layer chromatography (dichloromethane: methanol = 20:1) to obtain the title compound (600 mg, 80%) as a white solid. LC-MS: m/z $[M+H]^+$ = 254.

**121-3: 6-Bromo-7-methoxyisoquinolin-1(2*H*)-one**

6-Bromo-7-methoxyisoquinoline 2-oxide (600 mg, 2.3 mmol) was dissolved in acetic anhydride (50 mL), and the mixture was heated to reflux for 2 hours. After cooling to 100°C, 50 mL of water was added thereto, and the mixture was heated to reflux overnight. The reaction mixture was extracted with ethyl acetate, concentrated, and purified by preparative thin-layer chromatography (dichloromethane: methanol = 20:1) to obtain the title compound (200 mg, 33%) as a yellow solid. LC-MS: m/z $[M+H]^+$ = 254.

**121-4: 6-Bromo-7-methoxy-2-methylisoquinolin-1(2H)-one**

6-Bromo-7-methoxyisoquinolin-1(2*H*)-one (200 mg, 0.78 mmol), potassium carbonate (500 mg, 1.6 mmol), and iodomethane (0.5 mL) were sequentially dissolved in 10 mL of methanol, and the mixture was stirred at room temperature overnight. The reaction mixture was concentrated and purified by preparative thin-layer chromatography (dichloromethane: methanol = 20:1) to obtain the title compound (110 mg, 52%) as a white solid. LC-MS: m/z $[M+H]^+$ = 268.

**121: 6-(5-(7-Ethyl-7H-imidazo[4,5-c]pyridazin-4-yl)-2-fluorophenyl)-7-methoxy-2-methylisoquinolin-1(2H)-one**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (70 mg, 0.2 mmol) and 6-bromo-7-methoxy-2-methylisoquinolin-1(2*H*)-one (60 mg, 0.2 mmol) were used as starting materials to obtain the title compound (48 mg, 58%) as a white solid.

$^1$H NMR (400 MHz, CHLOROFORM-d) δ 9.39 (s, 1 H) 8.22 - 8.37 (m, 3 H) 7.97 (s, 1 H) 7.55 (s, 1 H) 7.37 (t, *J* = 9.05 Hz, 1 H) 7.03 (d, *J* = 6.85 Hz, 1 H) 6.51 (d, *J* = 7.34 Hz, 1 H) 4.59 (q, *J* = 7.34 Hz, 2 H) 3.96 (s, 3 H) 3.66 (s, 3 H) 1.70 (t, *J* = 7.09 Hz, 3 H). LC-MS: m/z [M+H]$^+$ = 430.

Example 122

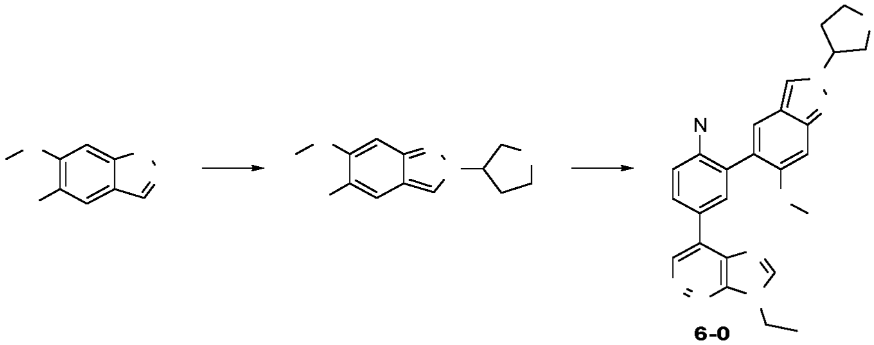

6-0

**122-1: 5-Bromo-6-methoxy-2-(tetrahydrofuran-3-yl)-2*H*-indazole**

5-Bromo-6-methoxy-1*H*-indazole (200 mg, 0.88 mmol) was added to anhydrous *N,N*-dimethylformamide (4 mL), then sodium hydride (35 mg, 0.88 mmol) was added thereto at room temperature, and the mixture was stirred at room temperature for 0.5 hours. 3-Iodo-tetrahydrofuran (523 mg, 2.64 mmol) was then added thereto, and the mixture was stirred at room temperature for another 16 hours. The reaction mixture was added with sodium sulfate decahydrate (100 mg), filtered, concentrated, and separated by preparative thin-layer chromatography (petroleum ether/ethyl acetate = 3/1) to obtain the title compound (40 mg, 15%). LC-MS: m/z [M+H]$^+$ = 297, 299.

**122: 7-Ethyl-4-(4-fluoro-3-(6-methoxy-2-(tetrahydrofuran-3-yl)-2*H*-indazol-5-yl)phenyl)-7*H*-imidazo[4,5-*c*]pyridazine**

Following the same experimental procedure as in Example 1, 5-bromo-6-methoxy-2-(tetrahydrofuran-3-yl)-2*H*-indazole (40 mg, 0.14 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (52 mg, 0.14 mmol) were used as starting materials to obtain the title compound (27 mg, 42%) as a yellow solid.

$^1$H NMR (400 MHz, CHLOROFORM-*d*) 9.37 (s, 1 H), 8.31 - 8.24 (m, 2 H), 8.22 - 8.17 (m, 1 H), 7.98 (s, 1 H), 7.60 (s, 1 H), 7.32 (t, *J* = 9.0 Hz, 1 H), 7.10 (s, 1 H), 5.23 (br. s., 1 H), 4.56 (q, *J* = 7.3 Hz, 2 H), 4.26 - 4.13 (m, 3 H), 4.03 - 3.96 (m, 1 H), 3.85 (s, 3 H), 2.64 - 2.53 (m, 1 H), 2.51 - 2.42 (m, 1 H), 1.67 (t, *J* = 7.3 Hz, 3 H). LC-MS: m/z [M+H]$^+$ = 459.

Example 123

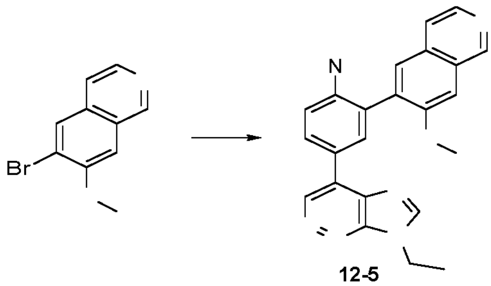

12-5

**123: 6-(5-(7-Ethyl-7*H*-imidazo[4,5-*c*]pyridazin-4-yl)-2-fluorophenyl)-7-methoxyisoquinoline**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (50 mg, 0.14 mmol) and 6-bromo-7-methoxyisoquinoline (30 mg, 0.16 mmol) were used as starting materials to obtain the title compound (19 mg, 35%) as a white solid.

$^1$H NMR (400 MHz, CHLOROFORM-d) δ 9.40 (s, 1 H) 9.25 (s, 1 H) 8.48 (d, *J* = 5.38 Hz, 1 H) 8.25 - 8.37 (m, 3 H) 7.85 (s, 1 H) 7.65 (d, *J* = 5.38 Hz, 1 H) 7.33 - 7.44 (m, 2 H) 4.59 (q, *J* = 7.01 Hz, 2 H) 3.98 (s, 3 H) 1.70 (t, *J* = 7.09 Hz, 3 H). LC-MS: m/z [M+H]$^+$ = 400.

Example 124

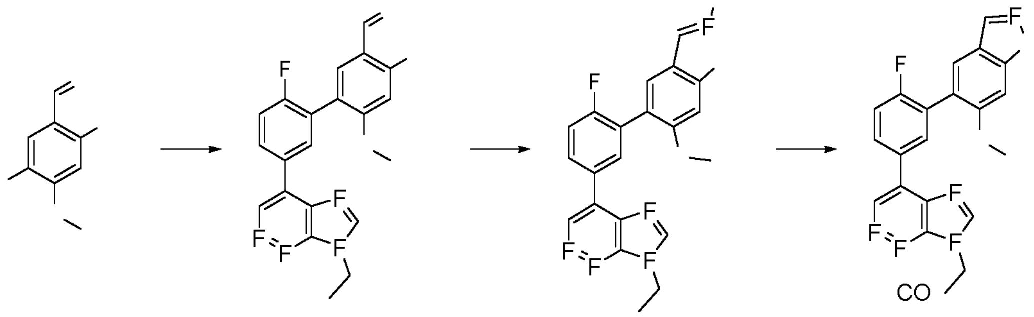

**124-1: 5'-(7-Ethyl-7*H*-imidazo[4,5-*c*]pyridazin-4-yl)-2'-fluoro-4-hydroxy-6-methoxy-[1,1'-biphenyl]-3-carbaldehyde**

Following the same experimental procedure as in Example 1, 5-bromo-2-hydroxy-4-methoxybenzaldehyde (70 mg, 0.299 mmol) and 7-ethyl-4-(4-fluoro-3-(4,4,5,5-tetramethyl-1,3,2-dioxaborolan-2-yl)phenyl)-7*H*-imidazo[4,5-c]pyridazine (100 mg, 0.272 mmol) were used as starting materials to obtain the title compound (66 mg, 62%). LC-MS: m/z [M+H]$^+$ = 393.

**124-2: 5'-(7-Ethyl-7*H*-imidazo[4,5-*c*]pyridazin-4-yl)-2'-fluoro-4-hydroxy-6-methoxy-[1,1'-biphenyl]-3-carbaldehyde carbaldehyde oxime**

5'-(7-Ethyl-7*H*-imidazo[4,5-*c*]pyridazin-4-yl)-2'-fluoro-4-hydroxy-6-methoxy-[1,1'-biphenyl]-3-carbaldehyde (66 mg, 0.168 mmol), hydroxylamine hydrochloride (35 mg, 0.505 mmol), and sodium carbonate (53 mg, 0.505 mmol) were added to ethanol (2 mL) and water (0.2 mL), and the mixture was stirred at room temperature overnight. The reaction mixture was subjected to suction filtration and concentrated to obtain a crude product (48 mg), which was directly used in the next step.

**124: 5-(5-(7-Ethyl-7*H*-imidazo[4,5-*c*]pyridazin-4-yl)-2-fluorophenyl)-6-methoxybenzo[*d*]isoxazole**

5'-(7-Ethyl-7*H*-imidazo[4,5-c]pyridazin-4-yl)-2'-fluoro-4-hydroxy-6-methoxy-[1,1'-biphenyl]-3-carbaldehyde carbaldehyde oxime (48 mg, 0.118 mmol) and triphenylphosphine (31 mg, 0.120 mmol) were added to acetonitrile (1 mL), and the mixture was stirred at room temperature for 5 minutes. Diisopropyl azodicarboxylate (24 mg, 0.12 mmol) was then dissolved in acetonitrile (0.5 mL) and added dropwise to the reaction mixture under an Ar atmosphere. After the dropwise addition was completed, the mixture was stirred at room temperature overnight. The reaction mixture was subjected to suction filtration, concentrated, and subjected to preparative thin-layer chromatography (petroleum ether/ethyl acetate = 1/3) to obtain the title compound (1 mg, 2%).

$^1$H NMR (400 MHz, CHLOROFORM-d) d = 9.20 (s, 1 H), 8.31 (s, 1 H), 8.12 - 7.99 (m, 2 H), 7.38 (s, 1 H), 7.26 - 7.23 (m, 2 H), 6.50 (s, 1 H), 4.48 (d, *J* = 7.8 Hz, 2 H), 3.73 (s, 3 H), 1.59 (t, *J* = 7.3 Hz, 3 H). LC-MS: m/z [M+H]$^+$ = 390.

Example 125

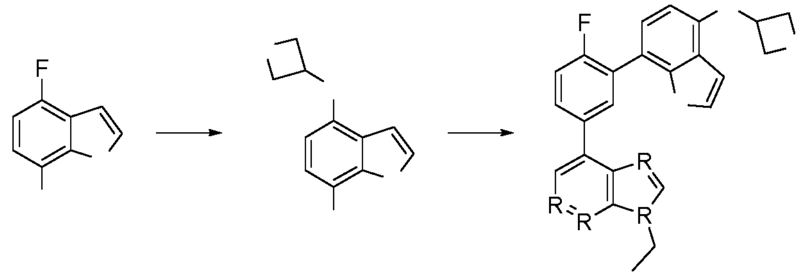

**125-1: 7-Bromo-4-(oxetan-3-yloxy)benzofuran**

7-Bromo-4-fluorobenzofuran (200 mg, 0.9 mmol), oxetan-3-ol (133 mg, 1.8 mmol), and cesium carbonate (472 mg, 1.45 mmol) were added to *N,N*-dimethylformamide (1 mL), and the mixture was reacted at 80°C for 6 hours. The reaction mixture was subjected to suction filtration, concentrated, and subjected to preparative thin-layer chromatography (petroleum ether/ethyl acetate = 3/1) to obtain the title compound (188 mg, 77%). LC-MS: m/z $[M+H]^+$ = 269, 271.

**125: 7-Ethyl-4-(4-fluoro-3-(4-(oxetan-3-yloxy)benzofuran-7-yl)phenyl)-7*H*-imidazo[4,5-*c*]pyridazine**

Following the same experimental procedure as in Example 1, 7-bromo-4-(oxetan-3-yloxy)benzofuran (46 mg, 0.163 mmol) and 7-ethyl-4-(4-fluoro-3-(4,4,5,5-tetramethyl-1,3,2-dioxaborolan-2-yl)phenyl)-7*H*-imidazo[4,5-c]pyridazine (60 mg, 0.163 mmol) were used as starting materials to obtain the title compound (33 mg, 47%).

$^1$H NMR (400 MHz, CHLOROFORM-d) 9.40 (s, 1 H), 8.45 (d, *J* = 6.8 Hz, 1 H), 8.32 - 8.25 (m, 2 H), 7.62 (s, 1 H), 7.44 - 7.35 (m, 2 H), 6.98 (s, 1 H), 6.35 (d, *J* = 7.8 Hz, 1 H), 5.41 (t, *J* = 5.4 Hz, 1 H), 5.07 (t, *J* = 6.6 Hz, 2 H), 4.89 (t, *J* = 5.9 Hz, 2 H), 4.58 (q, *J* = 7.3 Hz, 2 H), 1.71 - 1.67 (m, 3 H). LC-MS: m/z $[M+H]^+$ = 431.

Example 126

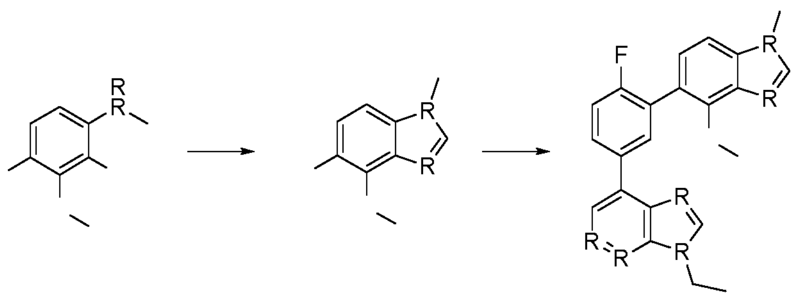

**126-1: 5-Bromo-4-methoxy-1-methyl-1*H*-benzo[*d*]imidazole**

4-Bromo-3-methoxy-$N^1$-methylbenzene-1,2-diamine (200 mg, 0.87 mmol) was added to triethyl orthoformate (5 mL), and the mixture was stirred at 80°C for 1 hour. The reaction mixture was concentrated and separated by preparative thin-layer chromatography (petroleum ether/ethyl acetate = 1/1) to obtain the title compound (100 mg, 47%) as a light yellow oily liquid. LC-MS: m/z $[M+H]^+$ = 241, 243.

**126: 7-Ethyl-4-(4-fluoro-3-(4-methoxy-1-methyl-1*H*-benzo[*d*]imidazol-5-yl)phenyl)-7*H*-imidazo[4,5-*c*]pyridazine**

Following the same experimental procedure as in Example 106, 5-bromo-4-methoxy-1-methyl-1*H*-benzo[*d*]imidazole (50 mg, 0.21 mmol) and 7-ethyl-4-(4-fluoro-3-(4,4,5,5-tetramethyl-1,3,2-dioxaborolan-2-yl)phenyl)-7*H*-imidazo[4,5-c]pyridazine (77 mg, 0.21 mmol) were used as starting materials to obtain the title compound (26 mg, 30%) as a white solid.

$^1$H NMR (400 MHz, CHLOROFORM-d) 9.38 (s, 1 H), 8.34 - 8.19 (m, 3 H), 7.89 (br. s., 1 H), 7.40 - 7.29 (m, 2 H), 7.14 (d, *J* = 7.8 Hz, 1 H), 4.57 (q, *J* = 6.8 Hz, 2 H), 4.39 (s, 3 H), 3.87 (s, 3 H), 1.68 (t, *J* = 7.1 Hz, 3 H). LC-MS: m/z $[M+H]^+$ = 403.

Example 127

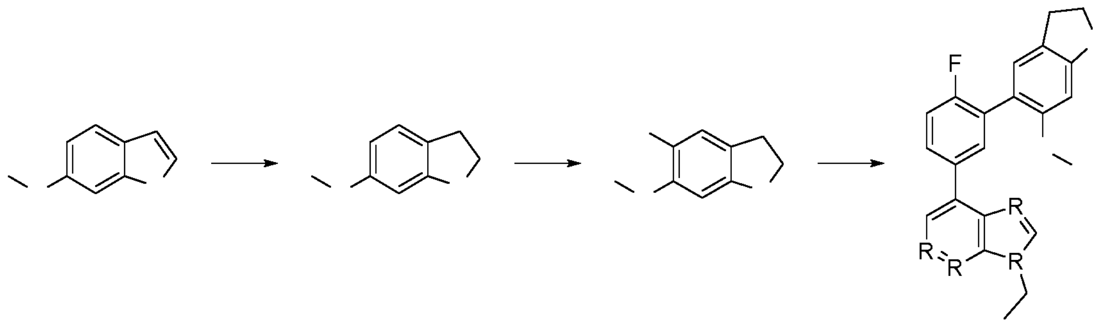

**127-1: 6-Methoxy-2,3-dihydrobenzofuran**

6-Methoxybenzofuran (500 mg, 3.38 mmol) and Pd/C (20 mg) were added to ethanol (5 mL), and the mixture was reacted at room temperature for 4 hours under a $H_2$ atmosphere. The reaction mixture was subjected to suction filtration and concentrated to obtain a crude product (321 mg), which was directly used in the next step. LC-MS: m/z $[M+H]^+$ = 151.

**127-2: 5-Bromo-6-methoxy-2,3-dihydrobenzofuran**

6-Methoxy-2,3-dihydrobenzofuran (200 mg, 1.333 mmol) was added to acetic acid (1 mL). Liquid bromine (229 mg, 1.466 mmol) was dissolved in acetic acid (1 mL) and slowly added dropwise to the reaction mixture. After the dropwise addition was completed, the mixture was reacted at room temperature for 30 minutes. The reaction mixture was poured into saturated sodium carbonate solution (20 mL), extracted with dichloromethane, dried over anhydrous sodium sulfate, concentrated, and subjected to preparative thin-layer chromatography (petroleum ether/ethyl acetate = 10/1) to obtain the title compound (126 mg, 41%). LC-MS: m/z $[M+H]^+$ = 228, 230.

**127: 7-Ethyl-4-(4-fluoro-3-(6-methoxy-2,3-dihydrobenzofuran-5-yl)phenyl)-7*H*-imidazo[4,5-*c*]pyridazine**

Following the same experimental procedure as in Example 1, 5-bromo-6-methoxy-2,3-dihydrobenzofuran (20 mg, 0.088 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (32 mg, 0.088 mmol) were used as starting materials to obtain the title compound (6 mg, 18%).

$^1$H NMR (400 MHz, CHLOROFORM-*d*) 9.39 - 9.33 (m, 1 H), 8.28 - 8.20 (m, 2 H), 8.14 (dd, *J* = 2.2, 7.1 Hz, 1 H), 7.33 - 7.28 (m, 1 H), 7.16 (s, 1 H), 6.53 (s, 1 H), 4.64 (t, *J* = 8.6 Hz, 2 H), 4.59 - 4.55 (m, 2 H), 3.78 (s, 3 H), 3.21 (t, *J* = 8.6 Hz, 2 H), 1.70 - 1.67 (m, 3 H). LC-MS: m/z $[M+H]^+$ = 391.

Example 128

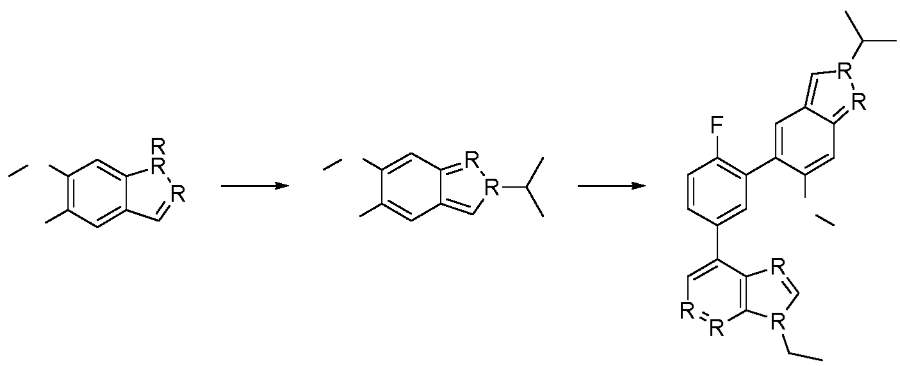

**128-1: 5-Bromo-2-isopropyl-6-methoxy-2*H*-indazole**

5-Bromo-6-methoxy-1*H*-indazole (200 mg, 0.88 mmol) was added to anhydrous *N,N*-dimethylformamide (5 mL), then sodium hydride (35 mg, 0.88 mmol) was added thereto at room temperature, and the mixture was stirred at room temperature for 0.5 hours. 2-Iodopropane (300 mg, 1.76 mmol) was then added thereto, and the mixture was stirred at room temperature for another 1 hour. The reaction mixture was added with sodium sulfate decahydrate (100 mg), filtered, concentrated, and separated by preparative thin-layer chromatography (petroleum ether/ethyl acetate = 3/1) to obtain the title compound (50 mg, 19%). LC-MS: m/z $[M+H]^+$ = 269, 271.

**128: 7-Ethyl-4-(4-fluoro-3-(2-isopropyl-6-methoxy-2*H*-indazol-5-yl)phenyl)-7*H*-imidazo[4,5-*c*]pyridazine**

Following the same experimental procedure as in Example 1, 5-bromo-2-isopropyl-6-methoxy-2H-indazole (50 mg, 0.19 mmol) and 7-ethyl-4-(4-fluoro-3-(4,4,5,5-tetramethyl-1,3,2-dioxaborolan-2-yl)phenyl)-7*H*-imidazo[4,5-c]pyridazine (68 mg, 0.19 mmol) were used as starting materials to obtain the title compound (13 mg, 15%) as a brown solid.

$^1$H NMR (400 MHz, CHLOROFORM-*d*) 9.37 (s, 1 H), 8.30 - 8.24 (m, 2 H), 8.19 (dd, *J* = 2.0, 6.8 Hz, 1 H), 7.91 (s, 1 H), 7.60 (s, 1 H), 7.31 (t, *J* = 8.8 Hz, 1 H), 7.13 (s, 1 H), 4.75 (td, *J* = 6.5, 13.5 Hz, 1 H), 4.55 (q, *J* = 7.3 Hz, 2 H), 3.85 (s, 3 H), 1.71 - 1.62 (m, 9 H). LC-MS: m/z $[M+H]^+$ = 431.

Example 129

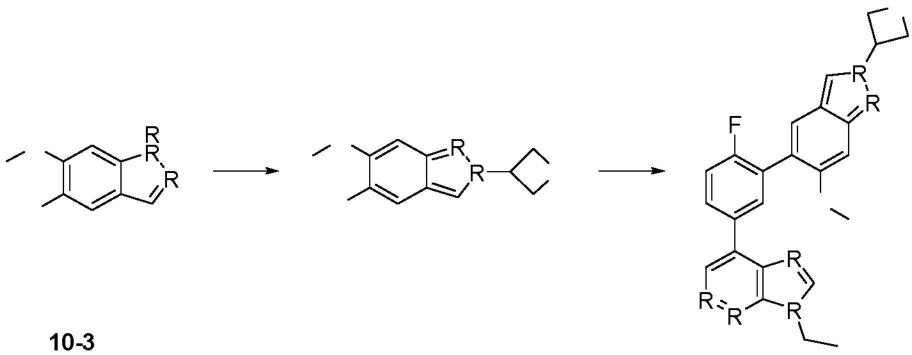

**10-3**

**129-1: 5-Bromo-6-methoxy-2-(oxetan-3-yl)-2*H*-indazole**

5-Bromo-6-methoxy-1*H*-indazole (100 mg, 0.44 mmol) was added to anhydrous *N,N*-dimethylformamide (4 mL), then sodium hydride (18 mg, 0.44 mmol) was added thereto at room temperature, and the mixture was stirred at room temperature for 0.5 hours. 3-Iodooxetane (162 mg, 0.88 mmol) was then added thereto, and the mixture was stirred at room temperature for another 1 hour. The reaction mixture was added with sodium sulfate decahydrate (100 mg), filtered, concentrated, and separated by preparative thin-layer chromatography (dichloromethane/methanol = 20/1) to obtain the crude title compound (100 mg, crude product) as a light yellow oily liquid. LC-MS: m/z $[M+H]^+$ = 283, 285.

**129: 7-Ethyl-4-(4-fluoro-3-(6-methoxy-2-(oxetan-3-yl)-2*H*-indazol-5-yl)phenyl)-7*H*-imidazo[4,5-*c*]pyridazine**

Following the same experimental procedure as in Example 1, 5-bromo-6-methoxy-2-(oxetan-3-yl)-2*H*-indazole (100 mg, crude product) and 7-ethyl-4-(4-fluoro-3-(4,4,5,5-tetramethyl-1,3,2-dioxaborolan-2-yl)phenyl)-7*H*-imidazo[4,5-c]pyridazine (129 mg, 0.35 mmol) were used as starting materials to obtain the title compound (6 mg, two-step yield: 3%) as a solid.

$^1$H NMR (400 MHz, CHLOROFORM-d) 9.38 (s, 1 H), 8.31 - 8.26 (m, 2 H), 8.20 (d, *J* = 6.8 Hz, 1 H), 8.05 (s, 1 H), 7.62 (s, 1 H), 7.33 (t, *J* = 8.6 Hz, 1 H), 7.14 (s, 1 H), 5.73 - 5.65 (m, 1 H), 5.25 - 5.20 (m, 2 H), 5.18 - 5.14 (m, 2 H), 4.57 (q, *J* = 7.3 Hz, 2 H), 3.87 (s, 3 H), 1.68 (s, 3 H). LC-MS: m/z $[M+H]^+$ = 445.

Example 130

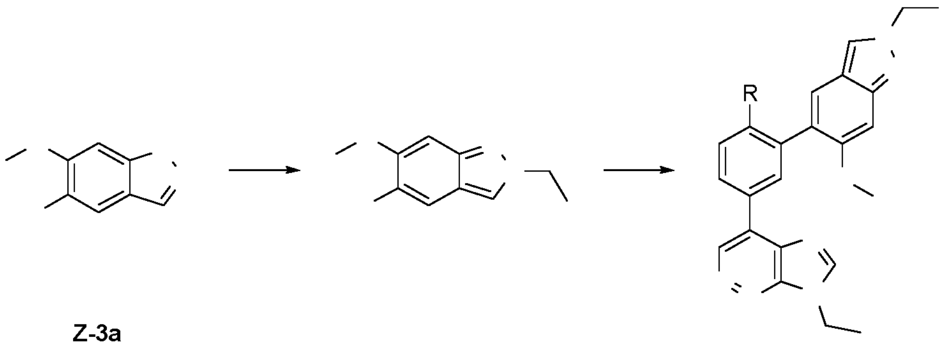

Z-3a

**130-1: 5-Bromo-2-ethyl-6-methoxy-2*H*-indazole**

5-Bromo-6-methoxy-1*H*-indazole (100 mg, 0.44 mmol) was added to anhydrous *N,N*-dimethylformamide (4 mL), then sodium hydride (18 mg, 0.44 mmol) was added thereto at room temperature, and the mixture was stirred at room temperature for 0.5 hours. Iodoethane (137 mg, 0.88 mmol) was then added thereto, and the mixture was stirred at room temperature for another 1 hour. The reaction mixture was added with sodium sulfate decahydrate (100 mg), filtered, concentrated, and separated by preparative thin-layer chromatography (petroleum ether/ethyl acetate = 3/1) to obtain the title compound (50 mg, 45%). LC-MS: m/z $[M+H]^+$ = 255, 257.

**130: 7-Ethyl-4-(3-(2-ethyl-6-methoxy-2*H*-indazol-5-yl)-4-fluorophenyl)-7*H*-imidazo[4,5-*c*]pyridazine**

Following the same experimental procedure as in Example 1, 5-bromo-2-ethyl-6-methoxy-2*H*-indazole (50 mg, 0.20 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (74 mg, 0.20 mmol) were used as starting materials to obtain the title compound (2 mg, 2%) as a brown solid.

$^1$H NMR (400 MHz, CHLOROFORM-d) 9.34 (s, 1 H), 8.28 - 8.21 (m, 1 H), 8.18 (dd, *J* = 2.0, 6.8 Hz, 1 H), 7.86 (s, 1 H), 7.58 (s, 1 H), 7.29 (t, *J* = 9.0 Hz, 1 H), 7.08 (s, 1 H), 4.52 (q, *J* = 7.3 Hz, 2 H), 4.41 (q, *J* = 7.3 Hz, 2 H), 3.83 (s, 3 H), 1.69 - 1.55 (m, 6 H). LC-MS: m/z $[M+H]^+$ = 417.

Example 131

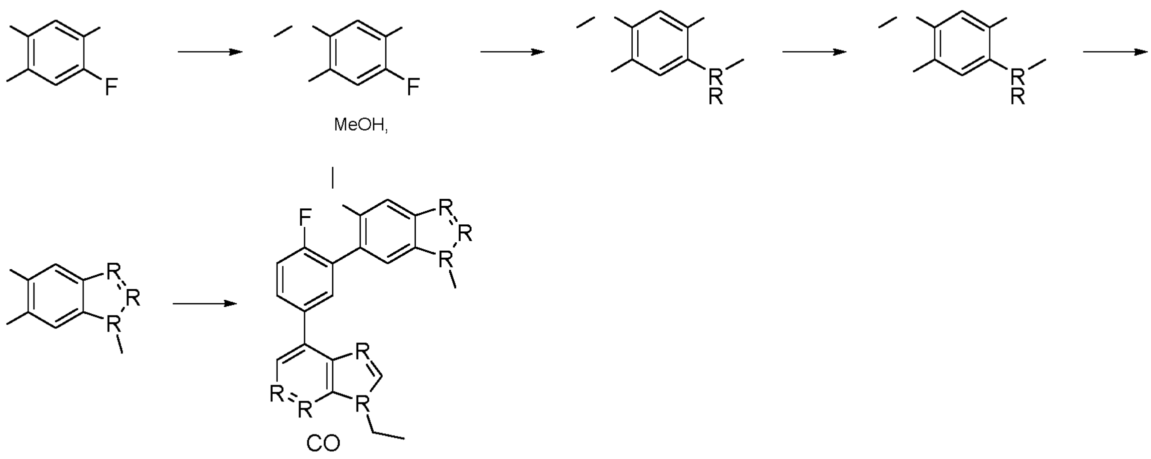


**131-1: 1-Bromo-5-fluoro-2-methoxy-4-nitrobenzene**

2-Bromo-4-fluoro-5-nitrophenol (1 g, 4.2 mmol), iodomethane (5.9 g, 42 mmol), and cesium carbonate (6.8 g, 21 mmol) were added to acetonitrile (10 mL), and the mixture was stirred at 100°C for 2 hours. The reaction mixture was concentrated and extracted with ethyl acetate and water. The organic phase was concentrated and purified by preparative thin-layer chromatography (petroleum ether/ethyl acetate = 5/1) to obtain the title compound (1 g, 95%). LC-MS: m/z $[M+H]^+$ = 250, 252.

**131-2: 5-Bromo-4-methoxy-*N*-methyl-2-nitroaniline**

1-Bromo-5-fluoro-2-methoxy-4-nitrobenzene (1 g, 4 mmol) and methylamine hydrochloride (1.35 g, 20 mmol) were added to acetonitrile (10 mL), and the mixture was stirred at 90°C for 2 hours. The reaction mixture was concentrated and extracted with ethyl acetate and water. The organic phase was concentrated and purified by preparative thin-layer chromatography (petroleum ether/ethyl acetate = 5/1) to obtain the title compound (1 g, 96%). LC-MS: m/z $[M+H]^+$ = 261, 263.

**131-3: 5-Bromo-4-methoxy-*N*$^1$-methylbenzene-1,2-diamine**

5-Bromo-4-methoxy-*N*-methyl-2-nitroaniline (1 g, 3.83 mmol) and Raney nickel (250 mg) were added to methanol (20 mL), and the mixture was stirred at room temperature for 0.5 hours under a hydrogen atmosphere. The reaction mixture was filtered and concentrated to obtain the crude title compound (850 mg, 96%). LC-MS: m/z $[M+H]^+$ = 231, 233.

**131-4: 6-Bromo-5-methoxy-1-methyl-1*H*-benzo[*d*][1,2,3]triazole**

5-Bromo-4-methoxy-*N*$^1$-methylbenzene-1,2-diamine (900 mg, 3.9 mmol), hydrogen bromide (3 mL), and sodium nitrite (538 mg, 7.8 mmol) were sequentially added to water (10 mL), and the mixture was stirred at room temperature for 5 hours. The reaction mixture was extracted with ethyl acetate and water. The organic phase was concentrated and purified by preparative thin-layer chromatography (dichloromethane/methanol = 20/1) to obtain the title compound (500 mg, 53%). LC-MS: m/z $[M+H]^+$ = 242, 244.

**131: 7-Ethyl-4-(4-fluoro-3-(5-methoxy-1-methyl-1*H*-benzo[*d*][1,2,3]triazol-6-yl)phenyl)-7*H*-imidazo[4,5-*c*]pyridazine**

Following the same experimental procedure as in Example 1, 6-bromo-5-methoxy-1-methyl-1*H*-benzo[*d*][1,2,3]triazole (22.4 mg, 0.09 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (30 mg, 0.01 mmol) were used as starting materials to obtain the title compound (18 mg, 50%).

$^1$H NMR (400 MHz, CHLOROFORM-d) 9.39 (s, 1 H), 8.35 - 8.23 (m, 3 H), 7.52 (d, *J* = 4.4 Hz, 2 H), 7.38 (t, *J* = 9.0 Hz, 1 H), 4.59 (q, *J* = 7.0 Hz, 2 H), 4.31 (s, 3 H), 3.92 (s, 3 H), 1.70 (t, *J* = 7.3 Hz, 3 H). LC-MS: m/z $[M+H]^+$ = 404.

Example 132

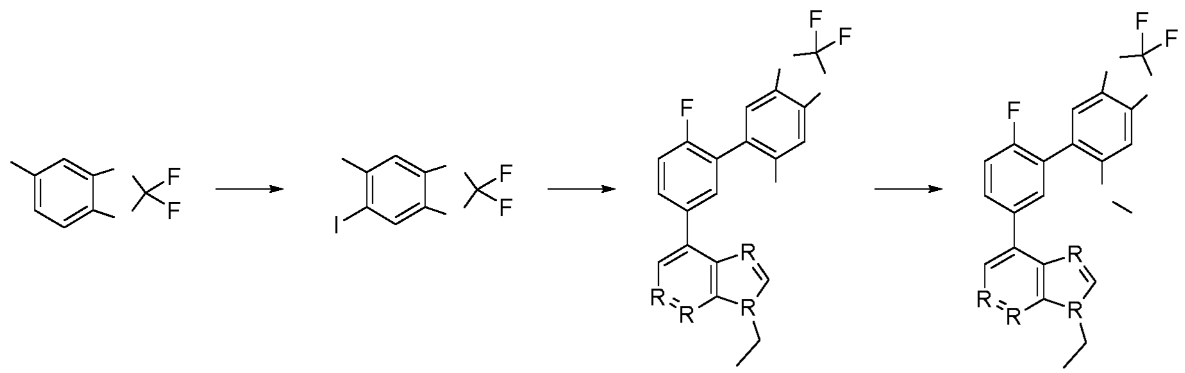

**132-1: 5-Bromo-2,2-difluoro-6-iodobenzo[*d*][1,3]dioxole**

5-Bromo-2,2-difluorobenzo[*d*][1,3]dioxole (1 g, 4.22 mmol), iodine (2.14 g, 8.44 mmol), and silver trifluoroacetate (560 mg, 2.53 mmol) were added to dichloromethane (20 mL), and the mixture was reacted at 40°C for 48 hours. The reaction mixture was poured into a solution of saturated sodium thiosulfate (30 mL), extracted with dichloromethane, dried over anhydrous sodium sulfate, concentrated, and subjected to column chromatography (petroleum ether) to obtain a crude product (purity: 80%).

**132-2: 4-(3-(6-Bromo-2,2-difluorobenzo[*d*][1,3]dioxol-5-yl)-4-fluorophenyl)-7-ethyl-7*H*-imidazo[4,5-*c*]pyridazine**

Following the same experimental procedure as in Example 1, 5-bromo-2,2-difluoro-6-iodobenzo[*d*][1,3]dioxole crude product (50 mg, 0.168 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (50 mg, 0.168 mmol) were used as starting materials to obtain the title compound (27 mg, 34%). LC-MS: m/z $[M+H]^+$ = 477, 479.

**132: 4-(3-(2,2-Difluoro-6-methoxybenzo[*d*][1,3]dioxol-5-yl)-4-fluorophenyl)-7-ethyl-7*H*-imidazo[4,5-*c*]pyridazine**

4-(3-(6-Bromo-2,2-difluorobenzo[*d*][1,3]dioxol-5-yl)-4-fluorophenyl)-7-ethyl-7*H*-imidazo[4,5-c]pyridazine (20 mg, 0.042 mmol), palladium(1-phenylallyl)chloride dimer (2 mg, 0.002 mmol), di-*tert*-butyl(2,4,6-triisopropyl-3,6-dimethoxy-[1,1-biphenyl]-2-yl)phosphine (3 mg, 0.004 mmol), cesium carbonate (28 mg, 0.084 mmol), and methanol (4 mg, 0.126 mmol) were added to toluene (1 mL), and the mixture was stirred at room temperature overnight under an argon atmosphere. The reaction mixture was subjected to suction filtration, concentrated, and subjected to preparative thin-layer chromatography (dichloromethane/methanol = 10/1) to obtain the title compound (12 mg, 67%).

$^1H$ NMR (400 MHz, CHLOROFORM-d) 9.37 (s, 1 H), 8.29 - 8.22 (m, 2 H), 8.19 (d, *J* = 6.8 Hz, 1 H), 7.33 (t, *J* = 9.0 Hz, 1 H), 7.09 (s, 1 H), 6.83 (s, 1 H), 4.65 - 4.48 (m, 2 H), 3.80 (s, 3 H), 1.69 (t, *J* = 7.3 Hz, 3 H). LC-MS: m/z $[M+H]^+$ = 429.

Example 133

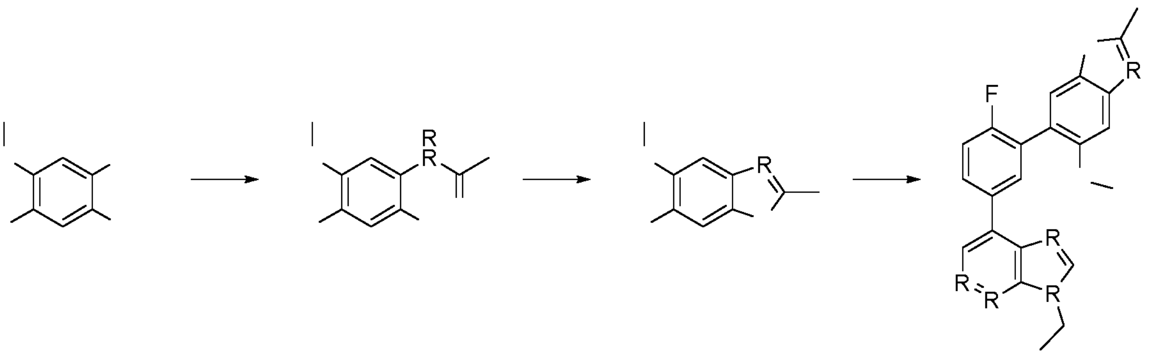

**133-1: N-(2,4-Dibromo-5-methoxyphenyl)acetamide**

2,4-Dibromo-5-methoxyaniline (300 mg, 1.075 mmol), acetic anhydride (132 mg, 1.290 mmol), and triethylamine (132 mg, 1.290 mmol) were added to dichloromethane (2 mL), and the mixture was stirred at room temperature overnight. The reaction mixture was poured into saturated ammonium chloride solution, extracted with dichloromethane, dried over anhydrous sodium sulfate, and concentrated to obtain a crude product (317 mg), which was directly used in the next step. LC-MS: m/z $[M+H]^+$ = 322, 324, 326.

**133-2: 6-Bromo-5-methoxy-2-methylbenzo[d]oxazole**

*N*-(2,4-Dibromo-5-methoxyphenyl)acetamide (300 mg, 0.934 mmol), cuprous iodide (18 mg, 0.094 mmol), *N,N*-dimethylethylenediamine (16 mg, 0.187 mmol), and potassium carbonate (258 mg, 1.872 mmol) were added to toluene (2 mL), and the mixture was reacted at 110°C overnight under an argon atmosphere. The reaction mixture was subjected to suction filtration, concentrated, and subjected to preparative thin-layer chromatography (petroleum ether/ethyl acetate = 1/1) to obtain the title compound (70 mg, 29%). LC-MS: m/z $[M+H]^+$ = 242, 244.

**133: 6-(5-(7-Ethyl-7H-imidazo[4,5-c]pyridazin-4-yl)-2-fluorophenyl)-5-methoxy-2-methylbenzo[d]oxazole**

6-Bromo-5-methoxy-2-methylbenzo[*d*]oxazole (62 mg, 0.257 mmol), 7-ethyl-4-(4-fluoro-3-(4,4,5,5-tetramethyl-1,3,2-dioxaborolan-2-yl)phenyl)-7*H*-imidazo[4,5-*c*]pyridazine (95 mg, 0.257 mmol), tetrakis(triphenylphosphine)palladium (30 mg, 0.026), and cesium carbonate (166 mg, 0.514 mmol) were added to 1,4-dioxane (2 mL), and the mixture was reacted at 100°C for 1 hour under an argon atmosphere. The reaction mixture was subjected to suction filtration, concentrated, and subjected to preparative thin-layer chromatography (dichloromethane/methanol =

30/1) to obtain the title compound (50 mg, 48%).

$^1H$ NMR (400 MHz, CHLOROFORM-d) 9.37 (s, 1 H), 8.27 (s, 2 H), 8.23 (d, *J* = 6.8 Hz, 1 H), 7.47 (s, 1 H), 7.35 (t, *J* = 9.0 Hz, 1 H), 7.26 (s, 1 H), 4.58 (q, *J* = 7.3 Hz, 2 H), 3.86 (s, 3 H), 2.66 (s, 3 H), 1.69 (t, *J* = 7.3 Hz, 3 H). LC-MS: m/z $[M+H]^+$ = 404.

Example 134 and Example 135

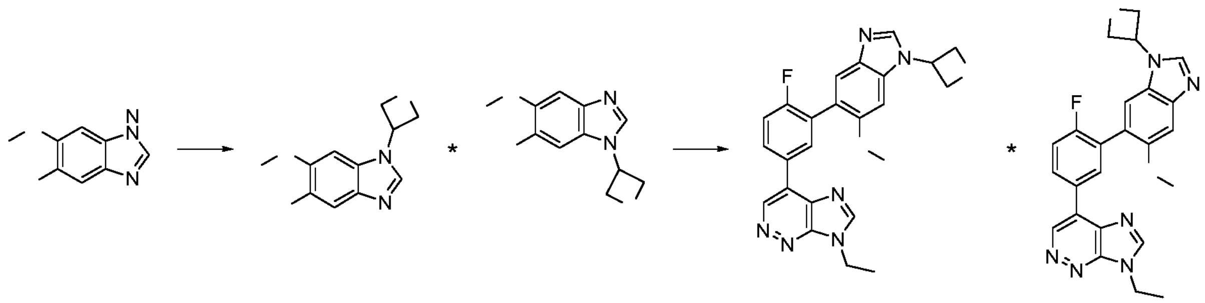

**134-1: A mixture of 5-bromo-6-methoxy-1-(oxetan-3-yl)-1*H*-benzo[*d*]imidazole and 6-bromo-5-methoxy-1-(oxetan-3-yl)-1*H*-benzo[d]imidazole**

5-Bromo-6-methoxy-1*H*-benzo[d]imidazole (100 mg, 0.44 mmol) was added to anhydrous *N,N*-dimethylformamide (5 mL), then sodium hydride (35 mg, 0.88 mmol) was added thereto at room temperature, and the mixture was stirred at room temperature for 0.5 hours. 3-Iodooxetane (162 mg, 0.88 mmol) was then added thereto, and the mixture was stirred at 80°C for another 16 hours. The reaction mixture was added dropwise to saturated ammonium chloride aqueous solution (20 mL) and extracted with ethyl acetate (20 mL × 3). The organic phases were combined, washed with saturated brine (30 mL), concentrated, and separated by preparative thin-layer chromatography (dichloromethane/methanol = 20/1) to obtain a mixture of 5-bromo-6-methoxy-1-(oxetan-3-yl)-1*H*-benzo[d]imidazole and 6-bromo-5-methoxy-1-(oxetan-3-yl)-1*H*-benzo[d]imidazole (80 mg, 64%). LC-MS: m/z $[M+H]^+$ = 283, 285.

**134: 7-Ethyl-4-(4-fluoro-3-(6-methoxy-1-(oxetan-3-yl)-1*H*-benzo[*d*]imidazol-5-yl)phenyl)-7*H*-imidazo[4,5-*c*]pyridazine**

**135: 7-Ethyl-4-(4-fluoro-3-(5-methoxy-1-(oxetan-3-yl)-1*H*-benzo[*d*]imidazol-6-yl)phenyl)-7*H*-imidazo[4,5-*c*]pyridazine**

Following the same experimental procedure as in Example 106, the mixture of 5-bromo-6-methoxy-1-(oxetan-3-yl)-1*H*-benzo[d]imidazole and 6-bromo-5-methoxy-1-(oxetan-3-yl)-1*H*-benzo[*d*]imidazole (80 mg, 0.28 mmol) and 7-ethyl-4-(4-fluoro-3-(4,4,5,5-tetramethyl-1,3,2-dioxaborolan-2-yl)phenyl)-7*H*-imidazo[4,5-c]pyridazine (100 mg, 0.28 mmol) were used as starting materials to obtain 30 mg of a solid, which was further subjected to SFC resolution to obtain (first peak, Example 134, 12 mg, 9%) and (second peak, Example 135, 6 mg, 4%). Resolution method: instrument: MG II preparative SFC (SFC-1); column: ChiralPak AD, 250 × 30 mm I.D., 10 μm; mobile phase: A for $CO_2$ and B for isopropanol; gradient: B 55%; flow rate: 80 mL/min; back pressure: 100 bar; column temperature: 38°C; wavelength: 220 nm.

Example 134: $^1H$ NMR (400 MHz, CHLOROFORM-d) 9.39 (s, 1 H), 8.33 - 8.21 (m, 3 H), 8.00 (s, 1 H), 7.83 (s, 1 H), 7.38 - 7.33 (m, 2 H), 5.58 - 5.52 (m, 1 H), 5.33 - 5.25 (m, 2 H), 5.23 - 5.18 (m, 2 H), 4.58 (q, *J* = 7.3 Hz, 2 H), 1.69 (t, *J* = 7.3 Hz, 3 H). LC-MS: m/z $[M+H]^+$ = 445.

Example 135: $^1H$ NMR (400 MHz, CHLOROFORM-d) 9.39 (s, 1 H), 8.34 - 8.21 (m, 3 H), 8.08 (s, 1 H), 7.70 (s, 1 H), 7.45 (s, 1 H), 7.36 (t, *J* = 9.0 Hz, 1 H), 5.59 - 5.50 (m, 1 H), 5.25 - 5.13 (m, 4 H), 4.58 (d, *J*=7.3 Hz, 2 H), 3.89 (s, 3 H), 1.69 (t, *J*= 7.3 Hz, 3 H). LC-MS: m/z $[M+H]^+$ = 445.

Example 136 and Example 137

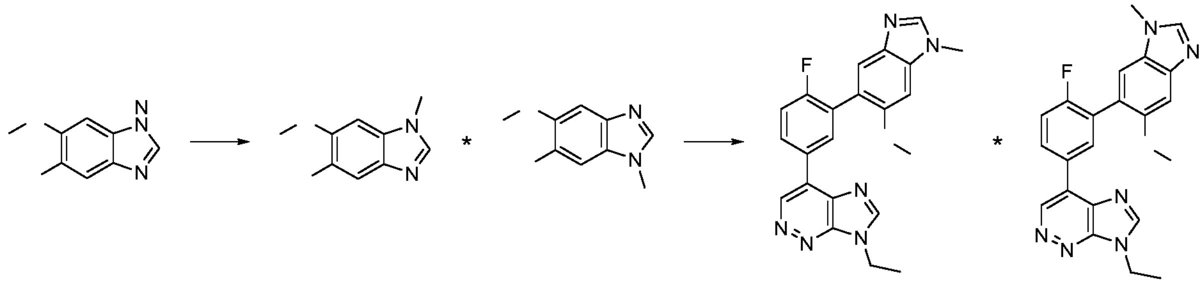

**136-1: A mixture of 5-bromo-6-methoxy-1-methyl-1*H*-benzo[*d*]imidazole and 6-bromo-5-methoxy-1-methyl-1*H*-benzo[*d*]imidazole**

5-Bromo-6-methoxy-1*H*-benzo[*d*]imidazole (250 mg, 1.10 mmol) was added to anhydrous *N,N*-dimethylformamide (5 mL), then sodium hydride (67 mg, 1.65 mmol) was added thereto at room temperature, and the mixture was stirred at room temperature for 0.5 hours. Iodomethane (391 mg, 2.75 mmol) was then added thereto, and the mixture was stirred at room temperature for another 1 hour. The reaction mixture was added dropwise to saturated ammonium chloride aqueous solution (20 mL) and extracted with ethyl acetate (20 mL × 3). The organic phases were combined, washed with saturated brine (30 mL), concentrated, and separated by preparative thin-layer chromatography (dichloromethane/-methanol = 30/1) to obtain a mixture of 5-bromo-6-methoxy-1-methyl-1*H*-benzo[*d*]imidazole and 6-bromo-5-methoxy-1-methyl-1*H*-benzo[*d*]imidazole (130 mg, 49%). LC-MS: m/z $[M+H]^+$ = 241, 243.

**136: 7-Ethyl-4-(4-fluoro-3-(5-methoxy-1-methyl-1*H*-benzo[*d*]imidazol-6-yl)phenyl)-7*H*-imidazo[4,5-c]pyridazine**

**137: 7-Ethyl-4-(4-fluoro-3-(6-methoxy-1-methyl-1*H*-benzo[*d*]imidazol-5-yl)phenyl)-7*H*-imidazo[4,5-*c*]pyridazine**

Following the same experimental procedure as in Example 1, the mixture of 5-bromo-6-methoxy-1-methyl-1*H*-benzo[*d*]imidazole and 6-bromo-5-methoxy-1-methyl-1*H*-benzo[d]imidazole (120 mg, 0.50 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (184 mg, 0.50 mmol) were used as starting materials to obtain 80 mg of a solid, which was further subjected to SFC resolution to obtain (first peak, Example 136, yellow solid, 18 mg, 8%) and (second peak, Example 137, white solid, 36 mg, 17%). Resolution method: instrument: MG II preparative SFC (SFC-1); column: ChiralPak AD, 250 × 30 mm I.D., 10 μm; mobile phase: A for $CO_2$ and B for isopropanol; gradient: B 55%; flow rate: 80 mL/min; back pressure: 100 bar; column temperature: 38°C; wavelength: 220 nm.

136: $^1$H NMR (400 MHz, CHLOROFORM-*d*) 9.55 (s, 1 H), 8.84 (s, 1 H), 8.50 - 8.41 (m, 2 H), 8.18 (s, 1 H), 7.59 (s, 1 H), 7.52 - 7.45 (m, 1 H), 7.36 (s, 1 H), 4.50 (q, *J* = 7.3 Hz, 2 H), 3.83 (s, 3 H), 3.79 (s, 3 H), 1.54 (t, *J* = 7.3 Hz, 3 H). LC-MS: m/z $[M+H]^+$ = 403.

137: $^1$H NMR (400 MHz, CHLOROFORM-d) 9.55 (s, 1 H), 8.84 (s, 1 H), 8.51 - 8.46 (m, 1 H), 8.45 - 8.39 (m, 1 H), 8.17 (s, 1 H), 7.60 (s, 1 H), 7.51 - 7.44 (m, 1 H), 7.32 (s, 1 H), 4.50 (d, *J* = 7.3 Hz, 2 H), 3.88 (s, 3 H), 3.84 (s, 3 H), 1.54 (t, *J* = 7.3 Hz, 3 H). LC-MS: m/z $[M+H]^+$ = 403.

Example 138

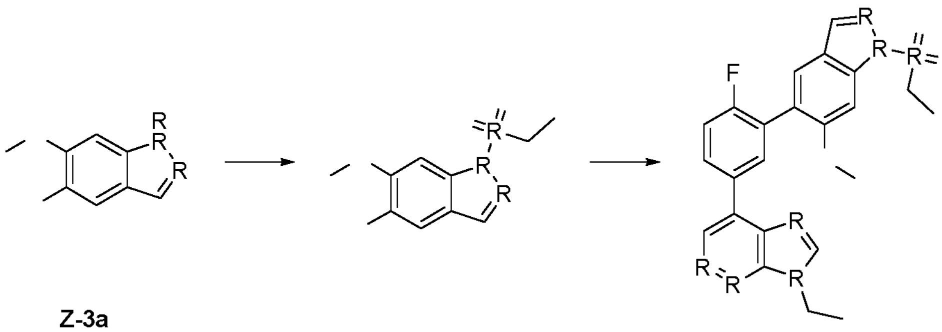

Z-3a

**138-1: 5-Bromo-1-(ethylsulfonyl)-6-methoxy-1*H*-indazole**

5-Bromo-6-methoxy-1*H*-indazole (200 mg, 0.88 mmol) was added to anhydrous *N,N*-dimethylformamide (5 mL), then sodium hydride (53 mg, 1.32 mmol) was added thereto at room temperature, and the mixture was stirred at room temperature for 0.5 hours. Ethylsulfonyl chloride (170 mg, 1.32 mmol) was then added thereto, and the mixture was stirred at room temperature for another 1 hour. The reaction mixture was added dropwise to saturated ammonium chloride aqueous solution (20 mL) and extracted with ethyl acetate (20 mL × 3). The organic phases were combined, washed with saturated brine (30 mL), concentrated, and separated by preparative thin-layer chromatography (petroleum ether/ethyl acetate = 5/1) to obtain the title compound (210 mg, 78%) as a yellow solid. LC-MS: m/z $[M+H]^+$ = 319, 321.

**138: 7-Ethyl-4-(3-(1-(ethylsulfonyl)-6-methoxy-1*H*-indazol-5-yl)-4-fluorophenyl)-7*H*-imidazo[4,5-c]pyridazine**

Following the same experimental procedure as in Example 1, 5-Bromo-1-(ethylsulfonyl)-6-methoxy-1*H*-indazole (35 mg, 0.11 mmol) and 7-ethyl-4-(4-fluoro-3-(4,4,5,5-tetramethyl-1,3,2-dioxaborolan-2-yl)phenyl)-7*H*-imidazo [4,5-*c*]pyridazine (40 mg, 0.11 mmol) were used as starting materials to obtain the title compound (25 mg, 47%) as a yellow solid.

$^1$H NMR (400 MHz, CHLOROFORM-*d*) 9.37 (s, 1 H), 8.28 (s, 2 H), 8.25 - 8.19 (m, 2 H), 7.71 (s, 1 H), 7.63 (s, 1 H), 7.35 (t, *J* = 9.0 Hz, 1 H), 4.58 (q, *J* = 7.2 Hz, 2 H), 3.92 (s, 3 H), 3.52 (q, *J* = 7.3 Hz, 2 H), 1.68 (t, *J* = 7.3 Hz, 3 H), 1.30 (t, *J* = 7.3 Hz, 3 H). LC-MS: m/z $[M+H]^+$ = 481.

Example 139

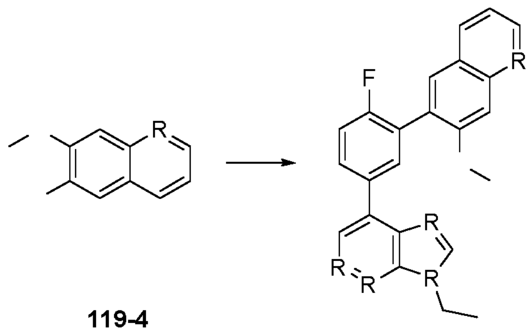

**119-4**

**139: 6-(5-(7-Ethyl-7*H*-imidazo[4,5-*c*]pyridazin-4-yl)-2-fluorophenyl)-7-methoxyquinoline**

Following the same experimental procedure as in Example 1, 6-bromo-7-methoxyquinoline (26 mg, 0.11 mmol) and 7-ethyl-4-(4-fluoro-3-(4,4,5,5-tetramethyl-1,3,2-dioxaborolan-2-yl)phenyl)-7*H*-imidazo[4,5-c]pyridazine (40 mg, 0.11 mmol) were used as starting materials to obtain the title compound (17 mg, 38%) as a yellow solid.

$^1$H NMR (400 MHz, CHLOROFORM-d) 9.37 (s, 1 H), 8.86 (d, *J* = 2.4 Hz, 1 H), 8.33 - 8.23 (m, 3 H), 8.11 (d, *J* = 7.8 Hz, 1 H), 7.79 (s, 1 H), 7.55 (s, 1 H), 7.39 - 7.27 (m, 2 H), 4.55 (q, *J* = 7.0 Hz, 2 H), 3.96 (s, 3 H), 1.66 (t, *J* = 7.1 Hz, 3 H). LC-MS: m/z $[M+H]^+$ = 400.

Example 140

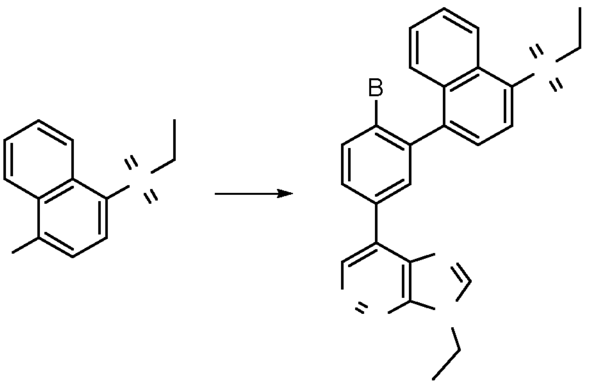

**140: 7-Ethyl-4-(3-(4-(ethylsulfonyl)naphthalen-1-yl)-4-fluorophenyl)-7*H*-imidazo[4,5-*c*]pyridazine**

Following the same experimental procedure as in Example 1, bromo-4-(ethylsulfonyl)naphthalene (25 mg, 0.084 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (31 mg, 0.084 mmol) were used as starting materials to obtain the title compound (12 mg, 29.1%).

$^1H$ NMR (400 MHz, CHLOROFORM-d) 9.39 (s, 1 H), 8.86 (d, *J* = 8.3 Hz, 1 H), 8.41 (d, *J* = 7.8 Hz, 2 H), 8.31 (d, *J* = 4.9 Hz, 1 H), 8.26 (s, 1 H), 7.87 (d, *J* = 8.3 Hz, 1 H), 7.78 - 7.65 (m, 2 H), 7.63 - 7.57 (m, 1 H), 7.47 (t, *J* = 9.0 Hz, 1 H), 4.58 (q, *J* = 7.7 Hz, 2 H), 3.39 (q, *J* = 7.3 Hz, 2 H), 1.68 (t, *J* = 7.3 Hz, 3 H), 1.33 (t, *J* = 7.6 Hz, 3 H). LC-MS: m/z $[M+H]^+$ = 491.

Example 141

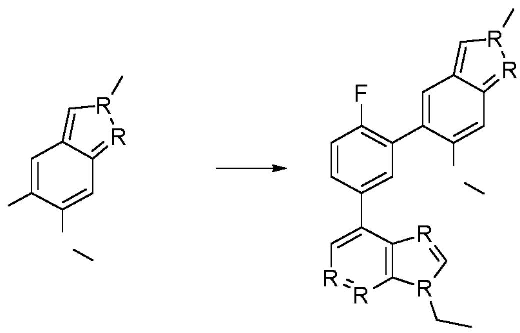

**141: 7-Ethyl-4-(4-fluoro-3-(6-methoxy-2-methyl-2*H*-indazol-5-yl)phenyl)-7*H*-imidazo[4,5-*c*]pyridazine**

Following the same experimental procedure as in Example 1, 5-bromo-6-methoxy-2-methyl-2*H*-indazole (50 mg, 0.21 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (77 mg, 0.21 mmol) were used as starting materials to obtain the title compound (14 mg, 16%) as a yellow solid.

$^1H$ NMR (400 MHz, CHLOROFORM-*d*) δ = 9.37 (br. s., 1 H), 8.26 (br. s., 2 H), 8.21 (d, *J* = 5.9 Hz, 1 H), 7.84 (br. s., 1 H), 7.59 (br. s., 1 H), 7.32 (t, *J* = 8.6 Hz, 1 H), 7.08 (br. s., 1 H), 4.55 (d, *J* = 7.3 Hz, 2 H), 4.17 (br. s., 3 H), 3.86 (br. s., 3 H), 1.67 (t, *J* = 7.1 Hz, 3 H). LC-MS: m/z $[M+H]^+$ = 403.

Example 142

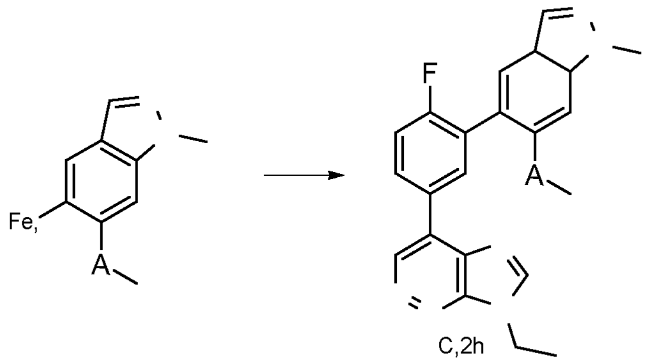

**142: 7-Ethyl-4-(4-fluoro-3-(6-methoxy-1-methyl-1*H*-indazol-5-yl)phenyl)-7*H*-imidazo[4,5-*c*]pyridazine**

Following the same experimental procedure as in Example 1, 5-bromo-6-methoxy-1-methyl-1*H*-indazole (50 mg, 0.21 mmol) and 7-ethyl-4-(4-fluoro-3-(4,4,5,5-tetramethyl-1,3,2-dioxaborolan-2-yl)phenyl)-7*H*-imidazo[4,5-c]pyridazine (77 mg, 0.21 mmol) were used as starting materials to obtain the title compound (35 mg, 41%) as a yellow solid.

$^{1}$H NMR (400 MHz, CHLOROFORM-*d*) 9.38 (s, 1 H), 8.27 (s, 2 H), 8.22 (d, *J* = 5.9 Hz, 1 H), 7.93 (s, 1 H), 7.68 (s, 1 H), 7.34 (t, *J* = 8.8 Hz, 1 H), 6.83 (s, 1 H), 4.57 (q, *J* = 7.3 Hz, 2 H), 4.08 (s, 3 H), 3.92 (s, 3 H), 1.69 (t, *J* = 7.3 Hz, 3 H). LC-MS: m/z $[M+H]^+$ = 403.

Example 143

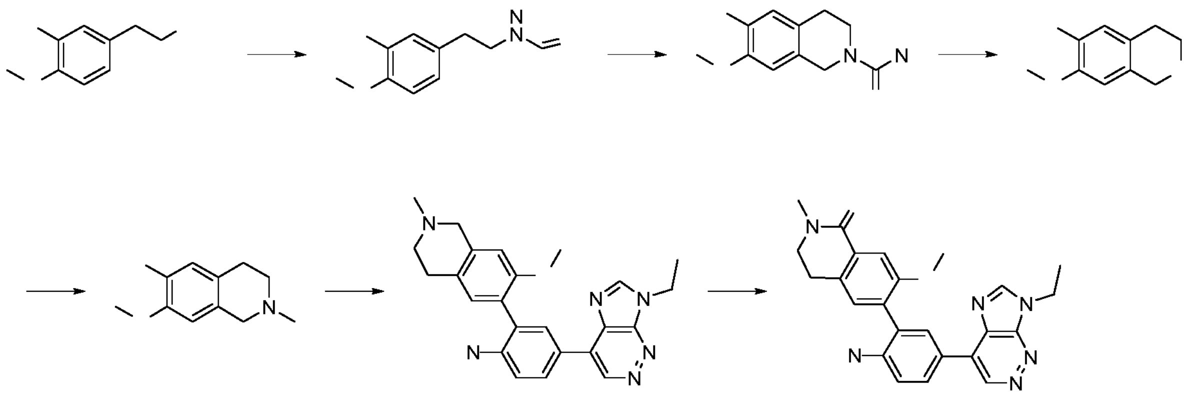

**143-1: 2-(3-Bromo-4-methoxyphenethyl)formamide**

2-(3-Bromo-4-methoxyphenyl)ethan-1-amine (1 g, 4.3 mmol) was added to formamide (10 mL), and the mixture was stirred at 120°C for 16 hours. The reaction mixture was concentrated, extracted with dichloromethane and water, and purified by preparative thin-layer chromatography (petroleum ether/ethyl acetate = 3/1) to obtain the title compound (900 mg, 81.8%). LC-MS: m/z $[M+H]^+$ = 258, 260.

**143-2: 6-Bromo-7-methoxy-3,4-dihydroisoquinoline-2(1H)-carbaldehyde**

2-(3-Bromo-4-methoxyphenethyl)formamide (900 mg, 3.48 mmol), paraformaldehyde (300 mg), and trifluoroacetic acid (1 mL) were added to toluene (6 mL), and the mixture was stirred at 60°C for 16 hours. The reaction mixture was concentrated, extracted with dichloromethane and water, and purified by preparative thin-layer chromatography (dichloromethane/methanol = 12/1) to obtain the title compound (460 mg, 49%). LC-MS: m/z $[M+H]^+$ = 258, 260.

**143-3: 6-Bromo-7-methoxy-1,2,3,4-tetrahydroisoquinoline**

6-Bromo-7-methoxy-3,4-dihydroisoquinoline-2(1*H*)-carbaldehyde (200 mg, 0.74 mmol) and sodium hydroxide (148 mg, 3.7 mmol) were added to ethanol (4 mL), and the mixture was stirred at 80°C for 16 hours. The reaction mixture

was concentrated and purified by preparative thin-layer chromatography (dichloromethane/methanol = 12/1) to obtain the title compound (120 mg, 67%). LC-MS: m/z $[M+H]^+$ = 242, 244.

**143-4: 6-Bromo-7-methoxy-2-methyl-1,2,3,4-tetrahydroisoquinoline**

6-Bromo-7-methoxy-1,2,3,4-tetrahydroisoquinoline (100 mg, 0.4 mmol), sodium cyanoborohydride (75 mg, 1.2 mmol), and paraformaldehyde (125 mg, 4 mmol) were added to methanol (4 mL), and the mixture was stirred at room temperature for 0.5 hours. The reaction mixture was quenched with methanol, concentrated, and purified by preparative thin-layer chromatography (dichloromethane/methanol = 12/1) to obtain the title compound (80 mg, 78.4%). LC-MS: m/z $[M+H]^+$ = 256, 258.

**143-5: 6-(5-(7-Ethyl-7H-imidazo[4,5-c]pyridazin-4-yl)-2-fluorophenyl)-7-methoxy-2-methyl-1,2,3,4-tetrahydroisoquinoline**

Following the same experimental procedure as in Example 1, 6-bromo-7-methoxy-2-methyl-1,2,3,4-tetrahydroisoquinoline (120 mg, 0.47 mmol) and 7-ethyl-4-(4-fluoro-3-(4,4,5,5-tetramethyl-1,3,2-dioxaborolan-2-yl)phenyl)-7*H*-imidazo[4,5-c]pyridazine (146 mg, 0.4 mmol) were used as starting materials to obtain the title compound (30 mg, 15%). LC-MS: m/z $[M+H]^+$ = 418.

**143: 6-(5-(7-Ethyl-7*H*-imidazo[4,5-*c*]pyridazin-4-yl)-2-fluorophenyl)-7-methoxy-2-methyl-3,4-dihydroisoquinolin-1(2H)-one**

6-(5-(7-Ethyl-7*H*-imidazo[4,5-c]pyridazin-4-yl)-2-fluorophenyl)-7-methoxy-2-methyl-1,2,3,4-tetrahydroisoquinoline (30 mg, 0.07 mmol), iodine (137 mg, 0.54 mmol), and sodium bicarbonate (42 mg, 0.7 mmol) were added to tetrahydrofuran/water (2.5/1 mL), and the mixture was stirred at room temperature for 16 hours. The reaction system was quenched with sodium thiosulfate aqueous solution and extracted with ethyl acetate and water. The organic phase was concentrated and purified by preparative thin-layer chromatography (dichloromethane/methanol = 20/1) to obtain the title compound (30 mg, 99%).

$^1H$ NMR (400 MHz, $CDCl_3$) δ 9.36 (s, 1 H), 8.31 - 8.22 (m, 2 H), 8.18 (d, *J* = 5.4 Hz, 1 H), 7.74 (s, 1 H), 7.33 (t, *J* = 9.0 Hz, 1 H), 7.17 (s, 1 H), 4.57 (q, *J* = 7.3 Hz, 2 H), 3.87 (s, 3 H), 3.60 (t, *J* = 6.6 Hz, 2 H), 3.19 (s, 3 H), 3.00 (t, *J* = 6.6 Hz, 2 H), 1.68 (t, *J* = 7.3 Hz, 3 H). LC-MS: m/z $[M+H]^+$ = 432.

Example 144

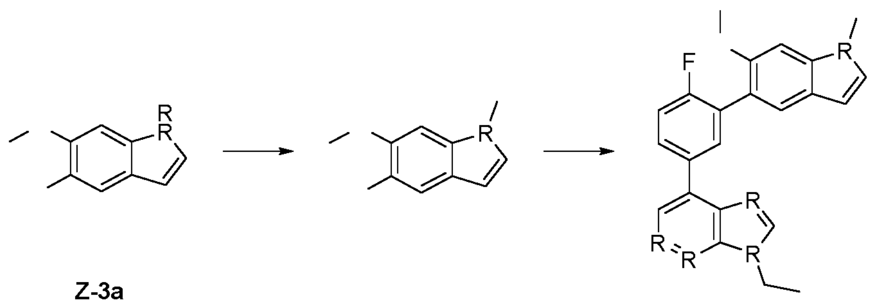

Z-3a

**144-1: 5-Bromo-6-methoxy-1-methyl-1*H*-indole**

5-Bromo-6-methoxy-1*H*-indole (CAS: 177360-11-1, 200 mg, 0.89 mmol), sodium hydride (53 mg, 1.3 mmol), and iodomethane (150 mg, 1.1 mmol) were sequentially added to 10 mL of tetrahydrofuran, and the mixture was stirred at room temperature for 1 hour. The reaction mixture was quenched with water, concentrated, and purified by preparative thin-layer chromatography (petroleum ether: ethyl acetate = 10:1) to obtain the title compound (170 mg, 80%) as a yellow solid. LC-MS: m/z $[M+H]^+$ = 240.

**144: 7-Ethyl-4-(4-fluoro-3-(6-methoxy-1-methyl-1*H*-indol-5-yl)phenyl)-7*H*-imidazo[4,5-*c*]pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-c]pyridazine (200 mg, 0.54 mmol) and 5-bromo-6-methoxy-1-methyl-1*H*-indole (130 mg, 0.54 mmol) were used as starting materials to obtain the title compound (150 mg, 68%) as a yellow solid.

$^1$H NMR (400 MHz, CHLOROFORM-d) δ 9.39 (s, 1 H) 8.27 (s, 2 H) 8.14 - 8.24 (m, 1 H) 7.59 (s, 1 H) 7.33 (s, 1 H) 7.01 (br. s., 1 H) 6.89 (s, 1 H) 6.48 (br. s., 1 H) 4.58 (d, *J* = 7.34 Hz, 2 H) 3.90 (s, 3 H) 3.82 (s, 3 H) 1.70 (t, *J* = 7.09 Hz, 3 H). LC-MS: m/z [M+H]$^+$ = 402.

Example 145

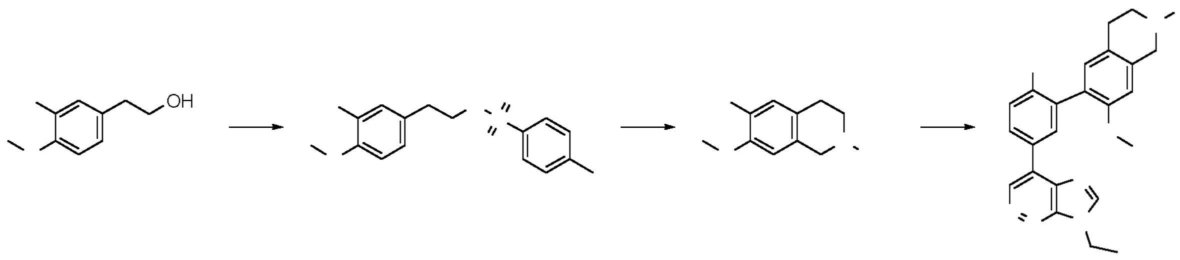

**145-1: N-(3-Bromo-4-methoxyphenethyl)-4-methylbenzenesulfonamide**

2-(3-Bromo-4-methoxyphenyl)ethan-1-amine (2 g, 8.69 mmol), triethylamine (3.64 g, 26.07 mmol), and 4-methylbenzenesulfonyl chloride (1.65 g, 8.69 mmol) were added to dichloromethane (20 mL), and the mixture was stirred at room temperature for 16 hours. The reaction mixture was poured into water, extracted with ethyl acetate, and the organic phase was concentrated to obtain the title compound (2 g, 60%). LC-MS: m/z [M+H]$^+$ = 384.

**145-2: 6-Bromo-7-methoxy-2-tosyl-1,2,3,4-tetrahydroisoquinoline**

*N*-(3-Bromo-4-methoxyphenethyl)-4-methylbenzenesulfonamide (1 g, 2.6 mmol), 85% phosphoric acid (5 mL), and dimethoxymethane (5 mL) were added to toluene (10 mL), and the mixture was stirred at 60°C for 16 hours under an argon atmosphere. The reaction mixture was concentrated, extracted with ethyl acetate and water, and purified by preparative thin-layer chromatography (dichloromethane/methanol = 20/1) to obtain the title compound (450 mg, 58%). LC-MS: m/z [M+H]$^+$ = 396, 398.

**145: 6-(5-(7-Ethyl-7H-imidazo[4,5-c]pyridazin-4-yl)-2-fluorophenyl)-7-methoxy-2-tosyl-1,2,3,4-tetrahydroisoquinoline**

Following the same experimental procedure as in Example 1, 6-bromo-7-methoxy-2-tosyl-1,2,3,4-tetrahydroisoquinoline (100 mg, 0.25 mmol) and 7-ethyl-4-(4-fluoro-3-(4,4,5,5-tetramethyl-1,3,2-dioxaborolan-2-yl)phenyl)-7*H*-imidazo[4,5-c]pyridazine (97 mg, 0.25 mmol) were used as starting materials to obtain the title compound (30 mg).

$^1$H NMR (400 MHz, $CDCl_3$) δ 9.33 (s, 1 H), 8.25 (br. s., 2 H), 8.11 (d, *J* = 4.9 Hz, 1 H), 7.74 (d, *J* = 7.3 Hz, 2 H), 7.36 - 7.28 (m, 3 H), 7.06 (s, 1 H), 6.66 (s, 1 H), 4.56 (q, *J* = 7.3 Hz, 2 H), 4.30 (br. s., 2 H), 3.76 (s, 3 H), 3.38 (br. s., 2 H), 2.90 (br. s., 2 H), 2.43 (s, 3 H), 1.32 (br. s., 3 H). LC-MS: m/z [M+H]$^+$ = 558.

Example 146 and Example 147

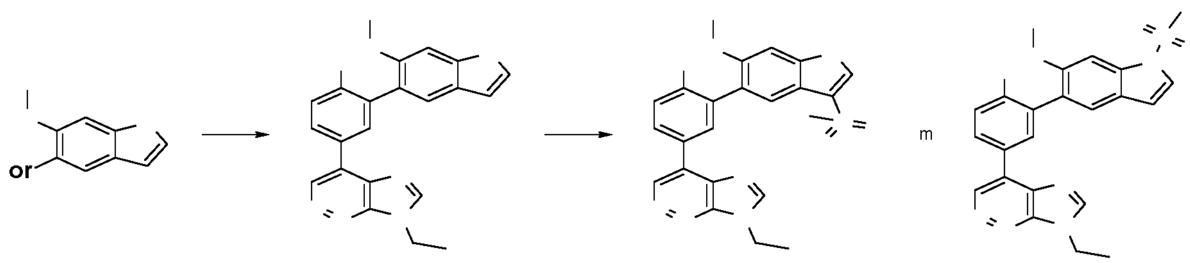

THF.65

**146-1: 7-Ethyl-4-(4-fluoro-3-(6-methoxy-1H-indol-5-yl)phenyl)-7H-imidazo[4,5-c]pyridazine**

Following the same experimental procedure as in Example 1, 7-ethyl-4-(4-fluoro-3-(4,4,5,5-tetramethyl-1,3,2-dioxaborolan-2-yl)phenyl)-7*H*-imidazo[4,5-*c*]pyridazine (81 mg, 0.2 mmol) and 5-bromo-6-methoxy-1*H*-indole (50 mg, 0.2 mmol) were used as starting materials to obtain the title compound (20 mg, 23%) as a yellow solid. LC-MS: m/z $[M+H]^+$ = 388.

**146: 7-Ethyl-4-(4-fluoro-3-(6-methoxy-3-(methylsulfonyl)-1*H*-indol-5-yl)phenyl)-7*H*-imidazo[4,5-*c*]pyridazine**

**147: 7-Ethyl-4-(4-fluoro-3-(6-methoxy-1-(methylsulfonyl)-1*H*-indol-5-yl)phenyl)-7*H*-imidazo[4,5-*c*]pyridazine**

7-Ethyl-4-(4-fluoro-3-(6-methoxy-1*H*-indol-5-yl)phenyl)-7*H*-imidazo[4,5-*c*]pyridazine (20 mg, 0.05 mmol), MsCl (12 mg, 0.1 mmol), and tetrabutylammonium hydrogen sulfate (20 mg, 0.01 mmol) were sequentially added to 2 mL of toluene and 2 mL of 50% potassium hydroxide aqueous solution, and the mixture was stirred at room temperature for 30 minutes. The reaction mixture was added with water, extracted with dichloromethane, dried, concentrated, and purified by column chromatography (dichloromethane: methanol = 30:1) to obtain the title compounds Example 200 (7 mg, 29%) and Example 199 (2 mg, 8%).

146: $^1$H NMR (400 MHz, DMSO-$d_6$) δ 11.98 - 12.16 (m, 1 H) 9.40 - 9.61 (m, 1 H) 8.73 - 8.87 (m, 1 H) 8.35 - 8.55 (m, 2 H) 7.85 - 8.05 (m, 1 H) 7.61 - 7.76 (m, 1 H) 7.39 - 7.52 (m, 1 H) 7.10 - 7.25 (m, 1 H) 4.37 - 4.60 (m, 2 H) 3.78 (br. s., 3 H) 3.16 (br. s., 3 H) 1.52 (br. s., 3 H). LC-MS: m/z $[M+H]^+$ = 466.

147: $^1$H NMR (400 MHz, DMSO-$d_6$) δ 9.56 (br. s., 1 H) 8.85 (br. s., 1 H) 8.48 (d, *J* = 9.29 Hz, 2 H) 7.66 (br. s., 1 H) 7.41 - 7.57 (m, 3 H) 6.82 (br. s., 1 H) 4.51 (d, *J* = 5.87 Hz, 2 H) 3.85 (br. s., 3 H) 3.50 (br. s., 3 H) 1.55 (br. s., 3 H). LC-MS: m/z $[M+H]^+$ = 466.

Example 148

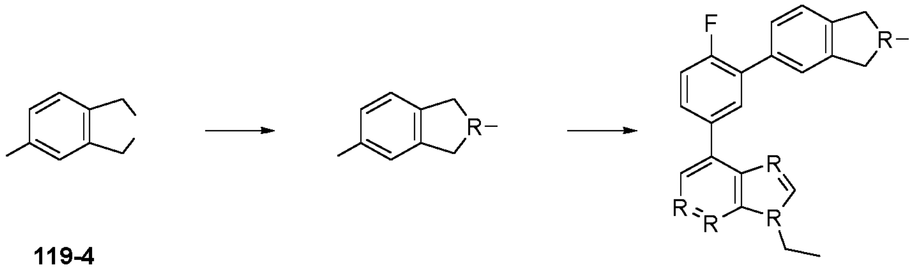

**119-4**

**148-1: 5-Bromo-2-(methylsulfonyl)isoindoline**

5-Bromoisoindoline (120 mg, 0.61 mmol), methylsulfonyl chloride (177 mg, 1.22 mmol), and triethylamine (185 mg, 1.83 mmol) were added to dichloromethane (5 mL), and the mixture was stirred at room temperature for 1 hour. The reaction mixture was added dropwise to saturated ammonium chloride aqueous solution (20 mL) and extracted with dichloromethane (20 mL × 3). The organic phases were combined, washed with saturated brine (20 mL), concentrated, and separated by preparative thin-layer chromatography (dichloromethane/methanol = 30/1) to obtain the title compound (40 mg, 24%) as a white solid. LC-MS: m/z $[M+H]^+$ = 235, 237.

**148: 7-Ethyl-4-(4-fluoro-3-(2-(methylsulfonyl)isoindol-5-yl)phenyl)-7*H*-imidazo[4,5-*c*]pyridazine**

Following the same experimental procedure as in Example 1, 5-bromo-2-(methylsulfonyl)isoindoline (40 mg, 0.14 mmol) and 7-ethyl-4-(4-fluoro-3-(4,4,5,5-tetramethyl-1,3,2-dioxaborolan-2-yl)phenyl)-7*H*-imidazo[4,5-*c*]pyridazine (52 mg, 0.14 mmol) were used as starting materials to obtain the title compound (28 mg, 46%) as a brown solid.

$^1$H NMR (400 MHz, CHLOROFORM-*d*) δ = 9.37 (br. s., 1 H), 8.39 - 8.24 (m, 2 H), 8.20 (br. s., 1 H), 7.58 (d, *J* = 7.3 Hz, 1 H), 7.52 (br. s., 1 H), 7.43 - 7.31 (m, 2 H), 4.78 (br. s., 4 H), 4.58 (d, *J* = 6.8 Hz, 2 H), 2.91 (br. s., 3 H), 1.69 (t, *J* = 7.1 Hz, 3 H). LC-MS: m/z $[M+H]^+$ = 438.

Example 149

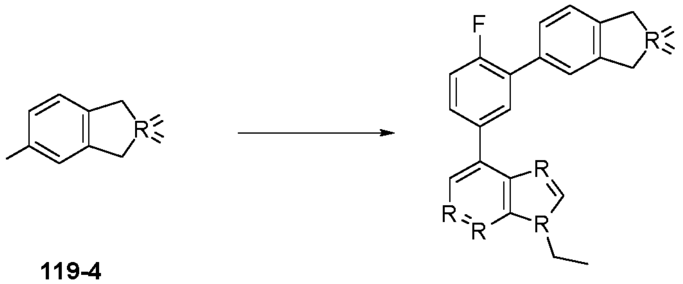

**119-4**

**149: 5-(5-(7-Ethyl-7*H*-imidazo[4,5-*c*]pyridazin-4-yl)-2-fluorophenyl)-1,3-dihydrobenzo[*c*]thiophene 2,2-dioxide**

Following the same experimental procedure as in Example 1, 5-(7-ethyl-7*H*-imidazo[4,5-*c*]pyridazin-4-yl)-2-fluorophenyl)boronic acid (138 mg, 0.48 mmol) and 5-bromo-1,3-dihydrobenzo[*c*]thiophene 2,2-dioxide (89 mg, 0.36 mmol) were used as starting materials to obtain the title compound (74 mg, 50%) as a brown solid.

$^1$H NMR (400 MHz, CHLOROFORM-*d*) δ = 9.37 (s, 1 H), 8.34 (d, *J* = 5.4 Hz, 1 H), 8.29 (s, 1 H), 8.20 (br. s., 1 H), 7.63 (d, *J* = 7.8 Hz, 1 H), 7.58 (br. s., 1 H), 7.49 - 7.33 (m, 2 H), 4.58 (d, *J* = 6.8 Hz, 2 H), 4.45 (d, *J* = 7.8 Hz, 4 H), 1.69 (br. s., 3 H). LC-MS: m/z [M+H]$^+$ = 409.

Example 150

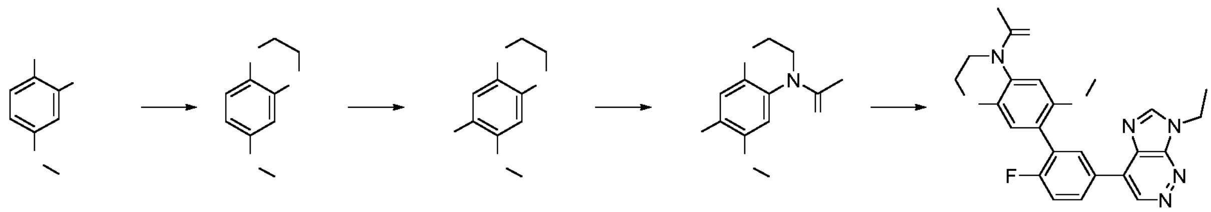

**150-1: 6-Methoxy-3,4-dihydro-2*H*-benzo[*b*][1,4]oxazine**

2-Amino-4-methoxyphenol (1000 mg, 7.19 mmol), 1,2-dibromoethane (1350 mg, 7.19 mmol), and potassium carbonate (2980 mg, 21.57 mmol) were sequentially added to *N,N*-dimethylformamide (10 mL), and the mixture was stirred at 100°C for 16 hours. The reaction mixture was added with water (60 mL) and extracted with ethyl acetate (40 mL × 3). The organic phase was washed with saturated brine (50 mL), dried over sodium sulfate, concentrated, and separated by preparative thin-layer chromatography (petroleum ether/ethyl acetate = 1/1) to obtain the title compound (300 mg, 25%). LC-MS: m/z [M+H]$^+$ = 166.

**150-2: 7-Bromo-6-methoxy-3,4-dihydro-2*H*-benzo[*b*][1,4]oxazine**

6-Methoxy-3,4-dihydro-2*H*-benzo[*b*][1,4]oxazine (200 mg, 1.21 mmol) and *N*-bromosuccinimide (215 mg, 1.21 mmol) were sequentially added to acetonitrile (5 mL), and the mixture was stirred at 60°C for 3 hours. The reaction mixture was separated by preparative thin-layer chromatography (petroleum ether/ethyl acetate = 1/1) to obtain the title compound (80 mg, 27%) as a red solid. LC-MS: m/z [M+H]$^+$ = 244, 246.

**150-3: 1-(7-Bromo-6-methoxy-2,3-dihydro-4*H*-benzo[*b*][1,4]oxazin-4-yl)ethan-1-one**

7-Bromo-6-methoxy-3,4-dihydro-2*H*-benzo[*b*][1,4]oxazine (100 mg, 0.41 mmol), acetic anhydride (42 mg, 0.41 mmol), and triethylamine (83 mg, 0.82 mmol) were sequentially added to dichloromethane (5 mL), and the mixture was stirred at room temperature for 2 hours. The reaction mixture was separated by preparative thin-layer chromatography (petroleum ether/ethyl acetate = 1/1) to obtain the title compound (50 mg, 43%). LC-MS: m/z [M+H]$^+$ = 286, 288.

**150: 1-(7-(5-(7-Ethyl-7H-imidazo[4,5-c]pyridazin-4-yl)-2-fluorophenyl)-6-methoxy-2,3-dihydro-4H-benzo[b][1,4]oxazin-4-yl)ethan-1-one**

Following the same experimental procedure as in Example 1, 1-(7-bromo-6-methoxy-2,3-dihydro-4*H*-benzo[*b*][1,4]oxazin-4-yl)ethan-1-one (40 mg, 0.14 mmol) and 5-(7-ethyl-7*H*-imidazo[4,5-*c*]pyridazin-4-yl)-2-fluorophenyl)boronic acid (40 mg, 0.14 mmol) were used as starting materials to obtain the title compound (44 mg, 70%).

$^1$H NMR (400 MHz, CHLOROFORM-d) δ = 9.37 (s, 1 H), 8.27 (s, 2 H), 8.18 (d, *J* = 6.4 Hz, 1 H), 7.33 (t, *J* = 9.3 Hz, 1 H), 7.27 (s, 1 H), 6.96 (s, 1 H), 4.58 (q, *J* = 7.2 Hz, 2 H), 4.30 (br. s., 2 H), 3.97 (br. s., 2 H), 3.78 (s, 3 H), 2.41 (br. s., 3 H), 1.69 (t, *J* = 7.3 Hz, 3 H). LC-MS: m/z [M+H]$^+$ = 448.

Example 151

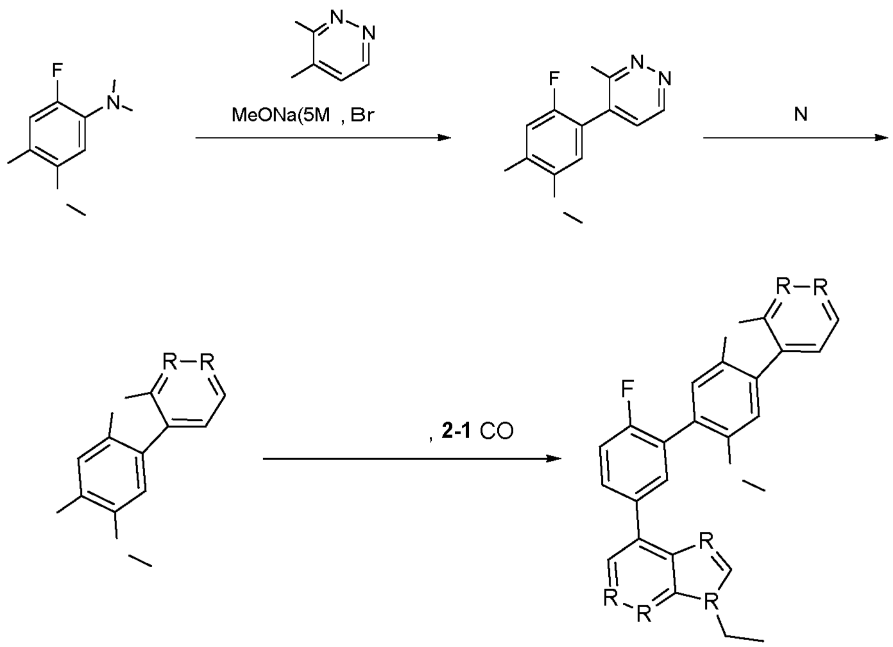


**151-1: 4-(4-Chloro-2-fluoro-5-methoxyphenyl)pyridazin-3-ol**

(4-Chloro-2-fluoro-5-methoxyphenyl)boronic acid (500 mg, 2.45 mmol) was added to a mixed solvent of 1,4-dioxane (18 mL) and water (2 mL) containing 4-chloropyridazin-3-ol (350 mg, 2.68 mmol), cesium carbonate (1.6 g, 4.9 mmol), and [1,1'-bis(di-*tert*-butylphosphino)ferrocene]dichloropalladium(II) (162 mg, 0.25 mmol) at 25°C. After the gas in the system was replaced with nitrogen, the mixture was reacted under microwave irradiation at 100°C for 3 hours. The reaction mixture was purified by prep-HPLC and lyophilized to obtain 4-(4-chloro-2-fluoro-5-methoxyphenyl)pyridazin-3-ol (150 mg, 24%) as an off-white solid. LC-MS: m/z [M+H]$^+$ = 255.

**151-2: 7-Chloro-6-methoxybenzofuro[2,3-c]pyridazine**

4-(4-Chloro-2-fluoro-5-methoxyphenyl)pyridazin-3-ol (150 mg, 0.59 mmol) was dissolved in *N*-methylpyrrolidone (4 mL), and potassium carbonate (409 mg, 2.95 mmol) was added thereto. After the gas in the system was replaced with nitrogen, the mixture was heated to 150°C under microwave irradiation and reacted for 1.5 hours. The reaction mixture was purified by prep-HPLC to obtain 7-chloro-6-methoxybenzofuro[2,3-*c*]pyridazine (80 mg, 57%) as an off-white solid. LC-MS: m/z [M+H]$^+$ = 236.

**151: 7-(5-(7-Ethyl-7H-imidazo[4,5-c]pyridazin-4-yl)-2-fluorophenyl)-6-methoxybenzofuro[2,3-c]pyridazine**

Following the same experimental procedure as in Example 1, 7-chloro-6-methoxybenzofuro[2,3-*c*]pyridazine (50 mg, 0.21 mmol) and 5-(7-ethyl-7*H*-imidazo[4,5-*c*]pyridazin-4-yl)-2-fluorophenyl)boronic acid (61 mg, 0.21 mmol) were used as starting materials to obtain the title compound (40 mg, 43%) as an off-white solid.

$^1$H NMR (400 MHz, DMSO-$d_6$) 9.60 (s, 1 H), 9.38 (d, $J$ = 5.0 Hz, 1 H), 8.87 (s, 1 H), 8.56 (t, $J$ = 5.6 Hz, 3 H), 8.17 (s, 1 H), 8.00 (s, 1 H), 7.57 (t, $J$ = 9.3 Hz, 1 H), 4.52 (q, $J$ = 7.2 Hz, 2 H), 3.93 (s, 3 H), 1.56 (t, $J$ = 7.3 Hz, 3 H). LC-MS: m/z $[M+H]^+$ = 443.

Example 152

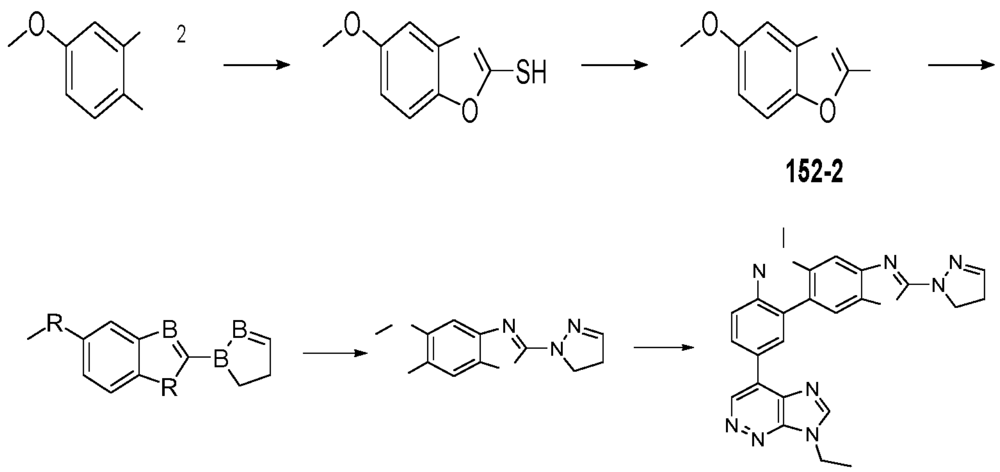

152-2

**152-1: 5-Methoxybenzo[*d*]oxazole-2-thiol**

2-Amino-4-methoxyphenol (CAS: 20734-76-3, 1 g, 7.2 mmol) was dissolved in 10 mL of ethanol, then potassium ethylxanthate (1.7 g, 10.8 mmol) was added thereto, and the mixture was heated to reflux for 2 hours. The reaction mixture was concentrated, added with water to dissolve the solid, acidified with 1 M hydrochloric acid, and a solid was precipitated. The residue was dried to obtain the title compound (800 mg, 61%) as a gray solid. LC-MS: m/z $[M+H]^+$ = 182.

**152-2: 2-Chloro-5-methoxybenzo[d]oxazole**

5-Methoxybenzo[*d*]oxazole-2-thiol (800 mg, 4.4 mmol) was dissolved in 10 mL of thionyl chloride, then a drop of *N,N*-dimethylformamide was added thereto, and the mixture was heated to 80°C and stirred for 1 hour. The reaction mixture was concentrated and purified by preparative thin-layer chromatography (petroleum ether: ethyl acetate = 3:1) to obtain the title compound (600 mg, 75%) as a white solid. LC-MS: m/z $[M+H]^+$ = 184.

**152-3: 2-(4,5-dihydro-1*H*-pyrazol-1-yl)-5-methoxybenzo[*d*]oxazole**

2-Chloro-5-methoxybenzo[*d*]oxazole (200 mg, 1.1 mmol), $Pd_2(dba)_3$ (98 mg, 0.1 mmol), 4,5-dihydro-1*H*-pyrazole (150 mg, 2.2 mmol), 1,3-bis[2,6-bis(propan-2-yl)phenyl]imidazol-1-ylium chloride (98 mg, 0.1 mmol), and cesium carbonate (1 g, 3.3 mmol) were dissolved in 10 mL of dioxane, and the mixture was heated to 90°C and stirred for 2 hours. The reaction mixture was concentrated and purified by preparative thin-layer chromatography (petroleum ether: ethyl acetate = 1:1) to obtain the title compound (100 mg, 42%) as a white solid. LC-MS: m/z $[M+H]^+$ = 218.

**152-4: 6-Bromo-2-(4,5-dihydro-1*H*-pyrazol-1-yl)-5-methoxybenzo[*d*]oxazole**

2-(4,5-Dihydro-1*H*-pyrazol-1-yl)-5-methoxybenzo[*d*]oxazole (100 mg, 0.46 mmol) was dissolved in 10 mL of acetonitrile, then *N*-Bromosuccinimide (82 mg, 0.46 mmol) was added thereto, and the mixture was stirred at room temperature for 1 hour. The reaction mixture was concentrated and purified by preparative thin-layer chromatography (petroleum ether: ethyl acetate = 3:1) to obtain the title compound (100 mg, 73%) as a white solid. LC-MS: m/z $[M+H]^+$ = 296.

**152: 2-(4,5-Dihydro-1*H*-pyrazol-1-yl)-6-(5-(7-ethyl-7*H*-imidazo[4,5-*c*]pyridazin-4-yl)-2-fluorophenyl)-5-methoxybenzo[*d*] oxazole**

Following the same experimental procedure as in Example 7, 7-ethyl-4-(4-fluoro-3-(4,4,5,5-tetramethyl-1,3,2-dioxaborolan-2-yl)phenyl)-7*H*-imidazo[4,5-*c*]pyridazine (80 mg, 0.28 mmol) and 6-bromo-2-(4,5-dihydro-1*H*-pyrazol-1-yl)-5-methoxybenzo[*d*]oxazole (83 mg, 0.28 mmol) were used as starting materials to obtain the title compound (29 mg, 23%) as a white solid.

$^1$H NMR (400 MHz, CHLOROFORM-*d*) δ 9.39 (s, 1 H) 8.17 - 8.31 (m, 3 H) 7.39 (s, 1 H) 7.34 (t, *J* = 8.56 Hz, 1 H) 7.13 (d, *J* = 14.67 Hz, 2 H) 4.53 - 4.63 (m, 2 H) 4.10 (t, *J* = 10.27 Hz, 2 H) 3.85 (s, 3 H) 3.12 (t, *J* = 10.03 Hz, 2 H) 1.70 (t, *J* = 7.34 Hz, 3 H). LC-MS: m/z [M+H]$^+$ = 458.

Example 153

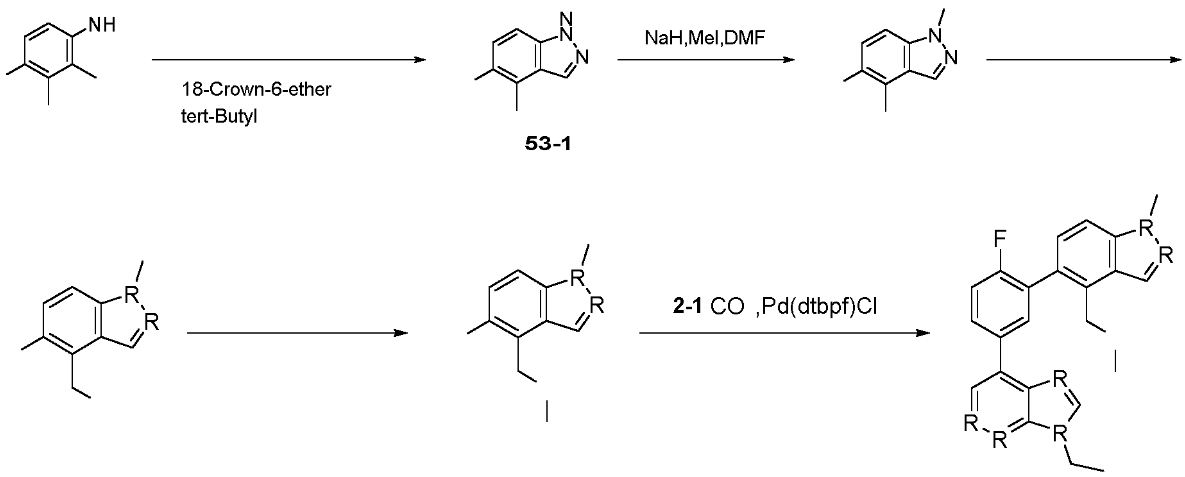


**153-1: 5-Bromo-4-methyl-1*H*-indazole**

4-Bromo-2,3-dimethylaniline (2.0 g, 10 mmol) was added to chloroform (30 mL), then the mixture was cooled to 0°C, and acetic anhydride (2.04 g, 20 mmol) was added dropwise thereto. After 5 minutes, potassium acetate (1.03 g, 10.5 mmol) and 18-crown-6 (5 mL) were added thereto. After 5 minutes, *tert*-butyl nitrite (2.6 mL) was added dropwise thereto, and the mixture was reacted at 0°C for 10 minutes. The mixture was stirred at 70°C for 16 hours. The completion of the reaction was detected by LCMS. The reaction mixture was added with dichloromethane and sodium bicarbonate solution to separate the organic phase, which was dried and concentrated. The crude product was added to 10 mL of methanol and 10 mL of hydrochloric acid, and the mixture was stirred at 50°C for 2 hours. The reaction mixture was poured into water, extracted with ethyl acetate, rotary evaporated to dryness, and purified by normal-phase silica gel column chromatography (petroleum ether: ethyl acetate = 100:1 to 1:1) to obtain 5-bromo-4-methyl-1*H*-indazole (2.5 g, 100%) as an off-white solid. LC-MS: m/z [M+H]$^+$ = 211.

**153-2: 5-Bromo-1,4-dimethyl-1*H*-indazole**

5-Bromo-4-methyl-1*H*-indazole (1.5 g, 7.1 mmol) was dispersed in *N,N*-dimethylformamide (15 mL), then NaH (298 mg, 7.1 mmol) was added thereto in batches, and the mixture was stirred for 20 minutes. Iodomethane (2.9 g, 21 mmol) was then added dropwise thereto, and the mixture was stirred for 1 hour. The reaction mixture was poured into water, extracted twice with ethyl acetate, rotary evaporated to dryness, concentrated, and purified by normal-phase chromatography (petroleum ether: ethyl acetate = 100:1 to 1:10) to obtain 5-bromo-1,4-dimethyl-1*H*-indazole (0.9 g, 56%, relatively small polarity) as a yellow oil; another product, **153-2B:** 5-bromo-2,4-dimethyl-2*H*-indazole (0.2 g, 13%, relatively large polarity). LC-MS: m/z [M+H]$^+$ = 225.

**153-3: 5-Bromo-4-(bromomethyl)-1-methyl-1*H*-indazole**

5-Bromo-1,4-dimethyl-1*H*-indazole (0.3 g, 1.33 mmol) was added to a mixed solvent of carbon tetrachloride (10 mL) and dichloromethane (4 mL) containing *N*-bromosuccinimide (260 mg, 1.46 mmol) and dibenzoyl peroxide (240 mg, 1 mmol) at 25°C. The mixture was stirred at 90°C for 16 hours. The reaction mixture was directly mixed with silica gel and then purified by column chromatography (petroleum ether: ethyl acetate = 100:1 to 10:2) to obtain 5-bromo-4-(bromomethyl)-1-methyl-1*H*-indazole (0.3 g, 75%). LC-MS: m/z [M+H]$^+$ = 304.

**153-4: 5-Bromo-4-(methoxymethyl)-1-methyl-1*H*-indazole**

5-Bromo-4-(bromomethyl)-1-methyl-1*H*-indazole (300 mg, 1.0 mmol) was dispersed in methanol (8 mL) at 25°C. 5 M sodium methoxide solution (0.2 mL, 1.0 mmol) was diluted to 2 mL of methanol and then added dropwise to the reaction mixture. The mixture was stirred at 25°C for 16 hours. After LCMS detection, the reaction mixture was added with a few drops of water, directly mixed with silica gel powder, and purified by column chromatography (petroleum ether: ethyl acetate = 100:1 to 5:1) to obtain 5-bromo-4-(methoxymethyl)-1-methyl-1*H*-indazole (200 mg, 78%) as a yellow solid. LC-MS: m/z $[M+H]^+$ = 257.

**153: 7-Ethyl-4-(4-fluoro-3-(4-(methoxymethyl)-1-methyl-1*H*-indazol-5-yl)phenyl)-7*H*-imidazo[4,5-*c*]pyridazine**

Following the same experimental procedure as in Example 1, 5-bromo-4-(methoxymethyl)-1-methyl-1*H*-indazole (200 mg, 0.78 mmol) and (5-(7-ethyl-7*H*-imidazo[4,5-*c*]pyridazin-4-yl)-2-fluorophenyl)boronic acid (223 mg, 0.78 mmol) were used as starting materials to obtain the title compound (95 mg, 30%) as an off-white solid.

$^1$H NMR (400 MHz, DMSO-$d_6$) 9.56 (s, 1 H), 8.86 (s, 1 H), 8.52 (dq, *J* = 3.8, 2.3 Hz, 2 H), 8.23 (d, *J* = 0.8 Hz, 1 H), 7.72 (d, *J*= 8.6 Hz, 1 H), 7.57 (t, *J* = 9.1 Hz, 1 H), 7.39 (d, *J* = 8.6 Hz, 1 H), 4.64 (s, 2 H), 4.51 (q, *J* = 7.2 Hz, 2 H), 4.11 (s, 3 H), 3.18 (s, 3 H), 1.55 (t, *J* = 7.3 Hz, 3 H). LC-MS: m/z $[M+H]^+$ = 417.

Example 154

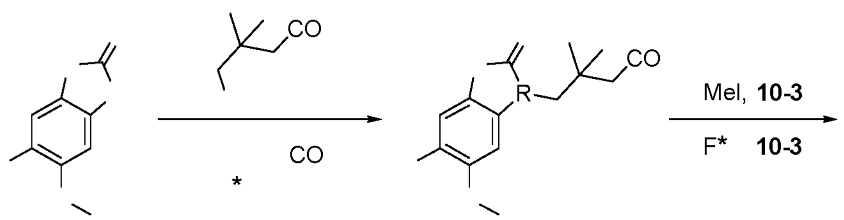


**10-3**

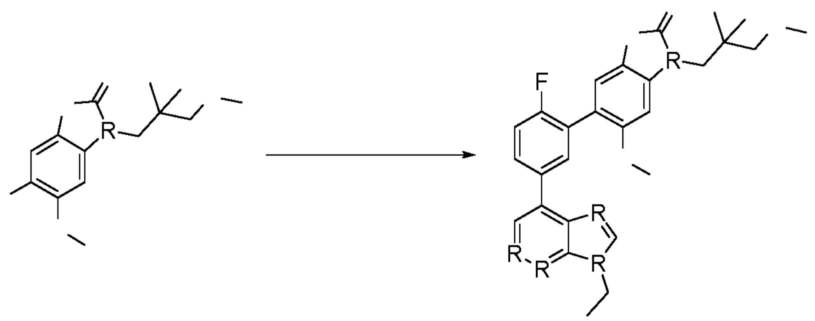

**154-1: 6-Bromo-3-(3-hydroxy-2,2-dimethylpropyl)-5-methoxybenzo[*d*]oxazol-2(3*H*)-one**

6-Bromo-5-methoxybenzo[d]oxazol-2(3*H*)-one (2 g, 40 mmol) was dissolved in NMP (80.0 mL), then potassium carbonate (7 g, 40 mmol) was added thereto, and the mixture was reacted under microwave irradiation at 100°C for 1 hour. The reaction mixture was quenched with saturated sodium bicarbonate aqueous solution (100 mL), extracted with dichloromethane (100 mL × 3), and rotary evaporated to dryness to obtain a crude product, which was purified by column chromatography (petroleum ether: ethyl acetate = 10:1 to 2:1) to obtain the title compound (100 mg, 8%) as an off-white solid. LC-MS: m/z $[M+1]^+$ = 331.

**154-2: 6-Bromo-5-methoxy-3-(3-methoxy-2,2-dimethylpropyl)benzo[*d*]oxazol-2(3*H*)-one**

6-Bromo-3-(3-hydroxy-2,2-dimethylpropyl)-5-methoxybenzo[*d*]oxazol-2(3H)-one (26 mg, 0.078 mmol), iodomethane (22 mg, 0.156 mmol), and sodium hydride (6.4 mg, 0.156 mmol) were dissolved in a solution of tetrahydrofuran (2.0 mL), and the mixture was reacted at room temperature for 5 hours. The reaction mixture was quenched with water and

extracted with ethyl acetate. The organic phase was dried over anhydrous sodium sulfate and concentrated to obtain the title compound (20 mg, 38%) as an off-white solid. LC-MS: m/z $[M+1]^+$ = 345.

**154: 6-(5-(7-Ethyl-7*H*-imidazo[4,5-*c*]pyridazin-4-yl)-2-fluorophenyl)-5-methoxy-3-(3-methoxy-2,2-dimethylpropyl)benzo[*d*]oxazol-2(3*H*)-one**

Following the same experimental procedure as in Example 1, 6-bromo-5-methoxy-3-(3-methoxy-2,2-dimethylpropyl)benzo[*d*]oxazol-2(3*H*)-one (10.0 mg, 0.028 mmol) and 5-(7-isopropyl-7*H*-imidazo[4,5-*c*]pyridazin-4-yl)-2-fluorophenyl)boronic acid (8 mg, 0.028 mmol) were used as starting materials to obtain the title product (10 mg, 45%) as an off-white solid.

$^1$H NMR (400 MHz, $CDCl_3$) 9.45 (s, 1 H), 8.34 (d, *J* = 58.9 Hz, 3 H), 7.36 (s, 1 H), 7.21 (s, 1 H), 6.97 (s, 1 H), 4.60 (s, 2 H), 3.86 (s, 3 H), 3.76 (s, 2 H), 3.42 (s, 3 H), 3.15 (s, 2 H), 1.72 (s, 3 H), 1.08 (s, 6 H). LC-MS: m/z $[M+1]^+$ = 507.

Example 155

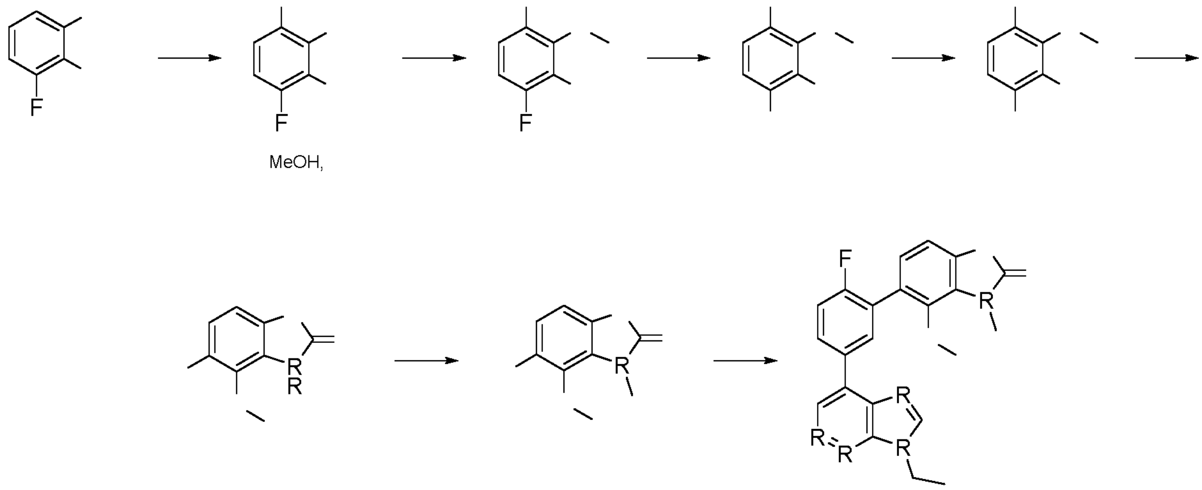


**155-1: 6-Bromo-3-fluoro-2-nitrophenol**

3-Fluoro-2-nitrophenol (2000 mg, 12.73 mmol) was added to acetic acid (10 mL). Liquid bromine (2023 mg, 12.73 mmol) was diluted with acetic acid (10 mL) and added dropwise to the reaction mixture in an ice bath. The reaction mixture was stirred at room temperature for 2 hours. The reaction mixture was poured into water (100 mL) and extracted with ethyl acetate (50 mL × 3). The organic phase was washed with saturated brine (50 mL), dried over sodium sulfate, concentrated, and separated by preparative thin-layer chromatography (petroleum ether/ethyl acetate = 20/1) to obtain the title compound (1400 mg, 47%). $^1$H NMR (400 MHz, CHLOROFORM-d) $\delta$ = 7.77 (dd, *J* = 5.4, 8.8 Hz, 1 H), 6.76 (t, *J* = 9.8 Hz, 1 H).

**155-2: 1-Bromo-4-fluoro-2-methoxy-3-nitrobenzene**

6-Bromo-3-fluoro-2-nitrophenol (1400 mg, 5.93 mmol), iodomethane (1700 mg, 11.86 mmol), and potassium carbonate (2460 mg, 14.79 mmol) were sequentially added to acetonitrile (10 mL), and the mixture was stirred at 80°C for 2 hours. The reaction mixture was filtered, and the filtrate was concentrated to dryness to obtain the crude title compound (1600 mg, crude product), which was directly used in the next step.

**155-3: 4-Bromo-3-methoxy-2-nitrophenol**

1-Bromo-4-fluoro-2-methoxy-3-nitrobenzene (1400 mg, 5.60 mmol) was added to dimethyl sulfoxide (10 mL), then a solution of sodium hydroxide (448 mg, 11.20 mmol) in water (5 mL) was added to the reaction mixture, and the resulting mixture was stirred at 80°C for 2 hours. The reaction mixture was poured into water (100 mL) and extracted with ethyl acetate (50 mL × 3). The organic phase was washed with saturated brine (50 mL), dried over sodium sulfate, and concentrated to obtain the crude title compound (900 mg, crude product), which was directly used in the next step.

**155-4: 2-Amino-4-bromo-3-methoxyphenol**

4-Bromo-3-methoxy-2-nitrophenol (900 mg, 3.63 mmol) and Raney nickel (426 mg, 7.26 mmol) were added to methanol (10 mL). After the gas in the system was replaced with hydrogen, the mixture was stirred at room temperature for 2 hours under a hydrogen atmosphere. The reaction mixture was filtered, and the filtrate was concentrated to dryness and separated by thin-layer chromatography (petroleum ether/ethyl acetate = 3/1) to obtain the title compound (700 mg, 88%). LC-MS: m/z $[M+H]^+$ = 218, 220.

**155-5: 5-Bromo-4-methoxybenzo[*d*]oxazol-2(3*H*)-one**

2-Amino-4-bromo-3-methoxyphenol (300 mg, 1.38 mmol) and triphosgene (819 mg, 2.76 mmol) were added to tetrahydrofuran (5 mL), and the mixture was stirred at room temperature for 2 hours. The reaction mixture was separated by thin-layer chromatography (petroleum ether/ethyl acetate = 3/1) to obtain the title compound (280 mg, 83%). LC-MS: m/z $[M+H]^+$ = 244, 246.

**155-6: 5-Bromo-4-methoxy-3-methylbenzo[*d*]oxazol-2(3*H*)-one**

5-Bromo-4-methoxybenzo[d]oxazol-2(3H)-one (280 mg, 1.15 mmol), iodomethane (329 mg, 2.30 mmol), and cesium carbonate (1124 mg, 3.45 mmol) were sequentially added to acetonitrile (5 mL), and the mixture was stirred at 80°C for 1 hour. The reaction mixture was filtered, and the filtrate was concentrated to dryness to obtain the crude title compound (210 mg, crude product), which was directly used in the next step. LC-MS: m/z $[M+H]^+$ = 258, 260.

**155: 5-(5-(7-Ethyl-7*H*-imidazo[4,5-*c*]pyridazin-4-yl)-2-fluorophenyl)-4-methoxy-3-methylbenzo[*d*]oxazol-2(3*H*)-one**

Following the same experimental procedure as in Example 1, 5-bromo-4-methoxy-3-methylbenzo[d]oxazol-2(3H)-one (200 mg, 0.77 mmol) and 5-(7-ethyl-7H-imidazo[4,5-c]pyridazin-4-yl)-2-fluorophenyl)boronic acid (220 mg, 0.77 mmol) were used as starting materials to obtain the title compound (109 mg, 34%).

$^1$H NMR (400 MHz, CHLOROFORM-d) δ = 9.38 (s, 1 H), 8.36 - 8.26 (m, 3 H), 7.39 (t, *J* = 8.8 Hz, 1 H), 7.16 - 7.11 (m, 1 H), 7.10 - 7.06 (m, 1 H), 4.58 (q, *J* = 7.2 Hz, 2 H), 3.64 (s, 3 H), 3.53 (s, 3 H), 1.69 (t, *J* = 7.3 Hz, 3 H). LC-MS: m/z $[M+H]^+$ = 420.

Example 156

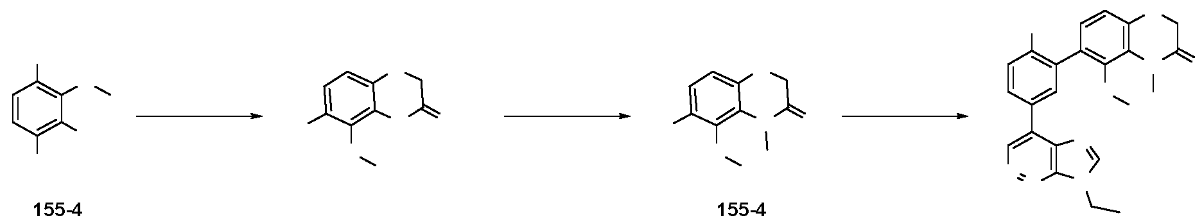

155-4

155-4

**156-1: 6-Bromo-5-methoxy-2*H*-benzo[*b*][1,4]oxazin-3(4*H*)-one**

2-Amino-4-bromo-3-methoxyphenol (300 mg, 1.38 mmol), chloroacetyl chloride (236 mg, 2.07 mmol), and potassium carbonate (572 mg, 4.14 mmol) were added to acetonitrile (8 mL), and the mixture was stirred at 80°C for 16 hours. The reaction mixture was separated by thin-layer chromatography (petroleum ether/ethyl acetate = 3/1) to obtain the title compound (200 mg, 56%). LC-MS: m/z $[M+H]^+$ = 258, 260.

**156-2: 6-Bromo-5-methoxy-4-methyl-2*H*-benzo[*b*][1,4]oxazin-3(4*H*)-one**

6-Bromo-5-methoxy-2*H*-benzo[b][1,4]oxazin-3(4*H*)-one (190 mg, 0.74 mmol), iodomethane (212 mg, 1.48 mmol), and cesium carbonate (723 mg, 2.22 mmol) were sequentially added to acetonitrile (5 mL), and the mixture was stirred at 80°C for 1 hour. The reaction mixture was filtered, and the filtrate was concentrated to dryness to obtain the crude title compound (160 mg, crude product), which was directly used in the next step. LC-MS: m/z $[M+H]^+$ = 272, 274.

**156: 6-(5-(7-Ethyl-7*H*-imidazo[4,5-*c*]pyridazin-4-yl)-2-fluorophenyl)-5-methoxy-4-methyl-2*H*-benzo[*b*][1,4]oxazin-3(4*H*)-one**

Following the same experimental procedure as in Example 1, 6-bromo-5-methoxy-4-methyl-2*H*-benzo[*b*][1,4]oxazin-3(4*H*)-one (150 mg, 0.55 mmol) and 5-(7-ethyl-7*H*-imidazo[4,5-*c*]pyridazin-4-yl)-2-fluorophenyl)boronic acid (157 mg, 0.55 mmol) were used as starting materials to obtain the title compound (46 mg, 19%).

$^1$H NMR (400 MHz, CHLOROFORM-d) δ = 9.38 (s, 1 H), 8.28 (s, 2 H), 8.24 (d, *J* = 6.4 Hz, 1 H), 7.37 (t, *J* = 9.0 Hz, 1 H), 7.09 (d, *J* = 7.8 Hz, 1 H), 6.92 (d, *J* = 8.3 Hz, 1 H), 4.63 - 4.53 (m, 4 H), 3.53 (s, 3 H), 3.48 (s, 3 H), 1.68 (t, *J* = 7.3 Hz, 3 H). LC-MS: m/z $[M+H]^+$ = 434.

Example 157

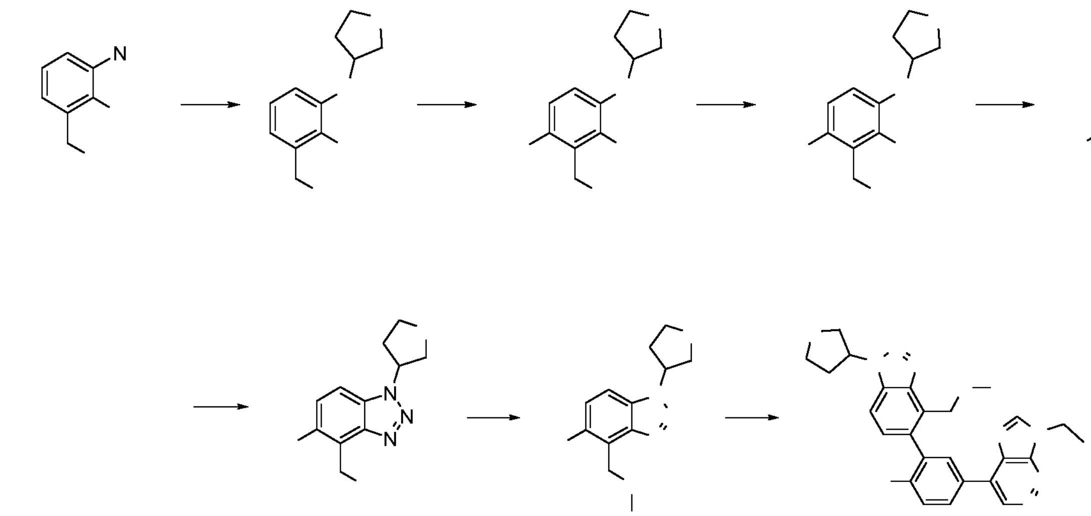

**157-1: (2-Nitro-3-((tetrahydrofuran-3-yl)amino)phenyl)methanol**

(3-Fluoro-2-nitrophenyl)methanol (500 mg, 2.92 mmol) and tetrahydrofuran-3-amine (508 mg, 5.84 mmol) were added to anhydrous ethanol (10 mL), and the mixture was stirred in a sealed container at 100°C overnight. The reaction mixture was directly purified by preparative thin-layer chromatography (petroleum ether/ethyl acetate = 3/1) to obtain the title compound (600 mg, 86%). LC-MS: m/z $[M+H]^+$ = 239, 241.

**157-2: (6-Bromo-2-nitro-3-((tetrahydrofuran-3-yl)amino)phenyl)methanol**

Following the same experimental procedure as in the synthesis method of 2-3 in Example 2, (2-nitro-3-((tetrahydrofuran-3-yl)amino)phenyl)methanol (560 mg, 2.35 mmol) was used as the starting material to obtain the title compound (320 mg, 43%). LC-MS: m/z $[M+H]^+$ = 317, 319.

**157-3: (2-Amino-6-bromo-3-((tetrahydrofuran-3-yl)amino)phenyl)methanol**

Following the same experimental procedure as in the synthesis method of 2-4 in Example 2, (6-bromo-2-nitro-3-((tetrahydrofuran-3-yl)amino)phenyl)methanol (300 mg, 1.05 mmol) was used as the starting material to obtain the title compound (210 mg, 70%). LC-MS: m/z $[M+H]^+$ = 287, 289.

**157-4: (5-Bromo-1-(tetrahydrofuran-3-yl)-1*H*-benzo[*d*][1,2,3]triazol-4-yl)methanol**

Following the same experimental procedure as in the synthesis method of 3-4 in Example 3, (2-amino-6-bromo-3-((tetrahydrofuran-3-yl)amino)phenyl)methanol (200 mg, 0.7 mol) was used as the starting material to obtain the title compound (180 mg, 86%). LC-MS: m/z $[M+H]^+$ = 298, 300.

**157-5: 5-Bromo-4-(methoxymethyl)-1-(tetrahydrofuran-3-yl)-1*H*-benzo[*d*][1,2,3]triazole**

Following the same experimental procedure as in the synthesis method of 2-6 in Example 2, (5-bromo-1-(tetrahydrofuran-3-yl)-1*H*-benzo[*d*][1,2,3]triazol-4-yl)methanol (180 mg, 0.6 mmol) was used as the starting material to obtain the title compound (135 mg, 73%).

**157: 7-Ethyl-4-(4-fluoro-3-(4-(methoxymethyl)-1-(tetrahydrofuran-3-yl)-1*H*-benzo[*d*][1,2,3]triazol-5-yl)phenyl)-7*H*-imidazo[4,5-*c*]pyridazine**

Following the same experimental procedure as in Example 1, 5-bromo-4-(methoxymethyl)-1-(tetrahydrofuran-3-yl)-1*H*-benzo[*d*][1,2,3]triazole (65 mg, 0.21 mmol) and (5-(7-ethyl-7*H*-imidazo[4,5-c]pyridazin-4-yl)-2-fluorophenyl) boronic acid (60 mg, 0.21 mmol) were used as starting materials to obtain the title compound (65 mg, 60%).

$^1$H NMR (400 MHz, CHLOROFORM-d) 9.40 (s, 1 H), 8.42 (d, *J* = 4.9 Hz, 1 H), 8.34 (d, *J* = 6.8 Hz, 1 H), 8.27 (s, 1 H), 7.74 (d, *J* = 8.3 Hz, 1 H), 7.55 (d, *J* = 8.8 Hz, 1 H), 7.40 (t, *J* = 9.0 Hz, 1 H), 5.67 (br. s., 1 H), 4.94 (br. s., 2 H), 4.58 (q, *J* = 7.0 Hz, 2 H), 4.40 - 4.31 (m, 2 H), 4.28 - 4.21 (m, 1 H), 4.08 - 3.99 (m, 1 H), 3.39 (s, 3 H), 2.82 - 2.50 (m, 2 H), 1.72 - 1.68 (m, 3 H). LC-MS: m/z $[M+H]^+$ = 474.

Example 158

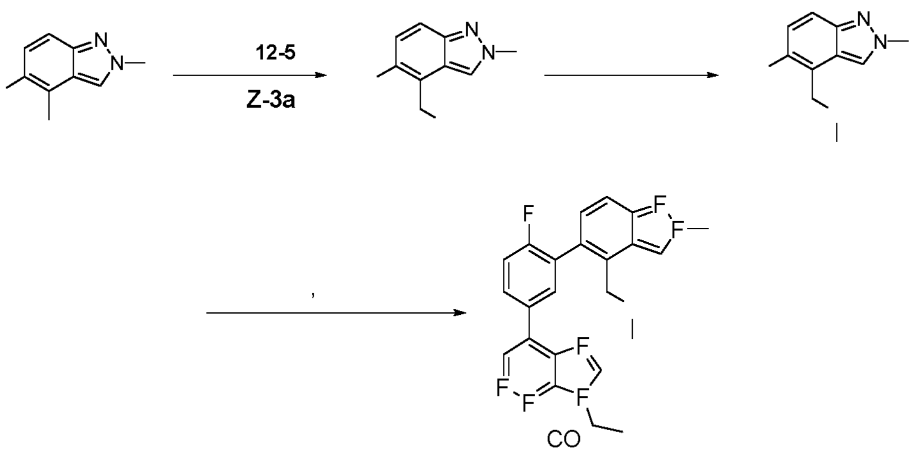


**158-1: 5-Bromo-4-(bromomethyl)-2-methyl-2*H*-indazole**

5-Bromo-2,4-dimethyl-2*H*-indazole (200 mg, 0.89 mmol) was dissolved in $CCl_4$ (20.0 mL), then dichloromethane (6 mL), dibenzoyl peroxide (430 mg, 1.78 mmol), and *N*-bromosuccinimide (178 mg, 1 mmol) were sequentially added thereto, and the mixture was reacted at 90°C for 16 hours. The reaction mixture was mixed with silica gel, purified by normal-phase silica gel column chromatography (ethyl acetate: petroleum ether = 1:5), and rotary evaporated to dryness to obtain 5-bromo-4-(bromomethyl)-2-methyl-2*H*-indazole (240 mg, 81%) as a yellow solid. LC-MS: m/z $[M+H]^+$ = 226.

**158-2: 5-Bromo-4-(methoxymethyl)-2-methyl-2*H*-indazole**

5-Bromo-4-(bromomethyl)-2-methyl-2*H*-indazole (240 mg, 1.061 mmol) was added to sodium methoxide (144 mg, 2.122 mmol) and methanol (15 mL), and the mixture was stirred at room temperature for 16 hours. After the reaction was completed, the system was poured into water, extracted with ethyl acetate (100 mL × 3), dried over anhydrous sodium sulfate, subjected to thin-layer chromatography (ethyl acetate: petroleum ether = 1:5), and subjected to column chromatography (ethyl acetate: petroleum ether = 1:5) to obtain the title compound (200 mg, 76%) as a yellow solid. LC-MS: m/z $[M+1]^+$ = 257.

**158: 7-Ethyl-4-(4-fluoro-3-(4-(methoxymethyl)-2-methyl-2*H*-indazol-5-yl)phenyl)-7*H*-imidazo[4,5-*c*]pyridazine**

Following the same experimental procedure as in Example 1, 5-bromo-4-(methoxymethyl)-2-methyl-2*H*-indazole (200 mg, 0.78 mmol) and (5-(7-ethyl-7*H*-imidazo[4,5-c]pyridazin-4-yl)-2-fluorophenyl)boronic acid (223.9 mg, 0.78

mmol) were used as starting materials to obtain the title compound (114 mg, 57%) as an off-white solid.

$^1H$ NMR (400 MHz, DMSO-$d_6$) 9.56 (s, 1 H), 8.86 (s, 1 H), 8.54 - 8.44 (m, 3 H), 7.65 (d, *J* = 8.8 Hz, 1 H), 7.56 (t, *J* = 9.3 Hz, 1 H), 7.21 (d, *J* = 8.8 Hz, 1 H), 4.57 (s, 2 H), 4.51 (q, *J* = 7.3 Hz, 2 H), 4.22 (s, 3 H), 3.19 (s, 3 H), 1.55 (t, *J* = 7.3 Hz, 3 H). LC-MS: m/z $[M+1]^+$ = 418.

Example 159

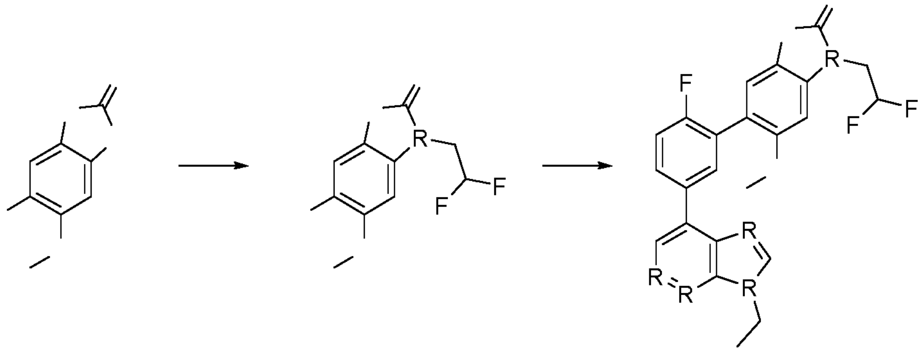

**159-1: 6-Bromo-3-(2,2-difluoroethyl)-5-methoxybenzo[*d*]oxazol-2(3*H*)-one**

6-Bromo-5-methoxybenzo[d]oxazol-2(3H)-one (100 mg, 0.41 mmol), cesium carbonate (400 mg, 1.2 mmol), and 2,2-difluoroethyl trifluoromethanesulfonate (105 mg, 0.49 mmol) were added to acetonitrile (6 mL), and the mixture was stirred at 60°C overnight. The reaction mixture was directly purified by preparative thin-layer chromatography (petroleum ether/ethyl acetate = 3/1) to obtain the title compound (60 mg, 48%). LC-MS: m/z $[M+H]^+$ = 308, 310.

**159: 3-(2,2-Difluoroethyl)-6-(5-(7-ethyl-7*H*-imidazo[4,5-*c*]pyridazin-4-yl)-2-fluorophenyl)-5-methoxybenzo[*d*]oxazol-2(3*H*)-one**

Following the same experimental procedure as in Example 1, 6-bromo-3-(2,2-difluoroethyl)-5-methoxybenzo[*d*]oxazol-2(3*H*)-one (50 mg, 0.17 mmol) and 5-(7-ethyl-7*H*-imidazo[4,5-*c*]pyridazin-4-yl)-2-fluorophenyl)boronic acid (52 mg, 0.17 mmol) were used as starting materials to obtain the title compound (25 mg, 31%).

$^1H$ NMR (400 MHz, CHLOROFORM-*d*) 9.37 (s, 1 H), 8.31 - 8.18 (m, 3 H), 7.34 (t, *J* = 9.0 Hz, 1 H), 7.28 - 7.26 (m, 1 H), 6.76 (s, 1 H), 6.30 - 5.97 (m, 1 H), 4.58 (q, *J* = 7.3 Hz, 2 H), 4.23 (dt, *J* = 3.7, 13.8 Hz, 2 H), 3.85 (s, 3 H), 1.72 - 1.68 (m, 3 H). LC-MS: m/z $[M+H]^+$ = 470.

Example 160

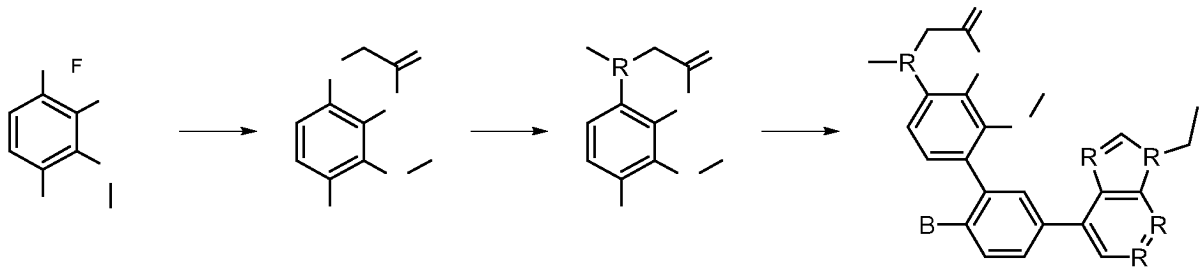

**160-1: 7-Bromo-8-methoxy-2*H*-benzo[*b*][1,4]oxazin-3(4*H*)-one**

6-Amino-3-bromo-2-methoxyphenol (100 mg, 0.46 mmol) was dissolved in 10 mL of acetonitrile, then chloroacetyl chloride (273 mg, 0.92 mmol) and potassium carbonate (127 mg, 0.92 mmol) were added thereto, and the mixture was stirred at 80°C for 2 hours. The reaction mixture was concentrated to obtain the title compound (100 mg, 84%) as a

white solid. LC-MS: m/z $[M+H]^+$ = 258.

**160-2: 7-Bromo-8-methoxy-4-methyl-2*H*-benzo[*b*][1,4]oxazin-3(4*H*)-one**

7-Bromo-8-methoxy-2H-benzo[b][1,4]oxazin-3(4*H*)-one (100 mg, 0.39 mmol) was dissolved in 10 mL of acetonitrile, then iodomethane (110 mg, 0.78 mmol) and cesium carbonate (380 mg, 1.2 mmol) were added thereto, and the mixture was stirred at room temperature overnight. The reaction mixture was filtered to remove the solid, concentrated, and purified by preparative thin-layer chromatography (petroleum ether: ethyl acetate = 3:1) to obtain the title compound (80 mg, 75%) as a yellow solid. LC-MS: m/z $[M+H]^+$ = 272.

**160: 7-(5-(7-Ethyl-7*H*-imidazo[4,5-*c*]pyridazin-4-yl)-2-fluorophenyl)-8-methoxy-4-methyl-2*H*-benzo[*b*][1,4]oxazin-3(4*H*)-one**

Following the same experimental procedure as in Example 7, 7-ethyl-4-(4-fluoro-3-(4,4,5,5-tetramethyl-1,3,2-dioxaborolan-2-yl)phenyl)-7*H*-imidazo[4,5-*c*]pyridazine (100 mg, 0.35 mmol) and 7-bromo-8-methoxy-4-methyl-2*H*-benzo[b][1,4]oxazin-3(4*H*)-one (90 mg, 0.35 mmol) were used as starting materials to obtain the title compound (45 mg, 30%) as a yellow solid.

$^1$H NMR (400 MHz, CHLOROFORM-d) δ ppm 9.38 (s, 1 H) 8.20 - 8.33 (m, 3 H) 7.34 (t, *J* = 9.05 Hz, 1 H) 7.08 (d, *J* = 8.80 Hz, 1 H) 6.85 (d, *J* = 8.80 Hz, 1 H) 4.55 - 4.77 (m, 4 H) 3.97 (s, 3 H) 2.88 (s, 3 H) 1.68 - 1.73 (m, 3 H). LC-MS: m/z $[M+H]^+$ = 434.

Example 161

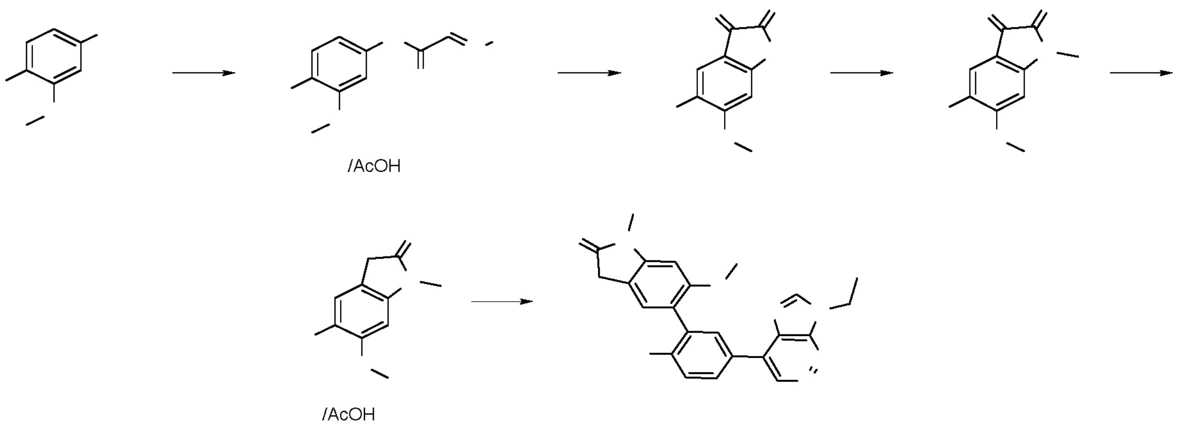


**161-1: (*E*)-*N*-(4-Bromo-3-methoxyphenyl)-2-(hydroxyimino)acetamide**

Trichloroacetaldehyde (4.4 g, 30 mmol) and sodium sulfate (35 g, 247 mmol) were dissolved in a mixture of hydrochloric acid (50 mL, 2 M) and water (75 mL), then an aqueous solution (25 mL) of hydroxylamine hydrochloride (5.2 g, 74 mmol) was added thereto, and the mixture was heated to 60°C and stirred for 30 minutes. 50 mL of 2 M hydrochloric acid solution of 4-bromo-3-methoxyaniline (CAS: 19056-40-7, 5 g, 25 mmol) was added thereto, and the mixture was heated to 90°C and stirred for 2 hours. The reaction mixture was cooled, filtered to obtain the solid, and dried to obtain the title compound (5 g, 74%) as a brown solid. LC-MS: m/z $[M+H]^+$ = 273.

**161-2: 5-Bromo-6-methoxyindoline-2,3-dione**

50 mL of concentrated sulfuric acid was heated to 60°C, and (*E*)-*N*-(4-bromo-3-methoxyphenyl)-2-(hydroxyimino)acetamide (5 g, 18 mmol) was added thereto. The mixture was heated to 90°C, stirred for 2 hours, cooled, poured into ice water, and filtered to obtain the solid to obtain the title compound (4 g, 85%) as a red solid. LC-MS: m/z $[M+H]^+$ = 256.

**161-3: 5-Bromo-1-methyl-6-methoxyindoline-2,3-dione**

5-Bromo-6-methoxyindoline-2,3-dione (400 mg, 1.56 mmol) was dissolved in 10 mL of acetonitrile, then

iodoethane (490 mg, 3.14 mmol) and cesium carbonate (1.5 g, 4.6 mmol) were added thereto, and the mixture was heated to 50°C and stirred for 2 hours. The reaction mixture was filtered to remove the solid, and concentrated to obtain the title compound (400 mg, 90%) as a yellow solid. LC-MS: m/z $[M+H]^+$ = 270.

**161-4: 5-Bromo-1-methyl-6-methoxyindol-2-one**

5-Bromo-1-methyl-6-methoxyindoline-2,3-dione (400 mg, 1.4 mmol) was dissolved in 10 mL of hydrazine hydrate, and the mixture was heated to 130°C and stirred for 3 hours. The reaction mixture was added with water, extracted with dichloromethane, and concentrated to obtain the title compound (230 mg, 60%) as a yellow solid. LC-MS: m/z $[M+H]^+$ = 256.

**161: 5-(5-(7-Ethyl-7*H*-imidazo[4,5-c]pyridazin-4-yl)-2-fluorophenyl)-6-methoxy-1-methyldihydro-2-one**

Following the same experimental procedure as in Example 7, 7-ethyl-4-(4-fluoro-3-(4,4,5,5-tetramethyl-1,3,2-dioxaborolan-2-yl)phenyl)-7*H*-imidazo[4,5-*c*]pyridazine (80 mg, 0.28 mmol) and 5-bromo-1-methyl-6-methoxyindol-2-one (72 mg, 0.28 mmol) were used as starting materials to obtain the title compound (8 mg, 7%) as a white solid.

$^1$H NMR (400 MHz, CHLOROFORM-*d*) 9.37 (s, 1 H) 8.22 - 8.33 (m, 2 H) 8.17 (d, *J* = 6.85 Hz, 1 H) 7.30 - 7.37 (m, 1 H) 7.24 (s, 1 H) 6.55 (s, 1 H) 4.59 (q, *J* = 7.34 Hz, 2 H) 3.89 (s, 3 H) 3.55 (s, 2 H) 3.29 (s, 3 H) 1.69 (t, *J* = 7.34 Hz, 3 H). LC-MS: m/z $[M+H]^+$ = 418.

Example 162 and Example 163

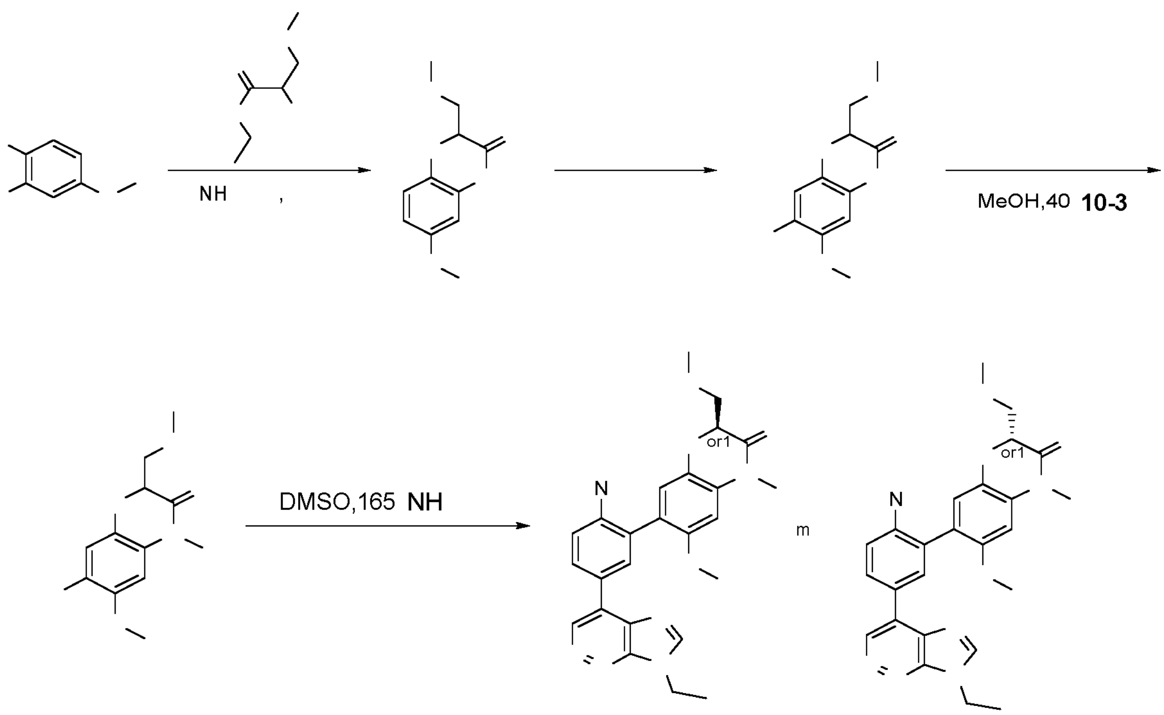


**162-1: 6-Methoxy-2-(methoxymethyl)-2*H*-benzo[*b*][1,4]oxazin-3(4*H*)-one**

2-Amino-4-methoxyphenol (1.9 g, 13.8 mmol) and ethyl 2-bromo-3-methoxypropanoate (3 g, 15.2 mmol) were dissolved in 20 mL of *N,N*-dimethylformamide under a nitrogen atmosphere, then cesium carbonate (9 g, 27.6 mmol) was added thereto, and the mixture was reacted at 95°C for 16 hours. After the reaction was completed, the reaction mixture was added to 50 mL of water, extracted with ethyl acetate (50 mL × 3), and backwashed with water (30 mL × 3). The organic phase was rotary evaporated to dryness and subjected to column chromatography (petroleum ether/ethyl acetate = 5/1) to obtain 6-methoxy-2-(methoxymethyl)-2H-benzo[b][1,4]oxazin-3(4*H*)-one (200 mg, 7%) as a white solid. LC-MS: m/z $[M+H]^+$ = 224.

**162-2: 7-Bromo-6-methoxy-2-(methoxymethyl)-2*H*-benzo[*b*][1,4]oxazin-3(4*H*)-**one

6-Methoxy-2-(methoxymethyl)-2H-benzo[*b*][1,4]oxazin-3(4*H*)-one (200 mg, 0.09 mmol) was dissolved in 1.5 mL of *N,N*-dimethylformamide under a nitrogen atmosphere, and the mixture was cooled to 0°C, at which temperature *N*-bromosuccinimide (19 mg, 0.11 mmol) was slowly added thereto. The mixture was reacted at room temperature for 2 hours, added to 20 mL of ice water, extracted with ethyl acetate (20 mL × 3), and the organic phase was rotary evaporated to dryness to obtain the title compound (195 mg, 71.8%) as a light yellow solid. LC-MS: m/z $[M+H]^+$ = 302.

**162-3: 7-Bromo-6-methoxy-2-(methoxymethyl)-4-methyl-2*H*-benzo[*b*][1,4] oxazin-3(4*H*)-one**

7-Bromo-6-methoxy-2-(methoxymethyl)-2H-benzo[b][1,4]oxazin-3(4*H*)-one (175 mg, 0.58 mmol) and potassium carbonate (166 mg, 1.2 mmol) were dissolve in methanol under a nitrogen atmosphere, then iodomethane (170 mg, 1.2 mmol) was added thereto, and the mixture was reacted at 40°C for 2 hours. After the reaction was completed, the reaction mixture was added to 20 mL of water, extracted with ethyl acetate (20 mL × 3), and the organic phase was rotary evaporated to dryness to obtain the title compound (85 mg, 47%) was a white solid. LC-MS: m/z $[M+H]^+$ = 317.

**162: *rel*-(*S*)-7-(5-(7-Ethyl-7*H*-imidazo[4,5-*c*]pyridazin-4-yl)-2-fluorophenyl)-6-methoxy-2-(methoxymethyl)-4-methyl-2*H*-benzo[*b*][1,4]oxazin-3(4*H*)-one**

**163: *rel*-(*R*)-7-(5-(7-Ethyl-7*H*-imidazo[4,5-*c*]pyridazin-4-yl)-2-fluorophenyl)-6-methoxy-2-(methoxymethyl)-4-methyl-2*H*-benzo[*b*][1,4]oxazin-3(4*H*)-one**

Following the same experimental procedure as in Example 1, 7-bromo-6-methoxy-2-(methoxymethyl)-4-methyl-2H-benzo[*b*][1,4]oxazin-3(4*H*)-one (75 mg, 0.24 mmol) and (5-(7-ethyl-7*H*-imidazo[4,5-*c*]pyridazin-4-yl)-2-fluorophenyl)boronic acid (69 mg, 0.24 mmol) were used as starting materials to obtain a mixture of the two title compounds (40 mg, 40%) as a white solid. The mixture was further subjected to chiral resolution to obtain compounds of Example 162 (first peak) and Example 163 (second peak). Resolution method: instrument: MG II preparative SFC (SFC-1); column: ChiralPak AD, 250 × 30 mm I.D., 10 μm; mobile phase: A for $CO_2$ and B for isopropanol; gradient: B 55%; flow rate: 80 mL/min; back pressure: 100 bar; column temperature: 38°C; wavelength: 220 nm.

Example 162: (15 mg) white solid. $^1H$ NMR (400 MHz, $CDCl_3$) δ 9.38 (s, 1 H), 8.30 (s, 1 H), 8.29 - 8.24 (m, 1H), 8.21 (dd, *J* = 6.9, 2.3 Hz, 1 H), 7.33 (t, *J* = 9.0 Hz, 1 H), 7.12 (s, 1 H), 6.61 (s, 1 H), 4.75 (dd, *J* = 5.4, 3.4 Hz, 1 H), 4.58 (q, *J* = 7.3 Hz, 2 H), 3.99 - 3.90 (m, 2 H), 3.83 (s, 3 H), 3.46 (s, 3 H), 3.44 (s, 3 H), 1.70 (t, *J* = 7.3 Hz, 3 H). LC-MS: m/z $[M+H]^+$ = 478.

Example 163: (16 mg) white solid. $^1H$ NMR (400 MHz, $CDCl_3$) 9.38 (s, 1 H), 8.30 (s, 1 H), 8.29 - 8.24 (m, 1 H), 8.21 (dd, *J* = 6.9, 2.3 Hz, 1 H), 7.33 (t, *J* = 9.0 Hz, 1 H), 7.12 (s, 1 H), 6.61 (s, 1 H), 4.75 (dd, *J* = 5.4, 3.4 Hz, 1 H), 4.58 (q, *J* = 7.3 Hz, 2 H), 3.99 - 3.90 (m, 2 H), 3.83 (s, 3 H), 3.46 (s, 3 H), 3.44 (s, 3 H), 1.70 (t, *J* = 7.3 Hz, 3 H). LC-MS: m/z $[M+H]^+$ = 478.

Example 164

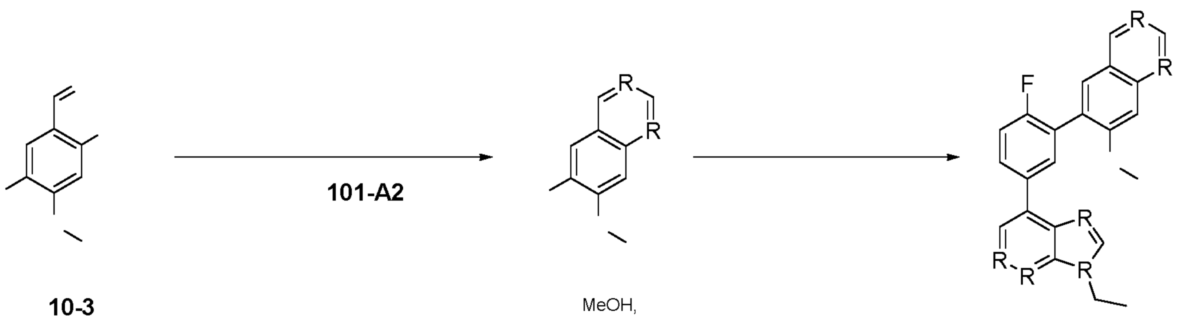


10-3

**164-1: 6-Bromo-7-methoxyquinazoline**

2-Amino-5-bromo-4-methoxybenzaldehyde (80 mg, 0.35 mmol), formimidamide acetate (305 mg, 3.5 mmol), and *N*,*N*-diisopropylethylamine (677 mg, 5.25 mmol) were dissolved in NMP (3 mL) at 25°C, and the mixture was reacted under microwave irradiation at 125°C for 2 hours. The reaction mixture was poured into water, extracted with ethyl acetate, rotary evaporated to dryness, purified by reversed-phase HPLC, and lyophilized to obtain 6-bromo-7-methoxyquinazoline (80 mg, 95%) as a gray solid. LC-MS: m/z $[M+H]^+$ = 240.

**164: 6-(5-(7-Ethyl-7*H*-imidazo[4,5-*c*]pyridazin-4-yl)-2-fluorophenyl)-7-methoxyquinazoline**

Following the same experimental procedure as in Example 1, 6-bromo-7-methoxyquinazoline (70 mg, 0.29 mmol) and (5-(7-ethyl-7*H*-imidazo[4,5-*c*]pyridazin-4-yl)-2-fluorophenyl)boronic acid (83.7 mg, 0.29 mmol) were used as starting materials to obtain the title compound (34 mg, 29%) as an off-white solid.

$^1$H NMR (400 MHz, DMSO-$d_6$) 9.60 (s, 1 H), 9.52 (d, *J* = 5.7 Hz, 1 H), 9.28 (s, 1 H), 8.87 (s, 1 H), 8.64 - 8.55 (m, 2 H), 8.22 (s, 1 H), 7.63 - 7.53 (m, 2 H), 4.52 (q, *J* = 7.3 Hz, 2 H), 4.00 (s, 3 H), 1.56 (t, *J* = 7.3 Hz, 3 H). LC-MS: m/z [M+H]$^+$ = 401.

Example 165

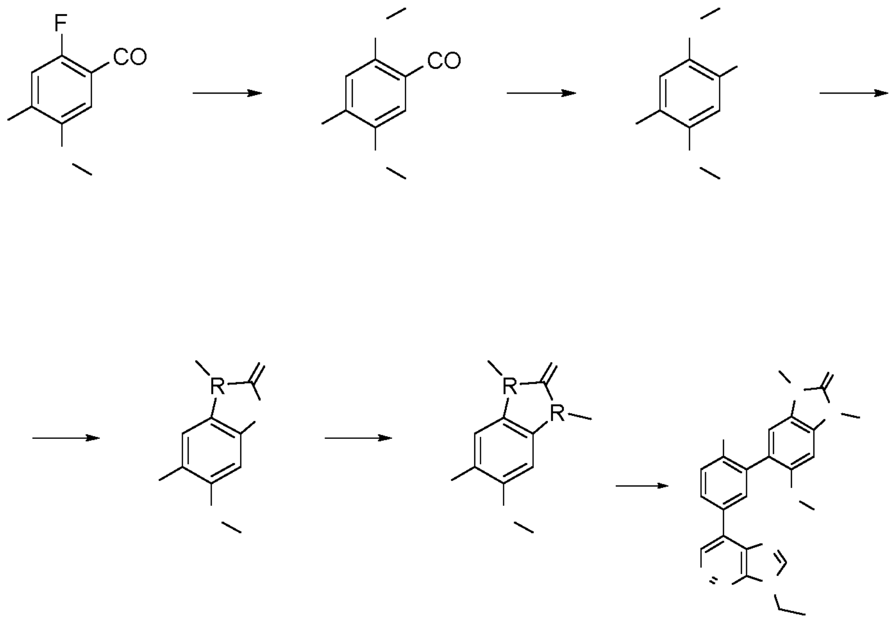

**165-1: 5-Chloro-4-methoxy-*N*-methyl-2-nitroaniline**

1-Chloro-5-fluoro-2-methoxy-4-nitrobenzene (1800 mg, 8.76 mmol) was added to methylamine solution (10 mL), and the mixture was stirred at 80°C for 5 minutes. The reaction mixture was concentrated to dryness to obtain a crude product (2000 mg), which was directly used in the next step. LC-MS: m/z [M+H]$^+$ = 217, 219.

**165-2: 5-Chloro-4-methoxy-*N*$^1$-methylbenzene-1,2-diamine**

5-Chloro-4-methoxy-*N*-methyl-2-nitroaniline (2000 mg, 9.23 mmol) and Raney nickel (2709 mg, 46.15 mmol) were added to methanol (10 mL). After hydrogen exchange, the mixture was stirred at room temperature for 2 hours under a hydrogen atmosphere. The reaction mixture was filtered, and the filtrate was concentrated to dryness to obtain a crude product (1500 mg), which was directly used in the next step. LC-MS: m/z [M+H]$^+$ = 187.

**165-3: 6-Chloro-5-methoxy-1-methyl-1,3-dihydro-2*H*-benzo[*d*]imidazol-2-one**

5-Chloro-4-methoxy-*N*$^1$-methylbenzene-1,2-diamine (1500 mg, 8.04 mmol) and triphosgene (2386 mg, 8.04 mmol) were added to tetrahydrofuran (20 mL), and the mixture was stirred at room temperature for 2 hours. The reaction mixture was added with saturated sodium bicarbonate aqueous solution to adjust the pH to alkaline, filtered, and the filtrate was concentrated to dryness to obtain a crude product (1500 mg), which was directly used in the next step. LC-MS: m/z [M+H]$^+$ = 213.

**165-4: 5-Chloro-6-methoxy-1,3-dimethyl-1,3-dihydro-2*H*-benzo[*d*]imidazol-2-**one

6-Chloro-5-methoxy-1-methyl-1,3-dihydro-2*H*-benzo[d]imidazol-2-one (500 mg, 2.35 mmol), iodomethane (672

mg, 4.70 mmol), and cesium carbonate (2297 mg, 7.05 mmol) were sequentially added to acetonitrile (10 mL), and the mixture was stirred at 80°C for 1 hour. The reaction mixture was filtered, and the filtrate was concentrated to dryness and separated by thin-layer chromatography (dichloromethane/methanol = 30/1) to obtain the title compound (230 mg, 43%). LC-MS: m/z $[M+H]^+$ = 227.

**165: 5-(5-(7-Ethyl-7*H*-imidazo[4,5-*c*]pyridazin-4-yl)-2-fluorophenyl)-6-methoxy-1,3-dimethyl-1,3-dihydro-2*H*-benzo[*d*]imidazol-2-one**

Following the same experimental procedure as in Example 4, 5-chloro-6-methoxy-1,3-dimethyl-1,3-dihydro-2*H*-benzo[*d*]imidazol-2-one (200 mg, 0.88 mmol) and 5-(7-ethyl-7*H*-imidazo[4,5-*c*]pyridazin-4-yl)-2-fluorophenyl)boronic acid (252 mg, 0.88 mmol) were used as starting materials to obtain the title compound (90 mg, 24%).

$^1H$ NMR (400 MHz, CHLOROFORM-*d*) δ = 9.38 (s, 1 H), 8.30 - 8.23 (m, 2 H), 8.20 (d, *J* = 6.8 Hz, 1 H), 7.34 (t, *J* = 9.0 Hz, 1 H), 6.97 (s, 1 H), 6.70 (s, 1 H), 4.58 (q, *J* = 7.3 Hz, 2 H), 3.86 (s, 3 H), 3.47 (s, 3 H), 3.42 (s, 3 H), 1.67 (br. s., 3 H). LC-MS: m/z $[M+H]^+$ = 433.

Example 166

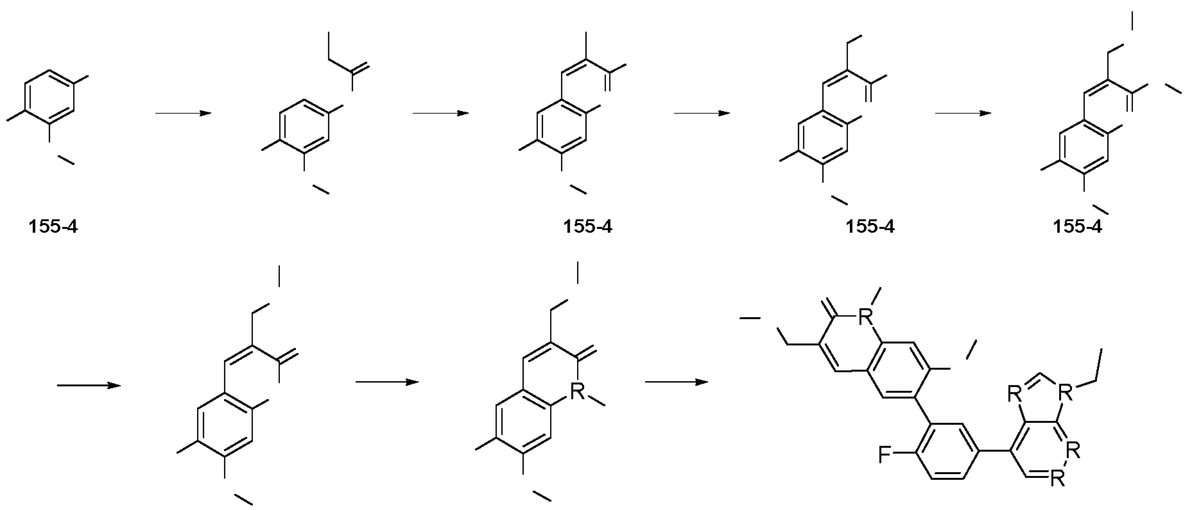

155-4 155-4 155-4 155-4

**166-1: *N*-(4-Bromo-3-methoxyphenyl)propionamide**

Acetonitrile (1.8 g), 4-bromo-methoxyaniline (2.25 g, 11 mmol), and water (9.0 g) were added to a reactor, and the mixture was heated to approximately 60°C. Propionic anhydride (2.17 g) was slowly added thereto. The reaction mixture was stirred at 60°C for 1 hour. After the reaction was completed, water was added to precipitate the solid. The crystalline compound was collected at 20°C, washed with water, and finally dried under vacuum to obtain *N*-(4-bromo-3-methoxyphenyl)propionamide (2.6 g, 92%) as a white solid. LC-MS: m/z $[M+H]^+$ = 258.0.

**166-2: 6-Bromo-2-chloro-7-methoxy-3-methylquinoline**

*N*-(4-Bromo-3-methoxyphenyl)propionamide (2.6 g, 10 mmol), dimethylformamide (1.02 g), and toluene (0.97 g) were added to a syringe. The resulting mixture was slowly added to another reactor containing phosphorus oxychloride (5.31 g, 34.7 mmol) and toluene (1.13 g) while maintaining the temperature between 20 and 30°C. The reaction mixture was then stirred at approximately 30°C for 1 hour. The mixture was then heated to a reaction temperature of approximately 80°C and stirred for approximately 1 hour. The mixture was then cooled to room temperature. The reaction mixture was poured into ice water (50 mL), extracted with ethyl acetate (60 mL × 3), concentrated, and purified by column chromatography (petroleum ether: ethyl acetate = 6:1) to obtain 6-bromo-2-chloro-7-methoxy-3-methylquinoline (2.3 g, yield: 53%) as a white solid. LC-MS: m/z $[M+H]^+$ = 286.0.

**166-3: 6-Bromo-3-(bromomethyl)-2-chloro-7-methoxyquinoline**

6-Bromo-2-chloro-7-methoxy-3-methylquinoline (2.3 g, 8.04 mmol), N-bromosuccinimide (1.43 g, 8.04 mmol),

benzoyl peroxide (389 mg, 1.2 mmol), and carbon tetrachloride (50 mL) were added to a reaction flask, and the mixture was reacted at 90°C for 16 hours. The reaction mixture was directly rotary evaporated to dryness and purified by normal-phase column chromatography (petroleum ether: ethyl acetate = 19:1) to obtain 3-(4-chloro-3-(methoxymethyl)phenyl)-6-(methoxymethyl)pyridazine (1.5 g, 51.0%) as a black oily liquid. LC-MS: m/z $[M+H]^+$ = 366.0.

**166-4: 6-Bromo-2,7-dimethoxy-3-(methoxymethyl)quinoline**

3-(4-Chloro-3-(methoxymethyl)phenyl)-6-(methoxymethyl)pyridazine (300 mg, 0.82 mmol) was dissolved in tetrahydrofuran (20 mL), then a solution of sodium methoxide in methanol (1.6 mL, 80 mmol) was added thereto, and the mixture was stirred at room temperature for 16 hours. After the reaction was completed, the reaction mixture was directly concentrated to obtain 6-bromo-2,7-dimethoxy-3-(methoxymethyl)quinoline (250 mg, 97.8%) as a white solid. LC-MS: m/z $[M+H]^+$ = 312.0.

**166-5: 6-Bromo-7-methoxy-3-(methoxymethyl)quinolin-2(1*H*)-one**

6-Bromo-2,7-dimethoxy-3-(methoxymethyl)quinoline (250 mg, 0.8 mmol) and a solution of hydrochloric acid in 1,4-dioxane (20 mL, 4 mol/L) were added to a reaction flask. The resulting mixture was refluxed at 125°C for 2 hours to complete the reaction, and concentrated to obtain 6-bromo-7-methoxy-3-(methoxymethyl)quinolin-2(1H)-one (200 mg, 83.8%) as a white solid. LC-MS: m/z $[M+H]^+$ = 298.0.

**166-6: 6-Bromo-7-methoxy-3-(methoxymethyl)-1-methylquinolin-2(1*H*)-one**

6-Bromo-7-methoxy-3-(methoxymethyl)quinolin-2(1*H*)-one (200 mg, 0.67 mmol) was added to tetrahydrofuran (10 mL), then NaH (40 mg, 1.0 mmol) was added thereto at 0°C, and the mixture was stirred for 30 minutes. Iodomethane (284 mg, 2.0 mmol) was then added thereto, and the mixture was stirred at room temperature for 2 hours. The reaction mixture was poured into ice water (20 mL), extracted with ethyl acetate (60 mL × 3), concentrated, and purified by column chromatography (petroleum ether: ethyl acetate = 7:1) to obtain 6-bromo-7-methoxy-3-(methoxymethyl)-1-methylquinolin-2(1*H*)-one (160 mg, yield: 76%) as a white solid. LC-MS: m/z $[M+H]^+$ = 312.0.

**166: 6-(5-(7-Ethyl-7H-imidazo[4,5-*c*]pyridazin-4-yl)-2-fluorophenyl)-7-methoxy-3-(methoxymethyl)-1-methylquinolin-2(1*H*)-one**

Following the same experimental procedure as in Example 1, 6-bromo-7-methoxy-3-(methoxymethyl)-1-methylquinolin-2(1*H*)-one (63 mg, 0.2 mmol) and (5-(7-ethyl-7*H*-imidazo[4,5-*c*]pyridazin-4-yl)-2-fluorophenyl)boronic acid (58 mg, 0.2 mmol) were used as starting materials to obtain the title compound (65 mg, 68%) as an off-white solid.

$^1$H NMR (400 MHz, $CDCl_3$) 9.44 (s, 1 H), 8.40 (s, 1 H), 8.31 (dd, *J* = 11.0, 5.3 Hz, 2 H), 7.77 (s, 1 H), 7.59 (s, 1 H), 7.38 (t, *J* = 8.9 Hz, 1 H), 6.87 (s, 1 H), 4.60 (d, *J* = 7.3 Hz, 2 H), 4.51 (s, 2 H), 3.96 (s, 3 H), 3.80 (s, 3 H), 3.53 (s, 3 H), 1.70 (d, *J* = 7.3 Hz, 3 H). LC-MS: m/z $[M+H]^+$ = 474.0.

Example 167

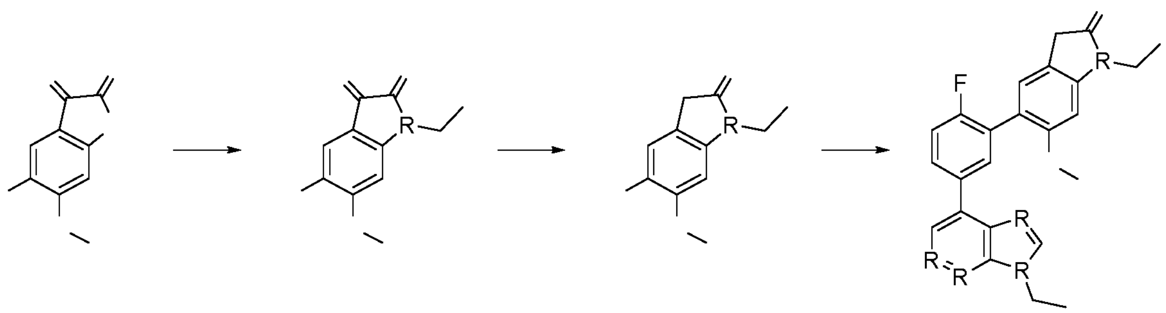

**167-1: 5-Bromo-1-ethyl-6-methoxyindoline-2,3-dione**

5-Bromo-6-methoxyindoline-2,3-dione (400 mg, 1.56 mmol) was dissolved in 10 mL of acetonitrile, then iodoethane (490 mg, 3.14 mmol) and cesium carbonate (1.5 g, 4.6 mmol) were added thereto, and the mixture was heated to 50°C and stirred for 2 hours. The reaction mixture was filtered to remove the solid, and concentrated to obtain the title compound (400 mg, 90%) as a yellow solid. LC-MS: m/z $[M+H]^+$ = 284.

**167-2: 5-Bromo-1-ethyl-6-methoxyindol-2-one**

5-Bromo-1-ethyl-6-methoxyindoline-2,3-dione (400 mg, 1.4 mmol) was dissolved in 10 mL of hydrazine hydrate, and the mixture was heated to 130°C and stirred for 3 hours. The reaction mixture was cooled, diluted with water, extracted with dichloromethane, and concentrated to obtain the title compound (230 mg, 60%) as a yellow solid. LC-MS: m/z $[M+H]^+$ = 270.

**167: 1-Ethyl-5-(5-(7-ethyl-7*H*-imidazo[4,5-*c*]pyridazin-4-yl)-2-fluorophenyl)-6-methoxyindol-2-one**

Following the same experimental procedure as in Example 7, 7-ethyl-4-(4-fluoro-3-*(4,4,5,5-tetramethyl-1,3,2-dioxaborolan-2-yl)phenyl)-7H-imidazo[4,5-c]pyridazine* (240 mg, 0.84 mmol) and 5-bromo-1-ethyl-6-methoxyindol-2-one (230 mg, 0.84 mmol) were used as starting materials to obtain the title compound (57 mg, 16%) as a white solid.

$^1$H NMR (400 MHz, CHLOROFORM-*d*) 9.36 (s, 1 H) 8.21 - 8.34 (m, 2 H) 8.18 (d, *J* = 6.85 Hz, 1 H) 7.33 (t, *J* = 9.05 Hz, 1 H) 7.24 (s, 1 H) 6.56 (s, 1 H) 4.58 (q, *J* = 7.34 Hz, 2 H) 3.74 - 3.92 (m, 5 H) 3.53 (s, 2 H) 1.69 (t, *J* = 7.34 Hz, 3 H) 1.33 (t, *J* = 7.09 Hz, 3 H). LC-MS: m/z $[M+H]^+$ = 432.

Example 168

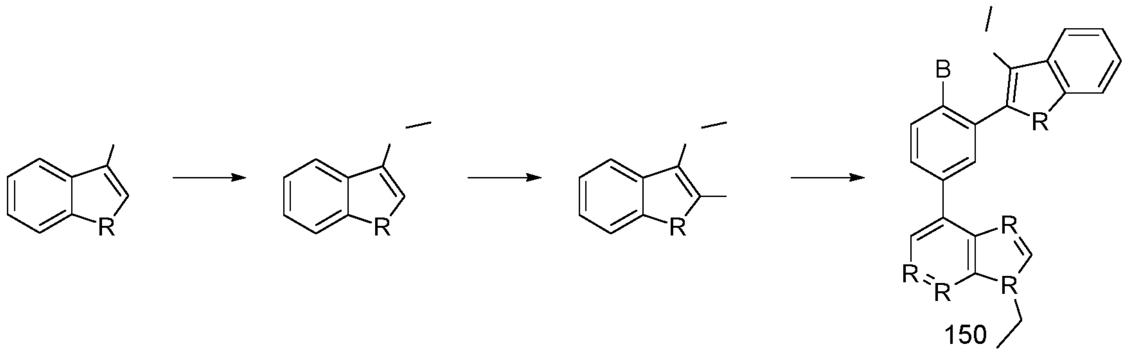

150

**168-1: 3-Methoxybenzo[b]thiophene**

3-Bromobenzo[b]thiophene (500 mg, 2.35 mmol), sodium methoxide (1267 mg, 23.5 mmol), copper oxide (93 mg, 1.18 mmol), cuprous iodide (22 mg, 0.12 mmol), and potassium iodide (19 mg, 0.12 mmol) were added to *N*,*N*-dimethylformamide (5 mL) and methanol (5 mL), and the mixture was reacted in a sealed tube at 120°C overnight. The reaction mixture was added with water (30 mL) and extracted with ethyl acetate. The organic phases were combined, washed with saturated sodium chloride aqueous solution, dried over anhydrous sodium sulfate, concentrated, and subjected to column chromatography (pure petroleum ether) to obtain the title compound (308 mg, 80%) as a colorless oily liquid. LC-MS: m/z $[M+H]^+$ = 165.

**168-2: 2-Bromo-3-methoxybenzo[*b*]thiophene**

3-Methoxybenzo[*b*]thiophene (308 mg, 1.88 mmol) and *N*-bromosuccinimide (368 mg, 2.07 mmol) were added to dichloromethane (5 mL), and the mixture was stirred at 25°C for 30 minutes. The reaction mixture was subjected to suction filtration, concentrated, and subjected to preparative thin-layer chromatography (pure petroleum ether) to obtain the title compound (311 mg, 68%) as a pink oily liquid. $^1$H NMR (400 MHz, CHLOROFORM-*d*) d = 7.72 (d, *J* = 7.3 Hz, 1 H), 7.66 (d, *J* = 7.8 Hz, 1 H), 7.39 - 7.31 (m, 2 H), 4.05 (s, 3 H).

**168: 7-Ethyl-4-(4-fluoro-3-(3-methoxybenzo[*b*]thiophen-2-yl)phenyl)-7*H*-imidazo[4,5-*c*]pyridazine**

7-Ethyl-4-(4-fluoro-3-(4,4,5,5-tetramethyl-1,3,2-dioxaborolan-2-yl)phenyl)-7*H*-imidazo[4,5-*c*]pyridazine (59 mg, 0.16 mmol), 2-bromo-3-methoxybenzo[*b*]thiophene (97 mg, 0.4 mmol), cesium carbonate (104 mg, 0.32 mmol), and tetrakis(triphenylphosphine)palladium (9 mg, 0.008 mmol) were added to 1,4-dioxane (2 mL) and water (0.2 mL), and the mixture was reacted at 100°C for 4 hours under an argon atmosphere. The reaction mixture was subjected to suction filtration, concentrated, and subjected to preparative thin-layer chromatography (petroleum ether/dichloromethane/-

methanol = 20/30/1) to obtain the title compound (6 mg, 9%) as a light yellow solid.

$^1H$ NMR (400 MHz, CHLOROFORM-d) 9.43 (br. s., 1 H), 8.68 (d, $J$ = 5.4 Hz, 1 H), 8.31 (br. s., 2 H), 7.90 - 7.76 (m, 2 H), 7.41 (br. s., 3 H), 4.60 (d, $J$ = 7.3 Hz, 2 H), 3.93 (br. s., 3 H), 1.70 (t, $J$ = 6.4 Hz, 3 H). LC-MS: m/z $[M+H]^+$ = 405.

## Experimental example

### Cell line construction and subculture

α subunit, β subunit, and γ subunit are essential for the formation of a complete and functional $GABA_A$ receptor. In this example of the present disclosure, HEK293 cells stably expressing the human α1-$GABA_A$ receptor (α1-$GABA_A$R) and α2-$GABA_A$ receptor (α2-$GABA_A$R) were constructed and separately selected using the lipofection method (Felgner, P.L., et al. Proceedings of the National Academy of Sciences, 1987, 84: 7413-7417). The α1-$GABA_A$R-HEK293 cell model simultaneously expressed the α1 subunit (for protein sequence, see GenBank accession No.: NM_000806.5), the β3 subunit (for protein sequence, see GenBank accession No.: NM_000814.5), and the γ2 subunit (for protein sequence, see GenBank accession No.: NM_000816.3). The α2-$GABA_A$R-HEK293 cell model simultaneously expressed the α2 subunit (for protein sequence, see GenBank accession No.: NM_000807.4), the β3 subunit (for protein sequence, see GenBank accession No.: NM_000814.5), and the γ2 subunit (for protein sequence, see GenBank accession No.: NM_000816.3).

The above cell lines were subcultured. The α2-$GABA_A$R-HEK293 cells were expanded for testing the affinity of compounds to the benzodiazepine (BZD) site of the $GABA_A$ receptor. During the passage process, some of the α1-$GABA_A$R-HEK293 cells and α2-$GABA_A$R-HEK293 cells were suspended and plated on a slide pre-treated with Poly-D-Lysine for electrophysiological testing (method referenced from patent CN107344936A, paragraph 0586).

### Affinity activity of compounds of the present disclosure for α2-$GABA_A$ receptors

The affinity of the compounds for the α2-$GABA_A$ receptor was determined by competing for the binding of $^3H$-flunitrazepam (isotope $^3H$-labelled flunitrazepam) to the BZD site on the membrane protein of HEK293 cells stably expressing the human α2-$GABA_A$R.

Membrane preparation: Cells were suspended in 50 mM Tris-HCl buffer (pH = 7.4), homogenized 10 times for 20 seconds with a homogenizer on ice, and centrifuged at 1000 g for 10 minutes at 4°C. The supernatant was taken, and the above steps were repeated. The supernatant was centrifuged at 4°C (33800 g; Thermo, rotor: A27-8 × 50) for 60 minutes. The precipitate was resuspended in Tris buffer (50 mM Tris-HCl, 10 mM $MgCl_2$, 0.5 mM EDTA, and 10% glycerol). The protein content was determined (BCA (bicinchoninic acid) protein quantification assay based on copper ion reduction, with a BCA kit purchased from Pierce (Annoron (Beijing) Biotechnology Co., Ltd.)), and 1 mL aliquots were prepared and stored at -80°C.

Radioligand competitive binding assay: This assay was performed in a 200 μL system (96-well plate) containing 100 μL of cell membrane. The concentration of $^3H$-flunitrazepam was 1 nM, and the concentrations of the test compounds were in the range of $1 \times 10^{-5}$ to $1 \times 10^{-6}$ M. Flumazenil was used as a control. 1 μL of 2 mM flumazenil (final concentration of 10 μM) was added to each low signal control (low control, LC) well, and 1 μL of dimethyl sulfoxide was added to each high signal control (high control, HC) well. The final concentration of target membrane protein was 5 μg/well. All sample stock solutions of test compounds were 10 mM dimethyl sulfoxide solutions. All the samples were diluted to a concentration of 0.2 mM with dimethyl sulfoxide, and then subjected to a 4-fold serial dilution for a total of 8 working concentration gradients. The 96-well plate was sealed with a sealing membrane and then incubated on a shaker at room temperature for 1 hour. Meanwhile, a GF/C filter plate was soaked in plate soaking buffer (0.3% PEI (polyethyleneimine, purchased from Sigma-Aldrich, model: P314), stored at 4°C) for at least 0.5 hours. After the binding incubation was completed, the cells were harvested onto the GF/C filter plate using a cell harvester and washed four times with plate washing buffer (50 mM Tris-HCl, pH 7.4, stored at 4°C). After drying in an oven at 50°C for 1 hour, the dried GF/C filter plate was bottom-sealed. The residual radioactivity on the filter membrane was detected by liquid scintillation counting. 50 μL/well of scintillation fluid was added, and the plate was sealed. Readings were taken using $Microbeta^2$ (microplate counter, purchased from PerkinElmer, model: CNLL0153). The inhibitory activity of the test samples on the binding of $^3H$-flunitrazepam to the $GABA_A$ receptor membrane protein was calculated. The $IC_{50}$ values of the test samples were calculated by dose-response curve fitting (GraphPad Prism 5 software), and the $K_i$ values of the samples were calculated from the $IC_{50}$ values in order to assess the binding capacity of the compounds to the BZD site of the α2-$GABA_A$ receptor.

The representative assay results obtained by the above method for determining the binding affinity of the compounds to the BZD site on the membrane protein of HEK293 cells expressing the human α2-$GABA_A$R are shown in Table 3.

**Functional activity of compounds of the present disclosure on different subtypes of $GABA_A$ receptors**

The positive regulatory activity of the test drugs on the α1-$GABA_A$ receptor and the α2-$GABA_A$ receptor was detected by the electrophysiological method. The specific method was as follows:

Compound concentration setting: The final concentration of the compounds used for screening was 100 nM. For cell lines expressing the α1-$GABA_A$ receptor, the concentration of GABA was 0.05 to 0.07 μM (approximately $EC_{2-4}$). For cell lines expressing the α2-$GABA_A$ receptor, the concentration of GABA was 0.10 to 0.11 μM (approximately $EC_{7-8}$). Electrophysiological testing was performed using the whole-cell patch-clamp technique, which can refer to the method reported in the literature (Nickolls, S.A., et al. British Journal of Pharmacology, 2018, 175: 708-725). The composition of the extracellular solution (ECS) for electrophysiology was as follows: 150 mM NaCl, 5 mM KCl, 2.5 mM $CaCl_2$, 1 mM $MgCl_2$, 10 mM HEPES, and 10 mM glucose (pH 7.4). The formulation of the intracellular solution (ICS) for electrophysiology was as follows: 140 mM CsCl, 11 mM EGTA, 10 mM HEPES, 2 mM $CaCl_2$, 1 mM $MgCl_2$, 4 mM MgATP, and 2 mM TEA (pH 7.3). GABA (γ-aminobutyric acid) powder was prepared into a mother liquor with pure water, which was then diluted in ECS. The compound was first prepared into a 4 mM mother liquor with dimethyl sulfoxide, which was then gradually diluted in the corresponding concentration of GABA-ECS. The solutions were freshly prepared prior to electrophysiological testing.

Electrophysiological signal acquisition was performed using an EPC 10 amplifier and PatchMaster software (HEKA) or an Axon 700B amplifier and Clampex software (AXON). The recording electrode was made through a drawing process using borosilicate glass, with an electrode resistance of 4 to 6 MΩ. Extracellular administration was performed using the ALA-VC-8PG™ system. A single, independently growing cell was selected. After the glass electrode formed a good seal with the cell, the membrane was ruptured to form the whole-cell configuration. The cell membrane potential was clamped at -60 mV and recorded in Gap-free mode. During the test, the extracellular solution was first applied extracellularly for approximately 20 seconds. After the baseline ($I_{prebaseline}$) stabilized, the extracellular solution was switched to GABA-ECS. At this point, the current induced by GABA ($I_{gaba}$) could be detected. After approximately 10 to 20 seconds, when the current stabilized, the extracellular solution was switched to a mixture of the compound and GABA-ECS. At this point, the current induced by both the compound and GABA ($I_{treatment}$) could be detected. Finally, the solution was switched to the extracellular solution, and the test was terminated after recording for 20 to 40 seconds. Only cells with a stable baseline, a control current magnitude ($I_{gaba}$ - $I_{prebaseline}$) with an absolute value greater than 40 pA, and a relatively stable current within 10 to 20 seconds were used for compound testing.

The experimental results for the positive allosteric modulatory activity of α1-$GABA_A$R and α2-$GABA_A$R were analyzed using PatchMaster v2x90.1 or PatchMaster v2x90.3 software (EPC 10 amplifier) and Clampex10.6 software (Axon 700B amplifier). The current values at each stage were calculated based on the average value of the current after stabilization under the corresponding conditions. We defined the control current as $I_{gaba}$ - $I_{prebaseline}$ and the current after compound treatment as $I_{treatment}$ - $I_{prebaseline}$. The allosteric modulatory activity of the compounds was expressed as a percentage and calculated according to the following formula: functional activity = $[(I_{treatment} - I_{gaba})/(I_{gaba} - I_{prebaseline})] \times 100\%$. If the value is negative, it indicates that the test compound has a reverse allosteric modulatory effect on the GABA receptor. If the value is positive, it indicates that the test compound has a positive allosteric modulatory effect on the GABA receptor. To facilitate comparison, the positive compound (CVL-865) in Example 4 of WO2014091368A1 was used as a reference for normalization, that is, positive allosteric modulatory activity = [functional activity of the compound/functional activity of the positive compound] × 100%. The test results for the positive allosteric modulatory activity of the compounds on the α1-$GABA_A$ receptor and the α2-$GABA_A$ receptor are shown in Table 3.

***In vitro* micronucleus assay**

**1. Guidelines for genotoxicity studies**

The experimental design is based on the experimental purpose and follows the following experimental design guidelines:

(1) OECD Guidelines for the Testing of Chemicals 487, *In Vitro* Mammalian Cell Micronucleus Test, revised in 2016;
(2) ICH S2 (R1): Guideline on Genotoxicity Testing and Data Interpretation for Pharmaceuticals Intended for Human Use, validated in November 2011.

**2. Materials and methods**

Experimental system and selection rationale: Chinese hamster ovary cells (CHO-WBL cells) were used as an experimental system in this experiment. The cell line has a karyotype containing 21 chromosomes and a growth cycle of 12 to 13 hours. CHO-WBL cells were originally derived from Dr. S. Wolff at the University of California in San Francisco, USA, then cloned in the laboratory of Dr. A. Bloom at Columbia University in New York, and cloned again by Dr. S. Galloway at

Litton Bionetics Laboratory in Maryland. The cell stock solution was stored in liquid nitrogen. The karyotype of each batch of cell stock solution was identified, and tests confirmed that it was free from mycoplasma contamination. The number of passages after cell cloning will not exceed 15. Chinese hamster ovary cells were chosen because the cell line is recommended for use in OECD Guidelines for the Testing of Chemicals 487 and has been proven to be sensitive to a variety of clastogens/aneugens (Marilyn J. *et al.,* 2006).

Medium and cell growth conditions: Chinese hamster ovary cells were cultured in McCoy's 5A complete medium (McCoy's 5A complete medium supplemented with 10 vt% fetal bovine serum, 2 mM GlutaMAX™ medium, 100 units/mL penicillin, and 100 μg/mL streptomycin), and grown in a standard environment (a high-humidity incubator with a temperature of 37°C and a $CO_2$ concentration of 5%). The day before administration, CHO-WBL cells in exponential growth phase were inoculated onto an eight-well cell culture slide at a density of $8 \times 10^3$ cells/400 μL McCoy's 5A complete medium/well.

Metabolic activation system: The *in vitro* metabolic activation system (Maron and Ames, 1983) contains a rat liver microsomal homogenate induced by β-naphthoflavone and phenobarbital (referred to as S9) and an NADPH-generating system (*i.e.*, NADP disodium and isocitric acid trisodium). S9 was purchased from CHI Scientific, Inc. and stored in a -75°C refrigerator prior to use. Preparation method for S9 homogenate: Male Sprague Dawley rats were intraperitoneally injected with a single dose of β-naphthoflavone and phenobarbital, followed by material preparation.

Preparation of S9 metabolic activation system: NADP disodium salt and isocitric acid trisodium salt were first dissolved in serum-free medium to prepare a core solution, which was then filtered through a 0.22 μm filter to sterilize. Immediately before use, the S9 homogenate was thawed, and the components of the S9 metabolic activation system were mixed in the proportions shown in Table 2 below.

Table 2 Components and proportions of S9 metabolic activation system

| Component | Final concentration in exposure medium |
|---|---|
| NADP disodium salt | 0.8 mg/mL |
| Isocitric acid trisodium salt | 1.5 mg/mL |
| S9 homogenate | 10 μL/mL |
| Note: The final concentration of S9 homogenate in exposure medium was 1% (v/v). | |

Dose exploration experiment: In the dose exploration experiment, 8 administration concentrations were established in each administration treatment series. For each concentration of the test substance, 1 well of cells was set up. According to the ICH S2 (R1) guidelines, when not limited by the solubility of the test substance in the solvent/medium or the cytotoxicity of the test substance, the recommended maximum concentration is 1 mM or 0.5 mg/mL, whichever is lower. However, when limited by solubility, the test maximum concentration is the minimum concentration at which a small amount of precipitate is visible in the medium under the microscope. To ensure that the upper limit of solubility in the medium can be reached, up to 4 concentrations with visible precipitate can be tested.

Administration treatment: In the 3-hour administration series with S9 metabolic activation, the medium was aspirated from each well, and with reference to the ICH S2 (R1) guidelines, serum-free McCoy's 5A/S9 mixture medium containing appropriate concentrations of test substance/control was added.

In the 3-hour administration series without metabolic activation, the medium was aspirated from each well, and McCoy's 5A complete medium containing appropriate concentrations of test substance/control was added.

Cells were incubated for 3 hours under standard conditions. After the 3-hour incubation, the old medium was aspirated from each well and washed twice with McCoy's 5A complete medium. McCoy's 5A complete medium containing cytochalasin B (final concentration of approximately 3 μg/mL in medium) was added per well, and then the cells were incubated for 21 hours until harvest.

For the 24-hour administration series without metabolic activation, the treatment was similar to that of the 3-hour administration series without metabolic activation, except that the cells were incubated with the test substance/control in the complete medium containing cytochalasin B (final concentration of approximately 3 μg/mL in medium) for 24 hours (± 30 minutes).

Slide preparation: At the time of harvest, the medium from the eight-well slide was poured off and blotted dry with absorbent paper. 75 mM KCl hypotonic solution was added per well. The cells were then fixed for 5 minutes with two consecutive changes of fixative solution (anhydrous methanol: glacial acetic acid, 3:1 v/v) and air-dried. The slide was stained with acridine orange and air-dried in the dark.

Cytotoxicity counting: The staining presented was examined under a low-power microscope to assess the analyzability of all wells on each slide (sufficient number of cells). At least 500 cells per well were analyzed under a fluorescence microscope. The numbers of mononuclear, binuclear, and multinuclear cells were counted separately to

calculate the cytokinesis-block proliferation index (CBPI).

CBPI was used to assess the cytotoxicity of the test substance. The calculation formula for CBPI is as follows:

$$CBPI = \frac{\text{(Number of mononuclear cells+(2×number of binuclear cells)+(3×number of multinuclear cells)}}{\text{Total number of cells}}$$

$$\text{\% cytotoxicity} = 100 - 100\ \{(CBPIT - 1)/(CBPIC - 1)\};$$

where T = test substance treatment culture group and C = vehicle control culture group.

When CBPI is 1, it equals 100% cytotoxicity.

Main micronucleus assay: In the main micronucleus assay, CHO-WBL cells were exposed to 3 to 8 concentrations of the test substance, while a positive control group (cyclophosphamide monohydrate) and a vehicle control group (dimethyl sulfoxide) treated in the same way as the solvent group were set up, with 2 wells of cells for each dose group. In the test system without activation, the administration times were 3 and 24 hours, and in the test system with S9 activation, the exposure time of the test substance was 3 hours. The upper limit for testing the test substance depends on its solubility in the medium, but generally, it will not exceed the maximum concentration of 1 mM or 0.5 mg/mL, whichever is lower. When selecting the highest dose for micronucleus frequency analysis, the cytotoxicity of the test substance dose group should not exceed 50% too much compared to that of the corresponding vehicle control group. If the maximum concentration was not limited by cytotoxicity, in order to reach the upper limit of solubility in the medium, multiple doses with visible precipitate can be tested, and a small amount of identifiable precipitate is visible in the medium of the highest dose group.

Dose selection for micronucleus analysis: The dose selection for micronucleus analysis of CHO-WBL cells will depend on the cytotoxicity of the test substance. The highest dose selected for assessment will induce cytotoxicity that does not exceed 50% too much. At least two additional groups with lower doses (in the range of moderately cytotoxic to non-cytotoxic, if applicable) were further analyzed. If the test substance is not cytotoxic, the dose at which the precipitate can be observed under a microscope at the end of the administration, or the maximum test concentration (1 mM or 0.5 mg/mL, whichever is lower) is selected as the maximum concentration for micronucleus analysis.

Micronucleus frequency counting: All slides selected for micronucleus analysis were labeled with randomly generated blind codes to reduce the subjectivity of reading the slides. The blind codes were made by personnel not involved in the reading. The blind code label on each slide contains the following information: experiment number, treatment series, and random number.

For each dose group, 2000 binuclear cells (1000 per well) were analyzed to count the frequency of binuclear cells containing micronuclei.

The binuclear cells should meet the following criteria to be counted:

a. The cell possesses an intact cell membrane. b. The two daughter nuclei are equal in size.

The criteria for judging micronuclei are as follows:

a. The micronucleus has a fluorescence intensity similar to that of the main nucleus. b. The micronucleus should be free in the cytoplasm. c. The micronucleus has a smooth edge and a diameter less than or equal to one-third of the diameter of the main nucleus.

The slides were discarded before signing the lab report.

**Data**

Data report: The cytotoxicity of the administration group was judged and presented based on its comparison with the cytotoxicity of the corresponding vehicle control. The micronucleus frequency for each well and each dose group in the main micronucleus assay was also presented.

Statistical analysis: Fisher's exact probability method was used to compare the significance of the difference in the frequency of binuclear cells containing micronuclei between the administration group and the corresponding vehicle control group. When making multiple comparisons, the P-value was corrected by Bonferroni. When comparing multiple groups of data, the Cochran-Armitage test was used to detect the dose-response relationship.

When $P \leq 0.05$, the difference was considered significant.

Criteria for experimental validity: An acceptable genotoxicity test must meet the following criteria: the frequency of micronucleus cells in the vehicle/negative control must be comparable to historical negative control data. There should be at least three concentrations that can be analyzed. The proportion of micronucleus cells in the positive control should be statistically significantly higher ($p \leq 0.05$) than that in the parallel vehicle/negative control group.

Criteria for judgment of experimental results: On the premise that the experiment is valid, according to the ICH S2

(R1) guidelines, the experimental results should be judged with reference to the results of micronucleus rates under different administration treatment conditions (3-hour treatment with S9, 3-hour treatment without S9, 24-hour treatment without S9) and at different concentrations of the test substance. Referring to the criteria for judging the experimental results, a comprehensive analysis should be conducted to judge the results of *in vitro* micronucleus assay of the test substance as negative, positive, or suspected positive, with negative results being preferred. The specific criteria for assessing the experimental results are as follows:

Positive: A test substance is considered positive if it meets the following criteria: a significant increase in the frequency of micronucleus cells is observed in one or more dose groups compared to the parallel vehicle control group. The increase in micronucleus frequency has a dose-response relationship. The frequency of micronucleus cells in one or more dose groups exceeds the range of historical negative data in the laboratory.

Negative: If none of the above three points are met, the test substance is considered negative.

Suspected positive: If a test substance only partially meets the above three points, it should be judged scientifically. Evidence of concentration-related effects is considered useful, but is not necessary in the evaluation of positive results. Biological relevance will be considered, such as the consistency of responses within the concentration range and between concentrations, as well as (if applicable) the consistency of responses between experiments, or effects that only occur at high or extremely strong cytotoxic concentrations.

The results for the affinity activity of each compound of the present disclosure for the $\alpha$2-$GABA_A$ receptor and the positive allosteric modulatory activity thereof on $\alpha$1- and $\alpha$2-$GABA_A$ receptors are shown in Table 3 below, and the results of the *in vitro* micronucleus assay are shown in Table 4 below.

It should be noted that the advantage of the compounds described in the present disclosure lies in their higher selectivity for the receptors, not just in the absolute activity of the drugs. At the same time, considering that the absolute value of the functional activity of the compounds may vary across different detection systems, which is not comparable, therefore, for the convenience of comparison, the experiments in the present disclosure were conducted in the same detection system to detect the control and the compounds involved simultaneously, and the positive compound (CVL-865) in Example 4 of WO2014091368A1 was used as a reference for normalization, that is, positive allosteric modulatory activity = [functional activity of the compound/functional activity of the positive compound] × 100%. The specific results are shown in Table 3 below.

Table 3 Affinity activity of compounds for $\alpha$2-$GABA_A$ receptor and positive allosteric modulatory activity thereof on $\alpha$1- and $\alpha$2-$GABA_A$ receptors

| No. | $\alpha$2 $K_i$ (nM) | $\alpha$1-$GABA_A$ | $\alpha$2-$GABA_A$ |
|---|---|---|---|
| CVL-865 | 2.23 | 100% | 100% |
| 1 | 6 | 13% | 151% |
| 8 | 4.76 | 79% | 130% |
| 10 | 26.91 | 1% | 98% |
| 11 | 16.42 | 75% | 97% |
| 16 | 11.63 | 38% | 104% |
| 17 | 14.43 | 47% | 125% |
| 18 | 13.93 | 30% | 106% |
| 22 | 10.04 | 53% | 115% |
| 26 | 8.95 | 48% | 120% |
| 28 | 8.56 | 38% | 115% |
| 31 | 6.85 | 13% | 96% |
| 34 | 5.71 | 70% | 124% |
| 35 | 7.73 | 80% | 134% |
| 36 | 5.73 | 25% | 137% |
| 42 | 2.92 | 27% | 145% |
| 49 | 9.16 | 58% | 94% |
| 51 | 3.44 | 14% | 90% |
| 52 | 3.95 | 17% | 106% |

(continued)

| No. | α2 $K_i$ (nM) | α1-$GABA_A$ | α2-$GABA_A$ |
|---|---|---|---|
| 53 | 3.78 | 21% | 94% |
| 55 | 2.43 | 21% | 81% |
| 63 | 3.51 | 37% | 81% |
| 69 | 3.09 | 56% | 102% |
| 70 | 0.50 | -27% | 90% |
| 74 | 6.99 | 9% | 104% |
| 76 | 2.43 | 61% | 107% |
| 77 | 1.13 | 66% | 104% |
| 78 | 3.91 | 10% | 110% |
| 81 | 2.34 | -25% | 83% |
| 87 | 3.38 | 3% | 114% |
| 93 | 8.32 | 79% | 115% |
| 99 | 4.31 | -2% | 106% |
| 101 | 4.46 | 56% | 157% |
| 111 | 3.35 | 64% | 102% |
| 112 | 7.62 | 64% | 171% |
| 114 | 9.97 | 68% | 107% |
| 115 | 16.38 | -25% | 85% |
| 117 | 15.22 | 37% | 86% |
| 119 | 15.43 | -10% | 86% |
| 121 | 3.02 | -6% | 90% |
| 122 | 4.76 | 39% | 125% |
| 123 | 7.70 | 33% | 95% |
| 126 | 5.22 | -35% | 90% |
| 129 | 3.23 | 56% | 128% |
| 130 | 4.80 | 36% | 139% |
| 133 | 12.70 | 16% | 130% |
| 135 | 28.20 | 68% | 142% |
| 139 | 13.18 | 35% | 122% |
| 141 | 9.15 | 37% | 105% |
| 142 | 4.54 | 36% | 99% |
| 157 | 23.33 | 50% | 99% |
| 161 | 8.70 | 65% | 118% |
| 162 | 14.51 | 42% | 114% |
| 164 | 9.24 | 29% | 122% |
| 166 | 2.39 | 12% | 128% |
| 167 | 13.87 | 35% | 125% |

The compounds in the examples of the present disclosure have better selectivity compared to the reference compound CVL-865, making the electrophysiological activity for the α1-$GABA_A$ receptor less than that of CVL-865 while

maintaining appropriate affinity and electrophysiological activity for the α2-$GABA_A$ receptor, so as to achieve smaller side effects while maintaining the *in vivo* efficacy.

Table 4 Results of *in vitro* micronucleus assay

| Example | Structure | Results of *in vitro* micronucleus assay |
|---|---|---|
| Example 4 of WO2014091368A1 (CVL-865) | 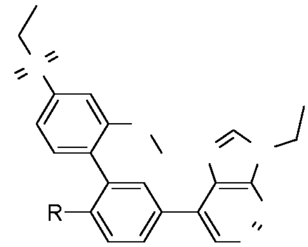 | Positive |
| Example 1 of WO2015189744A1 | 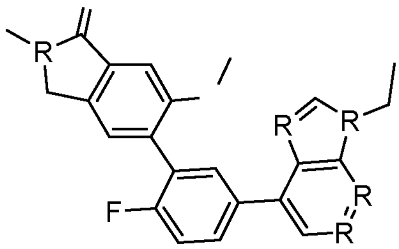 | Positive |
| Example 31 | 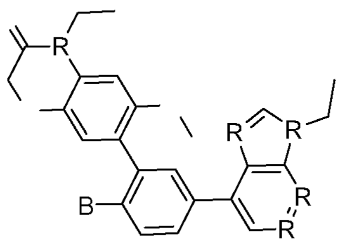 | Negative |
| Example 36 | 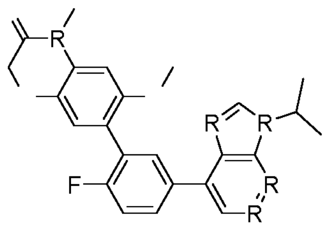 | Negative |
| Example 42 | 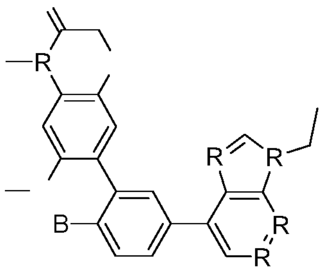 | Negative |
| Example 52 | 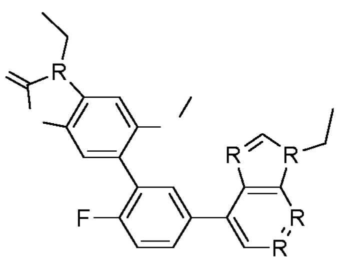 | Negative |

(continued)

| Example | Structure | Results of *in vitro* micronucleus assay |
|---|---|---|
| Example 69 | 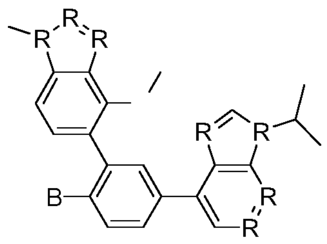 | Negative |
| Example 87 | 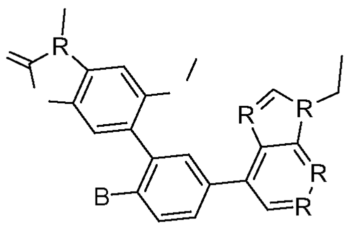 | Negative |
| Example 161 | 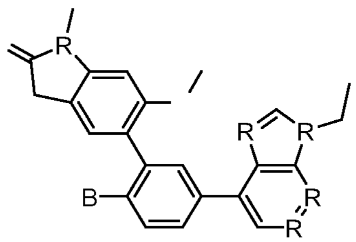 | Negative |

As can be seen from the results of *in vitro* micronucleus assay, the compounds of the present disclosure have better genotoxic safety and better druggability.

Obviously, the above examples are provided solely for the purpose of illustration and are not intended to limit the embodiments. Various changes and modifications can be made by those of ordinary skill in the art on the basis of the above description. It is neither necessary nor possible to exhaustively list all the embodiments. The obvious changes or modifications derived therefrom still fall within the scope of protection of the present disclosure.

## Claims

1. An imidazopyridazine-substituted benzene ring compound of formula (1), or a derivative, a stereoisomer, a tautomer, a prodrug, a pharmaceutically acceptable salt, an amorphous substance, an isotopic variant, a polymorph, or a solvate thereof:

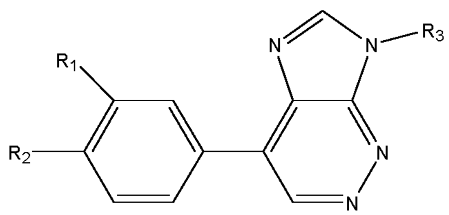

formula (1)

wherein

$R_1$ is a fused group formed by a substituted or unsubstituted benzene ring and a heterocyclic ring;

$R_2$ is selected from an H group, a halogen group, an OH group, a $C_1$-$C_6$ alkoxy group, or a CN group;
$R_3$ is selected from an H group, a substituted or unsubstituted linear or branched $C_1$-$C_6$ alkyl group, or a substituted or unsubstituted $C_3$-$C_6$ cycloalkyl group.

**2.** The compound according to claim 1, wherein the heterocyclic ring forming the fused group in $R_1$ is a 5- to 7-membered saturated or unsaturated monocyclic or bicyclic group containing 1 to 3 ring heteroatoms selected from N, O, or S.

**3.** The compound according to claim 1 or 2, wherein the $R_1$ has a structure of formula (M) as follows:

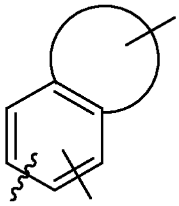

(M);

wherein m is 1, 2, or 3, n is 1 or 2, and 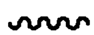 represents a connection site;
ring A refers to a 5- to 7-membered saturated or partially unsaturated monocyclic or bicyclic ring containing at least one heteroatom selected from N, O, or S.

**4.** The compound according to claim 3, wherein when the $R_1$ has the structure of formula (M), the $R_1$ has a structure of (a) to (e) as follows:

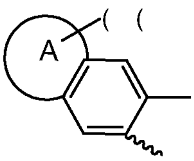 , 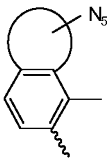 , 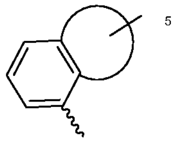 , 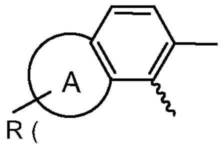 , or 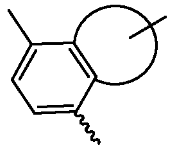 .

(a) (b) (c) (d) (e)

**5.** The compound according to claim 3 or 4, wherein when the $R_1$ has the structure of formula (M), the ring A is selected from the following structures:

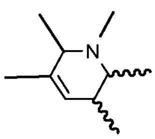 , 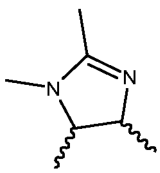 , 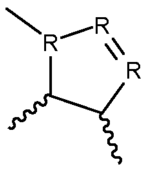 , 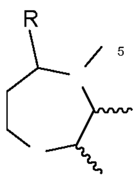 , 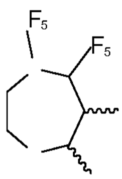 , 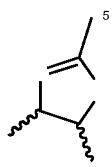 ,

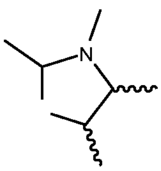 , 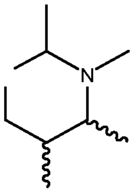 , 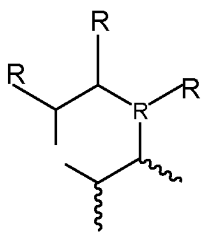 , 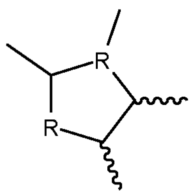 , 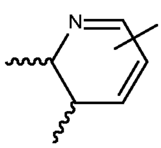 ,

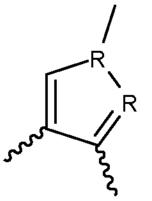, 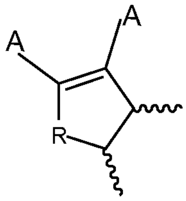, 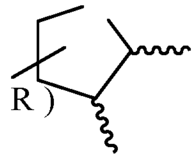, 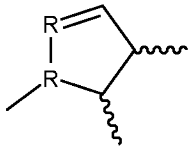, 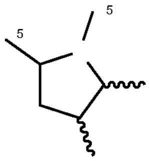,

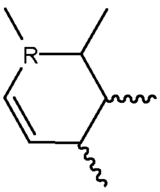, 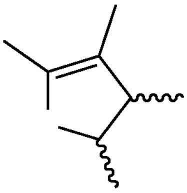, 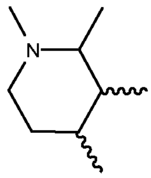, 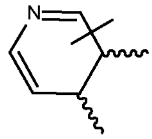, 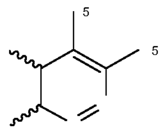, 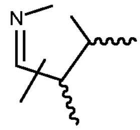,

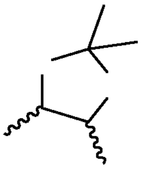, 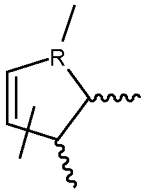, 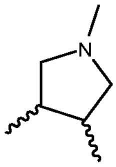, 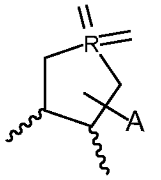, 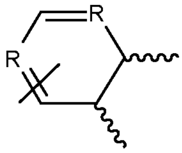, 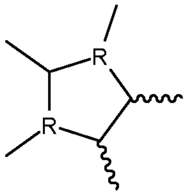,

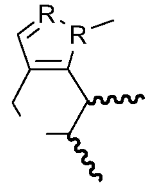, 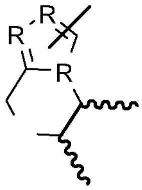, 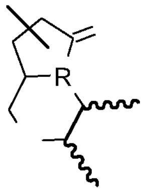, or 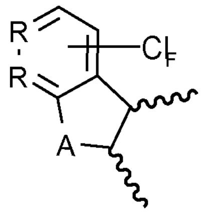.

**6.** The compound according to any one of claims 3 to 5, wherein $R_4$ is selected from a hydrogen group, a $C_1$-$C_6$ alkyl group, a $C_3$-$C_6$ cycloalkyl group, or a $C_1$-$C_6$ alkoxy group; the above groups are optionally unsubstituted or each independently substituted by 1 to 4 groups each selected from at least one of halogen, hydroxyl, $C_1$-$C_3$ alkyl, halo-$C_1$-$C_3$ alkyl, $C_1$-$C_3$ alkoxy, or halo-$C_1$-$C_3$ alkoxy.

**7.** The compound according to claim 6, wherein the $R_4$ is selected from a hydrogen group, a $C_1$-$C_3$ alkyl group, or a $C_1$-$C_3$ alkoxy group; the above groups are optionally unsubstituted or each independently substituted by a $C_1$-$C_3$ alkoxy group or a halogen group.

**8.** The compound according to any one of claims 3 to 7, wherein the $R_5$ is selected from a hydrogen group, a halogen group, a hydroxyl group, an oxo group, a $C_1$-$C_6$ alkylacyl group, a $C_1$-$C_6$ alkylamido group, a $C_1$-$C_6$ alkoxyimino group, a $C_1$-$C_6$ alkyl group, a $C_3$-$C_6$ cycloalkyl group, a $C_1$-$C_6$ alkoxy group, a $C_6$-$C_{10}$ aryl group, a 5- to 7-membered heteroaryl group containing 1 to 3 heteroatoms, or a $C_3$-$C_7$ non-aromatic heterocyclyl group containing 1 to 3 heteroatoms; the above groups are optionally unsubstituted or each independently substituted by 1 to 4 groups each selected from at least one of halogen, hydroxyl, oxo, $C_1$-$C_4$ acyl, $C_1$-$C_3$ alkyl, halo-$C_1$-$C_3$ alkyl, $C_1$-$C_3$ alkoxy, halo-$C_1$-$C_3$ alkoxy, or sulfonyl.

**9.** The compound according to claim 8, wherein the $R_5$ is selected from a hydrogen group, a halogen group, an oxo group, a $C_1$-$C_3$ alkylacyl group, a $C_1$-$C_3$ alkylamido group, a $C_1$-$C_3$ alkoxyimino group, a $C_1$-$C_3$ alkyl group, a $C_3$-$C_4$ cycloalkyl group, a $C_1$-$C_3$ alkoxy group, a $C_6$-$C_8$ aryl group, a 5- to 6-membered heteroaryl group containing 1 to 2 heteroatoms, or a $C_4$-$C_6$ non-aromatic heterocyclyl group containing 1 to 2 heteroatoms; the above groups are optionally unsubstituted or each independently substituted by 1 to 2 groups each selected from at least one of halogen, hydroxyl, oxo, $C_1$-$C_3$ acyl, $C_1$-$C_3$ alkyl, halo-$C_1$-$C_3$ alkyl, $C_1$-$C_3$ alkoxy, halo-$C_1$-$C_3$ alkoxy, or sulfonyl.

**10.** The compound according to claim 8 or 9, wherein the $R_5$ is selected from an H group, an oxo group, a methyl group, an

ethyl group, an isopropyl group, an acetyl group, a methylmethoxy group, an ethylmethoxy group, a cyclopropyl group, a methylcyclopropyl group, an ethylcyclopropyl group, a methylcyclobutyl group, a methylamino group, a phenyl group,

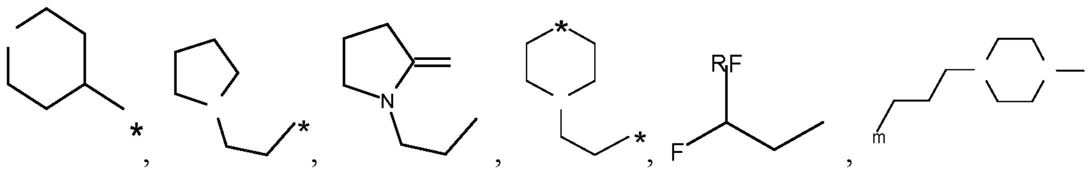
, , , , , ,

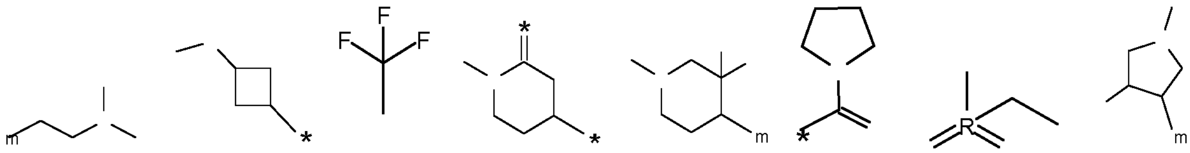
, , , , , , , ,

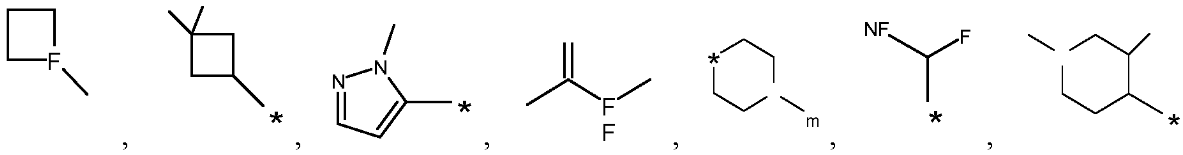
, , , , , , ,

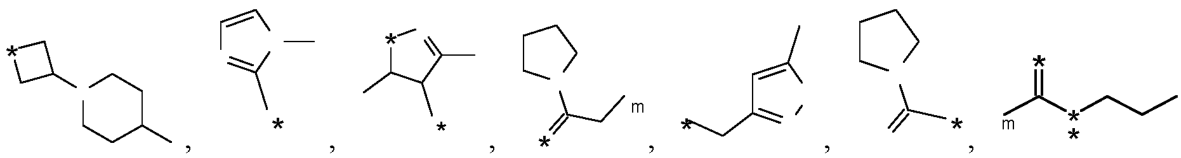
, , , , , , ,

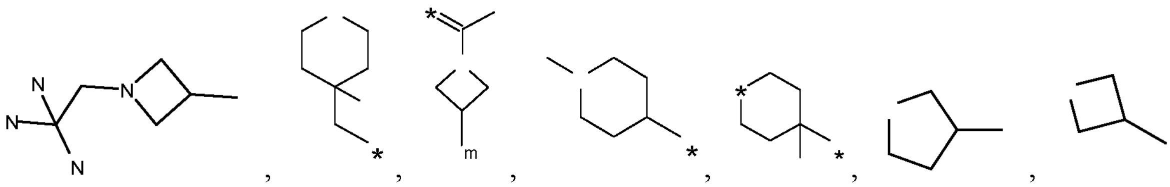
, , , , , , ,

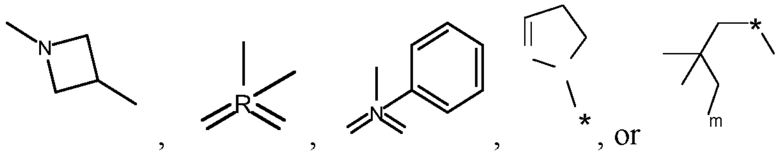
, , , , or .

11. The compound according to any one of claims 3 to 10, wherein the $R_1$ is selected from any one of the following structures:

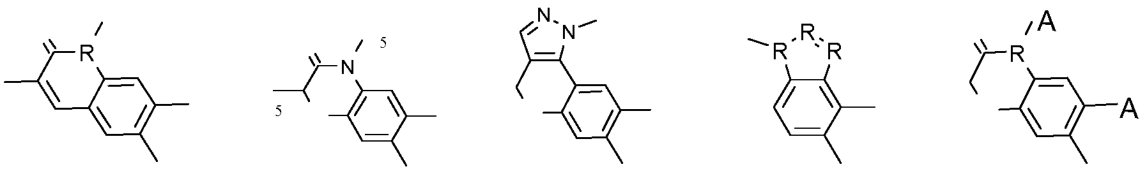
, , , , ,

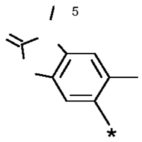, 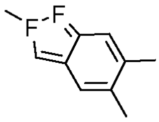, 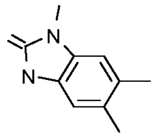, 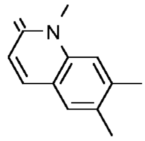, 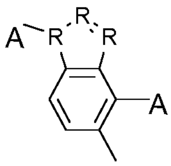,

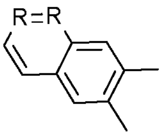, 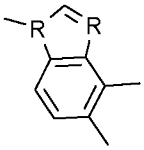, 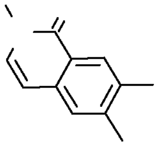, 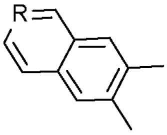, 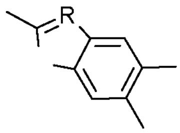,

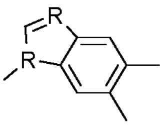, 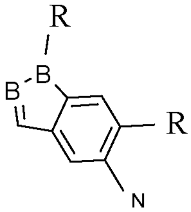, 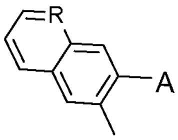, 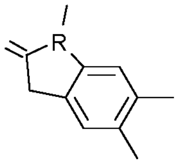, or 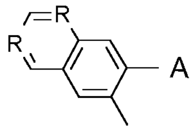;

and,

the $R_4$ is selected from an H group, a $C_1$-$C_3$ alkoxy group, or a substituted or unsubstituted methoxy-$C_1$-$C_3$ alkyl group; preferably an H group, a methylmethoxy group, an ethylmethoxy group, a methoxy group, or a difluoromethoxy group;

the $R_5$ is selected from an H group, a methyl group, an ethyl group, an acetyl group, an ethylmethoxy group, a methylmethoxy group, a methylcyclopropyl group, a phenyl group,

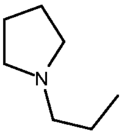, 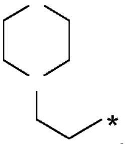, 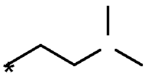, 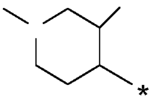, 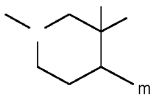, 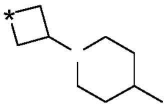, 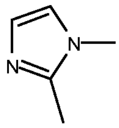,

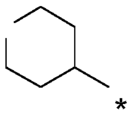, 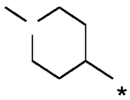, 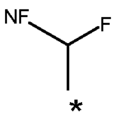, 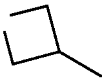, 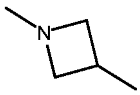, or 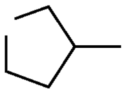.

**12.** The compound according to any one of claims 1 to 11, wherein

the $R_2$ is selected from an H group or an F group, preferably an F group;

the $R_3$ is selected from an H group or a linear or branched $C_1$-$C_6$ alkyl group, preferably an ethyl group or an isopropyl group.

**13.** The compound according to any one of claims 1 to 3, wherein the compound is selected from any one of the following compounds:

| No. | Structural formula | No. | Structural formula | No. | Structural formula |
|---|---|---|---|---|---|
| 1 | 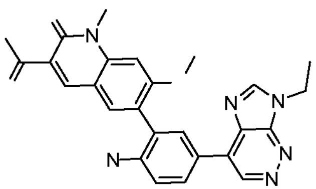 | 57 | 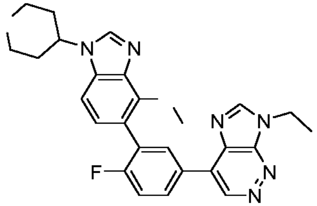 | 113 | 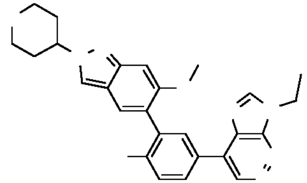 |
| 2 | 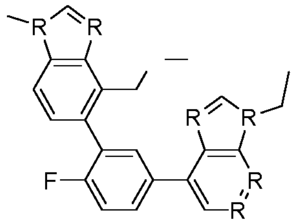 | 58 | 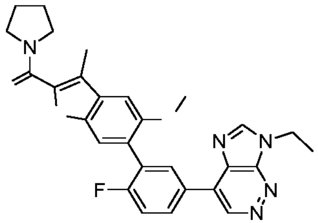 | 114 | 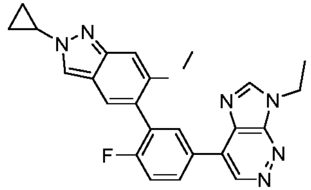 |
| 3 | 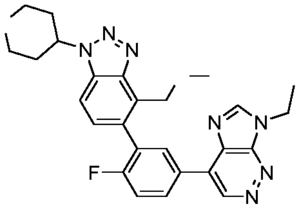 | 59 | 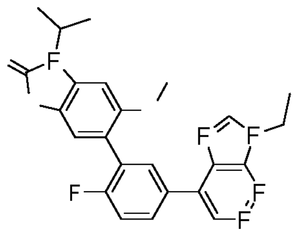 | 115 | 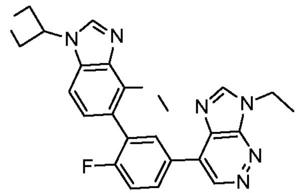 |
| 4 | 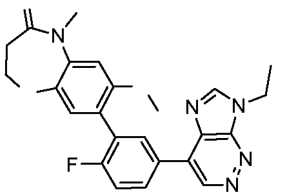 | 60 | 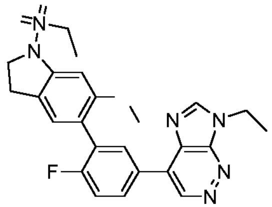 | 116 | 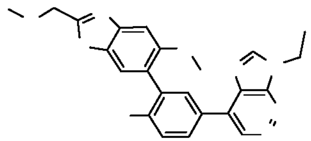 |

(continued)

| No. | Structural formula | No. | Structural formula | No. | Structural formula |
|---|---|---|---|---|---|
| 5 | 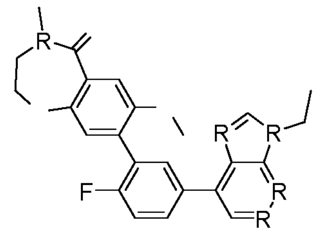 | 61 | 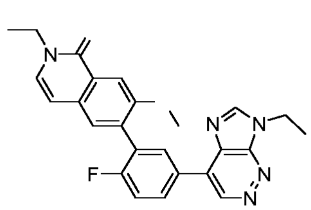 | 117 | 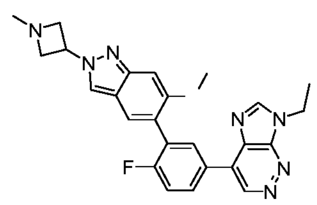 |
| 6 | 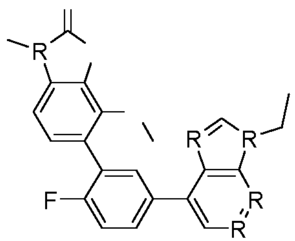 | 62 | 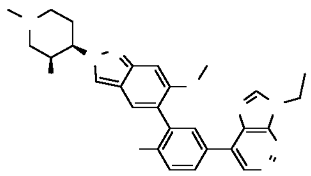 | 118 | 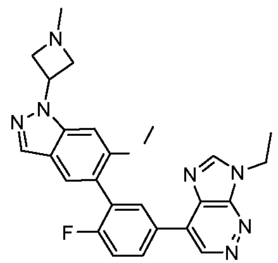 |
| 7 | 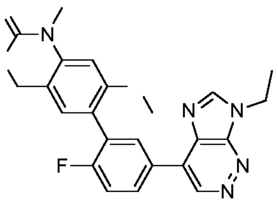 | 63 | 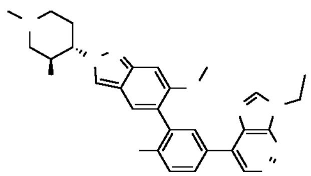 | 119 | 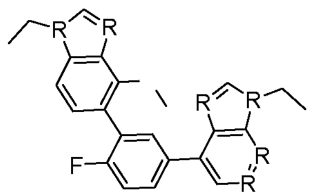 |
| 8 | 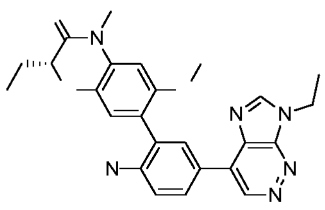 | 64 | 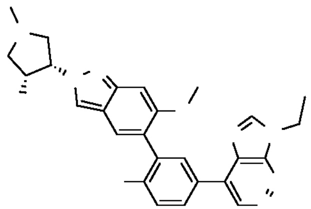 | 120 | 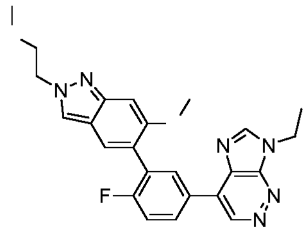 |

(continued)

| No. | Structural formula | No. | Structural formula | No. | Structural formula |
|---|---|---|---|---|---|
| 9 | 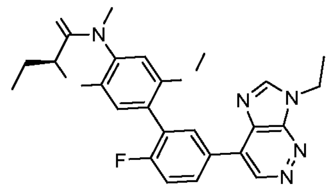 | 65 | 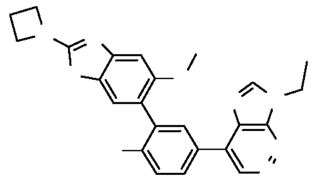 | 121 | 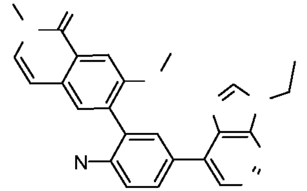 |
| 10 | 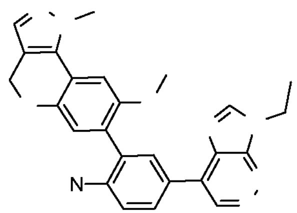 | 66 | 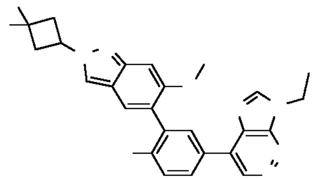 | 122 | 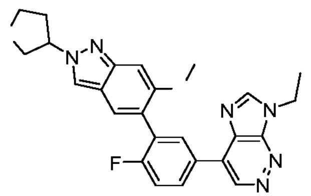 |
| 11 | 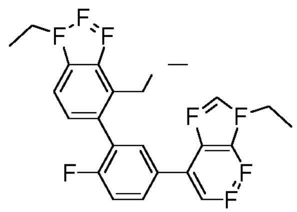 | 67 | 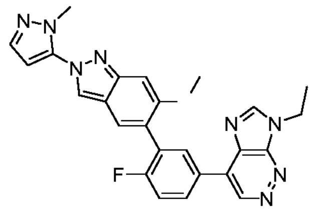 | 123 | 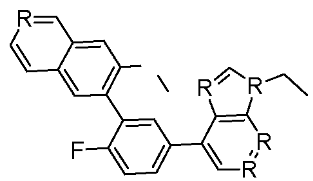 |
| 12 | 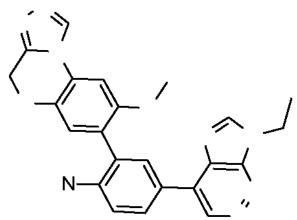 | 68 | 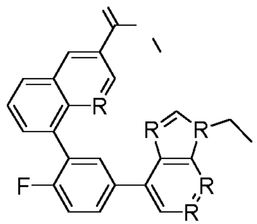 | 124 | 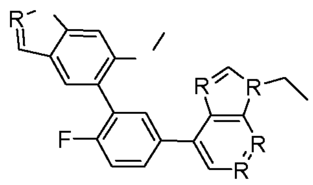 |

(continued)

| No. | Structural formula | No. | Structural formula | No. | Structural formula |
|---|---|---|---|---|---|
| 13 | 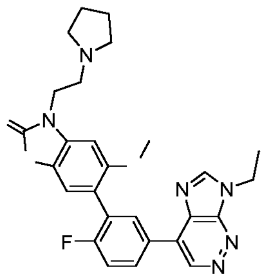 | 69 | 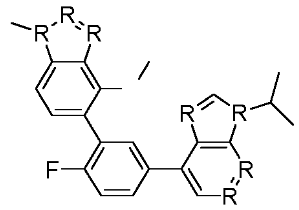 | 125 | 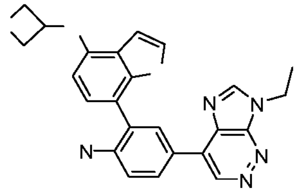 |
| 14 | 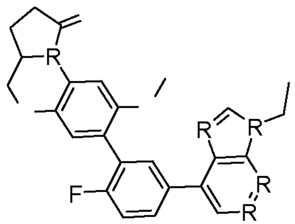 | 70 | 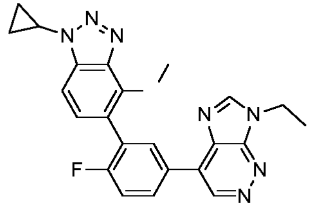 | 126 | 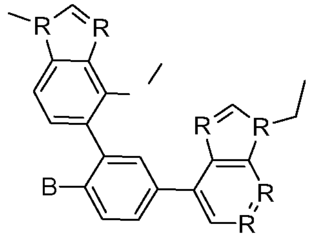 |
| 15 | 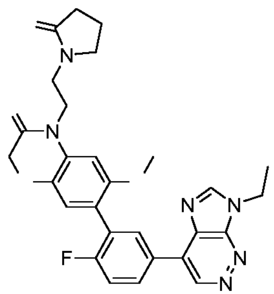 | 71 | 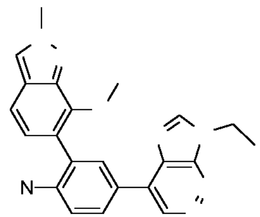 | 127 | 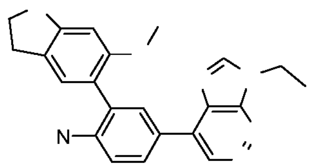 |
| 16 | 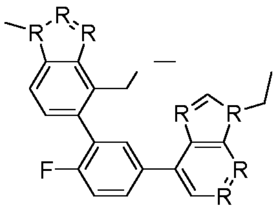 | 72 | 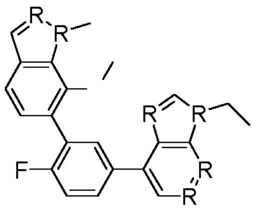 | 128 | 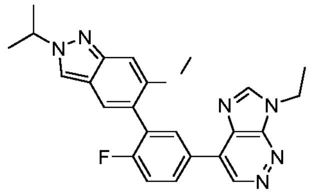 |

(continued)

| No. | Structural formula | No. | Structural formula | No. | Structural formula |
|---|---|---|---|---|---|
| 17 | 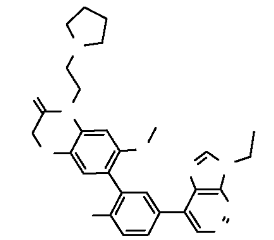 | 73 | 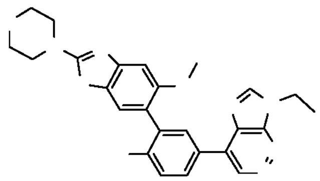 | 129 | 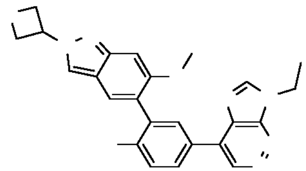 |
| 18 | 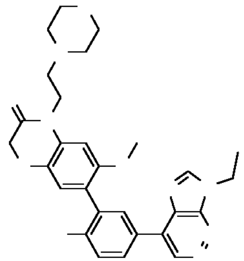 | 74 | 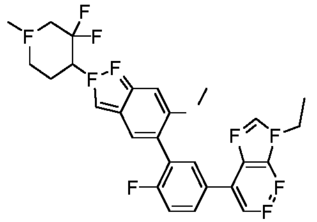 | 130 | 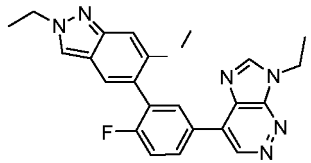 |
| 19 | 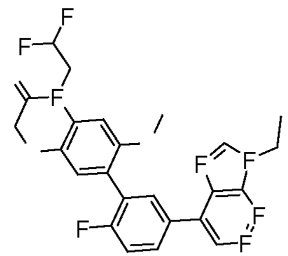 | 75 | 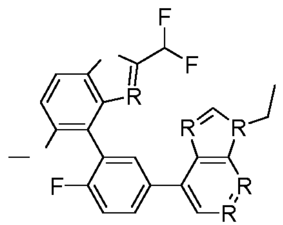 | 131 | 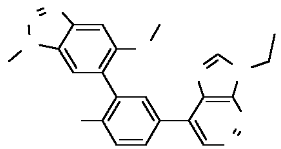 |
| 20 | 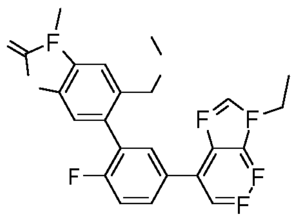 | 76 | 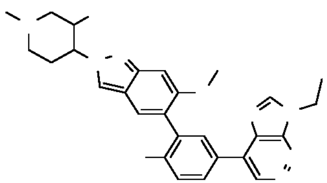 | 132 | 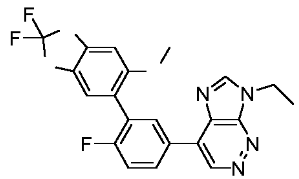 |

(continued)

| No. | Structural formula | No. | Structural formula | No. | Structural formula |
|---|---|---|---|---|---|
| 21 | 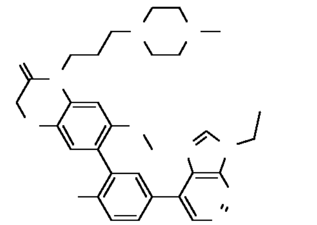 | 77 | 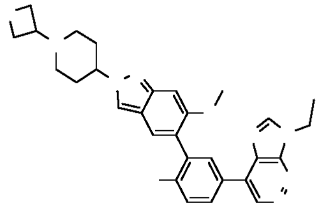 | 133 | 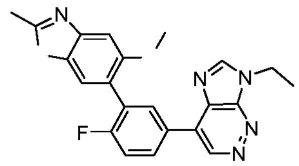 |
| 22 | 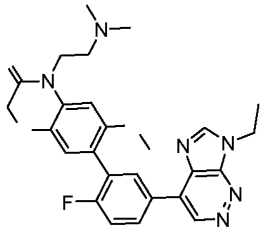 | 78 | 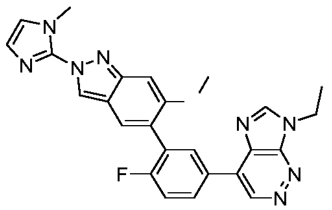 | 134 | 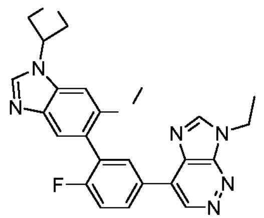 |
| 23 | 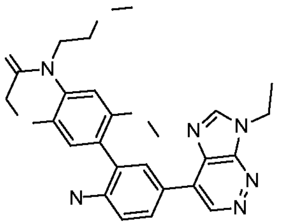 | 79 | 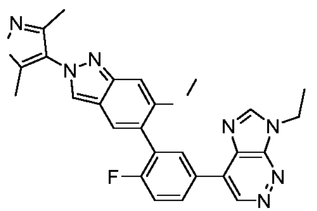 | 135 | 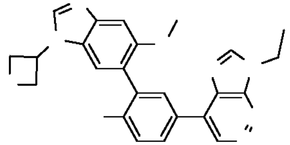 |
| 24 | 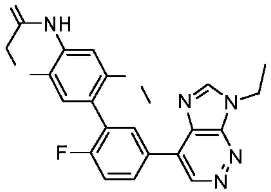 | 80 | 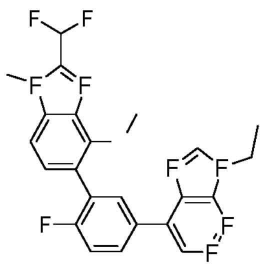 | 136 | 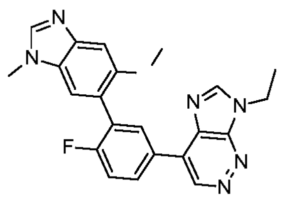 |

(continued)

| No. | Structural formula | No. | Structural formula | No. | Structural formula |
|---|---|---|---|---|---|
| 25 | 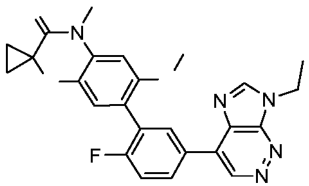 | 81 | 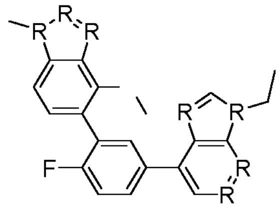 | 137 | 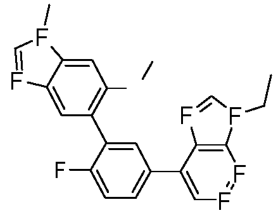 |
| 26 | 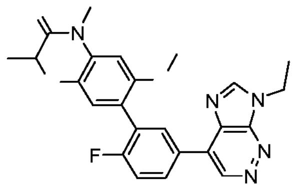 | 82 | 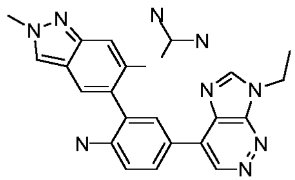 | 138 | 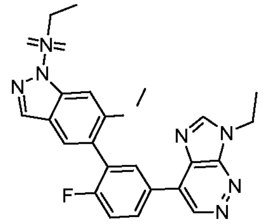 |
| 27 | 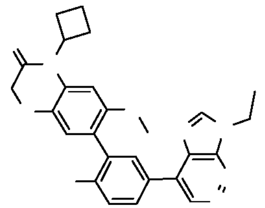 | 83 | 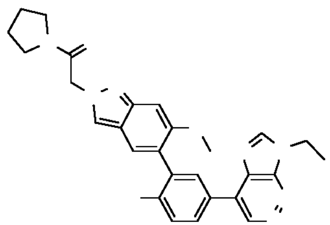 | 139 | 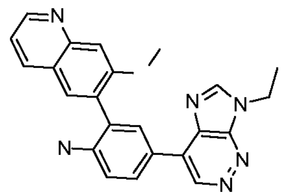 |
| 28 | 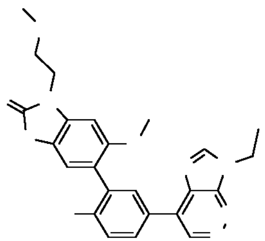 | 84 | 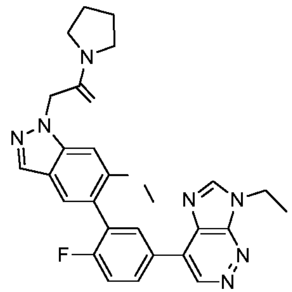 | 140 | 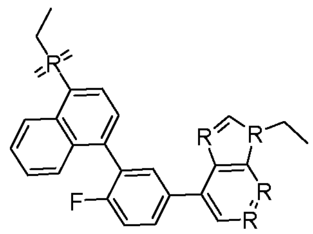 |

(continued)

| No. | Structural formula | No. | Structural formula | No. | Structural formula |
|---|---|---|---|---|---|
| 29 | 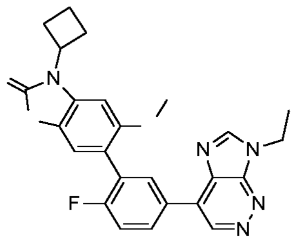 | 85 | 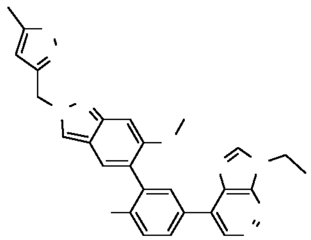 | 141 | 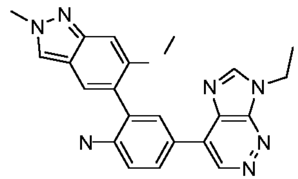 |
| 30 | 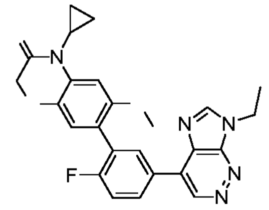 | 86 | 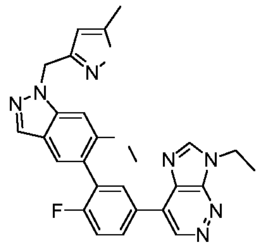 | 142 | 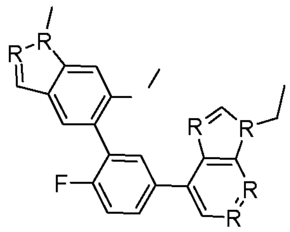 |
| 31 | 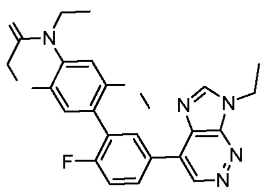 | 87 | 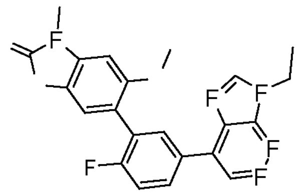 | 143 | 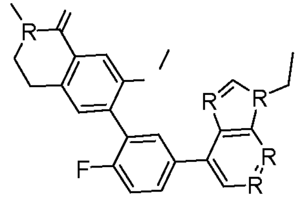 |
| 32 | 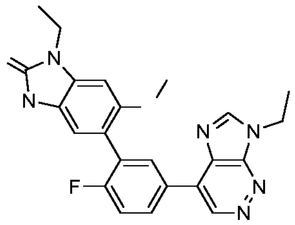 | 88 | 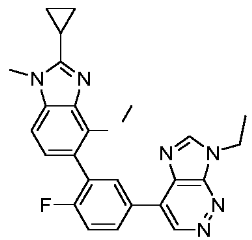 | 144 | 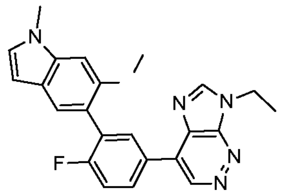 |

(continued)

| No. | Structural formula | No. | Structural formula | No. | Structural formula |
|---|---|---|---|---|---|
| 33 | 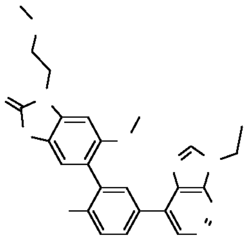 | 89 | 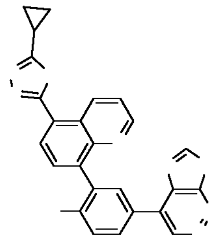 | 145 | 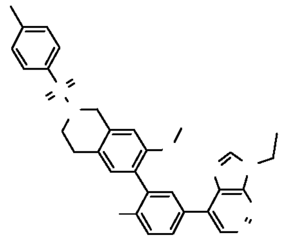 |
| 34 | 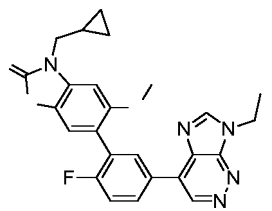 | 90 | 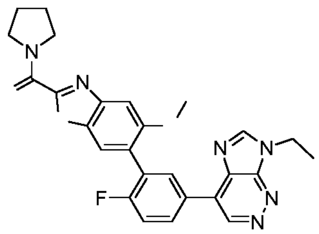 | 146 | 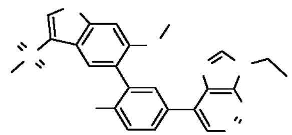 |
| 35 | 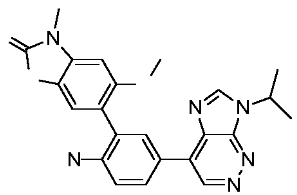 | 91 | 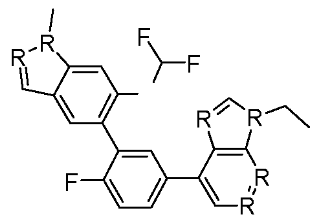 | 147 | 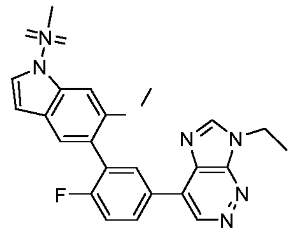 |
| 36 | 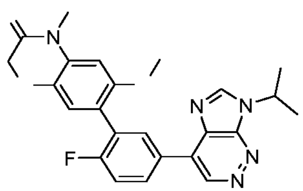 | 92 | 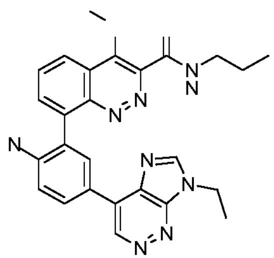 | 148 | 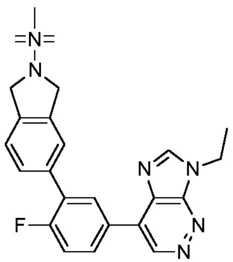 |

(continued)

| No. | Structural formula | No. | Structural formula | No. | Structural formula |
|---|---|---|---|---|---|
| 37 | 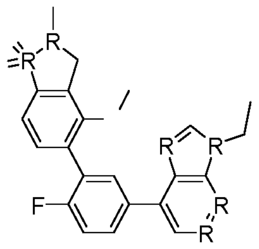 | 93 | 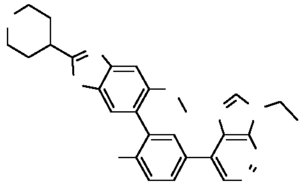 | 149 | 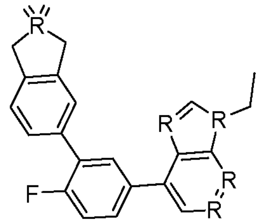 |
| 38 | 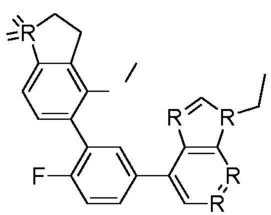 | 94 | 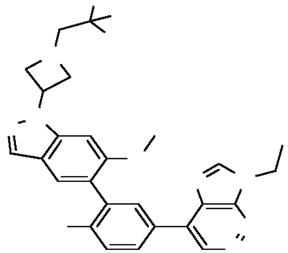 | 150 | 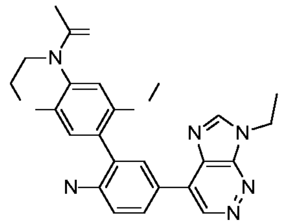 |
| 39 | 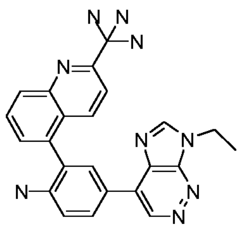 | 95 | 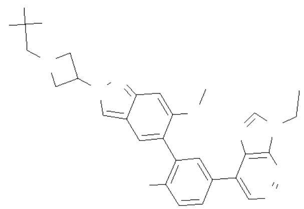 | 151 | 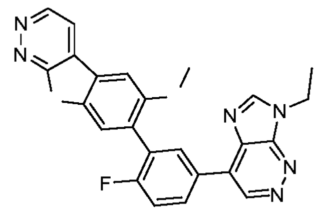 |
| 40 | 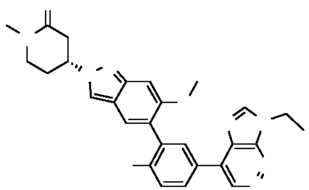 | 96 | 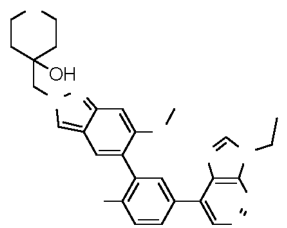 | 152 | 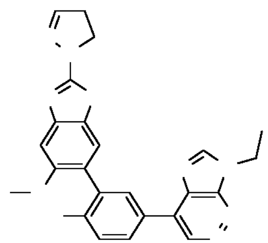 |

(continued)

| No. | Structural formula | No. | Structural formula | No. | Structural formula |
|---|---|---|---|---|---|
| 41 | 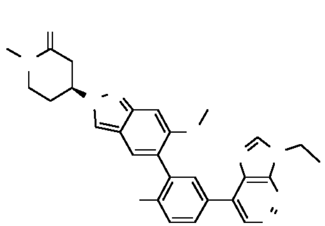 | 97 | 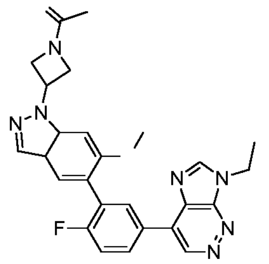 | 153 | 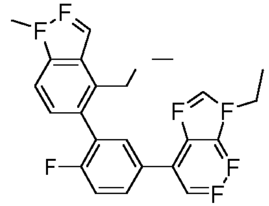 |
| 42 | 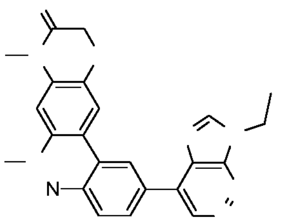 | 98 | 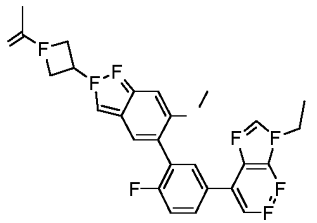 | 154 | 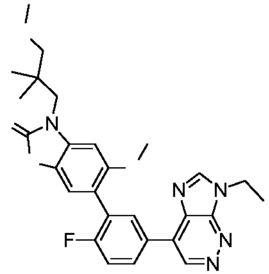 |
| 43 | 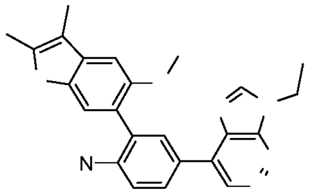 | 99 | 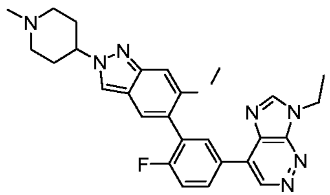 | 155 | 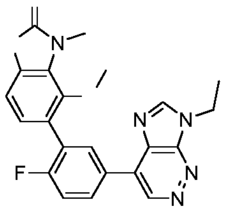 |
| 44 | 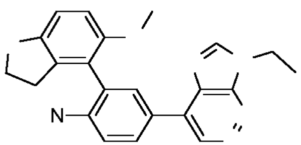 | 100 | 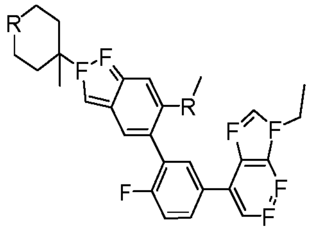 | 156 | 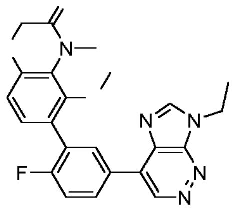 |

(continued)

| No. | Structural formula | No. | Structural formula | No. | Structural formula |
|---|---|---|---|---|---|
| 45 | 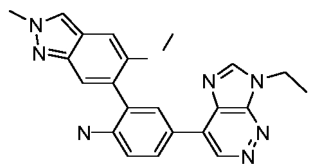 | 101 | 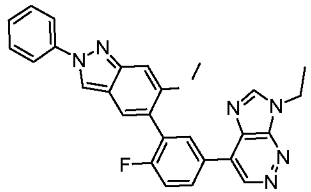 | 157 | 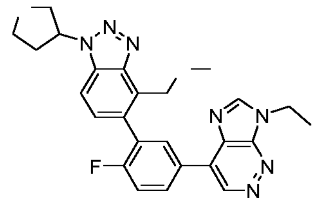 |
| 46 | 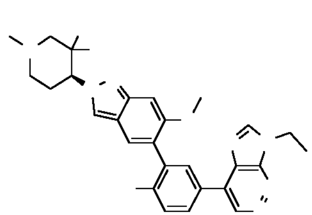 | 102 | 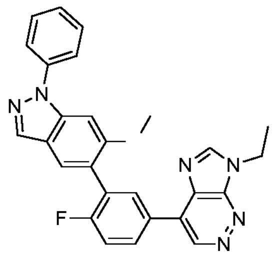 | 158 | 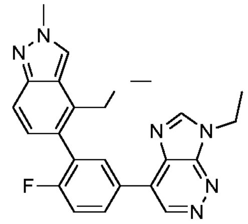 |
| 47 | 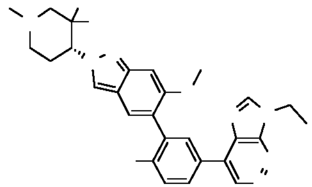 | 103 | 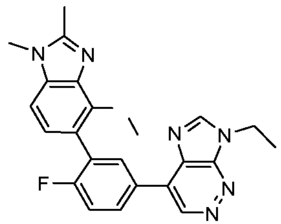 | 159 | 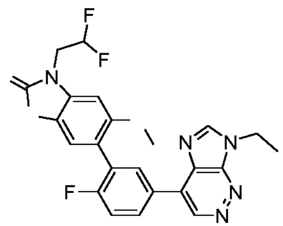 |
| 48 | 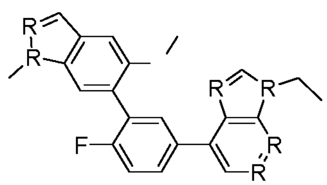 | 104 | 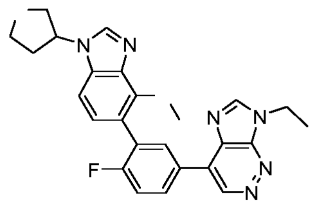 | 160 | 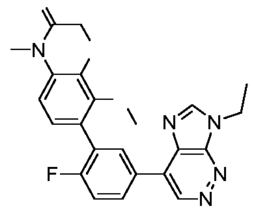 |

(continued)

| No. | Structural formula | No. | Structural formula | No. | Structural formula |
|---|---|---|---|---|---|
| 49 | 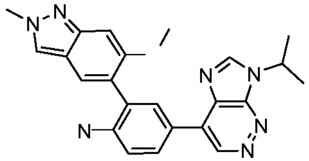 | 105 | 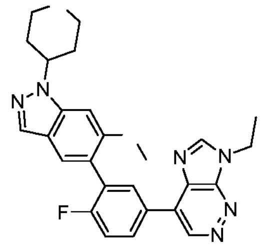 | 161 | 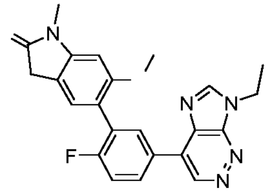 |
| 50 | 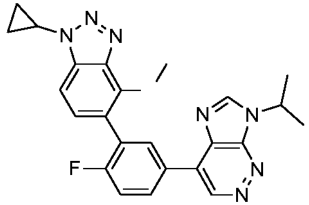 | 106 | 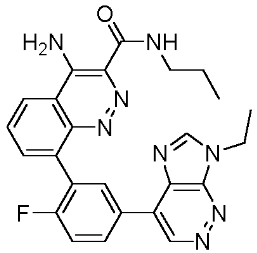 | 162 | 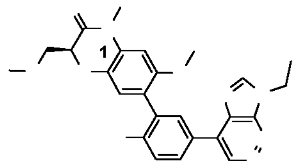 |
| 51 | 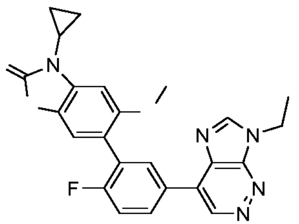 | 107 | 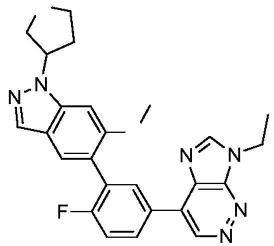 | 163 | 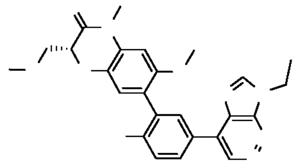 |
| 52 | 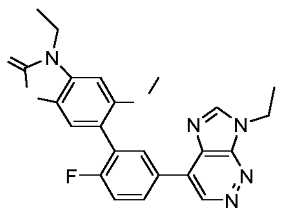 | 108 | 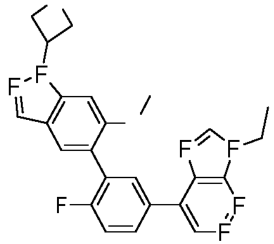 | 164 | 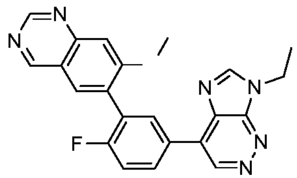 |

(continued)

| No. | Structural formula | No. | Structural formula | No. | Structural formula |
|---|---|---|---|---|---|
| 53 | 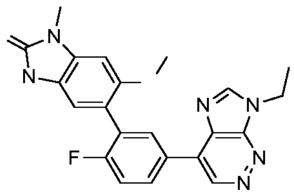 | 109 | 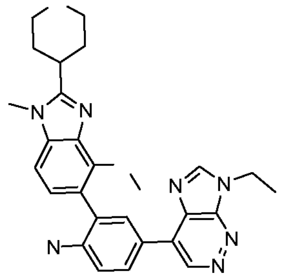 | 165 | 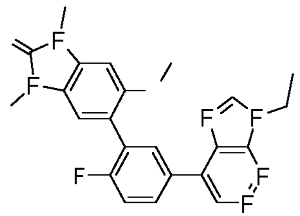 |
| 54 | 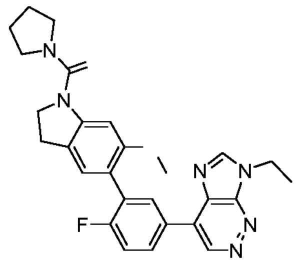 | 110 | 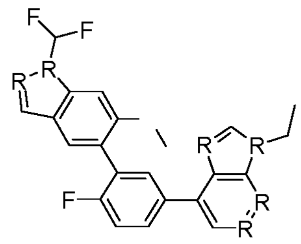 | 166 | 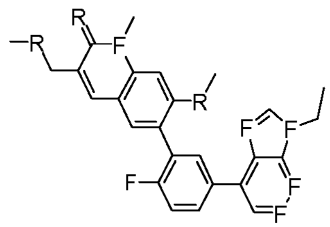 |
| 55 | 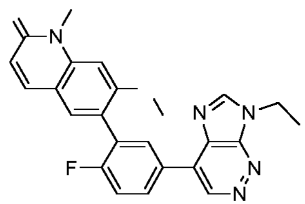 | 111 | 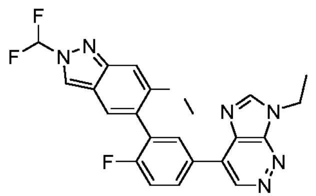 | 167 | 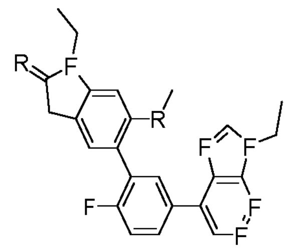 |
| 56 | 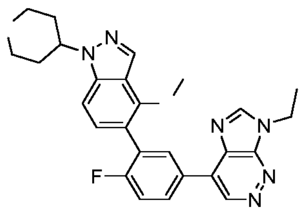 | 112 | 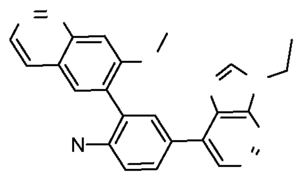 | 168 | 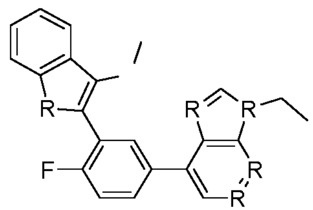 |

**14.** A pharmaceutical composition comprising at least one of the imidazopyridazine-substituted benzene ring compounds or the derivatives, the stereoisomers, the tautomers, the prodrugs, the pharmaceutically acceptable salts, the amorphous substances, the isotopic variants, the polymorphs, or the solvates thereof according to any one of claims 1 to 13, and optionally with the addition of a pharmaceutically acceptable carrier and/or adjuvant.

**15.** A use of the imidazopyridazine-substituted benzene ring compound or the derivative, the stereoisomer, the tautomer, the prodrug, the pharmaceutically acceptable salt, the amorphous substance, the isotopic variant, the polymorph, or the solvate thereof according to any one of claims 1 to 13, or the pharmaceutical composition according to claim 14 in the manufacture of a medicament for treating or preventing a $GABA_A$ receptor-associated disease.

**16.** The use according to claim 15, wherein the $GABA_A$ receptor-associated disease is selected from at least one of pain, Alzheimer's disease, multi-infarct dementia, epilepsy, pruritus, or stroke.

**17.** The use according to claim 16, wherein the pain comprises neuropathic pain, inflammatory pain, and cancer pain.

**18.** The use according to claim 16 or 17, wherein the pain is selected from: headache, facial pain, neck pain, shoulder pain, back pain, chest pain, abdominal pain, dorsalgia, lower back pain, lower limb pain, musculoskeletal pain, vascular pain, gout, arthritic pain, visceral pain, pain due to infectious disease, polyostotic pain, pain associated with sickle cell anemia, autoimmune disease, multiple sclerosis, or inflammation, chronic pain due to injury or surgery, nociceptive pain, diabetic pain, trigeminal neuralgia, pain of lumbar or cervical radiculopathy, glossopharyngeal neuralgia, autonomic nerve reflex pain, or pain associated with reflex sympathetic dystrophy, nerve root avulsion, cancer, chemical injury, toxin, nutritional deficiency, viral or bacterial infection, or degenerative osteoarthropathy.

**INTERNATIONAL SEARCH REPORT**

International application No.
**PCT/CN2023/084772**

**A. CLASSIFICATION OF SUBJECT MATTER**

C07D487/04(2006.01)i； C07D487/00 (2006.01)i； C07D417/14(2006.01)i； A61K31/5025(2006.01)i； A61P25/00(2006.01)i； A61P29/00(2006.01)i

According to International Patent Classification (IPC) or to both national classification and IPC

**B. FIELDS SEARCHED**

Minimum documentation searched (classification system followed by classification symbols)

C07D487/-; C07D417/-; A61K31/-; A61P25/-; A61P29/-

Documentation searched other than minimum documentation to the extent that such documents are included in the fields searched

Electronic data base consulted during the international search (name of data base and, where practicable, search terms used)

CNTXT; VEN; ENTXT; WOTXT; USTXT; EPTXT; GBTXT; JPTXT; CNKI; 万方, WANFANG; 百度学术, BAIDU SCHOLAR; Web of Science; STN: 上海赛默罗德生物科技有限公司, 王非, 陈南阳, 孙勇, 咪唑并哒嗪, 取代苯环, γ-氨基丁酸, 疼痛, SHANGHAI SIMRD BIOTECHNOLOGY CO. , LTD., WANG Fei, CHEN Nanyang, SUN Yong, imidazopyridazine, substituted phenyl, γ-aminobutyric acid, GABAA, pain, 式(I)结构

**C. DOCUMENTS CONSIDERED TO BE RELEVANT**

| Category* | Citation of document, with indication, where appropriate, of the relevant passages | Relevant to claim No. |
|---|---|---|
| X | WO 2015189744 A1 (PFIZER LIMITED) 17 December 2015 (2015-12-17)<br>description, embodiments 1, 2, 3, 5, 6, and 10, and pages 3-4 and 30-32 | 1-12, 14-18 |
| X | CN 104837843 A (PFIZER INC.) 12 August 2015 (2015-08-12)<br>description, pages 2-3, and embodiments 17 and 50 | 1-4, 6-10, 12 |
| Y | WO 2015189744 A1 (PFIZER LIMITED) 17 December 2015 (2015-12-17)<br>description, embodiments 1, 2, 3, 5, 6, and 10, and pages 3-4 and 30-32 | 1-18 |
| Y | CN 104837843 A (PFIZER INC.) 12 August 2015 (2015-08-12)<br>description, pages 2-3, and embodiments 17 and 50 | 1-18 |
| A | CN 112979655 A (SHANGHAI SIMR BIOTECHNOLOGY CO., LTD. et al.) 18 June 2021 (2021-06-18)<br>entire document | 1-18 |
| A | WO 2002038568 A1 (STREET, L. J. et al.) 16 May 2002 (2002-05-16)<br>entire document | 1-18 |

☑ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 July 2023** | **11 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/084772**

**C. DOCUMENTS CONSIDERED TO BE RELEVANT**

| Category* | Citation of document, with indication, where appropriate, of the relevant passages | Relevant to claim No. |
|---|---|---|
| A | Robert M. Owen et al. "Design and Identification of a Novel, Functionally Subtype Selective GABAA Positive Allosteric Modulator (PF-06372865)" *Journal of Medicinal Chemistry,* Vol. 62, No. 12, 09 April 2019 (2019-04-09), 5773-5796 ISSN: 1520-4804, pages 5773-5796 | 1-18 |

Form PCT/ISA/210 (second sheet) (July 2022)

**INTERNATIONAL SEARCH REPORT**
**Information on patent family members**

International application No.
**PCT/CN2023/084772**

| Patent document cited in search report | Publication date (day/month/year) | Patent family member(s) | Publication date (day/month/year) |
|---|---|---|---|
| WO 2015189744 A1 | 17 December 2015 | JP 2017517538 A | 29 June 2017 |
| | | JP 6491679 B2 | 27 March 2019 |
| | | CA 2951497 A1 | 17 December 2015 |
| | | CA 2951497 C | 09 April 2019 |
| | | ES 2664810 T3 | 23 April 2018 |
| | | EP 3154979 A1 | 19 April 2017 |
| | | EP 3154979 B1 | 07 March 2018 |
| | | US 2017197965 A1 | 13 July 2017 |
| | | US 9802945 B2 | 31 October 2017 |
| CN 104837843 A | 12 August 2015 | PH 12015501293 B1 | 24 August 2015 |
| | | AP 201508526 A0 | 30 June 2015 |
| | | EA 201590761 A1 | 30 December 2015 |
| | | EA 025635 B1 | 30 January 2017 |
| | | UY 35185 A | 31 July 2014 |
| | | ECSP 15029822 A | 31 August 2017 |
| | | EP 2938615 A1 | 04 November 2015 |
| | | EP 2938615 B1 | 26 October 2016 |
| | | ES 2608640 T3 | 12 April 2017 |
| | | US 2015259352 A1 | 17 September 2015 |
| | | AP 201508526 D0 | 30 June 2015 |
| | | CU 20150058 A7 | 27 November 2015 |
| | | CU 24338 B1 | 03 April 2018 |
| | | MX 2015007598 A | 22 October 2015 |
| | | MX 366023 B | 24 June 2019 |
| | | TN 2015000261 A1 | 03 October 2016 |
| | | HUE 032400 T2 | 28 September 2017 |
| | | CY 1118568 T1 | 12 July 2017 |
| | | SG 11201503735 YA | 30 July 2015 |
| | | WO 2014091368 A1 | 19 June 2014 |
| | | GT 201500159 A | 23 September 2015 |
| | | GEP 201706710 B | 25 July 2017 |
| | | NI 201500081 A | 16 September 2015 |
| | | US 2015105396 A1 | 16 April 2015 |
| | | CR 20150254 A | 09 July 2015 |
| | | AR 093937 A1 | 01 July 2015 |
| | | CL 2015001652 A1 | 14 August 2015 |
| | | DOP 2015000147 A | 15 September 2015 |
| | | LT 2938615 T | 27 December 2016 |
| | | AU 2013356894 A1 | 28 May 2015 |
| | | AU 2013356894 B2 | 12 November 2015 |
| | | BR 112015014097 A2 | 17 December 2019 |
| | | BR 112015014097 B1 | 29 November 2022 |
| | | TW 201434840 A | 16 September 2014 |
| | | TWI 478926 B | 01 April 2015 |
| | | US 2014171435 A1 | 19 June 2014 |
| | | US 8952008 B2 | 10 February 2015 |
| | | MA 38112 A1 | 30 March 2018 |
| | | MA 38112 B1 | 31 October 2018 |
| | | PE 20151163 A1 | 14 August 2015 |
| | | IL 239243 A0 | 30 July 2015 |

Form PCT/ISA/210 (patent family annex) (July 2022)

**INTERNATIONAL SEARCH REPORT**
**Information on patent family members**

International application No.
**PCT/CN2023/084772**

| Patent document cited in search report | Publication date (day/month/year) | Patent family member(s) | Publication date (day/month/year) |
|---|---|---|---|
| | | JP 2016503001 A | 01 February 2016 |
| | | JP 5869740 B2 | 24 February 2016 |
| | | HRP 20161559 T1 | 30 December 2016 |
| | | UA 112028 C2 | 11 July 2016 |
| | | PT 2938615 T | 26 December 2016 |
| | | MD 20150051 A2 | 30 September 2015 |
| | | MD 4651 B1 | 30 September 2019 |
| | | KR 20150088877 A | 03 August 2015 |
| | | KR 101746314 B1 | 12 June 2017 |
| | | ZA 201504226 B | 29 June 2016 |
| | | ME 02566 B | 20 February 2017 |
| | | PL 2938615 T3 | 28 April 2017 |
| | | SI 2938615 T1 | 28 February 2017 |
| | | DK 2938615 T3 | 02 January 2017 |
| | | RS 55307 B1 | 31 March 2017 |
| | | HK 1209743 A1 | 08 April 2016 |
| | | CA 2892174 A1 | 19 June 2014 |
| | | CA 2892174 C | 06 February 2018 |
| CN 112979655 A | 18 June 2021 | None | |
| WO 2002038568 A1 | 16 May 2002 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210542611
- GB 0104948 W
- WO 2002038568 A
- GB 0203114 W
- WO 2003008418 A
- US 9914935 W
- WO 2000001697 A
- WO IB2013060631 A
- WO 2014091368 A
- WO 2015189744 A
- US 2019033598 W
- WO 201926820 A1
- CN 107344936 A
- WO 2014091368 A1
- WO 2015189744 A1


### Non-patent literature cited in the description


- **MIRZA, N. R.** ; **MUNRO, G.** *Drug News and Perspectives*, 2010, vol. 23 (6), 351-360
- **ROBERT M. OWEN**. *J. Med. Chem.*, 2019, vol. 62, 5773-5796
- **DUVEAU, V.** *CNS Neurosci. Ther.*, 2019, vol. 25 (2), 255-260
- **UWE RUDOLPH** ; **FREDERIC KNOFLACH**. *Nature Reviews: Drug Discovery*, 2011, vol. 10 (9), 685-697
- Design of Prodrugs. Elsevier, 1985
- **BONICA et al.** The Management of Pain. Leas & Feboger, 1990, vol. 1
- Remington's Pharmaceutical Sciences. Maack Publishing Co
- *CHEMICAL ABSTRACTS*, 1445085-55-1
- *CHEMICAL ABSTRACTS*, 15969-08-1
- *CHEMICAL ABSTRACTS*, 15193-51-8
- *CHEMICAL ABSTRACTS*, 850363-67-6
- *CHEMICAL ABSTRACTS*, 705-15-7
- *CHEMICAL ABSTRACTS*, 1258833-80-5
- *CHEMICAL ABSTRACTS*, 20734-76-3
- *CHEMICAL ABSTRACTS*, 1008361-65-6
- *CHEMICAL ABSTRACTS*, 2081-44-9
- *CHEMICAL ABSTRACTS*, 19056-40-7
- **FELGNER, P.L. et al.** *Proceedings of the National Academy of Sciences*, 1987, vol. 84, 7413-7417
- **NICKOLLS, S.A et al.** *British Journal of Pharmacology*, 2018, vol. 175, 708-725